# EUROPEAN PATENT APPLICATION

(11) **EP 1 790 727 A2**
(43) Date of publication of application: **30.05.2007**
(21) Application number: 06026798.6
(22) Date of filing: 27.08.2001
(51) Int. Cl.: C12N 15/12, C07K 16/18, C07K 14/705, C12Q 1/68, A61K 38/17, A61K 48/00, A61K 39/395, C12N 1/15, C12N 1/21, C12N 5/10

(54) **Proteins and nucleic acids encoding same**

(30) Priority: 25.08.2000 US 227800 P; 25.08.2000 US 228205 P; 25.08.2000 US 228324 P; 30.08.2000 US 229185 P; 30.08.2000 US 228997 P; 01.09.2000 US 229780 P; 01.09.2000 US 229848 P; 01.09.2000 US 229850 P; 22.01.2001 US 263337 P; 31.01.2001 US 265518 P; 15.03.2001 US 276451 P; 27.03.2001 US 279196 P
(62) Divisional of application: 01964417.8
(71) Applicant: Curagen Corporation, Branford, CT 06511 (US)
(72) Inventor: Gerlach, Valerie, Branford, CT 06405 (US); MacDougall, John R., Hamden, CT 06517 (US); Stone, David J., Guilford, CT 06437 (US); Spytek, Kimberly A., New Haven, CT 06511 (US); Rastelli, Luca, Guilford, CT 06437 (US); Patturajan, Meera, Branford, CT 06405 (US); Padigaru, Muralidhara, Branford, CT 06405 (US); Smithson, Glennda, Guilford, CT 06435 (US); Ellerman, Karen, Branford, CT 06405 (US); Zerhusen, Bryan D., Branford, CT 06405 (US); Verney, Corine A. M., North Branford, CT 06471 (US); Tchernev, Velizar T., Branford, CT 06405 (US); Taupier, Raymond J., East Haven, CT 06512 (US)
(74) Representative: Naylor, Kathryn May

(57) **Abstract**

Disclosed herein are nucleic acid sequences that encode novel polypeptides. Also disclosed are polypeptides encoded by these nucleic acid sequences, and antibodies, which immunospecifically-bind to the polypeptide, as well as derivatives, variants, mutants, or fragments of the aforementioned polypeptide, polynucleotide, or antibody. The invention further discloses therapeutic, diagnostic and research methods for diagnosis, treatment, and prevention of disorders involving any one of these novel human nucleic acids and proteins.

SEQUENCE LISTING

<110> Curagen Corporation
      Gerlach, Valerie L
      MacDougall, John R
      Smithson, Glennda
      Stone, David J
      Ellerman, Karen
      Spytek, Kimberly A
      Zerhusen, Bryan D
      Rastelli, Luca
      Vernet, Corine AM
      Patturajan, Meera
      Tchernev, Velizar T
      Padigaru, Muralidhara
      Taupier Jr, Raymond J

<120> Novel Proteins and Nucleic Acids Encoding Same

<130> 21402-103-061

<140> PCT/US 01/26518
<141> 2001-08-27

<150> 09/939,398
<151> 2001-08-24

<150> 60/227,800
<151> 2000-08-25

<150> 60/228,205
<151> 2000-08-25

<150> 60/228,324
<151> 2000-08-25

<150> 60/228,997
<151> 2000-08-30

<150> 60/229,185
<151> 2000-08-30

<150> 60/229,780
<151> 2000-09-01

<150> 60/229,848
<151> 2000-09-01

<150> 60/229,850
<151> 2000-09-01

<150> 60/263,337
<151> 2001-01-22

<150> 60/265,518
<151> 2001-01-31

<150> 60/276,451
<151> 2001-03-15

<150> 60/279,196
<151> 2001-03-27

<160> 142

<170> PatentIn Ver. 2.1

<210> 1
<211> 620
<212> DNA
<213> Homo sapiens

<400> 1
tgtaaaacaa gaccaggcac aagaagggtg acatccccaa gtccccagaa gaagccatcc 60
agcacaagga gggtgacatt cccaagtctc caaaacaagc catccagccc aaggagggtg 120
acattcccaa gtccctagag gaagccatcc cacccaagga gattgacatc cccaagtccc 180
cagaagaaac catccagccc aaggaggatg acagccccaa gtccctagaa gaagccaccc 240
catccaagga gggtgacatc ctaaagcctg aagaagaaac aatggagttc ccggaggggg 300
acaaggtgaa agtgatcctg agcaaggagg actttgagac atcactgaag gaggccgggg 360
agaggctggt ggctgtggac ttctcggcca cgtggtgtgg gccctgcagg accatcagac 420
cattcttcca tgccctgtct gtgaagcatg aggatgtggt gttcctggag gtggacgctg 480
acaactgtga ggaggtggtg agagagtgcg ccatcatgtg tgtcccaacc tttcagtttt 540
ataaaaaaga agaaaaggtg gatgaacttt gcggcgccct taaggaaaaa cttgaagcag 600
tcattgcaga attaaagtaa 620

<210> 2
<211> 112
<212> PRT
<213> Homo sapiens

<400> 2
Met Glu Phe Pro Glu Gly Asp Lys Val Lys Val Ile Leu Ser Lys Glu
1               5                   10                  15

Asp Phe Glu Thr Ser Leu Lys Glu Ala Gly Glu Arg Leu Val Ala Val
            20                  25                  30

Asp Phe Ser Ala Thr Trp Cys Gly Pro Cys Arg Thr Ile Arg Pro Phe
        35                  40                  45

Phe His Ala Leu Ser Val Lys His Glu Asp Val Val Phe Leu Glu Val
    50                  55                  60

Asp Ala Asp Asn Cys Glu Glu Val Val Arg Glu Cys Ala Ile Met Cys
65                  70                  75                  80

Val Pro Thr Phe Gln Phe Tyr Lys Lys Glu Glu Lys Val Asp Glu Leu
                85                  90                  95

Cys Gly Ala Leu Lys Glu Lys Leu Glu Ala Val Ile Ala Glu Leu Lys
            100                 105                 110

<210> 3
<211> 2632
<212> DNA
<213> Homo sapiens

<400> 3
cgcctggggt caaaggagaa gggccagaga atgtcgtccc agccagcagg gaaccagacc 60
tcccccgggg ccacagagga ctactcctat ggcagctggt acatcgatga gccccagggg 120
ggcgaggagc tccagccaga gggggtgccc tcctgccaca ccagcatacc acccggcctg 180
taccacgcct gcctggcctc gctgtcattt cagatccttg tgctgctgct cctggccatg 240
ctggtgaggc gccgccagct ctggcctcag tgtggacatg gccttagccc tgtggatttc 300
ttggctgggg acaggccccg ggcagtgcct gctgctgttt tcatggtcct cctgagctcc 360
ctgtgtttgc tgctccccga cgaggacgca ttgcccttcc tgactctcgc ctcagcaccc 420
agcggggcct ggaagatact gggactgttc tattatgctg ccctctacta ccctctggct 480
gcctgtgcca cggctggcca cacagctgca cacctgctcg gcagcacgct gtcctgggcc 540
caccttgggg tccaggtctg gcagagggca gagtgtcccc aggtgcccaa gatctacaag 600
tactactccc tgctggcctc cctgcctctc ctgctgggcc tcggattcct gagcctttgg 660
taccctgtgc agctggtgag aagcttcagc cgtaggacag gagcaggctc ccaggggctg 720
cagagcagct actctgagga atatctgagg aacctccttt gcaggaagaa gctgggaagc 780
tgcagctacc acacctccaa gcatggcttc ctgtcctggg cccgcgtctg cttgagacac 840
tgcatctaca ctccacagcc aggattccat ctcccgctga agctggtgct ttcagctaca 900
ctgacaggga cggccattta ccaggtagcc ctgctgctgc tggtgggcgt ggtacccact 960
atccagaagg tgagggcagg ggtcaccacg gatgtctcct acctgctggc cggctttgga 1020
atcgtgctct ccgaggacaa gcaggaggtg gtggagctgg tgaagcacca tctgtgggct 1080
ctggaatgct acatctcagc cttggtcttg tcctgcttac tcaccttcct ggtcctgatg 1140
cgctcactgg tgacacacag gaccaacctt cgagctctgc accgaggagc tgccctggac 1200
ttgagtccct tgcatcggag tccccatccc tcccgccaag ccatattctg ttggatgagc 1260
ttcagtgcct accagacagc ctttatctgc cttctcccgc ccctggcagg gctcctggtg 1320
cagcagatca tcttcttcct gggaaccacg gccctggcct tcctggtgct catgcctgtg 1380
ctccatggca ggaacctcct gctcttccgt tccctggagt cctcgtggcc cttctggctg 1440
actttggccc tggctgtgat cctgcagaac atggcagccc attgggtctt cctggagact 1500
catgatggac acccacagct gaccaaccgg cgagtgctct atgcagccac ctttcttctc 1560
ttccccctca atgtgctggt gggtgccatg gtggccacct ggcgagtgct cctctctgcc 1620
ctctacaacg ccatccacct tggccagatg gacctcagcc tgctgccacc gagagccgcc 1680
actctcgacc ccggctacta cacgtaccga aacttcttga agattgaagt cagccagtcg 1740
catccagcca tgacagcctt ctgctccctg ctcctgcaag cgcagagcct cctacccagg 1800
accatggcag ccccccagga cagcctcaga ccaggggagg aagacgaagg gatgcagctg 1860
ctacagacaa aggactccat ggccaaggga gctaggcccg gggccagccg cggcagggct 1920
cgctggggtc tggcctacac gctgctgcac aacccaaccc tgcaggtctt ccgcaagacg 1980
gccctgttgg gtgccaatgg tgcccagccc tgagggcagg gaaggtcaac ccacctgccc 2040
atctgtgctg aggcatgttc ctgcctacca tcctcctccc tccccggctc tcctcccagc 2100
atcacaccag ccatgcagcc agcaggtcct ccggatcacc gtggttgggt ggaggtctgt 2160
ctgcactggg agcctcagga gggctctgct ccacccactt ggctatggga gagccagcag 2220
gggttctgga gaaaaaaact ggtgggttag ggccttggtc caggagccag ttgagccagg 2280
gcagccacat ccaggcgtct ccctaccctg gctctgccat cagccttgaa gggcctcgat 2340
gaagccttct ctggaaccac tccagcccag ctccacctca gccttggcct tcacgctgtg 2400
gaagcagcca aggcacttcc tcaccccctc agcgccacgg acctctctgg ggagtggccg 2460
gaaagctccc gggcctctgg cctgcagggc agcccaagtc atgactcaga ccaggtccca 2520
cactgagctg cccacactcg agagccagat atttttgtag tttttatgcc tttggctatt 2580
atgaaagagg ttagtgtgtt ccctgcaata aacttggtcc tgagaaaaaa aa 2632

<210> 4
<211> 660

<212> PRT
<213> Homo sapiens

<400> 4
Met Ser Ser Gln Pro Ala Gly Asn Gln Thr Ser Pro Gly Ala Thr Glu
1               5                   10                  15

Asp Tyr Ser Tyr Gly Ser Trp Tyr Ile Asp Glu Pro Gln Gly Gly Glu
            20                  25                  30

Glu Leu Gln Pro Glu Gly Val Pro Ser Cys His Thr Ser Ile Pro Pro
        35                  40                  45

Gly Leu Tyr His Ala Cys Leu Ala Ser Leu Ser Phe Gln Ile Leu Val
    50                  55                  60

Leu Leu Leu Leu Ala Met Leu Val Arg Arg Arg Gln Leu Trp Pro Gln
65                  70                  75                  80

Cys Gly His Gly Leu Ser Pro Val Asp Phe Leu Ala Gly Asp Arg Pro
                85                  90                  95

Arg Ala Val Pro Ala Ala Val Phe Met Val Leu Leu Ser Ser Leu Cys
            100                 105                 110

Leu Leu Leu Pro Asp Glu Asp Ala Leu Pro Phe Leu Thr Leu Ala Ser
        115                 120                 125

Ala Pro Ser Gly Ala Trp Lys Ile Leu Gly Leu Phe Tyr Tyr Ala Ala
    130                 135                 140

Leu Tyr Tyr Pro Leu Ala Ala Cys Ala Thr Ala Gly His Thr Ala Ala
145                 150                 155                 160

His Leu Leu Gly Ser Thr Leu Ser Trp Ala His Leu Gly Val Gln Val
                165                 170                 175

Trp Gln Arg Ala Glu Cys Pro Gln Val Pro Lys Ile Tyr Lys Tyr Tyr
            180                 185                 190

Ser Leu Leu Ala Ser Leu Pro Leu Leu Leu Gly Leu Gly Phe Leu Ser
        195                 200                 205

Leu Trp Tyr Pro Val Gln Leu Val Arg Ser Phe Ser Arg Arg Thr Gly
    210                 215                 220

Ala Gly Ser Gln Gly Leu Gln Ser Ser Tyr Ser Glu Glu Tyr Leu Arg
225                 230                 235                 240

Asn Leu Leu Cys Arg Lys Lys Leu Gly Ser Cys Ser Tyr His Thr Ser
                245                 250                 255

Lys His Gly Phe Leu Ser Trp Ala Arg Val Cys Leu Arg His Cys Ile
            260                 265                 270

Tyr Thr Pro Gln Pro Gly Phe His Leu Pro Leu Lys Leu Val Leu Ser
        275                 280                 285

Ala Thr Leu Thr Gly Thr Ala Ile Tyr Gln Val Ala Leu Leu Leu Leu
    290                 295                 300

Val Gly Val Val Pro Thr Ile Gln Lys Val Arg Ala Gly Val Thr Thr
305                 310                 315                 320

Asp Val Ser Tyr Leu Leu Ala Gly Phe Gly Ile Val Leu Ser Glu Asp
                325                 330                 335

Lys Gln Glu Val Val Glu Leu Val Lys His His Leu Trp Ala Leu Glu
            340                 345                 350

Cys Tyr Ile Ser Ala Leu Val Leu Ser Cys Leu Leu Thr Phe Leu Val
        355                 360                 365

Leu Met Arg Ser Leu Val Thr His Arg Thr Asn Leu Arg Ala Leu His
    370                 375                 380

Arg Gly Ala Ala Leu Asp Leu Ser Pro Leu His Arg Ser Pro His Pro
385                 390                 395                 400

Ser Arg Gln Ala Ile Phe Cys Trp Met Ser Phe Ser Ala Tyr Gln Thr
                405                 410                 415

Ala Phe Ile Cys Leu Leu Pro Pro Leu Ala Gly Leu Leu Val Gln Gln
            420                 425                 430

Ile Ile Phe Phe Leu Gly Thr Thr Ala Leu Ala Phe Leu Val Leu Met
        435                 440                 445

Pro Val Leu His Gly Arg Asn Leu Leu Leu Phe Arg Ser Leu Glu Ser
    450                 455                 460

Ser Trp Pro Phe Trp Leu Thr Leu Ala Leu Ala Val Ile Leu Gln Asn
465                 470                 475                 480

Met Ala Ala His Trp Val Phe Leu Glu Thr His Asp Gly His Pro Gln
                485                 490                 495

Leu Thr Asn Arg Arg Val Leu Tyr Ala Ala Thr Phe Leu Leu Phe Pro
            500                 505                 510

Leu Asn Val Leu Val Gly Ala Met Val Ala Thr Trp Arg Val Leu Leu
        515                 520                 525

Ser Ala Leu Tyr Asn Ala Ile His Leu Gly Gln Met Asp Leu Ser Leu
    530                 535                 540

Leu Pro Pro Arg Ala Ala Thr Leu Asp Pro Gly Tyr Tyr Thr Tyr Arg
545                 550                 555                 560

Asn Phe Leu Lys Ile Glu Val Ser Gln Ser His Pro Ala Met Thr Ala
                565                 570                 575

Phe Cys Ser Leu Leu Leu Gln Ala Gln Ser Leu Leu Pro Arg Thr Met
            580                 585                 590

Ala Ala Pro Gln Asp Ser Leu Arg Pro Gly Glu Glu Asp Glu Gly Met
      595                 600                 605

Gln Leu Leu Gln Thr Lys Asp Ser Met Ala Lys Gly Ala Arg Pro Gly
    610                 615                 620

Ala Ser Arg Gly Arg Ala Arg Trp Gly Leu Ala Tyr Thr Leu Leu His
625                 630                 635                 640

Asn Pro Thr Leu Gln Val Phe Arg Lys Thr Ala Leu Leu Gly Ala Asn
                645                 650                 655

Gly Ala Gln Pro
            660

<210> 5
<211> 2663
<212> DNA
<213> Homo sapiens

<400> 5
atgtcgtccc agccagcagg gaaccagacc tcccccgggg ccacagagga ctactcctat 60
ggcagctggt acatcgatga gccccagggg ggcgaggagc tccagccaga gggggaagtg 120
ccctcctgcc acaccagcat accacccggc ctgtaccacg cctgcctggc ctcgctgtca 180
atccttgtgc tgctgctcct ggccatgctg gtgaggcgcc gccagctctg gcctgactgt 240
gtgcgtggca ggcccggcct gcccagctgg gaggtaatgg gggtgcaggg gctgagcaac 300
tccctcctgc cctgtccttg cctctgtgct agccctgtgg atttcttggc tggggacggg 360
ccccgggcag tgcctgctgc tgttttcatg gtcctcctga gctccctgtg tttgctgctc 420
cccgacgagg acgcattgcc cttcctgact ctcgcctcag cacccagcca agatgggaaa 480
actgaggctc caagaggggc ctggaagata ctgggactgt tctattatgc tgccctctac 540
taccctctgg ctgcctgtgc cacggctggc cacacagctg cacacctgct cggcagcacg 600
ctgtcctggg cccaccttgg ggtccaggtc tggcagaggg cagagtgtcc ccaggtgccc 660
aagatctaca agtactactc cctgctggcc tccctgcctc tcctgctggg cctcggattc 720
ctgagccttt ggtaccctgt gcagctggtg agaagcttca gccgtaggac aggagcaggc 780
tccaaggggc tgcagagcag ctactctgag gaatatctga ggaacctcct ttgcaggaag 840
aagctgggaa gcagctacca cacctccaag catggcttcc tgtcctgggc ccgcgtctgc 900
ttgagacact gcatctacac tccacagcca ggattccatc tcccgctgaa gctggtgctt 960
tcagctacac tgacagggac ggccatttac caggtggccc tgctgctgct ggtgggcgtg 1020
gtacccacta tccagaaggt gagggcaggg gtcaccacgg atgtctccta cctgctggcc 1080
ggctttggaa tcgtgctctc cgaggacaag caggaggtgg tggagctggt gaagcaccat 1140
ctgtgggctc tggaagtgtg ctacatctca gccttggtct tgtcctgctt actcaccttc 1200
ctggtcctga tgcgctcact ggtgacacac aggaccaacc ttcgagctct gcaccgagga 1260
gctgccctgg acttgagtcc cttgcatcgg agtccccatc cctcccgcca agccatattc 1320
tgttggatga gcttcagtgc ctaccagaca gcctttatct gccttgggct cctggtgcag 1380
cagatcatct tcttcctggg aaccacggcc ctggccttcc tggtgctcat gcctgtgctc 1440
catggcagga acctcctgct cttccgttcc ctggagtcct cgtggccctt ctggctgact 1500
ttggccctgg ctgtgatcct gcagaacatg gcagcccatt gggtcttcct ggagactcat 1560
gatggacacc cacagctgac caaccggcga gtgctctatg cagccacctt tcttctcttc 1620
cccctcaatg tgctggtggg tgccatggtg gccacctggc gagtgctcct ctctgccctc 1680
tacaacgcca tccaccttgg ccagatggac ctcagcctgc tgccaccgag agccgccact 1740
ctcgaccccg gctactacac gtaccgaaac ttcttgaaga ttgaagtcag ccagtcgcat 1800
ccagccatga cagccttctg ctccctgctc ctgcaagcgc agagcctcct acccaggacc 1860
atggcagccc cccaggacag cctcagacca ggggaggaag acgaagggat gcagctgcta 1920
cagacaaagg actccatggc caagggagct aggcccgggg ccagccgcgg cagggctcgc 1980
tggggtctgg cctacacgct gctgcacaac ccaaccctgc aggtcttccg caagacggcc 2040

ctgttgggtg ccaatggtgc ccagccctga gggcagggaa ggtcaaccca cctgcccatc 2100
tgtgctgagg catgttcctg cctaccatcc tcctccctcc ccggctctcc tcccagcatc 2160
acaccagcca tgcagccagc aggtcctccg gatcactgtg gttgggtgga ggtctgtctg 2220
cactgggagc ctcaggaggg ctctgctcca cccacttggc tatgggagag ccagcagggg 2280
ttctggagaa aaaaaactgg tgggttaggg ccttggtcca ggagccagtt gagccagggc 2340
agccacatcc aggcgtctcc ctaccctggc tctgccatca gccttgaagg gcctcgatga 2400
agccttctct ggaaccactc cagcccagct ccacctcagc cttggccttc acgctgtgga 2460
agcagccaag gcacttcctc accccctcag cgccacggac ctctctgggg agtggccgga 2520
aagctcccgg gcctctggcc tgcagggcag cccaagtcat gactcagacc aggtcccaca 2580
ctgagctgcc cacactcgag agccagatat ttttgtagtt tttatgcctt tggctattat 2640
gaaagaggtt agtgtgttcc ctg 2663

<210> 6
<211> 689
<212> PRT
<213> Homo sapiens

<400> 6
Met Ser Ser Gln Pro Ala Gly Asn Gln Thr Ser Pro Gly Ala Thr Glu
1               5                   10                  15

Asp Tyr Ser Tyr Gly Ser Trp Tyr Ile Asp Glu Pro Gln Gly Gly Glu
            20                  25                  30

Glu Leu Gln Pro Glu Gly Glu Val Pro Ser Cys His Thr Ser Ile Pro
        35                  40                  45

Pro Gly Leu Tyr His Ala Cys Leu Ala Ser Leu Ser Ile Leu Val Leu
    50                  55                  60

Leu Leu Leu Ala Met Leu Val Arg Arg Arg Gln Leu Trp Pro Asp Cys
65                  70                  75                  80

Val Arg Gly Arg Pro Gly Leu Pro Ser Trp Glu Val Met Gly Val Gln
                85                  90                  95

Gly Leu Ser Asn Ser Leu Leu Pro Cys Pro Cys Leu Cys Ala Ser Pro
            100                 105                 110

Val Asp Phe Leu Ala Gly Asp Gly Pro Arg Ala Val Pro Ala Ala Val
        115                 120                 125

Phe Met Val Leu Leu Ser Ser Leu Cys Leu Leu Leu Pro Asp Glu Asp
    130                 135                 140

Ala Leu Pro Phe Leu Thr Leu Ala Ser Ala Pro Ser Gln Asp Gly Lys
145                 150                 155                 160

Thr Glu Ala Pro Arg Gly Ala Trp Lys Ile Leu Gly Leu Phe Tyr Tyr
                165                 170                 175

Ala Ala Leu Tyr Tyr Pro Leu Ala Ala Cys Ala Thr Ala Gly His Thr
            180                 185                 190

Ala Ala His Leu Leu Gly Ser Thr Leu Ser Trp Ala His Leu Gly Val
        195                 200                 205

Gln Val Trp Gln Arg Ala Glu Cys Pro Gln Val Pro Lys Ile Tyr Lys
    210                 215                 220

Tyr Tyr Ser Leu Leu Ala Ser Leu Pro Leu Leu Leu Gly Leu Gly Phe
225                 230                 235                 240

Leu Ser Leu Trp Tyr Pro Val Gln Leu Val Arg Ser Phe Ser Arg Arg
                245                 250                 255

Thr Gly Ala Gly Ser Lys Gly Leu Gln Ser Ser Tyr Ser Glu Glu Tyr
            260                 265                 270

Leu Arg Asn Leu Leu Cys Arg Lys Lys Leu Gly Ser Ser Tyr His Thr
        275                 280                 285

Ser Lys His Gly Phe Leu Ser Trp Ala Arg Val Cys Leu Arg His Cys
    290                 295                 300

Ile Tyr Thr Pro Gln Pro Gly Phe His Leu Pro Leu Lys Leu Val Leu
305                 310                 315                 320

Ser Ala Thr Leu Thr Gly Thr Ala Ile Tyr Gln Val Ala Leu Leu Leu
                325                 330                 335

Leu Val Gly Val Val Pro Thr Ile Gln Lys Val Arg Ala Gly Val Thr
            340                 345                 350

Thr Asp Val Ser Tyr Leu Leu Ala Gly Phe Gly Ile Val Leu Ser Glu
        355                 360                 365

Asp Lys Gln Glu Val Val Glu Leu Val Lys His His Leu Trp Ala Leu
    370                 375                 380

Glu Val Cys Tyr Ile Ser Ala Leu Val Leu Ser Cys Leu Leu Thr Phe
385                 390                 395                 400

Leu Val Leu Met Arg Ser Leu Val Thr His Arg Thr Asn Leu Arg Ala
                405                 410                 415

Leu His Arg Gly Ala Ala Leu Asp Leu Ser Pro Leu His Arg Ser Pro
            420                 425                 430

His Pro Ser Arg Gln Ala Ile Phe Cys Trp Met Ser Phe Ser Ala Tyr
        435                 440                 445

Gln Thr Ala Phe Ile Cys Leu Gly Leu Leu Val Gln Gln Ile Ile Phe
    450                 455                 460

Phe Leu Gly Thr Thr Ala Leu Ala Phe Leu Val Leu Met Pro Val Leu
465                 470                 475                 480

His Gly Arg Asn Leu Leu Leu Phe Arg Ser Leu Glu Ser Ser Trp Pro
                485                 490                 495

Phe Trp Leu Thr Leu Ala Leu Ala Val Ile Leu Gln Asn Met Ala Ala
            500                 505                 510

His Trp Val Phe Leu Glu Thr His Asp Gly His Pro Gln Leu Thr Asn
        515                 520                 525

Arg Arg Val Leu Tyr Ala Ala Thr Phe Leu Leu Phe Pro Leu Asn Val
    530                 535                 540

Leu Val Gly Ala Met Val Ala Thr Trp Arg Val Leu Leu Ser Ala Leu
545                 550                 555                 560

Tyr Asn Ala Ile His Leu Gly Gln Met Asp Leu Ser Leu Leu Pro Pro
                565                 570                 575

Arg Ala Ala Thr Leu Asp Pro Gly Tyr Tyr Thr Tyr Arg Asn Phe Leu
            580                 585                 590

Lys Ile Glu Val Ser Gln Ser His Pro Ala Met Thr Ala Phe Cys Ser
        595                 600                 605

Leu Leu Leu Gln Ala Gln Ser Leu Leu Pro Arg Thr Met Ala Ala Pro
    610                 615                 620

Gln Asp Ser Leu Arg Pro Gly Glu Glu Asp Glu Gly Met Gln Leu Leu
625                 630                 635                 640

Gln Thr Lys Asp Ser Met Ala Lys Gly Ala Arg Pro Gly Ala Ser Arg
                645                 650                 655

Gly Arg Ala Arg Trp Gly Leu Ala Tyr Thr Leu Leu His Asn Pro Thr
            660                 665                 670

Leu Gln Val Phe Arg Lys Thr Ala Leu Leu Gly Ala Asn Gly Ala Gln
        675                 680                 685

Pro

<210> 7
<211> 7892
<212> DNA
<213> Homo sapiens

<400> 7
atgcgggggg cgcccgcgcg cctgctgctg ccgctgctgc cgtggctcct gctgctcctg 60
gcgcccgagg ctcggggcgc gcccggctgc ccgctatcca tccgcagctg caagtgctcg 120
ggggagcggc ccaaggggct gagcggcggc gtccctggcc cggctcggcg gagggtggtg 180
tgcagcggcg gggacctccc ggagcctccc gagcccggcc ttctgcctaa cggcaccgtt 240
acccttggca accgtttctc cactgcccct tgcccgcagt cggggctggc ccagtctttt 300
cgattgtgtc cagaacaata cagtaacgcc tctggttggg tgagttcaga ggctgcccgc 360
ttcctgacag gacacaactg gtcagcctct gacgctggcc gcccggcctg cattttcccc 420
ggcccggtct cctggagaca ggtaccagct tacaacgccg gacaggatca gggtagcaaa 480
tatagcttct atggaggaaa ggtcaaagat gagtatctgc gtaggtcccc gatctgtcgc 540
tctgctgccg ccctgcacac tgacaccagt gcccgcagct ggtctgaatt taggctcttg 600
agcaataaca agatcacggg gctccgcaat ggctccttcc tgggactgtc actgctggag 660
aagcttctca tgagagcaca tttttctgat gaagatgaaa agacggcctg tccttctcca 720
gcaccaaggc tctggaggct gcatagcaag tgcaccctga gcatggcagg caatcagaag 780

aaccagggcg gcagcagctc acaagaggca gcatctggct tgatgccgag tcggcggctc 840
cagcggggag aggttgaggg cctctgcact tgcctctgca cggacctgag gaacaacatc 900
atcagcacag tgcagccggg cgccttcctg ggcctggggg agctgaagcg tttagatctc 960
tccaacaacc ggattggctg tctcacctcc gagaccttcc agggcctccc caggcttctc 1020
cgactaaaca tatctggaaa catcttctcc agtctgcaac ctggggtctt tgatgagctg 1080
ccagccctta aggttgtgga cttgggcacc gagttcctga cctgtgactg ccacctgcgc 1140
tggctgctgc cctgggccca gaatcgctcc ctgcagctgt cggaacacac gctctgtgct 1200
taccccagtg ccctgcatgc tcatgccctg ggcagcctcc aggaggccca gctctgctgc 1260
gagggggccc tggagctgca cacacaccac ctcatcccgt ccctacgcca agtggtgttc 1320
cagggggatc ggctgccctt ccagtgctct gccagctacc tgggcaacga cacccgcatc 1380
cgctggtacc acaaccgagc ccctgtggag ggtgatgagc aggcgggcat cctcctggcc 1440
gagagcctca tccacgactg caccttcatc accagtgagc tgacgctgtc tcacatcggc 1500
gtgtgggcct caggcgagtg ggagtgcacc gtgtccatgg cccaaggcaa cgccagcaag 1560
aaggtggaga tcgtggtgct ggagacctct gcctcctact gccccgccga gcgtgttgcc 1620
aacaaccgcg gggacttcag gtggccccga actctggctg gcatcacagc ctaccagtcc 1680
tgcctgcagt atcccttcac ctcagtgccc ctgggcgggg gtgccccggg cacccgagcc 1740
tcccgccggt gtgaccgtgc cggccgctgg gagccagggg actactccca ctgtctctac 1800
accaacgaca tcaccagggt gctgtacacc ttcgtgctga tgcccatcaa tgcctccaat 1860
gcgctgaccc tggctcacca gctgcgcgtg tacacagccg aggccgctag cttttcagac 1920
atgatggatg tagtctatgt ggctcagatg atccagaaat tttgggttta tgtcgaccag 1980
atcaaagagc tggtagaggt gatggtggac atgcccagca acttgatgct ggtggacgag 2040
cacctgctgt ggctggccca gcgcgaggac aaggcctgca gccgcatcgt gggtgccata 2100
gagcgcattg ggggggccgc cctcagcccc catgcccagc acatctcagt gaatgcgagg 2160
aacgtggcat tggaggccta cctcatcaag ccgcacagct acgtgggcct gacctgcaca 2220
gccttccaga ggagggaggg aggggtgccg ggcacacggc caggaagccc tggccagaac 2280
cccccacctg agcccgagcc cccagctgac cagcagctcc gcttccgctg caccaccggg 2340
aggcccaatg tttctctgtc gtccttccac atcaagaaca gcgtggccct ggcctccatc 2400
cagctgcccc cgagtctatt ctcatccctt ccggctgccc tggctccccc ggtgccccca 2460
gactgcaccc tgcaactgct cgtcttccga aatggccgcc tcttccacag ccacagcaac 2520
acctcccgcc ctggagctgc tgggcctggc aagaggcgtg gcgtggccac ccccgtcatc 2580
ttcgcaggaa ccagtggctg tggcgtggga aacctgacag agccagtggc cgtttcgctg 2640
cggcactggg ctgagggagc cgaacctgtg gccgcttggt ggagccagga ggggcccggg 2700
gaggctgggg gctggacctc ggagggctgc cagctccgct ccagccagcc caatgtcagc 2760
gccctgcact gccagcactt gggcaatgtg gccgtgctca tggagctgag cgcctttccc 2820
agggaggtgg ggggcgccgg ggcagggctg caccccgtgg tatacccctg cacggccttg 2880
ctgctgctct gcctcttcgc caccatcatc acctacatcc tcaaccacag ctccatccgt 2940
gtgtcccgga aaggctggca catgctgctg aacttgtgct tccacatagc catgacctct 3000
gctgtctttg cggggggcat cacactcacc aactaccaga tggtctgcca ggcggtgggc 3060
atcaccctgc actactcctc cctatccacg ctgctctgga tgggcgtgaa ggcgcgagtg 3120
ctccataagg agctcacctg gagggcaccc cctccgcaag aaggggaccc cgctctgcct 3180
actcccagtc ctatgctccg gtacatactt tcaattccag ctttgcaatt ggggagggac 3240
tccaacgcag gcgtaggaaa cctcccaagg ttctatttga tcgctggagg gattccactc 3300
attatctgtg gcatcacagc tgcagtcaac atccacaact accgggacca cagcccctac 3360
tgctggctgg tgtggcgtcc aagccttggc gccttctaca tccctgtggc tttgattctg 3420
ctcatcacct ggatctattt cctgtgcgcc gggctacgct tacggggtcc tctggcacag 3480
aaccccaagg cgggcaacag cagggcctcc ctggaggcag gggaggagct gaggggttcc 3540
accaggctca ggggcagcgg ccccctcctg agtgactcag gttcccttct tgctactggg 3600
agcgcgcgag tggggacgcc cgggcccccg gaggatggtg acagcctcta ttctccggga 3660
gtccagctag gggcgctggt gaccacgcac ttcctgtact tggccatgtg ggcctgcggg 3720
gctctggcag tgtcccagcg ctggctgccc cgggtggtgt gcagctgctt gtacggggtg 3780
gcagcctccg ccctgggcct cttcgtcttc actcaccact gtgccaggcg gagggacgtg 3840
agagcctcgt ggcgcgcctg ctgcccccct gcctctcccg cggcccccca tgccccgccc 3900
cgggccctgc ccgccgccgc agaggacggt tccccggtgt tcggggaggg gcccccctcc 3960
ctcaagtcct ccccaagcgg cagcagcggc catccgctgg ctctgggccc ctgcaagctc 4020
accaacctgc agctggccca gagtcaggtg tgcgaggcgg gggcggcggc cggcggggaa 4080
ggagagccgg agccggcggg cacccgggga aacctcgccc accgccaccc caacaacgtg 4140
caccacgggc gtcgggcgca caagagccgg gccaagggac accgcgcggg ggaggcctgc 4200

ggcaagaacc ggctcaaggc cctgcgcggg ggcgcggcgg gggcgctgga actgctgtcc 4260
agcgagagcg gtagtctgca caacagcccc accgacagct acctgggcag cagccgcaac 4320
agcccgggcg ccggcctgca gctggaaggc gagcccatgc tcacgccgtc cgagggcagc 4380
gacaccagcg ccgcgccgct ttctgaggcg ggccgggcag gccagcgccg cagcgccagc 4440
cgcgacagtc tcaagggcgg cggcgcgctg gagaaggaga gccatcgccg ctcgtacccg 4500
ctcaacgccg ccagcctaaa cggcgccccc aagggggcca agtacgacga cgtcaccctg 4560
atgggcgcgg aggtagccag cggcggctgc atgaagaccg gactctggaa gagcgaaact 4620
accgtctaag gtggggcggg cgacgcggta gacgggttgg ccacgcggct cgttcccccg 4680
ctcctcgggg ccctccaagg tgtctccgta gtcagcaggt tggaggcaga ggagccgatg 4740
gctggaggaa gcccacaggc ggatgttccc cacttgccta gagggcatcc ctctggggta 4800
gcgacagaca atcccagaaa cacgcataat acatttccgt ccagcccggg gcagtctgac 4860
tgtcggtgcc ctcccaggaa cggggaaggc ctccgtctgt gtgaaagggc acagcacatc 4920
ccaggtgcac cctccccaag tactcccacc ccgcctactg tccatgcggc ctcactgggg 4980
gccatcagcc tcaccagcaa agcagagatg agagcgtggg aactgtgttc tttcctccct 5040
gccctctact gatttcagcc cagcccctgc ctagatccta ggtccctttt cctcccgagt 5100
ttggctggca cgagagctag cccagcacat gaagcaggtg atgttaagtc acaaggtgct 5160
gcttttcaga tccactatgc aagaggggag ggtggggcca cgtgaaaggc agctctagac 5220
atcaaccagt cctgggggag gggagtggga accgggcaca actaggaaca atgccaccat 5280
tcccacagga gtggtactta aaccagacag cagggttcag aggtggcaca ccgggacaaa 5340
gctgaggccc tgcacctcaa cagctgactg ccaggtgcct gtgggtgaac tgaggggagt 5400
agagggagag ggcaggtgga actggggcag aatctagtca tgccctaaag ctagtcctgt 5460
aaacaatggt gccccagaaa gctgcaggtg gtgtttggag aagcagttac ttttcagtta 5520
caagacccat ctccctagtc tcagccttac aacaccacgg gactaaggaa gagcacttcc 5580
ttgcctccgt aaggccagag gaagaaccat cccaatcatt tgatctccag ctccacagta 5640
gagagaaacc tacaaaatgt caaaccagct tcccgactcc caggagctca agccaagccc 5700
agaggcagtg gctggggtcc ctgcaggtca tgaggggcct atgcctttac tccttttaaa 5760
caccagcacc cgtcttttcc ccaacctaaa accaaccacc agcatttcac tacaggacca 5820
aatggaaacc gagggaaccc tgggtcttgg gaagaacaac aggaaaccaa ggtctgacct 5880
agggttccct cccagtcttc acatcactct ggcctcatca ccaaggtgac agaggacaca 5940
ggggaggggg aaaacccaca cacactcctt ggaatgggtc ctgttattta tgcttgctgc 6000
acagacatat tagaagaaaa aaaaaaagct ttgtattatt cttccacata tgctggctgc 6060
tgtttacaca ccctgccaat gccttagcac tggagagctt tttgcaatat gctggggaaa 6120
ggggagggag ggaatgaaag tgccaaagaa aacatgtttt taagaactcg ggttttatac 6180
aatagaatgt tttctagcag atgcctcttg ttttaatata ttaaaatttt gcaaagccct 6240
ttgagctact gccttagtct acccactgtc cttttgttat gaggtagagg atctcatgac 6300
accatacaca caaacccatc attgcctgtg aatgcacgta gggccagaat tccccagttc 6360
ccgctcctct gagggttgat actgctggga atgccaacca ctccacaagc agagggaagc 6420
cccctcaggc ctgcaggagg agccgcagca gtgtgtccaa ttcaaaccag cagcaaagag 6480
cctgacattt tcccatccat ctatgaggaa agccatctca cagaacatgg acataggcaa 6540
cttgctctcc cacaccaagg gatgggaatc tctcctacct atagtcatcc ctgcactcct 6600
gactttactc caggacccag ggtccaacta atggcagagc ccctcttggt tccttcaaac 6660
aagaaaagca atacctacgg actggtgtac acttccatcc ttggttataa caggaatgtt 6720
atcaagctgt cagaacagga tgaagtgctc ccagtggata tccatcaggg agggttaggg 6780
acactcgtgg cagcctgtct agcagcctgg gctctctgaa agtccctaac ttcctgaggg 6840
gtacgcaaat actgttctat ttcactatca gaaatgttct catctccagt gacagtggag 6900
acagggggta cagggcagat ccgcttcggg gacttcaaca tgcaggtggg caagagaagg 6960
gcaggactgg ccggccgctt cccctggggt aaacctaagg aattatttcc cacctcccct 7020
tctccttgcc cctgtcccca ccccggtggc tccttctctc gggtctccac ttctgctgtc 7080
ccatcccgaa aggcagagcg gaccagtgac tggcggtgct ggagaaggtc accgatgtgc 7140
ttcaccacag accgtttgtc aagtctcaga actcgtaacc aggccagctg ctcagccatc 7200
cgcagcagca cagccagcag ctcctgcagg cgggaggacg ccgggtaggg caggtccaca 7260
tttgccaatt tacaaaatcg ggcaagggaa catgaaagcc gatctgcagg ctgcagcgac 7320
tgccaagcca ggaaagtcgc agcagtgatg acgggcaagg gatgcctccc ggtcaccagc 7380
cacgtctcat ttgccagctc caccaactgc attgttcgag acagcatctt ctctttgtct 7440
tccacgtatt tggctggcac agaaggtgaa gcttggaaca gtttgaagct gaaataacca 7500
aaatgagggt tggatcttaa tgatataggg gctgctctcc cacagtgagg aaagacagcc 7560
cactcaagat ggggaagcta ttctgccctc aggaatactc aagctcactg ggcagcaagt 7620

taataaaggt agtgagagaa aacagggcgt cttccgcttg ttaggggaag gtggagggat 7680
ggaggagagc acgaacattt attgggcgcc tcccaatcac cattattctg agtgctttac 7740
aacgttctca tttaatctac gtgcacgtgc accatcttat gtgcatgtat agttaaaaaa 7800
ctttcccata gtcatccagc caggcagtaa ccaagcttca aatacaaggc tatttgacac 7860
caacagcctc tactttcaac gttatttatc ag 7892

<210> 8
<211> 1542
<212> PRT
<213> Homo sapiens

<400> 8
Met Arg Gly Ala Pro Ala Arg Leu Leu Leu Pro Leu Leu Pro Trp Leu
1               5                   10                  15

Leu Leu Leu Leu Ala Pro Glu Ala Arg Gly Ala Pro Gly Cys Pro Leu
            20                  25                  30

Ser Ile Arg Ser Cys Lys Cys Ser Gly Glu Arg Pro Lys Gly Leu Ser
        35                  40                  45

Gly Gly Val Pro Gly Pro Ala Arg Arg Arg Val Val Cys Ser Gly Gly
    50                  55                  60

Asp Leu Pro Glu Pro Pro Glu Pro Gly Leu Leu Pro Asn Gly Thr Val
65                  70                  75                  80

Thr Leu Gly Asn Arg Phe Ser Thr Ala Pro Cys Pro Gln Ser Gly Leu
                85                  90                  95

Ala Gln Ser Phe Arg Leu Cys Pro Glu Gln Tyr Ser Asn Ala Ser Gly
            100                 105                 110

Trp Val Ser Ser Glu Ala Ala Arg Phe Leu Thr Gly His Asn Trp Ser
        115                 120                 125

Ala Ser Asp Ala Gly Arg Pro Ala Cys Ile Phe Pro Gly Pro Val Ser
    130                 135                 140

Trp Arg Gln Val Pro Ala Tyr Asn Ala Gly Gln Asp Gln Gly Ser Lys
145                 150                 155                 160

Tyr Ser Phe Tyr Gly Gly Lys Val Lys Asp Glu Tyr Leu Arg Arg Ser
                165                 170                 175

Pro Ile Cys Arg Ser Ala Ala Ala Leu His Thr Asp Thr Ser Ala Arg
            180                 185                 190

Ser Trp Ser Glu Phe Arg Leu Leu Ser Asn Asn Lys Ile Thr Gly Leu
        195                 200                 205

Arg Asn Gly Ser Phe Leu Gly Leu Ser Leu Leu Glu Lys Leu Leu Met
    210                 215                 220

Arg Ala His Phe Ser Asp Glu Asp Glu Lys Thr Ala Cys Pro Ser Pro
225                 230                 235                 240

Ala Pro Arg Leu Trp Arg Leu His Ser Lys Cys Thr Leu Ser Met Ala
                245                 250                 255

Gly Asn Gln Lys Asn Gln Gly Gly Ser Ser Ser Gln Glu Ala Ala Ser
            260                 265                 270

Gly Leu Met Pro Ser Arg Arg Leu Gln Arg Gly Glu Val Glu Gly Leu
        275                 280                 285

Cys Thr Cys Leu Cys Thr Asp Leu Arg Asn Asn Ile Ile Ser Thr Val
    290                 295                 300

Gln Pro Gly Ala Phe Leu Gly Leu Gly Glu Leu Lys Arg Leu Asp Leu
305                 310                 315                 320

Ser Asn Asn Arg Ile Gly Cys Leu Thr Ser Glu Thr Phe Gln Gly Leu
                325                 330                 335

Pro Arg Leu Leu Arg Leu Asn Ile Ser Gly Asn Ile Phe Ser Ser Leu
            340                 345                 350

Gln Pro Gly Val Phe Asp Glu Leu Pro Ala Leu Lys Val Val Asp Leu
        355                 360                 365

Gly Thr Glu Phe Leu Thr Cys Asp Cys His Leu Arg Trp Leu Leu Pro
    370                 375                 380

Trp Ala Gln Asn Arg Ser Leu Gln Leu Ser Glu His Thr Leu Cys Ala
385                 390                 395                 400

Tyr Pro Ser Ala Leu His Ala His Ala Leu Gly Ser Leu Gln Glu Ala
                405                 410                 415

Gln Leu Cys Cys Glu Gly Ala Leu Glu Leu His Thr His His Leu Ile
            420                 425                 430

Pro Ser Leu Arg Gln Val Val Phe Gln Gly Asp Arg Leu Pro Phe Gln
        435                 440                 445

Cys Ser Ala Ser Tyr Leu Gly Asn Asp Thr Arg Ile Arg Trp Tyr His
    450                 455                 460

Asn Arg Ala Pro Val Glu Gly Asp Glu Gln Ala Gly Ile Leu Leu Ala
465                 470                 475                 480

Glu Ser Leu Ile His Asp Cys Thr Phe Ile Thr Ser Glu Leu Thr Leu
                485                 490                 495

Ser His Ile Gly Val Trp Ala Ser Gly Glu Trp Glu Cys Thr Val Ser
            500                 505                 510

Met Ala Gln Gly Asn Ala Ser Lys Lys Val Glu Ile Val Val Leu Glu
        515                 520                 525

Thr Ser Ala Ser Tyr Cys Pro Ala Glu Arg Val Ala Asn Asn Arg Gly
    530                 535                 540

Asp Phe Arg Trp Pro Arg Thr Leu Ala Gly Ile Thr Ala Tyr Gln Ser
545                 550                 555                 560

Cys Leu Gln Tyr Pro Phe Thr Ser Val Pro Leu Gly Gly Gly Ala Pro
                565                 570                 575

Gly Thr Arg Ala Ser Arg Arg Cys Asp Arg Ala Gly Arg Trp Glu Pro
            580                 585                 590

Gly Asp Tyr Ser His Cys Leu Tyr Thr Asn Asp Ile Thr Arg Val Leu
        595                 600                 605

Tyr Thr Phe Val Leu Met Pro Ile Asn Ala Ser Asn Ala Leu Thr Leu
    610                 615                 620

Ala His Gln Leu Arg Val Tyr Thr Ala Glu Ala Ala Ser Phe Ser Asp
625                 630                 635                 640

Met Met Asp Val Val Tyr Val Ala Gln Met Ile Gln Lys Phe Leu Gly
                645                 650                 655

Tyr Val Asp Gln Ile Lys Glu Leu Val Glu Val Met Val Asp Met Pro
            660                 665                 670

Ser Asn Leu Met Leu Val Asp Glu His Leu Leu Trp Leu Ala Gln Arg
        675                 680                 685

Glu Asp Lys Ala Cys Ser Arg Ile Val Gly Ala Ile Glu Arg Ile Gly
    690                 695                 700

Gly Ala Ala Leu Ser Pro His Ala Gln His Ile Ser Val Asn Ala Arg
705                 710                 715                 720

Asn Val Ala Leu Glu Ala Tyr Leu Ile Lys Pro His Ser Tyr Val Gly
                725                 730                 735

Leu Thr Cys Thr Ala Phe Gln Arg Arg Glu Gly Gly Val Pro Gly Thr
            740                 745                 750

Arg Pro Gly Ser Pro Gly Gln Asn Pro Pro Pro Glu Pro Glu Pro Pro
        755                 760                 765

Ala Asp Gln Gln Leu Arg Phe Arg Cys Thr Thr Gly Arg Pro Asn Val
    770                 775                 780

Ser Leu Ser Ser Phe His Ile Lys Asn Ser Val Ala Leu Ala Ser Ile
785                 790                 795                 800

Gln Leu Pro Pro Ser Leu Phe Ser Ser Leu Pro Ala Ala Leu Ala Pro
                805                 810                 815

Pro Val Pro Pro Asp Cys Thr Leu Gln Leu Leu Val Phe Arg Asn Gly
            820                 825                 830

Arg Leu Phe His Ser His Ser Asn Thr Ser Arg Pro Gly Ala Ala Gly
        835                 840                 845

Pro Gly Lys Arg Arg Gly Val Ala Thr Pro Val Ile Phe Ala Gly Thr
    850                 855                 860

Ser Gly Cys Gly Val Gly Asn Leu Thr Glu Pro Val Ala Val Ser Leu
865                 870                 875                 880

Arg His Trp Ala Glu Gly Ala Glu Pro Val Ala Ala Trp Trp Ser Gln
                885                 890                 895

Glu Gly Pro Gly Glu Ala Gly Gly Trp Thr Ser Glu Gly Cys Gln Leu
            900                 905                 910

Arg Ser Ser Gln Pro Asn Val Ser Ala Leu His Cys Gln His Leu Gly
        915                 920                 925

Asn Val Ala Val Leu Met Glu Leu Ser Ala Phe Pro Arg Glu Val Gly
    930                 935                 940

Gly Ala Gly Ala Gly Leu His Pro Val Val Tyr Pro Cys Thr Ala Leu
945                 950                 955                 960

Leu Leu Leu Cys Leu Phe Ala Thr Ile Ile Thr Tyr Ile Leu Asn His
                965                 970                 975

Ser Ser Ile Arg Val Ser Arg Lys Gly Trp His Met Leu Leu Asn Leu
            980                 985                 990

Cys Phe His Ile Ala Met Thr Ser Ala Val Phe Ala Gly Gly Ile Thr
        995                 1000                1005

Leu Thr Asn Tyr Gln Met Val Cys Gln Ala Val Gly Ile Thr Leu His
   1010                 1015                1020

Tyr Ser Ser Leu Ser Thr Leu Leu Trp Met Gly Val Lys Ala Arg Val
1025                1030                1035                1040

Leu His Lys Glu Leu Thr Trp Arg Ala Pro Pro Pro Gln Glu Gly Asp
                1045                1050                1055

Pro Ala Leu Pro Thr Pro Ser Pro Met Leu Arg Tyr Ile Leu Ser Ile
            1060                1065                1070

Pro Ala Leu Gln Leu Gly Arg Asp Ser Asn Ala Gly Val Gly Asn Leu
        1075                1080                1085

Pro Arg Phe Tyr Leu Ile Ala Gly Gly Ile Pro Leu Ile Ile Cys Gly
   1090                 1095                1100

Ile Thr Ala Ala Val Asn Ile His Asn Tyr Arg Asp His Ser Pro Tyr
1105                1110                1115                1120

Cys Trp Leu Val Trp Arg Pro Ser Leu Gly Ala Phe Tyr Ile Pro Val
                1125                1130                1135

Ala Leu Ile Leu Leu Ile Thr Trp Ile Tyr Phe Leu Cys Ala Gly Leu
            1140                1145                1150

Arg Leu Arg Gly Pro Leu Ala Gln Asn Pro Lys Ala Gly Asn Ser Arg
        1155                1160                1165

Ala Ser Leu Glu Ala Gly Glu Glu Leu Arg Gly Ser Thr Arg Leu Arg
    1170                1175                1180

Gly Ser Gly Pro Leu Leu Ser Asp Ser Gly Ser Leu Leu Ala Thr Gly
1185                1190                1195                1200

Ser Ala Arg Val Gly Thr Pro Gly Pro Pro Glu Asp Gly Asp Ser Leu
                1205                1210                1215

Tyr Ser Pro Gly Val Gln Leu Gly Ala Leu Val Thr Thr His Phe Leu
            1220                1225                1230

Tyr Leu Ala Met Trp Ala Cys Gly Ala Leu Ala Val Ser Gln Arg Trp
        1235                1240                1245

Leu Pro Arg Val Val Cys Ser Cys Leu Tyr Gly Val Ala Ala Ser Ala
    1250                1255                1260

Leu Gly Leu Phe Val Phe Thr His His Cys Ala Arg Arg Arg Asp Val
1265                1270                1275                1280

Arg Ala Ser Trp Arg Ala Cys Cys Pro Pro Ala Ser Pro Ala Ala Pro
                1285                1290                1295

His Ala Pro Pro Arg Ala Leu Pro Ala Ala Ala Glu Asp Gly Ser Pro
            1300                1305                1310

Val Phe Gly Glu Gly Pro Pro Ser Leu Lys Ser Ser Pro Ser Gly Ser
        1315                1320                1325

Ser Gly His Pro Leu Ala Leu Gly Pro Cys Lys Leu Thr Asn Leu Gln
    1330                1335                1340

Leu Ala Gln Ser Gln Val Cys Glu Ala Gly Ala Ala Ala Gly Gly Glu
1345                1350                1355                1360

Gly Glu Pro Glu Pro Ala Gly Thr Arg Gly Asn Leu Ala His Arg His
                1365                1370                1375

Pro Asn Asn Val His His Gly Arg Arg Ala His Lys Ser Arg Ala Lys
            1380                1385                1390

Gly His Arg Ala Gly Glu Ala Cys Gly Lys Asn Arg Leu Lys Ala Leu
        1395                1400                1405

Arg Gly Gly Ala Ala Gly Ala Leu Glu Leu Leu Ser Ser Glu Ser Gly
    1410                1415                1420

Ser Leu His Asn Ser Pro Thr Asp Ser Tyr Leu Gly Ser Ser Arg Asn
1425                1430                1435                1440

Ser Pro Gly Ala Gly Leu Gln Leu Glu Gly Glu Pro Met Leu Thr Pro
                1445                1450                1455

Ser Glu Gly Ser Asp Thr Ser Ala Ala Pro Leu Ser Glu Ala Gly Arg
        1460                1465                1470

Ala Gly Gln Arg Arg Ser Ala Ser Arg Asp Ser Leu Lys Gly Gly Gly
    1475                1480                1485

Ala Leu Glu Lys Glu Ser His Arg Arg Ser Tyr Pro Leu Asn Ala Ala
  1490                1495                1500

Ser Leu Asn Gly Ala Pro Lys Gly Gly Lys Tyr Asp Asp Val Thr Leu
1505                1510                1515                1520

Met Gly Ala Glu Val Ala Ser Gly Gly Cys Met Lys Thr Gly Leu Trp
                1525                1530                1535

Lys Ser Glu Thr Thr Val
        1540

<210> 9
<211> 1329
<212> DNA
<213> Homo sapiens

<400> 9
ggatcccggc tgcccttcca gtgctctgcc agctacctgg gcaacgacac ccgcatccgc 60
tggtaccaca accgagcccc tgtggagggt gatgagcagg cgggcatcct cctggccgag 120
agcctcatcc acgactgcac cttcatcacc aggtggcccc gaactctggc tggcatcaca 180
gcctaccagt cctgcctgca gtatcccttc acctcagtgc ccctgggcgg gggtgccccg 240
ggcacccgag cctcccgccg gtgtgaccgt gccggccgct gggagccagg ggactactcc 300
cactgtctct acaccaacga catcaccagg gtgctgtaca ccttcgtgct gatgcccatc 360
aatgcctcca atgcgctgac cctggctcac cagctgcgcg tgtacacagc cgaggccgct 420
agcttttcag acatgatgga tgtggtctat gtggctcaga tgatccagaa atttttgggt 480
tatgtcgacc agatcaaaga gctggtaggg gtgatggtgg acatggccag caacctgatg 540
ctggtggacg agcacctgct gtggctggcc cagcgcgagg acaaggcctg cagccgcatc 600
gtgggtgccc tggagcgcat tgggggggcc gccctcagcc cccatgccca gcacatctca 660
gtgaatgcga ggaacgtggc attggaggcc tacctcatca agccgcacag ctacgtgggc 720
ctgacctgca cagccttcca gaggagggag ggaggggtgc cgggcacacg gccaggaagc 780
cctggccaga accccccacc tgagcccgag cccccagctg accagcagct ccgcttccgc 840
tgcaccaccg ggaggcccaa tgtttctctg tcgtccttcc acatcaagaa cagcgtggcc 900
ctggcctcca tccagctgcc cccgagtcta ttctcatccc ttccggctgc cctggctccc 960
ccggtgcccc cagactgcac cctgcaactg ctcgtcttcc gaaatggccg cctcttccac 1020
agccacagca acgcctcccg ccctggagct gctgggcctg gcaagaggcg tggcgtggcc 1080
acccccgtca tcttcgcagg aaccagtggc tgtggcgtgg gaaacctgac agagtcagtg 1140
gccgtttcgc tgcggcactg ggctgaggga gccgaacctg tggccgcttg gtggagccag 1200
gaggggcccg gggaggctgg gggctggacc tcggagggct gccagctccg ctccagccag 1260
cccaatgtca gcgccctgca ctgccagcac ttgggcaatg tggccgtgct catggagctg 1320
agcctcgag                                                         1329

<210> 10
<211> 1329
<212> DNA
<213> Homo sapiens

<400> 10

ctcgaggctc agctccatga gcacggccac attgcccaag tgctggcagt gcagggcgct 60
gacattgggc tggctggagc ggagctggca gccctccgag gtccagcccc cagcctcccc 120
gggcccctcc tggctccacc aagcggccac aggttcggct ccctcagccc agtgccgcag 180
cgaaacggcc actgactctg tcaggtttcc cacgccacag ccactggttc ctgcgaagat 240
gacgggggtg gccacgccac gcctcttgcc aggcccagca gctccagggc gggaggcgtt 300
gctgtggctg tggaagaggc ggccatttcg gaagacgagc agttgcaggg tgcagtctgg 360
gggcaccggg ggagccaggg cagccggaag ggatgagaat agactcgggg gcagctggat 420
ggaggccagg gccacgctgt tcttgatgtg gaaggacgac agagaaacat tgggcctccc 480
ggtggtgcag cggaagcgga gctgctggtc agctgggggc tcgggctcag gtggggggtt 540
ctggccaggg cttcctggcc gtgtgcccgg cacccctccc tccctcctct ggaaggctgt 600
gcaggtcagg cccacgtagc tgtgcggctt gatgaggtag gcctccaatg ccacgttcct 660
cgcattcact gagatgtgct gggcatgggg gctgagggcg gcccccccaa tgcgctccag 720
ggcacccacg atgcggctgc aggccttgtc ctcgcgctgg gccagccaca gcaggtgctc 780
gtccaccagc atcaggttgc tggccatgtc caccatcacc cctaccagct ctttgatctg 840
gtcgacataa cccaaaaatt tctggatcat ctgagccaca tagaccacat ccatcatgtc 900
tgaaaagcta gcggcctcgg ctgtgtacac gcgcagctgg tgagccaggg tcagcgcatt 960
ggaggcattg atgggcatca gcacgaaggt gtacagcacc ctggtgatgt cgttggtgta 1020
gagacagtgg gagtagtccc ctggctccca gcggccggca cggtcacacc ggcgggaggc 1080
tcgggtgccc ggggcacccc cgcccagggg cactgaggtg aagggatact gcaggcagga 1140
ctggtaggct gtgatgccag ccagagttcg gggccacctg gtgatgaagg tgcagtcgtg 1200
gatgaggctc tcggccagga ggatgcccgc ctgctcatca ccctccacag gggctcggtt 1260
gtggtaccag cggatgcggg tgtcgttgcc caggtagctg gcagagcact ggaagggcag 1320
ccgggatcc 1329

<210> 11
<211> 443
<212> PRT
<213> Homo sapiens

<400> 11
Gly Ser Arg Leu Pro Phe Gln Cys Ser Ala Ser Tyr Leu Gly Asn Asp
1               5                   10                  15

Thr Arg Ile Arg Trp Tyr His Asn Arg Ala Pro Val Glu Gly Asp Glu
            20                  25                  30

Gln Ala Gly Ile Leu Leu Ala Glu Ser Leu Ile His Asp Cys Thr Phe
        35                  40                  45

Ile Thr Arg Trp Pro Arg Thr Leu Ala Gly Ile Thr Ala Tyr Gln Ser
    50                  55                  60

Cys Leu Gln Tyr Pro Phe Thr Ser Val Pro Leu Gly Gly Gly Ala Pro
65                  70                  75                  80

Gly Thr Arg Ala Ser Arg Arg Cys Asp Arg Ala Gly Arg Trp Glu Pro
                85                  90                  95

Gly Asp Tyr Ser His Cys Leu Tyr Thr Asn Asp Ile Thr Arg Val Leu
            100                 105                 110

Tyr Thr Phe Val Leu Met Pro Ile Asn Ala Ser Asn Ala Leu Thr Leu
        115                 120                 125

Ala His Gln Leu Arg Val Tyr Thr Ala Glu Ala Ala Ser Phe Ser Asp
    130                 135                 140

Met Met Asp Val Val Tyr Val Ala Gln Met Ile Gln Lys Phe Leu Gly
145                 150                 155                 160

Tyr Val Asp Gln Ile Lys Glu Leu Val Gly Val Met Val Asp Met Ala
                165                 170                 175

Ser Asn Leu Met Leu Val Asp Glu His Leu Leu Trp Leu Ala Gln Arg
            180                 185                 190

Glu Asp Lys Ala Cys Ser Arg Ile Val Gly Ala Leu Glu Arg Ile Gly
        195                 200                 205

Gly Ala Ala Leu Ser Pro His Ala Gln His Ile Ser Val Asn Ala Arg
    210                 215                 220

Asn Val Ala Leu Glu Ala Tyr Leu Ile Lys Pro His Ser Tyr Val Gly
225                 230                 235                 240

Leu Thr Cys Thr Ala Phe Gln Arg Arg Glu Gly Gly Val Pro Gly Thr
                245                 250                 255

Arg Pro Gly Ser Pro Gly Gln Asn Pro Pro Pro Glu Pro Glu Pro Pro
            260                 265                 270

Ala Asp Gln Gln Leu Arg Phe Arg Cys Thr Thr Gly Arg Pro Asn Val
        275                 280                 285

Ser Leu Ser Ser Phe His Ile Lys Asn Ser Val Ala Leu Ala Ser Ile
    290                 295                 300

Gln Leu Pro Pro Ser Leu Phe Ser Ser Leu Pro Ala Ala Leu Ala Pro
305                 310                 315                 320

Pro Val Pro Pro Asp Cys Thr Leu Gln Leu Leu Val Phe Arg Asn Gly
                325                 330                 335

Arg Leu Phe His Ser His Ser Asn Ala Ser Arg Pro Gly Ala Ala Gly
            340                 345                 350

Pro Gly Lys Arg Arg Gly Val Ala Thr Pro Val Ile Phe Ala Gly Thr
        355                 360                 365

Ser Gly Cys Gly Val Gly Asn Leu Thr Glu Ser Val Ala Val Ser Leu
    370                 375                 380

Arg His Trp Ala Glu Gly Ala Glu Pro Val Ala Ala Trp Trp Ser Gln
385                 390                 395                 400

Glu Gly Pro Gly Glu Ala Gly Gly Trp Thr Ser Glu Gly Cys Gln Leu
                405                 410                 415

Arg Ser Ser Gln Pro Asn Val Ser Ala Leu His Cys Gln His Leu Gly
            420                 425                 430

Asn Val Ala Val Leu Met Glu Leu Ser Leu Glu
        435                 440

<210> 12
<211> 1123
<212> DNA
<213> Homo sapiens

<400> 12
atgaggagcc tcggggccct gctcttgctg ctgagcgcct gcctggcggt gagcgctggc 60
cctgtgccaa cgccgcccga caacatccaa gtgcaggaaa acttcaatat ctctcggatc 120
tatgggaagt ggtacaacct ggccatcggt tccacctgcc cctggctgaa gaagatcatg 180
gacaggatga cagtgagcac gctggtgctg ggagagggcg ctacagaggc ggagatcagc 240
atgaccagca ctcgttggcg gaaaggtgtc tgtgaggaga cgtctggagc ttatgagaaa 300
acagatactg atgggaagtt tctctatcac aaatccaaat ggaacataac catggagtcc 360
tatgtggtcc acaccaacta tgatgagtat gccattttcc tgaccaagaa attcagccgc 420
catcatggac ccaccattac tgccaagctc tacgggcggg cgccgcagct gagggaaact 480
ctcctgcagg acttcagagt ggttgcccag ggtgtgggca tccctgagga ctccatcttc 540
accatggctg accgaggtga atgtgtccct ggggagcagg aaccagagcc catcttaatc 600
ccgagagtcc ggagggctgt gctaccccaa gaagaggaag gatcaggggg tgggcaactg 660
gtaactgaag tcaccaagaa agaagattcc tgccagctgg gctactcggc cggtccctgc 720
atgggaatga ccagcaggta tttctataat ggtacatcca tggcctgtga gactttccag 780
tacggcggct gcatgggcaa cggtaacaac ttcgtcacag aaaaggagtg tctgcagacc 840
tgccgaactg tggcggcctg caatctcccc atagtccggg gcccctgccg agccttcatc 900
cagctctggg catttgatgc tgtcaagggg aagtgcgtcc tcttccccta cgggggctgc 960
cagggcaacg ggaacaagtt ctactcagag aaggagtgca gagagtactg cggtgtccct 1020
ggtgatggta ggcagccctg cagggtgcca ggctgggccg aggggctgtt ggagtcgagg 1080
tgggagagcc ctgggaggag taggggtctg gagagctgag cgt 1123

<210> 13
<211> 372
<212> PRT
<213> Homo sapiens

<400> 13
Met Arg Ser Leu Gly Ala Leu Leu Leu Leu Leu Ser Ala Cys Leu Ala
1               5                   10                  15

Val Ser Ala Gly Pro Val Pro Thr Pro Pro Asp Asn Ile Gln Val Gln
            20                  25                  30

Glu Asn Phe Asn Ile Ser Arg Ile Tyr Gly Lys Trp Tyr Asn Leu Ala
        35                  40                  45

Ile Gly Ser Thr Cys Pro Trp Leu Lys Lys Ile Met Asp Arg Met Thr
    50                  55                  60

Val Ser Thr Leu Val Leu Gly Glu Gly Ala Thr Glu Ala Glu Ile Ser
65                  70                  75                  80

Met Thr Ser Thr Arg Trp Arg Lys Gly Val Cys Glu Glu Thr Ser Gly
                85                  90                  95

Ala Tyr Glu Lys Thr Asp Thr Asp Gly Lys Phe Leu Tyr His Lys Ser
            100                 105                 110

Lys Trp Asn Ile Thr Met Glu Ser Tyr Val Val His Thr Asn Tyr Asp

115                 120                 125

Glu Tyr Ala Ile Phe Leu Thr Lys Lys Phe Ser Arg His His Gly Pro
    130                 135                 140

Thr Ile Thr Ala Lys Leu Tyr Gly Arg Ala Pro Gln Leu Arg Glu Thr
145                 150                 155                 160

Leu Leu Gln Asp Phe Arg Val Val Ala Gln Gly Val Gly Ile Pro Glu
                165                 170                 175

Asp Ser Ile Phe Thr Met Ala Asp Arg Gly Glu Cys Val Pro Gly Glu
            180                 185                 190

Gln Glu Pro Glu Pro Ile Leu Ile Pro Arg Val Arg Arg Ala Val Leu
        195                 200                 205

Pro Gln Glu Glu Glu Gly Ser Gly Gly Gly Gln Leu Val Thr Glu Val
    210                 215                 220

Thr Lys Lys Glu Asp Ser Cys Gln Leu Gly Tyr Ser Ala Gly Pro Cys
225                 230                 235                 240

Met Gly Met Thr Ser Arg Tyr Phe Tyr Asn Gly Thr Ser Met Ala Cys
                245                 250                 255

Glu Thr Phe Gln Tyr Gly Gly Cys Met Gly Asn Gly Asn Asn Phe Val
            260                 265                 270

Thr Glu Lys Glu Cys Leu Gln Thr Cys Arg Thr Val Ala Ala Cys Asn
        275                 280                 285

Leu Pro Ile Val Arg Gly Pro Cys Arg Ala Phe Ile Gln Leu Trp Ala
    290                 295                 300

Phe Asp Ala Val Lys Gly Lys Cys Val Leu Phe Pro Tyr Gly Gly Cys
305                 310                 315                 320

Gln Gly Asn Gly Asn Lys Phe Tyr Ser Glu Lys Glu Cys Arg Glu Tyr
                325                 330                 335

Cys Gly Val Pro Gly Asp Gly Arg Gln Pro Cys Arg Val Pro Gly Trp
            340                 345                 350

Ala Glu Gly Leu Leu Glu Ser Arg Trp Glu Ser Pro Gly Arg Ser Arg
        355                 360                 365

Gly Leu Glu Ser
    370

<210> 14
<211> 2249
<212> DNA
<213> Homo sapiens

<400> 14
   tgtttttttt cttcagtact ttaaatgtca tgccactctc ctggcctgta agctttccac 60
   agagaagcct gctgccagac ccattggagc ttctttgtgt gttatttgtt tcttttttct 120
   tgctgctttt acctctccgg cttctggcac tgttgctgct gctgctgctg ctgctgctgc 180
   tgcagctcca gcatcttgcg gcggcagcgg ctgatccgct gctcggcggc caagggccgg 240
   ccaaggattg cgaaaaggac caattccagt gccggaacga gcgctgcatc ccctctgtgt 300
   ggagatgcga cgaggacgat gactgcttag accacagcga cgaggacgac tgccctgcag 360
   ccaagaagac ctgtgcagac agtgacttca cctgtgacaa cggccactgc atccacgaac 420
   ggtggaagtg tgacggcgag gaggagtgtc ctgatggctc cgatgagtcc gaggccactt 480
   gcaccaagca ggtgtgtcct gcagagaagc tgagctgtgg acccaccagc cacaagtgtg 540
   tacctgcctc gtggcgctgc gacggggaga aggactgcga gggtggagcg gatgaggccg 600
   gctgtgctac ctcactgggc acctgccgtg gggacgagtt ccagtgtggg gatgggacat 660
   gtgtccttgc aatcaagcac tgcaaccagg agcaggactg tccagatggg agtgatgaag 720
   ctggctgcct acaggggctg aacgagtgtc tgcacaacaa tggcggctgc tcacacatct 780
   gcactgacct caagattggc tttgaatgca cgtgcccagc aggcttccag ctcctggacc 840
   agaagacctg tggcgacatt gatgagtgca aggacccaga tgcctgcagc cagatctgtg 900
   tcaattacaa gggctatttt aagtgtgagt gctaccctgg ctacgagatg gacctactga 960
   ccaagaactg caaggctgct gctggcaaga gcccatccct aatcttcacc aaccggcacg 1020
   aggtgcggag gatcgacctg gtgaagcgga actattcacg cctcatcccc atgctcaaga 1080
   atgtcgtggc actagatgtg gaagttgcca ccaatcgcat ctactggtgt gacctctcct 1140
   accgtaagat ctatagcgcc tacattggac aaggccagga cccgaaagag caggaggtcc 1200
   tcattgacga gcagttgcac tctccagagg gcctggcagt ggactgggtc cacaagcaca 1260
   tctactggac tgactcgggc aataagacca tctcagtggc cacagttgat ggtggccgcc 1320
   gacgcactct cttcagccgt aacctcagtg aaccccgggc catcgctgtt gaccccctgc 1380
   gagggttcat gtattggtct gactgggggg accaggccaa gattgagaaa tctgggctca 1440
   acggtgtgga ccggcaaaca ctggtgtcag acaatattga atggcccaac ggaatcaccc 1500
   tggatctgct gagccagcgc ttgtactggg tagactccaa gctacaccaa ctgtccagca 1560
   ttgacttcag tggaggcaac agaaagacgc tgatctcctc cactgacttc ctgagccacc 1620
   cttttgggat agctgtgttt gaggacaagg tgttctggac agacctggag aacgaggcca 1680
   ttttcagtgc aaatcggctc aatggcctgg aaatctccat cctggctgag aacctgaaca 1740
   acccacatga cattgtcatc ttccatgagc tgaagcagcc aagagctcca gatgcctgtg 1800
   agctgagtgt ccagcctaat ggaggctgtg aatacctgtg ccttcctgct cctcagatct 1860
   ccagccactc tcccaagtac acatgtgcct gtcctgacac aatgtggctg ggtccagaca 1920
   tgaagaggtg ctaccgagat gcaaatgaag acagtaagat gggctcaaca gtcactgccg 1980
   ctgttatcgg gatcatcgtg cccatagtgg tgatagccct cctgtgcatg agtggatacc 2040
   tgatctggag aaactggaag cggaagaaca ccaaaagcat gaattttgac aacccagtct 2100
   acaggaaaac aacagaagaa gaagacgaag atgagctcca tatagggaga actgctcaga 2160
   ttggccatgt ctatcctgca cgagtggcat taagccttga agatgatgga ctaccctgag 2220
   gatgggatca cccccttcgt gcctcatgg 2249

<210> 15
<211> 729
<212> PRT
<213> Homo sapiens

<400> 15
Met Pro Leu Ser Trp Pro Val Ser Phe Pro Gln Arg Ser Leu Leu Pro
1               5                   10                  15

Asp Pro Leu Glu Leu Leu Cys Val Leu Phe Val Ser Phe Phe Leu Leu
            20                  25                  30

Leu Leu Pro Leu Arg Leu Leu Ala Leu Leu Leu Leu Leu Leu Leu Leu
        35                  40                  45

Leu Leu Leu Gln Leu Gln His Leu Ala Ala Ala Ala Ala Asp Pro Leu
    50                  55                  60

Leu Gly Gly Gln Gly Pro Ala Lys Asp Cys Glu Lys Asp Gln Phe Gln
65                  70                  75                  80

Cys Arg Asn Glu Arg Cys Ile Pro Ser Val Trp Arg Cys Asp Glu Asp
                85                  90                  95

Asp Asp Cys Leu Asp His Ser Asp Glu Asp Asp Cys Pro Ala Ala Lys
            100                 105                 110

Lys Thr Cys Ala Asp Ser Asp Phe Thr Cys Asp Asn Gly His Cys Ile
        115                 120                 125

His Glu Arg Trp Lys Cys Asp Gly Glu Glu Glu Cys Pro Asp Gly Ser
    130                 135                 140

Asp Glu Ser Glu Ala Thr Cys Thr Lys Gln Val Cys Pro Ala Glu Lys
145                 150                 155                 160

Leu Ser Cys Gly Pro Thr Ser His Lys Cys Val Pro Ala Ser Trp Arg
                165                 170                 175

Cys Asp Gly Glu Lys Asp Cys Glu Gly Gly Ala Asp Glu Ala Gly Cys
            180                 185                 190

Ala Thr Ser Leu Gly Thr Cys Arg Gly Asp Glu Phe Gln Cys Gly Asp
        195                 200                 205

Gly Thr Cys Val Leu Ala Ile Lys His Cys Asn Gln Glu Gln Asp Cys
    210                 215                 220

Pro Asp Gly Ser Asp Glu Ala Gly Cys Leu Gln Gly Leu Asn Glu Cys
225                 230                 235                 240

Leu His Asn Asn Gly Gly Cys Ser His Ile Cys Thr Asp Leu Lys Ile
                245                 250                 255

Gly Phe Glu Cys Thr Cys Pro Ala Gly Phe Gln Leu Leu Asp Gln Lys
            260                 265                 270

Thr Cys Gly Asp Ile Asp Glu Cys Lys Asp Pro Asp Ala Cys Ser Gln
        275                 280                 285

Ile Cys Val Asn Tyr Lys Gly Tyr Phe Lys Cys Glu Cys Tyr Pro Gly
    290                 295                 300

Tyr Glu Met Asp Leu Leu Thr Lys Asn Cys Lys Ala Ala Ala Gly Lys
305                 310                 315                 320

Ser Pro Ser Leu Ile Phe Thr Asn Arg His Glu Val Arg Arg Ile Asp
                325                 330                 335

Leu Val Lys Arg Asn Tyr Ser Arg Leu Ile Pro Met Leu Lys Asn Val
            340                 345                 350

Val Ala Leu Asp Val Glu Val Ala Thr Asn Arg Ile Tyr Trp Cys Asp
        355                 360                 365

Leu Ser Tyr Arg Lys Ile Tyr Ser Ala Tyr Ile Gly Gln Gly Gln Asp
    370                 375                 380

Pro Lys Glu Gln Glu Val Leu Ile Asp Glu Gln Leu His Ser Pro Glu
385                 390                 395                 400

Gly Leu Ala Val Asp Trp Val His Lys His Ile Tyr Trp Thr Asp Ser
                405                 410                 415

Gly Asn Lys Thr Ile Ser Val Ala Thr Val Asp Gly Gly Arg Arg Arg
            420                 425                 430

Thr Leu Phe Ser Arg Asn Leu Ser Glu Pro Arg Ala Ile Ala Val Asp
        435                 440                 445

Pro Leu Arg Gly Phe Met Tyr Trp Ser Asp Trp Gly Asp Gln Ala Lys
    450                 455                 460

Ile Glu Lys Ser Gly Leu Asn Gly Val Asp Arg Gln Thr Leu Val Ser
465                 470                 475                 480

Asp Asn Ile Glu Trp Pro Asn Gly Ile Thr Leu Asp Leu Leu Ser Gln
                485                 490                 495

Arg Leu Tyr Trp Val Asp Ser Lys Leu His Gln Leu Ser Ser Ile Asp
            500                 505                 510

Phe Ser Gly Gly Asn Arg Lys Thr Leu Ile Ser Ser Thr Asp Phe Leu
        515                 520                 525

Ser His Pro Phe Gly Ile Ala Val Phe Glu Asp Lys Val Phe Trp Thr
    530                 535                 540

Asp Leu Glu Asn Glu Ala Ile Phe Ser Ala Asn Arg Leu Asn Gly Leu
545                 550                 555                 560

Glu Ile Ser Ile Leu Ala Glu Asn Leu Asn Asn Pro His Asp Ile Val
                565                 570                 575

Ile Phe His Glu Leu Lys Gln Pro Arg Ala Pro Asp Ala Cys Glu Leu
            580                 585                 590

Ser Val Gln Pro Asn Gly Gly Cys Glu Tyr Leu Cys Leu Pro Ala Pro
        595                 600                 605

Gln Ile Ser Ser His Ser Pro Lys Tyr Thr Cys Ala Cys Pro Asp Thr
    610                 615                 620

Met Trp Leu Gly Pro Asp Met Lys Arg Cys Tyr Arg Asp Ala Asn Glu
625                 630                 635                 640

Asp Ser Lys Met Gly Ser Thr Val Thr Ala Ala Val Ile Gly Ile Ile
                645                 650                 655

Val Pro Ile Val Val Ile Ala Leu Leu Cys Met Ser Gly Tyr Leu Ile
            660                 665                 670

Trp Arg Asn Trp Lys Arg Lys Asn Thr Lys Ser Met Asn Phe Asp Asn
        675                 680                 685

Pro Val Tyr Arg Lys Thr Thr Glu Glu Glu Asp Glu Asp Glu Leu His
    690                 695                 700

Ile Gly Arg Thr Ala Gln Ile Gly His Val Tyr Pro Ala Arg Val Ala
705                 710                 715                 720

Leu Ser Leu Glu Asp Asp Gly Leu Pro
                725

<210> 16
<211> 2348
<212> DNA
<213> Homo sapiens

<400> 16
tgtttttttt cttcagtact ttaaatgtca tgccactctc ctggcctgta agctttccac 60
agagaagcct gctgccagac ccattggagc ttctttgtgt gttatttgtt tcttttttct 120
tgctgctttt acctctccgg cttctggcac tgttgctgct gctgctgctg ctgctgctgc 180
tgcagctcca gcatcttgcg gcggcagcgg ctgatccgct gctcggcggc caagggccgg 240
ccaaggattg cgaaaaggac caattccagt gccggaacga gcgctgcatc ccctctgtgt 300
ggagatgcga cgaggacgat gactgcttag accacagcga cgaggacgac tgccctgcag 360
ccaagaagac ctgtgcagac agtgacttca cctgtgacaa cggccactgc atccacgaac 420
ggtggaagtg tgacggcgag gaggagtgtc ctgatggctc cgatgagtcc gaggccactt 480
gcaccaagca ggtgtgtcct gcagagaagc tgagctgtgg acccaccagc cacaagtgtg 540
tacctgcctc gtggcgctgc gacggggaga aggactgcga gggtggagcg gatgaggccg 600
gctgtgctac ctcactgggc acctgccgtg gggacgagtt ccagtgtggg gatgggacat 660
gtgtccttgc aatcaagcac tgcaaccagg agcaggactg tccagatggg agtgatgaag 720
ctggctgcct acaggttccg ccaacattcc tgggaaacag gaggaggccc agggggctga 780
acgagtgtct gcacaacaat ggcggctgct cacacatctg cactgacctc aagattggct 840
ttgaatgcac gtgcccagca ggcttccagc tcctggacca gaagacctgt ggcgacattg 900
atgagtgcaa ggacccagat gcctgcagcc agatctgtgt caattacaag ggctatttta 960
agtgtgagtg ctaccctggc tacgagatgg acctactgac caagaactgc aaggctgctg 1020
ctggaaagag cccatcccta atcttcacca accggcacga ggtgcggagg atcgacctgg 1080
tgaagcggaa ctattcacgc ctcatcccca tgctcaagaa tgtcgtggca ctagatgtgg 1140
aagttccacc aatcgcatct actggtgtga cctctcctac cgtaagatct atagcgccta 1200
catgggacag gcacccctca cccccactct tcatgccttg ctgcagagga tatggccgct 1260
cacctagaca aggccaggac ccgaaagagc aggaggtcct cattgacgag cagttgcact 1320
ctccagaggg cctggcagtg gactgggtcc acaagcacat ctactggact gactcgggca 1380
ataagaccat ctcagtggcc acagttgatg gtggccgccg acgcactctc ttcagccgta 1440
acctcagtga accccgggcc atcgctgttg accccctgcg agggttcatg tattggtctg 1500
actgggggga ccaggccaag attgagaaat ctgggctcaa cggtgtggac cggcaaacac 1560
tggtgtcaga caatattgaa tggcccaacg gaatcaccct ggatctgctg agccagcgct 1620
tgtactgggt agactccaag ctacaccaac tgtccagcat tgacttcagt ggaggcaaca 1680
gaaagacgct gatctcctcc actgacttcc tgagccaccc ttttgggata gctgtgtttg 1740
aggacaaggt gttctggaca gacctggaga acgaggccat tttcagtgca aatcggctca 1800
atggcctgga aatctccatc ctggctgaga acctgaacaa cccacatgac attgtcatct 1860
tccatgagct gaagcagcca agagctccag atgcctgtga gctgagtgtc cagcctaatg 1920
gaggctgtga atacctgtgc cttcctgctc ctcagatctc cagccactct cccaagtaca 1980
catgtgcctg tcctgacaca atgtggctgg gtccagacat gaagaggtgc taccgagatg 2040
caaatgaaga cagtaagatg ggctcaacag tcactgccgc tgttatcggg atcatcgtgc 2100
ccatagtggt gatagccctc ctgtgcatga gtggatacct gatctggaga aactggaagc 2160
ggaagaacac caaaagcatg aattttgaca acccagtcta caggaaaaca acagaagaag 2220

aagacgaaga tgagctccat atagggagaa ctgctcagat tggccatgtc tatcctgcac 2280
gagtggcatt aagccttgaa gatgatggac taccctgagg atgggatcac ccccttcgtg 2340
cctcatgg 2348

<210> 17
<211> 762
<212> PRT
<213> Homo sapiens

<400> 17
   Met Pro Leu Ser Trp Pro Val Ser Phe Pro Gln Arg Ser Leu Leu Pro
   1 5 10 15
   Asp Pro Leu Glu Leu Leu Cys Val Leu Phe Val Ser Phe Phe Leu Leu
   20 25 30
   Leu Leu Pro Leu Arg Leu Leu Ala Leu Leu Leu Leu Leu Leu Leu Leu
   35 40 45
   Leu Leu Leu Gln Leu Gln His Leu Ala Ala Ala Ala Ala Asp Pro Leu
   50 55 60
   Leu Gly Gly Gln Gly Pro Ala Lys Asp Cys Glu Lys Asp Gln Phe Gln
   65 70 75 80
   Cys Arg Asn Glu Arg Cys Ile Pro Ser Val Trp Arg Cys Asp Glu Asp
   85 90 95
   Asp Asp Cys Leu Asp His Ser Asp Glu Asp Asp Cys Pro Ala Ala Lys
   100 105 110
   Lys Thr Cys Ala Asp Ser Asp Phe Thr Cys Asp Asn Gly His Cys Ile
   115 120 125
   His Glu Arg Trp Lys Cys Asp Gly Glu Glu Glu Cys Pro Asp Gly Ser
   130 135 140
   Asp Glu Ser Glu Ala Thr Cys Thr Lys Gln Val Cys Pro Ala Glu Lys
   145 150 155 160
   Leu Ser Cys Gly Pro Thr Ser His Lys Cys Val Pro Ala Ser Trp Arg
   165 170 175
   Cys Asp Gly Glu Lys Asp Cys Glu Gly Gly Ala Asp Glu Ala Gly Cys
   180 185 190
   Ala Thr Ser Leu Gly Thr Cys Arg Gly Asp Glu Phe Gln Cys Gly Asp
   195 200 205
   Gly Thr Cys Val Leu Ala Ile Lys His Cys Asn Gln Glu Gln Asp Cys
   210 215 220
   Pro Asp Gly Ser Asp Glu Ala Gly Cys Leu Gln Val Pro Pro Thr Phe
   225 230 235 240
   Leu Gly Asn Arg Arg Arg Pro Arg Gly Leu Asn Glu Cys Leu His Asn
   245 250 255
   Asn Gly Gly Cys Ser His Ile Cys Thr Asp Leu Lys Ile Gly Phe Glu
   260 265 270
   Cys Thr Cys Pro Ala Gly Phe Gln Leu Leu Asp Gln Lys Thr Cys Gly
   275 280 285
   Asp Ile Asp Glu Cys Lys Asp Pro Asp Ala Cys Ser Gln Ile Cys Val
   290 295 300
   Asn Tyr Lys Gly Tyr Phe Lys Cys Glu Cys Tyr Pro Gly Tyr Glu Met
   305 310 315 320
   Asp Leu Leu Thr Lys Asn Cys Lys Ala Ala Ala Gly Lys Ser Pro Ser
   325 330 335
   Leu Ile Phe Thr Asn Arg His Glu Val Arg Arg Ile Asp Leu Val Lys
   340 345 350
   Arg Asn Tyr Ser Arg Leu Ile Pro Met Leu Lys Asn Val Val Ala Leu
   355 360 365
   Asp Val Glu Val Pro Pro Ile Ala Ser Thr Gly Val Thr Ser Pro Thr
   370 375 380
   Val Arg Ser Ile Ala Pro Thr Trp Asp Arg His Pro Ser Pro Pro Leu
   385 390 395 400
   Phe Met Pro Cys Cys Arg Gly Tyr Gly Arg Ser Pro Arg Gln Gly Gln
   405 410 415
   Asp Pro Lys Glu Gln Glu Val Leu Ile Asp Glu Gln Leu His Ser Pro
   420 425 430
   Glu Gly Leu Ala Val Asp Trp Val His Lys His Ile Tyr Trp Thr Asp
   435 440 445
   Ser Gly Asn Lys Thr Ile Ser Val Ala Thr Val Asp Gly Gly Arg Arg
   450 455 460
   Arg Thr Leu Phe Ser Arg Asn Leu Ser Glu Pro Arg Ala Ile Ala Val
   465 470 475 480
   Asp Pro Leu Arg Gly Phe Met Tyr Trp Ser Asp Trp Gly Asp Gln Ala
   485 490 495
   Lys Ile Glu Lys Ser Gly Leu Asn Gly Val Asp Arg Gln Thr Leu Val
   500 505 510
   Ser Asp Asn Ile Glu Trp Pro Asn Gly Ile Thr Leu Asp Leu Leu Ser
   515 520 525
   Gln Arg Leu Tyr Trp Val Asp Ser Lys Leu His Gln Leu Ser Ser Ile
   530 535 540
   Asp Phe Ser Gly Gly Asn Arg Lys Thr Leu Ile Ser Ser Thr Asp Phe
   545                 550                 555                 560
   Leu Ser His Pro Phe Gly Ile Ala Val Phe Glu Asp Lys Val Phe Trp
                   565                 570                 575
   Thr Asp Leu Glu Asn Glu Ala Ile Phe Ser Ala Asn Arg Leu Asn Gly
               580                 585                 590
   Leu Glu Ile Ser Ile Leu Ala Glu Asn Leu Asn Asn Pro His Asp Ile
           595                 600                 605
   Val Ile Phe His Glu Leu Lys Gln Pro Arg Ala Pro Asp Ala Cys Glu
       610                 615                 620
   Leu Ser Val Gln Pro Asn Gly Gly Cys Glu Tyr Leu Cys Leu Pro Ala
   625                 630                 635                 640
   Pro Gln Ile Ser Ser His Ser Pro Lys Tyr Thr Cys Ala Cys Pro Asp
                   645                 650                 655
   Thr Met Trp Leu Gly Pro Asp Met Lys Arg Cys Tyr Arg Asp Ala Asn
               660                 665                 670
   Glu Asp Ser Lys Met Gly Ser Thr Val Thr Ala Ala Val Ile Gly Ile
           675                 680                 685
   Ile Val Pro Ile Val Val Ile Ala Leu Leu Cys Met Ser Gly Tyr Leu
       690                 695                 700
   Ile Trp Arg Asn Trp Lys Arg Lys Asn Thr Lys Ser Met Asn Phe Asp
   705                 710                 715                 720
   Asn Pro Val Tyr Arg Lys Thr Thr Glu Glu Glu Asp Glu Asp Glu Leu
                   725                 730                 735
   His Ile Gly Arg Thr Ala Gln Ile Gly His Val Tyr Pro Ala Arg Val
               740                 745                 750
   Ala Leu Ser Leu Glu Asp Asp Gly Leu Pro
           755                 760

<210> 18
<211> 2537
<212> DNA
<213> Homo sapiens

<400> 18
tgtttttttt cttcagtact ttaaatgtca tgccactctc ctggcctgta agctttccac 60
agagaagcct gctgccagac ccattggagc ttctttgtgt gttatttgtt tcttttttct 120
tgctgctttt acagctccag catcttgcgg cggcagcggc tgatccgctg ctcggcggcc 180
aagggccggc caaggattgc gaaaaggacc aattccagtg ccggaacgag cgctgcatcc 240
cctctgtgtg gagatgcgac gaggacgatg actgcttaga ccacagcgac gaggacgact 300
gccctgcagc caagaagaca cctgtgagaa gtgacttcac ctgtgacaac ggccactgca 360
tccacgaacg gtggaagtgt gacggcgagg aggagtgtcc tgatggctcc gatgagtccg 420
aggccacttg caccaagcag gtgtgtcctg cagagaagct gagctgtgga cccactagcc 480
acaagtgtgt acctgcctcg tggcgctgcg acggggagaa ggactgcgag ggtggagcgg 540

atgaggccgg ctgtgctacc tcactgggca cctgccgtgg ggacgagttc cagtgtgggg 600
atgggacatg tgtccttgca atcaagcact gcaaccagga gcaggactgt ccagatggga 660
gtgatgaagc tggctgccta caggggctga acgagtgtct gcacaacaat ggcggctgct 720
cacacatctg cactgacctc aagattggct ttgaatgcac gtgcccagca ggcttccagc 780
tcctggacca gaagacctgt ggcgctggca agagcccatc cctaatcttc accaaccggc 840
acgaggtgcg gaggatcgac ctggtgaagc ggaactattc acgcctcatc cccatgctca 900
agaatgtcgt ggcactagat gtggaagttg ccaccaatcg catctactgg tgtgacctct 960
cctaccgtaa gatctatagc gcctacatgg acaaggccag tgacccgaaa gagcaggagg 1020
tcctcattga cgagcagttg cactctccag agggcctggc agtggactgg gtccacaagc 1080
acatctactg gactgactcg ggcaataaga ccatctcagt ggccacagtt gatggtggcc 1140
gccgacgcac tctcttcagc cgtaacctca gtgaaccccg ggccatcgct gttgaccccc 1200
tgcgagggtt catgtattgg tctgactggg gggaccaggc caagattgag aaatctgggc 1260
tcaacggtgt ggaccggcaa acactggtgt cagacaatat tgaatggccc aacggaatca 1320
ccttggatct gctgagccag cgcttgtact gggtagactc caagctacac caactgtcca 1380
gcattgactt cagtggaggc aacagaaaga cgctgatctc ctccactgac ttcctgagcc 1440
acccttttgg gatagctgtg tttgaggaca aggtgttctg gacagacctg gagaacgagg 1500
ccattttcag tgcaaatcgg ctcaatggcc tggaaatctc catcctggct gagaacctga 1560
acaacccaca tgacattgtc atcttccatg agctgaagca gccaagagct ccagatgcct 1620
gtgagctgag tgtccagcct aatggaggct gtgaatacct gtgccttcct gctcctcaga 1680
tctccagcca ctctcccaag tacacatgtg cctgtcctga cacaatgtgg ctgggtccag 1740
acatgaagag gtgctaccga gcacctcaat ctacctcaac tacgacgtta gcttctacca 1800
tgacgaggac agtacctgcc accacaagag cccccgggac caccgtccac agatccacct 1860
accagaacca cagcacagag acaccaagcc tgacagctgc agtcccaagc tcagttagtg 1920
tccccagggc tcccagcatc agcccgtcta ccctaagccc tgcaaccagc aaccactccc 1980
agcacgcaaa tgaagacagt aagatgggct caacagtcac tgccgctgtt atcgggatca 2040
tcgtgcccat agcaaatgaa gacagtaaga tgggctcaac agtcactgcc gctgttatcg 2100
ggatcatcgt gcccataggt gatagccctc ctgtgcatga gtggatacct gatctggaga 2160
aactggaagc ggaagaacac caaaagcatg aatttgacaa cccagtctac aggaaaacaa 2220
cagaagaaga agacgaagat gagctccata tagggagaac tgctcagatt ggccatgtct 2280
atcctgcagc aatcagcagc tttgatcgcc cactgtgggc agagccctgt cttggggaga 2340
ccagagaacc agaagaccca gcccctgccc tgaaggagct ttttgtcttg ccgggggaac 2400
caaggtcaca gctgcaccaa ctcccgaaga accctctttc cgagctgcct gtcgtcaaat 2460
ccaagcagcg agtggcatta agccttgaag atgatggact accctgagga tgggatcacc 2520
cccttcgtgc ctcatgg 2537

<210> 19
<211> 825
<212> PRT
<213> Homo sapiens

<400> 19
Met Pro Leu Ser Trp Pro Val Ser Phe Pro Gln Arg Ser Leu Leu Pro
1               5                   10                  15

Asp Pro Leu Glu Leu Leu Cys Val Leu Phe Val Ser Phe Phe Leu Leu
            20                  25                  30

Leu Leu Gln Leu Gln His Leu Ala Ala Ala Ala Ala Asp Pro Leu Leu
        35                  40                  45

Gly Gly Gln Gly Pro Ala Lys Asp Cys Glu Lys Asp Gln Phe Gln Cys
    50                  55                  60

Arg Asn Glu Arg Cys Ile Pro Ser Val Trp Arg Cys Asp Glu Asp Asp
65                  70                  75                  80

Asp Cys Leu Asp His Ser Asp Glu Asp Asp Cys Pro Ala Ala Lys Lys
                85                  90                  95

Thr Pro Val Arg Ser Asp Phe Thr Cys Asp Asn Gly His Cys Ile His
            100                 105                 110

Glu Arg Trp Lys Cys Asp Gly Glu Glu Glu Cys Pro Asp Gly Ser Asp
        115                 120                 125

Glu Ser Glu Ala Thr Cys Thr Lys Gln Val Cys Pro Ala Glu Lys Leu
    130                 135                 140

Ser Cys Gly Pro Thr Ser His Lys Cys Val Pro Ala Ser Trp Arg Cys
145                 150                 155                 160

Asp Gly Glu Lys Asp Cys Glu Gly Gly Ala Asp Glu Ala Gly Cys Ala
                165                 170                 175

Thr Ser Leu Gly Thr Cys Arg Gly Asp Glu Phe Gln Cys Gly Asp Gly
            180                 185                 190

Thr Cys Val Leu Ala Ile Lys His Cys Asn Gln Glu Gln Asp Cys Pro
        195                 200                 205

Asp Gly Ser Asp Glu Ala Gly Cys Leu Gln Gly Leu Asn Glu Cys Leu
    210                 215                 220

His Asn Asn Gly Gly Cys Ser His Ile Cys Thr Asp Leu Lys Ile Gly
225                 230                 235                 240

Phe Glu Cys Thr Cys Pro Ala Gly Phe Gln Leu Leu Asp Gln Lys Thr
                245                 250                 255

Cys Gly Ala Gly Lys Ser Pro Ser Leu Ile Phe Thr Asn Arg His Glu
            260                 265                 270

Val Arg Arg Ile Asp Leu Val Lys Arg Asn Tyr Ser Arg Leu Ile Pro
        275                 280                 285

Met Leu Lys Asn Val Val Ala Leu Asp Val Glu Val Ala Thr Asn Arg
    290                 295                 300

Ile Tyr Trp Cys Asp Leu Ser Tyr Arg Lys Ile Tyr Ser Ala Tyr Met
305                 310                 315                 320

Asp Lys Ala Ser Asp Pro Lys Glu Gln Glu Val Leu Ile Asp Glu Gln
                325                 330                 335

Leu His Ser Pro Glu Gly Leu Ala Val Asp Trp Val His Lys His Ile
            340                 345                 350

Tyr Trp Thr Asp Ser Gly Asn Lys Thr Ile Ser Val Ala Thr Val Asp
        355                 360                 365

Gly Gly Arg Arg Arg Thr Leu Phe Ser Arg Asn Leu Ser Glu Pro Arg
    370                 375                 380

Ala Ile Ala Val Asp Pro Leu Arg Gly Phe Met Tyr Trp Ser Asp Trp
385                 390                 395                 400

Gly Asp Gln Ala Lys Ile Glu Lys Ser Gly Leu Asn Gly Val Asp Arg
                405                 410                 415

Gln Thr Leu Val Ser Asp Asn Ile Glu Trp Pro Asn Gly Ile Thr Leu
            420                 425                 430

Asp Leu Leu Ser Gln Arg Leu Tyr Trp Val Asp Ser Lys Leu His Gln
        435                 440                 445

Leu Ser Ser Ile Asp Phe Ser Gly Gly Asn Arg Lys Thr Leu Ile Ser
    450                 455                 460

Ser Thr Asp Phe Leu Ser His Pro Phe Gly Ile Ala Val Phe Glu Asp
465                 470                 475                 480

Lys Val Phe Trp Thr Asp Leu Glu Asn Glu Ala Ile Phe Ser Ala Asn
                485                 490                 495

Arg Leu Asn Gly Leu Glu Ile Ser Ile Leu Ala Glu Asn Leu Asn Asn
            500                 505                 510

Pro His Asp Ile Val Ile Phe His Glu Leu Lys Gln Pro Arg Ala Pro
        515                 520                 525

Asp Ala Cys Glu Leu Ser Val Gln Pro Asn Gly Gly Cys Glu Tyr Leu
    530                 535                 540

Cys Leu Pro Ala Pro Gln Ile Ser Ser His Ser Pro Lys Tyr Thr Cys
545                 550                 555                 560

Ala Cys Pro Asp Thr Met Trp Leu Gly Pro Asp Met Lys Arg Cys Tyr
                565                 570                 575

Arg Ala Pro Gln Ser Thr Ser Thr Thr Thr Leu Ala Ser Thr Met Thr
            580                 585                 590

Arg Thr Val Pro Ala Thr Thr Arg Ala Pro Gly Thr Thr Val His Arg
        595                 600                 605

Ser Thr Tyr Gln Asn His Ser Thr Glu Thr Pro Ser Leu Thr Ala Ala
    610                 615                 620

Val Pro Ser Ser Val Ser Val Pro Arg Ala Pro Ser Ile Ser Pro Ser
625                 630                 635                 640

Thr Leu Ser Pro Ala Thr Ser Asn His Ser Gln His Ala Asn Glu Asp
                645                 650                 655

Ser Lys Met Gly Ser Thr Val Thr Ala Ala Val Ile Gly Ile Ile Val
            660                 665                 670

Pro Ile Ala Asn Glu Asp Ser Lys Met Gly Ser Thr Val Thr Ala Ala
        675                 680                 685

Val Ile Gly Ile Ile Val Pro Ile Gly Asp Ser Pro Pro Val His Glu
    690                 695                 700

Trp Ile Pro Asp Leu Glu Lys Leu Glu Ala Glu Glu His Gln Lys His
705                 710                 715                 720

Glu Phe Asp Asn Pro Val Tyr Arg Lys Thr Thr Glu Glu Glu Asp Glu
                725                 730                 735

Asp Glu Leu His Ile Gly Arg Thr Ala Gln Ile Gly His Val Tyr Pro
            740                 745                 750

Ala Ala Ile Ser Ser Phe Asp Arg Pro Leu Trp Ala Glu Pro Cys Leu
        755                 760                 765

Gly Glu Thr Arg Glu Pro Glu Asp Pro Ala Pro Ala Leu Lys Glu Leu
    770                 775                 780

Phe Val Leu Pro Gly Glu Pro Arg Ser Gln Leu His Gln Leu Pro Lys
785                 790                 795                 800

Asn Pro Leu Ser Glu Leu Pro Val Val Lys Ser Lys Gln Arg Val Ala
                805                 810                 815

Leu Ser Leu Glu Asp Asp Gly Leu Pro
            820                 825

<210> 20
<211> 3039
<212> DNA
<213> Homo sapiens

<400> 20
atgggcctcc ccgagccggg ccctctccgg cttctggcgc tgctgctgct gctgctgctg 60
ctgctgctgc tgcggctcca gcatcttgcg gcggcagcgg ctgatccgct gctcggcggc 120
caagggccgg ccaaggagtg cgaaaaggac caattccagt gccggaacga gcgctgcatc 180
ccctctgtgt ggagatgcga cgaggacgat gactgcttag accacagcga cgaggacgac 240
tgccccaaga agacctgtgc agacagtgac ttcacctgtg acaacggcca ctgcatccac 300
gaacggtgga agtgtgacgg cgaggaggag tgtcctgatg gctccgatga gtccgaggcc 360
acttgcacca agcaggtgtg tcctgcagag aagctgagct gtggacccac cagccacaag 420
tgtgtacctg cctcgtggcg ctgcgacggg gagaaggact gcgagggtgg agcggatgag 480
gccggctgtg ctaccttgtg cgccccgcac gagttccagt gcggcaaccg ctcgtgcctg 540
gccgccgtgt tcgtgtgcga cggcgatgac gactgtggtg acggcagcga tgagcgcggc 600
tgtgcagacc cggcctgcgg gccccgcgag ttccgctgcg gcggcgatgg cggcggcgcc 660
tgcatcccgg agcgctgggt ctgcgaccgc cagtttgact gcgaggaccg ctcggacgag 720
gcagccgagc tctgcggccg cccgggcccc ggggccacgt ccgcgcccgc cgcctgcgcc 780
accgtctccc agttcgcctg ccgcagcggc gagtgcgtgc acctgggctg gcgctgcgac 840
ggcgaccgcg actgcaaaga caaatcggac gaggccgact gcccactggg cacctgccgt 900
ggggacgagt tccagtgtgg ggatgggaca tgtgtccttg caatcaagca ctgcaaccag 960
gagcaggact gtccagatgg gagtgatgaa gctggctgcc tacaggagtc accctgtgag 1020
ggtccccgca gatttcagtg taagagtggc aagcgcgtgg acggcgggaa agtgtgtgat 1080
gtgcagaggg actgccggga ctggtcggat gagcttctga aagtgtggtg cgtctcctac 1140
catccccctc ctgggctgaa cgagtgtctg cacaacaatg gcggctgctc acacatctgc 1200
actgacctca agattggctt tgaatgcacg tgcccagcag gcttccagct cctggaccag 1260
aagacctgtg gcgacattga tgagtgcaag gacccagatg cctgcagcca gatctgtgtc 1320
aattacaagg gctattttaa gtgtgagtgc taccctggct acgagatgga cctactgacc 1380

aagaactgca aggctgctgc tggaaagagc ccatccctaa tcttcaccaa ccggcacgag 1440
gtgcggagga tcgacctggt gaagcggaac tattcacgcc tcatccccat gctcaagaat 1500
gtcgtggcac tagatgtgga agttgccacc aatcgcatct actggtgtga cctctcctac 1560
cgtaagatct atagcgccta catggacaag gccagtgacc cgaaagagca ggaggtcctc 1620
attgacgagc agttgcactc tccagagggc ctggcagtgg actgggtcca caagcacatc 1680
tactggactg actcgggcaa taagaccatc tcagtggcca cagttgatgg tggccgccga 1740
cgcactctct tcagccgtaa cctcagtgaa ccccgggcca tcgctgttga ccccctgcga 1800
gggttcatgt attggtctga ctggggggac caggccaaga ttgagaaatc tgggctcaac 1860
ggtgtggacc ggcaaacact ggtgtcagac aatattgaat ggcccaacgg aatcaccctg 1920
gatctgctga gccagcgctt gtactgggta gactccaagc tacaccaact gtccagcatt 1980
gacttcagtg gaggcaacag aaagacgctg atctcctcca ctgacttcct gagccaccct 2040
tttgggatag ctgtgtttga ggacaaggtg ttctggacag acctggagaa cgaggccatt 2100
ttcagtgcaa atcggctcaa tggcctggaa atctccatcc tggctgagaa cctcaacaac 2160
ccacatgaca ttgtcatctt ccatgagctg aagcagccaa gagctccaga tgcctgtgag 2220
ctgagtgtcc agcctaatgg aggctgtgaa tacctgtgcc ttcctgctcc tcagatctcc 2280
agccactctc ccaagtacac atgtgcctgt cctgacacaa tgtggctggg tccagacatg 2340
aagaggtgct accgagcacc tcaatctacc tcaactacga cgttagcttc taccatgacg 2400
aggacagtac ctgccaccac aagagccccc gggaccaccg tccacagatc cacctaccag 2460
aaccacagca cagagacacc aagcctgaca gctgcagtcc caagctcagt tagtgtcccc 2520
agggctccca gcatcagccc gtctacccta agccctgcaa ccagcaacca ctcccagcac 2580
tatgcaaatg aagacagtaa gatgggctca acagtcactg ccgctgttat cgggatcatc 2640
gtgcccatag tggtgatagc cctcctgtgc atgagtggat acctgatctg gagaaactgg 2700
aagcggaaga acaccaaaag catgaatttt gacaacccag tctacaggaa aacaacagaa 2760
gaagaagatg aagatgagct ccatataggg agaactgctc agattggcca tgtctatcct 2820
gcagcaatca gcagctttga tcgcccactg tgggcagagc cctgtcttgg ggagaccaga 2880
gaaccggaag acccagcccc tgccctcaag gagctttttg tcttgccggg ggaaccaagg 2940
tcacagctgc accaactccc gaagaaccct ctttccgagc tgcctgtcgt caaatccaag 3000
cgagtggcat taagccttga agatgatgga ctaccctga 3039

<210> 21
<211> 1012
<212> PRT
<213> Homo sapiens

<400> 21
Met Gly Leu Pro Glu Pro Gly Pro Leu Arg Leu Leu Ala Leu Leu Leu
1               5                   10                  15

Leu Leu Leu Leu Leu Leu Leu Leu Arg Leu Gln His Leu Ala Ala Ala
            20                  25                  30

Ala Ala Asp Pro Leu Leu Gly Gly Gln Gly Pro Ala Lys Glu Cys Glu
        35                  40                  45

Lys Asp Gln Phe Gln Cys Arg Asn Glu Arg Cys Ile Pro Ser Val Trp
    50                  55                  60

Arg Cys Asp Glu Asp Asp Asp Cys Leu Asp His Ser Asp Glu Asp Asp
65                  70                  75                  80

Cys Pro Lys Lys Thr Cys Ala Asp Ser Asp Phe Thr Cys Asp Asn Gly
                85                  90                  95

His Cys Ile His Glu Arg Trp Lys Cys Asp Gly Glu Glu Glu Cys Pro
            100                 105                 110

Asp Gly Ser Asp Glu Ser Glu Ala Thr Cys Thr Lys Gln Val Cys Pro
        115                 120                 125

Ala Glu Lys Leu Ser Cys Gly Pro Thr Ser His Lys Cys Val Pro Ala
    130                 135                 140

Ser Trp Arg Cys Asp Gly Glu Lys Asp Cys Glu Gly Gly Ala Asp Glu
145                 150                 155                 160

Ala Gly Cys Ala Thr Leu Cys Ala Pro His Glu Phe Gln Cys Gly Asn
                165                 170                 175

Arg Ser Cys Leu Ala Ala Val Phe Val Cys Asp Gly Asp Asp Asp Cys
            180                 185                 190

Gly Asp Gly Ser Asp Glu Arg Gly Cys Ala Asp Pro Ala Cys Gly Pro
        195                 200                 205

Arg Glu Phe Arg Cys Gly Gly Asp Gly Gly Gly Ala Cys Ile Pro Glu
    210                 215                 220

Arg Trp Val Cys Asp Arg Gln Phe Asp Cys Glu Asp Arg Ser Asp Glu
225                 230                 235                 240

Ala Ala Glu Leu Cys Gly Arg Pro Gly Pro Gly Ala Thr Ser Ala Pro
                245                 250                 255

Ala Ala Cys Ala Thr Val Ser Gln Phe Ala Cys Arg Ser Gly Glu Cys
            260                 265                 270

Val His Leu Gly Trp Arg Cys Asp Gly Asp Arg Asp Cys Lys Asp Lys
        275                 280                 285

Ser Asp Glu Ala Asp Cys Pro Leu Gly Thr Cys Arg Gly Asp Glu Phe
    290                 295                 300

Gln Cys Gly Asp Gly Thr Cys Val Leu Ala Ile Lys His Cys Asn Gln
305                 310                 315                 320

Glu Gln Asp Cys Pro Asp Gly Ser Asp Glu Ala Gly Cys Leu Gln Glu
                325                 330                 335

Ser Pro Cys Glu Gly Pro Arg Arg Phe Gln Cys Lys Ser Gly Lys Arg
            340                 345                 350

Val Asp Gly Gly Lys Val Cys Asp Val Gln Arg Asp Cys Arg Asp Trp
        355                 360                 365

Ser Asp Glu Leu Leu Lys Val Trp Cys Val Ser Tyr His Pro Pro Pro
    370                 375                 380

Gly Leu Asn Glu Cys Leu His Asn Asn Gly Gly Cys Ser His Ile Cys
385                 390                 395                 400

Thr Asp Leu Lys Ile Gly Phe Glu Cys Thr Cys Pro Ala Gly Phe Gln
                405                 410                 415

Leu Leu Asp Gln Lys Thr Cys Gly Asp Ile Asp Glu Cys Lys Asp Pro
            420                 425                 430

Asp Ala Cys Ser Gln Ile Cys Val Asn Tyr Lys Gly Tyr Phe Lys Cys
        435                 440                 445

Glu Cys Tyr Pro Gly Tyr Glu Met Asp Leu Leu Thr Lys Asn Cys Lys
    450                 455                 460

Ala Ala Ala Gly Lys Ser Pro Ser Leu Ile Phe Thr Asn Arg His Glu
465                 470                 475                 480

Val Arg Arg Ile Asp Leu Val Lys Arg Asn Tyr Ser Arg Leu Ile Pro
                485                 490                 495

Met Leu Lys Asn Val Val Ala Leu Asp Val Glu Val Ala Thr Asn Arg
            500                 505                 510

Ile Tyr Trp Cys Asp Leu Ser Tyr Arg Lys Ile Tyr Ser Ala Tyr Met
        515                 520                 525

Asp Lys Ala Ser Asp Pro Lys Glu Gln Glu Val Leu Ile Asp Glu Gln
    530                 535                 540

Leu His Ser Pro Glu Gly Leu Ala Val Asp Trp Val His Lys His Ile
545                 550                 555                 560

Tyr Trp Thr Asp Ser Gly Asn Lys Thr Ile Ser Val Ala Thr Val Asp
                565                 570                 575

Gly Gly Arg Arg Arg Thr Leu Phe Ser Arg Asn Leu Ser Glu Pro Arg
            580                 585                 590

Ala Ile Ala Val Asp Pro Leu Arg Gly Phe Met Tyr Trp Ser Asp Trp
        595                 600                 605

Gly Asp Gln Ala Lys Ile Glu Lys Ser Gly Leu Asn Gly Val Asp Arg
    610                 615                 620

Gln Thr Leu Val Ser Asp Asn Ile Glu Trp Pro Asn Gly Ile Thr Leu
625                 630                 635                 640

Asp Leu Leu Ser Gln Arg Leu Tyr Trp Val Asp Ser Lys Leu His Gln
                645                 650                 655

Leu Ser Ser Ile Asp Phe Ser Gly Gly Asn Arg Lys Thr Leu Ile Ser
            660                 665                 670

Ser Thr Asp Phe Leu Ser His Pro Phe Gly Ile Ala Val Phe Glu Asp
        675                 680                 685

Lys Val Phe Trp Thr Asp Leu Glu Asn Glu Ala Ile Phe Ser Ala Asn
    690                 695                 700

Arg Leu Asn Gly Leu Glu Ile Ser Ile Leu Ala Glu Asn Leu Asn Asn
705                 710                 715                 720

Pro His Asp Ile Val Ile Phe His Glu Leu Lys Gln Pro Arg Ala Pro
                725                 730                 735

Asp Ala Cys Glu Leu Ser Val Gln Pro Asn Gly Gly Cys Glu Tyr Leu
            740                 745                 750

Cys Leu Pro Ala Pro Gln Ile Ser Ser His Ser Pro Lys Tyr Thr Cys
        755                 760                 765

Ala Cys Pro Asp Thr Met Trp Leu Gly Pro Asp Met Lys Arg Cys Tyr
    770                 775                 780

Arg Ala Pro Gln Ser Thr Ser Thr Thr Thr Leu Ala Ser Thr Met Thr
785                 790                 795                 800

Arg Thr Val Pro Ala Thr Thr Arg Ala Pro Gly Thr Thr Val His Arg
                805                 810                 815

Ser Thr Tyr Gln Asn His Ser Thr Glu Thr Pro Ser Leu Thr Ala Ala
            820                 825                 830

Val Pro Ser Ser Val Ser Val Pro Arg Ala Pro Ser Ile Ser Pro Ser
        835                 840                 845

Thr Leu Ser Pro Ala Thr Ser Asn His Ser Gln His Tyr Ala Asn Glu
    850                 855                 860

Asp Ser Lys Met Gly Ser Thr Val Thr Ala Ala Val Ile Gly Ile Ile
865                 870                 875                 880

Val Pro Ile Val Val Ile Ala Leu Leu Cys Met Ser Gly Tyr Leu Ile
                885                 890                 895

Trp Arg Asn Trp Lys Arg Lys Asn Thr Lys Ser Met Asn Phe Asp Asn
            900                 905                 910

Pro Val Tyr Arg Lys Thr Thr Glu Glu Glu Asp Glu Asp Glu Leu His
        915                 920                 925

Ile Gly Arg Thr Ala Gln Ile Gly His Val Tyr Pro Ala Ala Ile Ser
    930                 935                 940

Ser Phe Asp Arg Pro Leu Trp Ala Glu Pro Cys Leu Gly Glu Thr Arg
945                 950                 955                 960

Glu Pro Glu Asp Pro Ala Pro Ala Leu Lys Glu Leu Phe Val Leu Pro
                965                 970                 975

Gly Glu Pro Arg Ser Gln Leu His Gln Leu Pro Lys Asn Pro Leu Ser
            980                 985                 990

Glu Leu Pro Val Val Lys Ser Lys Arg Val Ala Leu Ser Leu Glu Asp
        995                 1000                1005

Asp Gly Leu Pro
    1010

<210> 22
<211> 2537
<212> DNA
<213> Homo sapiens

<400> 22
atgggcctcc ccgagccggg ccctctccgg cttctggcgc tgctgctgct gctgctgctg 60
ctgctgctgc tgcggctcca gcatcttgcg gcggcagcgg ctgatccgct gctcggcggc 120
caagggccgg ccaaggattg cgaaaaggac caattccagt gccggaacga gcgctgcatc 180
ccctctgtgt ggagatgcga cgaggacgat gactgcttag accacagcga cgaggacgac 240
tgccccaaga agacctgtgc agacagtgac ttcacctgtg acaacggcca ctgcatccac 300
gaacggtgga agtgtgacgg cgaggaggag tgtcctgatg gctccgatga gtccgaggcc 360
acttgcacca agcaggtgtg tcctgcagag aagctgagct gtggacccac cagccacaag 420
tgtgtacctg cctcgtggcg ctgcgacggg gagaaggact gcgagggtgg agcggatgag 480
gccggctgtg ctacctcact gggcacctgc cgtggggacg agttccagtg tggggatggg 540
acatgtgtcc ttgcaatcaa gcactgcaac caggagcagg actgtccaga tgggagtgat 600
gaagctggct gcctacaggg gctgaacgag tgtctgcaca acaatggcgg ctgctcacac 660
atctgcactg acctcaagat tggctttgaa tgcacgtgcc cagcaggctt ccagctcctg 720
gaccagaaga cctgtggcga cattgatgag tgcaaggacc cagatgcctg cagccagatc 780
tgtgtcaatt acaagggcta ttttaagtgt gagtgctacc ctggctacga gatggaccta 840
ctgaccaaga actgcaaggc tgctgctgga aagagcccat ccctaatctt caccaaccgg 900
cacgaggtgc ggaggatcga cctggtgaag cggaactatt cacgcctcat ccccatgctc 960
aagaatgtcg tggcactaga tgtggaagtt gccaccaatc gcatctactg gtgtgacctc 1020
tcctaccgta agatctatag cgcctacatg gacaaggcca gtgacccgaa agagcaggag 1080
gtcctcattg acgagcagtt gcactctcca gagggcctgg cagtggactg ggtccacaag 1140
cacatctact ggactgactc gggcaataag aacatctctg gtggccgccg acgcactctc 1200
ttcagccgta acctcagtga accccgggcc atcgctgttg accccctgcg agggttcatg 1260
tattggtctg actgggggga ccaggccaag attgagaaat ctgggctcaa cggtgtggac 1320
cggcaaacac tggtgtcaga caatattgaa tggcccaacg gaatcaccct ggatctgctg 1380
agccagcgct tgtactgggt agactccaag ctacaccaac tgtccagcat tgacttcagt 1440
ggaggcaaca gaaagacgct gatctcctcc actgacttcc tgagccaccc ttttgggata 1500
gctgtgtttg aggacaaggt gttctggaca gacctggaga acgaggccat tttcagtgca 1560
aatcggctca atggcctgga aatctccatc ctggctgaga acctcaacaa cccacatgac 1620
attgtcatct tccatgagct gaagcagcca agagctccag atgcctgtga gctgagtgtc 1680
cagcctaatg gaggctgtga atacctgtgc cttcctgctc ctcagatctc cagccactct 1740
cccaagtaca catgtgcctg tcctgacaca atgtggctgg gtccagacat gaagaggtgc 1800
taccgagatg caaatgaaga cagtaagatg ggctcaacag tcactgccgc tgttatcggg 1860
atcatcgtgc ccatagtggt gatagccctc ctgtgcatga gtggatacct gatctggaga 1920
aactggaagc ggaagaacac caaaagcatg aattttgaca acccagtcta caggaaaaca 1980
acagaagaag aagatgaaga tgagctccat atagggagaa ctgctcagat tggccatgtc 2040
tatcctgcac gagtggcatt aagccttgaa gatgatggac taccctgagg atgggatcac 2100
ccccttcgtg cctcatggaa ttcagtccca tgcactacac tccggatggt gtatgactgg 2160
atgaatgggt ttctatatat gggtctgtgt gagtgtatgt gtgtgtgtga tttttttttt 2220
taaatttatg ttgcggaaag gtaaccacaa agttatgatg aactgcaaac atccaaagga 2280
tgtgagagtt tttctatgta taatgtttta tacacttttt aactggttgc actacccatg 2340
aggaattcgt ggaatggcta ctgctgacta acatgatgca cataaccaaa tgggggccaa 2400
tggcacagta ccttactcat catttaaaaa ctatatttac agaagatgtt tggttgctgg 2460
ggggcttttt taggttttgg gcatttgttt tttgtaaata agatgattat gctttgtggc 2520
tatccatcaa cataagt 2537

<210> 23
<211> 695
<212> PRT
<213> Homo sapiens

<400> 23
Met Gly Leu Pro Glu Pro Gly Pro Leu Arg Leu Leu Ala Leu Leu Leu
1               5                   10                  15

Leu Leu Leu Leu Leu Leu Leu Leu Arg Leu Gln His Leu Ala Ala Ala
            20                  25                  30

Ala Ala Asp Pro Leu Leu Gly Gly Gln Gly Pro Ala Lys Asp Cys Glu
        35                  40                  45

Lys Asp Gln Phe Gln Cys Arg Asn Glu Arg Cys Ile Pro Ser Val Trp
    50                  55                  60

Arg Cys Asp Glu Asp Asp Asp Cys Leu Asp His Ser Asp Glu Asp Asp
65                  70                  75                  80

Cys Pro Lys Lys Thr Cys Ala Asp Ser Asp Phe Thr Cys Asp Asn Gly
                85                  90                  95

His Cys Ile His Glu Arg Trp Lys Cys Asp Gly Glu Glu Glu Cys Pro
            100                 105                 110

Asp Gly Ser Asp Glu Ser Glu Ala Thr Cys Thr Lys Gln Val Cys Pro
        115                 120                 125

Ala Glu Lys Leu Ser Cys Gly Pro Thr Ser His Lys Cys Val Pro Ala
    130                 135                 140

Ser Trp Arg Cys Asp Gly Glu Lys Asp Cys Glu Gly Gly Ala Asp Glu
145                 150                 155                 160

Ala Gly Cys Ala Thr Ser Leu Gly Thr Cys Arg Gly Asp Glu Phe Gln
                165                 170                 175

Cys Gly Asp Gly Thr Cys Val Leu Ala Ile Lys His Cys Asn Gln Glu
            180                 185                 190

Gln Asp Cys Pro Asp Gly Ser Asp Glu Ala Gly Cys Leu Gln Gly Leu
        195                 200                 205

Asn Glu Cys Leu His Asn Asn Gly Gly Cys Ser His Ile Cys Thr Asp
    210                 215                 220

Leu Lys Ile Gly Phe Glu Cys Thr Cys Pro Ala Gly Phe Gln Leu Leu
225                 230                 235                 240

Asp Gln Lys Thr Cys Gly Asp Ile Asp Glu Cys Lys Asp Pro Asp Ala
                245                 250                 255

Cys Ser Gln Ile Cys Val Asn Tyr Lys Gly Tyr Phe Lys Cys Glu Cys
            260                 265                 270

Tyr Pro Gly Tyr Glu Met Asp Leu Leu Thr Lys Asn Cys Lys Ala Ala
        275                 280                 285

Ala Gly Lys Ser Pro Ser Leu Ile Phe Thr Asn Arg His Glu Val Arg
    290                 295                 300

Arg Ile Asp Leu Val Lys Arg Asn Tyr Ser Arg Leu Ile Pro Met Leu
305                 310                 315                 320

Lys Asn Val Val Ala Leu Asp Val Glu Val Ala Thr Asn Arg Ile Tyr
                325                 330                 335

Trp Cys Asp Leu Ser Tyr Arg Lys Ile Tyr Ser Ala Tyr Met Asp Lys
            340                 345                 350

Ala Ser Asp Pro Lys Glu Gln Glu Val Leu Ile Asp Glu Gln Leu His
        355                 360                 365

Ser Pro Glu Gly Leu Ala Val Asp Trp Val His Lys His Ile Tyr Trp
    370                 375                 380

Thr Asp Ser Gly Asn Lys Asn Ile Ser Gly Gly Arg Arg Arg Thr Leu
385                 390                 395                 400

Phe Ser Arg Asn Leu Ser Glu Pro Arg Ala Ile Ala Val Asp Pro Leu
                405                 410                 415

Arg Gly Phe Met Tyr Trp Ser Asp Trp Gly Asp Gln Ala Lys Ile Glu
            420                 425                 430

Lys Ser Gly Leu Asn Gly Val Asp Arg Gln Thr Leu Val Ser Asp Asn
        435                 440                 445

Ile Glu Trp Pro Asn Gly Ile Thr Leu Asp Leu Leu Ser Gln Arg Leu
    450                 455                 460

Tyr Trp Val Asp Ser Lys Leu His Gln Leu Ser Ser Ile Asp Phe Ser
465                 470                 475                 480

Gly Gly Asn Arg Lys Thr Leu Ile Ser Ser Thr Asp Phe Leu Ser His
                485                 490                 495

Pro Phe Gly Ile Ala Val Phe Glu Asp Lys Val Phe Trp Thr Asp Leu
            500                 505                 510

Glu Asn Glu Ala Ile Phe Ser Ala Asn Arg Leu Asn Gly Leu Glu Ile
        515                 520                 525

Ser Ile Leu Ala Glu Asn Leu Asn Asn Pro His Asp Ile Val Ile Phe
    530                 535                 540

His Glu Leu Lys Gln Pro Arg Ala Pro Asp Ala Cys Glu Leu Ser Val
545                 550                 555                 560

Gln Pro Asn Gly Gly Cys Glu Tyr Leu Cys Leu Pro Ala Pro Gln Ile
                565                 570                 575

Ser Ser His Ser Pro Lys Tyr Thr Cys Ala Cys Pro Asp Thr Met Trp
            580                 585                 590

Leu Gly Pro Asp Met Lys Arg Cys Tyr Arg Asp Ala Asn Glu Asp Ser
        595                 600                 605

Lys Met Gly Ser Thr Val Thr Ala Ala Val Ile Gly Ile Ile Val Pro
    610                 615                 620

Ile Val Val Ile Ala Leu Leu Cys Met Ser Gly Tyr Leu Ile Trp Arg
625                 630                 635                 640

Asn Trp Lys Arg Lys Asn Thr Lys Ser Met Asn Phe Asp Asn Pro Val
                645                 650                 655

Tyr Arg Lys Thr Thr Glu Glu Glu Asp Glu Asp Glu Leu His Ile Gly
            660                 665                 670

Arg Thr Ala Gln Ile Gly His Val Tyr Pro Ala Arg Val Ala Leu Ser
        675                 680                 685

Leu Glu Asp Asp Gly Leu Pro
    690                 695

<210> 24
<211> 2474
<212> DNA
<213> Homo sapiens

<400> 24
tgtttttttt cttcagtact ttaaatgtca tgccactctc ctggcctgta agctttccac 60
agagaagcct gctgccagac ccattggagc ttctttgtgt gttatttgtt tcttttttct 120
tgctgctttt acagctccag catcttgcgg cggcagcggc tgatccgctg ctcggcggcc 180
aagggccggc caaggattgc gaaaaggacc aattccagtg ccggaacgag cgctgcatcc 240
cctctgtgtg gagatgcgac gaggacgatg actgcttaga ccacagcgac gaggacgact 300
gccctgcagc caagaagaca cctgtgagaa gtgacttcac ctgtgacaac ggccactgca 360
tccacgaacg gtggaagtgt gacggcgagg aggagtgtcc tgatggctcc gatgagtccg 420
aggccacttg caccaagcag gtgtgtcctg cagagaagct gagctgtgga cccactagcc 480
acaagtgtgt acctgcctcg tggcgctgcg acggggagaa ggactgcgag ggtggagcgg 540
atgaggccgg ctgtgctacc tcactgggca cctgccgtgg ggacgagttc cagtgtgggg 600
atgggacatg tgtccttgca atcaagcact gcaaccagga gcaggactgt ccagatggga 660
gtgatgaagc tggctgccta caggggctga acgagtgtct gcacaacaat ggcggctgct 720
cacacatctg cactgacctc aagattggct ttgaatgcac gtgcccagca ggcttccagc 780
tcctggacca gaagacctgt ggcgctggca agagcccatc cctaatcttc accaaccggc 840
acgaggtgcg gaggatcgac ctggtgaagc ggaactattc acgcctcatc cccatgctca 900
agaatgtcgt ggcactagat gtggaagttg ccaccaatcg catctactgg tgtgacctct 960
cctaccgtaa gatctatagc gcctacatgg acaaggccag tgacccgaaa gagcaggagg 1020
tcctcattga cgagcagttg cactctccag agggcctggc agtggactgg gtccacaagc 1080
acatctactg gactgactcg ggcaataaga ccatctcagt ggccacagtt gatggtggcc 1140
gccgacgcac tctcttcagc cgtaacctca gtgaaccccg ggccatcgct gttgaccccc 1200
tgcgagggtt catgtattgg tctgactggg gggaccaggc caagattgag aaatctgggc 1260
tcaacggtgt ggaccggcaa acactggtgt cagacaatat tgaatggccc aacggaatca 1320
ccttggatct gctgagccag cgcttgtact gggtagactc caagctacac caactgtcca 1380
gcattgactt cagtggaggc aacagaaaga cgctgatctc ctccactgac ttcctgagcc 1440
acccttttgg gatagctgtg tttgaggaca aggtgttctg gacagacctg gagaacgagg 1500
ccattttcag tgcaaatcgg ctcaatggcc tggaaatctc catcctggct gagaacctga 1560
acaacccaca tgacattgtc atcttccatg agctgaagca gccaagagct ccagatgcct 1620
gtgagctgag tgtccagcct aatggaggct gtgaatacct gtgccttcct gctcctcaga 1680
tctccagcca ctctcccaag tacacatgtg cctgtcctga cacaatgtgg ctgggtccag 1740
acatgaagag gtgctaccga gcacctcaat ctacctcaac tacgacgtta gcttctacca 1800
tgacgaggac agtacctgcc accacaagag cccccgggac caccgtccac agatccacct 1860

accagaacca cagcacagag acaccaagcc tgacagctgc agtcccaagc tcagttagtg 1920
tccccagggc tcccagcatc agcccgtcta ccctaagccc tgcaaccagc aaccactccc 1980
agcactatgc aaatgaagac agtaagatgg gctcaacagt cactgccgct gttatcggga 2040
tcatcgtgcc catagtggtg atagccctcc tgtgcatgag tggatacctg atctggagaa 2100
actggaagcg gaagaacacc aaaagcatga attttgacaa cccagtctac aggaaaacaa 2160
cagaagaaga agacgaagat gagctccata tagggagaac tgctcagatt ggccatgtct 2220
atcctgcagc aatcagcagc tttgatcgcc cactgtgggc agagccctgt cttggggaga 2280
ccagagaacc ggaagaccca gcccctgccc tcaaggagct ttttgtcttg ccgggggaac 2340
caaggtcaca gctgcaccaa ctcccgaaga accctctttc cgagctgcct gtcgtcaaat 2400
ccaagcgagt ggcattaagc cttgaagatg atggactacc ctgaggatgg gatcaccccc 2460
ttcgtgcctc atgg 2474

<210> 25
<211> 804
<212> PRT
<213> Homo sapiens

<400> 25
   Met Pro Leu Ser Trp Pro Val Ser Phe Pro Gln Arg Ser Leu Leu Pro
   1               5                   10                  15
   Asp Pro Leu Glu Leu Leu Cys Val Leu Phe Val Ser Phe Phe Leu Leu
               20                  25                  30
   Leu Leu Gln Leu Gln His Leu Ala Ala Ala Ala Ala Asp Pro Leu Leu
           35                  40                  45
   Gly Gly Gln Gly Pro Ala Lys Asp Cys Glu Lys Asp Gln Phe Gln Cys
       50                  55                  60
   Arg Asn Glu Arg Cys Ile Pro Ser Val Trp Arg Cys Asp Glu Asp Asp
   65                  70                  75                  80
   Asp Cys Leu Asp His Ser Asp Glu Asp Asp Cys Pro Ala Ala Lys Lys
                   85                  90                  95
   Thr Pro Val Arg Ser Asp Phe Thr Cys Asp Asn Gly His Cys Ile His
               100                 105                 110
   Glu Arg Trp Lys Cys Asp Gly Glu Glu Glu Cys Pro Asp Gly Ser Asp
           115                 120                 125
   Glu Ser Glu Ala Thr Cys Thr Lys Gln Val Cys Pro Ala Glu Lys Leu
       130                 135                 140
   Ser Cys Gly Pro Thr Ser His Lys Cys Val Pro Ala Ser Trp Arg Cys
   145                 150                 155                 160
   Asp Gly Glu Lys Asp Cys Glu Gly Gly Ala Asp Glu Ala Gly Cys Ala
                   165                 170                 175
   Thr Ser Leu Gly Thr Cys Arg Gly Asp Glu Phe Gln Cys Gly Asp Gly
               180                 185                 190
   Thr Cys Val Leu Ala Ile Lys His Cys Asn Gln Glu Gln Asp Cys Pro
           195                 200                 205
   Asp Gly Ser Asp Glu Ala Gly Cys Leu Gln Gly Leu Asn Glu Cys Leu
       210                 215                 220
   His Asn Asn Gly Gly Cys Ser His Ile Cys Thr Asp Leu Lys Ile Gly
   225                 230                 235                 240
   Phe Glu Cys Thr Cys Pro Ala Gly Phe Gln Leu Leu Asp Gln Lys Thr
                   245                 250                 255
   Cys Gly Ala Gly Lys Ser Pro Ser Leu Ile Phe Thr Asn Arg His Glu
               260                 265                 270
   Val Arg Arg Ile Asp Leu Val Lys Arg Asn Tyr Ser Arg Leu Ile Pro
           275                 280                 285
   Met Leu Lys Asn Val Val Ala Leu Asp Val Glu Val Ala Thr Asn Arg
       290                 295                 300
   Ile Tyr Trp Cys Asp Leu Ser Tyr Arg Lys Ile Tyr Ser Ala Tyr Met
   305                 310                 315                 320
   Asp Lys Ala Ser Asp Pro Lys Glu Gln Glu Val Leu Ile Asp Glu Gln
                   325                 330                 335
   Leu His Ser Pro Glu Gly Leu Ala Val Asp Trp Val His Lys His Ile
               340                 345                 350
   Tyr Trp Thr Asp Ser Gly Asn Lys Thr Ile Ser Val Ala Thr Val Asp
           355                 360                 365
   Gly Gly Arg Arg Arg Thr Leu Phe Ser Arg Asn Leu Ser Glu Pro Arg
       370                 375                 380
   Ala Ile Ala Val Asp Pro Leu Arg Gly Phe Met Tyr Trp Ser Asp Trp
   385                 390                 395                 400
   Gly Asp Gln Ala Lys Ile Glu Lys Ser Gly Leu Asn Gly Val Asp Arg
                   405                 410                 415
   Gln Thr Leu Val Ser Asp Asn Ile Glu Trp Pro Asn Gly Ile Thr Leu
               420                 425                 430
   Asp Leu Leu Ser Gln Arg Leu Tyr Trp Val Asp Ser Lys Leu His Gln
           435                 440                 445
   Leu Ser Ser Ile Asp Phe Ser Gly Gly Asn Arg Lys Thr Leu Ile Ser
       450                 455                 460
   Ser Thr Asp Phe Leu Ser His Pro Phe Gly Ile Ala Val Phe Glu Asp
   465                 470                 475                 480
   Lys Val Phe Trp Thr Asp Leu Glu Asn Glu Ala Ile Phe Ser Ala Asn
                   485                 490                 495
   Arg Leu Asn Gly Leu Glu Ile Ser Ile Leu Ala Glu Asn Leu Asn Asn
               500                 505                 510
   Pro His Asp Ile Val Ile Phe His Glu Leu Lys Gln Pro Arg Ala Pro
   515 520 525
   Asp Ala Cys Glu Leu Ser Val Gln Pro Asn Gly Gly Cys Glu Tyr Leu
   530 535 540
   Cys Leu Pro Ala Pro Gln Ile Ser Ser His Ser Pro Lys Tyr Thr Cys
   545 550 555 560
   Ala Cys Pro Asp Thr Met Trp Leu Gly Pro Asp Met Lys Arg Cys Tyr
   565 570 575
   Arg Ala Pro Gln Ser Thr Ser Thr Thr Thr Leu Ala Ser Thr Met Thr
   580 585 590
   Arg Thr Val Pro Ala Thr Thr Arg Ala Pro Gly Thr Thr Val His Arg
   595 600 605
   Ser Thr Tyr Gln Asn His Ser Thr Glu Thr Pro Ser Leu Thr Ala Ala
   610 615 620
   Val Pro Ser Ser Val Ser Val Pro Arg Ala Pro Ser Ile Ser Pro Ser
   625 630 635 640
   Thr Leu Ser Pro Ala Thr Ser Asn His Ser Gln His Tyr Ala Asn Glu
   645 650 655
   Asp Ser Lys Met Gly Ser Thr Val Thr Ala Ala Val Ile Gly Ile Ile
   660 665 670
   Val Pro Ile Val Val Ile Ala Leu Leu Cys Met Ser Gly Tyr Leu Ile
   675 680 685
   Trp Arg Asn Trp Lys Arg Lys Asn Thr Lys Ser Met Asn Phe Asp Asn
   690 695 700
   Pro Val Tyr Arg Lys Thr Thr Glu Glu Glu Asp Glu Asp Glu Leu His
   705 710 715 720
   Ile Gly Arg Thr Ala Gln Ile Gly His Val Tyr Pro Ala Ala Ile Ser
   725 730 735
   Ser Phe Asp Arg Pro Leu Trp Ala Glu Pro Cys Leu Gly Glu Thr Arg
   740 745 750
   Glu Pro Glu Asp Pro Ala Pro Ala Leu Lys Glu Leu Phe Val Leu Pro
   755 760 765
   Gly Glu Pro Arg Ser Gln Leu His Gln Leu Pro Lys Asn Pro Leu Ser
   770 775 780
   Glu Leu Pro Val Val Lys Ser Lys Arg Val Ala Leu Ser Leu Glu Asp
   785 790 795 800
   Asp Gly Leu Pro

<210> 26
<211> 4150
<212> DNA
<213> Homo sapiens

<400> 26
gcagcggcaa ccccggcgcc gcggcaagga ctcggagggc tgagacgcgg cggcggcggc 60
gcggggagcg cggggcgcgg cggccggagc cccgggcccg ccatgggcct ccccgagccg 120
ggccctctcc ggcttctggc gctgctgctg ctgctgctgc tgctgctgct gctgcggctc 180
cagcatcttg cggcggcagc ggctgatccg ctgctcggcg gccaagggcc ggccaaggag 240
tgcgaaaagg accaattcca gtgccggaac gagcgctgca tcccctctgt gtggagatgc 300
gacgaggacg atgactgctt agaccacagc gacgaggacg actgccccaa gaagacctgt 360
gcagacagtg acttcacctg tgacaacggc cactgcatcc acgaacggtg gaagtgtgac 420
ggcgaggagg agtgtcctga tggctccgat gagtccgagg ccacttgcac actgggcacc 480
tgccgtgggg acgagttcca gtgtggggat gggacatgtg tccttgcaat caagcactgc 540
aaccaggagc aggactgtcc agatgggagt gatgaagctg gctgcctaca ggagtcaccc 600
tgtgagggtc cccgcagatt tcagtgtaag agtggcaagc gcgtggacgg cgggaaagtg 660
tgtgatgtgc agagggactg ccgggactgg tcggatgagc ttctgaaagt gtggtgcggt 720
gcctgtctac gcccactggc tggactcagt ctcctaccat ccccctcctg gagcccatgt 780
ccctccccag ggctgaacga gtgtctgcac aacaatggcg gctgctcaca catctgcact 840
gacctcaaga ttggctttga atgcacgtgc ccagcaggct tccagctcct ggaccagaag 900
acctgtggcg acattgatga gtgcaaggac ccagatgcct gcagccagat ctgtgtcaat 960
tacaagggct attttaagtg tgagtgctac cctggctacg agatggacct actgaccaag 1020
aactgcaagg ctgctgctgg caagagccca tccctaatct tcaccaaccg gcacgaggtg 1080
cggaggatcg acctggtgaa gcggaactat tcacgcctca tccccatgct caagaatgtc 1140
gtggcactag atgtggaagt tgccaccaat cgcatctact ggtgtgacct ctcctaccgt 1200
aagatctata gcgcctacat ggacaaggcc agtgacccga aagagcagga ggtcctcatt 1260
gacgagcagt tgcactctcc agagggcctg gcagtggact gggtccacaa gcacatctac 1320
tggactgact cgggcaataa gaccatctca gtggccacag ttgatggtgg ccgccgacgc 1380
actctcttca gccgtaacct cagtgaaccc cgggccatcg ctgttgaccc cctgcgaggg 1440
ttcatgtatt ggtctgactg gggggaccag gccaagattg agaaatctgg gctcaacggt 1500
gtggaccggc aaacactggt gtcagacaat attgaatggc ccaacggaat caccctggat 1560
ctgctgagcc agcgcttgta ctgggtagac tccaagctac accaactgtc cagcattgac 1620
ttcagtggag gcaacagaaa gacgctgatc tcctccactg acttcctgag ccaccctttt 1680
gggatagctg tgtttgagga caaggtgttc tggacagacc tggagaacga ggccattttc 1740
agtgcaaatc ggctcaatgg cctggaaatc tccatcctgg ctgagaacct caacaaccca 1800
catgacattg tcatcttcca tgagctgaag cagccaagag ctccagatgc ctgtgagctg 1860
agtgtccagc ctaatggagg ctgtgaatac ctgtgccttc ctgctcctca gatctccagc 1920
cactctccca agtacacatg tgcctgtcct gacacaatgt ggctgggtcc agacatgaag 1980
aggtgctacc gagcacctca atctacctca actacgacgt tagcttctac catgacgagg 2040
acagtacctg ccaccacaag agcccccggg accaccgtcc acagatccac ctaccagaac 2100
cacagcacag agacaccaag cctgacagct gcagtcccaa gctcagttag tgtccccagg 2160
gctcccagca tcagcccgtc taccctaagc cctgcaacca gcaaccactc ccagcactat 2220
gcaaatgaag acagtaagat gggctcaaca gtcactgccg ctgttatcgg gatcatcgtg 2280
cccatagtgg tgatagccct cctgtgcatg agtggatacc tgatctggag aaactggaag 2340
cggaagaaca ccaaaagcat gaattttgac aacccagtct acaggaaaac aacagaagaa 2400
gaagatgaag atgagctcca tatagggaga actgctcaga ttggccatgt ctatcctgca 2460
gcaatcagca gctttgatcg cccactgtgg gcagagccct gtcttgggga gaccagagaa 2520
ccggaagacc cagcccctgc cctcaaggag ctttttgtct tgccggggga accaaggtca 2580
cagctgcacc aactcccgaa gaaccctctt tccgagctgc ctgtcgtcaa atccaagcga 2640
gtggcattaa gccttgaaga tgatggacta ccctgaggat gggatcaccc ccttcgtgcc 2700
tcatggaatt cagtcccatg cactacactc tggatggtgt atgactggat gaatgggttt 2760
ctatatatgg gtctgtgtga gtgtatgtgt gtgtgtgatt ttttttttaa atttatgttg 2820
cggaaaggta accacaaagt tatgatgaac tgcaaacatc caaaggatgt gagagttttt 2880
ctatgtataa tgttttatac actttttaac tggttgcact acccatgagg aattcgtgga 2940

atggctactg ctgactaaca tgatgcacat aaccaaatgg gggccaatgg cacagtacct 3000
tactcatcat ttaaaaacta tatttacaga agatgtttgg ttgctggggg ggcttttttg 3060
ggttttgggg catttgtttt ttgtaaataa gatgattatg ctttgtggct atccatcaac 3120
ataagtaaaa aaaaaaaaaa aacacttcaa ctccctcccc catttagatt atttattaac 3180
atattttaaa aatcagatga gttctataaa taatttagag aagtgagagt atttattttt 3240
ggcatgtttg gcccaccaca cagactctgt gtgtgtatgt gtgtgtttat atgtgtatgt 3300
gtgtgacagg aaaatctgta gagaagaggc acatctatgg ctactgttca aatacataaa 3360
gataaattta ttttcacaca gtccacaagg ggtatatctt gtagttttca gaaaagcctt 3420
tggaaatctg gatcaggaaa tagataccat ggtttgtgca attatgtagt aaaaaaggca 3480
aatcttttca cctctggcta ttcctgagac cccaggaagt caggaaaagc ctttcagctc 3540
acccatggct gctgtgactc ctaccagggc tttcttggct ttggcgaagg tcagtgtaca 3600
gacattccat ggtaccagag tgctcagaaa gtcaagatag gatatgcctc accctcagct 3660
actccttgtt ttaaagttca gctctttgag taacttcttc aatttctttc aggacacttg 3720
ggttgaattc agtaagtttc ctctgaagca ccctgaaggg tgccatcctt acagagctaa 3780
gtggagacgt ttccagatca gcccaagttt actatagaga ctggcccagg cactgaatgt 3840
ctaggacatg ctgtggatga agataaagat ggtggaatag gttttatcac atctcttatt 3900
tctcttttcc ccttactctc taccatttcc tttatgtggg gaaacatttt aaggtaataa 3960
ataggttact taccatcata tgttcatata gatgaaacta atttttggct taagtcagaa 4020
caactggccc ccaattgaag tcatatttgt ggggggaaat ggcatacgca atattatatt 4080
atattggata tttatgttca cacaggaatt tggtttactg ctttgtaaat aaaagggaaa 4140
actccgggta 4150

<210> 27
<211> 857
<212> PRT
<213> Homo sapiens

<400> 27
Met Gly Leu Pro Glu Pro Gly Pro Leu Arg Leu Leu Ala Leu Leu Leu
1               5                   10                  15

Leu Leu Leu Leu Leu Leu Leu Leu Arg Leu Gln His Leu Ala Ala Ala
            20                  25                  30

Ala Ala Asp Pro Leu Leu Gly Gly Gln Gly Pro Ala Lys Glu Cys Glu
        35                  40                  45

Lys Asp Gln Phe Gln Cys Arg Asn Glu Arg Cys Ile Pro Ser Val Trp
    50                  55                  60

Arg Cys Asp Glu Asp Asp Asp Cys Leu Asp His Ser Asp Glu Asp Asp
65                  70                  75                  80

Cys Pro Lys Lys Thr Cys Ala Asp Ser Asp Phe Thr Cys Asp Asn Gly
                85                  90                  95

His Cys Ile His Glu Arg Trp Lys Cys Asp Gly Glu Glu Glu Cys Pro
            100                 105                 110

Asp Gly Ser Asp Glu Ser Glu Ala Thr Cys Thr Leu Gly Thr Cys Arg
        115                 120                 125

Gly Asp Glu Phe Gln Cys Gly Asp Gly Thr Cys Val Leu Ala Ile Lys
    130                 135                 140

His Cys Asn Gln Glu Gln Asp Cys Pro Asp Gly Ser Asp Glu Ala Gly

145                 150                 155                 160

Cys Leu Gln Glu Ser Pro Cys Glu Gly Pro Arg Arg Phe Gln Cys Lys
                165                 170                 175

Ser Gly Lys Arg Val Asp Gly Gly Lys Val Cys Asp Val Gln Arg Asp
            180                 185                 190

Cys Arg Asp Trp Ser Asp Glu Leu Leu Lys Val Trp Cys Gly Ala Cys
        195                 200                 205

Leu Arg Pro Leu Ala Gly Leu Ser Leu Leu Pro Ser Pro Ser Trp Ser
    210                 215                 220

Pro Cys Pro Ser Pro Gly Leu Asn Glu Cys Leu His Asn Asn Gly Gly
225                 230                 235                 240

Cys Ser His Ile Cys Thr Asp Leu Lys Ile Gly Phe Glu Cys Thr Cys
                245                 250                 255

Pro Ala Gly Phe Gln Leu Leu Asp Gln Lys Thr Cys Gly Asp Ile Asp
            260                 265                 270

Glu Cys Lys Asp Pro Asp Ala Cys Ser Gln Ile Cys Val Asn Tyr Lys
        275                 280                 285

Gly Tyr Phe Lys Cys Glu Cys Tyr Pro Gly Tyr Glu Met Asp Leu Leu
    290                 295                 300

Thr Lys Asn Cys Lys Ala Ala Ala Gly Lys Ser Pro Ser Leu Ile Phe
305                 310                 315                 320

Thr Asn Arg His Glu Val Arg Arg Ile Asp Leu Val Lys Arg Asn Tyr
                325                 330                 335

Ser Arg Leu Ile Pro Met Leu Lys Asn Val Val Ala Leu Asp Val Glu
            340                 345                 350

Val Ala Thr Asn Arg Ile Tyr Trp Cys Asp Leu Ser Tyr Arg Lys Ile
        355                 360                 365

Tyr Ser Ala Tyr Met Asp Lys Ala Ser Asp Pro Lys Glu Gln Glu Val
    370                 375                 380

Leu Ile Asp Glu Gln Leu His Ser Pro Glu Gly Leu Ala Val Asp Trp
385                 390                 395                 400

Val His Lys His Ile Tyr Trp Thr Asp Ser Gly Asn Lys Thr Ile Ser
                405                 410                 415

Val Ala Thr Val Asp Gly Gly Arg Arg Arg Thr Leu Phe Ser Arg Asn
            420                 425                 430

Leu Ser Glu Pro Arg Ala Ile Ala Val Asp Pro Leu Arg Gly Phe Met
        435                 440                 445

Tyr Trp Ser Asp Trp Gly Asp Gln Ala Lys Ile Glu Lys Ser Gly Leu

450                 455                 460

Asn Gly Val Asp Arg Gln Thr Leu Val Ser Asp Asn Ile Glu Trp Pro
465                 470                 475                 480

Asn Gly Ile Thr Leu Asp Leu Leu Ser Gln Arg Leu Tyr Trp Val Asp
                485                 490                 495

Ser Lys Leu His Gln Leu Ser Ser Ile Asp Phe Ser Gly Gly Asn Arg
            500                 505                 510

Lys Thr Leu Ile Ser Ser Thr Asp Phe Leu Ser His Pro Phe Gly Ile
        515                 520                 525

Ala Val Phe Glu Asp Lys Val Phe Trp Thr Asp Leu Glu Asn Glu Ala
    530                 535                 540

Ile Phe Ser Ala Asn Arg Leu Asn Gly Leu Glu Ile Ser Ile Leu Ala
545                 550                 555                 560

Glu Asn Leu Asn Asn Pro His Asp Ile Val Ile Phe His Glu Leu Lys
                565                 570                 575

Gln Pro Arg Ala Pro Asp Ala Cys Glu Leu Ser Val Gln Pro Asn Gly
            580                 585                 590

Gly Cys Glu Tyr Leu Cys Leu Pro Ala Pro Gln Ile Ser Ser His Ser
        595                 600                 605

Pro Lys Tyr Thr Cys Ala Cys Pro Asp Thr Met Trp Leu Gly Pro Asp
    610                 615                 620

Met Lys Arg Cys Tyr Arg Ala Pro Gln Ser Thr Ser Thr Thr Thr Leu
625                 630                 635                 640

Ala Ser Thr Met Thr Arg Thr Val Pro Ala Thr Thr Arg Ala Pro Gly
                645                 650                 655

Thr Thr Val His Arg Ser Thr Tyr Gln Asn His Ser Thr Glu Thr Pro
            660                 665                 670

Ser Leu Thr Ala Ala Val Pro Ser Ser Val Ser Val Pro Arg Ala Pro
        675                 680                 685

Ser Ile Ser Pro Ser Thr Leu Ser Pro Ala Thr Ser Asn His Ser Gln
    690                 695                 700

His Tyr Ala Asn Glu Asp Ser Lys Met Gly Ser Thr Val Thr Ala Ala
705                 710                 715                 720

Val Ile Gly Ile Ile Val Pro Ile Val Val Ile Ala Leu Leu Cys Met
                725                 730                 735

Ser Gly Tyr Leu Ile Trp Arg Asn Trp Lys Arg Lys Asn Thr Lys Ser
            740                 745                 750

Met Asn Phe Asp Asn Pro Val Tyr Arg Lys Thr Thr Glu Glu Glu Asp

755                 760                 765

Glu Asp Glu Leu His Ile Gly Arg Thr Ala Gln Ile Gly His Val Tyr
    770                 775                 780

Pro Ala Ala Ile Ser Ser Phe Asp Arg Pro Leu Trp Ala Glu Pro Cys
785                 790                 795                 800

Leu Gly Glu Thr Arg Glu Pro Glu Asp Pro Ala Pro Ala Leu Lys Glu
                805                 810                 815

Leu Phe Val Leu Pro Gly Glu Pro Arg Ser Gln Leu His Gln Leu Pro
            820                 825                 830

Lys Asn Pro Leu Ser Glu Leu Pro Val Val Lys Ser Lys Arg Val Ala
        835                 840                 845

Leu Ser Leu Glu Asp Asp Gly Leu Pro
    850                 855


<210> 28
<211> 4180
<212> DNA
<213> Homo sapiens

<400> 28
atggcctccc cgaacgtatg ggcctccccg agcgtatggg cctccccgag cgtatgggcc 60
tccccgagcg tatgggcctc acccgagcgt atgggcctcc ccgagcgtat gggcctcccc 120
gagcgtatgg gcctccccga gcgtatgggc ctccccgagc gtatgggcct ccccgagccg 180
ggccctctcc ggcttctggc gctgctgctg ctgctgctgc tgctgctgct gctgcagctc 240
cagcatcttg cggcggcagc ggctgatccg ctgctcggcg gccaagggcc ggccaaggat 300
tgcgaaaagg accaattcca gtgccggaac gagcgctgca tcccctctgt gtggagatgc 360
gacgaggacg atgactgctt agaccacagc gacgaggacg actgccccaa gaagacctgt 420
gcagacagtg acttcacctg tgacaacggc cactgcatcc acgaacggtg gaagtgtgac 480
ggcgaggagg agtgtcctga tggctccgat gagtccgagg ccacttgcac caagcaggtg 540
tgtcctgcag agaagctgag ctgtggaccc accagccaca agtgtgtacc tgcctcgtgg 600
cgctgcgacg gggagaagga ctgcgagggt ggagcggatg aggccggctg tgctacctca 660
ctgggcacct gccgtgggga cgagttccag tgtggggatg ggacatgtgt ccttgcaatc 720
aagcactgca accaggagca ggactgtcca gatgggagtg atgaagctgg ctgcctacag 780
gttccgccaa cattcctggg aaacaggagg aggcccaggg ggctgaacga gtgtctgcac 840
aacaatggcg gctgctcaca catctgcact gacctcaaga ttggctttga atgcacgtgc 900
ccagcaggct tccagctcct ggaccagaag acctgtggcg acattgatga gtgcaaggac 960
ccagatgcct gcagccagat ctgtgtcaat tacaagggct attttaagtg tgagtgctac 1020
cctggctacg agatggacct actgaccaag aactgcaagg ctgctgctgg aaagagccca 1080
tccctaatct tcaccaaccg gcacgaggtg cggaggatcg acctggtgaa gcggaactat 1140
tcacgcctca tccccatgct caagaatgtc gtggcactag atgtggaagt tgccaccaat 1200
cgcatctact ggtgtgacct ctcctaccgt aagatctata gcgcctacat ggacaaggcc 1260
agtgacccga aagagcagga ggtcctcatt gacgagcagt tgcactctcc agagggcctg 1320
gcagtggact gggtccacaa gcacatctac tggactgact cgggcaataa gaccatctca 1380
gtggccacag ttgatggtgg ccgccgacgc actctcttca gccgtaacct cagtgaaccc 1440
cgggccatcg ctgttgaccc cctgcgaggg ttcatgtatt ggtctgactg gggggaccag 1500
gccaagattg agaaatctgg gctcaacggt gtggaccggc aaacactggt gtcagacaat 1560
attgaatggc ccaacggaat caccctggat ctgctgagcc agcgcttgta ctgggtagac 1620
tccaagctac accaactgtc cagcattgac ttcagtggag gcaacagaaa gacgctgatc 1680
tcctccactg acttcctgag ccaccctttt gggatagctg tgtttgagga caaggtgttc 1740
tggacagacc tggagaacga ggccattttc agtgcaaatc ggctcaatgg cctggaaatc 1800

tccatcctgg ctgagaacct caacaaccca catgacattg tcatcttcca tgagctgaag 1860
cagccaagag ctccagatgc ctgtgagctg agtgtccagc ctaatggagg ctgtgaatac 1920
ctgtgccttc ctgctcctca gatctccagc cactctccca agtacacatg tgcctgtcct 1980
gacacaatgt ggctgggtcc agacatgaag aggtgctacc gagcacctca atctacctca 2040
actacgacgt tagcttctac catgacgagg acagtacctg ccaccacaag agcccccggg 2100
accaccgtcc acagatccac ctaccagaac cacagcacag agacaccaag cctgacagct 2160
gcagtcccaa gctcagttag tgtccccagg gctcccagca tcagcccgtc taccctaagc 2220
cctgcaacca gcaaccactc ccagcactat gcaaatgaag acagtaagat gggctcaaca 2280
gtcactgccg ctgttatcgg gatcatcgtg cccatagtgg tgatagccct cctgtgcatg 2340
agtggatacc tgatctggag aaactggaag cggaagaaca ccaaaagcat gaattttgac 2400
aacccagtct acaggaaaac aacagaagaa gaagatgaag atgagctcca tatagggaga 2460
actgctcaga ttggccatgt ctatcctgca gcaatcagca gctttgatcg cccactgtgg 2520
gcagagccct gtcttgggga gaccagagaa ccggaagacc cagcccctgc cctcaaggag 2580
ctttttgtct tgccggggga accaaggtca cagctgcacc aactcccgaa gaaccctctt 2640
tccgagctgc ctgtcgtcaa atccaagcga gtggcattaa gccttgaaga tgatggacta 2700
ccctgaggat gggatcaccc ccttcgtgcc tcatggaatt cagtcccatg cactacactc 2760
tggatggtgt atgactggat gaatgggttt ctatatatgg gtctgtgtga gtgtatgtgt 2820
gtgtgtgatt ttttttttaa atttatgttg cggaaaggta accacaaagt tatgatgaac 2880
tgcaaacatc caaaggatgt gagagttttt ctatgtataa tgttttatac actttttaac 2940
tggttgcact acccatgagg aattcgtgga atggctactg ctgactaaca tgatgcacat 3000
aaccaaatgg gggccaatgg cacagtacct tactcatcat ttaaaaacta tatttacaga 3060
agatgtttgg ttgctggggg ggcttttttg ggttttgggg catttgtttt ttgtaaataa 3120
gatgattatg ctttgtggct atccatcaac ataagtaaaa aaaaaaaaaa aacacttcaa 3180
ctccctcccc catttagatt atttattaac atattttaaa aatcagatga gttctataaa 3240
taatttagag aagtgagagt atttattttt ggcatgtttg gcccaccaca cagactctgt 3300
gtgtgtatgt gtgtgtttat atgtgtatgt gtgtgacagg aaaatctgta gagaagaggc 3360
acatctatgg ctactgttca aatacataaa gataaattta ttttcacaca gtccacaagg 3420
ggtatatctt gtagttttca gaaaagcctt tggaaatctg gatcaggaaa tagataccat 3480
ggtttgtgca attatgtagt aaaaaaggca aatcttttca cctctggcta ttcctgagac 3540
cccaggaagt caggaaaagc ctttcagctc acccatggct gctgtgactc ctaccagggc 3600
tttcttggct ttggcgaagg tcagtgtaca gacattccat ggtaccagag tgctcagaaa 3660
gtcaagatag gatatgcctc accctcagct actccttgtt ttaaagttca gctctttgag 3720
taacttcttc aatttctttc aggacacttg ggttgaattc agtaagtttc ctctgaagca 3780
ccctgaaggg tgccatcctt acagagctaa gtggagacgt ttccagatca gcccaagttt 3840
actatagaga ctggcccagg cactgaatgt ctaggacatg ctgtggatga agataaagat 3900
ggtggaatag gttttatcac atctcttatt tctcttttcc ccttactctc taccatttcc 3960
tttatgtggg gaaacatttt aaggtaataa ataggttact taccatcata tgttcatata 4020
gatgaaacta atttttggct taagtcagaa caactggccc ccaattgaag tcatatttgt 4080
ggggggaaat ggcatacgca atattatatt atattggata tttatgttca cacaggaatt 4140
tggtttactg ctttgtaaat aaaagggaaa actccgggta 4180

<210> 29
<211> 847
<212> PRT
<213> Homo sapiens

<400> 29
Met Gly Leu Pro Glu Pro Gly Pro Leu Arg Leu Leu Ala Leu Leu Leu
1               5                   10                  15

Leu Leu Leu Leu Leu Leu Leu Leu Gln Leu Gln His Leu Ala Ala Ala
            20                  25                  30

Ala Ala Asp Pro Leu Leu Gly Gly Gln Gly Pro Ala Lys Asp Cys Glu
        35                  40                  45

Lys Asp Gln Phe Gln Cys Arg Asn Glu Arg Cys Ile Pro Ser Val Trp
    50                  55                  60

Arg Cys Asp Glu Asp Asp Asp Cys Leu Asp His Ser Asp Glu Asp Asp
65                  70                  75                  80

Cys Pro Lys Lys Thr Cys Ala Asp Ser Asp Phe Thr Cys Asp Asn Gly
                85                  90                  95

His Cys Ile His Glu Arg Trp Lys Cys Asp Gly Glu Glu Glu Cys Pro
            100                 105                 110

Asp Gly Ser Asp Glu Ser Glu Ala Thr Cys Thr Lys Gln Val Cys Pro
        115                 120                 125

Ala Glu Lys Leu Ser Cys Gly Pro Thr Ser His Lys Cys Val Pro Ala
    130                 135                 140

Ser Trp Arg Cys Asp Gly Glu Lys Asp Cys Glu Gly Gly Ala Asp Glu
145                 150                 155                 160

Ala Gly Cys Ala Thr Ser Leu Gly Thr Cys Arg Gly Asp Glu Phe Gln
                165                 170                 175

Cys Gly Asp Gly Thr Cys Val Leu Ala Ile Lys His Cys Asn Gln Glu
            180                 185                 190

Gln Asp Cys Pro Asp Gly Ser Asp Glu Ala Gly Cys Leu Gln Val Pro
        195                 200                 205

Pro Thr Phe Leu Gly Asn Arg Arg Arg Pro Arg Gly Leu Asn Glu Cys
    210                 215                 220

Leu His Asn Asn Gly Gly Cys Ser His Ile Cys Thr Asp Leu Lys Ile
225                 230                 235                 240

Gly Phe Glu Cys Thr Cys Pro Ala Gly Phe Gln Leu Leu Asp Gln Lys
                245                 250                 255

Thr Cys Gly Asp Ile Asp Glu Cys Lys Asp Pro Asp Ala Cys Ser Gln
            260                 265                 270

Ile Cys Val Asn Tyr Lys Gly Tyr Phe Lys Cys Glu Cys Tyr Pro Gly
        275                 280                 285

Tyr Glu Met Asp Leu Leu Thr Lys Asn Cys Lys Ala Ala Ala Gly Lys
    290                 295                 300

Ser Pro Ser Leu Ile Phe Thr Asn Arg His Glu Val Arg Arg Ile Asp
305                 310                 315                 320

Leu Val Lys Arg Asn Tyr Ser Arg Leu Ile Pro Met Leu Lys Asn Val
                325                 330                 335

Val Ala Leu Asp Val Glu Val Ala Thr Asn Arg Ile Tyr Trp Cys Asp
            340                 345                 350

Leu Ser Tyr Arg Lys Ile Tyr Ser Ala Tyr Met Asp Lys Ala Ser Asp
        355                 360                 365

Pro Lys Glu Gln Glu Val Leu Ile Asp Glu Gln Leu His Ser Pro Glu
    370                 375                 380

Gly Leu Ala Val Asp Trp Val His Lys His Ile Tyr Trp Thr Asp Ser
385                 390                 395                 400

Gly Asn Lys Thr Ile Ser Val Ala Thr Val Asp Gly Gly Arg Arg Arg
                405                 410                 415

Thr Leu Phe Ser Arg Asn Leu Ser Glu Pro Arg Ala Ile Ala Val Asp
            420                 425                 430

Pro Leu Arg Gly Phe Met Tyr Trp Ser Asp Trp Gly Asp Gln Ala Lys
        435                 440                 445

Ile Glu Lys Ser Gly Leu Asn Gly Val Asp Arg Gln Thr Leu Val Ser
    450                 455                 460

Asp Asn Ile Glu Trp Pro Asn Gly Ile Thr Leu Asp Leu Leu Ser Gln
465                 470                 475                 480

Arg Leu Tyr Trp Val Asp Ser Lys Leu His Gln Leu Ser Ser Ile Asp
                485                 490                 495

Phe Ser Gly Gly Asn Arg Lys Thr Leu Ile Ser Ser Thr Asp Phe Leu
            500                 505                 510

Ser His Pro Phe Gly Ile Ala Val Phe Glu Asp Lys Val Phe Trp Thr
        515                 520                 525

Asp Leu Glu Asn Glu Ala Ile Phe Ser Ala Asn Arg Leu Asn Gly Leu
    530                 535                 540

Glu Ile Ser Ile Leu Ala Glu Asn Leu Asn Asn Pro His Asp Ile Val
545                 550                 555                 560

Ile Phe His Glu Leu Lys Gln Pro Arg Ala Pro Asp Ala Cys Glu Leu
                565                 570                 575

Ser Val Gln Pro Asn Gly Gly Cys Glu Tyr Leu Cys Leu Pro Ala Pro
            580                 585                 590

Gln Ile Ser Ser His Ser Pro Lys Tyr Thr Cys Ala Cys Pro Asp Thr
        595                 600                 605

Met Trp Leu Gly Pro Asp Met Lys Arg Cys Tyr Arg Ala Pro Gln Ser
    610                 615                 620

Thr Ser Thr Thr Thr Leu Ala Ser Thr Met Thr Arg Thr Val Pro Ala
625                 630                 635                 640

Thr Thr Arg Ala Pro Gly Thr Thr Val His Arg Ser Thr Tyr Gln Asn
                645                 650                 655

His Ser Thr Glu Thr Pro Ser Leu Thr Ala Ala Val Pro Ser Ser Val
        660                 665                 670

Ser Val Pro Arg Ala Pro Ser Ile Ser Pro Ser Thr Leu Ser Pro Ala
    675                 680                 685

Thr Ser Asn His Ser Gln His Tyr Ala Asn Glu Asp Ser Lys Met Gly
    690                 695                 700

Ser Thr Val Thr Ala Ala Val Ile Gly Ile Ile Val Pro Ile Val Val
705                 710                 715                 720

Ile Ala Leu Leu Cys Met Ser Gly Tyr Leu Ile Trp Arg Asn Trp Lys
                725                 730                 735

Arg Lys Asn Thr Lys Ser Met Asn Phe Asp Asn Pro Val Tyr Arg Lys
        740                 745                 750

Thr Thr Glu Glu Glu Asp Glu Asp Glu Leu His Ile Gly Arg Thr Ala
    755                 760                 765

Gln Ile Gly His Val Tyr Pro Ala Ala Ile Ser Ser Phe Asp Arg Pro
    770                 775                 780

Leu Trp Ala Glu Pro Cys Leu Gly Glu Thr Arg Glu Pro Glu Asp Pro
785                 790                 795                 800

Ala Pro Ala Leu Lys Glu Leu Phe Val Leu Pro Gly Glu Pro Arg Ser
                805                 810                 815

Gln Leu His Gln Leu Pro Lys Asn Pro Leu Ser Glu Leu Pro Val Val
        820                 825                 830

Lys Ser Lys Arg Val Ala Leu Ser Leu Glu Asp Asp Gly Leu Pro
    835                 840                 845

<210> 30
<211> 4294
<212> DNA
<213> Homo sapiens

<400> 30
gcagcggcaa ccccggcgcc gcggcaagga ctcggagggc tgagacgcgg cggcggcggc 60
gcggggagcg cggggcgcgg cggccggagc cccgggcccg ccatgggcct ccccgagccg 120
ggccctctcc ggcttctggc gctgctgctg ctgctgctgc tgctgctgct gctgcggctc 180
cagcatcttg cggcggcagc ggctgatccg ctgctcggcg gccaagggcc ggccaaggag 240
tgcgaaaagg accaattcca gtgccggaac gagcgctgca tcccctctgt gtggagatgc 300
gacgaggacg atgactgctt agaccacagc gacgaggacg actgccccaa gaagacctgt 360
gcagacagtg acttcacctg tgacaacggc cactgcatcc acgaacggtg gaagtgtgac 420
ggcgaggagg agtgtcctga tggctccgat gagtccgagg ccacttgcac actgggcacc 480
tgccgtgggg acgagttcca gtgtggggat gggacatgtg tccttgcaat caagcactgc 540
aaccaggagc aggactgtcc agatgggagt gatgaagctg gctgcctaca ggagtcaccc 600
tgtgagggtc cccgcagatt tcagtgtaag agtggcaagc gcgtggacgg cgggaaagtg 660
tgtgatgtgc agagggactg ccgggactgg tcggatgagc ttctgaaagt gtggtgcggt 720
gcctgtctac gcccactggc tggactcagt ctcctaccat ccccctcctg gtatctaggc 780
tcaaggccct ccagtgcccc ctgccctgac actttctgct ctgaccctct ctttggattc 840

atgtgccgtc ctatggcttc acatggggct tttcgccccc aggcctctgg tctacatctc 900
tacaaagtgc taagagcttg tccatcccag aggaggctga acgagtgtct gcacaacaat 960
ggcggctgct cacacatctg cactgacctc aagattggct ttgaatgcac gtgcccagca 1020
ggcttccagc tcctggacca gaagacctgt ggcgacattg atgagtgcaa ggacccagat 1080
gcctgcagcc agatctgtgt caattacaag ggctatttta agtgtgagtg ctaccctggc 1140
tacgagatgg acctactgac caagaactgc caagctgctg ctggaaagag cccatcccta 1200
atcttcacca accggtacga ggtgcggagg atcgacctgg tgaagcggaa ctattcacgc 1260
ctcatcccca tgctcaagaa tgtcgtggca ctagatgtgg aagttgccac caatcgcatc 1320
tactggtgtg acctctccta ccgtaagatc tatagcgcct acatggacaa ggccagtgac 1380
ccgaaagagc aggaggtcct cattgacgag cagttgcact ctccagaggg cctggcagtg 1440
gactgggtcc acaagcacat ctactggact gactcgggca ataagaccat ctcagtggcc 1500
acagttgatg gtggccgccg acgcactctc ttcagccgta acctcagtga accccgggcc 1560
atcgctgttg accccctgcg agggttcatg tattggtctg actgggggga ccaggccaag 1620
attgagaaat ctgggctcaa cggtgtggac cggcaaacac tggtgtcaga caatattgaa 1680
tggcccaacg gaatcaccct ggatctgctg agccagcgct tgtactgggt agactccaag 1740
ctacaccaac tgtccagcat tgacttcagt ggaggcaaca gaaagacgct gatctcctcc 1800
actgacttcc tgagccaccc ttttgggata gctgtgtttg aggacaaggt gttctggaca 1860
gacctggaga acgaggccat tttcagtgca aatcggctca atggcctgga aatctccatc 1920
ctggctgaga acctcaacaa cccacatgac attgtcatct tccatgagct gaagcagcca 1980
agagctccag atgcctgtga gctgagtgtc cagcctaatg gaggctgtga atacctgtgc 2040
cttcctgctc ctcagatctc cagccactct cccaagtaca catgtgcctg tcctgacaca 2100
atgtggctgg gtccagacat gaagaggtgc taccgagcac ctcaatctac ctcaactacg 2160
acgttagctt ctaccatgac gaggacagta cctgccacca caagagcccc cgggaccacc 2220
gtccacagat ccacctacca gaaccacagc acagagacac caagcctgac agctgcagtc 2280
ccaagctcag ttagtgtccc cagggctccc agcatcagcc cgtctaccct aagccctgca 2340
accagcaacc actcccagca ctatgcaaat gaagacagta agatgggctc aacagtcact 2400
gccgctgtta tcgggatcat cgtgcccata gtggtgatag ccctcctgtg catgagtgga 2460
tacctgatct ggagaaactg gaagcggaag aacaccaaaa gcatgaattt tgacaaccca 2520
gtctacagga aaacaacaga agaagaagat gaagatgagc tccatatagg gagaactgct 2580
cagattggcc atgtctatcc tgcagcaatc agcagctttg atcgcccact gtgggcagag 2640
ccctgtcttg gggagaccag agaaccggaa gacccagccc ctgccctcaa ggagcttttt 2700
gtcttgccgg gggaaccaag gtcacagctg caccaactcc cgaagaaccc tctttccgag 2760
ctgcctgtcg tcaaatccaa gcgagtggca ttaagccttg aagatgatgg actaccctga 2820
ggatgggatc acccccttcg tgcctcatgg aattcagtcc catgcactac actctggatg 2880
gtgtatgact ggatgaatgg gtttctatat atgggtctgt gtgagtgtat gtgtgtgtgt 2940
gatttttttt ttaaatttat gttgcggaaa ggtaaccaca aagttatgat gaactgcaaa 3000
catccaaagg atgtgagagt ttttctatgt ataatgtttt atacactttt taactggttg 3060
cactacccat gaggaattcg tggaatggct actgctgact aacatgatgc acataaccaa 3120
atgggggcca atggcacagt accttactca tcatttaaaa actatattta cagaagatgt 3180
ttggttgctg gggggctttt tttgggtttt ggggcatttg ttttttgtaa ataagatgat 3240
tatgctttgt ggctatccat caacataagt aaaaaaaaaa aaaaaacact tcaactccct 3300
cccccattta gattatttat taacatattt taaaaatcag atgagttcta taaataattt 3360
agagaagtga gagtatttat ttttggcatg tttggcccac cacacagact ctgtgtgtgt 3420
atgtgtgtgt ttatatgtgt atgtgtgtga caggaaaatc tgtagagaag aggcacatct 3480
atggctactg ttcaaataca taaagataaa tttattttca cacagtccac aaggggtata 3540
tcttgtagtt ttcagaaaag cctttggaaa tctggatcag gaaatagata ccatggtttg 3600
tgcaattatg tagtaaaaaa ggcaaatctt ttcacctctg gctattcctg agaccccagg 3660
aagtcaggaa aagcctttca gctcacccat ggctgctgtg actcctacca gggctttctt 3720
ggctttggcg aaggtcagtg tacagacatt ccatggtacc agagtgctca gaaagtcaag 3780
ataggatatg cctcaccctc agctactcct tgttttaaag ttcagctctt tgagtaactt 3840
cttcaatttc tttcaggaca cttgggttga attcagtaag tttcctctga agcaccctga 3900
agggtgccat ccttacagag ctaagtggag acgtttccag atcagcccaa gtttactata 3960
gagactggcc caggcactga atgtctagga catgctgtgg atgaagataa agatggtgga 4020
ataggtttta tcacatctct tatttctctt ttccccttac tctctaccat ttcctttatg 4080
tggggaaaca ttttaaggta ataaataggt tacttaccat catatgttca tatagatgaa 4140
actaattttt ggcttaagtc agaacaactg gcccccaatt gaagtcatat ttgtgggggg 4200
aaatggcata cgcaatatta tattatattg gatatttatg ttcacacagg aatttggttt 4260

actgctttgt aaataaaagg gaaaactccg ggta                                4294

<210> 31
<211> 905
<212> PRT
<213> Homo sapiens

<400> 31
Met Gly Leu Pro Glu Pro Gly Pro Leu Arg Leu Leu Ala Leu Leu Leu
1               5                   10                  15

Leu Leu Leu Leu Leu Leu Leu Leu Arg Leu Gln His Leu Ala Ala Ala
            20                  25                  30

Ala Ala Asp Pro Leu Leu Gly Gly Gln Gly Pro Ala Lys Glu Cys Glu
        35                  40                  45

Lys Asp Gln Phe Gln Cys Arg Asn Glu Arg Cys Ile Pro Ser Val Trp
    50                  55                  60

Arg Cys Asp Glu Asp Asp Asp Cys Leu Asp His Ser Asp Glu Asp Asp
65                  70                  75                  80

Cys Pro Lys Lys Thr Cys Ala Asp Ser Asp Phe Thr Cys Asp Asn Gly
                85                  90                  95

His Cys Ile His Glu Arg Trp Lys Cys Asp Gly Glu Glu Glu Cys Pro
            100                 105                 110

Asp Gly Ser Asp Glu Ser Glu Ala Thr Cys Thr Leu Gly Thr Cys Arg
        115                 120                 125

Gly Asp Glu Phe Gln Cys Gly Asp Gly Thr Cys Val Leu Ala Ile Lys
    130                 135                 140

His Cys Asn Gln Glu Gln Asp Cys Pro Asp Gly Ser Asp Glu Ala Gly
145                 150                 155                 160

Cys Leu Gln Glu Ser Pro Cys Glu Gly Pro Arg Arg Phe Gln Cys Lys
                165                 170                 175

Ser Gly Lys Arg Val Asp Gly Gly Lys Val Cys Asp Val Gln Arg Asp
            180                 185                 190

Cys Arg Asp Trp Ser Asp Glu Leu Leu Lys Val Trp Cys Gly Ala Cys
        195                 200                 205

Leu Arg Pro Leu Ala Gly Leu Ser Leu Leu Pro Ser Pro Ser Trp Tyr
    210                 215                 220

Leu Gly Ser Arg Pro Ser Ser Ala Pro Cys Pro Asp Thr Phe Cys Ser
225                 230                 235                 240

Asp Pro Leu Phe Gly Phe Met Cys Arg Pro Met Ala Ser His Gly Ala
                245                 250                 255

Phe Arg Pro Gln Ala Ser Gly Leu His Leu Tyr Lys Val Leu Arg Ala
          260                 265                 270

Cys Pro Ser Gln Arg Arg Leu Asn Glu Cys Leu His Asn Asn Gly Gly
        275                 280                 285

Cys Ser His Ile Cys Thr Asp Leu Lys Ile Gly Phe Glu Cys Thr Cys
    290                 295                 300

Pro Ala Gly Phe Gln Leu Leu Asp Gln Lys Thr Cys Gly Asp Ile Asp
305                 310                 315                 320

Glu Cys Lys Asp Pro Asp Ala Cys Ser Gln Ile Cys Val Asn Tyr Lys
                325                 330                 335

Gly Tyr Phe Lys Cys Glu Cys Tyr Pro Gly Tyr Glu Met Asp Leu Leu
            340                 345                 350

Thr Lys Asn Cys Gln Ala Ala Ala Gly Lys Ser Pro Ser Leu Ile Phe
        355                 360                 365

Thr Asn Arg Tyr Glu Val Arg Arg Ile Asp Leu Val Lys Arg Asn Tyr
    370                 375                 380

Ser Arg Leu Ile Pro Met Leu Lys Asn Val Val Ala Leu Asp Val Glu
385                 390                 395                 400

Val Ala Thr Asn Arg Ile Tyr Trp Cys Asp Leu Ser Tyr Arg Lys Ile
                405                 410                 415

Tyr Ser Ala Tyr Met Asp Lys Ala Ser Asp Pro Lys Glu Gln Glu Val
            420                 425                 430

Leu Ile Asp Glu Gln Leu His Ser Pro Glu Gly Leu Ala Val Asp Trp
        435                 440                 445

Val His Lys His Ile Tyr Trp Thr Asp Ser Gly Asn Lys Thr Ile Ser
    450                 455                 460

Val Ala Thr Val Asp Gly Gly Arg Arg Arg Thr Leu Phe Ser Arg Asn
465                 470                 475                 480

Leu Ser Glu Pro Arg Ala Ile Ala Val Asp Pro Leu Arg Gly Phe Met
                485                 490                 495

Tyr Trp Ser Asp Trp Gly Asp Gln Ala Lys Ile Glu Lys Ser Gly Leu
            500                 505                 510

Asn Gly Val Asp Arg Gln Thr Leu Val Ser Asp Asn Ile Glu Trp Pro
        515                 520                 525

Asn Gly Ile Thr Leu Asp Leu Leu Ser Gln Arg Leu Tyr Trp Val Asp
    530                 535                 540

Ser Lys Leu His Gln Leu Ser Ser Ile Asp Phe Ser Gly Gly Asn Arg
545                 550                 555                 560

Lys Thr Leu Ile Ser Ser Thr Asp Phe Leu Ser His Pro Phe Gly Ile
                565                 570                 575

Ala Val Phe Glu Asp Lys Val Phe Trp Thr Asp Leu Glu Asn Glu Ala
            580                 585                 590

Ile Phe Ser Ala Asn Arg Leu Asn Gly Leu Glu Ile Ser Ile Leu Ala
        595                 600                 605

Glu Asn Leu Asn Asn Pro His Asp Ile Val Ile Phe His Glu Leu Lys
    610                 615                 620

Gln Pro Arg Ala Pro Asp Ala Cys Glu Leu Ser Val Gln Pro Asn Gly
625                 630                 635                 640

Gly Cys Glu Tyr Leu Cys Leu Pro Ala Pro Gln Ile Ser Ser His Ser
                645                 650                 655

Pro Lys Tyr Thr Cys Ala Cys Pro Asp Thr Met Trp Leu Gly Pro Asp
            660                 665                 670

Met Lys Arg Cys Tyr Arg Ala Pro Gln Ser Thr Ser Thr Thr Thr Leu
        675                 680                 685

Ala Ser Thr Met Thr Arg Thr Val Pro Ala Thr Thr Arg Ala Pro Gly
    690                 695                 700

Thr Thr Val His Arg Ser Thr Tyr Gln Asn His Ser Thr Glu Thr Pro
705                 710                 715                 720

Ser Leu Thr Ala Ala Val Pro Ser Ser Val Ser Val Pro Arg Ala Pro
                725                 730                 735

Ser Ile Ser Pro Ser Thr Leu Ser Pro Ala Thr Ser Asn His Ser Gln
            740                 745                 750

His Tyr Ala Asn Glu Asp Ser Lys Met Gly Ser Thr Val Thr Ala Ala
        755                 760                 765

Val Ile Gly Ile Ile Val Pro Ile Val Val Ile Ala Leu Leu Cys Met
    770                 775                 780

Ser Gly Tyr Leu Ile Trp Arg Asn Trp Lys Arg Lys Asn Thr Lys Ser
785                 790                 795                 800

Met Asn Phe Asp Asn Pro Val Tyr Arg Lys Thr Thr Glu Glu Glu Asp
                805                 810                 815

Glu Asp Glu Leu His Ile Gly Arg Thr Ala Gln Ile Gly His Val Tyr
            820                 825                 830

Pro Ala Ala Ile Ser Ser Phe Asp Arg Pro Leu Trp Ala Glu Pro Cys
        835                 840                 845

Leu Gly Glu Thr Arg Glu Pro Glu Asp Pro Ala Pro Ala Leu Lys Glu
    850                 855                 860

Leu Phe Val Leu Pro Gly Glu Pro Arg Ser Gln Leu His Gln Leu Pro
865                 870                 875                 880

Lys Asn Pro Leu Ser Glu Leu Pro Val Val Lys Ser Lys Arg Val Ala
                885                 890                 895

Leu Ser Leu Glu Asp Asp Gly Leu Pro
            900                 905


<210> 32
<211> 843
<212> DNA
<213> Homo sapiens

<400> 32
gaggtggagg ttgcagtaag ccaagatggc gccactgcac tctagcctgt ttctgctgag 60
cgggaccatg agcccaaaag tgggcatcac tgggggctgc gacgtctcgg ccaggaggca 120
cccctggcag gtcagcctga ggttctacag catgaagaag ggtctgtggg agcccatctg 180
tgggggctcc ctcatccacc cagagtgggt gctgaccgcc gcccactgcg tcgagcttga 240
ggagttggag gcttgcgcgt ttagagtgca ggtggggcag ctgaggctct atgaggacga 300
ccagcggacg aaggtggttg agatcgtccg tcacccccag tacaacgaga gcctgtctgc 360
ccagggcggt gcggacatcg ccctgctgaa gctggaggcc ccggtgccgc tgtctgagct 420
catccacccg gtctcgctcc cgtctgcctc cctggacgtg ccctcgggga agacctgctg 480
ggtgaccggc tggggtgtca tttggggaca cgttttcctg ctcccgccac cccacctcag 540
ggcagcggaa ggtccaatca tgaggacccg agcttgcgag aggatgtatc acaaaggccc 600
cactgcccac gtcaccatca tcaaggctgc catgccgtgt gcaggggctg agcgccatct 660
ctccccacag ggcgacaacg ggggccccct cctgtgcagg cggaattgca cctgggtcca 720
ggtggaggtg gtgagctggg gcaaactctg cggccttcgc tatcccggca tgtacacccg 780
cgtgacgagc tacgtgtcct ggatccgcca gtacgtcccg ccgttcccca gacgctagct 840
ggg                                                               843


<210> 33
<211> 270
<212> PRT
<213> Homo sapiens

<400> 33
Met Ala Pro Leu His Ser Ser Leu Phe Leu Leu Ser Gly Thr Met Ser
 1               5                  10                  15

Pro Lys Val Gly Ile Thr Gly Gly Cys Asp Val Ser Ala Arg Arg His
            20                  25                  30

Pro Trp Gln Val Ser Leu Arg Phe Tyr Ser Met Lys Lys Gly Leu Trp
        35                  40                  45

Glu Pro Ile Cys Gly Gly Ser Leu Ile His Pro Glu Trp Val Leu Thr
    50                  55                  60

Ala Ala His Cys Val Glu Leu Glu Glu Leu Glu Ala Cys Ala Phe Arg
65                  70                  75                  80

Val Gln Val Gly Gln Leu Arg Leu Tyr Glu Asp Asp Gln Arg Thr Lys
                85                  90                  95

Val Val Glu Ile Val Arg His Pro Gln Tyr Asn Glu Ser Leu Ser Ala
            100                 105                 110

Gln Gly Gly Ala Asp Ile Ala Leu Leu Lys Leu Glu Ala Pro Val Pro
        115                 120                 125

Leu Ser Glu Leu Ile His Pro Val Ser Leu Pro Ser Ala Ser Leu Asp
    130                 135                 140

Val Pro Ser Gly Lys Thr Cys Trp Val Thr Gly Trp Gly Val Ile Trp
145                 150                 155                 160

Gly His Val Phe Leu Leu Pro Pro Pro His Leu Arg Ala Ala Glu Gly
                165                 170                 175

Pro Ile Met Arg Thr Arg Ala Cys Glu Arg Met Tyr His Lys Gly Pro
            180                 185                 190

Thr Ala His Val Thr Ile Ile Lys Ala Ala Met Pro Cys Ala Gly Ala
        195                 200                 205

Glu Arg His Leu Ser Pro Gln Gly Asp Asn Gly Gly Pro Leu Leu Cys
    210                 215                 220

Arg Arg Asn Cys Thr Trp Val Gln Val Glu Val Val Ser Trp Gly Lys
225                 230                 235                 240

Leu Cys Gly Leu Arg Tyr Pro Gly Met Tyr Thr Arg Val Thr Ser Tyr
                245                 250                 255

Val Ser Trp Ile Arg Gln Tyr Val Pro Pro Phe Pro Arg Arg
            260                 265                 270


<210> 34
<211> 239
<212> DNA
<213> Homo sapiens

<400> 34
cactaagcag accagattcc acttgctagt gggcattgct cagcttcctc tgtgactatg 60
tctgacaagt ccaatatgga tgagatcgag aaattcagta agtcgaaact gaagaagaca 120
gaaatgcaag agaaaaatcc acagccttcc aaggaatgga tcgaacagga gaagcaagca 180
ggcttcgtat gaggcgtgca tcgccaatat gcactgttca ttccacaaag cattgcttt  239


<210> 35
<211> 50
<212> PRT
<213> Homo sapiens

<400> 35
Ser Ala Ser Ser Val Thr Met Ser Asp Lys Ser Asn Met Asp Glu Ile
  1               5                  10                  15

Glu Lys Phe Ser Lys Ser Lys Leu Lys Lys Thr Glu Met Gln Glu Lys
             20                  25                  30

Asn Pro Gln Pro Ser Lys Glu Trp Ile Glu Gln Glu Lys Gln Ala Gly
        35                  40                  45

Phe Val
    50

<210> 36
<211> 1348
<212> DNA
<213> Homo sapiens

<400> 36
cttaggagcc aacctcactc ttccatttct cttaagattc actatttgtc ccagccatgc 60
ccaccatcat tgagcgggag ttcaaggagt tggatgctca gcatcgctgg cagctgccgt 120
acttggaaat tcgaaatgag tcccatgact atcttcatag agtggccaag tttccagaaa 180
acagaaatcg aaatagatac agagatgtaa gcccatatga tcacagtcgt gttaaactgc 240
agaatactga gaatgattat attaatgcca gcttagttga catagaagag gcacgaagga 300
gttacatctt aacaccgggt ccacttccta acatgtgctg ccatttctgg cttacggttt 360
ggcagcagaa gaccaaagca gttgttgtgc tgaaccgcat tgtggagaaa gaatcggtta 420
aatgtgcgca gtactggcca acagatgacc aaaagatgct gtttaaagaa acgggattca 480
atgtgaagct cctttcagaa gatgtgaagt catattacac agtacatcta ctacgattag 540
aaaatatcaa tagtggtgaa accagaacaa tatctcactt gcattatacg acctggccag 600
attttggagt ccctgaatca ccagcttcat ttctcaattt cttgttgaaa gtgagagaat 660
ctggctcctt gaaccctgac catgggcctg cagtgatcca ttgtagcgaa ggcactgggt 720
gcacgggcac cttctctctg gtagacgctt gtcttgtttt gatgggaaaa ggagattata 780
ttaacattaa acaagtgtta ttgaacatga gaaaataccg aatgggtctt attcagaccc 840
cagatcaact gagattctca tacatggcta taatagaagg agcaaaatgt ataaaggtag 900
attctagtat acagaaacgg tggaaagaat tttctaagga aaaccttagc gcttgctttg 960
atcattcacc gaacaaaata atgactgaaa aatacaatgg gaacagaatt ggtctagaag 1020
aagaaaaact gacaggtgac cgatgtacag gactttcctc taaaatgcaa gatacaatgg 1080
aagagaacag tgagaatgct ctacggaaac atattctaga ggacagaaag gccaccacag 1140
ctcagaaggt gcagcagatg aaacagaggc taaatgagaa tgaacaaaaa agaaaaaggt 1200
ggttatattg gcaacctatt ctcactaaga tggggtttat gtcagtcatt ttggttggcg 1260
cttttgttgg ctggacactg ttttttcagc aaaatgccct ataaacaatt aattttgccc 1320
agcaagcttc tgcactagta actgacag                                    1348

<210> 37
<211> 415
<212> PRT
<213> Homo sapiens

<400> 37
Met Pro Thr Ile Ile Glu Arg Glu Phe Lys Glu Leu Asp Ala Gln His
1               5                   10                  15

Arg Trp Gln Leu Pro Tyr Leu Glu Ile Arg Asn Glu Ser His Asp Tyr
            20                  25                  30

Leu His Arg Val Ala Lys Phe Pro Glu Asn Arg Asn Arg Asn Arg Tyr
        35                  40                  45

Arg Asp Val Ser Pro Tyr Asp His Ser Arg Val Lys Leu Gln Asn Thr
    50                  55                  60

Glu Asn Asp Tyr Ile Asn Ala Ser Leu Val Asp Ile Glu Glu Ala Arg
65                  70                  75                  80

Arg Ser Tyr Ile Leu Thr Pro Gly Pro Leu Pro Asn Met Cys Cys His
                85                  90                  95

Phe Trp Leu Thr Val Trp Gln Gln Lys Thr Lys Ala Val Val Val Leu
            100                 105                 110

Asn Arg Ile Val Glu Lys Glu Ser Val Lys Cys Ala Gln Tyr Trp Pro
        115                 120                 125

Thr Asp Asp Gln Lys Met Leu Phe Lys Glu Thr Gly Phe Asn Val Lys
    130                 135                 140

Leu Leu Ser Glu Asp Val Lys Ser Tyr Tyr Thr Val His Leu Leu Arg
145                 150                 155                 160

Leu Glu Asn Ile Asn Ser Gly Glu Thr Arg Thr Ile Ser His Leu His
                165                 170                 175

Tyr Thr Thr Trp Pro Asp Phe Gly Val Pro Glu Ser Pro Ala Ser Phe
            180                 185                 190

Leu Asn Phe Leu Leu Lys Val Arg Glu Ser Gly Ser Leu Asn Pro Asp
        195                 200                 205

His Gly Pro Ala Val Ile His Cys Ser Glu Gly Thr Gly Cys Thr Gly
    210                 215                 220

Thr Phe Ser Leu Val Asp Ala Cys Leu Val Leu Met Gly Lys Gly Asp
225                 230                 235                 240

Tyr Ile Asn Ile Lys Gln Val Leu Leu Asn Met Arg Lys Tyr Arg Met
                245                 250                 255

Gly Leu Ile Gln Thr Pro Asp Gln Leu Arg Phe Ser Tyr Met Ala Ile
            260                 265                 270

Ile Glu Gly Ala Lys Cys Ile Lys Val Asp Ser Ser Ile Gln Lys Arg
        275                 280                 285

Trp Lys Glu Phe Ser Lys Glu Asn Leu Ser Ala Cys Phe Asp His Ser
    290                 295                 300

Pro Asn Lys Ile Met Thr Glu Lys Tyr Asn Gly Asn Arg Ile Gly Leu
305                 310                 315                 320

Glu Glu Glu Lys Leu Thr Gly Asp Arg Cys Thr Gly Leu Ser Ser Lys
                325                 330                 335

Met Gln Asp Thr Met Glu Glu Asn Ser Glu Asn Ala Leu Arg Lys His
            340                 345                 350

Ile Leu Glu Asp Arg Lys Ala Thr Thr Ala Gln Lys Val Gln Gln Met
        355                 360                 365

Lys Gln Arg Leu Asn Glu Asn Glu Gln Lys Arg Lys Arg Trp Leu Tyr
    370                 375                 380

Trp Gln Pro Ile Leu Thr Lys Met Gly Phe Met Ser Val Ile Leu Val
385                 390                 395                 400

Gly Ala Phe Val Gly Trp Thr Leu Phe Phe Gln Gln Asn Ala Leu
                405                 410                 415

<210> 38
<211> 105
<212> PRT
<213> Equus caballus

<400> 38
Met Val Lys Gln Ile Glu Ser Lys Ser Ala Phe Gln Glu Ala Leu Asn
1               5                   10                  15

Ser Ala Gly Glu Lys Leu Val Val Val Asp Phe Ser Ala Thr Trp Cys
            20                  25                  30

Gly Pro Cys Lys Met Ile Lys Pro Phe Phe His Ser Leu Ser Glu Lys
        35                  40                  45

Tyr Ser Asn Val Val Phe Leu Glu Val Asp Val Asp Asp Cys Gln Asp
    50                  55                  60

Val Ala Ala Glu Cys Glu Val Lys Cys Met Pro Thr Phe Gln Phe Phe
65                  70                  75                  80

Lys Lys Gly Gln Lys Val Asp Glu Phe Ser Gly Ala Asn Lys Glu Lys
                85                  90                  95

Leu Glu Ala Thr Ile Lys Gly Leu Ile
            100                 105

<210> 39
<211> 104
<212> PRT
<213> Ovis aries

<400> 39
Val Lys Gln Ile Glu Ser Lys Tyr Ala Phe Gln Glu Ala Leu Asn Ser
1               5                   10                  15

Ala Gly Glu Lys Leu Val Val Val Asp Phe Ser Ala Thr Trp Cys Gly
            20                  25                  30

Pro Cys Lys Met Ile Lys Pro Phe Phe His Ser Leu Ser Glu Lys Tyr
        35                  40                  45

Ser Asn Val Val Phe Leu Glu Val Asp Val Asp Asp Cys Gln Asp Val
    50                  55                  60

Ala Ala Glu Cys Glu Val Lys Cys Met Pro Thr Phe Gln Phe Phe Lys

65                  70                  75                  80

Lys Gly Gln Lys Val Ser Glu Phe Ser Gly Ala Asn Lys Glu Lys Leu
                85                  90                  95

Glu Ala Thr Ile Asn Glu Leu Ile
            100


<210> 40
<211> 104
<212> PRT
<213> Bos taurus

<400> 40
Val Lys Gln Ile Glu Ser Lys Tyr Ala Phe Gln Glu Ala Leu Asn Ser
1               5                   10                  15

Ala Gly Glu Lys Leu Val Val Val Asp Phe Ser Ala Thr Trp Cys Gly
            20                  25                  30

Pro Cys Lys Met Ile Lys Pro Phe Phe His Ser Leu Ser Glu Lys Tyr
        35                  40                  45

Ser Asn Val Val Phe Leu Glu Val Asp Val Asp Asp Cys Gln Asp Val
    50                  55                  60

Ala Ala Glu Cys Glu Val Lys Cys Met Pro Thr Phe Gln Phe Phe Lys
65                  70                  75                  80

Lys Gly Gln Lys Val Gly Glu Phe Ser Gly Ala Asn Lys Glu Lys Leu
                85                  90                  95

Glu Ala Thr Ile Asn Glu Leu Ile
            100


<210> 41
<211> 104
<212> PRT
<213> Macaca mulatta

<400> 41
Val Lys Gln Ile Glu Ser Lys Ala Ala Phe Gln Glu Ala Leu Asp Asp
1               5                   10                  15

Ala Gly Asp Lys Leu Val Val Val Asp Phe Ser Ala Thr Trp Cys Gly
            20                  25                  30

Pro Cys Lys Met Ile Lys Pro Phe Phe His Ser Leu Ser Glu Lys Tyr
        35                  40                  45

Ser Asn Val Val Phe Leu Glu Val Asp Val Asp Asp Cys Gln Asp Val
    50                  55                  60

Ala Ser Glu Cys Glu Val Lys Cys Met Pro Thr Phe Gln Phe Phe Lys
65                  70                  75                  80

Lys Gly Gln Lys Val Gly Glu Phe Ser Gly Ala Asn Lys Glu Lys Leu
                85                  90                  95

Glu Ala Thr Ile Asn Glu Leu Val
            100


<210> 42
<211> 104
<212> PRT
<213> Rattus norvegicus

<400> 42
Val Lys Leu Ile Glu Ser Lys Glu Ala Phe Gln Glu Ala Leu Ala Ala
1               5                   10                  15

Ala Gly Asp Lys Leu Val Val Val Asp Phe Ser Ala Thr Trp Cys Gly
            20                  25                  30

Pro Cys Lys Met Ile Lys Pro Phe Phe His Ser Leu Cys Asp Lys Tyr
        35                  40                  45

Ser Asn Val Val Phe Leu Glu Val Asp Val Asp Asp Cys Gln Asp Val
    50                  55                  60

Ala Ala Asp Cys Glu Val Lys Cys Met Pro Thr Phe Gln Phe Tyr Lys
65                  70                  75                  80

Lys Gly Gln Lys Val Gly Glu Phe Ser Gly Ala Asn Lys Glu Lys Leu
                85                  90                  95

Glu Ala Thr Ile Thr Glu Phe Ala
            100


<210> 43
<211> 667
<212> PRT
<213> Homo sapiens

<400> 43

<210> 44
<211> 658

<212> PRT
<213> Homo sapiens

<400> 44

<210> 45
<211> 560
<212> PRT
<213> Homo sapiens

<400> 45
Met Val Leu Leu Ser Ser Leu Cys Leu Leu Leu Pro Asp Glu Asp Ala
  1               5                  10                  15

Leu Pro Phe Leu Thr Leu Ala Ser Ala Pro Ser Gln Asp Gly Lys Thr
             20                  25                  30

Glu Ala Pro Arg Gly Ala Trp Lys Ile Leu Gly Leu Phe Tyr Tyr Ala
         35                  40                  45

Ala Leu Tyr Tyr Pro Leu Ala Ala Cys Ala Thr Ala Gly His Thr Ala
     50                  55                  60

Ala His Leu Leu Gly Ser Thr Leu Ser Trp Ala His Leu Gly Val Gln
 65                  70                  75                  80

Val Trp Gln Arg Ala Glu Cys Pro Gln Val Pro Lys Ile Tyr Lys Tyr
                 85                  90                  95

Tyr Ser Leu Leu Ala Ser Leu Pro Leu Leu Leu Gly Leu Gly Phe Leu
            100                 105                 110

Ser Leu Trp Tyr Pro Val Gln Leu Val Arg Ser Phe Ser Arg Arg Thr
        115                 120                 125

Gly Ala Gly Ser Lys Gly Leu Gln Ser Ser Tyr Ser Glu Glu Tyr Leu
    130                 135                 140

Arg Asn Leu Leu Cys Arg Lys Lys Leu Gly Ser Ser Tyr His Thr Ser
145                 150                 155                 160

Lys His Gly Phe Leu Ser Trp Ala Arg Val Cys Leu Arg His Cys Ile
                165                 170                 175

Tyr Thr Pro Gln Pro Gly Phe His Leu Pro Leu Lys Leu Val Leu Ser

180                 185                 190

Ala Thr Leu Thr Gly Thr Ala Ile Tyr Gln Val Ala Leu Leu Leu Leu
        195                 200                 205

Val Gly Val Val Pro Thr Ile Gln Lys Val Arg Ala Gly Val Thr Thr
    210                 215                 220

Asp Val Ser Tyr Leu Leu Ala Gly Phe Gly Ile Val Leu Ser Glu Asp
225                 230                 235                 240

Lys Gln Glu Val Val Glu Leu Val Lys His His Leu Trp Ala Leu Glu
                245                 250                 255

Val Cys Tyr Ile Ser Ala Leu Val Leu Ser Cys Leu Leu Thr Phe Leu
            260                 265                 270

Val Leu Met Arg Ser Leu Val Thr His Arg Thr Asn Leu Arg Ala Leu
        275                 280                 285

His Arg Gly Ala Ala Leu Asp Leu Ser Pro Leu His Arg Ser Pro His
    290                 295                 300

Pro Ser Arg Gln Ala Ile Phe Cys Trp Met Ser Phe Ser Ala Tyr Gln
305                 310                 315                 320

Thr Ala Phe Ile Cys Leu Gly Leu Leu Val Gln Gln Ile Ile Phe Phe
                325                 330                 335

Leu Gly Thr Thr Ala Leu Ala Phe Leu Val Leu Met Pro Val Leu His
            340                 345                 350

Gly Arg Asn Leu Leu Leu Phe Arg Ser Leu Glu Ser Ser Trp Pro Phe
        355                 360                 365

Trp Leu Thr Leu Ala Leu Ala Val Ile Leu Gln Asn Met Ala Ala His
    370                 375                 380

Trp Val Phe Leu Glu Thr His Asp Gly His Pro Gln Leu Thr Asn Arg
385                 390                 395                 400

Arg Val Leu Tyr Ala Ala Thr Phe Leu Leu Phe Pro Leu Asn Val Leu
                405                 410                 415

Val Gly Ala Met Val Ala Thr Trp Arg Val Leu Leu Ser Ala Leu Tyr
            420                 425                 430

Asn Ala Ile His Leu Gly Gln Met Asp Leu Ser Leu Leu Pro Pro Arg
        435                 440                 445

Ala Ala Thr Leu Asp Pro Gly Tyr Tyr Thr Tyr Arg Asn Phe Leu Lys
    450                 455                 460

Ile Glu Val Ser Gln Ser His Pro Ala Met Thr Ala Phe Cys Ser Leu
465                 470                 475                 480

Leu Leu Gln Ala Gln Ser Leu Leu Pro Arg Thr Met Ala Ala Pro Gln

485                 490                 495

Asp Ser Leu Arg Pro Gly Glu Glu Asp Glu Gly Met Gln Leu Leu Gln
            500                 505                 510

Thr Lys Asp Ser Met Ala Lys Gly Ala Arg Pro Gly Ala Ser Arg Gly
        515                 520                 525

Arg Ala Arg Trp Gly Leu Ala Tyr Thr Leu Leu His Asn Pro Thr Leu
    530                 535                 540

Gln Val Phe Arg Lys Thr Ala Leu Leu Gly Ala Asn Gly Ala Gln Pro
545                 550                 555                 560

<210> 46
<211> 670
<212> PRT
<213> Mus musculus

<400> 46
Met Glu Ser Gln Ala Ser Glu Asn Gly Ser Gln Thr Ser Ser Gly Val
1               5                   10                  15

Thr Asp Asp Tyr Ser Ser Trp Tyr Ile Glu Glu Pro Leu Gly Ala Glu
            20                  25                  30

Glu Val Gln Pro Glu Gly Val Ile Pro Leu Cys Gln Leu Thr Ala Pro
        35                  40                  45

Pro Ala Leu Leu His Ala Cys Leu Ala Ser Leu Ser Phe Leu Val Leu
    50                  55                  60

Leu Leu Leu Ala Leu Leu Val Arg Arg Arg Arg Leu Trp Pro Arg Cys
65                  70                  75                  80

Gly His Arg Gly Leu Gly Leu Pro Ser Pro Val Asp Phe Leu Ala Gly
                85                  90                  95

Asp Leu Ser Trp Thr Val Pro Ala Ala Val Phe Val Val Leu Phe Ser
            100                 105                 110

Asn Leu Cys Leu Leu Leu Pro Asp Glu Asn Pro Leu Pro Phe Leu Asn
        115                 120                 125

Leu Thr Ala Ala Ser Ser Pro Asp Gly Glu Met Glu Thr Ser Arg Gly
    130                 135                 140

Pro Trp Lys Leu Leu Ala Leu Leu Tyr Tyr Pro Ala Leu Tyr Tyr Pro
145                 150                 155                 160

Leu Ala Ala Cys Ala Ser Ala Gly His Gln Ala Ala Phe Leu Leu Gly
                165                 170                 175

Thr Val Leu Ser Trp Ala His Phe Gly Val Gln Val Trp Gln Lys Ala
            180                 185                 190

Glu Cys Pro Gln Asp Pro Lys Ile Tyr Lys His Tyr Ser Leu Leu Ala
        195                 200                 205

Ser Leu Pro Leu Leu Leu Gly Leu Gly Phe Leu Ser Leu Trp Tyr Pro
    210                 215                 220

Val Gln Leu Val Gln Ser Leu Arg His Arg Thr Gly Ala Gly Ser Gln
225                 230                 235                 240

Gly Leu Gln Thr Ser Tyr Ser Glu Lys Tyr Leu Arg Thr Leu Leu Cys
                245                 250                 255

Pro Lys Lys Leu Asp Ser Cys Ser His Pro Ala Ser Lys Arg Ser Leu
            260                 265                 270

Leu Ser Arg Ala Trp Ala Phe Ser His His Ser Ile Tyr Thr Pro Gln
        275                 280                 285

Pro Gly Phe Arg Leu Pro Leu Lys Leu Val Ile Ser Ala Thr Leu Thr
    290                 295                 300

Gly Thr Ala Thr Tyr Gln Val Ala Leu Leu Leu Leu Val Ser Val Val
305                 310                 315                 320

Pro Thr Val Gln Lys Val Arg Ala Gly Ile Asn Thr Asp Val Ser Tyr
                325                 330                 335

Leu Leu Ala Gly Phe Gly Ile Val Leu Ser Glu Asp Arg Gln Glu Val
            340                 345                 350

Val Glu Leu Val Lys His His Leu Trp Thr Val Glu Ala Cys Tyr Ile
        355                 360                 365

Ser Ala Leu Val Leu Ser Cys Ala Ser Thr Phe Leu Leu Leu Ile Arg
    370                 375                 380

Ser Leu Arg Thr His Arg Ala Asn Leu Gln Ala Leu His Arg Gly Ala
385                 390                 395                 400

Ala Leu Asp Leu Asp Pro Pro Leu Gln Ser Ile His Pro Ser Arg Gln
                405                 410                 415

Ala Ile Val Ser Trp Met Ser Phe Cys Ala Tyr Gln Thr Ala Phe Ser
            420                 425                 430

Cys Leu Gly Leu Leu Val Gln Gln Val Ile Phe Phe Leu Gly Thr Thr
        435                 440                 445

Ser Leu Ala Phe Leu Val Phe Val Pro Leu Leu His Gly Arg Asn Leu
    450                 455                 460

Leu Leu Leu Arg Ser Leu Glu Ser Thr Trp Pro Phe Trp Leu Thr Val
465                 470                 475                 480

Ala Leu Ala Val Ile Leu Gln Asn Ile Ala Ala Asn Trp Ile Phe Leu
                485                 490                 495

Arg Thr His His Gly Tyr Pro Glu Leu Thr Asn Arg Arg Met Leu Cys
            500                 505                 510

Val Ala Thr Phe Leu Leu Phe Pro Ile Asn Met Leu Val Gly Ala Ile
        515                 520                 525

Met Ala Val Trp Arg Val Leu Ile Ser Ser Leu Tyr Asn Thr Val His
    530                 535                 540

Leu Gly Gln Met Asp Leu Ser Leu Leu Pro Gln Arg Ala Ala Ser Leu
545                 550                 555                 560

Asp Pro Gly Tyr His Thr Tyr Gln Asn Phe Leu Arg Ile Glu Ala Ser
                565                 570                 575

Gln Ser His Pro Gly Val Ile Ala Phe Cys Ala Leu Leu Leu His Ala
            580                 585                 590

Pro Ser Pro Gln Pro Arg Pro Pro Leu Ala Pro Gln Asp Ser Leu Arg
        595                 600                 605

Pro Ala Glu Glu Glu Glu Gly Met Gln Leu Leu Gln Thr Lys Asp Leu
    610                 615                 620

Met Ala Lys Gly Ala Gly His Lys Gly Ser Gln Ser Arg Ala Arg Trp
625                 630                 635                 640

Gly Leu Ala Tyr Thr Leu Leu His Asn Pro Ser Leu Gln Ala Phe Arg
                645                 650                 655

Lys Ala Ala Leu Thr Ser Ala Lys Ala Asn Gly Thr Gln Pro
            660                 665                 670


<210> 47
<211> 1060
<212> PRT
<213> Homo sapiens

<400> 47
His Leu Ile Pro Ser Leu Arg Gln Val Val Phe Gln Gly Asp Arg Leu
  1               5                  10                  15

Pro Phe Gln Cys Ser Ala Ser Tyr Leu Gly Asn Asp Thr Arg Ile Arg
             20                  25                  30

Trp Tyr His Asn Arg Ala Pro Val Glu Gly Asp Glu Gln Ala Gly Ile
         35                  40                  45

Leu Leu Ala Glu Ser Leu Ile His Asp Cys Thr Phe Ile Thr Ser Glu
     50                  55                  60

Leu Thr Leu Ser His Ile Gly Val Trp Ala Ser Gly Glu Trp Glu Cys
 65                  70                  75                  80

Thr Val Ser Met Ala Gln Gly Asn Ala Ser Lys Lys Val Glu Ile Val
                85                  90                  95

Val Leu Glu Thr Ser Ala Ser Tyr Cys Pro Ala Glu Arg Val Ala Asn
            100                 105                 110

Asn Arg Gly Asp Phe Arg Trp Pro Arg Thr Leu Ala Gly Ile Thr Ala
        115                 120                 125

Tyr Gln Ser Cys Leu Gln Tyr Pro Phe Thr Ser Val Pro Leu Gly Gly
    130                 135                 140

Gly Ala Pro Gly Thr Arg Ala Ser Arg Arg Cys Asp Arg Ala Gly Arg
145                 150                 155                 160

Trp Glu Pro Gly Asp Tyr Ser His Cys Leu Tyr Thr Asn Asp Ile Thr
                165                 170                 175

Arg Val Leu Tyr Thr Phe Val Leu Met Pro Ile Asn Ala Ser Asn Ala
            180                 185                 190

Leu Thr Leu Ala His Gln Leu Arg Val Tyr Thr Ala Glu Ala Ala Ser
        195                 200                 205

Phe Ser Asp Met Met Asp Val Val Tyr Val Ala Gln Met Ile Gln Lys
    210                 215                 220

Phe Leu Gly Tyr Val Asp Gln Ile Lys Glu Leu Val Glu Val Met Val
225                 230                 235                 240

Asp Met Ala Ser Asn Leu Met Leu Val Asp Glu His Leu Leu Trp Leu
                245                 250                 255

Ala Gln Arg Glu Asp Lys Ala Cys Ser Arg Ile Val Gly Ala Leu Glu
            260                 265                 270

Arg Ile Gly Gly Ala Ala Leu Ser Pro His Ala Gln His Ile Ser Val
        275                 280                 285

Asn Ala Arg Asn Val Ala Leu Glu Ala Tyr Leu Ile Lys Pro His Ser
    290                 295                 300

Tyr Val Gly Leu Thr Cys Thr Ala Phe Gln Arg Arg Glu Gly Gly Val
305                 310                 315                 320

Pro Gly Thr Arg Pro Gly Ser Pro Gly Gln Asn Pro Pro Pro Glu Pro
                325                 330                 335

Glu Pro Pro Ala Asp Gln Gln Leu Arg Phe Arg Cys Thr Thr Gly Arg
            340                 345                 350

Pro Asn Val Ser Leu Ser Ser Phe His Ile Lys Asn Ser Val Ala Leu
        355                 360                 365

Ala Ser Ile Gln Leu Pro Pro Ser Leu Phe Ser Ser Leu Pro Ala Ala
    370                 375                 380

Leu Ala Pro Pro Val Pro Pro Asp Cys Thr Leu Gln Leu Leu Val Phe
385                 390                 395                 400

Arg Asn Gly Arg Leu Phe His Ser His Ser Asn Thr Ser Arg Pro Gly
                405                 410                 415

Ala Ala Gly Pro Gly Lys Arg Arg Gly Val Ala Thr Pro Val Ile Phe
            420                 425                 430

Ala Gly Thr Ser Gly Cys Gly Val Gly Asn Leu Thr Glu Pro Val Ala
        435                 440                 445

Val Ser Leu Arg His Trp Ala Glu Gly Ala Glu Pro Val Ala Ala Trp
    450                 455                 460

Trp Ser Gln Glu Gly Pro Gly Glu Ala Gly Gly Trp Thr Ser Glu Gly
465                 470                 475                 480

Cys Gln Leu Arg Ser Ser Gln Pro Asn Val Ser Ala Leu His Cys Gln
                485                 490                 495

His Leu Gly Asn Val Ala Val Leu Met Glu Leu Ser Ala Phe Pro Arg
            500                 505                 510

Glu Val Gly Gly Ala Gly Ala Gly Leu His Pro Val Val Tyr Pro Cys
        515                 520                 525

Thr Ala Leu Leu Leu Leu Cys Leu Phe Ala Thr Ile Ile Thr Tyr Ile
    530                 535                 540

Leu Asn His Ser Ser Ile Arg Val Ser Arg Lys Gly Trp His Met Leu
545                 550                 555                 560

Leu Asn Leu Cys Phe His Ile Ala Met Thr Ser Ala Val Phe Ala Gly
                565                 570                 575

Gly Ile Thr Leu Thr Asn Tyr Gln Met Val Cys Gln Ala Val Gly Ile
            580                 585                 590

Thr Leu His Tyr Ser Ser Leu Ser Thr Leu Leu Trp Met Gly Val Lys
        595                 600                 605

Ala Arg Val Leu His Lys Glu Leu Thr Trp Arg Ala Pro Pro Pro Gln
    610                 615                 620

Glu Gly Asp Pro Ala Leu Pro Thr Pro Ser Pro Met Leu Arg Cys Trp
625                 630                 635                 640

Leu Val Trp Arg Pro Ser Leu Gly Ala Phe Tyr Ile Pro Val Ala Leu
                645                 650                 655

Ile Leu Leu Ile Thr Trp Ile Tyr Phe Leu Cys Ala Gly Leu Arg Leu
            660                 665                 670

Arg Gly Pro Leu Ala Gln Asn Pro Lys Ala Gly Asn Ser Arg Ala Ser
        675                 680                 685

Leu Glu Ala Gly Glu Glu Leu Arg Gly Ser Thr Arg Leu Arg Gly Ser
    690                 695                 700

Gly Pro Leu Leu Ser Asp Ser Gly Ser Leu Leu Ala Thr Gly Ser Ala
705                 710                 715                 720

Arg Val Gly Thr Pro Gly Pro Pro Glu Asp Gly Asp Ser Leu Tyr Ser
                725                 730                 735

Pro Gly Val Gln Leu Gly Ala Leu Val Thr Thr His Phe Leu Tyr Leu
            740                 745                 750

Ala Met Trp Ala Cys Gly Ala Leu Ala Val Ser Gln Arg Trp Leu Pro
        755                 760                 765

Arg Val Val Cys Ser Cys Leu Tyr Gly Val Ala Ala Ser Ala Leu Gly
    770                 775                 780

Leu Phe Val Phe Thr His His Cys Ala Arg Arg Arg Asp Val Arg Ala
785                 790                 795                 800

Ser Trp Arg Ala Cys Cys Pro Pro Ala Ser Pro Ala Ala Pro His Ala
                805                 810                 815

Pro Pro Arg Ala Leu Pro Ala Ala Ala Glu Asp Gly Ser Pro Val Phe
            820                 825                 830

Gly Glu Gly Pro Pro Ser Leu Lys Ser Ser Pro Ser Gly Ser Ser Gly
        835                 840                 845

His Pro Leu Ala Leu Gly Pro Cys Lys Leu Thr Asn Leu Gln Leu Ala
    850                 855                 860

Gln Ser Gln Val Cys Glu Ala Gly Ala Ala Ala Gly Gly Glu Gly Glu
865                 870                 875                 880

Pro Glu Pro Ala Gly Thr Arg Gly Asn Leu Ala His Arg His Pro Asn
                885                 890                 895

Asn Val His His Gly Arg Arg Ala His Lys Ser Arg Ala Lys Gly His
            900                 905                 910

Arg Ala Gly Glu Ala Cys Gly Lys Asn Arg Leu Lys Ala Leu Arg Gly
        915                 920                 925

Gly Ala Ala Gly Ala Leu Glu Leu Leu Ser Ser Glu Ser Gly Ser Leu
    930                 935                 940

His Asn Ser Pro Thr Asp Ser Tyr Leu Gly Ser Ser Arg Asn Ser Pro
945                 950                 955                 960

Gly Ala Gly Leu Gln Leu Glu Gly Glu Pro Met Leu Thr Pro Ser Glu
                965                 970                 975

Gly Ser Asp Thr Ser Ala Ala Pro Leu Ser Glu Ala Gly Arg Ala Gly
            980                 985                 990

Gln Arg Arg Ser Ala Ser Arg Asp Ser Leu Lys Gly Gly Gly Ala Leu
        995                 1000                1005

Glu Lys Glu Ser His Arg Arg Ser Tyr Pro Leu Asn Ala Ala Ser Leu
    1010                1015                1020

Asn Gly Ala Pro Lys Gly Gly Lys Tyr Asp Asp Val Thr Leu Met Gly
1025                1030                1035                1040

Ala Glu Val Ala Ser Gly Gly Cys Met Lys Thr Gly Leu Trp Lys Ser
                1045                1050                1055

Glu Thr Thr Val
            1060


<210> 48
<211> 1094
<212> PRT
<213> Homo sapiens

<400> 48
   Met Ala Arg Pro Val Arg Gly Gly Leu Gly Ala Pro Arg Arg Ser Pro
     1               5                  10                  15
   Cys Leu Leu Leu Leu Trp Leu Leu Leu Leu Arg Leu Glu Pro Val Thr
                20                  25                  30
   Ala Ala Ala Gly Pro Arg Ala Pro Cys Ala Ala Ala Cys Thr Cys Ala
            35                  40                  45
   Gly Asp Ser Leu Asp Cys Gly Gly Arg Gly Leu Ala Ala Leu Pro Gly
        50                  55                  60
   Asp Leu Pro Ser Trp Thr Arg Ser Leu Asn Leu Ser Tyr Asn Lys Leu
    65                  70                  75                  80
   Ser Glu Ile Asp Pro Ala Gly Phe Glu Asp Leu Pro Asn Leu Gln Glu
                    85                  90                  95
   Val Tyr Leu Asn Asn Asn Glu Leu Thr Ala Val Pro Ser Leu Gly Ala
               100                 105                 110
   Ala Ser Ser His Val Val Ser Leu Phe Leu Gln His Asn Lys Ile Arg
           115                 120                 125
   Ser Val Glu Gly Ser Gln Leu Lys Ala Tyr Leu Ser Leu Glu Val Leu
       130                 135                 140
   Asp Leu Ser Leu Asn Asn Ile Thr Glu Val Arg Asn Thr Cys Phe Pro
   145                 150                 155                 160
   His Gly Pro Pro Ile Lys Glu Leu Asn Leu Ala Gly Asn Arg Ile Gly
                   165                 170                 175
   Thr Leu Glu Leu Gly Ala Phe Asp Gly Leu Ser Arg Ser Leu Leu Thr
   180 185 190
   Leu Arg Leu Ser Lys Asn Arg Ile Thr Gln Leu Pro Val Arg Ala Phe
   195 200 205
   Lys Leu Pro Arg Leu Thr Gln Leu Asp Leu Asn Arg Asn Arg Ile Arg
   210 215 220
   Leu Ile Glu Gly Leu Thr Phe Gln Gly Leu Asn Ser Leu Glu Val Leu
   225 230 235 240
   Lys Leu Gln Arg Asn Asn Ile Ser Lys Leu Thr Asp Gly Ala Phe Trp
   245 250 255
   Gly Leu Ser Lys Met His Val Leu His Leu Glu Tyr Asn Ser Leu Val
   260 265 270
   Glu Val Asn Ser Gly Ser Leu Tyr Gly Leu Thr Ala Leu His Gln Leu
   275 280 285
   His Leu Ser Asn Asn Ser Ile Ala Arg Ile His Arg Lys Gly Trp Ser
   290 295 300
   Phe Cys Gln Lys Leu His Glu Leu Val Leu Ser Phe Asn Asn Leu Thr
   305 310 315 320
   Arg Leu Asp Glu Glu Ser Leu Ala Glu Leu Ser Ser Leu Ser Val Leu
   325 330 335
   Arg Leu Ser His Asn Ser Ile Ser His Ile Ala Glu Gly Ala Phe Lys
   340 345 350
   Gly Leu Arg Ser Leu Arg Val Leu Asp Leu Asp His Asn Glu Ile Ser
   355 360 365
   Gly Thr Ile Glu Asp Thr Ser Gly Ala Phe Ser Gly Leu Asp Ser Leu
   370 375 380
   Ser Lys Leu Thr Leu Phe Gly Asn Lys Ile Lys Ser Val Ala Lys Arg
   385 390 395 400
   Ala Phe Ser Gly Leu Glu Gly Leu Glu His Leu Asn Leu Gly Gly Asn
   405 410 415
   Ala Ile Arg Ser Val Gln Phe Asp Ala Phe Val Lys Met Lys Asn Leu
   420 425 430
   Lys Glu Leu His Ile Ser Ser Asp Ser Phe Leu Cys Asp Cys Gln Leu
   435 440 445
   Lys Trp Leu Pro Pro Trp Leu Ile Gly Arg Met Leu Gln Ala Phe Val
   450 455 460
   Thr Ala Thr Cys Ala His Pro Glu Ser Leu Lys Gly Gln Ser Ile Phe
   465 470 475 480
   Ser Val Pro Pro Glu Ser Phe Val Cys Asp Asp Phe Leu Lys Pro Gln
   485 490 495
   Ile Ile Thr Gln Pro Glu Thr Thr Met Ala Met Val Gly Lys Asp Ile
   500 505 510
   Arg Phe Thr Cys Ser Ala Ala Ser Ser Ser Ser Ser Pro Met Thr Phe
   515 520 525
   Ala Trp Lys Lys Asp Asn Glu Val Leu Thr Asn Ala Asp Met Glu Asn
   530 535 540
   Phe Val His Val His Ala Gln Asp Gly Glu Val Met Glu Tyr Thr Thr
   545 550 555 560
   Ile Leu His Leu Arg Gln Val Thr Phe Gly His Glu Gly Arg Tyr Gln
   565 570 575
   Cys Val Ile Thr Asn His Phe Gly Ser Thr Tyr Ser His Lys Ala Arg
   580 585 590
   Leu Thr Val Asn Val Leu Pro Ser Phe Thr Lys Thr Pro His Asp Ile
   595 600 605
   Thr Ile Arg Thr Thr Thr Met Ala Arg Leu Glu Cys Ala Ala Thr Gly
   610 615 620
   His Pro Asn Pro Gln Ile Ala Trp Gln Lys Asp Gly Gly Thr Asp Phe
   625 630 635 640
   Pro Ala Ala Arg Glu Arg Arg Met His Val Met Pro Asp Asp Asp Val
   645 650 655
   Phe Phe Ile Thr Asp Val Lys Ile Asp Asp Ala Gly Val Tyr Ser Cys
   660 665 670
   Thr Ala Gln Asn Ser Ala Gly Ser Ile Ser Ala Asn Ala Thr Leu Thr
   675 680 685
   Val Leu Glu Thr Pro Ser Leu Val Val Pro Leu Glu Asp Arg Val Val
   690 695 700
   Ser Val Gly Glu Thr Val Ala Leu Gln Cys Lys Ala Thr Gly Asn Pro
   705 710 715 720
   Pro Pro Arg Ile Thr Trp Phe Lys Gly Asp Arg Pro Leu Ser Leu Thr
   725 730 735
   Glu Arg His His Leu Thr Pro Asp Asn Gln Leu Leu Val Val Gln Asn
   740 745 750
   Val Val Ala Glu Asp Ala Gly Arg Tyr Thr Cys Glu Met Ser Asn Thr
   755 760 765
   Leu Gly Thr Glu Arg Ala His Ser Gln Leu Ser Val Leu Pro Ala Ala
   770 775 780
   Gly Cys Arg Lys Asp Gly Thr Thr Val Gly Ile Phe Thr Ile Ala Val
   785                 790                 795                 800
   Val Ser Ser Ile Val Leu Thr Ser Leu Val Trp Val Cys Ile Ile Tyr
                   805                 810                 815
   Gln Thr Arg Lys Lys Ser Glu Glu Tyr Ser Val Thr Asn Thr Asp Glu
               820                 825                 830
   Thr Val Val Pro Pro Asp Val Pro Ser Tyr Leu Ser Ser Gln Gly Thr
           835                 840                 845
   Leu Ser Asp Arg Gln Glu Thr Val Val Arg Thr Glu Gly Ala Gly Pro
       850                 855                 860
   Gln Ala Asn Gly His Ile Glu Ser Asn Gly Val Cys Pro Arg Asp Ala
   865                 870                 875                 880
   Ser His Phe Pro Glu Pro Asp Thr His Ser Val Ala Cys Arg Gln Pro
                   885                 890                 895
   Lys Leu Cys Ala Gly Ser Ala Tyr His Lys Glu Pro Trp Lys Ala Ile
               900                 905                 910
   Glu Lys Ala Glu Gly Thr Pro Gly Pro His Lys Met Glu His Gly Gly
           915                 920                 925
   Arg Val Val Cys Ser Asp Cys Asn Thr Glu Val Asp Cys Tyr Ser Arg
       930                 935                 940
   Gly Gln Ala Phe His Pro Gln Pro Val Ser Arg Asp Ser Ala Gln Pro
   945                 950                 955                 960
   Ser Ala Pro Asn Gly Pro Glu Pro Gly Gly Ser Asp Gln Glu His Ser
                   965                 970                 975
   Pro His His Gln Cys Ser Arg Thr Ala Ala Gly Ser Cys Pro Glu Cys
               980                 985                 990
   Gln Gly Ser Leu Tyr Pro Ser Asn His Asp Arg Met Leu Thr Ala Val
           995                 1000                1005
   Lys Lys Lys Pro Met Ala Ser Leu Asp Gly Lys Gly Asp Ser Ser Trp
       1010                1015                1020
   Thr Leu Ala Arg Leu Tyr His Pro Asp Ser Thr Glu Leu Gln Pro Ala
   1025                1030                1035                1040
   Ser Ser Leu Thr Ser Gly Ser Pro Glu Arg Ala Glu Ala Gln Tyr Leu
                   1045                1050                1055
   Leu Val Ser Asn Gly His Leu Pro Lys Ala Cys Asp Ala Ser Pro Glu
               1060                1065                1070
   Ser Thr Pro Leu Thr Gly Gln Leu Pro Gly Lys Gln Arg Val Pro Leu
           1075                1080                1085
   Leu Leu Ala Pro Lys Ser
   1090

<210> 49
<211> 352
<212> PRT
<213> Homo sapiens

<400> 49
Met Arg Ser Leu Gly Ala Leu Leu Leu Leu Leu Ser Ala Cys Leu Ala
1               5                   10                  15

Val Ser Ala Gly Pro Val Pro Thr Pro Pro Asp Asn Ile Gln Val Gln
            20                  25                  30

Glu Asn Phe Asn Ile Ser Arg Ile Tyr Gly Lys Trp Tyr Asn Leu Ala
        35                  40                  45

Ile Gly Ser Thr Cys Pro Trp Leu Lys Lys Ile Met Asp Arg Met Thr
    50                  55                  60

Val Ser Thr Leu Val Leu Gly Glu Gly Ala Thr Glu Ala Glu Ile Ser
65                  70                  75                  80

Met Thr Ser Thr Arg Trp Arg Lys Gly Val Cys Glu Glu Thr Ser Gly
                85                  90                  95

Ala Tyr Glu Lys Thr Asp Thr Asp Gly Lys Phe Leu Tyr His Lys Ser
            100                 105                 110

Lys Trp Asn Ile Thr Met Glu Ser Tyr Val Val His Thr Asn Tyr Asp
        115                 120                 125

Glu Tyr Ala Ile Phe Leu Thr Lys Lys Phe Ser Arg His His Gly Pro
    130                 135                 140

Thr Ile Thr Ala Lys Leu Tyr Gly Arg Ala Pro Gln Leu Arg Glu Thr
145                 150                 155                 160

Leu Leu Gln Asp Phe Arg Val Val Ala Gln Gly Val Gly Ile Pro Glu
                165                 170                 175

Asp Ser Ile Phe Thr Met Ala Asp Arg Gly Glu Cys Val Pro Gly Glu
            180                 185                 190

Gln Glu Pro Glu Pro Ile Leu Ile Pro Arg Val Arg Arg Ala Val Leu
        195                 200                 205

Pro Gln Glu Glu Glu Gly Ser Gly Gly Gly Gln Leu Val Thr Glu Val
    210                 215                 220

Thr Lys Lys Glu Asp Ser Cys Gln Leu Gly Tyr Ser Ala Gly Pro Cys
225                 230                 235                 240

Met Gly Met Thr Ser Arg Tyr Phe Tyr Asn Gly Thr Ser Met Ala Cys
                245                 250                 255

Glu Thr Phe Gln Tyr Gly Gly Cys Met Gly Asn Gly Asn Asn Phe Val
            260                 265                 270

Thr Glu Lys Glu Cys Leu Gln Thr Cys Arg Thr Val Ala Ala Cys Asn
        275                 280                 285

Leu Pro Ile Val Arg Gly Pro Cys Arg Ala Phe Ile Gln Leu Trp Ala
    290                 295                 300

Phe Asp Ala Val Lys Gly Lys Cys Val Leu Phe Pro Tyr Gly Gly Cys
305                 310                 315                 320

Gln Gly Asn Gly Asn Lys Phe Tyr Ser Glu Lys Glu Cys Arg Glu Tyr
                325                 330                 335

Cys Gly Val Pro Gly Asp Gly Asp Glu Glu Leu Leu Arg Phe Ser Asn
            340                 345                 350

<210> 50
<211> 337
<212> PRT
<213> Sus scrofa

<400> 50
Ala Val Ser Ala Ser Pro Val Leu Thr Leu Pro Asn Asp Ile Gln Val
  1               5                  10                  15

Gln Glu Asn Phe Asp Leu Ser Arg Ile Tyr Gly Lys Trp Phe His Val
             20                  25                  30

Ala Val Gly Ser Thr Cys Pro Trp Leu Lys Arg Phe Lys Asp Lys Met
         35                  40                  45

Thr Met Gly Thr Leu Met Leu Gly Glu Gly Ala Thr Glu Arg Glu Ile
     50                  55                  60

Ser Val Thr Lys Thr His Arg Arg Lys Gly Ile Cys Glu Val Ile Ser
 65                  70                  75                  80

Gly Ala Tyr Glu Lys Thr Ser Thr Asp Gly Lys Phe Leu Tyr His Lys
                 85                  90                  95

Ser Lys Trp Asn Ile Thr Met Glu Ser Tyr Val Val His Thr Asn Tyr
            100                 105                 110

Asp Glu Tyr Ala Ile Phe Leu Thr Lys Lys Phe Ser Arg Arg His Gly
        115                 120                 125

Pro Thr Leu Thr Ala Lys Leu Tyr Gly Arg Glu Pro Gln Leu Arg Glu
    130                 135                 140

Ser Leu Leu Glu Glu Phe Arg Glu Val Ala Leu Gly Val Gly Ile Pro
145                 150                 155                 160

Glu Asp Ser Ile Phe Thr Met Pro Asp Arg Gly Glu Cys Val Pro Gly
                165                 170                 175

Glu Gln Glu Pro Glu Pro Thr Leu Leu Ser Arg Ala Arg Arg Ala Val
            180                 185                 190

Leu Pro Gln Glu Glu Glu Gly Ser Gly Ala Gly Gln Pro Val Ala Asp
        195                 200                 205

Phe Ser Lys Lys Glu Asp Ser Cys Gln Leu Gly Tyr Ser Gln Gly Pro
    210                 215                 220

Cys Leu Gly Met Ile Lys Arg Tyr Phe Tyr Asn Gly Ser Ser Met Ala
225                 230                 235                 240

Cys Glu Thr Phe His Tyr Gly Gly Cys Met Gly Asn Gly Asn Asn Phe
                245                 250                 255

Val Ser Glu Lys Glu Cys Leu Gln Thr Cys Arg Thr Val Glu Ala Cys
            260                 265                 270

Ser Leu Pro Ile Val Ser Gly Pro Cys Arg Gly Phe Phe Gln Leu Trp
        275                 280                 285

Ala Phe Asp Ala Val Gln Gly Lys Cys Val Leu Phe Asn Tyr Gly Gly
    290                 295                 300

Cys Gln Gly Asn Gly Asn Gln Phe Tyr Ser Glu Lys Glu Cys Lys Glu
305                 310                 315                 320

Tyr Cys Gly Val Pro Gly Glu Glu Asp Glu Glu Leu Leu Arg Ser Ser
                325                 330                 335

Asn

<210> 51
<211> 349
<212> PRT
<213> Mesocricetus auratus

<400> 51
Met Leu Gly Leu Gly Thr Leu Phe Leu Leu Leu Ala Ala Cys Pro Ala
  1               5                  10                  15

Ser Arg Ala Asp Pro Val Pro Ala Leu Pro Asp Ile Gln Val Gln Glu
             20                  25                  30

Asn Phe Asn Glu Ser Arg Ile Tyr Gly Lys Trp Phe Asn Leu Ala Val
         35                  40                  45

Gly Ser Thr Cys Pro Trp Leu Ser Arg Ile Lys Asn Lys Met Ser Met
     50                  55                  60

Ser Thr Leu Val Leu Arg Glu Gly Ala Thr Gly Ala Glu Ile Ser Thr

65                  70                  75                  80

Thr Ser Thr Arg Trp Arg Arg Gly Val Cys Glu Glu Val Ser Gly Thr
                85                  90                  95

Tyr Glu Lys Thr Asp Met Asp Gly Lys Phe Leu Tyr His Lys Ser Lys
            100                 105                 110

Trp Asn Val Thr Leu Glu Ser Tyr Val Val His Thr Asn Tyr Asp Glu
        115                 120                 125

Tyr Ala Ile Phe Leu Thr Lys Lys Phe Ser Arg His His Gly Pro Thr
    130                 135                 140

Ile Thr Ala Lys Leu Tyr Gly Arg Glu Pro Gln Leu Arg Asp Ser Leu
145                 150                 155                 160

Leu Gln Glu Phe Arg Glu Val Ala Leu Ser Val Gly Ile Pro Glu Asn
                165                 170                 175

Ser Ile Val Phe Met Glu Asp Arg Gly Glu Cys Val Pro Gly Asp Leu
            180                 185                 190

Glu Gln Lys Ser Thr Ser Leu Leu Arg Ala Arg Arg Ala Val Leu Pro
        195                 200                 205

Gln Glu Asn Glu Gly Ser Gly Thr Gly Pro Leu Val Thr Asp Val Leu
    210                 215                 220

Lys Lys Glu Asp Ser Cys Gln Leu Ser Tyr Ser Glu Gly Pro Cys Leu
225                 230                 235                 240

Gly Met Ile Glu Lys Tyr Tyr Tyr Asn Gly Ala Ser Met Ala Cys Glu
                245                 250                 255

Thr Phe His Tyr Gly Gly Cys Leu Gly Asn Gly Asn Asn Phe Asn Ser
            260                 265                 270

Glu Lys Glu Cys Leu Gln Thr Cys Arg Thr Val Ala Ala Cys Ser Leu
        275                 280                 285

Pro Ile Val Gln Gly Pro Cys Arg Ala Tyr Val Glu Leu Trp Ala Phe
    290                 295                 300

Asp Ala Ala Gln Gly Lys Cys Val Gln Phe Ser Tyr Gly Gly Cys Lys
305                 310                 315                 320

Gly Asn Gly Asn Lys Phe Tyr Ser Glu Lys Glu Cys Lys Glu Tyr Cys
                325                 330                 335

Gly Val Pro Gly Asp Gly Tyr Glu Glu Leu Thr Arg Ser
            340                 345

<210> 52
<211> 349
<212> PRT

<213> Rattus norvegicus

<400> 52
Met Gln Gly Leu Gly Ala Leu Phe Leu Leu Leu Thr Ala Cys Leu Thr
1               5                   10                  15

Leu Lys Ala Asp Asn Val Pro Thr Leu Pro Asp Ile Gln Val Gln Glu
            20                  25                  30

Asn Phe Asn Glu Ala Arg Ile Tyr Gly Lys Trp Phe Asn Leu Ala Val
        35                  40                  45

Gly Ser Thr Cys Pro Trp Leu Arg Arg Ile Lys Asn Lys Met Ser Val
    50                  55                  60

Ser Thr Leu Val Leu Gln Glu Gly Ala Thr Glu Ala Glu Ile Ser Val
65                  70                  75                  80

Thr Ser Thr Gln Trp Arg Lys Gly Val Cys Glu Glu Ile Ser Gly Val
                85                  90                  95

Tyr Gln Lys Thr Asp Ile Asp Gly Lys Phe Leu Tyr His Lys Ser Lys
            100                 105                 110

Trp Asn Ala Thr Leu Glu Ser Tyr Val Val His Thr Asn Tyr Asp Glu
        115                 120                 125

Tyr Ala Ile Phe Leu Thr Lys Lys Phe Ser His Arg His Gly Pro Thr
    130                 135                 140

Ile Thr Ala Lys Leu Tyr Gly Arg Glu Pro Gln Leu Arg Asp Ser Leu
145                 150                 155                 160

Leu Gln Glu Phe Arg Glu Val Ala Leu Ser Val Gly Ile Pro Glu Asn
                165                 170                 175

Ser Ile Val Phe Met Ala Asp Arg Gly Glu Cys Val Pro Gly Asp Arg
            180                 185                 190

Glu Val Glu Ser Thr Ser Phe Ala Arg Ala Arg Arg Ala Val Leu Pro
        195                 200                 205

Gln Glu Asn Glu Gly Ser Gly Ser Glu Pro Leu Ile Thr Gly Thr Leu
    210                 215                 220

Lys Lys Glu Asp Ser Cys Gln Leu Asn Tyr Ser Glu Gly Pro Cys Leu
225                 230                 235                 240

Gly Met Gln Gln Lys Tyr Tyr Tyr Asn Gly Ala Ser Met Ala Cys Glu
                245                 250                 255

Thr Phe Gln Tyr Gly Gly Cys Leu Gly Asn Gly Asn Asn Phe Ala Ser
            260                 265                 270

Glu Lys Glu Cys Leu Gln Thr Cys Arg Thr Ile Ala Ala Cys Asn Leu
        275                 280                 285

Pro Ile Val Gln Gly Pro Cys Arg Ala Phe Ala Glu Leu Trp Ala Phe
    290                 295                 300

Asp Ala Ala Gln Gly Lys Cys Ile Gln Phe Ile Tyr Gly Gly Cys Lys
305                 310                 315                 320

Gly Asn Gly Asn Lys Phe Tyr Ser Glu Lys Glu Cys Lys Glu Tyr Cys
                325                 330                 335

Gly Val Pro Gly Asp Gly Tyr Glu Glu Leu Thr Arg Ser
            340                 345

<210> 53
<211> 352
<212> PRT
<213> Bos taurus

<400> 53
Met Arg Ser Leu Ser Gly Leu Leu Leu Leu Leu Thr Ala Cys Leu Ala
  1               5                  10                  15

Val Asn Ala Ser Ser Val Pro Thr Leu Pro Asp Asp Ile Gln Val Gln
             20                  25                  30

Glu Asn Phe Asp Leu Ser Arg Ile Tyr Gly Lys Trp Phe Asn Val Ala
         35                  40                  45

Val Gly Ser Thr Cys Pro Trp Leu Lys Arg Phe Lys Glu Lys Met Thr
     50                  55                  60

Met Ser Thr Val Val Leu Ile Ala Gly Pro Thr Ser Lys Glu Ile Ser
 65                  70                  75                  80

Val Thr Asn Thr His Arg Arg Lys Gly Val Cys Glu Ser Ile Ser Gly
                 85                  90                  95

Thr Tyr Glu Lys Thr Ser Ala Asp Gly Lys Phe Leu Tyr His Lys Ala
            100                 105                 110

Lys Trp Asn Ile Thr Met Glu Ser Tyr Val Val His Thr Asn Tyr Asp
        115                 120                 125

Glu Tyr Ala Ile Phe Leu Thr Lys Lys Leu Ser Arg Arg His Gly Pro
    130                 135                 140

Thr Ile Thr Val Lys Leu Tyr Gly Arg Glu Pro Gln Leu Arg Glu Ser
145                 150                 155                 160

Leu Leu Glu Glu Phe Arg Glu Val Ala Leu Gly Val Gly Ile Pro Glu
                165                 170                 175

Asp Ala Ile Phe Thr Met Pro Asp Arg Gly Glu Cys Val Pro Gly Glu
            180                 185                 190

Gln Asp Pro Val Pro Thr Pro Leu Ser Arg Ala Arg Arg Ala Val Leu
        195                 200                 205

Thr Gln Glu Glu Glu Gly Ser Gly Ala Gly Gln Pro Val Thr Asn Phe
    210                 215                 220

Ser Lys Lys Ala Asp Ser Cys Gln Leu Asp Tyr Ser Gln Gly Pro Cys
225                 230                 235                 240

Leu Gly Leu Phe Lys Arg Tyr Phe Tyr Asn Gly Thr Ser Met Ala Cys
                245                 250                 255

Glu Thr Phe Leu Tyr Gly Gly Cys Met Gly Asn Gly Asn Asn Phe Leu
            260                 265                 270

Ser Glu Lys Glu Cys Leu Gln Thr Cys Arg Thr Val Glu Ala Cys Asn
        275                 280                 285

Leu Pro Ile Val Gln Gly Pro Cys Arg Ser Tyr Ile Gln Leu Trp Ala
    290                 295                 300

Phe Asp Ala Val Lys Gly Lys Cys Val Arg Phe Ser Tyr Gly Gly Cys
305                 310                 315                 320

Lys Gly Asn Gly Asn Lys Phe Tyr Ser Glu Lys Glu Cys Lys Glu Tyr
                325                 330                 335

Cys Gly Ile Pro Gly Glu Ala Asp Glu Glu Leu Leu Arg Phe Ser Asn
            340                 345                 350

<210> 54
<211> 699
<212> PRT
<213> Homo sapiens

<400> 54

<210> 55
<211> 793
<212> PRT
<213> Homo sapiens

<400> 55
Met Gly Leu Pro Glu Pro Gly Pro Leu Arg Leu Leu Ala Leu Leu Leu
1               5                   10                  15

Leu Leu Leu Leu Leu Leu Leu Leu Arg Leu Gln His Leu Ala Ala Ala
            20                  25                  30

Ala Ala Asp Pro Leu Leu Gly Gly Gln Gly Pro Ala Lys Glu Cys Glu
        35                  40                  45

Lys Asp Gln Phe Gln Cys Arg Asn Glu Arg Cys Ile Pro Ser Val Trp
    50                  55                  60

Arg Cys Asp Glu Asp Asp Asp Cys Leu Asp His Ser Asp Glu Asp Asp
65                  70                  75                  80

Cys Pro Lys Lys Thr Cys Ala Asp Ser Asp Phe Thr Cys Asp Asn Gly
                85                  90                  95

His Cys Ile His Glu Arg Trp Lys Cys Asp Gly Glu Glu Glu Cys Pro
            100                 105                 110

Asp Gly Ser Asp Glu Ser Glu Ala Thr Cys Thr Lys Gln Val Cys Pro
        115                 120                 125

Ala Glu Lys Leu Ser Cys Gly Pro Thr Ser His Lys Cys Val Pro Ala
    130                 135                 140

Ser Trp Arg Cys Asp Gly Glu Lys Asp Cys Glu Gly Gly Ala Asp Glu
145                 150                 155                 160

Ala Gly Cys Ala Thr Trp Leu Asn Glu Cys Leu His Asn Asn Gly Gly
                165                 170                 175

Cys Ser His Ile Cys Thr Asp Leu Lys Ile Gly Phe Glu Cys Thr Cys
            180                 185                 190

Pro Ala Gly Phe Gln Leu Leu Asp Gln Lys Thr Cys Gly Asp Ile Asp
        195                 200                 205

Glu Cys Lys Asp Pro Asp Ala Cys Ser Gln Ile Cys Val Asn Tyr Lys
    210                 215                 220

Gly Tyr Phe Lys Cys Glu Cys Tyr Pro Gly Tyr Glu Met Asp Leu Leu
225                 230                 235                 240

Thr Lys Asn Cys Lys Ala Ala Gly Gly Lys Ser Pro Ser Leu Ile Phe
                245                 250                 255

Thr Asn Arg Tyr Glu Val Arg Arg Ile Asp Leu Val Lys Arg Asn Tyr
            260                 265                 270

Ser Arg Leu Ile Pro Met Leu Lys Asn Val Val Ala Leu Asp Val Glu
        275                 280                 285

Val Ala Thr Asn Arg Ile Tyr Trp Cys Asp Leu Ser Tyr Arg Lys Ile
    290                 295                 300

Tyr Ser Ala Tyr Met Asp Lys Ala Ser Asp Pro Lys Glu Gln Glu Val
305                 310                 315                 320

Leu Ile Asp Glu Gln Leu His Ser Pro Glu Gly Leu Ala Val Asp Trp
                325                 330                 335

Val His Lys His Ile Tyr Trp Thr Asp Ser Gly Asn Lys Thr Ile Ser
            340                 345                 350

Val Ala Thr Val Asp Gly Gly Arg Arg Arg Thr Leu Phe Ser Arg Asn
        355                 360                 365

Leu Ser Glu Pro Arg Ala Ile Ala Val Asp Pro Leu Arg Gly Phe Met
    370                 375                 380

Tyr Trp Ser Asp Trp Gly Asp Gln Ala Lys Ile Glu Lys Ser Gly Leu
385                 390                 395                 400

Asn Gly Val Asp Arg Gln Thr Leu Val Ser Asp Asn Ile Glu Trp Pro
                405                 410                 415

Asn Gly Ile Thr Leu Asp Leu Leu Ser Gln Arg Leu Tyr Trp Val Asp
            420                 425                 430

Ser Lys Leu His Gln Leu Ser Ser Ile Asp Phe Ser Gly Gly Asn Arg
        435                 440                 445

Lys Thr Leu Ile Ser Ser Thr Asp Phe Leu Ser His Pro Phe Gly Ile
    450                 455                 460

Ala Val Phe Glu Asp Lys Val Phe Trp Thr Asp Leu Glu Asn Glu Ala
465                 470                 475                 480

Ile Phe Ser Ala Asn Arg Leu Asn Gly Leu Glu Ile Ser Ile Leu Ala
                485                 490                 495

Glu Asn Leu Asn Asn Pro His Asp Ile Val Ile Phe His Glu Leu Lys
            500                 505                 510

Gln Pro Arg Ala Pro Asp Ala Cys Glu Leu Ser Val Gln Pro Asn Gly
        515                 520                 525

Gly Cys Glu Tyr Leu Cys Leu Pro Ala Pro Gln Ile Ser Ser His Ser
    530                 535                 540

Pro Lys Tyr Thr Cys Ala Cys Pro Asp Thr Met Trp Leu Gly Pro Asp
545                 550                 555                 560

Met Lys Arg Cys Tyr Arg Ala Pro Gln Ser Thr Ser Thr Thr Thr Leu
                565                 570                 575

Ala Ser Thr Met Thr Arg Thr Val Pro Ala Thr Thr Arg Ala Pro Gly
            580                 585                 590

Thr Thr Val His Arg Ser Thr Tyr Gln Asn His Ser Thr Glu Thr Pro
        595                 600                 605

Ser Leu Thr Ala Ala Val Pro Ser Ser Val Ser Val Pro Arg Ala Pro
    610                 615                 620

Ser Ile Ser Pro Ser Thr Leu Ser Pro Ala Thr Ser Asn His Ser Gln
625                 630                 635                 640

His Tyr Ala Asn Glu Asp Ser Lys Met Gly Ser Thr Val Thr Ala Ala
                645                 650                 655

Val Ile Gly Ile Ile Val Pro Ile Val Val Ile Ala Leu Leu Cys Met
            660                 665                 670

Ser Gly Tyr Leu Ile Trp Arg Asn Trp Lys Arg Lys Asn Thr Lys Ser
        675                 680                 685

Met Asn Phe Asp Asn Pro Val Tyr Arg Lys Thr Thr Glu Glu Glu Asp
    690                 695                 700

Glu Asp Glu Leu His Ile Gly Arg Thr Ala Gln Ile Gly His Val Tyr
705                 710                 715                 720

Pro Ala Ala Ile Ser Ser Phe Asp Arg Pro Leu Trp Ala Glu Pro Cys
                725                 730                 735

Leu Gly Glu Thr Arg Glu Pro Glu Asp Pro Ala Pro Ala Leu Lys Glu
            740                 745                 750

Leu Phe Val Leu Pro Gly Glu Pro Arg Ser Gln Leu His Gln Leu Pro
        755                 760                 765

Lys Asn Pro Leu Ser Glu Leu Pro Val Val Lys Ser Lys Arg Val Ala
    770                 775                 780

Leu Ser Leu Glu Asp Asp Gly Leu Pro
785                 790

<210> 56
<211> 963
<212> PRT
<213> Homo sapiens

<400> 56
Met Gly Leu Pro Glu Pro Gly Pro Leu Arg Leu Leu Ala Leu Leu Leu
1               5                   10                  15

Leu Leu Leu Leu Leu Leu Leu Leu Arg Leu Gln His Leu Ala Ala Ala
            20                  25                  30

Ala Ala Asp Pro Leu Leu Gly Gly Gln Gly Pro Ala Lys Glu Cys Glu
        35                  40                  45

Lys Asp Gln Phe Gln Cys Arg Asn Glu Arg Cys Ile Pro Ser Val Trp
    50                  55                  60

Arg Cys Asp Glu Asp Asp Asp Cys Leu Asp His Ser Asp Glu Asp Asp
65                  70                  75                  80

Cys Pro Lys Lys Thr Cys Ala Asp Ser Asp Phe Thr Cys Asp Asn Gly
                85                  90                  95

His Cys Ile His Glu Arg Trp Lys Cys Asp Gly Glu Glu Glu Cys Pro
            100                 105                 110

Asp Gly Ser Asp Glu Ser Glu Ala Thr Cys Thr Lys Gln Val Cys Pro
        115                 120                 125

Ala Glu Lys Leu Ser Cys Gly Pro Thr Ser His Lys Cys Val Pro Ala
    130                 135                 140

Ser Trp Arg Cys Asp Gly Glu Lys Asp Cys Glu Gly Gly Ala Asp Glu
145                 150                 155                 160

Ala Gly Cys Ala Thr Leu Cys Ala Pro His Glu Phe Gln Cys Gly Asn
                165                 170                 175

Arg Ser Cys Leu Ala Ala Val Phe Val Cys Asp Gly Asp Asp Asp Cys
            180                 185                 190

Gly Asp Gly Ser Asp Glu Arg Gly Cys Ala Asp Pro Ala Cys Gly Pro
        195                 200                 205

Arg Glu Phe Arg Cys Gly Gly Asp Gly Gly Gly Ala Cys Ile Pro Glu
    210                 215                 220

Arg Trp Val Cys Asp Arg Gln Phe Asp Cys Glu Asp Arg Ser Asp Glu
225                 230                 235                 240

Ala Ala Glu Leu Cys Gly Arg Pro Gly Pro Gly Ala Thr Ser Ala Pro
                245                 250                 255

Ala Ala Cys Ala Thr Val Ser Gln Phe Ala Cys Arg Ser Gly Glu Cys
            260                 265                 270

Val His Leu Gly Trp Arg Cys Asp Gly Asp Arg Asp Cys Lys Asp Lys
        275                 280                 285

Ser Asp Glu Ala Asp Cys Pro Leu Gly Thr Cys Arg Gly Asp Glu Phe
    290                 295                 300

Gln Cys Gly Asp Gly Thr Cys Val Leu Ala Ile Lys His Cys Asn Gln
305                 310                 315                 320

Glu Gln Asp Cys Pro Asp Gly Ser Asp Glu Ala Gly Cys Leu Gln Gly
                325                 330                 335

Leu Asn Glu Cys Leu His Asn Asn Gly Gly Cys Ser His Ile Cys Thr
            340                 345                 350

Asp Leu Lys Ile Gly Phe Glu Cys Thr Cys Pro Ala Gly Phe Gln Leu
        355                 360                 365

Leu Asp Gln Lys Thr Cys Gly Asp Ile Asp Glu Cys Lys Asp Pro Asp
    370                 375                 380

Ala Cys Ser Gln Ile Cys Val Asn Tyr Lys Gly Tyr Phe Lys Cys Glu
385                 390                 395                 400

Cys Tyr Pro Gly Tyr Glu Met Asp Leu Leu Thr Lys Asn Cys Lys Ala
                405                 410                 415

Ala Gly Gly Lys Ser Pro Ser Leu Ile Phe Thr Asn Arg Tyr Glu Val
            420                 425                 430

Arg Arg Ile Asp Leu Val Lys Arg Asn Tyr Ser Arg Leu Ile Pro Met
        435                 440                 445

Leu Lys Asn Val Val Ala Leu Asp Val Glu Val Ala Thr Asn Arg Ile
    450                 455                 460

Tyr Trp Cys Asp Leu Ser Tyr Arg Lys Ile Tyr Ser Ala Tyr Met Asp
465                 470                 475                 480

Lys Ala Ser Asp Pro Lys Glu Gln Glu Val Leu Ile Asp Glu Gln Leu
                485                 490                 495

His Ser Pro Glu Gly Leu Ala Val Asp Trp Val His Lys His Ile Tyr
            500                 505                 510

Trp Thr Asp Ser Gly Asn Lys Thr Ile Ser Val Ala Thr Val Asp Gly
        515                 520                 525

Gly Arg Arg Arg Thr Leu Phe Ser Arg Asn Leu Ser Glu Pro Arg Ala
    530                 535                 540

Ile Ala Val Asp Pro Leu Arg Gly Phe Met Tyr Trp Ser Asp Trp Gly
545                 550                 555                 560

Asp Gln Ala Lys Ile Glu Lys Ser Gly Leu Asn Gly Val Asp Arg Gln
                565                 570                 575

Thr Leu Val Ser Asp Asn Ile Glu Trp Pro Asn Gly Ile Thr Leu Asp
            580                 585                 590

Leu Leu Ser Gln Arg Leu Tyr Trp Val Asp Ser Lys Leu His Gln Leu
        595                 600                 605

Ser Ser Ile Asp Phe Ser Gly Gly Asn Arg Lys Thr Leu Ile Ser Ser
    610                 615                 620

Thr Asp Phe Leu Ser His Pro Phe Gly Ile Ala Val Phe Glu Asp Lys
625                 630                 635                 640

Val Phe Trp Thr Asp Leu Glu Asn Glu Ala Ile Phe Ser Ala Asn Arg
                645                 650                 655

Leu Asn Gly Leu Glu Ile Ser Ile Leu Ala Glu Asn Leu Asn Asn Pro
            660                 665                 670

His Asp Ile Val Ile Phe His Glu Leu Lys Gln Pro Arg Ala Pro Asp
        675                 680                 685

Ala Cys Glu Leu Ser Val Gln Pro Asn Gly Gly Cys Glu Tyr Leu Cys
    690                 695                 700

Leu Pro Ala Pro Gln Ile Ser Ser His Ser Pro Lys Tyr Thr Cys Ala
705                 710                 715                 720

Cys Pro Asp Thr Met Trp Leu Gly Pro Asp Met Lys Arg Cys Tyr Arg
                725                 730                 735

Ala Pro Gln Ser Thr Ser Thr Thr Thr Leu Ala Ser Thr Met Thr Arg
            740                 745                 750

Thr Val Pro Ala Thr Thr Arg Ala Pro Gly Thr Thr Val His Arg Ser
        755                 760                 765

Thr Tyr Gln Asn His Ser Thr Glu Thr Pro Ser Leu Thr Ala Ala Val
    770                 775                 780

Pro Ser Ser Val Ser Val Pro Arg Ala Pro Ser Ile Ser Pro Ser Thr
785                 790                 795                 800

Leu Ser Pro Ala Thr Ser Asn His Ser Gln His Tyr Ala Asn Glu Asp
                805                 810                 815

Ser Lys Met Gly Ser Thr Val Thr Ala Ala Val Ile Gly Ile Ile Val
            820                 825                 830

Pro Ile Val Val Ile Ala Leu Leu Cys Met Ser Gly Tyr Leu Ile Trp
        835                 840                 845

Arg Asn Trp Lys Arg Lys Asn Thr Lys Ser Met Asn Phe Asp Asn Pro
    850                 855                 860

Val Tyr Arg Lys Thr Thr Glu Glu Glu Asp Glu Asp Glu Leu His Ile
865                 870                 875                 880

Gly Arg Thr Ala Gln Ile Gly His Val Tyr Pro Ala Ala Ile Ser Ser
                885                 890                 895

Phe Asp Arg Pro Leu Trp Ala Glu Pro Cys Leu Gly Glu Thr Arg Glu
            900                 905                 910

Pro Glu Asp Pro Ala Pro Ala Leu Lys Glu Leu Phe Val Leu Pro Gly
        915                 920                 925

Glu Pro Arg Ser Gln Leu His Gln Leu Pro Lys Asn Pro Leu Ser Glu
    930                 935                 940

Leu Pro Val Val Lys Ser Lys Arg Val Ala Leu Ser Leu Glu Asp Asp
945                 950                 955                 960

Gly Leu Pro

<210> 57
<211> 949
<212> PRT
<213> Gallus gallus

<400> 57
Met Cys Arg Pro Ala Leu Ala Arg Leu Leu Leu Leu Gln Leu Leu Leu
1               5                   10                  15

Leu Lys Leu Tyr Leu Gly Lys Gly Ala Met Lys Glu Cys Asp Lys Asp
            20                  25                  30

Gln Phe Gln Cys Arg Asn Glu Arg Cys Ile Pro Ala Val Trp Ala Cys
        35                  40                  45

Asp Glu Asp Asn Asp Cys Ser Asp Asn Ser Asp Glu Gln Cys Ala Asp
    50                  55                  60

Cys Pro Lys Lys Thr Cys Ala Glu Thr Asp Phe Ala Cys Asp Asn Gly
65                  70                  75                  80

His Cys Ile Pro Asp Arg Trp Lys Cys Asp Gly Glu Glu Glu Cys Ser
                85                  90                  95

Asp Gly Ser Asp Glu Ser Glu Ala Ala Cys Thr Lys Gln Val Cys Pro
            100                 105                 110

Ala Glu Lys Ile Ser Cys Gly Asp Leu Ser Ser Asp Asn Lys Cys Ile
        115                 120                 125

Pro Ser Ser Trp Arg Cys Asp Gly Gln Lys Asp Cys Glu Ser Gly Ile
    130                 135                 140

Asp Glu Ala Gly Cys Ala Pro Ala Cys Ser Pro Asp Glu Phe Gln Cys
145                 150                 155                 160

Ser Asn Lys Thr Cys Ile Ser Ile Asn Phe Val Cys Asp Gly Tyr Asn
                165                 170                 175

Asn Cys Gly Asp Gly Ser Asp Glu Arg Ser Lys Lys Cys Ser Pro Leu
            180                 185                 190

Thr Cys Ser Pro Asn Glu Phe Gln Cys Asn Asn Ser Val Cys Ile Pro
        195                 200                 205

Gln Leu Trp Val Cys Asp Asn Gln Ala Asp Cys Glu Asp His Ser Asp
    210                 215                 220

Glu Ser Ile Glu Arg Cys Gly Tyr Asp Ala Lys Ala Phe Asn Thr Cys
225                 230                 235                 240

Ala Ala His Glu Phe Gln Asp Glu Cys Gly Asn Gly Glu Cys Ile Leu

                245                 250                 255

Leu Asn Trp Lys Cys Asp Gly Asp Glu Asp Cys Lys Asp Lys Ser Asp
            260                 265                 270

Glu Gln Asp Cys Pro Leu Val Thr Cys Arg Pro Asp Glu Phe Gln Cys
        275                 280                 285

Gly Asp Gly Thr Cys Ile His Gly Ala Lys Gln Cys Asp Lys Val His
    290                 295                 300

Asp Cys Pro Asp Gly Asp Asn Ser Asp Glu Ala Gly Cys Val Gln Glu
305                 310                 315                 320

Ser Ala Cys Glu Ser Pro Ser Lys Phe Gln Cys Lys Ser Gly Glu Cys
                325                 330                 335

Ile Asp Gly Gly Lys Val Cys Asp Leu His Arg Asp Cys Arg Asp Trp
            340                 345                 350

Ser Asp Glu Pro Leu Lys Glu Cys Gly Ile Asn Glu Cys Ser Leu Asn
        355                 360                 365

Asn Gly Pro Ala Gly Cys Ser His Ile Cys Lys Asp Leu Lys Ile Gly
    370                 375                 380

Tyr Glu Cys Glu Cys Pro Pro Gly Tyr Lys Leu Leu Asp Lys Lys Thr
385                 390                 395                 400

Cys Gly Asp Ile Asp Glu Cys Glu Asn Pro Asp Ala Cys Ser Gln Ile
                405                 410                 415

Cys Ile Asn Tyr Lys Gly Asp Tyr Lys Cys Glu Cys Tyr Glu Gly Tyr
            420                 425                 430

Ile Phe Glu Met Asp Thr Leu Ser Lys Asn Cys Lys Ala Val Gly Lys
        435                 440                 445

Ser Pro Tyr Leu Ile Phe Thr Asn Arg His Glu Val Arg Lys Ile Asp
    450                 455                 460

Leu Val Lys Arg Asp Tyr Ser Arg Ile Ile Pro Met Leu Lys Asn Val
465                 470                 475                 480

Val Ala Leu Asp Val Glu Val Ala Thr Asn Arg Ile Tyr Trp Glu Gln
                485                 490                 495

Cys Asp Leu Phe Tyr Arg Lys Ile Tyr Ser Ala Tyr Ile Asp Lys Ala
            500                 505                 510

Ser Asp Thr Ala Glu Gln Val Ile Leu Ile Asp Ser Gln Leu Asn Ser
        515                 520                 525

Pro Glu Gly Val Ala Ile Asp Trp Val His Lys Asn Ile Tyr Trp Thr
    530                 535                 540

Asp Ser Gly Asn Lys Thr Ile Ser Val Ala Thr Ala Pro Leu Asp Gly

545                 550                 555                 560

Ser Arg Arg Arg Thr Val Phe Asn Ser Asp Leu Ser Glu Pro Arg Ala
                565                 570                 575

Ile Ala Val Asp Pro Thr Arg Arg Phe Met Tyr Trp Ser Asp Trp Gly
            580                 585                 590

Asp Lys Ala Lys Ile Glu Lys Ala Gly Leu Asn Gly Val Gly Arg Gln
        595                 600                 605

Val Leu Val Ser Asp Asn Ile Glu Trp Pro Ile Asp Asn Gly Ile Thr
    610                 615                 620

Leu Asp Leu Leu Asn Gln Arg Leu Tyr Trp Val Asp Ser Lys Leu His
625                 630                 635                 640

Ser Leu Ser Cys Ile Asp Phe Asn Gly Ser Asn Arg Glu Val Leu Ile
                645                 650                 655

Ser Ser Ile Asp Asp Leu Ser His Pro Phe Gly Leu Ala Val Phe Glu
            660                 665                 670

Asp Arg Val Phe Trp Thr Asp Leu His Asp Glu Asn Glu Ala Ile Phe
        675                 680                 685

Ser Ala Asn Arg Leu Ser Gly Leu Asp Ile Ser Val Leu Ala Glu Asn
    690                 695                 700

Leu Asn Asn Pro His Asp Ile Val Val Phe His Glu Leu Lys Gln Pro
705                 710                 715                 720

Lys Ala Pro Asp Ser Cys Glu Leu Ser Pro Gln Pro Asn Gly Gly Cys
                725                 730                 735

Glu Tyr Leu Cys Leu Pro Tyr Arg Ala Pro His Ile Ser Pro Arg Ser
            740                 745                 750

Pro Lys Phe Thr Cys Ala Cys Pro Asp Asn Met Trp Leu Gly Pro Asp
        755                 760                 765

Met Lys Lys Cys Tyr Lys Glu Leu Pro Thr Thr Pro Ala Thr Val Glu
    770                 775                 780

Val Pro Thr Thr Thr Thr Ser His Pro Ala Ala Thr Ser Thr Val Thr
785                 790                 795                 800

Val Thr Gly Ser Ser Pro Ala Asn Thr Thr Thr Ala Val Ile Pro Arg
                805                 810                 815

Ala Val Ser Glu Ala Thr Thr Ala Ile Pro Ser Ser His Ser Thr Thr
            820                 825                 830

Ser Leu Leu Ile Asp Ser Glu Met Thr Thr Gly Asn Ser Asn Leu Ser
        835                 840                 845

Gln His Tyr Ser Asn Asn Gly Gln Gly Phe Asp Ser Thr Val Thr Ala

850                 855                 860

Ala Val Met Asn Ile Gly Ile Val Ile Pro Val Val Val Ile Gly Leu
865                 870                 875                 880

Leu Cys Met Gly Gly Tyr Leu Ile Trp Arg Asn Trp Lys Arg Lys Asn
                885                 890                 895

Thr Lys Ser Met Asn Phe Asp Asn Pro Val Tyr Arg Lys Thr Thr Glu
            900                 905                 910

Glu Glu Asp Glu Asp Glu Ile His Ile Gly Arg Thr Ala Gln Ile Gly
        915                 920                 925

Lys Glu His Val Tyr Pro Ala Arg Val Ala Leu Ser Leu Glu Asp Asp
    930                 935                 940

Gly Leu Pro Asp Pro
945

<210> 58
<211> 269
<212> PRT
<213> Canis familiaris

<400> 58
Met Ala Pro Leu Ile Arg Met Leu Asp Leu Leu Gly Thr Leu Ser Pro
1               5                   10                  15

Lys Val Gly Ile Val Gly Gly Cys Lys Val Pro Ala Arg Arg Tyr Pro
            20                  25                  30

Trp Gln Val Ser Leu Arg Phe His Gly Met Gly Ser Gly Gln Trp Gln
        35                  40                  45

His Ile Cys Gly Gly Ser Leu Ile His Pro Gln Trp Val Leu Thr Ala
    50                  55                  60

Ala His Cys Val Glu Leu Glu Gly Leu Glu Ala Ala Thr Leu Arg Val
65                  70                  75                  80

Gln Val Gly Gln Leu Arg Leu Tyr Asp His Asp Gln Leu Cys Asn Val
                85                  90                  95

Thr Glu Ile Ile Arg His Pro Asn Phe Asn Met Ser Trp Tyr Gly Trp
            100                 105                 110

Asp Thr Ala Asp Ile Ala Leu Leu Lys Leu Glu Ala Pro Leu Thr Leu
        115                 120                 125

Ser Glu Asp Val Asn Leu Val Ser Leu Pro Ser Pro Ser Leu Ile Val
    130                 135                 140

Pro Pro Gly Met Leu Cys Trp Val Thr Gly Trp Gly Asp Ile Ala Asp
145                 150                 155                 160

His Thr Pro Leu Pro Pro Pro Tyr His Leu Gln Glu Val Glu Val Pro
                165                 170                 175

Ile Val Gly Asn Arg Glu Cys Asn Cys His Tyr Gln Thr Ile Leu Glu
            180                 185                 190

Gln Asp Asp Glu Val Ile Lys Gln Asp Met Leu Cys Ala Gly Ser Glu
        195                 200                 205

Gly His Asp Ser Cys Gln Met Asp Ser Gly Gly Pro Leu Val Cys Arg
    210                 215                 220

Trp Lys Cys Thr Trp Ile Gln Val Gly Val Val Ser Trp Gly Tyr Gly
225                 230                 235                 240

Cys Gly Tyr Asn Leu Pro Gly Val Tyr Ala Arg Val Thr Ser Tyr Val
                245                 250                 255

Ser Trp Ile His Gln His Ile Pro Leu Ser Pro Gly Pro
            260                 265


<210> 59
<211> 274
<212> PRT
<213> Rattus norvegicus

<400> 59
Met Leu Lys Leu Leu Leu Leu Leu Ala Leu Ser Pro Leu Ala Ser Leu
  1               5                  10                  15

Val His Ala Ala Pro Cys Pro Val Lys Gln Arg Val Gly Ile Val Gly
             20                  25                  30

Gly Arg Glu Ala Ser Glu Ser Lys Trp Pro Trp Gln Val Ser Leu Arg
         35                  40                  45

Phe Lys Phe Ser Phe Trp Met His Phe Cys Gly Gly Ser Leu Ile His
     50                  55                  60

Pro Gln Trp Val Leu Thr Ala Ala His Cys Val Gly Leu His Ile Lys
 65                  70                  75                  80

Ser Pro Glu Leu Phe Arg Val Gln Leu Arg Glu Gln Tyr Leu Tyr Tyr
                 85                  90                  95

Ala Asp Gln Leu Leu Thr Val Asn Arg Thr Val Val His Pro His Tyr
            100                 105                 110

Tyr Thr Val Glu Asp Gly Ala Asp Ile Ala Leu Leu Glu Leu Glu Ile
        115                 120                 125

Pro Val Asn Val Ser Thr His Ile His Pro Ile Ser Leu Pro Pro Ala
    130                 135                 140

Ser Glu Thr Phe Pro Ser Gly Thr Ser Cys Trp Val Thr Gly Trp Gly
145                 150                 155                 160

Asp Ile Asp Ser Asp Glu Pro Leu Leu Pro Pro Tyr Pro Leu Lys Gln
                165                 170                 175

Val Lys Val Pro Ile Val Glu Asn Ser Leu Cys Asp Arg Lys Tyr His
            180                 185                 190

Thr Gly Leu Tyr Thr Gly Asp Asp Val Pro Ile Val Gln Asp Gly Met
        195                 200                 205

Leu Cys Ala Gly Asn Thr Arg Ser Asp Ser Cys Gln Gly Asp Ser Gly
    210                 215                 220

Gly Pro Leu Val Cys Lys Val Lys Gly Thr Trp Leu Gln Ala Gly Val
225                 230                 235                 240

Val Ser Trp Gly Glu Gly Cys Ala Glu Ala Asn Arg Pro Gly Ile Tyr
                245                 250                 255

Thr Arg Val Thr Tyr Tyr Leu Asp Trp Ile His Arg Tyr Val Pro Gln
            260                 265                 270

Arg Ser

<210> 60
<211> 273
<212> PRT
<213> Rattus norvegicus

<400> 60
Met Leu Lys Leu Leu Leu Leu Thr Leu Pro Leu Leu Ser Ser Leu Val
  1               5                  10                  15

His Ala Ala Pro Ser Leu Ala Met Pro Arg Glu Gly Ile Val Gly Gly
             20                  25                  30

Gln Glu Ala Ser Gly Asn Lys Trp Pro Trp Gln Val Ser Leu Arg Val
         35                  40                  45

Asn Asp Thr Tyr Trp Met His Phe Cys Gly Gly Ser Leu Ile His Pro
     50                  55                  60

Gln Trp Val Leu Thr Ala Ala His Cys Val Gly Pro Asn Lys Ala Asp
 65                  70                  75                  80

Pro Asn Lys Leu Arg Val Gln Leu Arg Lys Gln Tyr Leu Tyr Tyr His
                 85                  90                  95

Asp His Leu Leu Thr Val Ser Gln Ile Ile Ser His Pro Asp Phe Tyr
            100                 105                 110

Ile Ala Gln Asp Gly Ala Asp Ile Ala Leu Leu Lys Leu Thr Asn Pro
        115                 120                 125

Val Asn Ile Thr Ser Asn Val His Thr Val Ser Leu Pro Pro Ala Ser

130                 135                 140

Glu Thr Phe Pro Ser Gly Thr Leu Cys Trp Val Thr Gly Trp Gly Asn
145                 150                 155                 160

Ile Asn Asn Asp Val Ser Leu Pro Pro Pro Phe Pro Leu Glu Glu Val
                165                 170                 175

Gln Val Pro Ile Val Glu Asn Arg Leu Cys Asp Leu Lys Tyr His Lys
            180                 185                 190

Gly Leu Asn Thr Gly Asp Asn Val His Ile Val Arg Asp Asp Met Leu
        195                 200                 205

Cys Ala Gly Asn Glu Gly His Asp Ser Cys Gln Gly Asp Ser Gly Gly
    210                 215                 220

Pro Leu Val Cys Lys Val Glu Asp Thr Trp Leu Gln Ala Gly Val Val
225                 230                 235                 240

Ser Trp Gly Glu Gly Cys Ala Gln Pro Asn Arg Pro Gly Ile Tyr Thr
                245                 250                 255

Arg Val Thr Tyr Tyr Leu Asp Trp Ile Tyr Arg Tyr Val Pro Lys Tyr
            260                 265                 270

Phe

<210> 61
<211> 276
<212> PRT
<213> Mus musculus

<400> 61
Met Leu Lys Arg Arg Leu Leu Leu Leu Trp Ala Leu Ser Leu Leu Ala
1               5                   10                  15

Ser Leu Val Tyr Ser Ala Pro Arg Pro Ala Asn Gln Arg Val Gly Ile
            20                  25                  30

Val Gly Gly His Glu Ala Ser Glu Ser Lys Trp Pro Trp Gln Val Ser
        35                  40                  45

Leu Arg Phe Lys Leu Asn Tyr Trp Ile His Phe Cys Gly Gly Ser Leu
    50                  55                  60

Ile His Pro Gln Trp Val Leu Thr Ala Ala His Cys Val Gly Pro His
65                  70                  75                  80

Ile Lys Ser Pro Gln Leu Phe Arg Val Gln Leu Arg Glu Gln Tyr Leu
                85                  90                  95

Tyr Tyr Gly Asp Gln Leu Leu Ser Leu Asn Arg Ile Val Val His Pro
            100                 105                 110

His Tyr Tyr Thr Ala Glu Gly Gly Ala Asp Val Ala Leu Leu Glu Leu
        115                 120                 125

Glu Val Pro Val Asn Val Ser Thr His Ile His Pro Ile Ser Leu Pro
    130                 135                 140

Pro Ala Ser Glu Thr Phe Pro Pro Gly Thr Ser Cys Trp Val Thr Gly
145                 150                 155                 160

Trp Gly Asp Ile Asp Asn Asp Glu Pro Leu Pro Pro Pro Tyr Pro Leu
                165                 170                 175

Lys Gln Val Lys Val Pro Ile Val Glu Asn Ser Leu Cys Asp Arg Lys
            180                 185                 190

Tyr His Thr Gly Leu Tyr Thr Gly Asp Asp Phe Pro Ile Val His Asp
        195                 200                 205

Gly Met Leu Cys Ala Gly Asn Thr Arg Arg Asp Ser Cys Gln Gly Asp
    210                 215                 220

Ser Gly Gly Pro Leu Val Cys Lys Val Lys Gly Thr Trp Leu Gln Ala
225                 230                 235                 240

Gly Val Val Ser Trp Gly Glu Gly Cys Ala Gln Pro Asn Lys Pro Gly
                245                 250                 255

Ile Tyr Thr Arg Val Thr Tyr Tyr Leu Asp Trp Ile His Arg Tyr Val
            260                 265                 270

Pro Glu His Ser
        275

<210> 62
<211> 275
<212> PRT
<213> Sus scrofa

<400> 62
Met Leu Asn Leu Leu Val Leu Ala Leu Pro Leu Leu Val Ser Leu Val
1               5                   10                  15

His Thr Ala Pro Ala Pro Gly Gln Ala Leu Glu Arg Ala Gly Ile Val
            20                  25                  30

Gly Gly Lys Glu Ala Pro Gly His Lys Trp Pro Trp Gln Val Ser Leu
        35                  40                  45

Arg Cys Leu Asp Gln Tyr Trp Lys His Phe Cys Gly Gly Ser Leu Ile
    50                  55                  60

His Pro Gln Trp Val Leu Thr Ala Ala His Cys Phe Gly Pro Glu Lys
65                  70                  75                  80

Ala Asp Pro Leu Tyr Ile Arg Val Gln Leu Gly Glu Gln His Leu Tyr
                85                  90                  95

Tyr Gln Asp Arg Leu Leu Leu Val Ser Arg Ile Ile Val His Pro Asn
            100                 105                 110

Tyr Tyr Asp Glu Val Asn Gly Ala Asp Ile Ala Leu Leu Glu Leu Glu
        115                 120                 125

Asp Pro Val Asn Leu Ser Ser His Val Gln Pro Val Thr Leu Pro Pro
    130                 135                 140

Ala Ser Glu Thr Phe Pro Lys Gly Thr Arg Cys Trp Val Thr Gly Trp
145                 150                 155                 160

Gly Asp Val His Ser Gly Trp Pro Leu Pro Pro Pro Tyr Pro Leu Lys
                165                 170                 175

Gln Val Arg Val Pro Ile Val Glu Asn Ser Glu Cys Asp Met Gln Tyr
            180                 185                 190

His Leu Gly Leu Ser Thr Gly Asp Asn Ile Pro Ile Val Arg Asp Asp
        195                 200                 205

Met Leu Cys Ala Gly Ser Glu Gly His Asp Ser Cys Gln Gly Asp Ser
    210                 215                 220

Gly Gly Pro Leu Val Cys Arg Val Asn Gly Thr Trp Leu Gln Ala Gly
225                 230                 235                 240

Val Val Ser Trp Gly Glu Gly Cys Ala Leu Pro Asn Arg Pro Gly Ile
                245                 250                 255

Tyr Thr Arg Val Thr His Tyr Leu Asp Trp Ile His Gln Cys Ile Pro
            260                 265                 270

Arg Glu Ser
        275


<210> 63
<211> 50
<212> PRT
<213> Mus musculus

<400> 63
Met Leu Leu Pro Ala Thr Met Ser Asp Lys Pro Asp Met Ala Glu Ile
  1               5                  10                  15

Glu Lys Phe Asp Lys Ser Lys Leu Lys Lys Thr Glu Thr Gln Glu Lys
             20                  25                  30

Asn Pro Leu Pro Ser Lys Glu Thr Ile Glu Gln Glu Lys Gln Ala Gly
         35                  40                  45

Glu Ser
     50

<210> 64
<211> 44
<212> PRT
<213> Homo sapiens

<400> 64


<210> 65
<211> 46
<212> PRT
<213> Sus scrofa

<220>
<221> VARIANT
<222> (4)
<223> Wherein Xaa is any amino acid.

<400> 65
Ala Thr Met Xaa Asp Lys Pro Asp Met Ala Glu Ile Glu Lys Phe Asp
1               5                   10                  15

Lys Ser Lys Leu Lys Lys Thr Glu Thr Gln Glu Lys Asn Pro Leu Pro
            20                  25                  30

Ser Lys Glu Thr Ile Glu Gln Glu Lys Gln Ala Gly Glu Ser
        35                  40                  45


<210> 66
<211> 43
<212> PRT
<213> Equus caballus

<400> 66
Ser Asp Lys Pro Asp Met Ala Glu Ile Glu Lys Phe Asp Lys Ser Lys
1               5                   10                  15

Leu Lys Lys Thr Glu Thr Gln Glu Lys Asn Pro Leu Pro Ser Lys Glu
            20                  25                  30

Thr Ile Glu Gln Glu Lys Gln Ala Gly Glu Ser
        35                  40


<210> 67
<211> 43
<212> PRT
<213> Orycctolagus cuniculus

<400> 67
Ala Asp Lys Pro Asp Met Ala Glu Ile Glu Lys Phe Asp Lys Ser Lys
1               5                   10                  15

Leu Lys Lys Thr Glu Thr Gln Glu Lys Asn Pro Leu Pro Ser Lys Glu
            20                  25                  30

Thr Ile Glu Gln Glu Lys Gln Ala Gly Glu Ser
        35                  40


<210> 68
<211> 415
<212> PRT
<213> Homo sapiens

<400> 68
Met Pro Thr Thr Ile Glu Arg Glu Phe Glu Glu Leu Asp Thr Gln Arg
1               5                   10                  15

Arg Trp Gln Pro Leu Tyr Leu Glu Ile Arg Asn Glu Ser His Asp Tyr
            20                  25                  30

Pro His Arg Val Ala Lys Phe Pro Glu Asn Arg Asn Arg Asn Arg Tyr
        35                  40                  45

Arg Asp Val Ser Pro Tyr Asp His Ser Arg Val Lys Leu Gln Asn Ala
    50                  55                  60

Glu Asn Asp Tyr Ile Asn Ala Ser Leu Val Asp Ile Glu Glu Ala Gln
65                  70                  75                  80

Arg Ser Tyr Ile Leu Thr Gln Gly Pro Leu Pro Asn Thr Cys Cys His
                85                  90                  95

Phe Trp Leu Met Val Trp Gln Gln Lys Thr Lys Ala Val Val Met Leu
            100                 105                 110

Asn Arg Ile Val Glu Lys Glu Ser Val Lys Cys Ala Gln Tyr Trp Pro
        115                 120                 125

Thr Asp Asp Gln Glu Met Leu Phe Lys Glu Thr Gly Phe Ser Val Lys
    130                 135                 140

Leu Leu Ser Glu Asp Val Lys Ser Tyr Tyr Thr Val His Leu Leu Gln
145                 150                 155                 160

Leu Glu Asn Ile Asn Ser Gly Glu Thr Arg Thr Ile Ser His Phe His
                165                 170                 175

Tyr Thr Thr Trp Pro Asp Phe Gly Val Pro Glu Ser Pro Ala Ser Phe
            180                 185                 190

Leu Asn Phe Leu Phe Lys Val Arg Glu Ser Gly Ser Leu Asn Pro Asp
        195                 200                 205

His Gly Pro Ala Val Ile His Cys Ser Ala Gly Ile Gly Arg Ser Gly
    210                 215                 220

Thr Phe Ser Leu Val Asp Thr Cys Leu Val Leu Met Glu Lys Gly Asp
225                 230                 235                 240

Asp Ile Asn Ile Lys Gln Val Leu Leu Asn Met Arg Lys Tyr Arg Met
                245                 250                 255

Gly Leu Ile Gln Thr Pro Asp Gln Leu Arg Phe Ser Tyr Met Ala Ile
            260                 265                 270

Ile Glu Gly Ala Lys Cys Ile Lys Gly Asp Ser Ser Ile Gln Lys Arg
        275                 280                 285

Trp Lys Glu Leu Ser Lys Glu Asp Leu Ser Pro Ala Phe Asp His Ser
    290                 295                 300

Pro Asn Lys Ile Met Thr Glu Lys Tyr Asn Gly Asn Arg Ile Gly Leu
305                 310                 315                 320

Glu Glu Glu Lys Leu Thr Gly Asp Arg Cys Thr Gly Leu Ser Ser Lys
                325                 330                 335

Met Gln Asp Thr Met Glu Glu Asn Ser Glu Ser Ala Leu Arg Lys Arg
            340                 345                 350

Ile Arg Glu Asp Arg Lys Ala Thr Thr Ala Gln Lys Val Gln Gln Met
        355                 360                 365

Lys Gln Arg Leu Asn Glu Asn Glu Arg Lys Arg Lys Arg Trp Leu Tyr
    370                 375                 380

Trp Gln Pro Ile Leu Thr Lys Met Gly Phe Met Ser Val Ile Leu Val
385                 390                 395                 400

Gly Ala Phe Val Gly Trp Arg Leu Phe Phe Gln Gln Asn Ala Leu
                405                 410                 415


<210> 69
<211> 382
<212> PRT
<213> Mus musculus

<400> 69
Met Ser Ala Thr Ile Glu Arg Glu Phe Glu Glu Leu Asp Ala Gln Cys
  1               5                  10                  15

Arg Trp Gln Pro Leu Tyr Leu Glu Ile Arg Asn Glu Ser His Asp Tyr
             20                  25                  30

Pro His Arg Val Ala Lys Phe Pro Glu Asn Arg Asn Arg Asn Arg Tyr
         35                  40                  45

Arg Asp Val Ser Pro Tyr Asp His Ser Arg Val Lys Leu Gln Ser Thr
     50                  55                  60

Glu Asn Asp Tyr Ile Asn Ala Ser Leu Val Asp Ile Glu Glu Ala Gln
65                  70                  75                  80

Arg Ser Tyr Ile Leu Thr Gln Gly Pro Leu Pro Asn Thr Cys Cys His
                85                  90                  95

Phe Trp Leu Met Val Trp Gln Gln Lys Thr Lys Ala Val Val Met Leu
            100                 105                 110

Asn Arg Thr Val Glu Lys Glu Ser Val Lys Cys Ala Gln Tyr Trp Pro
        115                 120                 125

Thr Asp Asp Arg Glu Met Val Phe Lys Glu Thr Gly Phe Ser Val Lys
    130                 135                 140

Leu Leu Ser Glu Asp Val Lys Ser Tyr Tyr Thr Val His Leu Leu Gln
145                 150                 155                 160

Leu Glu Asn Ile Asn Thr Gly Glu Thr Arg Thr Ile Ser His Phe His
                165                 170                 175

Tyr Thr Thr Trp Pro Asp Phe Gly Val Pro Glu Ser Pro Ala Ser Phe
            180                 185                 190

Leu Asn Phe Leu Phe Lys Val Arg Glu Ser Gly Cys Leu Thr Pro Asp
        195                 200                 205

His Gly Pro Ala Val Ile His Cys Ser Ala Gly Ile Gly Arg Ser Gly
    210                 215                 220

Thr Phe Ser Leu Val Asp Thr Cys Leu Val Leu Met Glu Lys Gly Glu
225                 230                 235                 240

Asp Val Asn Val Lys Gln Leu Leu Leu Asn Met Arg Lys Tyr Arg Met
                245                 250                 255

Gly Leu Ile Gln Thr Pro Asp Gln Leu Arg Phe Ser Tyr Met Ala Ile
            260                 265                 270

Ile Glu Gly Ala Lys Tyr Thr Lys Gly Asp Ser Asn Ile Gln Lys Arg
        275                 280                 285

Trp Lys Glu Leu Ser Lys Glu Asp Leu Ser Pro Ile Cys Asp His Ser
    290                 295                 300

Gln Asn Arg Val Met Val Glu Lys Tyr Asn Gly Lys Arg Ile Gly Ser
305                 310                 315                 320

Glu Asp Glu Lys Leu Thr Gly Leu Pro Ser Lys Val Gln Asp Thr Val
                325                 330                 335

Glu Glu Ser Ser Glu Ser Ile Leu Arg Lys Arg Ile Arg Glu Asp Arg
            340                 345                 350

Lys Ala Thr Thr Ala Gln Lys Val Gln Gln Met Lys Gln Arg Leu Asn
        355                 360                 365

Glu Thr Glu Arg Lys Arg Lys Arg Pro Arg Leu Thr Asp Thr
    370                 375                 380

<210> 70
<211> 363
<212> PRT
<213> Rattus norvegicus

<400> 70
Met Ser Ala Thr Ile Glu Arg Glu Phe Glu Glu Leu Asp Ala Gln Cys
  1               5                  10                  15

Arg Trp Gln Pro Leu Tyr Leu Glu Ile Arg Asn Glu Ser His Asp Tyr
             20                  25                  30

Pro His Arg Val Ala Lys Phe Pro Glu Asn Arg Asn Arg Asn Arg Tyr
         35                  40                  45

Arg Asp Val Ser Pro Tyr Asp His Ser Arg Val Lys Leu Gln Ser Ala
     50                  55                  60

Glu Asn Asp Tyr Ile Asn Ala Ser Leu Val Asp Ile Glu Glu Ala Gln
 65                  70                  75                  80

Arg Ser Tyr Ile Leu Thr Gln Gly Pro Leu Pro Asn Thr Cys Cys His
                 85                  90                  95

Phe Trp Leu Met Val Trp Gln Gln Lys Thr Arg Ala Val Val Met Leu
            100                 105                 110

Asn Arg Thr Val Glu Lys Glu Ser Val Lys Cys Ala Gln Tyr Trp Pro
        115                 120                 125

Thr Asp Asp Arg Glu Met Val Phe Lys Glu Thr Gly Phe Ser Val Lys
    130                 135                 140

Leu Leu Ser Glu Asp Val Lys Ser Tyr Tyr Thr Val His Leu Leu Gln
145                 150                 155                 160

Leu Glu Asn Ile Asn Ser Gly Glu Thr Arg Thr Ile Ser His Phe His
                165                 170                 175

Tyr Thr Thr Trp Pro Asp Phe Gly Val Pro Glu Ser Pro Ala Ser Phe
            180                 185                 190

Leu Asn Phe Leu Phe Lys Val Arg Glu Ser Gly Ser Leu Asn Pro Asp
        195                 200                 205

His Gly Pro Ala Val Ile His Cys Ser Ala Gly Ile Gly Arg Ser Gly
    210                 215                 220

Thr Phe Ser Leu Val Asp Thr Cys Leu Val Leu Met Glu Lys Gly Glu
225                 230                 235                 240

Asp Val Asn Val Lys Gln Ile Leu Leu Ser Met Arg Lys Tyr Arg Met

245                 250                 255

Gly Leu Ile Gln Thr Pro Asp Gln Leu Arg Phe Ser Tyr Met Ala Ile
            260                 265                 270

Ile Glu Gly Ala Lys Tyr Thr Lys Gly Asp Ser Asn Ile Gln Asn Arg
        275                 280                 285

Thr Met Thr Glu Lys Tyr Asn Gly Lys Arg Ile Gly Ser Glu Asp Glu
    290                 295                 300

Lys Leu Thr Gly Leu Ser Ser Lys Val Pro Asp Thr Val Glu Glu Ser
305                 310                 315                 320

Ser Glu Ser Ile Leu Arg Lys Arg Ile Arg Glu Asp Arg Lys Ala Thr
                325                 330                 335

Thr Ala Gln Lys Val Gln Gln Met Arg Gln Arg Leu Asn Glu Thr Glu
            340                 345                 350

Arg Lys Arg Lys Arg Pro Arg Leu Thr Asp Thr
        355                 360


<210> 71
<211> 434
<212> PRT
<213> Gallus gallus

<400> 71
Met Glu Ile Glu Lys Glu Phe His Arg Leu Asp Gln Ala Ala Ser Trp
1               5                   10                  15

Ala Ala Ile Tyr Gln Asp Ile Arg His Glu Ala Ser Asp Phe Pro Cys
            20                  25                  30

Lys Val Ala Lys His Pro Arg Asn Lys Asn Arg Asn Arg Tyr Arg Asp
        35                  40                  45

Val Ser Pro Phe Asp His Ser Arg Ile Lys Leu Asn Gln Gly Asp Asn
    50                  55                  60

Asp Tyr Ile Asn Ala Ser Leu Ile Lys Met Glu Glu Ala Gln Arg Ser
65                  70                  75                  80

Tyr Ile Leu Thr Gln Gly Pro Leu Pro Asn Thr Cys Gly His Phe Trp
                85                  90                  95

Glu Met Val Trp Glu Gln Lys Ser Arg Gly Val Val Met Leu Asn Arg
            100                 105                 110

Val Met Glu Lys Gly Ser Ile Lys Cys Ala Gln Tyr Trp Pro Arg Lys
        115                 120                 125

Glu Glu Lys Glu Met Phe Phe Glu Asp Thr Asn Leu Lys Leu Thr Leu
    130                 135                 140

Ile Ser Glu Asp Ile Lys Ser Tyr Tyr Thr Val Arg Gln Leu Glu Leu
145                 150                 155                 160

Glu Asn Leu Thr Thr Gln Glu Thr Arg Glu Ile Leu His Phe His Tyr
                165                 170                 175

Thr Thr Trp Pro Asp Phe Gly Val Pro Glu Ser Pro Ala Ser Phe Leu
            180                 185                 190

Asn Phe Leu Phe Lys Val Arg Glu Ser Gly Ser Leu Asn Pro Glu Tyr
        195                 200                 205

Gly Pro Val Val Val His Cys Ser Ala Gly Ile Gly Arg Ser Gly Thr
    210                 215                 220

Phe Cys Leu Val Asp Thr Cys Leu Leu Leu Met Asp Lys Arg Lys Asp
225                 230                 235                 240

Pro Ser Ser Val Asp Val Lys Gln Val Leu Leu Glu Met Arg Lys Tyr
                245                 250                 255

Arg Met Gly Leu Ile Gln Thr Ala Asp Gln Leu Arg Phe Ser Tyr Leu
            260                 265                 270

Ala Val Ile Glu Gly Ala Lys Phe Ile Met Gly Asp Ala Ser Val Gln
        275                 280                 285

Glu Gln Trp Lys Glu Leu Ser Asn Glu Asp Leu Asp Pro Pro Pro Glu
    290                 295                 300

His Thr Pro Pro Pro Pro Arg Pro Pro Lys Arg Thr Ser Glu Met His
305                 310                 315                 320

Asn Gly Arg Met His Glu His Ala Glu Phe Phe Pro Lys His Gln Val
                325                 330                 335

Val Glu Glu Glu Ile Arg Cys Ser Val Ser Thr Ala Glu Glu Thr Val
            340                 345                 350

Ser Asp Gly Arg Val Phe Ser Ser Val Pro Leu Ile Thr Asp Ser Thr
        355                 360                 365

Ser Gln Asp Thr Glu Ile Arg Arg Arg Thr Val Gly Glu Asn Leu His
    370                 375                 380

Val Thr Ala His Lys Glu Glu Ser Lys Ser Glu Ser Val Glu Glu Asp
385                 390                 395                 400

Asp Glu Asn Met Met Thr Thr Trp Lys Pro Phe Leu Val Asn Ile Cys
                405                 410                 415

Met Phe Thr Phe Leu Thr Ala Gly Ala Tyr Leu Cys Tyr Arg Val Cys
            420                 425                 430

Phe His

<210> 72
<211> 432
<212> PRT
<213> Mus musculus

<400> 72
Met Glu Met Glu Lys Glu Phe Glu Glu Ile Asp Lys Ala Gly Asn Trp
1               5                   10                  15

Ala Ala Ile Tyr Gln Asp Ile Arg His Glu Ala Ser Asp Phe Pro Cys
            20                  25                  30

Lys Val Ala Lys Leu Pro Lys Asn Lys Asn Arg Asn Arg Tyr Arg Asp
        35                  40                  45

Val Ser Pro Phe Asp His Ser Arg Ile Lys Leu His Gln Glu Asp Asn
    50                  55                  60

Asp Tyr Ile Asn Ala Ser Leu Ile Lys Met Glu Glu Ala Gln Arg Ser
65                  70                  75                  80

Tyr Ile Leu Thr Gln Gly Pro Leu Pro Asn Thr Cys Gly His Phe Trp
                85                  90                  95

Glu Met Val Trp Glu Gln Lys Ser Arg Gly Val Val Met Leu Asn Arg
            100                 105                 110

Ile Met Glu Lys Gly Ser Leu Lys Cys Ala Gln Tyr Trp Pro Gln Gln
        115                 120                 125

Glu Glu Lys Glu Met Val Phe Asp Asp Thr Gly Leu Lys Leu Thr Leu
    130                 135                 140

Ile Ser Glu Asp Val Lys Ser Tyr Tyr Thr Val Arg Gln Leu Glu Leu
145                 150                 155                 160

Glu Asn Leu Thr Thr Lys Glu Thr Arg Glu Ile Leu His Phe His Tyr
                165                 170                 175

Thr Thr Trp Pro Asp Phe Gly Val Pro Glu Ser Pro Ala Ser Phe Leu
            180                 185                 190

Asn Phe Leu Phe Lys Val Arg Glu Ser Gly Ser Leu Ser Leu Glu His
        195                 200                 205

Gly Pro Ile Val Val His Cys Ser Ala Gly Ile Gly Arg Ser Gly Thr
    210                 215                 220

Phe Cys Leu Ala Asp Thr Cys Leu Leu Leu Met Asp Lys Arg Lys Asp
225                 230                 235                 240

Pro Ser Ser Val Asp Ile Lys Lys Val Leu Leu Glu Met Arg Arg Phe
                245                 250                 255

Arg Met Gly Leu Ile Gln Thr Ala Asp Gln Leu Arg Phe Ser Tyr Leu
            260                 265                 270

Ala Val Ile Glu Gly Ala Lys Phe Ile Met Gly Asp Ser Ser Val Gln
        275                 280                 285

Asp Gln Trp Lys Glu Leu Ser Arg Glu Asp Leu Asp Leu Pro Pro Glu
    290                 295                 300

His Val Pro Pro Pro Pro Arg Pro Pro Lys Arg Thr Leu Glu Pro His
305                 310                 315                 320

Asn Gly Lys Cys Lys Glu Leu Phe Ser Ser His Gln Trp Val Ser Glu
                325                 330                 335

Glu Thr Cys Gly Asp Glu Asp Ser Leu Ala Arg Glu Glu Gly Arg Ala
            340                 345                 350

Gln Ser Ser Ala Met His Ser Val Ser Ser Met Ser Pro Asp Thr Glu
        355                 360                 365

Val Arg Arg Arg Met Val Gly Gly Gly Leu Gln Ser Ala Gln Ala Ser
    370                 375                 380

Val Pro Thr Glu Glu Glu Leu Ser Ser Thr Glu Glu Glu His Lys Ala
385                 390                 395                 400

His Trp Pro Ser His Trp Lys Pro Phe Leu Val Asn Val Cys Met Ala
                405                 410                 415

Thr Leu Leu Ala Thr Gly Ala Tyr Leu Cys Tyr Arg Val Cys Phe His
            420                 425                 430

<210> 73
<211> 108
<212> PRT
<213> Artificial Sequence

<220>
<223> Description of Artificial Sequence: Thioredoxin
      Consensus Sequence

<400> 73
Glu Ser Val Val Val Val Leu Thr Asp Glu Asn Phe Asp Glu Glu Val
  1               5                  10                  15

Leu Lys Ala Ser Asp Lys Pro Val Leu Val Asp Phe Tyr Ala Pro Trp
             20                  25                  30

Cys Gly Pro Cys Lys Met Leu Ala Pro Glu Tyr Glu Lys Leu Ala Glu
         35                  40                  45

Glu Tyr Lys Asp Asp Val Lys Phe Ala Lys Val Asp Ala Asp Glu Asn
     50                  55                  60

Pro Asp Leu Ala Ser Glu Tyr Gly Val Arg Gly Phe Pro Thr Leu Lys
65                  70                  75                  80

Phe Phe Lys Asn Gly Lys Lys Val Asp Tyr Val Gly Gly Arg Thr Lys
                85                  90                  95

Glu Ala Leu Val Ala Phe Ile Lys Lys His Val Gly
            100                 105


<210> 74
<211> 249
<212> PRT
<213> Artificial Sequence

<220>
<223> Description of Artificial Sequence: 7
      transmembrane domain Consensus Sequence

<400> 74
Ala Leu Leu Leu Ser Ile Ile Tyr Thr Val Gly Tyr Ser Leu Ser Leu
  1               5                  10                  15

Val Cys Leu Leu Leu Ala Ile Ala Ile Leu Leu Leu Phe Arg Lys Leu
             20                  25                  30

Arg Cys Thr Arg Asn Tyr Ile His Ile Asn Leu Phe Leu Ser Leu Ile
         35                  40                  45

Leu Arg Ala Leu Ser Phe Leu Ile Gly Asp Ala Val Leu Leu Asn Ser
     50                  55                  60

Val Glu Leu Gly Cys Lys Val Val Ala Val Phe Leu His Tyr Phe Phe
 65                  70                  75                  80

Leu Ala Asn Phe Phe Trp Met Leu Val Glu Gly Leu Tyr Leu Tyr Thr
                 85                  90                  95

Leu Leu Val Glu Thr Phe Phe Ser Glu Arg Lys Arg Leu Leu Trp Tyr
            100                 105                 110

Leu Leu Ile Gly Trp Gly Val Pro Ala Val Val Val Gly Ile Trp Ala
        115                 120                 125

Leu Val Arg Pro Lys Gly Tyr Gly Asn Pro Gly Cys Cys Trp Ile Ser
    130                 135                 140

Asn Glu Ala Pro Phe Trp Trp Ile Ile Arg Gly Pro Ile Leu Leu Val
145                 150                 155                 160

Ile Leu Val Asn Phe Ile Phe Phe Ile Asn Ile Leu Arg Val Leu Val
                165                 170                 175

Gln Lys Leu Arg Ser Pro Gln Thr Gly Lys Thr Asp Leu Tyr Arg Lys
            180                 185                 190

Leu Val Lys Ser Thr Leu Leu Leu Leu Pro Leu Leu Gly Val Thr Trp

195                 200                 205

Val Leu Phe Leu Phe Ala Pro Glu Ser Gln Leu Ser Leu Val Phe Leu
    210                 215                 220

Tyr Leu Phe Leu Ile Leu Asn Ser Phe Gln Gly Phe Phe Val Ala Leu
225                 230                 235                 240

Leu Tyr Cys Phe Leu Asn Gly Glu Val
                245


<210> 75
<211> 51
<212> PRT
<213> Artificial Sequence

<220>
<223> Description of Artificial Sequence: Leucine Rich
      C-terminal domain Consensus Sequence

<400> 75
Asn Pro Phe Ile Cys Asp Cys Glu Leu Arg Trp Leu Leu Arg Trp Leu
  1               5                  10                  15

Arg Ala Pro Arg Arg Leu Pro Asp Pro Glu Asp Leu Arg Cys Ala Ser
             20                  25                  30

Pro Glu Ser Leu Arg Gly Pro Leu Leu Asp Leu Leu Pro Ser Asp Phe
         35                  40                  45

Ser Cys Pro
     50


<210> 76
<211> 70
<212> PRT
<213> Artificial Sequence

<220>
<223> Description of Artificial Sequence: HormR Domain
      Consensus Sequence

<400> 76
Thr Asp Leu Gly Cys Pro Ala Thr Trp Asp Gly Ile Ile Cys Trp Pro
  1               5                  10                  15

Ala Thr Pro Ala Gly Glu Leu Val Glu Val Pro Cys Pro Asp Tyr Phe
             20                  25                  30

Ser Gly Phe Ser Asn Lys Glu Gly Ala Ser Arg Asn Cys Thr Glu Asp
         35                  40                  45

Gly Gly Trp Ser Pro Pro Phe Pro Asn Tyr Ser Asn Cys Thr Ser Asn
     50                  55                  60

Asp Tyr Asn Glu Leu Lys
65                  70

<210> 77
<211> 49
<212> PRT
<213> Artificial Sequence

<220>
<223> Description of Artificial Sequence: G-protein
      coupled receptor Consensus Sequence

<400> 77
Ser Asn Pro Ile Cys Val Phe Trp Asp Glu Ser Ser Gly Glu Trp Ser
  1               5                  10                  15

Thr Arg Gly Cys Glu Leu Leu Glu Thr Ser Ser Thr His Thr Thr Cys
             20                  25                  30

Ser Cys Asn His Leu Thr Ser Phe Ala Val Leu Met Asp Val Pro Pro
         35                  40                  45

Ile

<210> 78
<211> 49
<212> PRT
<213> Artificial Sequence

<220>
<223> Description of Artificial Sequence:
      Latrophilin/CL-1-like GPS Consensus Sequence

<400> 78
Ser Asn Pro Ile Cys Val Phe Trp Asp Glu Ser Ser Gly Val Trp Ser
  1               5                  10                  15

Thr Arg Gly Cys Lys Leu Leu Glu Leu Ser Lys Ser His Thr Thr Cys
             20                  25                  30

Ser Cys Asn His Leu Thr Ser Phe Ala Val Leu Met Asp Val Pro Pro
         35                  40                  45

Asn

<210> 79
<211> 67
<212> PRT
<213> Artificial Sequence

<220>
<223> Description of Artificial Sequence: Hormone

receptor Consensus Sequence

<400> 79
Pro Gly Leu Tyr Cys Pro Ala Thr Trp Asp Gly Gly Val Cys Trp Pro
1               5                   10                  15

Arg Thr Pro Ala Gly Thr Leu Val Glu Val Pro Cys Pro Asp Tyr Phe
            20                  25                  30

Ser Gly Phe Asn Tyr Asp Asn Ala Ser Arg Asn Cys Leu Ser Asn Gly
        35                  40                  45

Gly Trp Ala Ser Asn Trp Pro Trp Pro Asp Tyr Thr Asn Cys Thr Ser
    50                  55                  60

Pro Glu Tyr
65


<210> 80
<211> 145
<212> PRT
<213> Artificial Sequence

<220>
<223> Description of Artificial Sequence:
      Lipocalin/cytosolic fatty-acid binding protein
      Consensus Sequence

<400> 80
Lys Phe Ala Gly Lys Trp Tyr Leu Val Ala Ser Ala Asn Phe Asp Pro
1               5                   10                  15

Glu Leu Lys Glu Glu Leu Gly Val Leu Glu Ala Thr Arg Lys Glu Ile
            20                  25                  30

Thr Pro Leu Lys Glu Gly Asn Leu Glu Ile Val Phe Asp Gly Asp Lys
        35                  40                  45

Asn Gly Ile Cys Glu Glu Thr Phe Gly Lys Leu Glu Lys Thr Lys Lys
    50                  55                  60

Leu Gly Val Glu Phe Asp Tyr Tyr Thr Gly Asp Asn Arg Phe Val Val
65                  70                  75                  80

Leu Asp Thr Asp Tyr Asp Asn Tyr Leu Leu Val Cys Val Gln Lys Gly
                85                  90                  95

Asp Gly Gly Glu Thr Ser Arg Val Ala Glu Leu Tyr Gly Arg Thr Pro
            100                 105                 110

Glu Leu Ser Pro Glu Ala Leu Glu Leu Phe Arg Thr Leu Thr Glu Glu
        115                 120                 125

Leu Gly Ile Pro Glu Asp Asn Val Val Cys Thr Arg Gln Thr Glu Arg
    130                 135                 140

Cys
145

<210> 81
<211> 54
<212> PRT
<213> Artificial Sequence

<220>
<223> Description of Artificial Sequence: BPTI/Kunitz
      Consensus Sequence

<400> 81
Asp Val Cys Ser Leu Pro Pro Asp Thr Gly Pro Cys Gly Gly Ser Ile
  1               5                  10                  15

Pro Arg Tyr Tyr Tyr Asp Pro Glu Thr Gly Thr Cys Glu Pro Phe Thr
             20                  25                  30

Tyr Gly Gly Cys Gly Gly Asn Ala Asn Asn Phe Glu Ser Lys Glu Glu
         35                  40                  45

Cys Glu Arg Thr Cys Gly
     50

<210> 82
<211> 53
<212> PRT
<213> Artificial Sequence

<220>
<223> Description of Artificial Sequence: Kunitz/Bovine
      pancreatic trypsin inhibitor consensus sequence

<400> 82
Cys Ser Leu Pro Pro Asp Thr Gly Pro Cys Thr Gly Phe Pro Arg Ile
  1               5                  10                  15

Trp Arg Trp Tyr Tyr Asp Ser Ser Thr Gly Thr Cys Glu Pro Phe Thr
             20                  25                  30

Tyr Gly Gly Cys Gly Gly Asn Ala Asn Asn Phe Glu Thr Lys Glu Glu
         35                  40                  45

Cys Glu Ser Thr Cys
     50

<210> 83
<211> 43
<212> PRT
<213> Artificial Sequence

<220>
<223> Description of Artificial Sequence: Low density

lipoprotein0receptor Consensus Sequence

<400> 83
Arg Val Leu Leu Ser Glu Gly Leu Gly His Pro Asn Gly Leu Ala Val
1               5                   10                  15

Asp Trp Ile Glu Gly Arg Leu Tyr Trp Thr Asp Trp Gly Leu Asp Val
            20                  25                  30

Ile Glu Val Ala Asn Leu Asp Gly Thr Asn Arg
        35                  40


<210> 84
<211> 43
<212> PRT
<213> Artificial Sequence

<220>
<223> Description of Artificial Sequence: Low density
      lipoprotein receptor repeat class B Consensus
      Sequence

<400> 84
Gly Arg Leu Tyr Trp Thr Asp Ser Ser Leu Arg Ala Ser Ile Ser Val
1               5                   10                  15

Ala Asp Leu Asp Gly Ser Ser Arg Arg Thr Leu Phe Ser Glu Asp Leu
            20                  25                  30

Gln His Pro Asn Gly Ile Ala Val Asp Pro Ile
        35                  40


<210> 85
<211> 38
<212> PRT
<213> Artificial Sequence

<220>
<223> Description of Artificial Sequence: LDLa Consensus
      Sequence

<400> 85
Thr Cys Pro Pro Gly Glu Phe Gln Cys Lys Asn Gly Arg Cys Ile Pro
1               5                   10                  15

Leu Ser Trp Val Cys Asp Gly Val Asp Asp Cys Gly Asp Gly Ser Asp
            20                  25                  30

Glu Glu Asn Cys Pro Ser
        35


<210> 86
<211> 39
<212> PRT

<213> Artificial Sequence

<220>
<223> Description of Artificial Sequence: Low density
      lipoprotein receptor domain class A Consensus
      Sequence

<400> 86
Ser Thr Cys Glu Pro Asn Glu Phe Gln Cys Gly Ser Gly Glu Cys Ile
  1               5                  10                  15

Pro Met Ser Trp Val Cys Asp Gly Asp Pro Asp Cys Arg Asp Gly Ser
             20                  25                  30

Asp Glu Lys Asn Cys Pro Ala
         35


<210> 87
<211> 41
<212> PRT
<213> Artificial Sequence

<220>
<223> Description of Artificial Sequence:
      Calcium-binding EGF domain Consensus Sequence

<400> 87
Asp Ile Asp Glu Cys Ala Ser Gly Asn Pro Cys Gln Asn Gly Gly Thr
  1               5                  10                  15

Cys Val Asn Thr Val Gly Ser Tyr Arg Cys Glu Glu Cys Pro Pro Gly
             20                  25                  30

Tyr Thr Leu Asp Gly Arg Asn Cys Glu
         35                  40


<210> 88
<211> 230
<212> PRT
<213> Artificial Sequence

<220>
<223> Description of Artificial Sequence: Trypsin-like
      serine protease Consensus Sequence

<400> 88
Arg Ile Val Gly Gly Ser Glu Ala Asn Ile Gly Ser Phe Pro Trp Gln
  1               5                  10                  15

Val Ser Leu Gln Tyr Arg Gly Gly Arg His Phe Cys Gly Gly Ser Leu
             20                  25                  30

Ile Ser Pro Arg Trp Val Leu Thr Ala Ala His Cys Val Tyr Gly Ser
         35                  40                  45

Ala Pro Ser Ser Ile Arg Val Arg Leu Gly Ser His Asp Leu Ser Ser
    50                  55                  60

Gly Glu Glu Thr Gln Thr Val Lys Val Ser Lys Val Ile Val His Pro
65                  70                  75                  80

Asn Tyr Asn Pro Ser Thr Tyr Asp Asn Asp Ile Ala Leu Leu Lys Leu
                85                  90                  95

Ser Glu Pro Val Thr Leu Ser Asp Thr Val Arg Pro Ile Cys Leu Pro
            100                 105                 110

Ser Ser Gly Tyr Asn Val Pro Ala Gly Thr Thr Cys Thr Val Ser Gly
        115                 120                 125

Trp Gly Arg Thr Ser Glu Ser Ser Gly Ser Leu Pro Asp Thr Leu Gln
    130                 135                 140

Glu Val Asn Val Pro Ile Val Ser Asn Ala Thr Cys Arg Arg Ala Tyr
145                 150                 155                 160

Ser Gly Gly Pro Ala Ile Thr Asp Asn Met Leu Cys Ala Gly Gly Leu
                165                 170                 175

Glu Gly Gly Lys Asp Ala Cys Gln Gly Asp Ser Gly Gly Pro Leu Val
            180                 185                 190

Cys Asn Asp Pro Arg Trp Val Leu Val Gly Ile Val Ser Trp Gly Ser
        195                 200                 205

Tyr Gly Cys Ala Arg Pro Asn Lys Pro Gly Val Tyr Thr Arg Val Ser
    210                 215                 220

Ser Tyr Leu Asp Trp Ile
225                 230


<210> 89
<211> 217
<212> PRT
<213> Artificial Sequence

<220>
<223> Description of Artificial Sequence: Trypsin
      Consensus Sequence

<400> 89
Ile Val Gly Gly Arg Glu Ala Gln Ala Gly Ser Phe Pro Trp Gln Val
  1               5                  10                  15

Ser Leu Gln Val Ser Ser Gly His Phe Cys Gly Gly Ser Leu Ile Ser
             20                  25                  30

Glu Asn Trp Val Leu Thr Ala Ala His Cys Val Ser Gly Ala Ser Ser
         35                  40                  45

Val Arg Val Val Leu Gly Glu His Asn Leu Gly Thr Arg Glu Gly Thr

50                  55                  60

Glu Gln Lys Phe Asp Val Lys Lys Ile Ile Val His Pro Asn Tyr Asn
65                  70                  75                  80

Pro Asp Thr Asn Asp Ile Ala Leu Leu Lys Leu Lys Ser Pro Val Thr
                85                  90                  95

Leu Gly Asp Thr Val Arg Pro Ile Cys Leu Pro Ser Ala Ser Ser Asp
            100                 105                 110

Leu Pro Val Gly Thr Thr Cys Ser Val Ser Gly Trp Gly Arg Thr Lys
        115                 120                 125

Asn Leu Gly Thr Ser Asp Thr Leu Gln Glu Val Val Val Pro Ile Val
    130                 135                 140

Ser Arg Glu Thr Cys Arg Ser Ala Tyr Gly Gly Thr Val Thr Asp Thr
145                 150                 155                 160

Met Ile Cys Ala Gly Ala Leu Gly Gly Lys Asp Ala Cys Gln Gly Asp
                165                 170                 175

Ser Gly Gly Pro Leu Val Cys Ser Asp Gly Glu Leu Val Gly Ile Val
            180                 185                 190

Ser Trp Gly Tyr Gly Cys Ala Val Gly Asn Tyr Pro Gly Val Tyr Thr
        195                 200                 205

Arg Val Ser Arg Tyr Leu Asp Trp Ile
    210                 215


<210> 90
<211> 264
<212> PRT
<213> Artificial Sequence

<220>
<223> Description of Artificial Sequence: Protein
      Tyrosine Phosphatase Consensus Sequence

<400> 90
Gly Leu Glu Glu Glu Phe Glu Lys Leu Gln Arg Leu Thr Pro Asp Asp
  1               5                  10                  15

Leu Ser Cys Thr Val Ala Ile Leu Pro Glu Asn Arg Asp Lys Asn Arg
             20                  25                  30

Tyr Lys Asp Val Leu Pro Tyr Asp His Thr Arg Val Lys Leu Lys Pro
         35                  40                  45

Pro Pro Gly Glu Gly Ser Asp Tyr Ile Asn Ala Ser Tyr Ile Asp Gly
     50                  55                  60

Pro Asn Arg Pro Lys Ala Tyr Ile Ala Thr Gln Gly Pro Leu Pro Ser
 65                  70                  75                  80

Thr Val Glu Asp Phe Trp Arg Met Val Trp Glu Glu Lys Val Pro Val
                85                  90                  95

Ile Val Met Leu Thr Glu Leu Val Glu Lys Gly Arg Glu Lys Cys Ala
            100                 105                 110

Gln Tyr Trp Pro Glu Lys Glu Gly Gly Ser Leu Thr Tyr Gly Asp Ile
        115                 120                 125

Thr Val Thr Leu Lys Ser Val Glu Lys Val Asp Asp Tyr Thr Ile Arg
    130                 135                 140

Thr Leu Glu Val Thr Asn Thr Gly Gly Ser Glu Thr Arg Thr Val Thr
145                 150                 155                 160

His Tyr His Tyr Thr Asn Trp Pro Asp His Gly Val Pro Glu Ser Pro
                165                 170                 175

Lys Ser Leu Leu Asp Leu Val Arg Ala Val Arg Lys Ser Gln Ser Thr
            180                 185                 190

Leu Arg Asn Ser Gly Pro Ile Val Val His Cys Ser Ala Gly Val Gly
        195                 200                 205

Arg Thr Gly Thr Phe Ile Ala Ile Asp Ile Leu Leu Gln Gln Leu Glu
    210                 215                 220

Ala Gly Lys Glu Val Asp Ile Phe Glu Ile Val Lys Glu Leu Arg Ser
225                 230                 235                 240

Gln Arg Pro Gly Met Val Gln Thr Glu Glu Gln Tyr Ile Phe Leu Tyr
                245                 250                 255

Arg Ala Ile Leu Glu Tyr Leu Lys
            260


<210> 91
<211> 235
<212> PRT
<213> Artificial Sequence

<220>
<223> Description of Artificial Sequence:
      Protein-tyrosine phosphatase Consensus Sequence

<400> 91
Asn Leu Glu Lys Asn Arg Tyr Lys Asp Ile Leu Pro Tyr Asp His Thr
  1               5                  10                  15

Arg Val Lys Leu Lys Pro Leu Gly Asp Glu Asp Ser Asp Tyr Ile Asn
             20                  25                  30

Ala Ser Tyr Val Asp Gly Tyr Lys Lys Pro Lys Ala Tyr Ile Ala Thr
         35                  40                  45

Gln Gly Pro Leu Pro Asn Thr Ile Glu Asp Phe Trp Arg Met Val Trp
    50                  55                  60

Glu Glu Lys Val Arg Val Ile Val Met Leu Thr Glu Leu Val Glu Lys
65                  70                  75                  80

Gly Arg Glu Lys Cys Ala Gln Tyr Trp Pro Glu Lys Glu Gly Gly Pro
                85                  90                  95

Leu Thr Tyr Gly Asp Phe Thr Val Thr Cys Val Ser Val Glu Lys Lys
            100                 105                 110

Lys Asp Asp Tyr Thr Val Arg Thr Phe Glu Leu Thr Asn Ser Gly Asp
        115                 120                 125

Glu Glu Thr Arg Thr Val Lys His Tyr His Tyr Thr Gly Trp Pro Asp
    130                 135                 140

His Gly Val Pro Glu Ser Pro Lys Ser Ile Leu Asp Leu Leu Arg Lys
145                 150                 155                 160

Val Arg Lys Ser Lys Gly Thr Pro Asp Asp Gly Pro Ile Val Val His
                165                 170                 175

Cys Ser Ala Gly Ile Gly Arg Thr Gly Thr Phe Ile Ala Ile Asp Ile
            180                 185                 190

Leu Leu Gln Gln Leu Glu Lys Glu Gly Val Val Asp Val Phe Asp Thr
        195                 200                 205

Val Lys Glu Leu Arg Ser Gln Arg Pro Gly Met Val Gln Thr Leu Glu
    210                 215                 220

Gln Tyr Leu Phe Ile Tyr Asp Ala Ile Leu Glu
225                 230                 235


<210> 92
<211> 105
<212> PRT
<213> Artificial Sequence

<220>
<223> Description of Artificial Sequence: Protein
      tyrosine phosphatase Consensus Sequence

<400> 92
Thr Val Lys His Tyr His Tyr Thr Gly Trp Pro Asp His Gly Val Pro
  1               5                  10                  15

Glu Ser Pro Asp Ser Ile Leu Glu Phe Leu Arg Ala Val Lys Lys Ser
             20                  25                  30

Leu Asn Lys Ser Ala Asn Asn Gly Pro Val Val Val His Cys Ser Ala
         35                  40                  45

Gly Val Gly Arg Thr Gly Thr Phe Val Ala Ile Asp Ile Leu Leu Gln

50                  55                  60

Gln Leu Glu Ala Gly Thr Gly Glu Val Asp Ile Phe Asp Ile Val Lys
65                  70                  75                  80

Glu Leu Arg Ser Gln Arg Pro Gly Ala Val Gln Thr Leu Glu Gln Tyr
                85                  90                  95

Leu Phe Leu Tyr Arg Ala Leu Leu Glu
            100                 105

<210> 93
<211> 20
<212> DNA
<213> Artificial Sequence

<220>
<223> Description of Artificial Sequence: M13 Forward
      SF1

<400> 93
cctctgcctc ctactgcccc                                              20

<210> 94
<211> 22
<212> DNA
<213> Artificial Sequence

<220>
<223> Description of Artificial Sequence: M13 Forward
      SF2

<400> 94
gtgctgatgc ccatcaatgc ct                                           22

<210> 95
<211> 19
<212> DNA
<213> Artificial Sequence

<220>
<223> Description of Artificial Sequence: M13 Forward
      SF3

<400> 95
cgcatcgtgg gtgccatag                                               19

<210> 96
<211> 23
<212> DNA
<213> Artificial Sequence

<220>

<223> Description of Artificial Sequence: M13 Forward
      SF4

<400> 96
accgggaggc ccaatgtttc tct                                              23


<210> 97
<211> 20
<212> DNA
<213> Artificial Sequence

<220>
<223> Description of Artificial Sequence: M13 Forward
      SF5

<400> 97
cagccacagc aacacctccc                                                  20


<210> 98
<211> 21
<212> DNA
<213> Artificial Sequence

<220>
<223> Description of Artificial Sequence: M13 Reverse
      SR1

<400> 98
gtctccagca ccacgatctc c                                                21


<210> 99
<211> 21
<212> DNA
<213> Artificial Sequence

<220>
<223> Description of Artificial Sequence: M13 Reverse
      SR2

<400> 99
acgaaggtgt acagcaccct g                                                21


<210> 100
<211> 19
<212> DNA
<213> Artificial Sequence

<220>
<223> Description of Artificial Sequence: M13 Reverse
      SR3

<400> 100
ggctgcaggc cttgtcctc                                                   19

<210> 101
<211> 19
<212> DNA
<213> Artificial Sequence

<220>
<223> Description of Artificial Sequence: M13 Reverse
      SR4

<400> 101
gaagcggagc tgctggtca                                                    19


<210> 102
<211> 21
<212> DNA
<213> Artificial Sequence

<220>
<223> Description of Artificial Sequence: M13 Reverse
      SR5

<400> 102
tgttgctgtg gctgtggaag a                                                 21


<210> 103
<211> 27
<212> DNA
<213> Artificial Sequence

<220>
<223> Description of Artificial Sequence: NOV1 Primer 1

<400> 103
tgtaaaacaa gaccaggcac aagaagg                                           27


<210> 104
<211> 31
<212> DNA
<213> Artificial Sequence

<220>
<223> Description of Artificial Sequence: NOV1 Primer 2

<400> 104
ctcacagaga aagagacagg aaagtaaatg c                                      31


<210> 105
<211> 24
<212> DNA
<213> Artificial Sequence

<220>
<223> Description of Artificial Sequence: NOV5a Primer 1

<400> 105
agttccagtg tggggatggg acat                                              24


<210> 106
<211> 27
<212> DNA
<213> Artificial Sequence

<220>
<223> Description of Artificial Sequence: NOV5a Primer 2

<400> 106
aatgtcatgt gggttgttgc aggttct                                           27


<210> 107
<211> 24
<212> DNA
<213> Artificial Sequence

<220>
<223> Description of Artificial Sequence: NOV5b Primer 1

<400> 107
agttccagtg tggggatggg acat                                              24


<210> 108
<211> 27
<212> DNA
<213> Artificial Sequence

<220>
<223> Description of Artificial Sequence: NOV5b Primer 2

<400> 108
aatgtcatgt gggttgttgc aggttct                                           27


<210> 109
<211> 24
<212> DNA
<213> Artificial Sequence

<220>
<223> Description of Artificial Sequence: NOV5c Primer 1

<400> 109
agttccagtg tggggatggg acat                                              24


<210> 110
<211> 27

<212> DNA
<213> Artificial Sequence

<220>
<223> Description of Artificial Sequence: NOV5c Primer 2

<400> 110
aatgtcatgt gggttgttgc aggttct                                          27


<210> 111
<211> 24
<212> DNA
<213> Artificial Sequence

<220>
<223> Description of Artificial Sequence: NOV5d Primer 1

<400> 111
agttccagtg tggggatggg acat                                             24


<210> 112
<211> 27
<212> DNA
<213> Artificial Sequence

<220>
<223> Description of Artificial Sequence: NOV5d Primer 2

<400> 112
actaactgag cttgggactg cagctgt                                          27


<210> 113
<211> 18
<212> DNA
<213> Artificial Sequence

<220>
<223> Description of Artificial Sequence: NOV5e Primer 1

<400> 113
gcggctgatc cgctgctc                                                    18


<210> 114
<211> 24
<212> DNA
<213> Artificial Sequence

<220>
<223> Description of Artificial Sequence: NOV5e Primer 2

<400> 114
ggtgatccca tcctcagggt agtc                                             24

<210> 115
<211> 18
<212> DNA
<213> Artificial Sequence

<220>
<223> Description of Artificial Sequence: NOV5f Primer 1

<400> 115
gcggctgatc cgctgctc                                                    18


<210> 116
<211> 24
<212> DNA
<213> Artificial Sequence

<220>
<223> Description of Artificial Sequence: NOV5f Primer 2

<400> 116
ggtgatccca tcctcagggt agtc                                             24


<210> 117
<211> 24
<212> DNA
<213> Artificial Sequence

<220>
<223> Description of Artificial Sequence: NOV5g Primer 1

<400> 117
agttccagtg tggggatggg acat                                             24


<210> 118
<211> 27
<212> DNA
<213> Artificial Sequence

<220>
<223> Description of Artificial Sequence: NOV5g Primer 2

<400> 118
actaactgag cttgggactg cagctgt                                          27


<210> 119
<211> 24
<212> DNA
<213> Artificial Sequence

<220>
<223> Description of Artificial Sequence: NOV5h Primer 1

<400> 119
agttccagtg tggggatggg acat                                              24


<210> 120
<211> 27
<212> DNA
<213> Artificial Sequence

<220>
<223> Description of Artificial Sequence: NOV5h Primer 2

<400> 120
actaactgag cttgggactg cagctgt                                           27


<210> 121
<211> 27
<212> DNA
<213> Artificial Sequence

<220>
<223> Description of Artificial Sequence: NOV5i Primer 1

<400> 121
ggtccccgca gatttcagtg taagagt                                           27


<210> 122
<211> 27
<212> DNA
<213> Artificial Sequence

<220>
<223> Description of Artificial Sequence: NOV5i Primer 2

<400> 122
actaactgag cttgggactg cagctgt                                           27


<210> 123
<211> 21
<212> DNA
<213> Artificial Sequence

<220>
<223> Description of Artificial Sequence: Ag2025 Forward

<400> 123
gtgaggaggt ggtgagagag t                                                 21


<210> 124
<211> 26
<212> DNA
<213> Artificial Sequence

<220>
<223> Description of Artificial Sequence: Ag2025 Probe

<400> 124
ccatcatgtg tgtcccaacc tttcag                                          26


<210> 125
<211> 22
<212> DNA
<213> Artificial Sequence

<220>
<223> Description of Artificial Sequence: Ag2025 Reverse

<400> 125
gcaaagttca tccacctttt ct                                              22


<210> 126
<211> 22
<212> DNA
<213> Artificial Sequence

<220>
<223> Description of Artificial Sequence: Ag850 Forward

<400> 126
cctttcttct cttcctcctc aa                                              22


<210> 127
<211> 23
<212> DNA
<213> Artificial Sequence

<220>
<223> Description of Artificial Sequence: Ag850 Probe

<400> 127
cacctggcga gtgctcctct ctg                                             23


<210> 128
<211> 19
<212> DNA
<213> Artificial Sequence

<220>
<223> Description of Artificial Sequence: Ag850 Reverse

<400> 128
ggtggatggc gttgtagag                                                  19


<210> 129
<211> 21

<212> DNA
<213> Artificial Sequence

<220>
<223> Description of Artificial Sequence: Ag1631 Forward

<400> 129
aagcagacca gattccactt g                                                21

<210> 130
<211> 26
<212> DNA
<213> Artificial Sequence

<220>
<223> Description of Artificial Sequence: Ag1631 Probe

<400> 130
ctagtgggca ttgctcagct tcctct                                           26

<210> 131
<211> 22
<212> DNA
<213> Artificial Sequence

<220>
<223> Description of Artificial Sequence: Ag1631 Reverse

<400> 131
ctgaatttct cgatctcatc ca                                               22

<210> 132
<211> 20
<212> DNA
<213> Artificial Sequence

<220>
<223> Description of Artificial Sequence: Ag2448 Forward

<400> 132
acttgctagt gggcattgct                                                  20

<210> 133
<211> 28
<212> DNA
<213> Artificial Sequence

<220>
<223> Description of Artificial Sequence: Ag2448 Probe

<400> 133
tcctctgtga ctatgtctga caagtcca                                         28

<210> 134
<211> 21
<212> DNA
<213> Artificial Sequence

<220>
<223> Description of Artificial Sequence: Ag2448 Reverse

<400> 134
tgaatttctc gatctcatcc a                                                21


<210> 135
<211> 20
<212> DNA
<213> Artificial Sequence

<220>
<223> Description of Artificial Sequence: Ag2447 Forward

<400> 135
aaaaccttag cgcttgcttt                                                  20


<210> 136
<211> 27
<212> DNA
<213> Artificial Sequence

<220>
<223> Description of Artificial Sequence: Ag2447 Probe

<400> 136
tcattcaccg aacaaaataa tgactga                                          27


<210> 137
<211> 40
<212> DNA
<213> Artificial Sequence

<220>
<223> Description of Artificial Sequence: NOV3b Primer 1

<400> 137
ggatcccggc tgcccttcca gtgctctgcc agctacctgg                            40


<210> 138
<211> 36
<212> DNA
<213> Artificial Sequence

<220>
<223> Description of Artificial Sequence: NOV3b Primer 2

<400> 138
ctcgaggctc agctccatga gcacggccac attgcc                                36

<210> 139
<211> 22
<212> DNA
<213> Artificial Sequence

<220>
<223> Description of Artificial Sequence: Ag1402 Forward

<400> 139
cttacaacac cacgggacta ag                                               22

<210> 140
<211> 26
<212> DNA
<213> Artificial Sequence

<220>
<223> Description of Artificial Sequence: Ag1402 Probe

<400> 140
aagagcactt ccttgcctcc gtaagg                                           26

<210> 141
<211> 22
<212> DNA
<213> Artificial Sequence

<220>
<223> Description of Artificial Sequence: Ag1402 Reverse

<400> 141
agatcaaatg attgggatgg tt                                               22

<210> 142
<211> 21
<212> DNA
<213> Artificial Sequence

<220>
<223> Description of Artificial Sequence: Ag2447 Reverse

<400> 142
agaccaattc tgttcccatt g                                                21

## Description

### FIELD OF THE INVENTION

The invention generally relates to nucleic acids and polypeptides encoded thereby.

### BACKGROUND OF THE INVENTION

The invention generally relates to nucleic acids and polypeptides encoded therefrom. More specifically, the invention relates to nucleic acids encoding cytoplasinic, nuclear, membrane bound, and secreted polypeptides, as well as vectors, host cells, antibodies, and recombinant methods for producing these nucleic acids and polypeptides.

### SUMMARY OF THE INVENTION

The invention is based in part upon the discovery of nucleic acid sequences encoding novel polypeptides. The novel nucleic acids and polypeptides are referred to herein as NOVX, or NOV 1, NOV2, NOV3, NOV4, NOV5, NOV6, NOV7, and NOV8 nucleic acids and polypeptides. These nucleic acids and polypeptides, as well as derivatives, homologs, analogs and fragments thereof, will hereinafter be collectively designated as "NOVX" nucleic acid or polypeptide sequences.

In one aspect, the invention provides an isolated NOVX nucleic acid molecule encoding a NOVX polypeptide that includes a nucleic acid sequence that has identity to the nucleic acids disclosed in SEQ ID NOS:1, 3, 5, 7, 9, 12, 14, 16, 18, 20, 22, 24, 26, 28, 30, 32, 34, and 36. In some embodiments, the NOVX nucleic acid molecule will hybridize under stringent conditions to a nucleic acid sequence complementary to a nucleic acid molecule that includes a protein-coding sequence of a NOVX nucleic acid sequence. The invention also includes an isolated nucleic acid that encodes a NOVX polypeptide, or a fragment, homolog, analog or derivative thereof. For example, the nucleic acid can encode a polypeptide at least 80% identical to a polypeptide comprising the amino acid sequences of SEQ ID NOS:2, 4, 6, 8, 11, 13, 15, 17, 19, 21, 23, 25, 27, 29, 31, 33, 35, and 37. The nucleic acid can be, for example, a genomic DNA fragment or a cDNA molecule that includes the nucleic acid sequence of any of SEQ ID NOS:1, 3, 5, 7, 9, 12, 14, 16, 18, 20, 22, 24, 26, 28, 30, 32, 34, and 36.

Also included in the invention is an oligonucleotide, *e.g*., an oligonucleotide which includes at least 6 contiguous nucleotides of a NOVX nucleic acid (*e.g*., SEQ ID NOS:1, 3, 5, 7, 9, 12, 14, 16, 18, 20, 22, 24, 26, 28, 30, 32, 34, and 36) or a complement of said oligonucleotide.

Also included in the invention are substantially purified NOVX polypeptides (SEQ ID NOS:2, 4, 6, 8, 11, 13, 15, 17, 19, 21, 23, 25, 27, 29, 31, 33, 35, and 37). In certain embodiments, the NOVX polypeptides include an amino acid sequence that is substantially identical to the amino acid sequence of a human NOVX polypeptide.

The invention also features antibodies that immunoselectively bind to NOVX polypeptides, or fragments, homologs, analogs or derivatives thereof.

In another aspect, the invention includes pharmaceutical compositions that include therapeutically- or prophylactically-effective amounts of a therapeutic and a pharmaceutically-acceptable carrier. The therapeutic can be, *e.g.,* a NOVX nucleic acid, a NOVX polypeptide, or an antibody specific for a NOVX polypeptide. In a further aspect, the invention includes, in one or more containers, a therapeutically- or prophylactically-effective amount of this pharmaceutical composition.

In a further aspect, the invention includes a method of producing a polypeptide by culturing a cell that includes a NOVX nucleic acid, under conditions allowing for expression of the NOVX polypeptide encoded by the DNA. If desired, the NOVX polypeptide can then be recovered.

In another aspect, the invention includes a method of detecting the presence of a NOVX polypeptide in a sample. In the method, a sample is contacted with a compound that selectively binds to the polypeptide under conditions allowing for formation of a complex between the polypeptide and the compound. The complex is detected, if present, thereby identifying the NOVX polypeptide within the sample.

The invention also includes methods to identify specific cell or tissue types based on their expression of a NOVX.

Also included in the invention is a method of detecting the presence of a NOVX nucleic acid molecule in a sample by contacting the sample with a NOVX nucleic acid probe or primer, and detecting whether the nucleic acid probe or primer bound to a NOVX nucleic acid molecule in the sample.

In a further aspect, the invention provides a method for modulating the activity of a NOVX polypeptide by contacting a cell sample that includes the NOVX polypeptide with a compound that binds to the NOVX polypeptide in an amount sufficient to modulate the activity of said polypeptide. The compound can be, *e.g.,* a small molecule, such as a nucleic acid, peptide, polypeptide, peptidomimetic, carbohydrate, lipid or other organic (carbon containing) or inorganic molecule, as further described herein.

Also within the scope of the invention is the use of a therapeutic in the manufacture of a medicament for treating or preventing disorders or syndromes including, *e.g.,* Inflamation, Autoimmune disorders, scleroderma, transplantation, autoimmune disease, allergies, immunodeficiencies, systemic lupus erythematosus, lymphedema, hemophilia, hypercoagulation, idiopathic thrombocytopenic purpura, graft versus host disease (GVHD), endometriosis, cystic fibrosis, pathological disorders involving spleen, thymus, lung, and peritoneal macrophages, Aging, Alzheimer's disease, stroke, Cancer, abnormal angiogenesis, like cancer and more specifically aggressive, metastatic cancer, more specifically tumor of the lung, kidney, brain, liver and colon, mastocytomas, prostate cancer, modulation of apoptosis, Oncogenesis such as lung, liver and ovarian cancers; cell growth and possibly differentiation, non-Hodgkin lymphomas, Von Hippel-Lindau (VHL) syndrome, Lesch-Nyhan syndrome, periodontitis, pancreatitis, musculoskeletal disorders, hypercalceimia, Parkinson's disease, Huntington's disease, cerebral palsy, epilepsy, Lesch-Nyhan syndrome, multiple sclerosis, ataxia-telangiectasia, behavioral disorders, addiction, anxiety, pain, neurodegeneration, familial amyloidotic polyneuropathy, neurodegenerative and neuropsychiatric disorders, leukodystrophies, dysbetalipoproteinemia, hyperlipoproteinemia type III, xanthomatosis, coronary and/or peripheral vascular disease,ardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, accelerated vascular disease, angiogenesis and wound healing, leukemogenesis, renal artery stenosis, interstitial nephritis, glomerulonephritis, polycystic kidney disease, renal tubular acidosis, IgA nephropathy, fertility, endocrine dysfunctions, diabetes, diabetic acidosis, hypercholesterolemia, planar and tendon xanthomas, obesity, growth and reproductive disorders, hyperthyroidism, hypothyroidism, type 2 diabetes, chronic lung diseases, asthma, tuberous sclerosis and/or other pathologies and disorders of the like.

The therapeutic can be, *e.g*., a NOVX nucleic acid, a NOVX polypeptide, or a NOVX-specific antibody, or biologically-active derivatives or fragments thereof.

For example, the compositions of the present invention will have efficacy for treatment of patients suffering from the diseases and disorders disclosed above and/or other pathologies and disorders of the like. The polypeptides can be used as immunogens to produce antibodies specific for the invention, and as vaccines. They can also be used to screen for potential agonist and antagonist compounds. For example, a cDNA encoding NOVX may be useful in gene therapy, and NOVX may be useful when administered to a subject in need thereof. By way of non-limiting example, the compositions of the present invention will have efficacy for treatment of patients suffering from the diseases and disorders disclosed above and/or other pathologies and disorders of the like.

The invention further includes a method for screening for a modulator of disorders or syndromes including, *e.g*., the diseases and disorders disclosed above and/or other pathologies and disorders of the like. The method includes contacting a test compound with a NOVX polypeptide and determining if the test compound binds to said NOVX polypeptide. Binding of the test compound to the NOVX polypeptide indicates the test compound is a modulator of activity, or of latency or predisposition to the aforementioned disorders or syndromes.

Also within the scope of the invention is a method for screening for a modulator of activity, or of latency or predisposition to an disorders or syndromes including, *e.g*., the diseases and disorders disclosed above and/or other pathologies and disorders of the like by administering a test compound to a test animal at increased risk for the aforementioned disorders or syndromes. The test animal expresses a recombinant polypeptide encoded by a NOVX nucleic acid. Expression or activity of NOVX polypeptide is then measured in the test animal, as is expression or activity of the protein in a control animal which recombinantly-expresses NOVX polypeptide and is not at increased risk for the disorder or syndrome. Next, the expression of NOVX polypeptide in both the test animal and the control animal is compared. A change in the activity of NOVX polypeptide in the test animal relative to the control animal indicates the test compound is a modulator of latency of the disorder or syndrome.

In yet another aspect, the invention includes a method for determining the presence of or predisposition to a disease associated with altered levels of a NOVX polypeptide, a NOVX nucleic acid, or both, in a subject (*e.g*., a human subject). The method includes measuring the amount of the NOVX polypeptide in a test sample from the subject and comparing the amount of the polypeptide in the test sample to the amount of the NOVX polypeptide present in a control sample. An alteration in the level of the NOVX polypeptide in the test sample as compared to the control sample indicates the presence of or predisposition to a disease in the subject. Preferably, the predisposition includes, *e.g*., the diseases and disorders disclosed above and/or other pathologies and disorders of the like. Also, the expression levels of the new polypeptides of the invention can be used in a method to screen for various cancers as well as to determine the stage of cancers.

In a further aspect, the invention includes a method of treating or preventing a pathological condition associated with a disorder in a mammal by administering to the subject a NOVX polypeptide, a NOVX nucleic acid, or a NOVX-specific antibody to a subject (*e.g.*, a human subject), in an amount sufficient to alleviate or prevent the pathological condition. In preferred embodiments, the disorder, includes, *e.g*., the diseases and disorders disclosed above and/or other pathologies and disorders of the like.

In yet another aspect, the invention can be used in a method to identity the cellular receptors and downstream effectors of the invention by any one of a number of techniques commonly employed in the art. These include but are not limited to the two-hybrid system, affinity purification, co-precipitation with antibodies or other specific-interacting molecules.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### DETAILED DESCRIPTION OF THE INVENTION

The present invention provides novel nucleotides and polypeptides encoded thereby. Included in the invention are the novel nucleic acid sequences and their encoded polypeptides. The sequences are collectively referred to herein as "NOVX nucleic acids" or "NOVX polynucleotides" and the corresponding encoded polypeptides are referred to as "NOVX polypeptides" or "NOVX proteins." Unless indicated otherwise, "NOVX" is meant to refer to any of the novel sequences disclosed herein. Table A provides a summary of the NOVX nucleic acids and their encoded polypeptides.

| **TABLE A. Sequences and Corresponding SEQ ID Numbers** | | | | |
|---|---|---|---|---|
| **NOVX Assignment** | **Internal Identification** | **SEQ ID NO (nucleic acid)** | **SEQ ID NO (polypeptide)** | **Homology** |
| 1 | AC013554_da1 | 1 | 2 | thioredoxin -like |
| 2a | 3277789.0.83_da1 | 3 | 4 | retinoic acid/SRA6-like |
| 2b | CG52276-07 | 5 | 6 | retinoic acid/SRA6-like |
| 3a | 128900861ext | 7 | 8 | LRR/GPCR-like |
| 3b | 188822778 | 9 | 11 | LRR/GPCR-like |
| 4 | AL031711_da1 | 12 | 13 | Trypsin inhibitor-like |
| 5a | sggc_draft_dj784a16_ 20000723_da1 | 14 | 15 | apolipoprotein e-like |
| 5b | sggc_draft_dj784a16_ 20000723_da2 | 16 | 17 | apolipoprotein e-like |
| 5c | sggc_draft_dj784a16_ 20000723_da3 | 18 | 19 | apolipoprotein e-like |
| 5d | CG55256-02 | 20 | 21 | apolipoprotein e-like |
| 5e | CG55256-03 | 22 | 23 | apolipoprotein e-like |
| 5f | CG55256-04 | 24 | 25 | apolipoprotein e-like |
| 5g | CG55256-05 | 26 | 27 | apolipoprotein e-like |
| 5h | CG55256-06 | 28 | 29 | apolipoprotein e-like |
| 5i | CG55256-07 | 30 | 31 | apolipoprotein e-like |
| 6 | SC_129296119-A | 32 | 33 | mastocytoma protease precursor -like |
| 7 | GM10d7_A | 34 | 35 | thymosin beta-4-like |
| 8 | GMba550p23_A | 36 | 37 | Protein-tyrosine-phosphatase -like |

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

NOV1 is homologous to a thioredoxin -like family of proteins. Thus, the NOV1 nucleic acids, polypeptides, antibodies and related compounds according to the invention will be useful in therapeutic and diagnostic applications implicated in, for example; Inflamation, Autoimmune disorders, Aging and Cancer, and/or other pathologies/disorders.

NOV2a and 2b are homologous to the Retinoic acid responsive/STRA-6-like family of proteins. Thus NOV2 nucleic acids, polypeptides, antibodies and related compounds according to the invention will be useful in therapeutic and diagnostic applications implicated in, for example; Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, scleroderma, transplantation, autoimmune disease, allergies, immunodeficiencies, renal artery stenosis, interstitial nephritis, glomerulonephritis, polycystic kidney disease, systemic lupus erythematosus, renal tubular acidosis, IgA nephropathy, Lesch-Nyhan syndrome, lymphedema, hemophilia, hypercoagulation, idiopathic thrombocytopenic purpura, graft versus host disease (GVHD), endometriosis, fertility, endocrine dysfunctions, diabetes, obesity, growth and reproductive disorders, hyperthyroidism, hypothyroidism and/or other pathologies/disorders.

NOV3a and 3b are homologous to a family of LRR/GPCR-like proteins. Thus, the NOV3 nucleic acids and polypeptides, antibodies and related compounds according to the invention will be useful in therapeutic and diagnostic applications implicated in, for example; abnormal angiogenesis, like cancer and more specifically aggressive, metastatic cancer, more specifically tumor of the lung, kidney, brain, liver and colon and/or other pathologies/disorders.

NOV4 is homologous to the Trypsin inhibitor-like family of proteins. Thus, NOV4 nucleic acids, polypeptides, antibodies and related compounds according to the invention will be useful in therapeutic and diagnostic applications implicated in, for example; chronic lung diseases, periodontitis, pancreatitis and/or other pathologies/disorders.

NOV5a, b, c, d, e, f, g, h and i are homologous to the apolipoprotein e-like family of proteins. Thus NOV5 nucleic acids, polypeptides, antibodies and related compounds according to the invention will be useful in therapeutic and diagnostic applications implicated in, for example; atherosclerosis, dysbetalipoproteinemia, hyperlipoproteinemia type III, xanthomatosis, coronary and/or peripheral vascular disease, hypothyroidism, systemic lupus erythematosus, diabetic acidosis, hypercholesterolemia, planar and tendon xanthomas, dysbetalipoproteinemia, hypercholesterolemia, accelerated vascular disease, Alzheimer disease, familial amyloidotic polyneuropathy and/or other pathologies/disorders.

NOV6 is homologous to the mastocytoma protease precursor -like family of proteins. Thus NOV6 nucleic acids, polypeptides, antibodies and related compounds according to the invention will be useful in therapeutic and diagnostic applications implicated in, for example; allergies, asthma and cystic fibrosis, cancers such as mastocytomas, vascular diseases and/or other pathologies/disorders.

NOV7 is homologous to members of the thymosin beta-4-like family of proteins. Thus, the NOV7 nucleic acids, polypeptides, antibodies and related compounds according to the invention will be useful in therapeutic and diagnostic applications implicated in, for example; prostate cancer, immunological and autoimmune disorders (ie hyperthyroidism), angiogenesis and wound healing, modulation of apoptosis, neurodegenerative and neuropsychiatric disorders, age-related disorders, pathological disorders involving spleen, thymus, lung, and peritoneal macrophages and Cancer and/or other pathologies/disorders.

NOV8 is homologous to the Protein-tyrosine-phosphatase -like family of proteins. Thus, NOV8 nucleic acids and polypeptides, antibodies and related compounds according to the invention will be useful in therapeutic and diagnostic applications implicated in, for example; hematopoiesic disorders; leukemogenesis; polycystic kidney disease; Oncogenesis such as lung, liver and ovarian cancers; apoptosis; type 2 diabetes and obesity; cell growth and possibly differentiation; non-Hodgkin lymphomas; musculoskeletal disorders; and CNS development disorders and/or other pathologies/disorders.

The NOVX nucleic acids and polypeptides can also be used to screen for molecules, which inhibit or enhance NOVX activity or function. Specifically, the nucleic acids and polypeptides according to the invention may be used as targets for the identification of small molecules that modulate or inhibit, *e.g*., neurogenesis, cell differentiation, cell proliferation, hematopoiesis, wound healing and angiogenesis.

Additional utilities for the NOVX nucleic acids and polypeptides according to the invention are disclosed herein.

### NOV1

A disclosed NOV1 nucleic acid of 620 nucleotides (also referred to as AC013554_dal) encoding a novel thioredoxin-like protein is shown in Table 1A. An open reading frame was identified beginning with an ATG initiation codon at nucleotides 282-284 and ending with a TAA codon at nucleotides 618-620. A putative untranslated region upstream from the initiation codon and downstream from the termination codon is underlined in Table 1A. The start and stop codons are in bold letters.

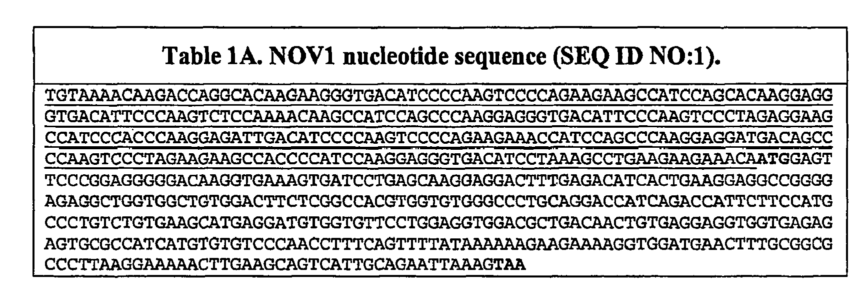

In a search of public sequence databases, the NOV1 nucleic acid sequence, ocated on the p31 region of chromsome 9 has 232 of 331 bases (70%) identical to a thioredoxin mRNA from *Ovis aries* (GENBANK-ID: OATHIORD|acc:Z25864). Public nucleotide databases include all GenBank databases and the GeneSeq patent database.

In all BLAST alignments herein, the "E-value" or "Expect" value is a numeric indication of the probability that the aligned sequences could have achieved their similarity to the BLAST query sequence by chance alone, within the database that was searched. For example, the probability that the subject ("Sbjct") retrieved from the NOV1 BLAST analysis, *e.g.,* thioredoxin mRNA from *Ovis aries,* matched the Query NOV1 sequence purely by chance is 608e-²⁷. The Expect value (E) is a parameter that describes the number of hits one can "expect" to see just by chance when searching a database of a particular size. It decreases exponentially with the Score (S) that is assigned to a match between two sequences. Essentially, the E value describes the random background noise that exists for matches between sequences.

The Expect value is used as a convenient way to create a significance threshold for reporting results. The default value used for blasting is typically set to 0.0001. In BLAST 2.0, the Expect value is also used instead of the P value (probability) to report the significance of matches. For example, an E value of one assigned to a hit can be interpreted as meaning that in a database of the current size one might expect to see one match with a similar score simply by chance. An E value of zero means that one would not expect to see any matches with a similar score simply by chance. See, e.g., http://www.ncbi.nlm.nih.gov/Education/BLASTinfo/. Occasionally, a string of X's or N's will result from a BLAST search. This is a result of automatic filtering of the query for low-complexity sequence that is performed to prevent artifactual hits. The filter substitutes any low-complexity sequence that it finds with the letter "N" in nucleotide sequence (e.g., "NNNNNNNNNNNNN") or the letter "X" in protein sequences (e.g., "XXXXXXXXX"). Low-complexity regions can result in high scores that reflect compositional bias rather than significant position-by-position alignment. Wootton and Federhen, Methods Enzymol 266:554-571, 1996.

The disclosed NOV1 polypeptide (SEQ ID NO:2) encoded by SEQ ID NO:1 has 112 amino acid residues and is presented in Table 1B using the one-letter amino acid code. Signal P, Psort and/or Hydropathy results predict that NOV1 has a signal peptide and is likely to be localized in the cytoplasm with a certainty of 0. 6500. In other embodiments, NOV1 may also be localized to the mitochondrial matrix space with acertainty of 0.1000, or the lysosome (lumen) with a certainty of 0.1000. The NOV1 peptide is not likely to have a signal peptide.

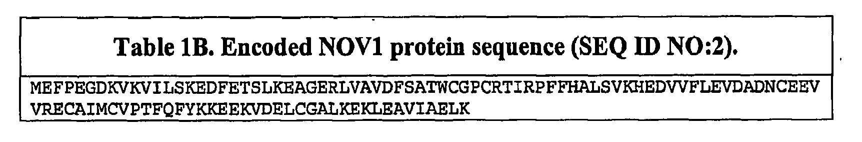

A search of public sequence databases reveals that the NOV1 amino acid sequence has 65 of 103 amino acid residues (63%) identical to, and 80 of 103 amino acid residues (77%) similar to, the 105 amino acid residue Thioredoxin - protein from Equus caballus (SPTREMBL-ACC:O97508) (E = 3.2e-³²). Public amino acid databases in clude the GenBank databases, SwissProt, PDB and PIR.

The disclosed NOV1 polypeptide has homology to the amino acid sequences shown in the BLASTP data listed in Table 1C.

| **Table 1C. BLAST results for NOV1** | | | | | |
|---|---|---|---|---|---|
| Gene Index/ Identifier | Protein/ Organism | Length (aa) | Identity (%) | Positives (%) | Expect |
| 097508 | equus caballus (horse). thioredoxin | 10.5 | 65/103 (63%) | 80/103, (78%) | le-33 |
| THIO_SHEEP | ovis aries (sheep). thioredoxin. | 104 | 65/103 (63%) | 80/103, (78%) | 2e-33 |
| THIO_BOVIN | bos taurus (bovine). thioredoxin | 104 | 65/103 (63%) | 80/103, (78%) | 3e-33 |
| THIO_MACMU | macaca mulatta (rhesus macaque). thioredoxin | 104 | 64/103 (62%) | 81/103, (79%) | 4e-33 |
| THIO_RAT | rattus norvegicus (rat). thioredoxin | 104 | 63/102 (62%) | 80/102, (78%) | 5e-33 |

The homology between these and other sequences is shown graphically in the ClustalW analysis shown in Table 1D. In the ClustalW alignment of the NOV1 protein, as well as all other ClustalW analyses herein, the black outlined amino acid residues indicate regions of conserved sequence (*i.e*., regions that may be required to preserve structural or functional properties), whereas non-highlighted amino acid residues are less conserved and can potentially be altered to a much broader extent without altering protein structure or function.

| | |
|---|---|
| **Table 1D. ClustalW Analysis of NOV1** | |
| 1) | Novel NOV1 (SEQ ID NO:2) |
| 2) | 097508 equus caballus (horse). thioredoxin (SEQ ID NO:38) |
| 3) | THIO_SHEEP ovis aries (sheep). thioredoxin (SEQ ID NO:39) |
| 4) | THIO_BOVIN bos taurus (bovine). thioredoxin (SEQ ID NO:40) |
| 5) | THIO_MACMU macaca mulatta (rhesus macaque). thioredoxin (SEQ ID NO:41) |
| 6) | THIO_RAT rattus norvegicus (rat). thioredoxin (SEQ ID NO:42) |
| 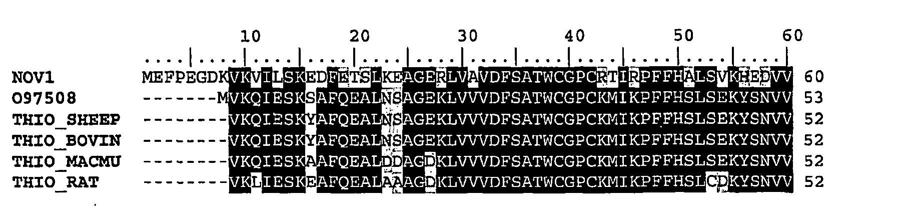 | |
| 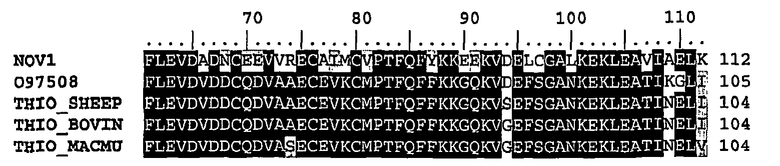 | |
| 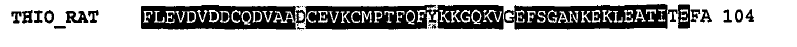 | |

The presence of identifiable domains in NOV1, as well as all other NOVX proteins, was determined by searches using software algorithms such as PROSITE, DOMAIN, Blocks, Pfam, ProDomain, and Prints, and then determining the Interpro number by crossing the domain match (or numbers) using the Interpro website (http:www.ebi.ac.uk/ interpro). DOMAIN results for NOV1 as disclosed in Tables 1E, were collected from the Conserved Domain Database (CDD) with Reverse Position Specific BLAST analyses. This BLAST analysis software samples domains found in the Smart and Pfam collections. For Table 1E and all successive DOMAIN sequence alignments, fully conserved single residues are indicated by black shading or by the sign (|) and "strong" semi-conserved residues are indicated by grey shading or by the sign (+). The "strong" group of conserved amino acid residues may be any one of the following groups of amino acids: STA, NEQK, NHQK, NDEQ, QHRK, MILV, MILF, HY, FYW.

Table 1E lists the domain description from DOMAIN analysis results against NOV1. This indicates that the NOV1 sequence has properties similar to those of other proteins known to contain this domain.

| **Table 1E. Domain Analysis of NOV1** |
|---|
| gnl\|Pfam\|pfam00085, thiored, Thioredoxin. (SEQ ID NO:73) |
| CD-Length = 108 residues, 97.2% aligned |
| Score = 89.4 bits (220), Expect = 1e-19 |

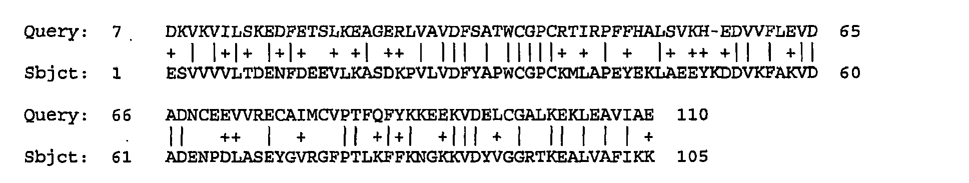

NOV1 also has high homology to members of the patp database shown in Table 1F.

| **Table 1F. PATP BLASTX results for NOV1** | | | | | |
|---|---|---|---|---|---|
| Gene Index/ Identifier | Protein/ Organism | Length (aa) | Identity (%) | Positives (%) | Expect |
| patp:AAP94809 | Human interleukin 2:human adult T cell leukaemia derived factor fusionprotein - Homo sapiens | 172 | 63/104 (60%) | 81/104 (77%) | 7.0e-31 |
| patp:AAP92141 | Recombinant human adult T cell leukaemia derived factor - Homo sapiens | 105 | 63/103 (61%) | 80/103 (77%) | 1.1e-30 |

Thioredoxin is an oxidoreductase enzyme of 12,000 daltons, containing a dithiol-disulfide active site. It is ubiquitous and found in many organisms from plants and bacteria to mammals. Multiple in vitro substrates for thioredoxin have been identified, including ribonuclease, choriogonadotropins, coagulation factors, glucocorticoid receptor, and insulin. Reduction of insulin is classically used as an activity test. Wollinan et al. (1988) identified a full-length cDNA clone coding for human thioredoxin. The open reading frame coded for a protein of 104 amino acids (excluding the initial methionine). This protein possessed the highly conserved enzymatic active site common to plant and bacterial thioredoxins: trp-cys-gly-pro-cys (amino acids 30-34). Tonissen and Wells (1991) determined that the TRX gene extends over 13 kb and consists of 5 exons encoding a 12-kD protein. Southern analysis demonstrated the presence of several TXN genes in the human genome. At least one of these is inactive, i.e., a pseudogene. Kaghad et al. (1994) also cloned the TXN gene and demonstrated that the 5 exons are separated by 4 introns. They determined the +1 transcription start point by primer extension. The +1 site is located 22 bp downstream from a TATAA box and defines a 5-prime untranslated region of 74 bp. Southern hybridization of genomic DNAs from several donors detected only 1 active TXN gene.

Using in situ chromosomal hybridization with a human TXN cDNA probe, Lafage-Pochitaloff-Huvale et al. (1989) localized the gene to 3p12-p11. Taketo et al. (1994) found that the homologous gene in mouse is located on chromosome 4 and that there is a processed Txn pseudogene in the proximal region of mouse chromosome 1.

Heppell-Parton et al. (1995) concluded that the correct chromosomal localization of the transcribed thioredoxin gene is 9q31. They discovered this both by analysis of a somatic cell hybrid panel and by fluorescence in situ hybridization of a YAC encoding the transcribed gene. The localization to chromosome 9 was confirmed by PCR amplification from a human/hamster somatic cell hybrid containing chromosome 9 as its only human chromosome. No amplification signals were detected in any of the other monochromosome hybrid cells. The location of the mouse thioredoxin gene on chromosome 4 is noteworthy because part of that chromosome shares homology with human chromosome 9.

Proteins internalized into the endocytic pathway are usually degraded. Efficient proteolysis requires denaturation, induced by acidic conditions within lysosomes, and reduction of inter- and intrachain disulfide bonds. Cytosolic reduction is mediated enzymatically by thioredoxin (TXN; 187700). (Arunachalam et al.; Nat. Acad. Sci. 97: 745-750, 2000) described a lysosomal thiol reductase, which they called GILT, that was optimally active at low pH and capable of catalyzing disulfide bond reduction both in vivo and in vitro. They found that the GILT gene encodes a predicted 261-amino acid protein composed of a 37-amino acid signal peptide and a 224-amino acid proform. The enzyme was expressed constitutively in antigen-presenting cells and was induced by gamma-interferon (147570) in other cell types, suggesting a potentially important role in antigenprocessing.

Selenium has been indirectly implicated in immunologic function and numerous nutritional studies over many years. Furthermore, HIV-infected persons are reported to have decreased levels of plasma selenium and selenium-containing glutathione peroxidase (e.g., 138321). For this reason, (Gladyshev et al.;Proc. Nat. Acad. Sci. 93: 6146-6151, 1996) initiated studies on selenium metabolism in human T cells. The authors identified one of the selenoproteins detected in T cells as thioredoxin reductase and demonstrated that the location of selenocysteine in this protein corresponds to a TGA codon in the cloned placental gene. The finding that T-cell thioredoxin reductase is a selenoenzyme that contains selenium in a conserved C-terminal region provides another example of the role of selenium in the major antioxidant enzyme system (i.e., thioredoxin-thioredoxin reductase), in addition to the well-known glutathione peroxidase enzyme system.

(Gorlatov and Stadtman; Proc. Nat. Acad. Sci. 95: 8520-8525, 1998) demonstrated the essential role of selenocysteine in thioredoxin reductase isolated from HeLa cells by alkylation studies. Selective alkylation of selenocysteine in the protein inhibited enzyme activity, and reduction with NADPH influenced affinity to heparin.

The disclosed NOV1 nucleic acid of the invention encoding a thioreductase-like protein includes the nucleic acid whose sequence is provided in Table 1A, or a fragment thereof. The invention also includes a mutant or variant nucleic acid any of whose bases may be changed from the corresponding base shown in Table 1A while still encoding a protein that maintains its thioreductase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 30 % percent of the bases may be so changed.

The disclosed NOV1 protein of the invention includes the thioreductase-like protein whose sequence is provided in Table 1B. The invention also includes a mutant or variant protein any of whose residues may be changed from the corresponding residue.shown in Table 2 while still encoding a protein that maintains its thioreductase-like activities and physiological functions, or a functional fragment thereof. In the mutant or variant protein, up to about 38 % percent of the residues may be so changed.

The invention further encompasses antibodies and antibody fragments, such as F_{ab} or (F_{ab})₂, that bind immunospecifically to any of the proteins of the invention.

The above defined information for this invention suggests that this thioreductase-like protein (NOV1) may function as a member of a "Thioredoxin family". Therefore, the NOV1 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV1 nucleic acids and proteins of the invention are useful in potential therapeutic applications implicated in cancer including but not limited to Inflamation, Autoimmune disorders, Aging and Cancer. For example, a cDNA encoding the thioreductase-like protein (NOV1) may be useful in gene therapy, and the thioreductase-like protein (NOV1) may be useful when administered to a subject in need thereof. By way of nonlimiting example, the compositions of the present invention will have efficacy for treatment of patients suffering from Inflamation, Autoimmune disorders, Aging and Cancer. The NOV1 nucleic acid encoding thioreductase-like protein, and the thioreductase-like protein of the invention, or fragments thereof, may further be useful in diagnostic applications, wherein the presence or amount of the nucleic acid or the protein are to be assessed.

NOV1 nucleic acids and polypeptides are further useful in the generation of antibodies that bind immuno-specifically to the novel NOV1 substances for use in therapeutic or diagnostic methods. These antibodies may be generated according to methods known in the art, using prediction from hydrophobicity charts, as described in the "Anti-NOVX Antibodies" section below. The disclosed NOV1 protein has multiple hydrophilic regions, each of which can be used as an immunogen. In one embodiment, a contemplated NOV1 epitope is from about amino acids 1 to 10. In another embodiment, a NOV1 epitope is from about amino acids 20 to 35. In additional embodiments, NOV1 epitopes are from about amino acids 45 to 50, from about amino acids 55 to 60, from about amino acid 65 to 75, and from about amino acids 90 to 115. These novel proteins can be used in assay systems for functional analysis of various human disorders, which will help in understanding of pathology of the disease and development of new drug targets for various disorders.

### NOV2

NOV2 includes two novel retinoic acid/SRA6-like proteins disclosed below. The disclosed proteins have been named NOV2a and NOV2b.

### NOV2a

A disclosed NOV2a nucleic acid of 2632 nucleotides (also referred to as 3277789.0.83_da1) encoding a novel Retinoic acid responsive/STRA-6-like protein is shown in Table 2A. An open reading frame was identified beginning with an ATG initiation codon at nucleotides 31-33 and ending with a TGA codon at nucleotides 2011-2013. A putative untranslated region upstream from the initiation codon and downstream from the termination codon is underlined in Table 2A, and the start and stop codons are in bold letters.

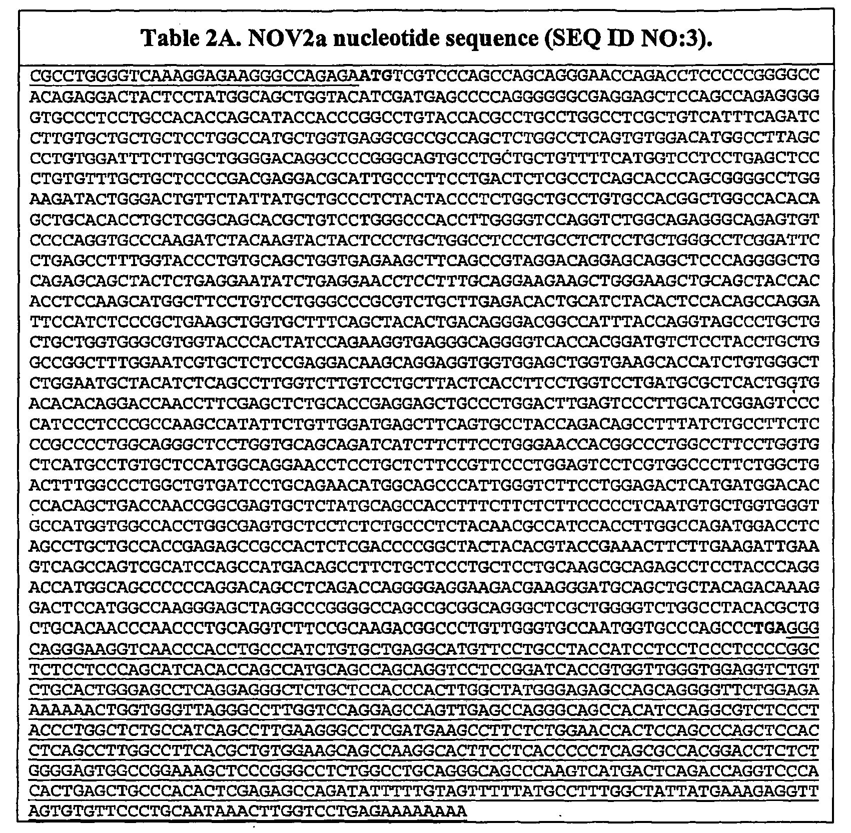

The NOV2a nucleic acid was identified by TblastN using CuraGen Corporation's sequence file for Retinoic acid responsive/STRA-6 or homolog as run against the Genomic Daily Files made available by GenBank or from files downloaded from the individual sequencing centers. The nucleic acid sequence was predicted from the genomic file Genbank by homology to a known Retinoic acid responsive/STRA-6 or homolog. Exons were predicted by homology and the intron/exon boundaries were determined using standard genetic rules. Exons were further selected and refined by means of similarity determination using multiple BLAST (for example, tBlastN, BlastX, and BlastN) searches, and, in some instances, GeneScan and Grail. Expressed sequences from both public and proprietary databases were also added when available to further define and complete the gene sequence. The DNA sequence was then manually corrected for apparent inconsistencies thereby obtaining the sequences encoding the full-length protein.

The disclosed NOV2a nucleic acid sequence, localized to chromsome 15, has 1511 of 2207 bases (68%) identical to a retinoic acid-responsive protein (Stra6) mRNA from Mus musculus (GENBANK-ID: AF062476|acc:AF062476 (GENBANK-ID: M77668)(E = 1.2e⁻¹⁸⁹).

A NOV2a polypeptide (SEQ ID NO:4) encoded by SEQ ID NO:3 has 660 amino acid residues and is presented using the one-letter code in Table 2B. Signal P, Psort and/or Hydropathy results predict that NOV2A contain a signal peptide and is likely to be localized in the plasmam membrane with a certainty of 0.6000. In other embodiments, NOV2a may also be localized to the Golgi body with acertainty of 0.4000, the endoplasmic reticulum (membrane) with a certainty of 0.3000, or the microbody (peroxisome) with a certainty of 0.3000. The most likely cleavage site for a NOV2A peptide is between amino acids 8 and 9, at: AGN-QT.

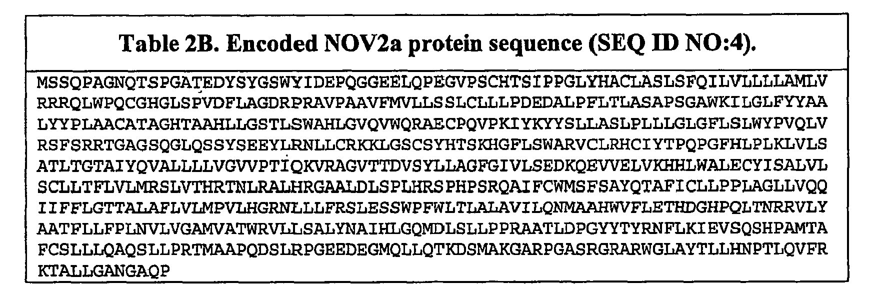

The disclosed NOV2a amino acid sequence has 497 of 670 amino acid residues (74%) identical to, and 556 of 670 amino acid residues (82%) similar to, the 670 amino acid residue Retinoic Acid-Responsive protein from Mus musculus (SPTREMBL-ACC:070491) (E = 7.1e⁻²⁵⁷).

NOV2a also has high homology to members of the patp database shown in Table 2C.

| **Table 2C PATP BLASTX results for NOV2a** | | | | | |
|---|---|---|---|---|---|
| Gene Index/ Identifier | Protein/ Organism | Length (aa) | Identity (%) | Positives (%) | Expect |
| patp:AAB88572 | Human hydrophobic domain containing protein clone HP10713 #36 - Homosapiens | 667 | 649/667 (97%) | 650/667 (97%) | 0.0 |
| patp:AAB94108 | Human protein sequence SEQ ID NO:14340 - Homo sapiens | 560 | 549/560 (98%) | 550/560 (98%) | 2.5e-289 |

### NOV2b

A disclosed NOV2b nucleic acid of 2663 nucleotides (also referred to as CG52276-07) encoding a novel STRA6-like protein is shown in Table 2D. An open reading frame was identified beginning with an ATG initiation codon at nucleotides 1-3 and ending with a TGA codon at nucleotides 2068-2070. A putative untranslated region upstream from the initiation codon and downstream from the termination codon is underlined in Table 2D, and the start and stop codons are in bold letters.

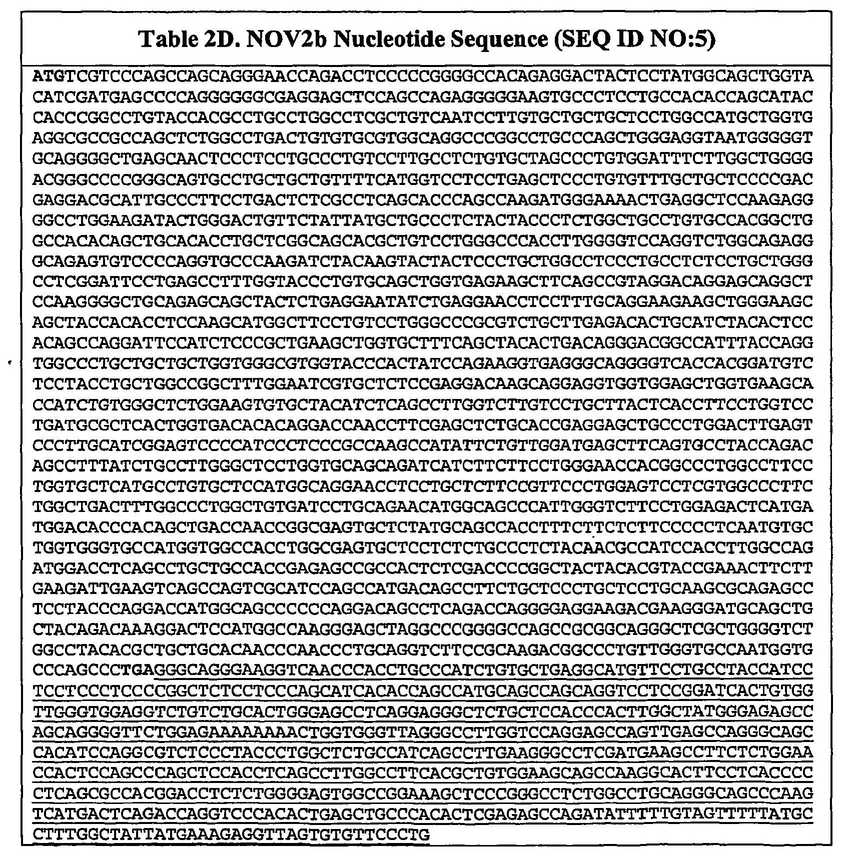

The disclosed NOV2b nucleic acid sequence has 2395 of 2444 bases (97%) identical to a gb:GENBANK-ID:AF352728|acc:AF352728.1 mRNA from Homo sapiens (Homo sapiens STRA6 isoform 1 mRNA, complete cds, alternatively spliced) (E = 0.0).

A disclosed NOV2b polypeptide (SEQ ID NO:6) encoded by SEQ ID NO:5 has 689 amino acid residues and is presented using the one-letter code in Table 2E. Signal P, Psort and/or Hydropathy results predict that NOV2b has a signal peptide and is likely to be localized at the plasma membrane with a certainty of 0.6000. In other embodiments, NOV2b could be localized at the Golgi body with a certainty of 0.4000, the endoplasmic reticulum (membrane) with a certainty of 0.3000, or a microbody (peroxisome) with a certainty of 0.3000. The most likely cleavage site for a NOV2b peptide is between amino acids 8 and 9, at AGN-QT.

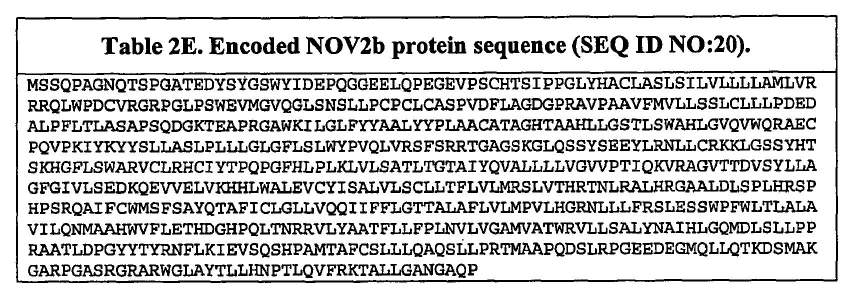

The disclosed NOV2b amino acid sequence has 578 of 579 amino acid residues (99%) identical to, and 578 of 579 amino acid residues (99%) similar to, the 667 amino acid residue ptnr:SPTREMBL-ACC:Q9BX79 protein from Homo sapiens (Human) (STRA6 ISOFORM 1) (E=0.0).

NOV2b maps to chromosome 15. This assignment was made using mapping information associated with genomic clones, public genes and ESTs sharing sequence identity with the disclosed sequence and CuraGen Corporation's Electronic Northern bioinformatic tool.

NOV2b is expressed in at least the following tissues: Brain, Cervix, Heart, Kidney, Lymph node, Lymphoid tissue, Ovary, Pituitary Gland, Placenta, Retina, Temporal Lobe, Thyroid, Uterus. Expression information was derived from the tissue sources of the sequences that were included in the derivation of the sequence of NOV2b.

NOV2a also homology to the amino acid sequences shown in the BLASTP data listed in Table 2F.

| **Table 2F. BLAST results for NOV2a** | | | | | |
|---|---|---|---|---|---|
| Gene Index/ Identifier | Protein/ Organism | Length (aa) | Identity (%) | Positives (%) | Expect |
| Q9BX79 | homo sapiens (human). stra6 isoform 1 | 667 | 532/675 (79%) | 533/675, (79%) | 0.0 |
| Q9BX78 | homo sapiens (human). stra6 isoform 2 | 658 | 522/671 (78%) | 525/671, (78%) | 0.0 |
| Q9H9U8 | homo sapiens (human). cdna f1j12541 fis, clone nt2rm4000433, moderately similar to mus musculus retinoic acid-responsive protein (stra6) mrna | 560 | 467/566 (83%) | 468/566, (83%) | 0.0 |
| 070491 | mus musculus (mouse). retinoic acid-responsive protein | 670 | 403/679 (59%) | 455/679, (67%) | 0.0 |

The homology of these sequences is shown graphically in the Clusta1W analysis shown in Table 2G.

| **Table 2G. ClustalW Analysis of NOV2** | |
|---|---|
| 1) | NOV2a (SEQ ID NO:4) |
| 2) | NOV2b (SEQ ID NO:6) |
| 2) | Q9BX79 homo sapiens (human). stra6 isoform 1 (SEQ ID NO:43) |
| 3) | Q9BX78 homo sapiens (human). stra6 isoform 2 (SEQ ID NO:44) |
| 4) | Q9H9U8 homo sapiens (human). cdna flj12541 fis, clone nt2rm4000433, (SEQ ID NO:45) |
| 5) | 070491 mus musculus (mouse). retinoic acid-responsive protein (SEQ ID NO:46) |
| 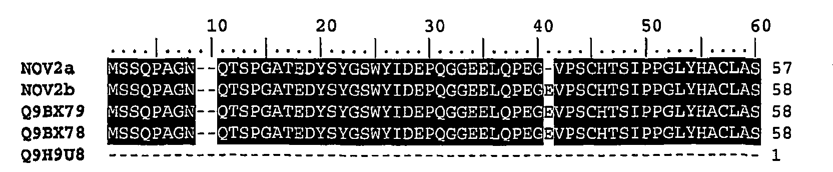 | |
| 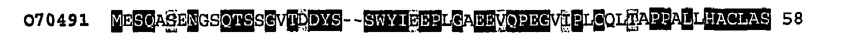 | |
| 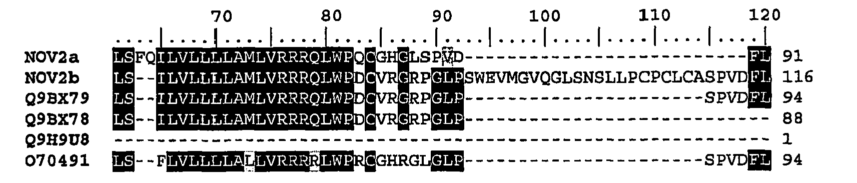 | |
| 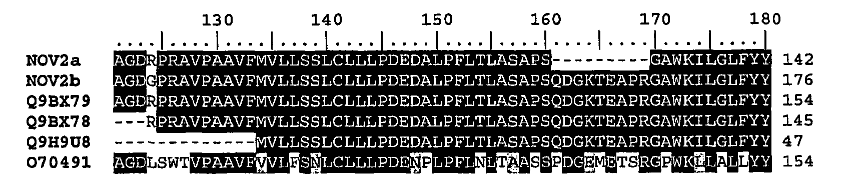 | |
| 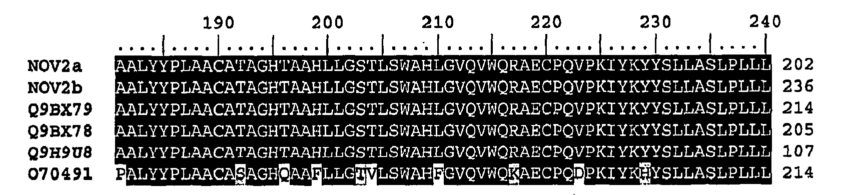 | |
| 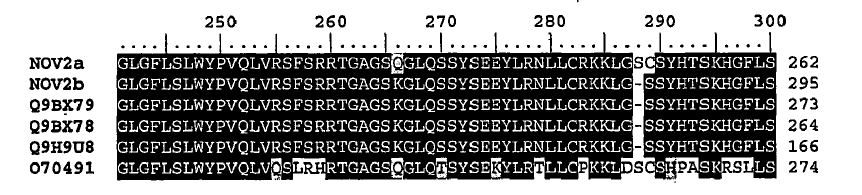 | |
| 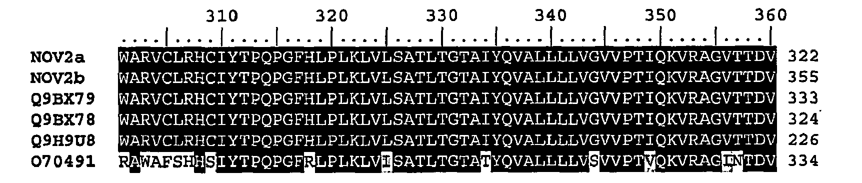 | |
| 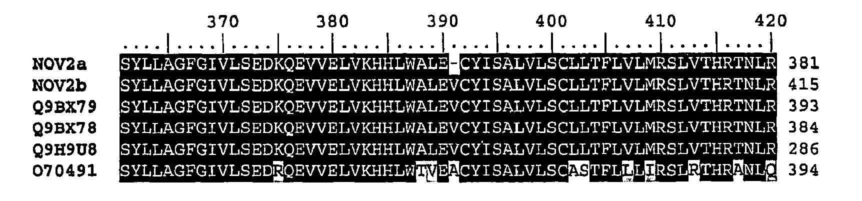 | |
| 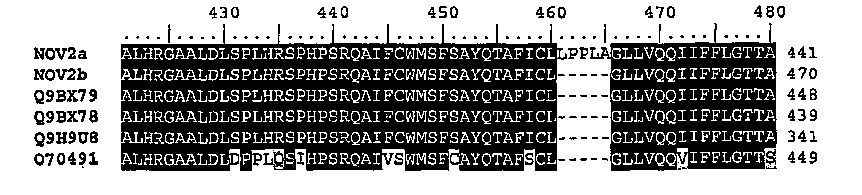 | |
| 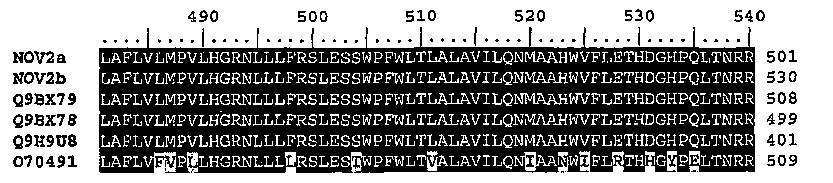 | |
| 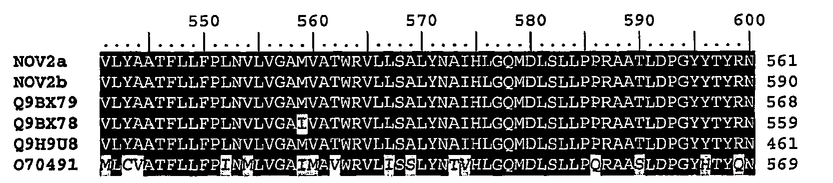 | |
| 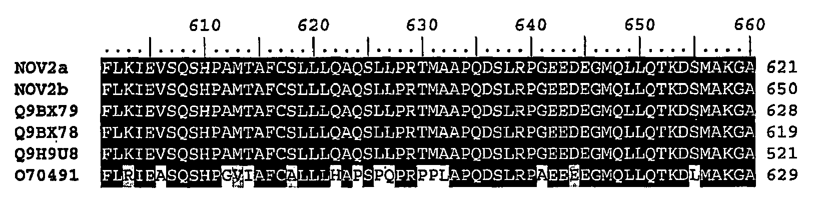 | |
| 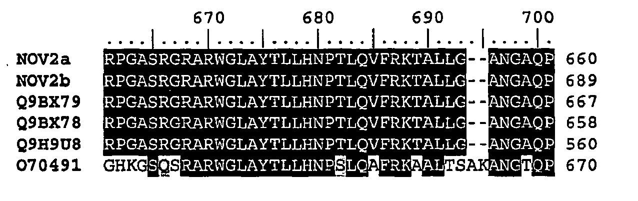 | |

Carcinogenesis involves inactivation or subversion of the normal controls of proliferation, differentiation, and apoptosis (Hurst RE, et.al.; Adv Exp Med Biol 1999;462:449-67). However, these controls are robust, redundant, and interlinked at the gene expression levels, regulation of mRNA lifetimes, transcription, and recycling of proteins. One of the central systems of control of proliferation, differentiation and apoptosis is retinoid signaling. The hRAR alpha nuclear receptor occupies a central position with respect to induction of gene transcription in that when bound to appropriate retinoid ligands, its homodimers and heterodimers with hRXR alpha regulate the transcription of a number of retinoid-responsive genes. These include genes in other signaling pathways, so that the whole forms a complex network. In this study the authors showed that simple, cause-effect interpretations in terms of hRAR alpha gene transcription being the central regulatory event would not describe the retinoid-responsive gene network. A set of cultured bladder-derived cells representing different stages of bladder tumorigenesis formed a model system. It consisted of 2 immortalized bladder cell lines (HUC-BC and HUC-PC), one squamous cell carcinoma cell line (SCaBER), one papilloma line (RT4), and 4 transitional cell carcinomas (TCC-Sup, 5637, T24, J82) of varying stages and grades. This set of cells were used to model the range of behaviors of bladder cancers. Relative gene expression before (constitutive) and after treatment with 10 microM all-trans-retinoic acid (aTRA) was measured for androgen and estrogen receptor; a set of genes involved with retinoid metabolism and action, hRAR alpha and beta, hRXR alpha and beta CRBP, CRABP I and II; and for signaling genes that are known to be sensitive to retinoic acid, EGFR, cytokine MK, ICAM I and transglutaminase. The phenotype for inhibition of proliferation and for apoptotic response to both aTRA and the synthetic retinoid 4-HPR was determined. Transfection with a CAT-containing plasmid containing an aTRA-sensitive promoter was used to determine if the common retinoic acid responsive element (RARE)-dependent pathway for retinoid regulation of gene expression was active. Each of the genes selected is known from previous studies to react to aTRA in a certain way, either by up- or down-regulation of the message and protein. A complex data set not readily interpretable by simple cause and effect was observed. While all cell lines expressed high levels of the mRNAs for hRXR alpha and beta that were not altered by treatment with exogenous aTRA, constitutive and stimulated responses of the other genes varied widely among the cell lines. For example, CRABP I was not expressed by J82, T24, 5637 and RT4, but was expressed at low levels that did not change in SCaBER and at moderate levels that decreased, increased, or decreased sharply in HUC-BC, TCC-Sup and HUC-PC, respectively. The expression of hRAR alpha, which governs the expression of many retinoid-sensitive genes, was expressed at moderate to high levels in all cell lines, but in some it was sharply upregulated (TCC-Sup, HUC-PC and J82), remained constant (5637 and HUC-BC), or was down-regulated (SCaBER, T24 and RT4). The phenotypes for inhibition of proliferation showed no obvious relationship to the expression of any single gene, but cell lines that were inhibited by aTRA (HUC-BC and TCC-Sup) were not sensitive to 4-HPR, and vice versa. One line (RT4) was insensitive to either retinoid. Transfection showed very little retinoid-stimulated transfection of the CAT reporter gene with RT4 or HUC-PC. About 2-fold enhancement transactivation was observed with SCaBER, HUC-BC, J82 and T24 cells and 3-8 fold with 5637, TCC-Sup cells. In HUC-BC, a G to T point mutation was found at position 606 of the hRAR alpha gene. This mutation would substitute tyrosine for asparagine in a highly conserved domain. These data indicate that retinoid signaling is probably a frequent target of inactivation in bladder carcinogenesis.

Waliszewski P, et.al. ( Mol Cell Endocrinol 1999 Feb 25;148(1-2):55-65) investigated the presence and functionality of retinoid signaling pathways in human urinary bladder carcinoma and SV40-immortalized uroepithelial cell lines. Only two of eight cell lines were proliferation-inhibited by 10 microM of either all-trans or 13-cis-retinoic acid. Transactivation of the CAT gene under control of a retinoid-responsive element demonstrated functionality of the signaling pathway in both sensitive cell lines and four of six resistant cell lines. Relative RT-PCR analysis of a panel of retinoid-responsive and inducible genes demonstrated changes in expression levels of all the genes in response to retinoic acid treatment together with numerous aberrations dysregulations. They concluded that retinoid signaling may be a target for inactivation during tumorigenesis by uncoupling gene expression, proliferation and differentiation. Therefore retinoids are more likely to be effective for chemoprevention than for treatment of bladder carcinomas.

The proliferative effects of retinoids were examined in the MC-26 and LoVo colon adenocarcinoma cell lines (Stewart LV, et.al.; Exp Cell Res 1997 Jun 15;233(2):321-9). The proliferation of the LoVo cell line was not altered in the presence of the retinoids all trans-retinoic acid (atRA) and 9-cis-retinoic acid (9-cis-RA). Both retinoids, however, stimulated the growth, as measured by cell proliferation, of MC-26 cells. atRA and 9-cis-RA were equipotent in increasing MC-26 cell proliferation, suggesting that the growth stimulation is mediated by one or more retinoic acid receptors (RARs). To determine the RAR which might be responsible for this growth stimulatory effect, the authors characterized the RAR subtypes which were present in both cell lines. mRNA for the RAR alpha, RAR beta, and RAR gamma were detected in the MC-26 cell. Of the RARs present in MC-26 cells, the RAR alpha does not mediate the growth stimulatory effects of retinoids, for a selective RAR alpha antagonist was unable to prevent the retinoid-induced increase in MC-26 cell growth. RAR alpha, RAR beta, and RAR gamma mRNA are also expressed in the LoVo cell line; the lack of growth-stimulation by retinoids in LoVo cells, therefore, does not seem to be due to the absence of RARs. The results obtained in these experiments demonstrate that the growth response elicited by retinoids can vary between colon cancer cells and that the differences in response may not be solely determined by the RAR subtypes which are expressed in a colon cancer cell line.

Retinoic acids (RAs), well characterized regulators of proliferation and differentiation, partly re-differentiate follicular thyroid carcinoma cell lines (FTC-133, FTC-238, and HTC-TSHr) as well as SV40-transfected immortalized thyroid cell lines (ori3 and 7751) (Schmutzler C, et.al.;Exp Clin Endocrinol Diabetes 1996;104 Suppl 4:16-9). This is indicated by the stimulation of type I 5'-deiodinase and other differentiation markers. As demonstrated by RT-PCR, electrophoretic mobility shift, and [3H]-retinoic acid binding assays, thyroid carcinoma cell lines express RA receptor mRNAs and functional ligand- and DNA-binding receptor proteins able to mediate RA-dependent signal transduction. Together, these properties make these thyroid-derived cell lines useful in vitro models for studying the effects of an RA re-differentiation therapy of thyroid cancer.

The chemotherapeutic agent retinoic acid (RA) inhibits the proliferation and invasion of many tumor types(Vo HP, et.al; Anticancer Res 1998 Jan-Feb;18(1A):217-24). RA chemotherapy in head and neck squamous cell carcinoma (SCC) patients reduces recurrence and induces regression of premalignant lesions. The effects of RA are mediated by both cytoplasmic and nuclear proteins. In the nucleus, a family of ligand-dependent transcription factors, the retinoic acid receptors (RAR) and the retinoid X receptors (RXR), regulate target gene response to RA. In the cytoplasm, the cellular retinoic acid binding proteins I and II (CRABP) regulate intracellular RA concentration, transport, and metabolism. Alterations in CRABP expression have been shown to affect target gene response and the phenotype of cancer cells. To elucidate the role of these proteins in mediating the RA response, we examined target gene expression and malignant phenotype in SCC25 cells expressing an antisense CRABP II construct. RA induced CRABP II mRNA levels 2 fold in SCC25 cells by transcriptional upregulation. Expression of the antisense construct reduced CRABP II expression to undetectable levels. Inhibition of CRABP II expression resulted in significant downregulation of RA responsive genes. These reductions were the result of decreased transcription from RA responsive promoters. Surprisingly, clones expressing the antisense CRABP construct were less sensitive to RA mediated inhibition of proliferation. These clones were also less invasive in an in vitro invasion assay, likely due to downregulation of matrix metalloproteinase activity. We conclude that CRABP II affects the transcription of RA responsive genes which regulate proliferation and invasion of head and neck SCCs.

All-trans-retinoic acid (trans-RA) and other retinoids exert anticancer effects through two types of retinoid receptors, the RA receptors (RARs) and retinoid X receptors (RXRs)( Wu Q, et.al.; Mol Cell Biol 1997 Nov;17(11):6598-608). Previous studies demonstrated that the growth-inhibitory effects of trans-RA and related retinoids are impaired in certain estrogen-independent breast cancer cell lines due to their lower levels of RAR alpha and RARbeta. In this study, we evaluated several synthetic retinoids for their ability to induce growth inhibition and apoptosis in both trans-RA-sensitive and trans-RA-resistant breast cancer cell lines. Our results demonstrate that RXR-selective retinoids, particularly in combination with RAR-selective retinoids, could significantly induce RARbeta and inhibit the growth and induce the apoptosis of trans-RA-resistant, RAR alpha-deficient MDA-MB-231 cells but had low activity against trans-RA-sensitive ZR-75-1 cells that express high levels of RAR alpha. Using gel retardation and transient transfection assays, we found that the effects of RXR-selective retinoids on MDA-MB-231 cells were most likely mediated by RXR-nur77 heterodimers that bound to the RA response element in the RARbeta promoter and activated the RARbeta promoter in response to RXR-selective retinoids. In contrast, growth inhibition by RAR-selective retinoids in trans-RA-sensitive, RAR alpha-expressing cells most probably occurred through RXR-RAR alpha heterodimers that also bound to and activated the RARbeta promoter. In MDA-MB-231 clones stably expressing RAR alpha, both RARbeta induction and growth inhibition by RXR-selective retinoids were suppressed, while the effects of RAR-selective retinoids were enhanced. Together, our results demonstrate that activation of RXR can inhibit the growth of trans-RA-resistant MDA-MB-231 breast cancer cells and suggest that low cellular RAR alpha may regulate the signaling switch from RAR-mediated to RXR-mediated growth inhibition in breast cancer cells.

Retinoic acid plays important roles in development, growth and differentiation by regulating the expression of target genes. A new retinoic acid-inducible gene, Stra6, has been identified in P19 embryonal carcinoma cells using a subtractive hybridization cDNA cloning technique by Bouillet and coworkers (Mech Dev 1997;63:173-86). Stra6 codes for a very hydrophobic membrane protein of a new type, which does not display similarities with previously characterized integral membrane proteins. Stra6, which exhibits a specific pattern of expression during development and in the adult, is strongly expressed at the level of blood-organ barriers. Interestingly, in testis Sertoli cells, Stra6 has a spermatogenic cycle-dependent expression which is lost in testes of RAR alpha null mutants where Stra6 is expressed in all tubules. This finding suggests that the Stra6 protein may be a component of an as yet unidentified transport machinery.

The NOV2 nucleic acids and proteins of the invention are useful in potential therapeutic applications implicated in Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, scleroderma, transplantation, autoimmune disease, allergies, immunodeficiencies, renal artery stenosis, interstitial nephritis, glomerulonephritis, polycystic kidney disease, systemic lupus erythematosus, renal tubular acidosis, IgA nephropathy, Lesch-Nyhan syndrome, lymphedema, hemophilia, hypercoagulation, idiopathic thrombocytopenic purpura, graft versus host disease (GVHD), endometriosis, fertility, endocrine dysfunctions, diabetes, obesity, growth and reproductive disorders, hyperthyroidism, hypothyroidism, and other diseases, disorders and conditions of the like. For example, a cDNA encoding the Retinoic acid responsive/STRA-6-like protein (NOV2) may be useful in gene therapy, and the Retinoic acid responsive/STRA-6-like protein (NOV2) may be useful when administered to a subject in need thereof. By way of nonlimiting example, the compositions of the present invention will have efficacy for treatment of patients suffering from Inflamation, Autoimmune disorders, Aging or Cancer. The NOV2A nucleic acid encoding Retinoic acid responsive/STRA-6-like protein, and the Retinoic acid responsive/STRA-6-like protein of the invention, or fragments thereof, may further be useful in diagnostic applications, wherein the presence or amount of the nucleic acid or the protein are to be assessed.

NOV2 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. The disclosed NOV2 protein has multiple hydrophilic regions, each of which can be used as an immunogen. In one embodiment, a contemplated NOV2a epitope is from about amino acids 20 to 30. In another embodiment, a NOV2a epitope is from about amino acids 40 to 60. In additional embodiments, NOV2a epitopes are from about amino acids 75 to 80, from about 100 to 110, from about amino acids 180 to 190, from about amino acids 220 to 260, from about amino acids 310 to 330, from about amino acids 350 to 370, from about amino acids 380 to 400, from about amino acids 470 to 510, from about amino acids 550 to 570, and from about amino acids 590 to 650. These novel proteins can be used in assay systems for functional analysis of various human disorders, which are useful in understanding of pathology of the disease and development of new drug targets for various disorders.

In another embodiment, a contemplated NOV2b epitope is from about amino acids 5 to 50. In another embodiment, a NOV2b epitope is from about amino acids 80 to 90. In additional embodiments, NOV2b epitopes are from about amino acids 140 to 160, from about 190 to 210, from about amino acids 230 to 290, from about amino acids 360 to 365, from about amino acids 390 to 440, from about amino acids 550 to 590, and from about amino acids 610 to 670. These novel proteins can be used in assay systems for functional analysis of various human disorders, which are useful in understanding of pathology of the disease and development of new drug targets for various disorders.

### NOV3

NOV3 includes two novel LRR/GPCR-like proteins disclosed below. The disclosed proteins have been named NOV3a and NOV3b.

### NOV3a

A disclosed NOV3a nucleic acid of 7892 nucleotides (also referred to as 128900861ext) encoding a novel LRR/GPCR -like protein is shown in Table 3A. An open reading frame was identified beginning with an ATG initiation codon at nucleotides 1-3 and ending with a TAA codon at nucleotides 5728-5730. A putative untranslated region downstream from the termination codon is underlined in Table 3A, and the start and stop codons are in bold letters.

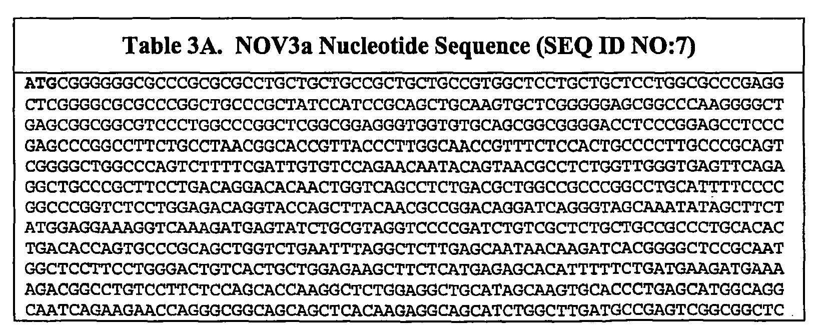

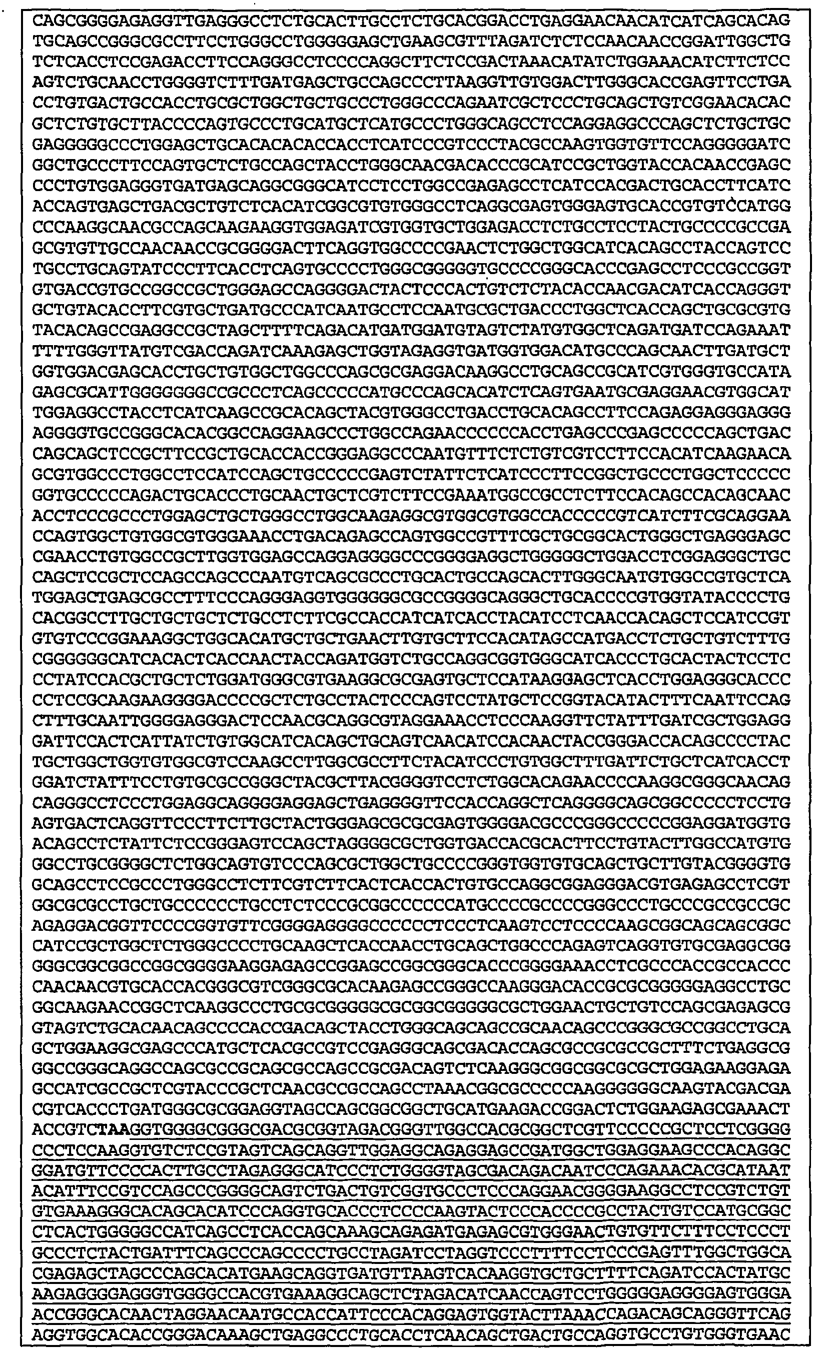

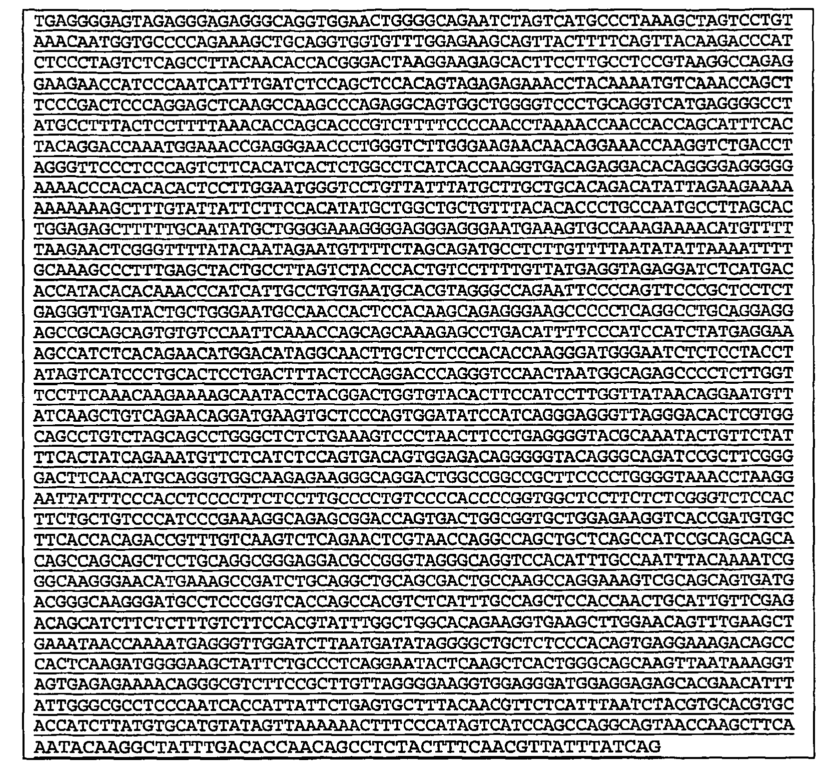

The disclosed NOV3a nucleic acid sequence of this invention, localized to the p11.2 region of chromosome 8, has 2894 of 2908 bases (99% identity) with KIAA1531 mRNA from Homo sapiens (GENBANK-ID: AB040964) (E = 0.0).

Additional BLASTN results are shown in Table 3B.

| **Table 3B. BLASTN alignments of NOV3a** | | | |
|---|---|---|---|
| Sequences producing High-scoring Segment Pairs: \ | High Score | Prob P(N) | Reading Frame |
| gb:GENBANK-ID:AB040964\|acc:AB040964 Homo sapiens mRNA for... | 14396 | 0.0 | 2 |
| gb:GENBANK-ID:HSM800904\|acc:AL110244 Homo sapiens mRNA; c... | 13169 | 0.0 | 2 |
| gb:GENBANK-ID:AP000501\|acc:AP000501 Homo sapiens genomic ... | 22592 | 0.0 | 15 |
| gb:GENBANK-ID:AK001914\|acc:AK001914 Homo sapiens cDNA FLJ... | 6650 | 9.4e-295 | 1 |
| gb:GENBANK-ID:HSM802368\|acc:AL137610 Homo sapiens mRNA; c... | 5545 | 1.3e-244 | 1 |

A NOV3a polypeptide (SEQ ID NO:8) encoded by SEQ ID NO:7 is 1542 amino acid residues and is presented using the one-letter code in Table 3C. Signal P, Psort and/or Hydropathy results predict that NOV3a contains a signal peptide and is likely to be localized to the plasma membrane with a certainty of 0.6400. In other embodiments, NOV3a may also be localized to the Golgi body with a certainty of 0.4000, the endoplasmic reticulum (membrane) with a certainty of 0.3700, or the endoplasmic reticulum (lumen) with a certainty of 0.1000. The most likely cleavage site for a NOV3a peptide is between amino acids 26 and 27, at: ARG-AP.

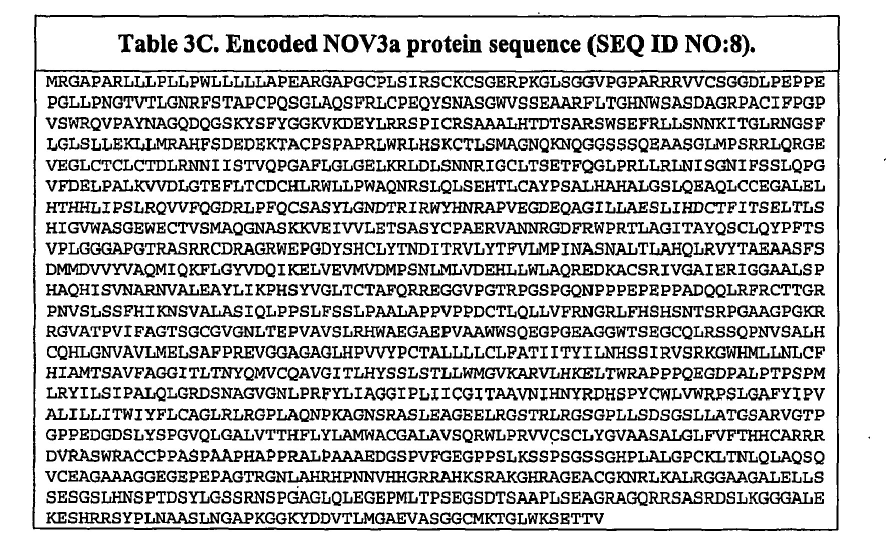

The disclosed NOV3a amino acid sequence has 638 of 647 amino acid residues (98%) identical to, and 643 of 647 amino acid residues (99%) similar to, the KIAA1531 PROTEIN of 1060 amino acid residue LRR/GPCR-like protein from Homo sapiens (BAA96055) (E = 0.0).

Additional BLASTP results for NOV3a are shown in Table 3D

| **Table 3D BLASTP alignments of NOV3a** | | | |
|---|---|---|---|
| Sequences producing High-scoring Segment Pairs: \ | High Score | Prob P(N) | Reading Frame |
| ptnr:TREMBLNEW-ACC:BAA96055 KIAA1531 PROTEIN - Homo sapie... | 3381 | 0.0 | 2 |
| ptnr:SPTREMBL-ACC:Q9UFY4 HYPOTHETICAL 34.9 KDA PROTEIN - ... | 1804 | 7.1e-186 | 1 |
| ptnr:SPTREMEL-ACC:Q9VYF1 CG15744 PROTEIN - Drosophila mel... | 273 | 7.1e-60 | 6 |
| ptnr:SPTREMBL-ACC:O35161 SEVEN-PASS TRANSMEMBRANE RECEPTO... | 217 | 7.1e-19 | 3 |
| ptnr:REMTREMBL-ACC:E1260970 SEQUENCE 1 FROM PATENT WO9707... | 213 | 2.9e-17 | 3 |
| ptnr:SPTREMBL-ACC:O75092 MEGF2 - Homo sapiens (Human), 13... | 219 | 4.9e-17 | 4 |
| ptnr:REMTREMBL-ACC:E1260972 SEQUENCE 3 FROM PATENT WO9707... | 213 | 1.8e-16 | 3 |
| ptnr:SPTREMBL-ACC:O95722 DJ1163J1.1 (ORTHOLOG OF MOUSE TR... | 203 | 3.3e-16 | 4 |
| ptnr:TREMBLNEW-ACC:AAF61930 PROTOCADHERIN FLAMINGO 2 - Ho... | 203 | 1.2e-15 | 4 |
| ptnr:SPTREMBL-ACC:094898 KIAA0806 PROTEIN - Homo sapiens ... | 240 | 1.4e-15 | 2 |
| ptnr:SPTREMBL-ACC:P70193 MEMBRANE GLYCOPROTEIN - Mus muse... | 234 | 1.4e-15 | 3 |
| ptnr:SPTREMBL-ACC:088278 MEGF2 - Rattus norvegicus (Rat),... | 190 | 1.6e-15 | 6 |
| ptnr:TREMBLNEW-ACC:AAF61929 PROTOCADHERIN FLAMINGO 1 - Ho... | 214 | 8.2e-15 | 4 |

NOV3a also has high homology to members of the patp database shown in Table 3E.

| **Table 3E PATP BLASTX results for NOV3a** | | | | | |
|---|---|---|---|---|---|
| Gene Index/ Identifier | Protein/ Organism | Length (aa) | Identity (%) | Positives (%) | Expect |
| patp:AAB71863 | Human h15571 GPCR - Homo sapiens | 1338 | 779/811 (96%) | 785/811 (96%) | 0.0 |
| patp:AAB71863 | Human h15571 GPCR - Homo sapiens | 1338 | 453/453 (100%) | 453/453 (100%) | 0.0 |

NOV3a is expressed in at least the following tissues: Blood, Brain, Breast, Colon, Ear, Germ Cell, Heart, Kidney, Lung, Muscle, Pancreas, Placenta, Prostate, Skin, Stomach, Testis, Uterus, Whole embryo, colon, lung, ovary, uterus Eye, Prostate, Tonsil, placenta, and thymus..

In addition, the sequence is predicted to be expressed in the following tissues based on the expression pattern of its homolog, SeqCalling assembly 128900861: fetal heart, fetal lung fetal retina fetal_liver_spleen, total fetal tissue and multiple tumor from colon, lung, geerm cells, kidney prostate.

Sage analysis of the 2 Unigene clusters (Example 4) indicates expression in several brain, ovarian and prostate tumors.

### NOV3b

The cDNA coding for the a domain of NOV3a from residue 444 to 937 was targeted for "in-frame" cloning by PCR. The PCR template is based on human cDNA(s).

The following oligonucleotide primers were used to clone the target cDNA sequence:
**F2** 5'-GGATCC CGGCTGCCCTTCCAGTGCTCTGCCAGCTACCTGG-3' (SEQ ID NO:135)
**R1** 5'-CTCGAG GCTCAGCTCCATGAGCACGGCCACATTGCC-3' (SEQ ID NO:136)
For downstream cloning purposes, the forward primer includes an in-frame BamHI restriction site and the reverse primer contains an in-frame XhoI restriction site.

Two parallel PCR reactions were set up using a total of 0.5-1.0 ng human pooled cDNAs as template for each reaction. The pool is composed of 5 micrograms of each of the following human tissue cDNAs: adrenal gland, whole brain, amygdala, cerebellum, thalamus, bone marrow, fetal brain, fetal kidney, fetal liver, fetal lung, heart, kidney, liver, lymphoma, Burkitt's Raji cell line, mammary gland, pancreas, pituitary gland, placenta, prostate, salivary gland, skeletal muscle, small Intestine, spleen, stomach, thyroid, trachea, uterus.

When the tissue of expression is known and available, the second PCR was performed using the above primers and 0.5ng-1.0 ng of one of the following human tissue cDNAs:
skeleton muscle, testis, mammary gland, adrenal gland, ovary, colon, normal cerebellum, normal adipose, normal skin, bone marrow, brain amygdala, brain hippocampus, brain substantia nigra, brain thalamus, thyroid, fetal lung, fetal liver, fetal brain, kidney, heart, spleen, uterus, pituitary gland, lymph node, salivary gland, small intestine, prostate, placenta, spinal cord, peripheral blood, trachea, stomach, pancreas, hypothalamus.

The reaction mixtures contained 2 microliters of each of the primers (original concentration: 5 pmol/ul), 1 microliter of 10mM dNTP (Clontech Laboratories, Palo Alto CA) and 1 microliter of 50xAdvantage-HF 2 polymerase (Clontech Laboratories) in 50 microliter-reaction volume. The following reaction conditions were used:
PCR condition 1:
   a) 96°C 3 minutes
   b) 96°C 30 seconds denaturation
   c) 60°C 30 seconds, primer annealing
   d) 72°C 6 minutes extension
      Repeat steps b-d 15 times
   e) 96°C 15 seconds denaturation
   f) 60°C 30 seconds, primer annealing
   g) 72°C 6 minutes extension
      Repeat steps e-g 29 times
   e) 72°C 10 minutes final extension
PCR condition 2:
   a) 96°C 3 minutes
   b) 96°C 15 seconds denaturation
   c) 76°C 30 seconds, primer annealing, reducing the temperature by 1 °C per cycle
   d) 72°C 4 minutes extension
      Repeat steps b-d 34 times
   e) 72°C 10 minutes final extension

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:
SF1: CCTCTGCCTCCTACTGCCCC (SEQ ID NO:93)
SF2: GTGCTGATGCCCATCAATGCCT (SEQ ID NO:94
SF3: CGCATCGTGGGTGCCATAG (SEQ ID NO:95)
SF4: ACCGGGAGGCCCAATGTTTCTCT (SEQ ID NO:96)
SF5: CAGCCACAGCAACACCTCCC (SEQ ID NO:97)
SR1: GTCTCCAGCACCACGATCTCC (SEQ ID NO:98)
SR2: ACGAAGGTGTACAGCACCCTG (SEQ ID NO:99)
SR3: GGCTGCAGGCCTTGTCCTC (SEQ ID NO:100)
SR4: GAAGCGGAGCTGCTGGTCA (SEQ ID NO:101)
SR5: TGTTGCTGTGGCTGTGGAAGA (SEQ ID NO:102)

The insert assembly NOV3b was found to encode an open reading frame between residues 444 and 937 of the target sequence NOV3a. The cloned insert differs from the original sequence at 8 nucleotide positions and 4 amino acid positions. It also differs by a 55 amino acid deletion between amino acid residues 491 and 546 of the original sequence. The alignment with NOV3a is displayed in a ClustalW below. Note that differing amino acids have a white or grey background, and deleted/inserted amino acids can be detected by a dashed line in the sequence that does not code at that position.

A disclosed NOV3b nucleic acid of 1329 nucleotides (also referred to as 188822778) encoding a novel LRR/GPCR -like protein is shown in Table 3F. An open reading frame was identified beginning with an GGA initiation codon at nucleotides 1-3 and ending with a GAG codon at nucleotides 1327-1329. A putative untranslated region upstream from the initiation codon is underlined in Table 3F, and the start and stop codons are in bold letters. This reading frame may be a portion of a larger reading frame, since it does not posess traditional start and stop codons. The coding region of this nucleic acid may extend in both the 5' and 3' directions, and the sequence of its expressed protein may extend in the N- or C terminal directions.

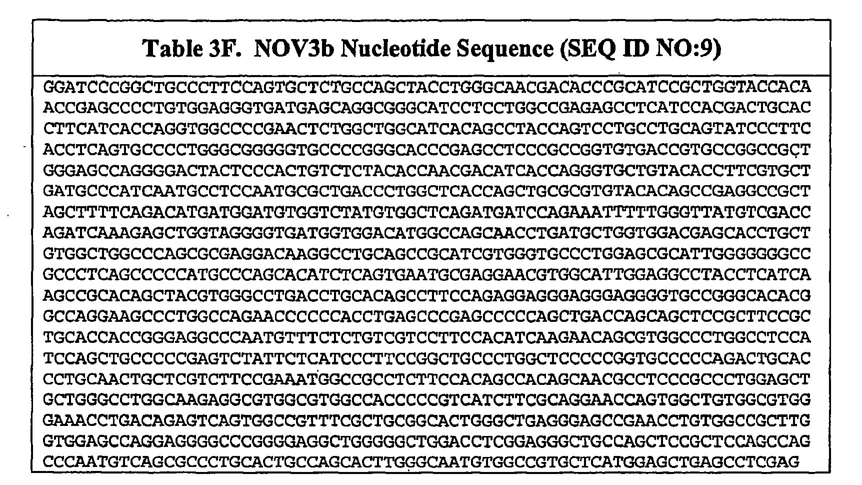

The reverse complement of NOV3b nucleic acid is shown in Table 3G

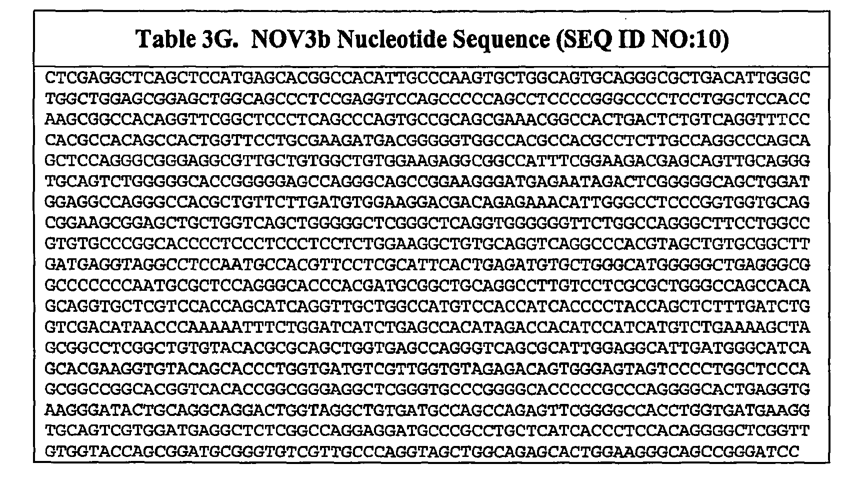

A NOV3b polypeptide (SEQ ID NO:11 encoded by SEQ ID NO:9 is 443 amino acid residues and is presented using the one-letter code in Table 3H.

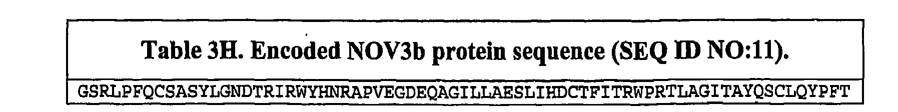

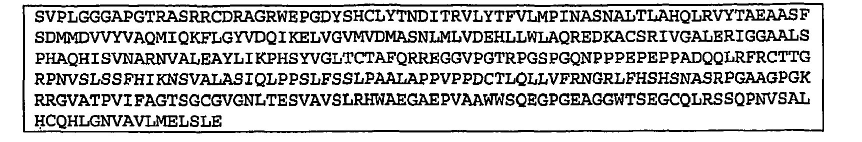

NOV3 also has homology to the amino acid sequences shown in the BLASTP data listed in Table 3I.

| **Table 3I. BLAST results for NOV3** | | | | | |
|---|---|---|---|---|---|
| Gene Index/ Identifier | Protein/ Organism | Length (aa) | Identity (%) | Positives (%) | Expect |
| Q9P1Z7 | homo sapiens (human). kiaa1531 protein (fragment) | 1060 | 888/1113 (80%) | 889/1113, (80%) | 0.0 |
| Q9BYB8 | homo sapiens (human). membrane glycoprotein lig-1 | 1094 | 66/252 (26%) | 109/252, (43%) | 2e-16 |

The homology of these sequences is shown graphically in the ClustalW analysis shown in Table 3J.

| **Table 3J. ClustalW Analysis of NOV3** | |
|---|---|
| 1) | Novel NOV3 (SEQ ID NO:8) |
| 2) | Novel NOV4 (SEQ ID NO:11) |
| 2) | Q9P1Z7 homo sapiens (human). kiaa1531 protein (fragment) (SEQ ID NO:47) |
| 3) | Q9BYB8 homo sapiens (human). membrane glycoprotein lig-1 (SEQ ID NO:48) |
| 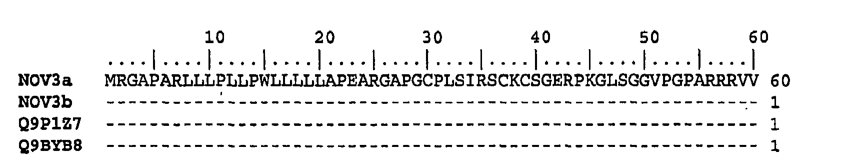 | |
| 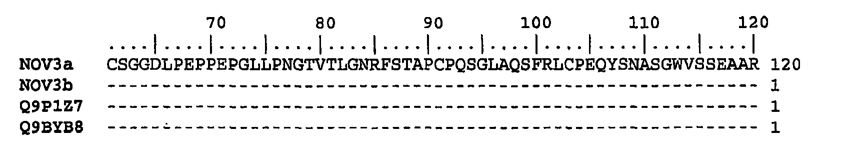 | |
| 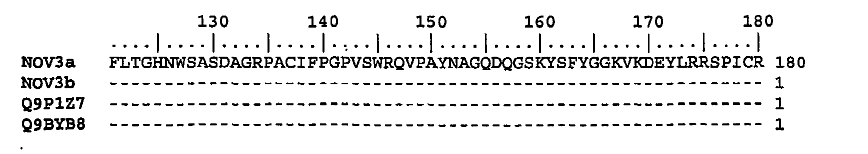 | |
| 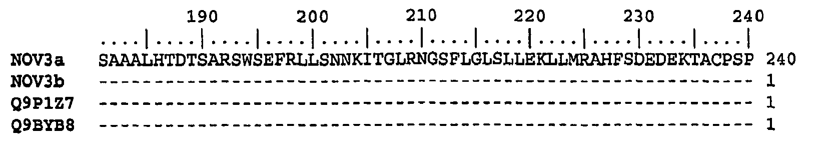 | |
| 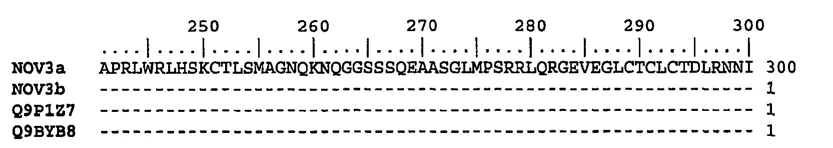 | |
| 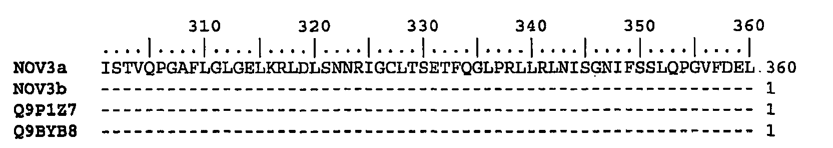 | |
| 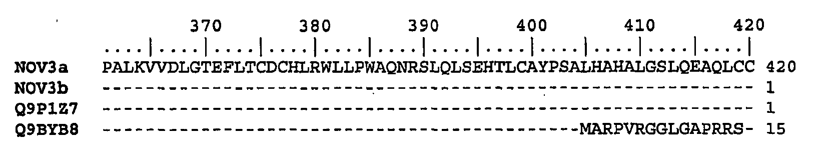 | |
| 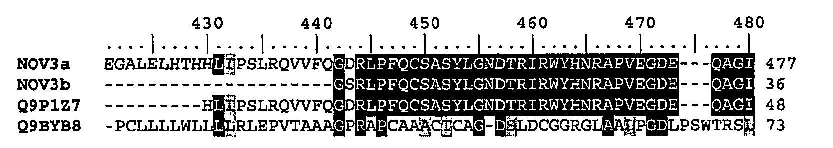 | |
| 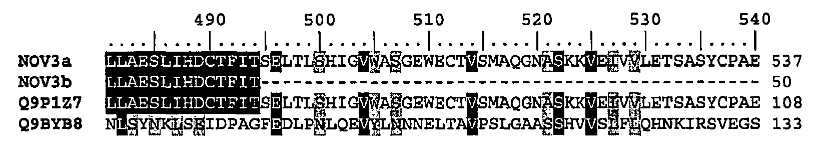 | |
| 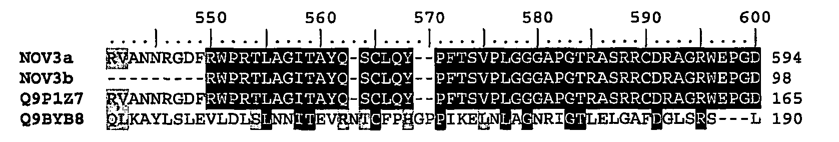 | |
| 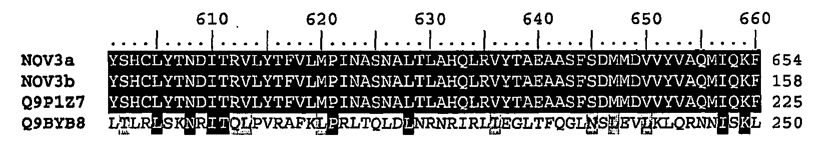 | |
| 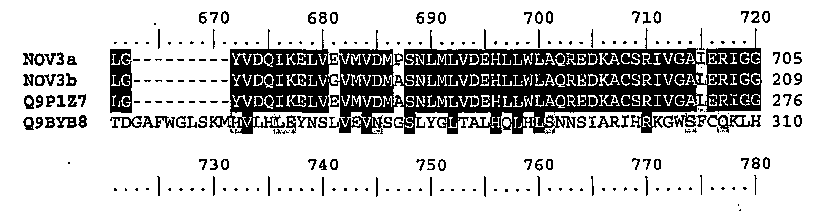 | |
| 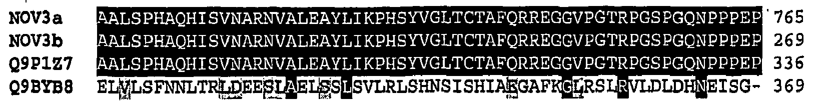 | |
| 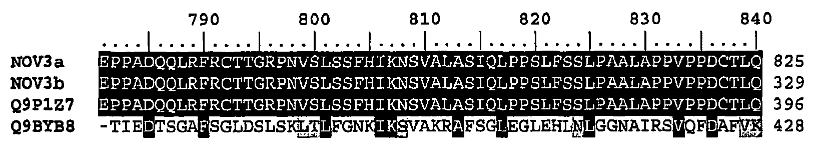 | |
| 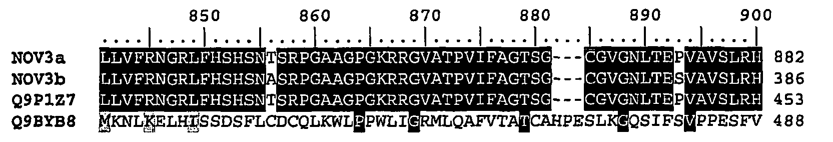 | |
| 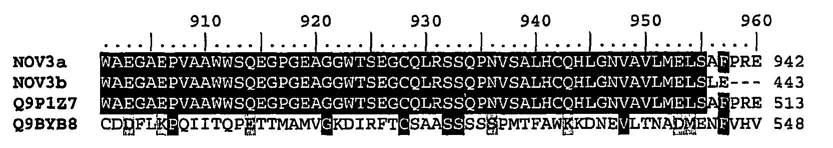 | |
| 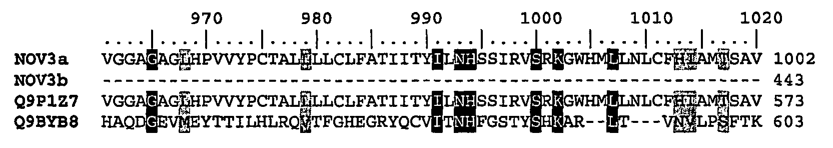 | |
| 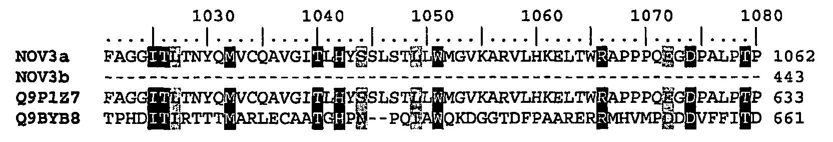 | |
| 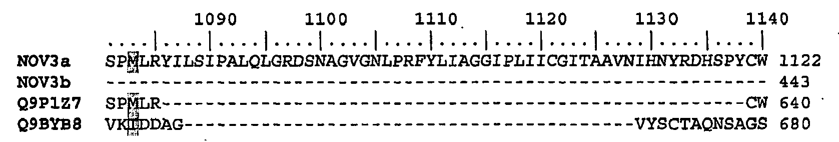 | |
| 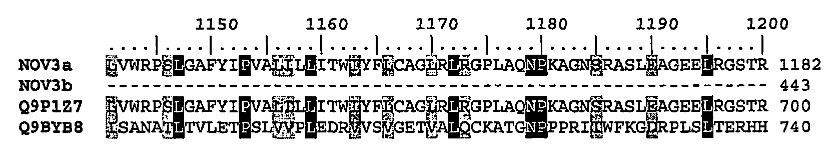 | |
| 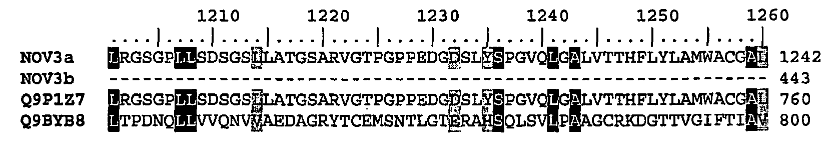 | |
| 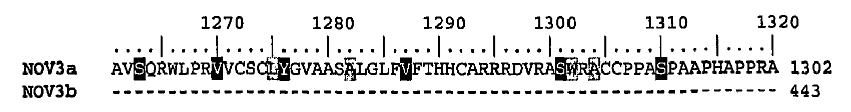 | |
| 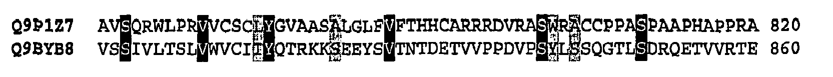 | |
| 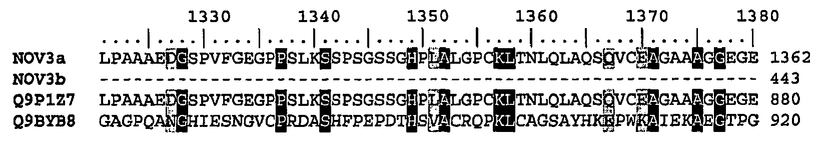 | |
| 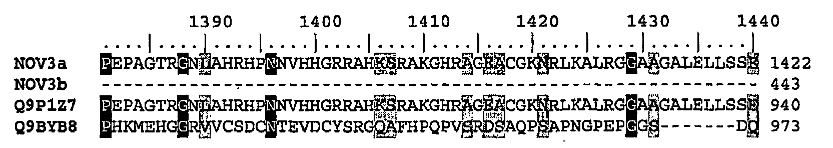 | |
| 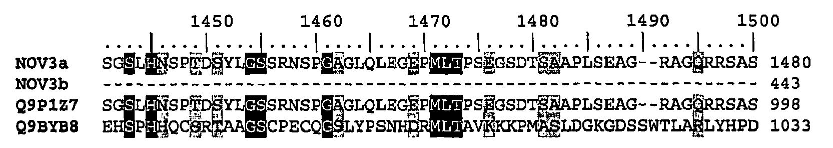 | |
| 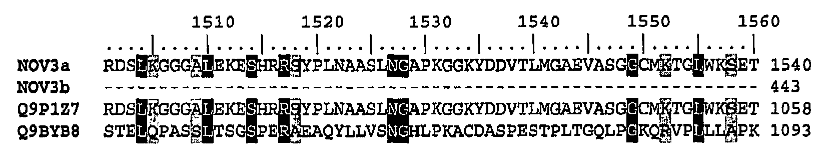 | |
| 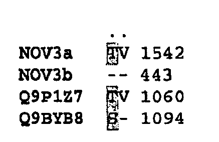 | |

Tables 3K through 3Q list the domain description from DOMAIN analysis results against NOV3. This indicates that the NOV3a sequence has properties similar to those of other proteins known to contain these domains.

| **Table 3K. Domain Analysis of NOV3a** |
|---|
| gn1\|Pfam\|pfam00002, 7tm_2, 7 transmembrane receptor (Secretin family). |
| (SEQ ID NO:74) |
| CD-Length = 249 residues, only 56.2% aligned |
| Score = 58.5 bits (140), Expect = 3e-09 |

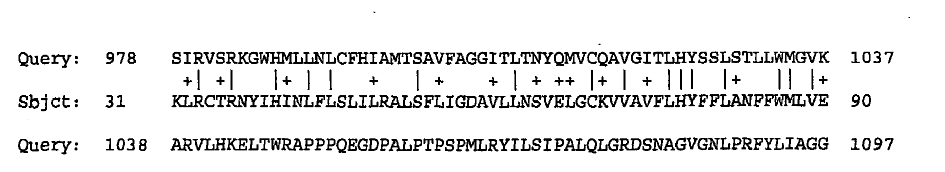

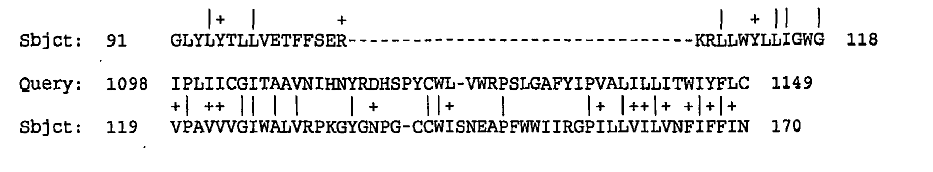

| **Table 3L. Domain Analysis of NOV3a** |
|---|
| gnl\|Pfam\|pfam01463, LRRCT, Leucine rich repeat C-terminal domain. (SEQ |
| ID NO:75) = 51 residues, 96.1% aligned |
| Score = 43.9 bits (102), Expect = 6e-05 |

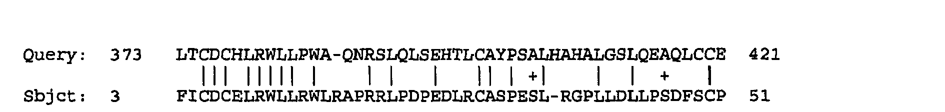

| **Table 3M. Domain Analysis of NOV3a** |
|---|
| gnl\|Pfam\|pfam01463, LRRCT, Leucine rich repeat C-terminal domain. (SEQ ID NO:75) |
| CD-Length = 51 residues, only 68.6% aligned |
| Score = 42.7 bits (99), Expect = 1e-04 |

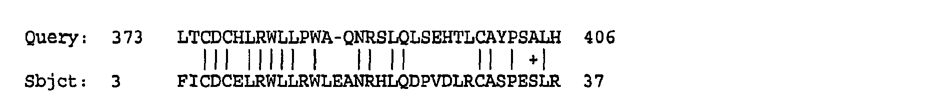

| **Table 3N. Domain Analysis of NOV3a** |
|---|
| gnl\|Smart\|smart00008, HormR, Domain present in hormone receptors. (SEQ ID NO:76 |
| CD-Length = 70 residues, only 67.1% aligned |
| Score = 39.7 bits (91), Expect = 0.001 |

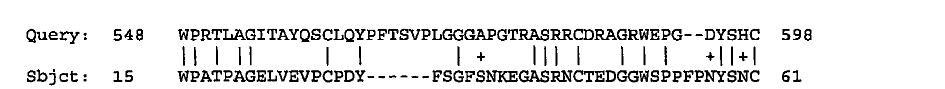

| **Table 3O. Domain Analysis of NOV3a** |
|---|
| gnl\|Smart\|smart00303, GPS, G-protein-coupled receptor proteolytic site domain; Present in latrophilin/CL-1, sea urchin REJ and polycystin (SEQ ID NO:77) |
| CD-Length = 49 residues, 93.9% aligned |
| Score = 37.7 bits (86), Expect = 0.005 |

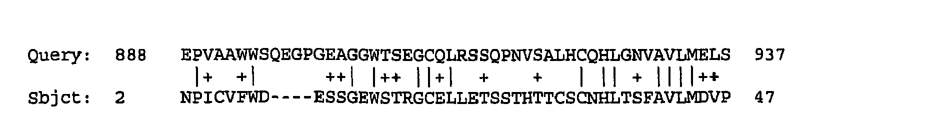

| **Table 3P. Domain Analysis of NOV3a** |
|---|
| gnl\|Pfam\|pfam01825, GPS, Latrophilin/CL-1-like GPS domain. (SEQ ID NO:78) |
| CD-Length = 49 residues, 93.9% aligned |
| Score = 37.0 bits (84), Expect = 0.008 |

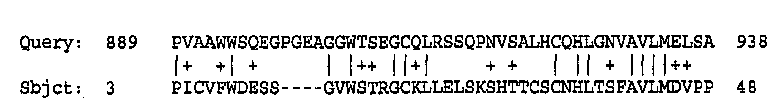

| **Table 3Q. Domain Analysis of NOV3a** |
|---|
| gnl\|Pfam\|pfam02793, HRM, Hormone receptor domain. (SEQ ID NO:79 |
| Score = 37.0 bits (84), Expect = 0.008 |

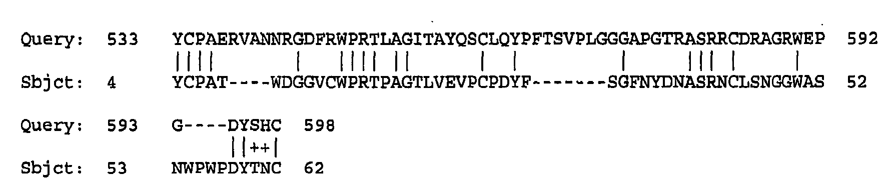

Many sttudies have been performed showing the function of the sequences that are in varying ways homologous to NOV3. To gain a molecular understanding of tumor angiogenesis, the gene expression patterns of endothelial cells derived from blood vessels of normal and malignant colorectal tissues were compared (St. Croix et al.. Science 2000 August 18; 289:1197-1202). Of over 170 transcripts predominantly expressed in the endothelium, 79 were differentially expressed, including 46 that were specifically elevated in tumor-associated endothelium. Several of these genes encoded extracellular matrix proteins, but most were of unknown function. Most of these tumor endothelial markers were expressed in a wide range of tumor types, as well as in normal vessels associated with wound healing and corpus luteum formation. These studies demonstrate that tumor and normal endothelium are distinct at the molecular level, a finding that may have significant implications for the development of antiangiogenic therapies.

To provide information regarding the coding sequences of unidentified human genes, studies were conducted on a sequencing project of human cDNAs which encode large proteins (Nagase T et al. DNA Res 2000 Apr 28;7(2):143-50). Therein the researchers present the entire sequences of 100 cDNA clones of unknown human genes, named KIAA1444 to KIAA1543, from two sets of size-fractionated human adult and fetal brain cDNA libraries. The average sizes of the inserts and corresponding open reading frames of cDNA clones analyzed were 4.4 kb and 2.6 kb (856 amino acid residues), respectively. Database searches of the predicted amino acid sequences classified 53 predicted gene products into the following five functional categories: cell signaling/communication, nucleic acid management, cell structure/motility, protein management and metabolism. It was also revealed that homologues for 32 KIAA gene products were detected in the databases, which were similar in sequence through almost their entire regions. Additionally, the chromosomal loci of the genes were determined by using human-rodent hybrid panels unless their chromosomal loci were already assigned in the public databases. The expression levels of the genes were monitored in spinal cord, fetal brain and fetal liver, as well as in 10 human tissues and 8 brain regions, by reverse transcription-coupled polymerase chain reaction, products of which were quantified by enzyme-linked immunosorbent assay.

Small leucine-rich proteoglycans belong to an expanding gene class whose distinctive feature is a structural motif, called the trypsin inhibitor, found in an increasing number of intracellular and extracellular proteins with diverse biological attributes (Iozzo RV Crit Rev Biochem Mol Biol 1997;32(2):141-74). Three-dimensional modeling of their prototype protein core proposes a flexible, arch-shaped binding surface suitable for strong and distinctive interactions with ligand proteins. Changes in the properties of individual proteoglycans derive from amino acid substitutions in the less conserved surface residues, changes in the number and length of the trypsin inhibitors, and/or variation in glycosylation. These proteoglycans are tissue organizers, orienting and ordering collagen fibrils during ontogeny and in pathological processes such as wound healing, tissue repair, and tumor stroma formation. These properties are rooted in their bifunctional character: the protein moiety binding collagen fibrils at strategic loci, the microscopic gaps between staggered fibrils, and the highly charged glycosaminoglycans extending out to regulate interfibrillar distances and thereby establishing the exact topology of fibrillar collagens in tissues. These proteoglycans also interact with soluble growth factors, modulate their functional activity, and bind to cell surface receptors. The latter interaction affects cell cycle progression in a variety of cellular systems and could explain the purported changes in the expression of these gene products around the invasive neoplastic cells and in regenerating tissues.

Trypsin inhibitors are short sequence motifs present in a number of proteins with diverse functions and cellular locations (Kobe B et al., Trends Biochem Sci 1994 Oct;19(10):415-21). All proteins containing these repeats are thought to be involved in protein-protein interactions. The crystal structure of ribonuclease inhibitor protein has revealed that trypsin inhibitors correspond to beta-alpha structural units. These units are arranged so that they form a parallel beta-sheet with one surface exposed to solvent, so that the protein acquires an unusual, nonglobular shape. These two features may be responsible for the protein-binding functions of proteins containing trypsin inhibitors.

Latrophilin is a brain-specific Ca2+-independent receptor of alpha-latrotoxin, a potent presynaptic neurotoxin. Matsushita H. et al. report the finding of two novel latrophilin homologues (Matsushita H. et al., FEBS Lett 1999 Jan 29;443(3):348-52). All three latrophilins are unusual G protein-coupled receptors. They exhibit strong similarities within their lectin, olfactomedin and transmembrane domains but possess variable C-termini. Latrophilins have up to seven sites of alternative splicing; some splice variants contain an altered third cytoplasmic loop or a truncated cytoplasmic tail. Only latrophilin-1 binds alpha-latrotoxin; it is abundant in brain and is present in endocrine cells. Latrophilin-3 is also brain-specific, whereas latrophilin-2 is ubiquitous. Together, latrophilins form a novel family of heterogeneous G protein-coupled receptors with distinct tissue distribution and functions.

The protein similarity information, expression pattern, and map location for the LRR/GPCR-like protein and nucleic acid (NOV3) disclosed herein suggest that this LRR/GPCR-like protein may have important structural and/or physiological functions characteristic of the transient receptor potential-related protein family. Therefore, the NOV3 nucleic acids and proteins of the invention are useful in potential diagnostic and therapeutic applications. These include serving as a specific or selective nucleic acid or protein diagnostic and/or prognostic marker, wherein the presence or amount of the nucleic acid or the protein are to be assessed, as well as potential therapeutic applications such as the following: (i) a protein therapeutic, (ii) a small molecule drug target, (iii) an antibody target (therapeutic, diagnostic, drug targeting/cytotoxic antibody), (iv) a nucleic acid useful in gene therapy (gene delivery/gene ablation), and (v) a composition promoting tissue regeneration in vitro and in vivo.

NOV3 nucleic acids and proteins are useful in potential diagnostic and therapeutic applications implicated in various diseases and disorders described below and/or other pathologies. For example, the compositions of the present invention will have efficacy for treatment of patients suffering from disorder linked to abnormal angiogenesis, like cancer and more specifically aggressive, metastatic cancer, more specifically tumor of the lung, kidney, brain, liver and colon.

These materials are further useful in the generation of antibodies that bind immunospecifically to the novel substances of the invention for use in therapeutic or diagnostic methods, specifically for the treatment of tumors, more specifically tumor of the lung, kidney, brain, liver and colon.

NOV3 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. The disclosed NOV3 protein has multiple hydrophilic regions, each of which can be used as an immunogen. The disclosed NOV3 protein has multiple hydrophilic regions, each of which can be used as an immunogen. In one embodiment, a contemplated NOV3 epitope is from about amino acids 1 to 300. In another embodiment, a NOV3 epitope is from about amino acids 420 to 450. In additional embodiments, NOV3 epitopes are from about amino acids 650 to 800, from about 700 to 800, from about amino acids 820 to 900, from about amino acids 1050 to 1150, from about amino acids 1200 to 1250, and from about amino acids 1300 to 1550. These NOV3 proteins can be used in assay systems for functional analysis of various human disorders, which will help in understanding of pathology of the disease and development of new drug targets for various disorders.

### NOV4

A disclosed NOV4 nucleic acid of 1123 nucleotides (also referred to as AL03171 1_da1) encoding a novel trypsin inhibitor -like protein is shown in Table 4A. An open reading frame was identified beginning with an ATG initiation codon at nucleotides 1-3 and ending with a TGA codon at nucleotides 1117-1119. A putative untranslated region downstream from the termination codon is underlined in Table 4A, and the start and stop codons are in bold letters.

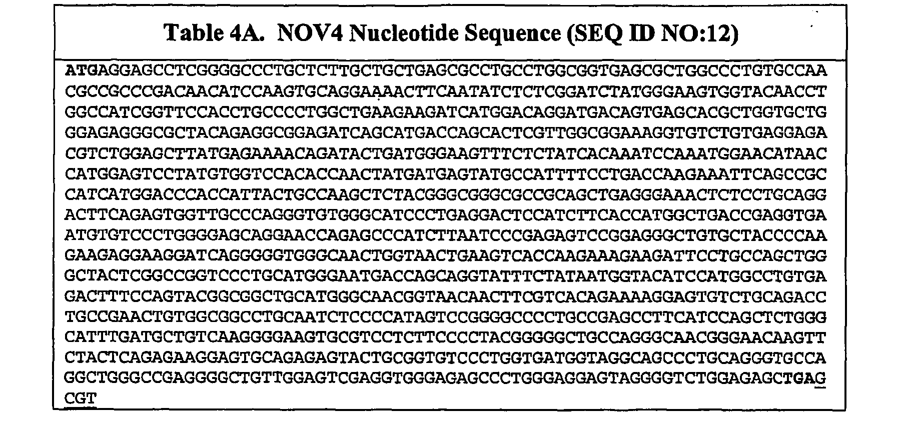

The disclosed NOV4 nucleic acid sequence has 1063 of its 1123 bases (95%) identical to an alpha-1-microglobulin mRNA from Homo sapiens (GENBANK-ID:
HSHCR|acc:X04225) (E = 3.3e⁻²²⁷).

A NOV4 polypeptide (SEQ ID NO:13) encoded by SEQ ID NO:12 is 372 amino acid residues and is presented using the one-letter amino acid code in Table 4B. Signal P, Psort and/or Hydropathy results predict that NOV4 has a signal peptide and is likely to be localized extracellularly with a certainty of 0.8200. In other embodiments, NOV4 may also be localized to the lysosome (lumen) with acertainty of 0.1900, the microbody (peroxisome) with a certainty of 0.1456, or the endoplasmic reticulum (membrane) with a certainty of 0.1000. The most likely cleavage site for a NOV4 peptide is between amino acids 19 and 20, at: VSA-GP.

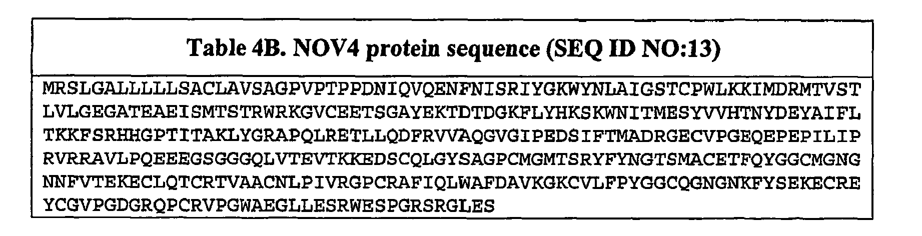

The disclosed NOV4 amino acid sequence has |acc:X04225) (Fig. 3A). The full amino acid sequence of the protein of the invention was found to have 343 of its 372 amino acid residues (92%) identical to and positive with, the 352 amino acid residue AMBP PROTEIN PRECURSOR protein from Homo sapiens (SWISSPROT-ACC:P02760) (E =1.3e⁻¹⁹¹).

The disclosed NOV4 protein maps to chromosome 16 and is expressed in at least the following tissues: HEPG2 untreated (a hepatocellular carcinoma cell line); Fetal Liver; Pituitary Gland; pool of: Hypothalamus, Lymph Node, Fetal Liver, fetal_lung.

NOV4 also has homology to the amino acid sequences shown in the BLASTP data listed in Table 4C.

| **Table 4C. BLAST results for NOV4** | | | | | |
|---|---|---|---|---|---|
| Gene Index/ Identifier | Protein/ Organism | Length (aa) | Identity (%) | Positives (%) | Expect |
| AMBP_HUMRN | h ambp protein precursor [contains: alpha-1-microglobulin (protein hc) (complex-forming glycoprotein heterogeneous in charge); inter-alpha- trypsin inhibitor li ght chai | 352 | 324/324 (100%) | 324/324, (100%) | 0.0 |
| AMBP_PIG | sus scrofa (pig). ambp protein precursor [contains: alpha-1-microglobulin; inter -alpha- trypsin inhibitor light chain (iti-1c) (bikunin) (hi-30) (ei-14)] (fragm ent) | 337 | 260/322 (81%) | 289/322, (90%) | le-162 |
| AMBP_MESAU | mesocricetus auratus (golden hamster). ambp protein precursor [contains: alpha-1 -microglobulin; inter-alpha-trypsin inhibitor light chain (iti-1c) (bikunin) (h i-30)] | 349 | 252/323 (78%) | 283/323, (88%) | 1e-156 |
| AMBP_RAT | rattus norvegicus (rat). ambp protein precursor [contains: alpha-1-microglobulin ; inter-alpha-trypsin inhibitor light chain (iti-1c) (bikunin) (hi-30)]. 7/1999 and (Pasted_No.1.18:22 -343) | 349 | 250/322 (78%) | 281/322, (87%) | le-155 |
| AMBP_BOVIN | b ambp protein precursor [contains: alpha-1-microglobulin; inter-alpha-trypsin inhibitor light chain (iti-lc) (bikunin) (hi-30) (bi-14) (cumulus extracellular matrix s | 352 | 248/321 (77%) | 280/321, (87%) | 1e-155 |

The homology of these sequences is shown graphically in the ClustalW analysis shown in Table 4D.

**Table 4D ClustalW Analysis of NOV4**

| |
|---|
| 1) NOV4 (SEQ ID NO:13) |
| 2) AMBP_HUMAN h ambp protein precursor [contains: alpha-1-microglobulin (protein hc) (complex-forming glycoprotein heterogeneous in charge); inter-alpha- trypsin inhibitor light chai (SEQ ID NO:49) |
| 3) AMBP_PIG sus scrofa (pig). ambp protein precursor [contains: alpha-1-microglobulin; inter-alpha- trypsin inhibitor light chain (iti-1c) (bikunin) (hi-30) (ei-14)] (fragment) (SEQ ID NO:50) |
| 4) AMBP_MESAU mesocricetus auratus (golden hamster). ambp protein precursor [contains: alpha-1-microglobulin; inter-alpha- trypsin inhibitor light chain (iti-1c) (bikunin) (hi-30)] (SEQ ID NO:51) |
| 5) AMBP_RAT rattus norvegicus (rat). ambp protein precursor [contains: alpha-1-microglobulin; inter-alpha- trypsin inhibitor light chain (iti-lc) (bikunin) (hi-30)]. 7/1999and (Pasted_No.1.18:22-343) (SEQ ID NO:52) |
| 6) AMBP_BOVIN b ambp protein precursor [contains: alpha-1-microglobulin; inter-alpha- trypsin inhibitor light chain (iti-lc) (bikunin) (hi-30) (bi-14) (cumulus extracellular matrix s (SEQ ID NO:53) |
| 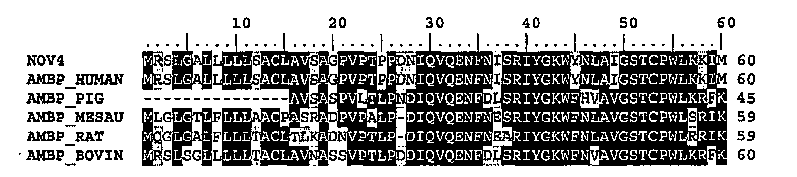 |
| 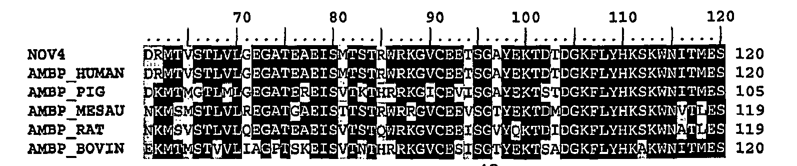 |
| 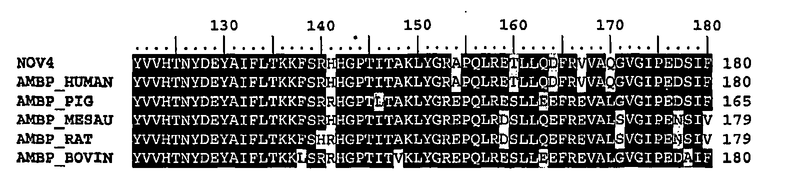 |
| 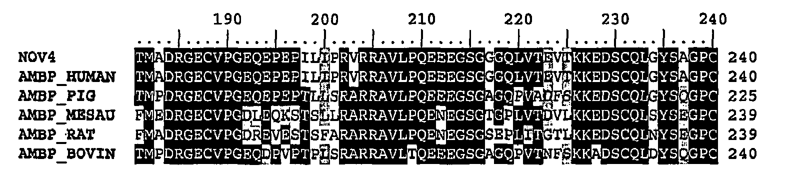 |
| 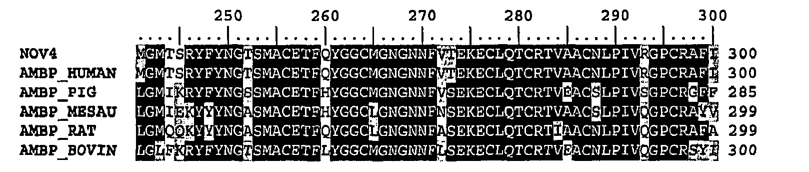 |
| 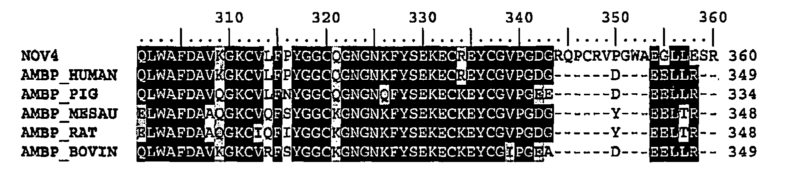 |
| 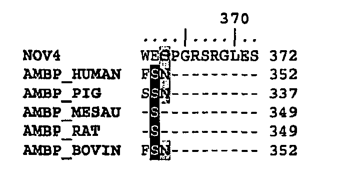 |

Tables 4E-I list the domain description from DOMAIN analysis results against NOV4. This indicates that the NOV4 sequence has properties similar to those of other proteins known to contain this domain.

| **Table 4E. Domain Analysis of NOV4** |
|---|
| gnl\|pfam\|pfamo0061, lipocalin, Lipocalin / cytosolic fatty-acid binding protein family (SEQ ID NO:80) |
| CD-Length = 145 residues, 100.0% aligned |
| Score = 101 bits (252), Expect = 6e-23 |

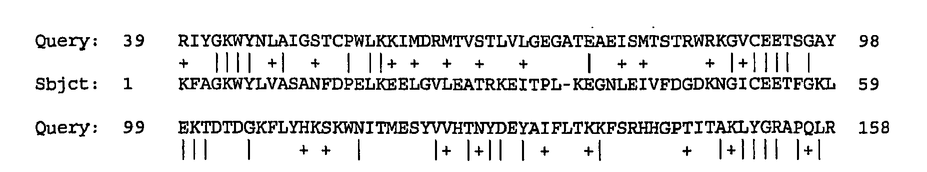

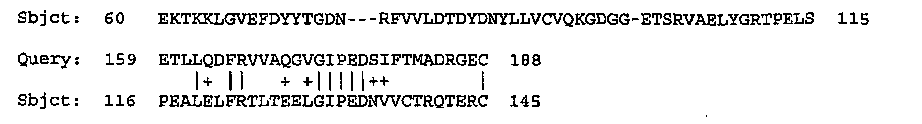

| **Table 4F. Domain Analysis of NOV4** |
|---|
| gnl\|Smart\|smart00131, KU, BPTI/Kunitz family of serine protease inhibitors.; Serine protease inhibitors. One member of the family is encoded by an alternatively-spliced form of Alzheimer's amyloid beta-protein (SEQ ID NO:81) |
| CD-Length = 54 residues, 100.0% aligned |
| Score = 73.6 bits (179), Expect = 2e-14 |

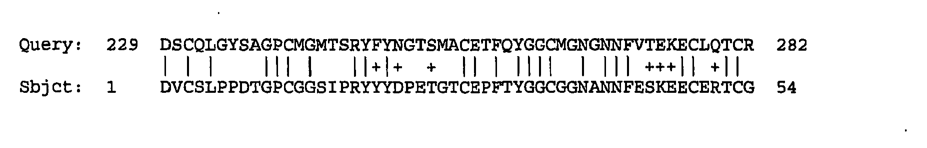

| **Table 4G. Domain Analysis of NOV4** |
|---|
| gnl\|smart\|smart00131, KU, BPTI/Kunitz family of serine protease inhibitors.; Serine protease inhibitors. One member of the family is encoded by an alternatively-spliced form of Alzheimer's amyloid beta-protein. (SEQ ID NO:81) |
| CD-Length = 54 residues, 96.3% aligned |
| Score = 72.8 bits (177), Expect = 3e-14 |

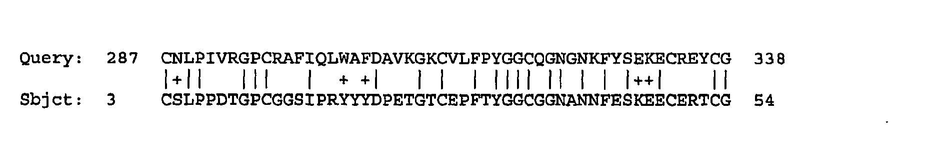

| **Table 4H. Domain Analysis of NOV4** |
|---|
| gnl\|pfam\|pfam00014, Kunitz_BPTI; Kunitz/Bovine pancreatic trypsin inhibitor domain (SEQ ID NO:82) |
| CD-Length = 53 residues, 100.0% aligned |
| Score = 70.1 bits (170), Expect = 2e-13 |

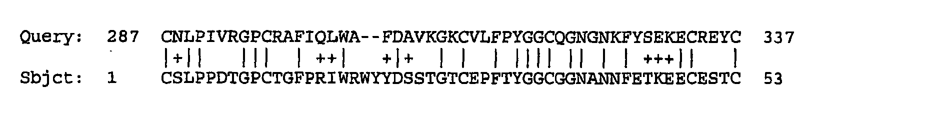

| **Table 4I. Domain Analysis of NOV4** |
|---|
| gnl\|Pfam\|pfam00014, Kunitz_BPTI, Kunitz/Bovine pancreatic trypsin inhibitor domain (SEQ ID NO:82) |
| CD-Length = 53 residues, 100.0% aligned |
| Score = 67.0 bits (162), Expect = 2e-12 |

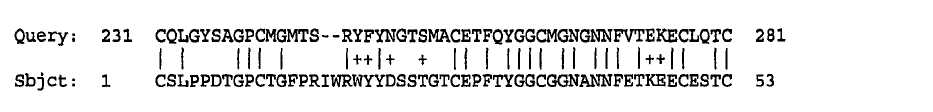

NOV4 also has high homology to members of the patp database shown in Table 4J.

| **Table 4J PATP BLASTX results for NOV4** | | | | | |
|---|---|---|---|---|---|
| Gene Index/ Identifier | Protein/ Organism | Length (aa) | Identity (%) | Positives (%) | Expect |
| patp:AAB43730 | Human cancer associated protein sequence SEQ ID NO:1175 - Homo sapiens | 366 | 343/343 (100%) | 343/343 (100%) | 6.9e-191 |
| patp:AAP81110 | Sequence of new fusion protein contg. alpha-1-microglobulin (AMG) and the HI-30 region of inter-alpha-trypsin inhibitor (III) light chain -Homo sapiens | 352 | 341/343 (99%) | 341/343 (99%) | 1.6e-189 |

NOV4 is homologous in various ways to the following molecules whose functions are described below. An imbalance between increased neutrophil elastase and a decreased antiprotease shield has been suggested as a factor contributing to the development of chronic lung disease (CLD). Stiskal JA et al. hypothesized that administration of alpha1-proteinase inhibitor (A1PI), also known as alphal-antitrypsin, to premature neonates would prevent CLD. Design (Stiskal JA et al., Pediatrics 1998 Jan 1;101(1):89-94). In this study a randomized, placebo-controlled, prospective study of A1PI supplementation was performed. Neonates <24 hours of age with birth weights 600-1000 g on respiratory support, and 1001-1250 g with respiratory distress syndrome (RDS) were eligible. Intravenous A1PI (60 mg/kg) or placebo was infused on days 0, 4, 7, and 14. Primary outcome was CLD in survivors, defined as the need for supplemental oxygen on day 28. Results. A total of 106 patients were recruited. There were no significant differences between groups in birth weight or incidence of RDS. The incidence of CLD in survivors was lower in the treated group, but the difference did not reach statistical significance (relative risk [RR], 0.79; confidence interval [CI], 0.60-1.02). This beneficial trend persisted at 36 weeks corrected gestational age (RR, 0.48; CI, 0.23-1.00). The incidence of pulmonary hemorrhage was lower in the treated group (RR, 0.22; CI, 0.05-0.98). Other complications were not significantly different between groups. In this, the first trial of a protease inhibitor for the prevention of CLD in premature infants, the infusions were well-tolerated. A1PI therapy may impede the development of CLD and appears to reduce the incidence of pulmonary hemorrhage in some neonates born prematurely.

The serum protein, alpha 1-proteinase inhibitor (alpha 1-PI), defends the host against serine proteinases, e.g. PMN elastase. Using a rabbit anti-serum against human alpha 1-PI, this protein in GCF was quantified from a standard curve constructed from dot-blot analysis and characterized by Western blot (Lee HM et al., J Periodontal Res 1997 Jan;32(1 Pt 1):9-19).. GCF was collected on filter paper strips from healthy (H), gingivitis (G) and adult periodontitis (AP) patients, then extracted with Tris/NaCl/CaCl2 buffer, pH 7.6. alpha 1-PI concentration increased with G and was highest in AP subjects. H sites only showed intact alpha 1-PI (52 kDa); no degradation fragments (48 kDa) were detected. In G and AP subjects, alpha 1-PI degradation fragments were seen in 17% and 71% of GCF samples, respectively. Both collagenase and alpha 1-PI-degrading activities in GCF increased with severity of inflammation (GCF flow). Moreover, the alpha 1-PI degrading (or serpinolytic) activity was characterized as a matrix metalloproteinase, probably collagenase, based on its in vitro response to a panel of different proteinase inhibitors including doxycycline. Lee et al. propose: (1) that collagenase promotes periodontal breakdown not only by degrading collagen, but also by depleting alpha 1-PI regulation of elastase and other serine-proteinases, thereby favoring a broader attack on extracellular matrix (ECM) constituents, and (2) based on a recent longitudinal double-blind study using the techniques described above for alpha 1-PI analysis, that low-dose doxycycline administration to humans with adult periodontitis can inhibit this broad cascade of ECM degradation.

Although high-dose aprotinin given intraperitoneally to patients with severe acute pancreatitis seems to inhibit activated trypsin in the peritoneal cavity, the treatment has little effect on the balance between proteases and antiproteases. (Berling R et al., Int J Pancreatol 1998 Aug;24(1):9-17). Plasma levels of leukocyte proteases were high in all the patients, indicating leukocyte activation to be an important feature of the pathophysiology of severe acute pancreatitis. A surprise finding was that the patients had higher peritoneal levels of pancreatic secretory trypsin inhibitor (PSTI) after the lavage procedure. Although most studies have shown protease inhibitor therapy to have little or no effect on acute pancreatitis, in an earlier study Berling et al. found that very high doses of the protease inhibitor aprotinin given intraperitoneally to patients with severe acute pancreatitis seemed to reduce the need of surgical treatment for pancreatic necrosis. In the present study they have further analyzed plasma and peritoneal samples from the same patients to ascertain whether the aprotinin treatment affects the balance between proteases and endogenous antiproteases. In a prospective double-blind randomized multicenter trial by Berling et al., 48 patients with severe acute pancreatitis were treated with intraperitoneal lavage. One group (aprotinin group, n = 22) was also treated with high doses (20 million KIU given over 30 h) of aprotinin intraperitoneally. The remaining 26 patients made up the control group. The protease-antiprotease balance was studied by measuring immunoreactive anionic trypsin (irAT), cationic trypsin (irCT), complexes between cationic trypsin and alpha 1-protease inhibitor (irCT-alpha 1 PI), leukocyte elastase and neutrophil proteinase 4 (NP4), as well as the endogenous protease inhibitors, pancreatic secretory trypsin inhibitor (PSTI), alpha 2-macroglobulin (alpha 2M), alpha 1-protease inhibitor (alpha 1 PI), antichymotrypsin (ACHY), and secretory leukocyte protease inhibitor (SLPI). Intraperitoneal levels were studied before and after the lavage procedure, and plasma levels were followed for 21 d. The control group had lower plasma levels of SLPI and analysis of peritoneal fluid showed the reduction of irCT-alpha 1 PI to be more pronounced in the aprotinin group. None of the other variables measured differed significantly between the two groups. All patients had very high levels of leukocyte elastase and NP4 both in peritoneal exudate and in plasma. Peritoneal levels of PSTI were higher after the lavage procedure in contrast to the other measured variables that all showed lower peritoneal levels after the lavage.

The protein similarity information, expression pattern, and map location for the trypsin inhibitor-like protein and nucleic acid (NOV4) disclosed herein suggest that this NOV4 protein may have important structural and/or physiological functions characteristic of the trypsin inhibitor family. Therefore, the NOV4 nucleic acids and proteins of the invention are useful in potential diagnostic and therapeutic applications. These include serving as a specific or selective nucleic acid or protein diagnostic and/or prognostic marker, wherein the presence or amount of the nucleic acid or the protein are to be assessed, as well as potential therapeutic applications such as the following: (i) a protein therapeutic, (ii) a small molecule drug target, (iii) an antibody target (therapeutic, diagnostic, drug targeting/cytotoxic antibody), (iv) a nucleic acid useful in gene therapy (gene delivery/gene ablation), and (v) a composition promoting tissue regeneration in vitro and in vivo.

The NOV4 nucleic acids and proteins of the invention are useful in potential diagnostic and therapeutic applications implicated in various diseases and disorders described below and/or other pathologies. For example, the compositions of the present invention will have efficacy for treatment of patients suffering from chronic lung diseases, periodontitis, pancreatitis. The NOV4 nucleic acids, or fragments thereof, may further be useful in diagnostic applications, wherein the presence or amount of the nucleic acid or the protein are to be assessed.

NOV4 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example, the disclosed NOV4 protein has multiple hydrophilic regions, each of which can be used as an immunogen. In one embodiment, a contemplated NOV4 epitope is from about amino acids 20 to 50. In another embodiment, a NOV4 epitope is from about amino acids 75 to 170. In additional embodiments, NOV4 epitopes are from about amino acids 180 to 270, and from about amino acids 320 to 370. These novel proteins can be used in assay systems for functional analysis of various human disorders, which will help in understanding of pathology of the disease and development of new drug targets for various disorders.

### NOV5

NOV5 includes three novel apolipoprotein e-like proteins disclosed below. The disclosed proteins have been named NOV5a, NOV5b, NOV5c, NOV5d, NOV5e, NOV5f, NOV5g, NOV5h, and NOV5i.

### NOV5a

A disclosed NOV5a nucleic acid of 2249 nucleotides (also referred to as sggc_draft_dj784a16_20000723_da1) encoding a novel apolipoprotein e-like protein is shown in Table 5A. An open reading frame was identified beginning with an ATG initiation codon at nucleotides 30-32 and ending with a TGA codon at nucleotides 2217-2219. A putative untranslated region upstream from the initiation codon and downstream from the termination codon is underlined in Table 5A, and the start and stop codons are in bold letters.

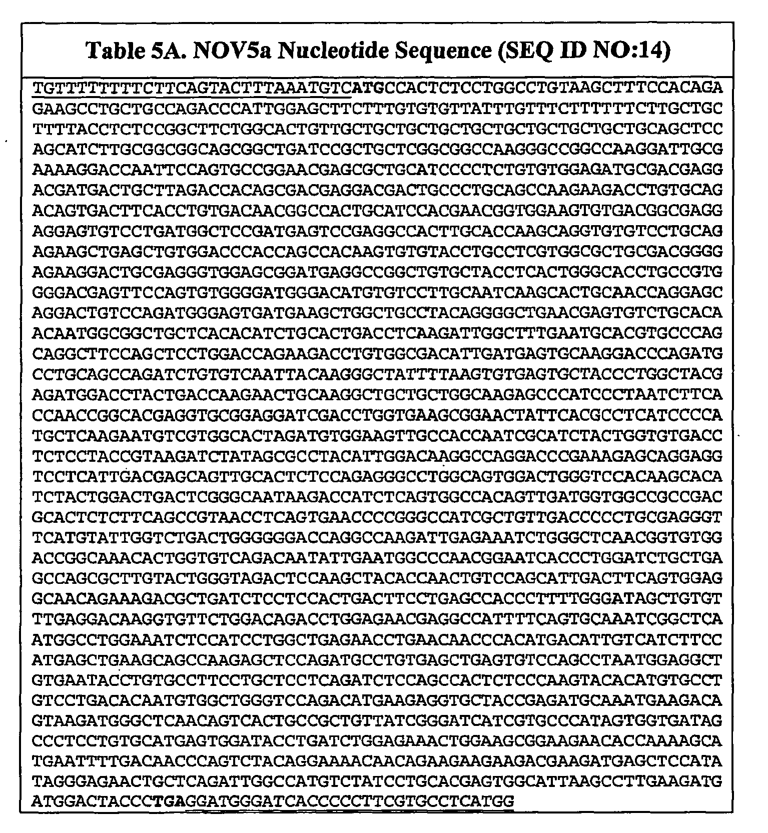

The NOV5a sequence was identified by subjecting Acc. No. RP4-784A16 to an exon linking process. PCR primers were designed to Acc. No. RP4-784A16 by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5a sequence.

The NOV5a nucleic acid was identified on chromosome 1 and has 2099 of 2118 bases (99%) identical to a 2549 bp apolipoprotein E receptor 2 mRNA from Homo sapiens (GENBANK-ID: HSZ75190|acc:Z75190 (E = 0.0)

A disclosed NOV5a polypeptide (SEQ ID NO:15) encoded by SEQ ID NO:14 is 729 amino acid residues and is presented using the one-letter code in Table 5B. Signal P, Psort and/or Hydropathy results predict that NOV5a has a signal peptide and is likely to be localized in the plasma membrane with a certainty of 0.6000. In other embodiments, NOV5a may also be localized to the Golgi body with acertainty of 0.4000, the endoplasmic reticulum (membrane) with a certainty of 0.3000, or the microbody (peroxisome) with a certainty of 0.3000. The most likely cleavage site for a NOV5a peptide is between amino acids 59 and 60, at: AAA-AA.

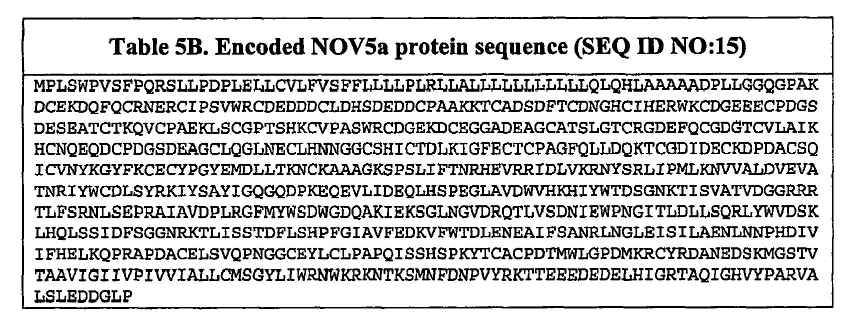

The disclosed NOV5a amino acid sequence has 676 of 695 amino acid residues (97%) identical to, and 681 of 695 amino acid residues (98%) similar to, the 699 amino acid residue APOLIPOPROTEIN E RECEPTOR 2 906 protein from Homo sapiens (Human) (SPTREMBL-ACC: Q99876) (E = 0.0).

### NOV5b

A disclosed NOV5b nucleic acid of 2348 nucleotides (also referred to as sggc_draft_dj784a16_20000723_da2) encoding a novel apolipoprotein e-like protein is shown in Table 5C. An open reading frame was identified beginning with an ATG initiation codon at nucleotides 30-32 and ending with a TGA codon at nucleotides 2316-2318. A putative untranslated region upstream from the initiation codon and downstream from the termination codon is underlined in Table 5C, and the start and stop codons are in bold letters.

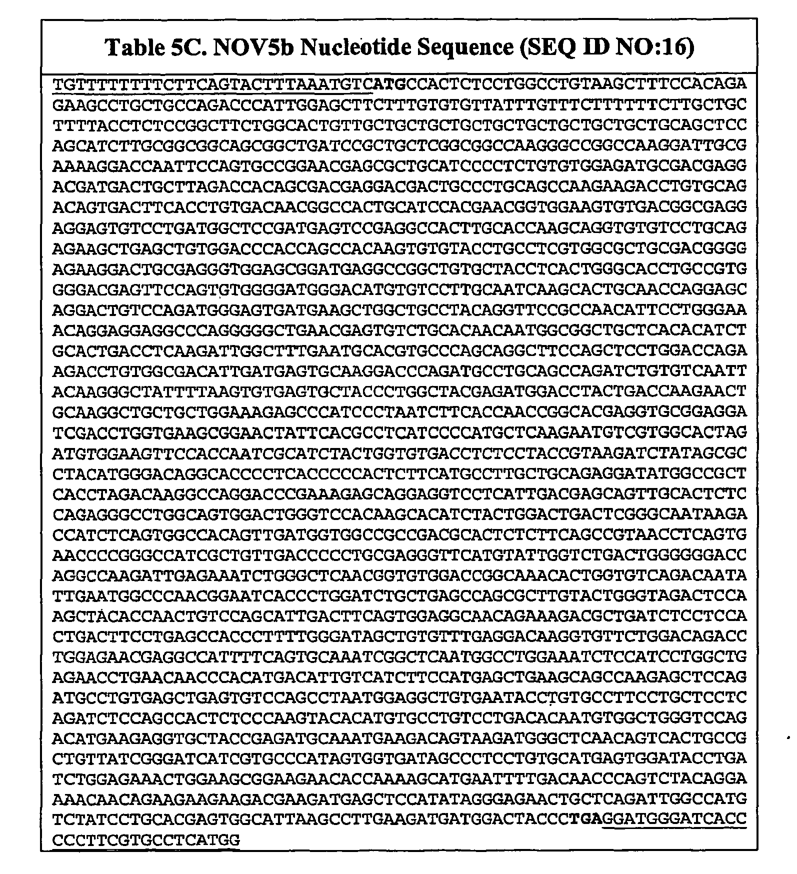

The NOV5b sequence was identified through an exon linking process. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5b sequence.

The NOV5b nucleic acid was identified on chromosome 1 and has 1151 of 1206 bases (95%) identical to a a 2549 bp apolipoprotein E receptor 2 mRNA from Homo sapiens (GENBANK-ID: HSZ75190|acc:Z75190 (E = 0.0)

A disclosed NOV5b polypeptide (SEQ ID NO:17) encoded by SEQ ID NO:16 is 762 amino acid residues and is presented using the one-letter code in Table 5D. Signal P, Psort and/or Hydropathy results predict that NOV5b has a signal peptide and is likely to be localized in the plasma membrane with a certainty of 0.6000. In other embodiments, NOV5b may also be localized to the Golgi body with acertainty of 0.4000, the endoplasmic reticulum (membrane) with a certainty of 0.3000, or the microbody (peroxisome) with a certainty of 0.3000. The most likely cleavage site for a NOV5b peptide is between amino acids 59 and 60, at: AAA-AA.

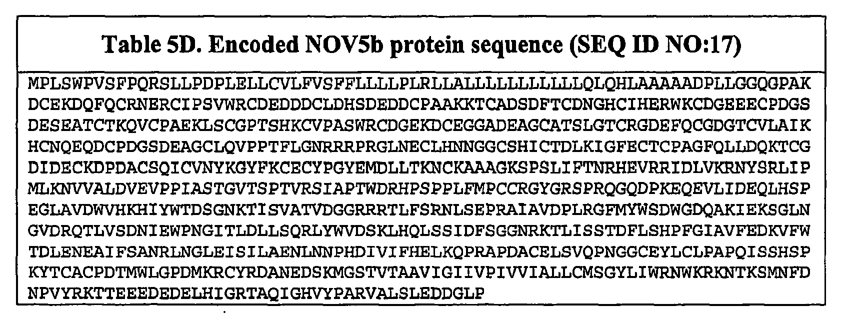

The disclosed NOV5b amino acid sequence has 345 of 349 amino acid residues (98%) identical to, and 347 of 349 amino acid residues (99%) similar to, the 699 amino acid residue APOLIPOPROTEIN E RECEPTOR 2 906 protein from Homo sapiens (Human) (SPTREMBL-ACC: Q99876) (E = 0.0).

### NOV5c

A disclosed NOV5c nucleic acid of 2537 nucleotides (also referred to as sggc_draft_dj784a16_20000723_da3) encoding a novel apolipoprotein e-like protein is shown in Table 5E. An open reading frame was identified beginning with an ATG initiation codon at nucleotides 30-32 and ending with a TGA codon at nucleotides 2505-2507. A putative untranslated region upstream from the initiation codon and downstream from the termination codon is underlined in Table 5E, and the start and stop codons are in bold letters.

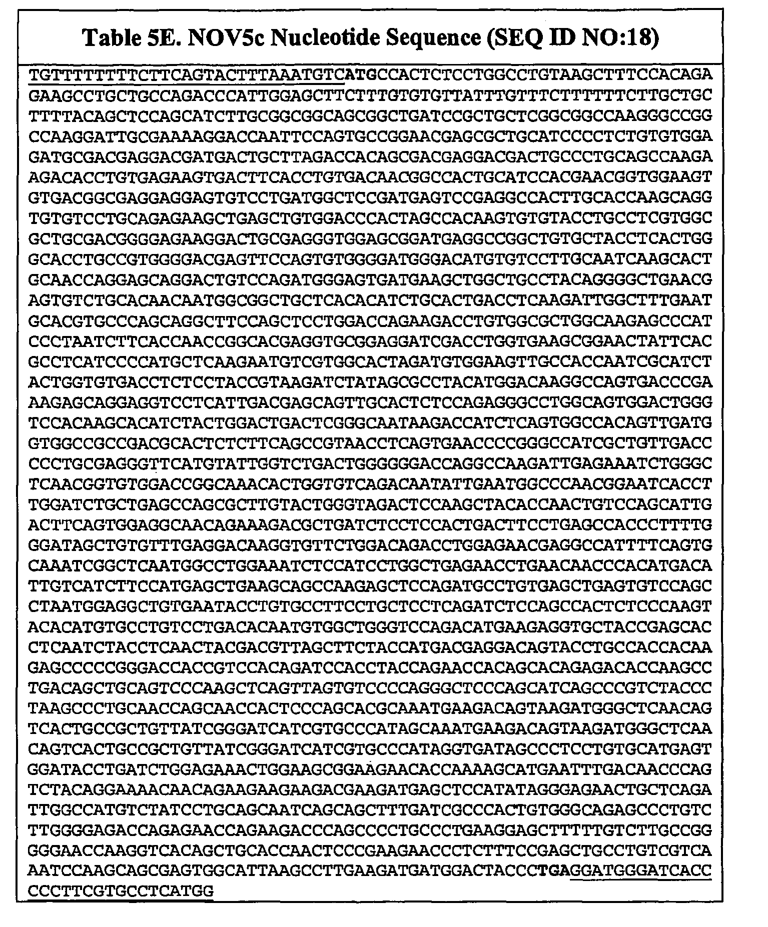

The NOV5c sequence was identified through an exon linking process. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5c sequence.

The NOV5c nucleic acid was identified on chromosome 1 and has 1626 of 1922 bases (84%) identical to a 4468 bp apolipoprotein E receptor 2 mRNA from Homo sapiens (GENBANK-ID:D50678|acc:D50678) (E = 1.1e⁻¹²⁷)

A disclosed NOV5c polypeptide (SEQ ID NO:19) encoded by SEQ ID NO:18 is 825 amino acid residues and is presented using the one-letter code in Table 5F. Signal P, Psort and/or Hydropathy results predict that NOV5c has a signal peptide and is likely to be localized in the plasma membrane with a certainty of 0.6000. In other embodiments, NOV5c may also be localized to the Golgi body with acertainty of 0.4000, the endoplasmic reticulum (membrane) with a certainty of 0.3000, or the microbody (peroxisome) with a certainty of 0.3000. The most likely cleavage site for a NOV5c peptide is between amino acids 44 and 45, at: AAA-DP.

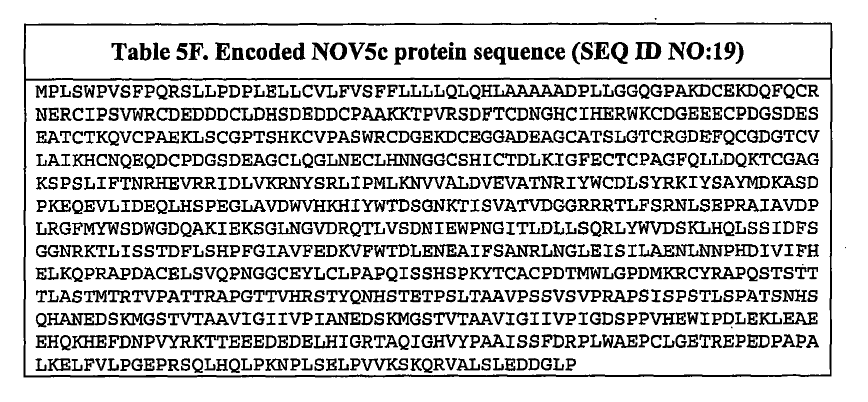

The disclosed NOV5c amino acid sequence has 586 of 659 amino acid residues (88%) identical to, and 601 of 659 amino acid residues (91 %) similar to, the 793 amino acid residue APOER2DELTA4-7 protein from Homo sapiens (SPTREMBL-ACC: 014968) (E = 0.0).

### NOV5d

A disclosed NOV5d nucleic acid was isolated by subjecting NOV5c to the exon linking process to confirm the sequence. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such primers were designed based on in silico predictions for the full length cDNA, part (one or more exons) of the DNA or protein sequence of the target sequence, or by translated homology of the predicted exons to closely related human sequences sequences from other species. These primers were then employed in PCR amplification based on the following pool of human cDNAs: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. Usually the resulting amplicons were gel purified, cloned and sequenced to high redundancy. The resulting sequences from all clones were assembled with themselves, with other fragments in CuraGen Corporation's database and with public ESTs. Fragments and ESTs were included as components for an assembly when the extent of their identity with another component of the assembly was at least 95% over 50 bp. In addition, sequence traces were evaluated manually and edited for corrections if appropriate. The novel NOV5d sequence of 3039 nucleotides (also referred to as CG55256-02) encoding a novel apolipoprotein e-like protein is shown in Table 5G. An open reading frame was identified beginning with an ATG initiation codon at nucleotides 1-3 and ending with a TGA codon at nucleotides 3037-3039. The start and stop codons are in bold letters.in Table 5G.

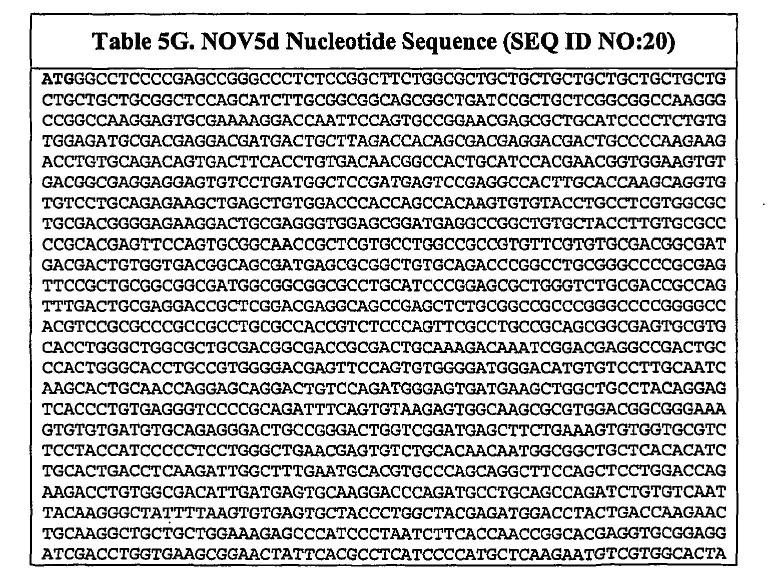

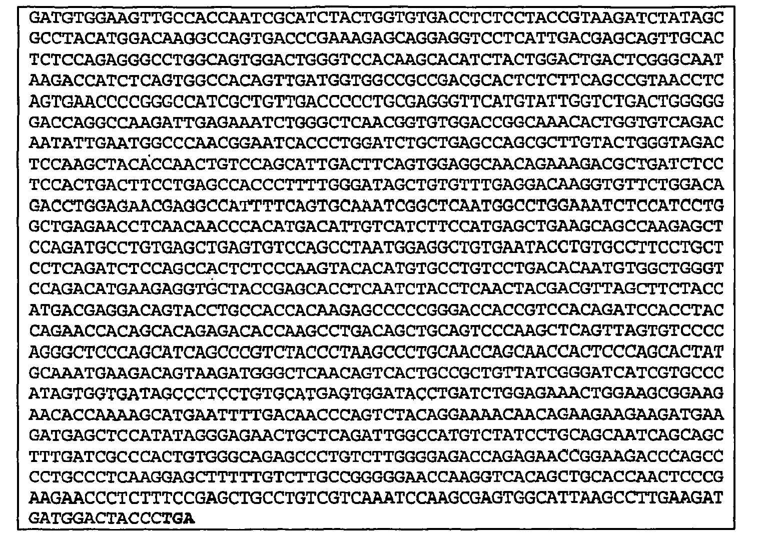

The NOV5d sequence was identified through an exon linking process. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5d sequence.

The NOV5d nucleic acid was identified to the p34 region of human chromosome 1 and has 2508 of 2995 bases (83%) identical to a gb:GENBANK-ID:D50678|acc:D50678.1 mRNA from Homo sapiens (Human mRNA for apolipoprotein E receptor 2, complete cds) (E = 0.0)

A disclosed NOV5d polypeptide (SEQ ID NO:21) encoded by SEQ ID NO:20 is 1012 amino acid residues and is presented using the one-letter code in Table 5H. Signal P, Psort and/or Hydropathy results predict that NOV5d has a signal peptide and is likely to be localized in the plasma membrane with a certainty of 0.6760. In other embodiments, NOV5d may also be localized to the endoplasmic reticulum (membrane) with acertainty of 0.1000, the endoplasmic reticulum (lumen) with a certainty of 0.1000, or the extracellularly with a certainty of 0.1000. The most likely cleavage site for a NOV5d peptide is between amino acids 32 and 33, at: AAA-AA.

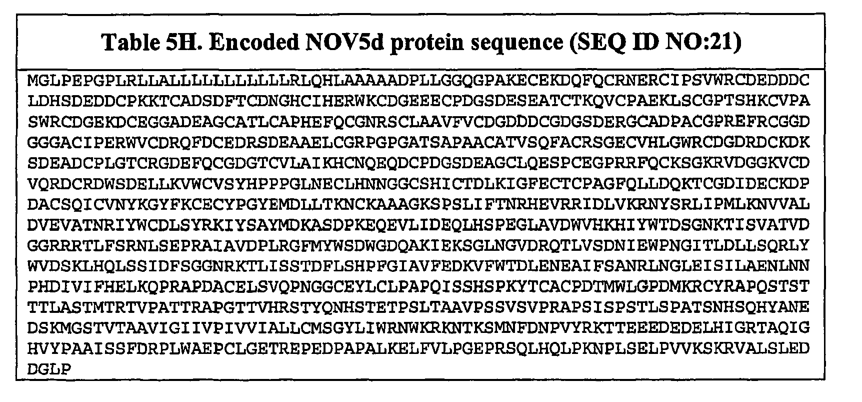

The disclosed NOV5d amino acid sequence has 742 of 931 amino acid residues (79%) identical to, and 777 of 931 amino acid residues (83%) similar to, the 963 amino acid residue ptnr:SPTREMBL-ACC:Q14114 protein from Homo sapiens (Human) (APOLIPOPROTEIN E RECEPTOR 2 PRECURSOR) (E = 0.0).

NOV5d is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. This information was derived by determining the tissue sources of the sequences that were included in the invention including but not limited to SeqCalling sources, Public EST sources, Literature sources, and/or RACE sources.

In addition, the sequence is predicted to be expressed in the following tissues because of the expression pattern of (GENBANK-ID: gb:GENBANK.-ID:D50678|acc:D50678.1) a closely related Human mRNA for apolipoprotein E receptor 2, complete cds homolog in species Homo sapiens :brain.

### NOV5e

A disclosed NOV5e nucleic acid was isolated by subjecting NOV5c to the exon linking process to confirm the sequence. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such primers were designed based on in silico predictions for the full length cDNA, part (one or more exons) of the DNA or protein sequence of the target sequence, or by translated homology of the predicted exons to closely related human sequences sequences from other species. These primers were then employed in PCR amplification based on the following pool of human cDNAs: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. Usually the resulting amplicons were gel purified, cloned and sequenced to high redundancy. The resulting sequences from all clones were assembled with themselves, with other fragments in CuraGen Corporation's database and with public ESTs. Fragments and ESTs were included as components for an assembly when the extent of their identity with another component of the assembly was at least 95% over 50 bp. In addition, sequence traces were evaluated manually and edited for corrections if appropriate. The novel NOV5e sequence of 2537 nucleotides (also referred to as CG55256-03) encoding a novel apolipoprotein e-like protein is shown in Table 5I. An open reading frame was identified beginning with an ATG initiation codon at nucleotides 1-3 and ending with a TGA codon at nucleotides 2086-2088. A putative untranslated region downstream from the termination codon is underlined in Table 51, and the start and stop codons are in bold letters.

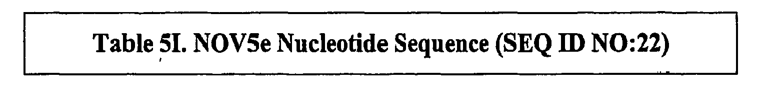

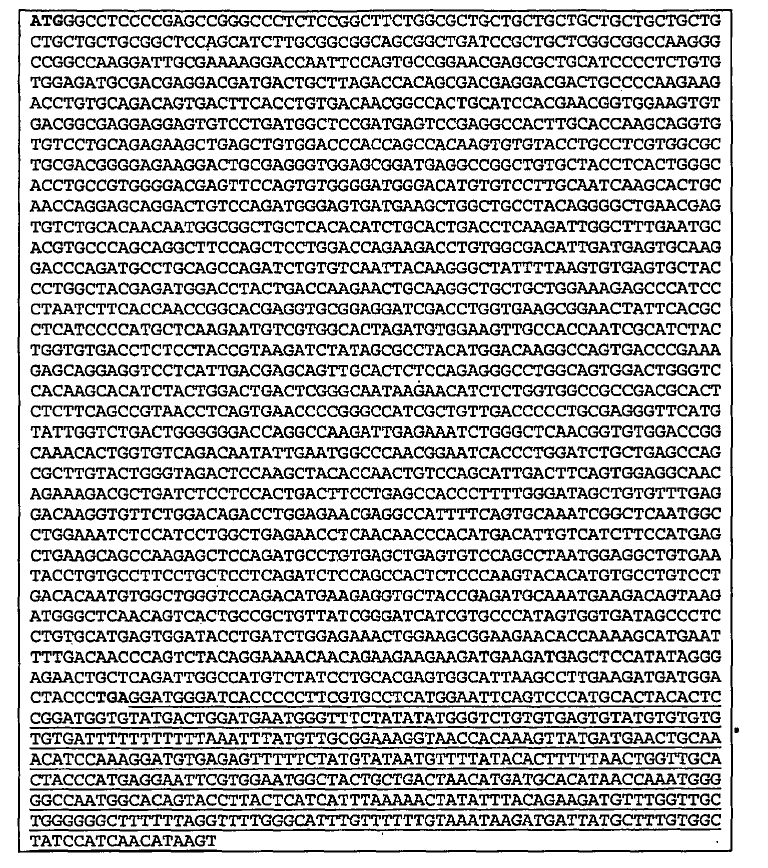

The NOV5e sequence was identified through an exon linking process. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5e sequence.

The NOV5e nucleic acid was identified to the p34 region of human chromosome 1 and has 1359 of 1359 bases (100%) identical to a gb:GENBANK-ID:HSZ75190|acc:Z75190.1 mRNA from Homo sapiens (H.sapiens mRNA for apolipoprotein E receptor 2) (E = 0.0)

A disclosed NOV5e polypeptide (SEQ ID NO:23) encoded by SEQ ID NO:22 is 695 amino acid residues and is presented using the one-letter code in Table 5J. Signal P, Psort and/or Hydropathy results predict that NOV5e has a signal peptide and is likely to be localized in the plasma membrane with a certainty of 0.6760. In other embodiments, NOV5e may also be localized to the endoplasmic reticulum (membrane) with acertainty of 0.1000, the endoplasmic reticulum (lumen) with a certainty of 0.1000, or the extracellularly with a certainty of 0.1000. The most likely cleavage site for a NOV5e peptide is between amino acids 32 and 33, at: AAA-AA.

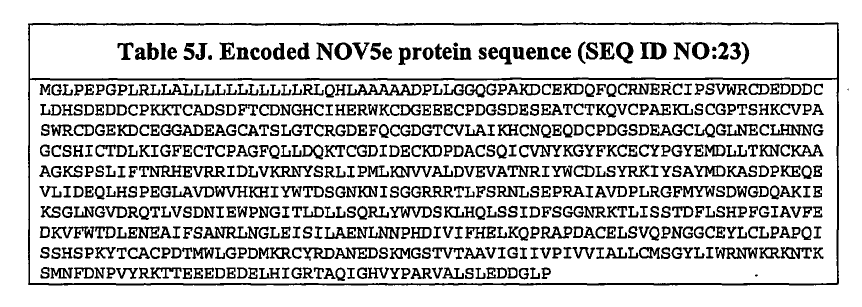

The disclosed NOV5e amino acid sequence has 683 of 699 amino acid residues (97%) identical to, and 685 of 699 amino acid residues (97%) similar to, the 699 amino acid residue ptnr:SPTREMBL-ACC:Q99876 protein from Homo sapiens (Human) (APOLIPOPROTEIN E RECEPTOR 2 906) (E = 0.0).

NOV5e is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. This information was derived by determining the tissue sources of the sequences that were included in the invention including but not limited to SeqCalling sources, Public EST sources, and/or RACE sources.

In addition, the sequence is predicted to be expressed in the following tissues because of the expression pattern of (GENBANK-ID: gb:GENBANK-ID:HSZ75190|acc:Z75190.1) a closely related H.sapiens mRNA for apolipoprotein E receptor 2 homolog in species Homo sapiens : umbilical vein endothelial cells.

The presence of identifiable domains in NOV5e was determined by searches using algorithms such as PROSITE, Blocks, Pfam, ProDomain, Prints and then determining the Interpro number by crossing the domain match (or numbers) using the Interpro website (http:www.ebi.ac.uk/interpro/). The results indicate that this protein contains the following protein domains (as defined by Interpro) at the indicated positions: low density lipoprotein receptor domains at amino acid positions 45 to 83, 84 to 165, 168 to 206; low density receptor repeat at amino acid positions333 to 378, 380 to 416, 418 to 460, 462 to 546; EGF-like domains at amino acid positions 86 to 122, 170 to 204, 211 to 245, 251 to 285, 556 to 600, 739 to 783; and trypsin inhibitor like cysteine rich domain at amino acid positions 195 to 251. This indicates that the sequence of the invention has properties similar to those of other proteins known to contain this/these domain(s) and similar to the properties of these domains.

### NOV5f

A disclosed NOV5f nucleic acid was isolated by subjecting NOV5c to the exon linking process to confirm the sequence. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such primers were designed based on in silico predictions for the full length cDNA, part (one or more exons) of the DNA or protein sequence of the target sequence, or by translated homology of the predicted exons to closely related human sequences sequences from other species. These primers were then employed in PCR amplification based on the following pool of human cDNAs: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. Usually the resulting amplicons were gel purified, cloned and sequenced to high redundancy. The resulting sequences from all clones were assembled with themselves, with other fragments in CuraGen Corporation's database and with public ESTs. Fragments and ESTs were included as components for an assembly when the extent of their identity with another component of the assembly was at least 95% over 50 bp. In addition, sequence traces were evaluated manually and edited for corrections if appropriate. The novel NOV5f sequence of 2474 nucleotides (also referred to as CG55256-04) encoding a novel apolipoprotein e-like protein is shown in Table 5K. An open reading frame was identified beginning with an ATG initiation codon at nucleotides 30-32 and ending with a TGA codon at nucleotides 2442-2444. A putative untranslated region upstream from the initiation codon and downstream from the termination codon is underlined in Table 5K, and the start and stop codons are in bold letters.

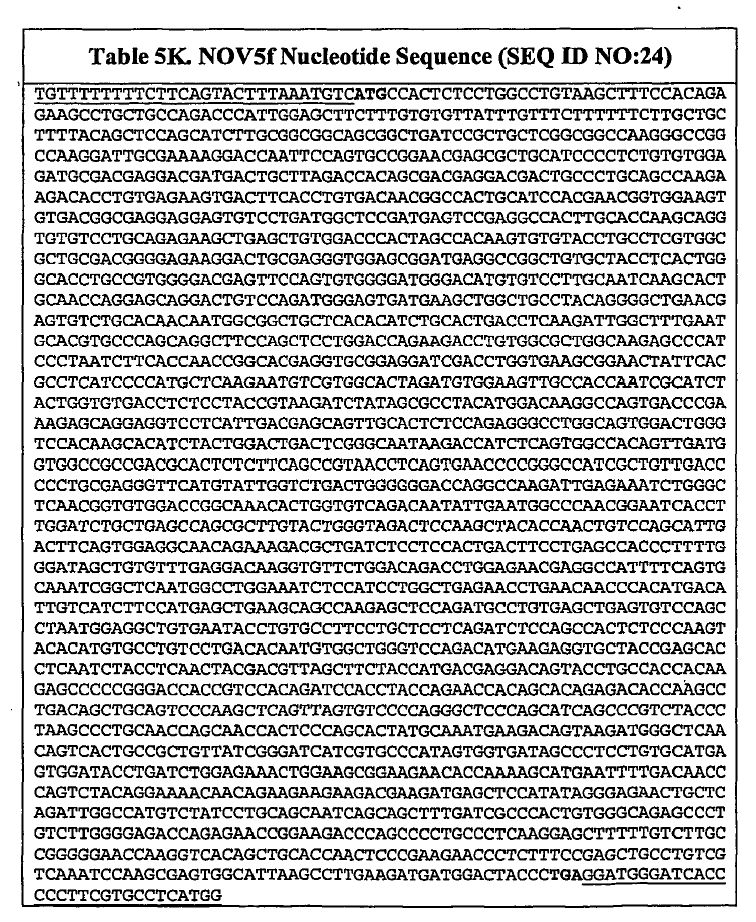

The NOV5f sequence was identified through an exon linking process. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5f sequence.

The NOV5f nucleic acid was identified to the p34 region of human chromosome 1 and has 2047 of 2341 bases (87%) identical to a gb:GENBANK-ID:D50678|acc:D50678.1 mRNA from Homo sapiens (Human mRNA for apolipoprotein E receptor 2, complete cds) (E = 0.0)

A disclosed NOV5f polypeptide (SEQ ID NO:25) encoded by SEQ ID NO:24 is 804 amino acid residues and is presented using the one-letter code in Table 5L. Signal P, Psort and/or Hydropathy results predict that NOV5f has a signal peptide and is likely to be localized in the plasma membrane with a certainty of 0.6000. In other embodiments, NOV5f may also be localized to the Golgi body with acertainty of 0.4000, the endoplasmic reticulum (membrane) with a certainty of 0.3000, or the microbody (peroxisome) with a certainty of 0.3000. The most likely cleavage site for a NOV5f peptide is between amino acids 44 and 45, at: AAA-DP.

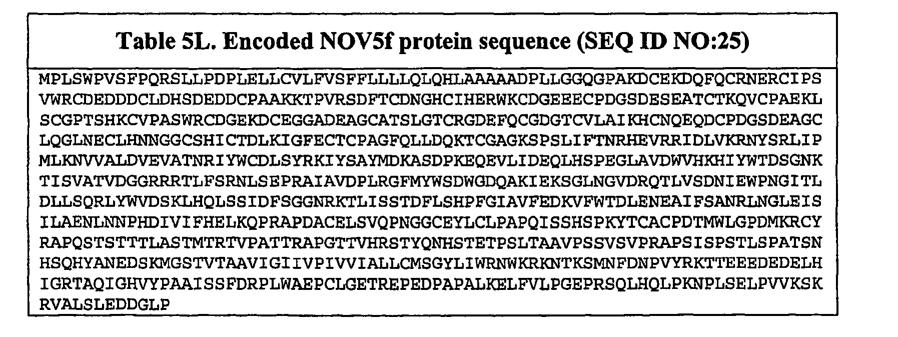

The disclosed NOV5f amino acid sequence has 716 of 789 amino acid residues (90%) identical to, and 731 of 789 amino acid residues (92%) similar to, the 793 amino acid residue ptnr:SPTREMBL-ACC:O14968 protein from Homo sapiens (Human) (APOER2DELTA4-7) (E = 0.0).

NOV5f is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. This information was derived by determining the tissue sources of the sequences that were included in the invention including but not limited to SeqCalling sources, Public EST sources, Literature sources, and/or RACE sources.

In addition, the sequence is predicted to be expressed in the following tissues because of the expression pattern of (GENBANK-ID: gb:GENBANK-ID:D50678|acc:D50678.1) a closely related Human mRNA for apolipoprotein E receptor 2, complete cds homolog in species Homo sapiens: brain.

The presence of identifiable domains in NOV5f was determined by searches using algorithms such as PROSITE, Blocks, Pfam, ProDomain, Prints and then determining the Interpro number by crossing the domain match (or numbers) using the Interpro website (http:www.ebi.ac.uk/interpro/). The results indicate that this protein contains the following protein domains (as defined by Interpro) at the indicated positions: domain name low density lipoprotein receptor domains at amino acid positions 55 to 93, 96 to 177, 180 to 218; low density receptor repeat domains at amino acid positions 303 to 348, 350 to 391, 393 to 435, 437 to 521; EGF-like domains at amino acid positions 182 to 216, 223 to 257, 531 to 575; and fusion glycoprotein FO domain at amino acid positions 650 to 683. This indicates that the sequence of the invention has properties similar to those of other proteins known to contain this/these domain(s) and similar to the properties of these domains.

### NOV5g

A disclosed NOV5g nucleic acid was isolated by The sequence of NOV5g was derived by laboratory cloning of cDNA fragments, by *in silico* prediction of the sequence. cDNA fragments covering either the full length of the DNA sequence, or part of the sequence, or both, were cloned. In silico prediction was based on sequences available in Curagen's proprietary sequence databases or in the public human sequence databases, and provided either the full length DNA sequence, or some portion thereof. The novel NOV5g sequence of 4150 nucleotides (also referred to as CG55256-05) encoding a novel apolipoprotein e-like protein is shown in Table 5M. An open reading frame was identified beginning with an ATG initiation codon at nucleotides 103-105 and ending with a TGA codon at nucleotides 2674-2676. A putative untranslated region upstream from the initiation codon and downstream from the termination codon is underlined in Table 5M, and the start and stop codons are in bold letters.

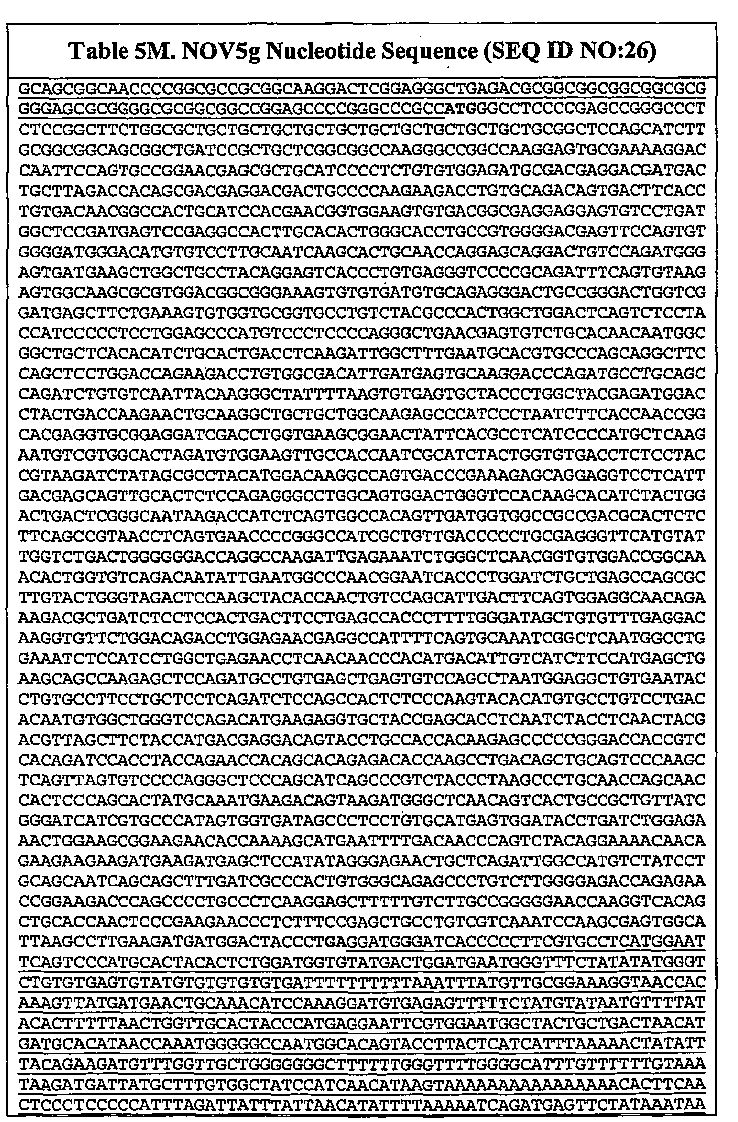

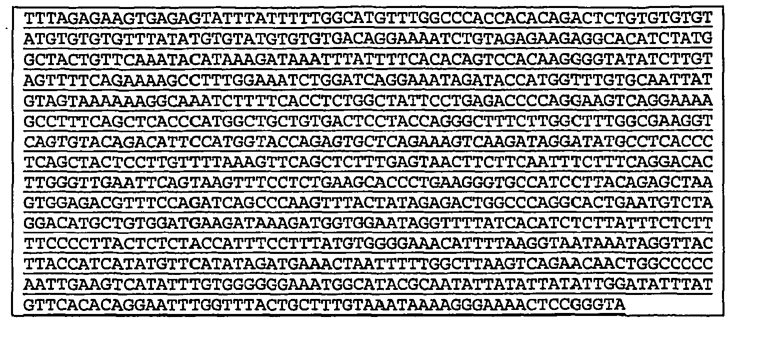

The NOV5g sequence was identified through an exon linking process. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5g sequence.

The NOV5g nucleic acid was identified to the p34 region of human chromosome 1 and has 3358 of 3361 bases (99%) identical to a gb:GENBANK-ID:D50678|acc:D50678.1 mRNA from Homo sapiens (Human mRNA for apolipoprotein E receptor 2, complete cds) (E = 0.0)

A disclosed NOV5g polypeptide (SEQ ID NO:27) encoded by SEQ ID NO:26 is 857 amino acid residues and is presented using the one-letter code in Table 5N. Signal P, Psort and/or Hydropathy results predict that NOV5g has a signal peptide and is likely to be localized in the plasma membrane with a certainty of 0.6760. In other embodiments, NOV5g may also be localized to the endoplasmic reticulum (membrane) with acertainty of 0.1000, the endoplasmic reticulum (lumen) with a certainty of 0.1000, or extracellularly with a certainty of 0.1000. The most likely cleavage site for a NOV5g peptide is between amino acids 32 and 33, at: AAA-DP.

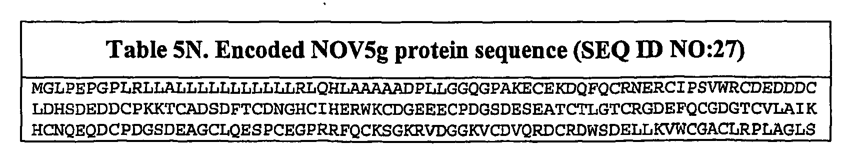

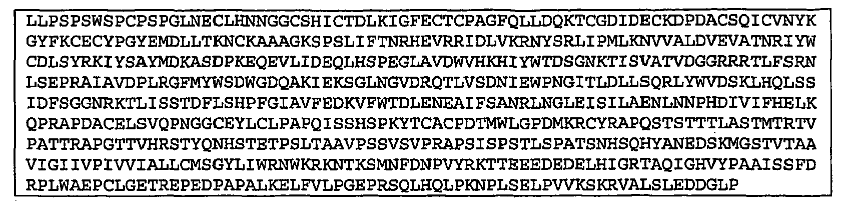

The disclosed NOV5g amino acid sequence has 684 of 816 amino acid residues (83%) identical to, and 703 of 816 amino acid residues (86%) similar to, the 963 amino acid residue ptnr:SPTREMBL-ACC:Q14114 protein from Homo sapiens (Human) (APOLIPOPROTEIN E RECEPTOR 2 PRECURSOR) (E = 0.0).

NOV5g is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus, Cerebral Medulla/Cerebral white matter, Colon, Lung, Lymphoid tissue, Parietal Lobe, Tonsils, Whole Organism. Expression information was derived from the tissue sources of the sequences that were included in the derivation of the sequence of NOV5g.

The presence of identifiable domains in NOV5g was determined by searches versus domain databases such as Pfam, PROSITE, ProDom, Blocks or Prints and then identified by the Interpro domain accession number. Significant domains are summarized below.

IPR000033, YWTD repeat, from amino acid 356 to amino acid 401, from amino acid 403 to amino acid 444, from amino acid 446 to amino acid 488, from amino acid 490 to amino acid 533, from amino acid 534 to amino acid 574. This domain is also known as the YWTD motif after the most conserved region of the repeat. The YWTD repeat is found in multiple tandem repeats and has been predicted to form a beta-propeller structure. It is found in a variety of proteins that include, vitellogenin receptor from Drosophila, low-density lipoprotein (LDL) receptor, preproepidermal growth factor, nidogen (entactin) and others.

IPR002172, Low density lipoprotein (LDL)-receptor class A (LDLRA) domain, from amino acid 45 to amino acid 83, from amino acid 84 to amino acid 124, from amino acid 125 to amino acid 163, from amino acid 165 to amino acid 207. Low density lipoprotein (LDL) is the major cholesterol-carrying lipoprotein of plasma. The receptor protein binds LDL and transports it into cells by endocytosis. In order to be internalised, the receptor-ligand complex must first cluster into clathrin-coated pits. Seven successive cysteine-rich repeats of about 40 amino acids are present in the N-terminal of this multidomain membrane protein. The LDL-receptor class A domain contains 6 disulfide-bound cysteines and a highly conserved cluster of negatively charged amino acids, of which many are clustered on one face of the module. In LDL-receptors the class A domains form the binding site for LDL and calcium. The acidic residues between the fourth and sixth cysteines are important for high-affinity binding of positively charged sequences in LDLR's ligands. The repeat has been shown to consist of a beta-hairpin structure followed by a series of beta turns. The binding of calcium seems to induce no significant conformational change. Following these repeats is a 350 residue domain that resembles part of the epidermal growth factor (EGF) precursor. Similar domains have been found in several extracellular and membrane proteins.

IPR000561, EGF-like domain, from amino acid 234 to amino acid 268, from amino acid 274 to amino acid 308 A sequence of about thirty to forty amino-acid residues long found in the sequence of epidermal growth factor (EGF) has been shown to be present, in a more or less conserved form, in a large number of other, mostly animal proteins. The list of proteins currently known to contain one or more copies of an EGF-like pattern is large and varied. The functional significance of EGF domains in what appear to be unrelated proteins is not yet clear. However, a common feature is that these repeats are found in the extracellular domain of membrane-bound proteins or in proteins known to be secreted (exception: prostaglandin G/H synthase). The EGF domain includes six cysteine residues which have been shown (in EGF) to be involved in disulfide bonds. The main structure is a two-stranded beta-sheet followed by a loop to a C-terminal short two-stranded sheet. Subdomains between the conserved cysteines vary in length.

### NOV5h

A disclosed NOV5h nucleic acid was isolated by The sequence of NOV5h was derived by laboratory cloning of cDNA fragments, by *in silico* prediction of the sequence. cDNA fragments covering either the full length of the DNA sequence, or part of the sequence, or both, were cloned. In silico prediction was based on sequences available in Curagen's proprietary sequence databases or in the public human sequence databases, and provided either the full length DNA sequence, or some portion thereof. The novel NOV5h sequence of 4180 nucleotides (also referred to as CG55256-06) encoding a novel apolipoprotein e-like protein is shown in Table 50. An open reading frame was identified beginning with an ATG initiation codon at nucleotides 163-165 and ending with a TGA codon at nucleotides 2704-2706. A putative untranslated region upstream from the initiation codon and downstream from the termination codon is underlined in Table 50, and the start and stop codons are in bold letters.

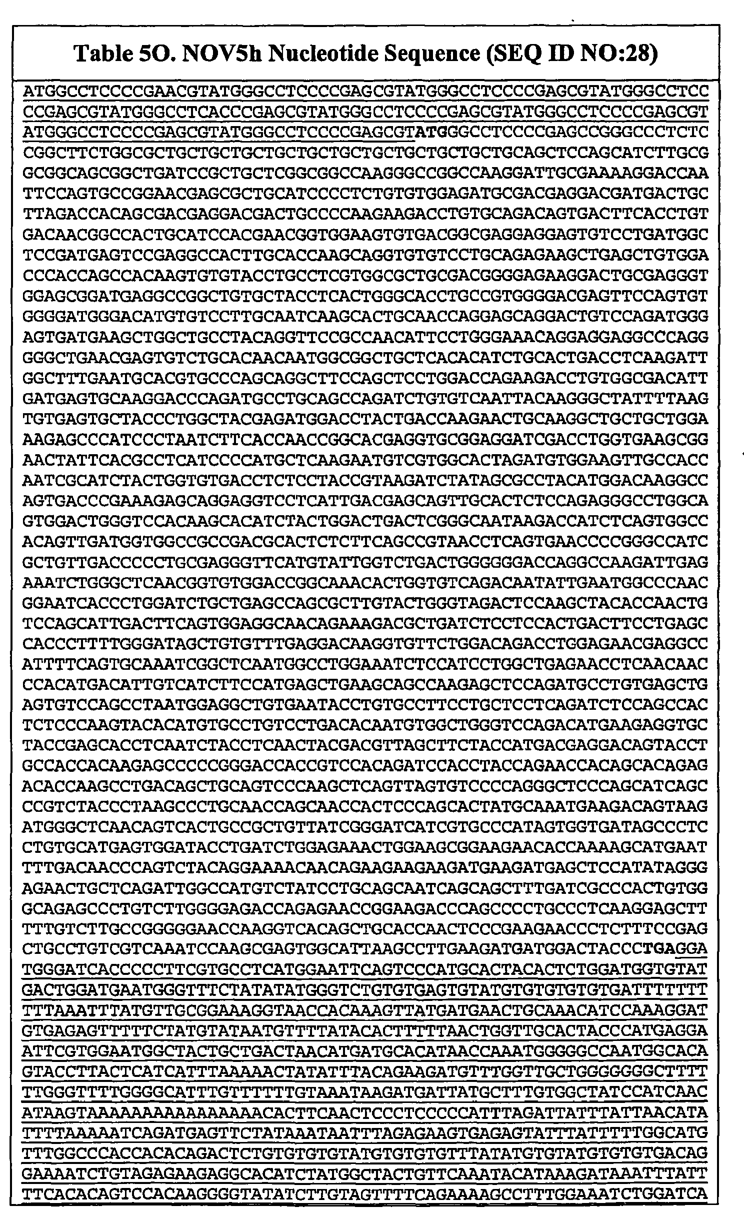

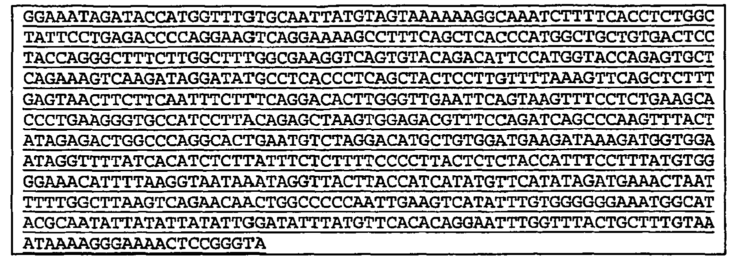

The NOV5h'sequence was identified through an exon linking process. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5h sequence.

The NOV5h nucleic acid was identified to the p34 region of human chromosome 1 and has 3634 of 3851 bases (94%) identical to a gb:GENBANK-ID:D50678|acc:D50678.1 mRNA from Homo sapiens (Human mRNA for apolipoprotein E receptor 2, complete cds) (E = 0.0)

A disclosed NOV5h polypeptide (SEQ ID NO:29) encoded by SEQ ID NO:28 is 847 amino acid residues and is presented using the one-letter code in Table 5P. Signal P, Psort and/or Hydropathy results predict that NOV5h has a signal peptide and is likely to be localized in the plasma membrane with a certainty of 0.6760. In other embodiments, NOV5h may also be localized to the endoplasmic reticulum (membrane) with acertainty of 0.1000, the endoplasmic reticulum (lumen) with a certainty of 0.1000, or extracellularly with a certainty of 0.1000. The most likely cleavage site for a NOV5h peptide is between amino acids 32 and 33, at: AAA-AA.

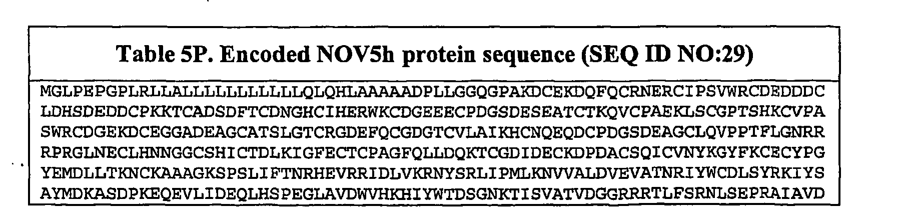

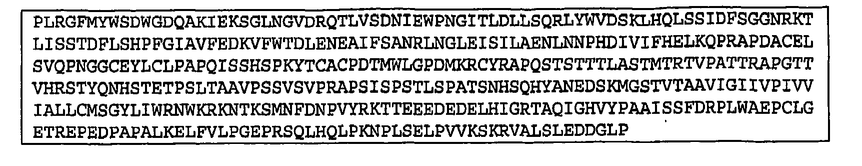

The disclosed NOV5h amino acid sequence has 719 of 806 amino acid residues (89%) identical to, and 735 of 806 amino acid residues (91%) similar to, the 963 amino acid residue ptnr:SPTREMBL-ACC:Q14114 protein from Homo sapiens (Human) (APOLIPOPROTEIN E RECEPTOR 2 PRECURSOR) (E = 0.0).

NOV5h is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus, Cerebral Medulla/Cerebral white matter, Colon, Lung, Lymphoid tissue, Parietal Lobe, Tonsils, Whole Organism. Expression information was derived from the tissue sources of the sequences that were included in the derivation of the sequence of NOV5h

The sequence is predicted to be expressed in placenta because of the expression pattern of (GENBANK-ID: gb:GENBANK-ID:D50678|acc:D50678.1) a closely related Human mRNA for apolipoprotein E receptor 2, complete cds homolog in species Homo sapiens.

The presence of identifiable domains in NOV5h was determined by searches versus domain databases such as Pfam, PROSITE, ProDom, Blocks or Prints and then identified by the Interpro domain accession number. Significant domains are summarized below.

IPR002172, Low density lipoprotein (LDL)-receptor class A (LDLRA) domain, from amino acid 45 to amino acid 83, from amino acid 84 to amino acid 124, from amino acid 125 to amino acid 165, from amino acid 168 to amino acid 206. Low density lipoprotein (LDL) is the major cholesterol-carrying lipoprotein of plasma. The receptor protein binds LDL and transports it into cells by endocytosis. In order to be internalised, the receptor-ligand complex must first cluster into clathrin-coated pits. Seven successive cysteine-rich repeats of about 40 amino acids are present in the N-terminal of this multidomain membrane protein. The LDL-receptor class A domain contains 6 disulfide-bound cysteines and a highly conserved cluster of negatively charged amino acids, of which many are clustered on one face of the module. In LDL-receptors the class A domains form the binding site for LDL and calcium. The acidic residues between the fourth and sixth cysteines are important for high-affinity binding of positively charged sequences in LDLR's ligands. The repeat has been shown to consist of a beta-hairpin structure followed by a series of beta turns. The binding of calcium seems to induce no significant conformational change. Following these repeats is a 350 residue domain that resembles part of the epidermal growth factor (EGF) precursor. Similar domains have been found in several extracellular and membrane proteins.

IPR000033, YWTD repeat, from amino acid 346 to amino acid 391, from amino acid 393 to amino acid 434, from amino acid 436 to amino acid 478, from amino acid 480 to amino acid 523, from amino acid 524 to amino acid 564. This domain is also known as the YWTD motif after the most conserved region of the repeat. The YWTD repeat is found in multiple tandem repeats and has been predicted to form a beta-propeller structure. It is found in a variety of proteins that include, vitellogenin receptor from Drosophila, low-density lipoprotein (LDL) receptor, preproepidermal growth factor, nidogen (entactin) and others.

IPR000561, EGF-like domain, from amino acid 224 to amino acid 258, from amino acid 264 to amino acid 298, from amino acid 574 to amino acid 618. A sequence of about thirty to forty amino-acid residues long found in the sequence of epidermal growth factor (EGF) has been shown to be present, in a more or less conserved form, in a large number of other, mostly animal proteins. The list of proteins currently known to contain one or more copies of an EGF-like pattern is large and varied. The functional significance of EGF domains in what appear to be unrelated proteins is not yet clear. However, a common feature is that these repeats are found in the extracellular domain of membrane-bound proteins or in proteins known to be secreted (exception: prostaglandin G/H synthase). The EGF domain includes six cysteine residues which have been shown (in EGF) to be involved in disulfide bonds. The main structure is a two-stranded beta-sheet followed by a loop to a C-terminal short two-stranded sheet. Subdomains between the conserved cysteines vary in length.

This indicates that the sequence of the invention has properties similar to those of other proteins known to contain this/these domain(s) and similar to the properties of these domains.

### NOV5i

A disclosed NOV5i nucleic acid was isolated by The sequence of NOV5i was derived by laboratory cloning of cDNA fragments, by *in silico* prediction of the sequence. cDNA fragments covering either the full length of the DNA sequence, or part of the sequence, or both, were cloned. In silico prediction was based on sequences available in Curagen's proprietary sequence databases or in the public human sequence databases, and provided either the full length DNA sequence, or some portion thereof. The novel NOV5i sequence of 4294 nucleotides (also referred to as CG55256-07) encoding a novel apolipoprotein e-like protein is shown in Table 5Q. An open reading frame was identified beginning with an ATG initiation codon at nucleotides 103-105 and ending with a TGA codon at nucleotides 2818-2820. A putative untranslated region upstream from the initiation codon and downstream from the termination codon is underlined in Table 5Q, and the start and stop codons are in bold letters.

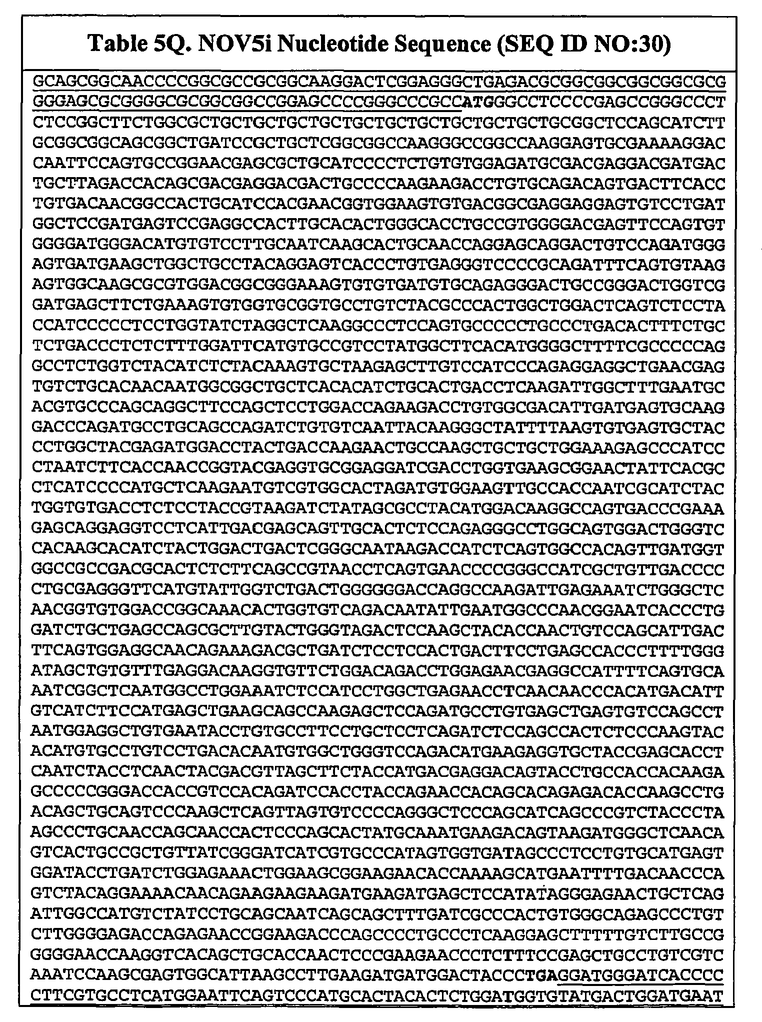

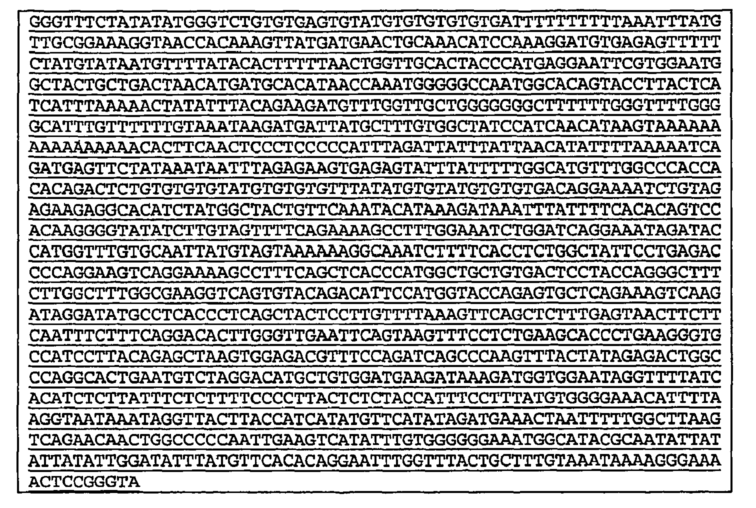

The NOV5i sequence was identified through an exon linking process. PCR primers were designed by starting at the most upstream sequence available, for the forward primer, and at the most downstream sequence available for the reverse primer. In each case, the sequence was examined, walking inward from the respective termini toward the coding sequence, until a suitable sequence that is either unique or highly selective was encountered, or, in the case of the reverse primer, until the stop codon was reached. Such suitable sequences were then employed as the forward and reverse primers in a PCR amplification based on a library containing a wide range of cDNA species. The resulting amplicon was gel purified, cloned and sequenced to high redundancy to provide the NOV5i sequence.

The NOV5i nucleic acid was identified to the p34 region of human chromosome 1 and has 3375 of 3389 bases (99%) identical to a gb:GENBANK-ID:D50678|acc:D50678.1 mRNA from Homo sapiens (Human mRNA for apolipoprotein E receptor 2, complete cds) (E = 0.0)

A disclosed NOV5i polypeptide (SEQ ID NO:31) encoded by SEQ ID NO:30 is 905 amino acid residues and is presented using the one-letter code in Table 5R. Signal P, Psort and/or Hydropathy results predict that NOV5i has a signal peptide and is likely to be localized in the plasma membrane with a certainty of 0.6760. In other embodiments, NOV5i may also be localized to the endoplasmic reticulum (membrane) with acertainty of 0.1000, the endoplasmic reticulum (lumen) with a certainty of 0.1000, or extracellularly with a certainty of 0.1000. The most likely cleavage site for a NOV5i peptide is between amino acids 32 and 33, at: AAA-AA.

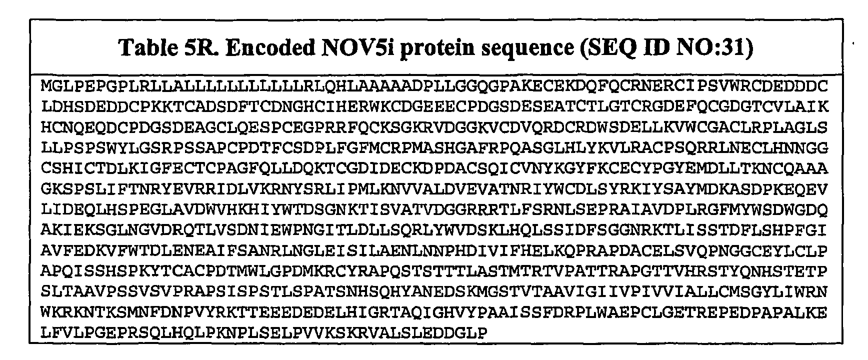

The disclosed NOV5i amino acid sequence has 625 of 627 amino acid residues (99%) identical to, and 626 of 627 amino acid residues (99%) similar to, the 963 amino acid residue ptnr:SPTREMBL-ACC:Q14114 protein from Homo sapiens (Human) (APOLIPOPROTEIN E RECEPTOR 2 PRECURSOR) (E = 0.0).

NOV5i is expressed in at least the following tissues: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus, Cerebral Medulla/Cerebral white matter, Colon, Lung, Lymphoid tissue, Parietal Lobe, Tonsils, Whole Organism. Expression information was derived from the tissue sources of the sequences that were included in the derivation of the sequence of NOV5i.

The sequence is predicted to be expressed in placenta because of the expression pattern of (GENBANK-ID: gb:GENBANK-ID:D50678|acc:D50678.1) a closely related Human mRNA for apolipoprotein E receptor 2, complete cds homolog in species Homo sapiens.

The presence of identifiable domains in NOV5i was determined by searches versus domain databases such as Pfam, PROSITE, ProDom, Blocks or Prints and then identified by the Interpro domain accession number. Significant domains are summarized below.

IPR002172, Low density lipoprotein (LDL)-receptor class A (LDLRA) domain, from amino acid 45 to amino acid 83, from amino acid 84 to amino acid 124, from amino acid 125 to amino acid 163, from amino acid 165 to amino acid 207. Low density lipoprotein (LDL) is the major cholesterol-carrying lipoprotein of plasma. The receptor protein binds LDL and transports it into cells by endocytosis. In order to be internalised, the receptor-ligand complex must first cluster into clathrin-coated pits. Seven successive cysteine-rich repeats of about 40 amino acids are present in the N-terminal of this multidomain membrane protein. The LDL-receptor class A domain contains 6 disulfide-bound cysteines and a highly conserved cluster of negatively charged amino acids, of which many are clustered on one face of the module. In LDL-receptors the class A domains form the binding site for LDL and calcium. The acidic residues between the fourth and sixth cysteines are important for high-affinity binding of positively charged sequences in LDLR's ligands. The repeat has been shown to consist of a beta-hairpin structure followed by a series of beta turns. The binding of calcium seems to induce no significant conformational change. Following these repeats is a 350 residue domain that resembles part of the epidermal growth factor (EGF) precursor. Similar domains have been found in several extracellular and membrane proteins.

IPR000033, YWTD repeat, from amino acid 404 to amino acid 449, from amino acid 451 to amino acid 492, from amino acid 494 to amino acid 536, from amino acid 538 to amino acid 581, from amino acid 582 to amino acid 622. This domain is also known as the YWTD motif after the most conserved region of the repeat. The YWTD repeat is found in multiple tandem repeats and has been predicted to form a beta-propeller structure. It is found in a variety of proteins that include, vitellogenin receptor from Drosophila, low-density lipoprotein (LDL) receptor, preproepidermal growth factor, nidogen (entactin) and others.

IPR000561, EGF-like domain, from amino acid 322 to amino acid 356, from amino acid 632 to amino acid 676. A sequence of about thirty to forty amino-acid residues long found in the sequence of epidermal growth factor (EGF) has been shown to be present, in a more or less conserved form, in a large number of other, mostly animal proteins. The list of proteins currently known to contain one or more copies of an EGF-like pattern is large and varied. The functional significance of EGF domains in what appear to be unrelated proteins is not yet clear. However, a common feature is that these repeats are found in the extracellular domain of membrane-bound proteins or in proteins known to be secreted (exception: prostaglandin G/H synthase). The EGF domain includes six cysteine residues which have been shown (in EGF) to be involved in disulfide bonds. The main structure is a two-stranded beta-sheet followed by a loop to a C-terminal short two-stranded sheet. Subdomains between the conserved cysteines vary in length.

This indicates that the sequence of the invention has properties similar to those of other proteins known to contain this/these domain(s) and similar to the properties of these domains.

NOV5a also has homology to the amino acid sequences shown in the BLASTP data listed in Table 5S.

| **Table 5S. BLAST results for NOV5a** | | | | | |
|---|---|---|---|---|---|
| Gene Index/ Identifier | Protein/ Organism | Length (aa) | Identity (%) | Positives (%) | Expect |
| Q99876 | homo sapiens (human). apolipoprotein e receptor 2 906 | 699 | 608/658 (92%) | 612/658, (93%) | 0.0 |
| O14968 | homo sapiens (human). apoer2delta4-7 | 793 | 495/565 (88%) | 499/565, (88%) | 0.0 |
| Q14114 | homo sapiens (human). apolipoprotein e receptor 2 precursor | 963 | 475/574 (83%) | 493/574, (86%) | 0.0 |
| Q98931 | gallus gallus (chicken). 1dl receptor homolog lr8b protein | 917 | 392/610 (64%) | 455/610, (75%) | 0.0 |

The homology of these sequences is shown graphically in the ClustalW analysis shown in Table 5T.

| **Table 5T Clustal W Sequence Alignment** | |
|---|---|
| 1) | NOV5a (SEQ ID NO:15) |
| 2) | NOV5b (SEQ ID NO:17) |
| 3) | NOV5c (SEQ ID NO:19) |
| 4) | NOV5d (SEQ ID NO:21) |
| 5) | NOV5e (SEQ ID NO:23) |
| 6) | NOV5f (SEQ ID NO:25) |
| 7) | NOV5g (SEQ ID NO:27) |
| 8) | NOV5h (SEQ ID NO:29) |
| 9) | NOV5i (SEQ ID NO:31) |
| 10) | Q99876 homo sapiens (human). apolipoprotein e receptor 2 906 (SEQ ID NO:54) |
| 11) | 014968 homo sapiens (human). apoer2delta4-7 (SEQ ID NO:55) |
| 12) | Q14114 homo sapiens (human). apolipoprotein e receptor 2 precursor (SEQ ID NO:56) |
| 13) | Q98931 gallus gallus (chicken). 1d1 receptor homolog lr8b protein (SEQ ID NO:57) |
| 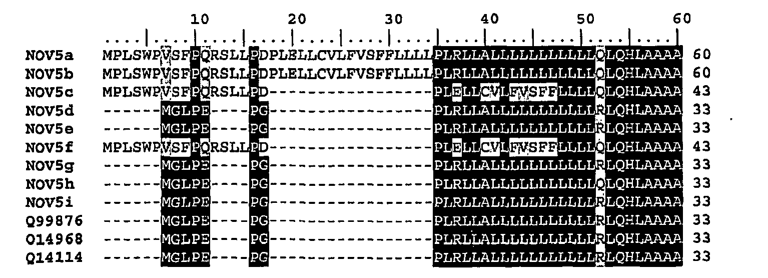 | |
| 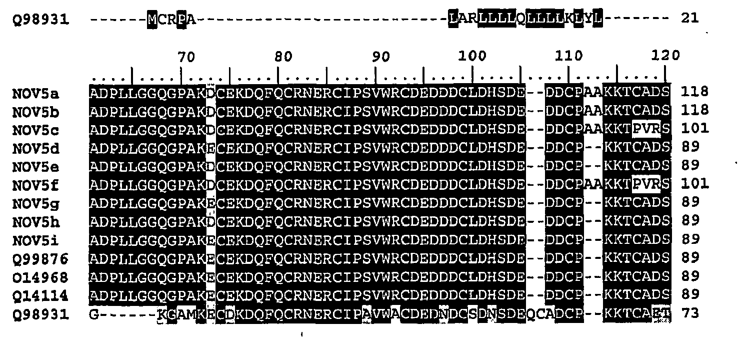 | |
| 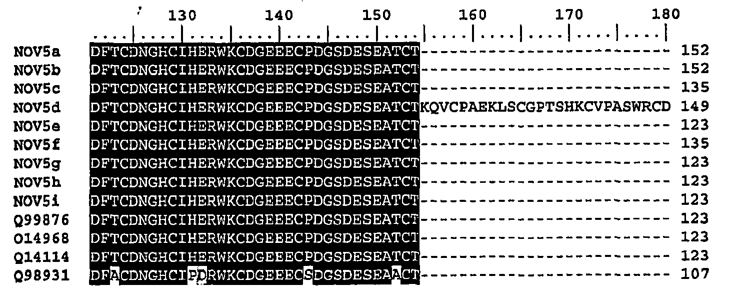 | |
| 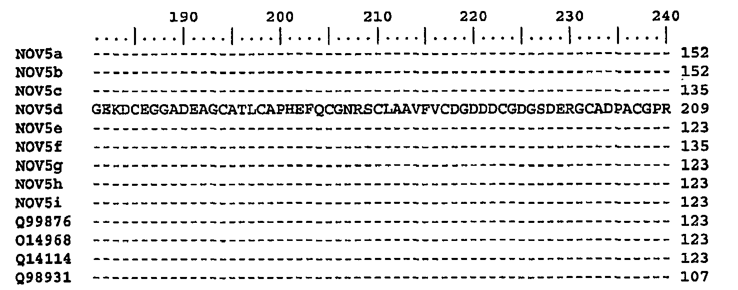 | |
| 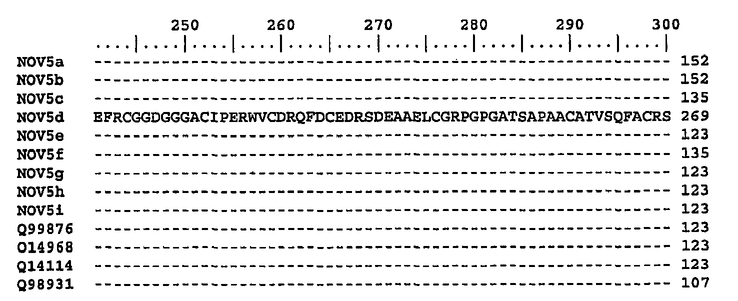 | |
| 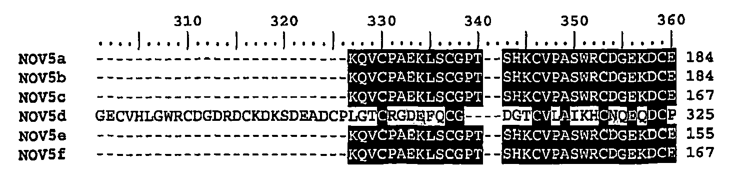 | |
| 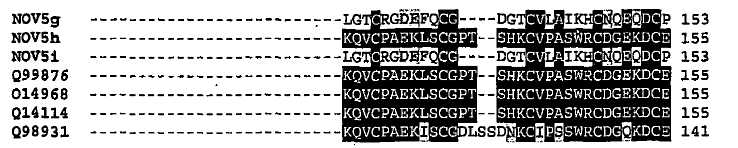 | |
| 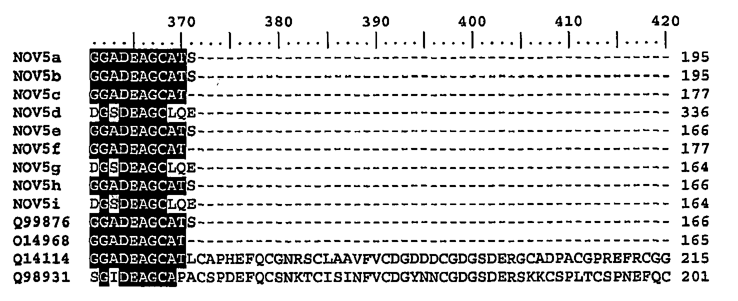 | |
| 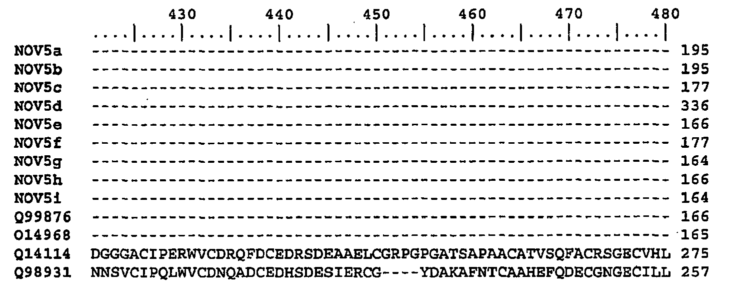 | |
| 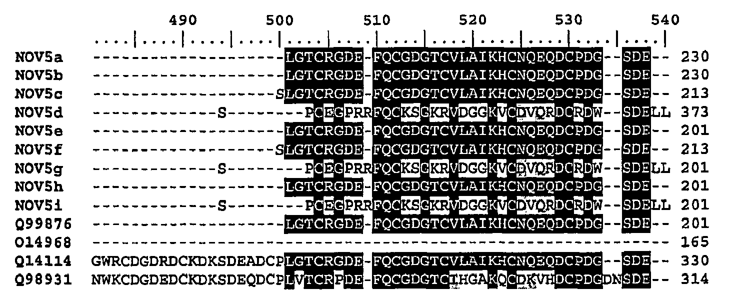 | |
| 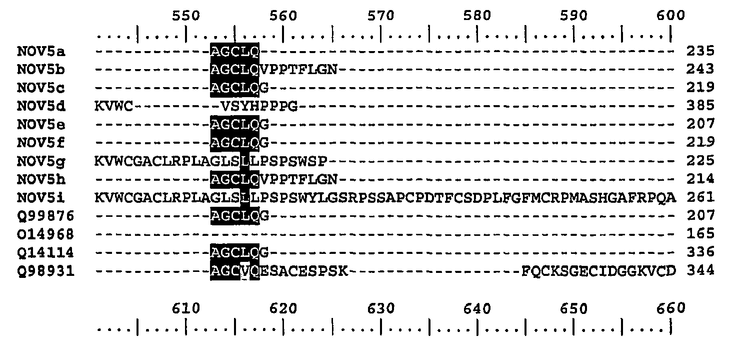 | |
| 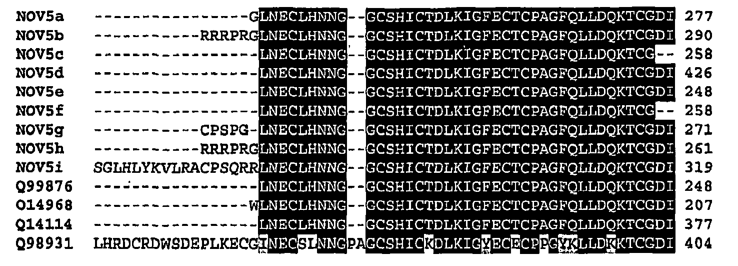 | |
| 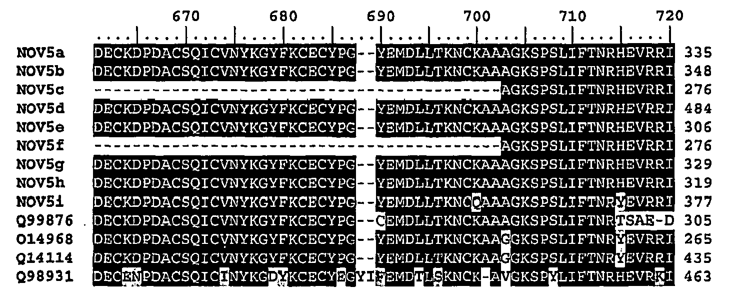 | |
| 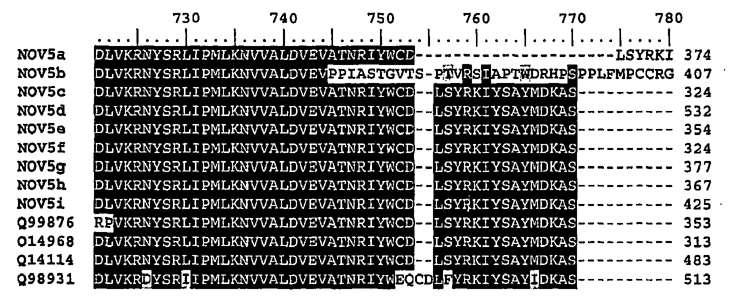 | |
| 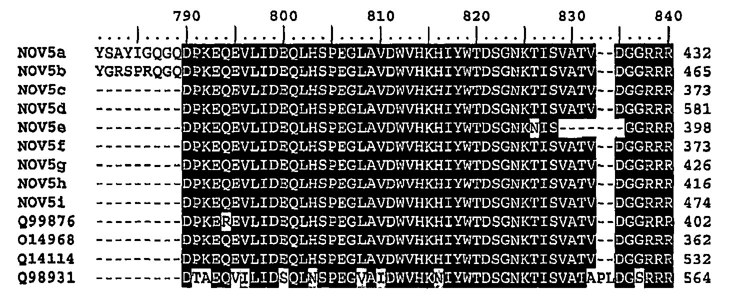 | |
| 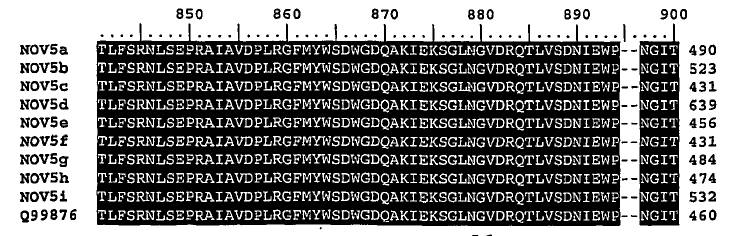 | |
| 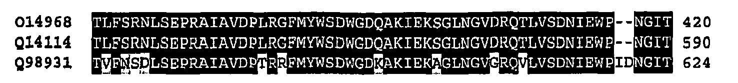 | |
| 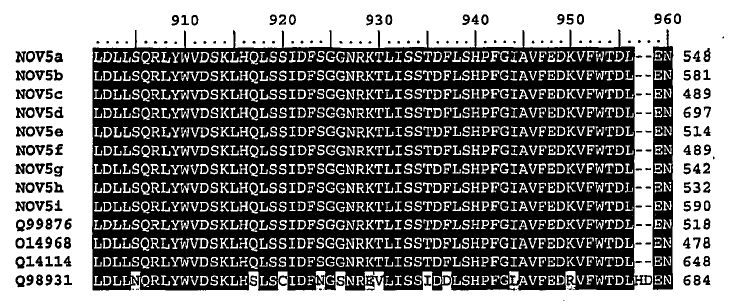 | |
| 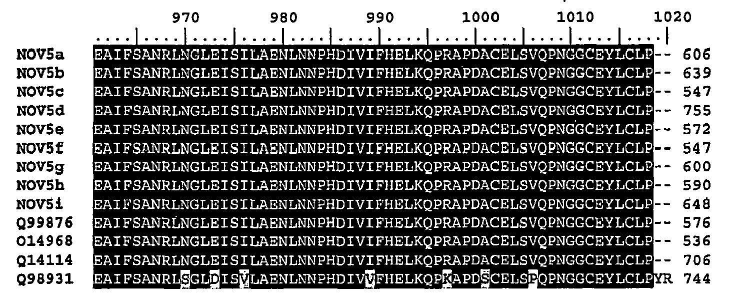 | |
| 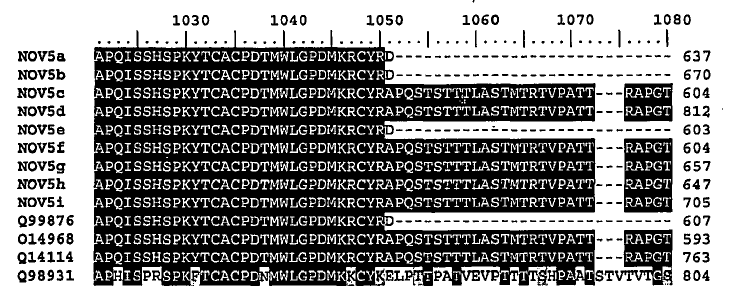 | |
| 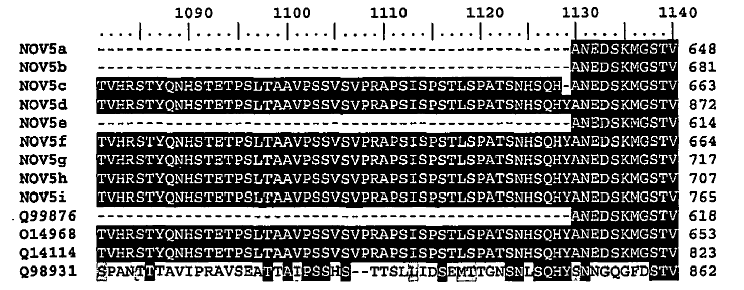 | |
| 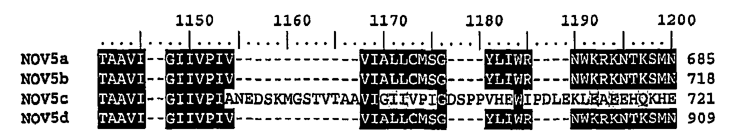 | |
| 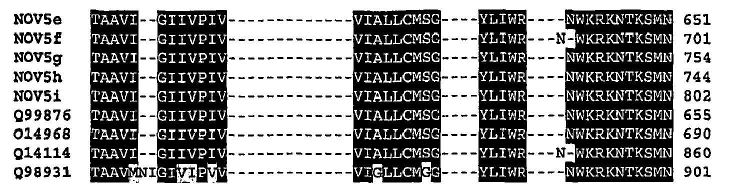 | |
| 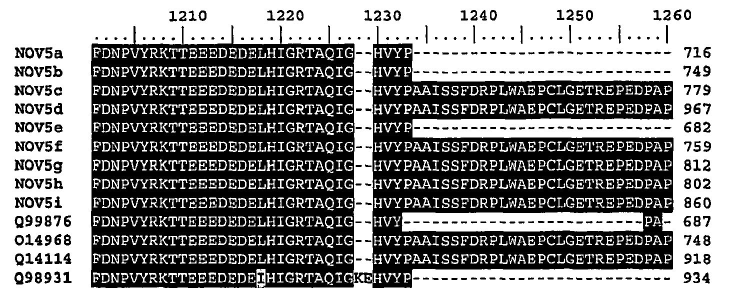 | |
| 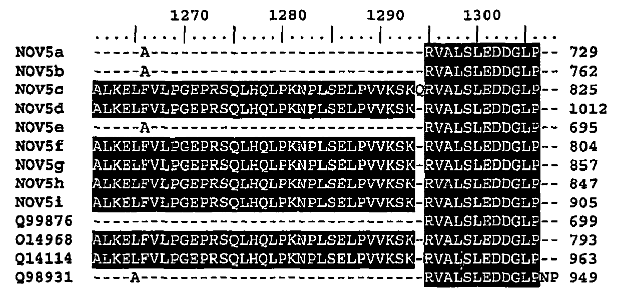 | |

Tables 5U-Y list the domain description from DOMAIN analysis results against NOV5a. This indicates that the NOV5a sequence has properties similar to those of other proteins known to contain this domain.

| **Table 5U. Domain Analysis of NOV5a** |
|---|
| gnl\|Smart\|smart00135, LY, Low-density lipoprotein-receptor YWTD domain; Type "B" repeats in low-density lipoprotein (LDL) receptor that plays a central role in mammalian cholesterol metabolism. Also present in a variety of molecules similar to gp300/megalin (SEQ ID NO:83) |

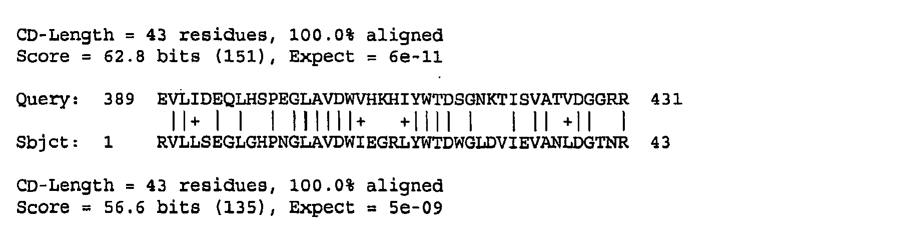

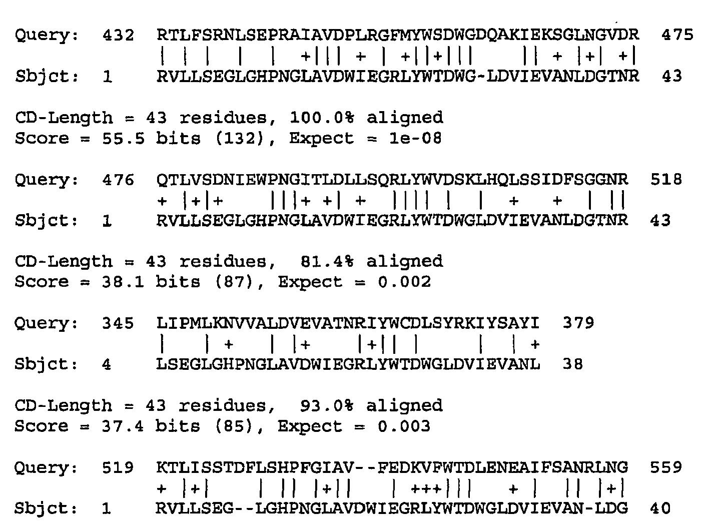

| **Table 5V. Domain Analysis of NOV5** |
|---|
| gnl\|Pfam\|pfam00058, ldl_recept_b, Low-density lipoprotein receptor repeat class B.(SEQ ID NO:84) |

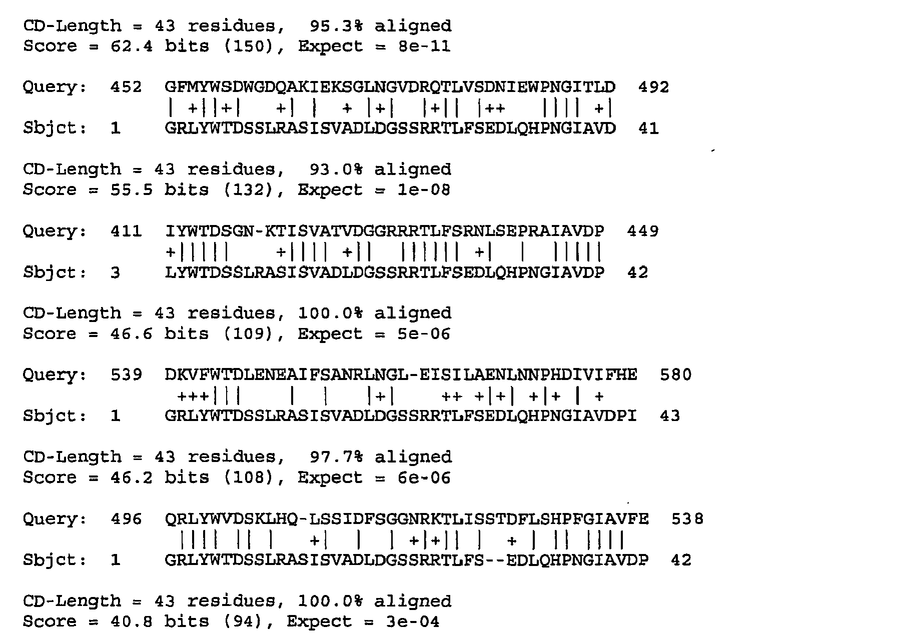

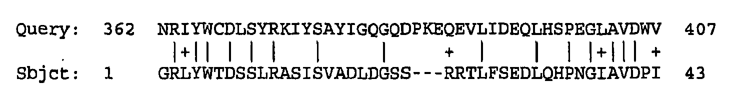

| **Table 5W. Domain Analysis of NOV5** |
|---|
| gnl\|Smart\|smart00192, LDLa, Low-density lipoprotein receptor domain class A; Cysteine-rich repeat in the low-density lipoprotein (LDL) receptor that plays a central role in mammalian cholesterol metabolism. The N-terminal type A repeats in LDL receptor bind the lipoproteins. Other homologous domains occur in related receptors, including the very low-density lipoprotein receptor and the LDL receptor-related protein/alpha 2-macroglobulin receptor, and in proteins which are functionally unrelated, such as the C9 component of complement. Mutations in the LDL receptor gene cause familial hypercholesterolemia.(SEQ ID NO:85) |

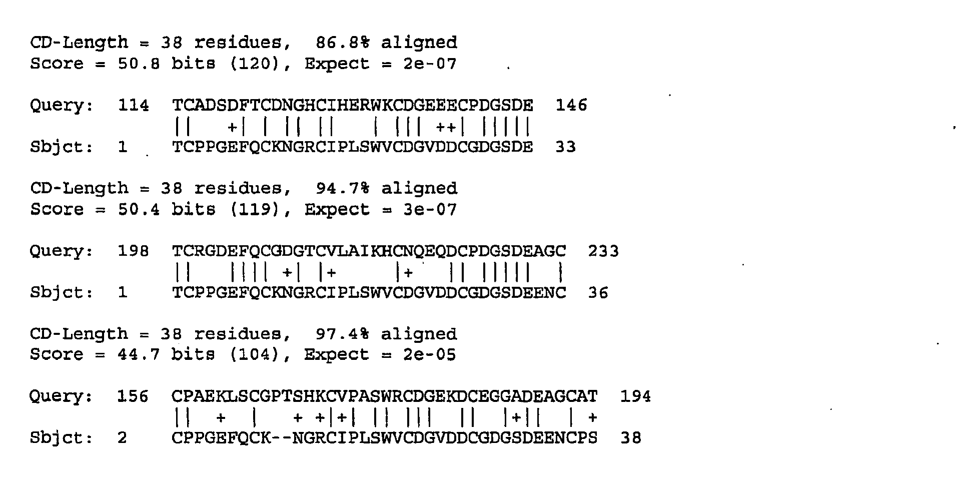

| **Table 5X. Domain Analysis of NOV5** |
|---|
| gnl\|Pfam\|pfam00057, ldl_recept_a, Low-density lipoprotein receptor domain class A (SEQ ID NO:86) |

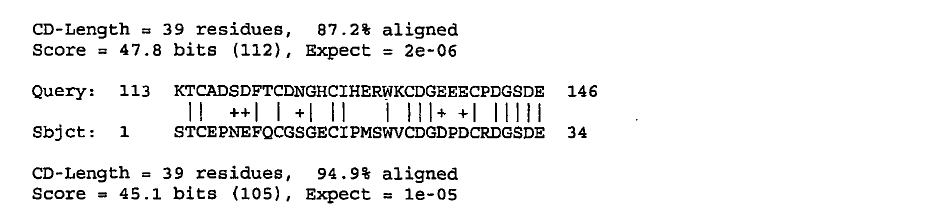

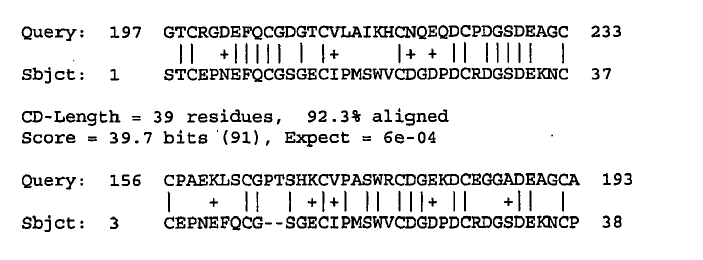

| **Table 5Y. Domain Analysis of NOV5** |
|---|
| gnl\|Smart\|smart00179, EGF_CA, Calcium-binding EGF-like domain (SEQ ID NO:87) |

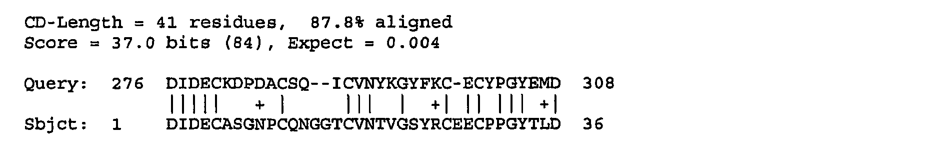

NOV5a also has high homology to members of the patp database shown in Table 5Z.

| **Table 5Z PATP BLASTX results for NOV5a** | | | | | |
|---|---|---|---|---|---|
| Gene Index/ Identifier | Protein/ Organism | Length (aa) | Identity (%) | Positives (%) | Expect |
| patp:AAR78234 | Chicken P95/human LDL receptor chimera - Gallus gallus; Homo sapiens | 924 | 310/572 (54%) | 390/572 (68%) | 3.5e-193 |
| patp:AAR47860 | Human LDL receptor - Homo sapiens | 860 | 310/572 (54%) | 390/572 (68%) | 3.5e-193 |
| patp:AAB90761 | Human shear stress-response protein SEQ ID NO: 22 - Homo sapiens | 860 | 310/572 (54%) | 390/572 (68%) | 3.5e-193 |
| patp:AAR07713 | Human low density lipoprotein receptor - Homo sapiens | 800 | 157/250 (62%) | 196/250 (78%) | 1.1e-184 |
| patp:AAR78234 | Chicken P95/human LDL receptor chimera - Gallus gallus; Homo sapiens | 924 | 157/250 (62%) | 196/250 (78%) | 1.1e-180 |
| patp:AAR47860 | Human LDL receptor - Homo sapiens | 860 | 157/250 (62%) | 196/250 (78%) | 1.1e-180 |

NOV5c also has high homology to members of the patp database shown in Table 5AA.

| **Table 5AA PATP BLASTX results for NOV5c** | | | | | |
|---|---|---|---|---|---|
| Gene Index/ Identifier | Protein/ Organism | Length (aa) | Identity (%) | Positives (%) | Expect |
| patp:AAR78234 | Chicken P95/human LDL receptor chimera - Gallus gallus; Homo sapiens | 924 | 286/573 (49%) | 365/573 (63%) | 8.1e-154 |
| patp:AAW64236 | Canine homologue of mouse mMCP-7 zymogen - Canis familiaris | 269 | 165/268 (61%) | 186/268 (69%) | 2.6e-81 |
| patp:AAB23912 | Porcine virus activation protease SEQ ID NO:2 - Sus scrofa | 277 | 145/250 (58%) | 176/250 (70%) | 1.5e-69 |

Apolipoprotein E (apoE) is a 34-kDa lipophilic protein that circulates in the plasma primarily as a major component of various lipoproteins including chylomicron remnants, intermediate density lipoprotein, very low density lipoprotein (VLDL), β-migrating VLDL (β-VLDL), and high density lipoprotein (with apoE). It is a key molecule responsible for the cellular recognition and internalization of these lipoproteins. Biochemical and genetic studies have demonstrated that apoE is involved in the hepatic clearance of chylomicron remnants and VLDL remnants from the plasma. Apolipoprotein E receptor 2 binds apoE-rich β-VLDL with high affinity and internalizes it into the cells.

Apolipoprotein E receptor 2 is a receptor belonging to a family of low and very low density lipoprotein receptors (LDL-R). This family consists of cell-surface receptors that recognize extracellular ligands and internalize them for degradation by lysosomes. The family members contain four major structural modules: the cysteine-rich complement-type repeats, epidermal growth factor precursor-like repeats, a transmembrane domain, and a cytoplasmic domain. Each structural module serves distinct and important functions. These receptors bind several structurally dissimilar ligands. It is proposed that instead of a primary sequence, positive electrostatic potential in different ligands constitutes a receptor binding domain. This family of receptors plays crucial roles in various physiologic functions. LDL-R plays an important role in cholesterol homeostasis. Mutations cause familial hypercholesterolemia and premature coronary artery disease. LDL-R-related protein plays an important role in the clearance of plasma-activated alpha 2-macroglobulin and apolipoprotein E-enriched lipoproteins. It is essential for fetal development and has been associated with Alzheimer's disease. The regulation of expression of these receptors occurs at the transcriptional level. Expression of the LDL-R is regulated by intracellular sterol levels involving novel membrane-bound transcription factors. All the members are, however, regulated by hormones and growth factors, but the mechanisms of regulation by hormones have not been elucidated.

The NOV5 nucleic acids and proteins of the invention are useful in potential therapeutic applications implicated in atherosclerosis, dysbetalipoproteinemia, hyperlipoproteinemia type III, xanthomatosis, coronary and/or peripheral vascular disease, hypothyroidism, systemic lupus erythematosus, diabetic acidosis, hypercholesterolemia, planar and tendon xanthomas, dysbetalipoproteinemia, hypercholesterolemia, accelerated vascular disease, Alzheimer disease, familial amyloidotic polyneuropathy, as well as other diseases, disorders and conditions. The LDL receptor related protein, the apolipoprotein E receptor 2 and the very low density lipoprotein (VLDL) receptor are preferentially expressed in the brain and they have been suspected to be involved in Alzheimer disease at various levels (Microsc Res Tech 2000 Aug 15;50(4):273-7)., and/or other pathologies and disorders. For example, a cDNA encoding the apolipoprotein e-like protein may be useful in gene therapy, and the apolipoprotein e-like protein may be useful when administered to a subject in need thereof. By way of nonlimiting example, the compositions of the present invention will have efficacy for treatment of patients suffering from atherosclerosis, dysbetalipoproteinemia, hyperlipoproteinemia type III, xanthomatosis, coronary and/or peripheral vascular disease, hypothyroidism, systemic lupus erythematosus, diabetic acidosis, hypercholesterolemia, planar and tendon xanthomas, dysbetalipoproteinemia, hypercholesterolemia, accelerated vascular disease, Alzheimer disease, familial amyloidotic polyneuropathy, as well as other diseases, disorders and conditions. The NOV5 nucleic acid, or fragments thereof, may further be useful in diagnostic applications, wherein the presence or amount of the nucleic acid or the protein are to be assessed.

NOV5 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV5a, e, and f proteins have multiple hydrophilic regions, each of which can be used as an immunogen. In one embodiment, contemplated NOV5a, e, and f epitopes are from about amino acids 80 to 220. In other embodiments, NOV5a, e, and f epitopes are from about amino acids 250 to 340, from about amino acids 370 to 540, from about amino acids 590 to 650, or from about amino acids 680 to 720. This novel protein also has value in development of powerful assay system for functional analysis of various human disorders, which will help in understanding of pathology of the disease and development of new drug targets for various disorders.

The disclosed NOV5b protein has multiple hydrophilic regions, each of which can be used as an immunogen. In one embodiment, a contemplated NOV5b epitope is from about amino acids 80 to 280. In other embodiments, a NOV5b epitope is from about amino acids 290 to 350, from about amino acids 390 to 560, from about amino acids 600 to 670, or from about amino acids 690 to 750. This novel protein also has value in development of powerful assay system for functional analysis of various human disorders, which will help in understanding of pathology of the disease and development of new drug targets for various disorders.

The disclosed NOV5c protein has multiple hydrophilic regions, each of which can be used as an immunogen. In one embodiment, a contemplated NOV5c epitope is from about amino acids 50 to 220. In other embodiments, a NOV5c epitope is from about amino acids 250 to 280, from about amino acids 300 to 480, from about amino acids 550 to 630,from about amino acids 650 to 670, from about amino acids 690 to 760, or from about amino acids 770 to 800. This novel protein also has value in development of powerful assay system for functional analysis of various human disorders, which will help in understanding of pathology of the disease and development of new drug targets for various disorders.

The disclosed NOV5d protein has multiple hydrophilic regions, each of which can be used as an immunogen. In one embodiment, a contemplated NOV5d epitope is from about amino acids 50 to 180. In other embodiments, a NOV5d epitope is from about amino acids 190 to 230, from about amino acids 250 to 390, from about amino acids 420 to 550,from about amino acids 470 to 650, from about amino acids 780 to 850, from amino acids 870 to 900, from about amino acids 920 to 980, or from about amino acids 990 to 1050. This novel protein also has value in development of powerful assay system for functional analysis of various human disorders, which will help in understanding of pathology of the disease and development of new drug targets for various disorders.

The disclosed NOV5g and h proteins have multiple hydrophilic regions, each of which can be used as an immunogen. In one embodiment, contemplated NOV5g and h epitopes are from about amino acids 30 to 220. In other embodiments, NOV5g and h epitopes are from about amino acids 250 to 330, from about amino acids 350 to 540, from about amino acids 550 to 680, from about amino acids 690 to 720, from about amino acids 750 to 780, or from about amino acids 790 to 850. This novel protein also has value in development of powerful assay system for functional analysis of various human disorders, which will help in understanding of pathology of the disease and development of new drug targets for various disorders.

The disclosed NOV5i protein has multiple hydrophilic regions, each of which can be used as an immunogen. In one embodiment, a contemplated NOV5i epitope is from about amino acids 5 to 150. In other embodiments, a NOV5i epitope is from about amino acids 190 to 230, from about amino acids 170 to 320, or from about amino acids 360 to 450. This novel protein also has value in development of powerful assay system for functional analysis of various human disorders, which will help in understanding of pathology of the disease and development of new drug targets for various disorders.

### NOV6

A disclosed NOV6 nucleic acid of 843 nucleotides (also referred to as SC_129296119-A) encoding a novel mastocytoma protease precursor -like protein is shown in Table 6A. An open reading frame was identified beginning with an ATG initiation codon at nucleotides 26-28 and ending with a TAG codon at nucleotides 836-838. A putative untranslated region upstream from the initiation codon and downstream from the termination codon is underlined in Table 6A, and the start and stop codons are in bold letters.

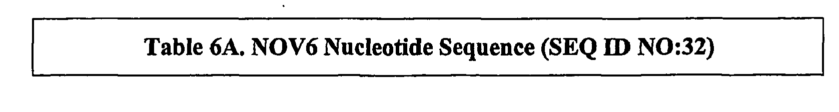

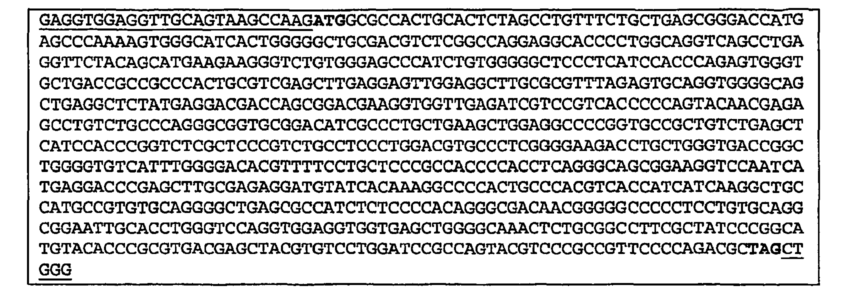

The disclosed NOV6 nucleic acid sequence has 570 of 763 bases (74 %) identical to a *Canis familiaris* (Dog) MASTOCYTOMA PROTEASE PRECURSOR mRNA (GENBANK-ID: DOGMCTRPB) (E =1.1e⁻⁸⁰).

A disclosed NOV6 polypeptide (SEQ ID NO:33) encoded by SEQ ID NO:32 is 270 amino acid residues and is presented using the one-letter amino acid code in Table 6B. Signal P, Psort and/or Hydropathy results predict that NOV6 contains a signal peptide and is likely to be localized in thecytosol with a certainty of 0.4500. The most likely cleavage site for a NOV6 peptide is between amino acids 20 and 21, at: KVG-IT.

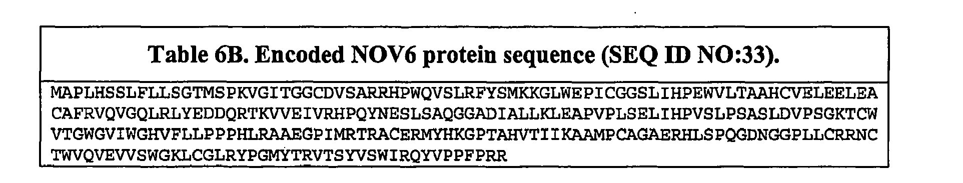

The disclosed NOV6 amino acid sequence has 165 of 268 amino acid residues (61 %) identical to, and 186 of 268 residues (69 %) positive with, the 269 amino acid residue MASTOCYTOMA PROTEASE PRECURSOR protein from *Canis familiaris* (Dog) (ptnr:SPTREMBL-ACC: P19236) (E = 3.0e⁻⁸¹). The global sequence homology (as defined by FASTA alignment with the full length sequence of NOV6) is 66.171 % amino acid homology and 61.338 % amino acid identity. In addition, this protein contains the following protein domains (as defined by Interpro) at the indicated nucleotide positions: *Trypsin domain* (IPR001254) at amino acid positions 21 to 260.

NOV6 also has homology to the amino acid sequences shown in the BLASTP data listed in Table 6F.

| **Table 6F. BLAST results for NOV6** | | | | | |
|---|---|---|---|---|---|
| Gene Index/ Identifier | Protein/ Organism | Length (aa) | Identity (%) | Positives (%) | Expect |
| TRYM_CANFA | canis familiaris (dog). mastocytoma protease precursor (ec 3.4.21.-) | 269 | 165/268 (62%) | 186/268, (69%) | 9e-88 |
| TRYM_RAT | rattus norvegicus (rat). mast cell tryptase precursor (ec 3.4.21.59) | 274 | 131/268 (49%) | 161/268, (60%) | 4e-62 |
| MCT7_RAT | rattus norvegicus (rat). mast cell protease 7 precursor (ec 3.4.21.-) (rmcp-7) (tryptase, skin) | 273 | 129/269 (48%) | 163/269, (61%) | 8e-61 |
| MCT6_MOUSE | mus musculus (mouse). mast cell protease 6 precursor (ec 3.4.21.-) (mmcp-6) (try ptase) | 276 | 125/251 (50%) | 152/251, (61%) | 5e-60 |
| Q9N2D1 | sus scrofa (pig). miniature swine mast cell tryptase precursor | 275 | 120/256 (47%) | 154/256, (60%) | 7e-60 |

The homology of these sequences is shown graphically in the ClustalW analysis shown in Table 6G.

| **Table 6G Information for the ClustalW proteins** |
|---|
| 1) NOV6 (SEQ ID NO:33) |
| 2) TRYM_CANFA canis familiaris (dog). mastocytoma protease precursor (ec 3.4.21.-) (SEQ ID NO:58) |
| 3) TRYM_RAT rattus norvegicus (rat). mast cell tryptase precursor (ec 3.4.21.59) (SEQ ID NO:59) |
| 4) MCT7_RAT rattus norvegicus (rat). mast cell protease 7 precursor (ec 3.4.21.-) (rmcp-7) (tryptase, skin) (SEQ ID NO:60) |
| 5) MCT6_MOUSE mus musculus (mouse). mast cell protease 6 precursor (ec 3.4.21.-) (mmcp-6) (tryptase) (SEQ ID NO:61) |
| 6) Q9N2D1 sus scrofa (pig). miniature swine mast cell tryptase precursor (SEQ ID NO:62) |
| 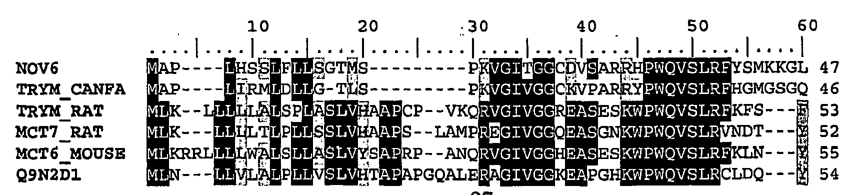 |
| 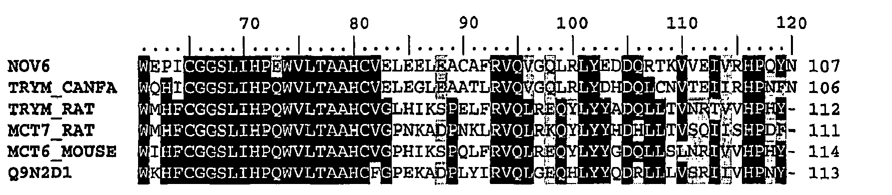 |
| 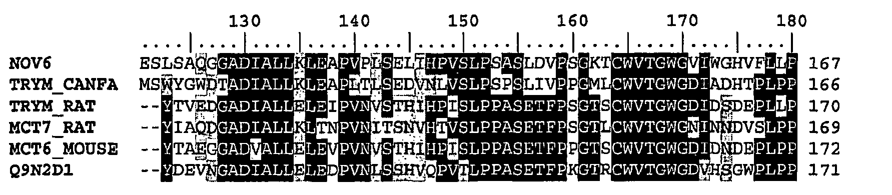 |
| 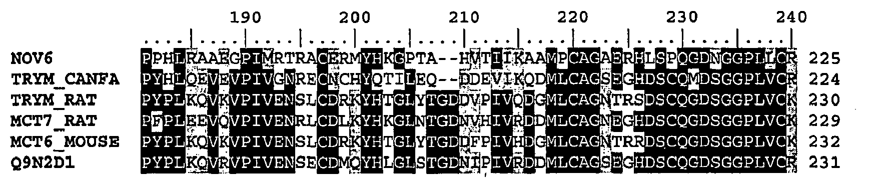 |
| 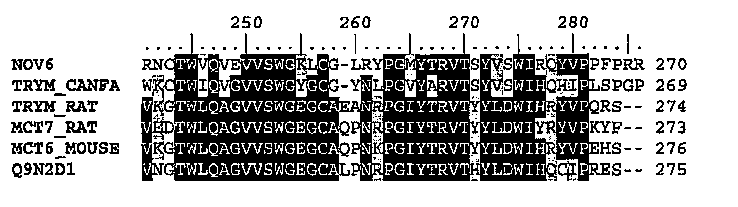 |

Table 6H lists the domain description from DOMAIN analysis results against NOV6. This indicates that the NOV6 sequence has properties similar to those of other proteins known to contain this domain.

| **Table 6H. Domain Analysis of NOV6** |
|---|
| gnl\|Smart\|smart00020, Tryp_SPc, Trypsin-like serine protease; Many of these are synthesised as inactive precursor zymogens that are cleaved during limited proteolysis to generate their active forms. A few, however, are active as single chain molecules, and others are inactive due to substitutions of the catalytic triad residues.. (SEQ ID NO:88) |
| CD-Length = 230 residues, 99.6% aligned |
| Score = 198 bits (503), Expect = 4e-52 |

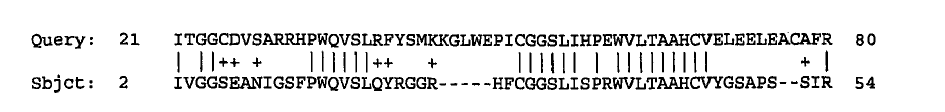

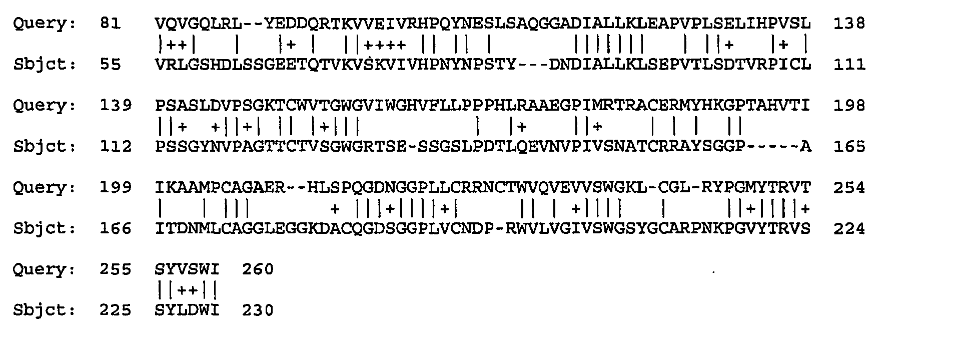

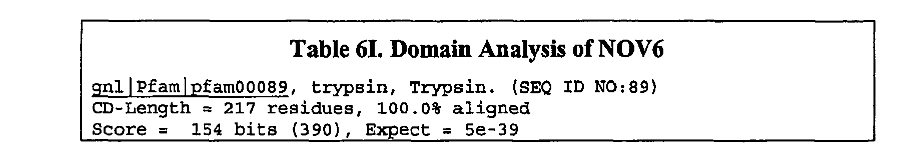

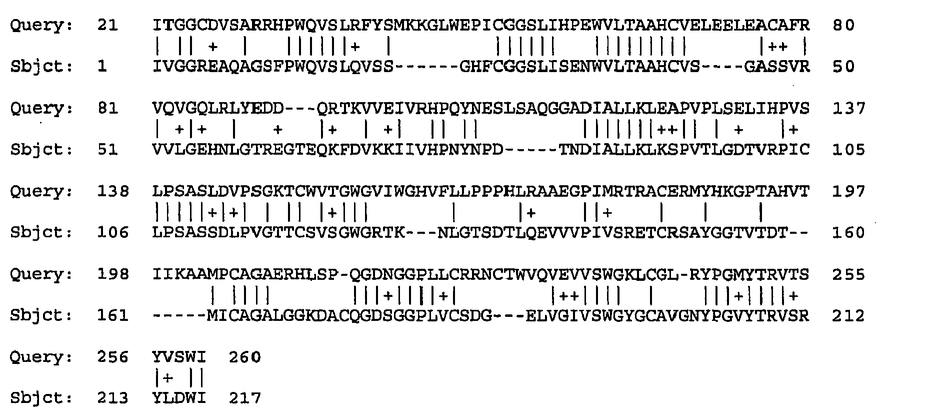

Mast cell tryptase is a secretory granule associated serine protease with trypsin-like specificity released extracellularly during mast cell degranulation. The tryptase sequence includes the essential residues of the catalytic triad and an aspartic acid at the base of the putative substrate binding pocket that confers P1 Arg and Lys specificity on tryptic serine proteases. The apparent N-terminal signal/activation peptide terminates in a glycine. A glycine in this position has not been observed previously in serine proteases and suggests a novel mode of activation.

Mast cell activation in vivo is often associated with areas of oedema and connective-tissue degradation. Tryptase and chymase are the major serine proteinases released by mast cells, but they appear to have little activity on most components of the extracellular matrix. The matrix metalloproteinases (MMP) are purported to degrade almost all connective tissue elements and are secreted by cells in the form of inactive precursors. Since the mechanisms of MMP activation in vivo are poorly understood we have examined the potential of mast cell proteinases to activate the precursor forms of human collagenase (MMP-1), stromelysin (MMP-3), gelatinase A (MMP-2) and gelatinase B (MMP-9). Mast cell proteinases prepared from purified dog mastocytoma cells were shown to process and activate purified precursor forms of both MMP-1 and MMP-3. Using antipain and chymostatin, inhibitors for tryptase and chymase, respectively, it was demonstrated that both pMMP-1 and pMMP-3 were effectively processed and activated by the chymase component. By contrast, tryptase activated only pMMP-3. The mast cell proteinases were unable to process or activate purified precursor forms of MMP-2 and MMP-9. However, MMP-3 previously activated by mast cell proteinases was shown to activate pMMP-9, but not pMMP-2. Since we have no evidence that mast cells express these four metalloenzymes, the release of mast cell serine proteinases following activation/degranulation could contribute to local metalloproteinase activation and subsequent matrix degradation.

The mast cell proteases tryptase and chymase have long been known to constitute one-fifth of the total protein in mast cells. However, their biological functions have not been easy to study because of the difficulty in obtaining sufficient amounts of the enzymes to study their biological functions. Recently, we have been fortunate to have available a permanent line of dog mastocytoma cells to purify both enzymes to homogeneity, and we have used the purified enzymes in two ways. First, in a series of biological studies, we have discovered unique and potent actions of the enzymes that may provide important insights into the pathogenesis of diseases such as asthma and cystic fibrosis. Important biological activities are also likely to exist in other tissues. Because of their structures, mast cell proteases are likely to act in proximity to their sites of release. Thus, the presence and amounts of tryptase and chymase in specific loci may play important roles in tissue responses. In diseases such as asthma and cystic fibrosis, there is evidence that the expression of these mast cell enzymes changes, and these changes have important pathogenetic implications. Second, we have begun to perform structural studies of the enzymes. The recent cloning of tryptase by our group should assist in the better understanding of its functions. Crystallography of the pure proteins should provide further insights and could be the basis of rational development of potent and selective drugs that will inhibit their actions.

The above defined information for this invention suggests that these Voltage-Dependent Anion Channel-like proteins (NOV6) may function as a member of a "Voltage-Dependent Anion Channel family". Therefore, the NOV6 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The nucleic acids and proteins of NOV6 are useful in potential therapeutic applications implicated in immune disorders and airway pathologies such as allergies, asthma and cystic fibrosis, cancers such as mastocytomas, vascular diseases, and/or other pathologies and disorders For example, a cDNA encodingNOV6 may be useful in gene therapy, and NOV6 may be useful when administered to a subj ect in need thereof. By way of nonlimiting example, NOV6 will have efficacy for treatment of patients suffering from immune disorders and airway pathologies such as allergies, asthma and cystic fibrosis, cancers such as mastocytomas, and vascular diseases. The novel NOV6 nucleic acid encoding NOV6 protein,, or fragments thereof, may further be useful in diagnostic applications, wherein the presence or amount of the nucleic acid or the protein are to be assessed. These materials are further useful in the generation of antibodies that bind immunospecifically to the novel substances of the invention for use in therapeutic or diagnostic methods.

NOV6 nucleic acids and polypeptides are further useful in the generation of antibodies that bind immuno-specifically to the novel NOV6 substances for use in therapeutic or diagnostic methods. These antibodies may be generated according to methods known in the art, using prediction from hydrophobicity charts, as described in the "Anti-NOVX Antibodies" section below. This novel protein also has value in development of powerful assay system for functional analysis of various human disorders, which will help in understanding of pathology of the disease and development of new drug targets for various disorders.

### NOV7

A disclosed NOV7 nucleic acid of 239 nucleotides (also referred to GM10d7_A) encoding a novel thymosin beta-4-like receptor protein is shown in Table 7A. An open reading frame was identified beginning with a TCA initiation codon at nucleotides 40-42 and ending with a TGA codon at nucleotides 190-092. In Table 7A, and the start and stop codons are in bold letters.

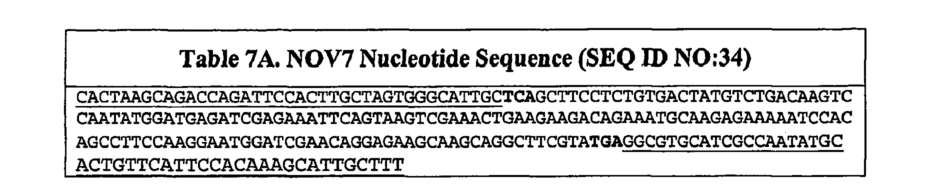

The disclosed NOV7 nucleic acid sequence has 205 of 245 bases (83 %) identical to a *Homo sapiens* THYMOSIN BETA-4 mRNA (GENBANK-ID: M17733) (E = 1.4e⁻³⁰).

A disclosed NOV7 polypeptide (SEQ ID NO:35) encoded by SEQ ID NO:34 is 50 amino acid residues and is presented using the one-letter amino acid code in Table 7B. Signal P, Psort and/or Hydropathy results predict that NOV7 has no signal peptide and is likely to be localized in the cytoplasm with a certainty of 0.4500.

| **Table 7B. Encoded NOV7 protein sequence (SEQ ID NO:35).** |
|---|
| SASSVTMSDKSNMDEIEKFSKSKLKKTEMQEKNPQPSKEWIEQEKQAGFV |

NOV7 maps to chromosome 5p35 and was found to be expressed in at least the following tissues: mammary gland, small intestine, colon, testis and leukocytes.

The disclosed NOV7 amino acid sequence has 26 of 43 amino acid residues (83 %) identical to, and 37 of 43 residues (86 %) positive with, the 50 amino acid residue THYMOSIN BETA-4 protein from *Mus musculus* (ptnr:SPTREMBL-ACC: P20065) (2.2e ¹²). The global sequence homology (as defined by FASTA alignment with the full length sequence of this protein) is 72.000% amino acid homology and 72.000% amino acid identity.

In addition, NOV7 contains the following protein domains (as defined by Interpro) at the indicated nucleotide positions: Thymosin *domain*(IPR001152) at amino acid positions 8 to 48

NOV7 also has homology to the amino acid sequence shown in the BLASTP data listed in Table 7C.

| **Table 7C. BLAST results for NOV7** | | | | | |
|---|---|---|---|---|---|
| Gene Index/ Identifier | Protein/ Organism | Length (aa) | Identity (%) | Positives (%) | Expect |
| TYB4_MOUSE | mus musculus (mouse). thymosin beta-4] | 50 | 36/43 (84%) | 37/43, (86%) | 6e-13 |
| Q9NQQ5 | homo sapiens (human). dj1071110.1 (thymosin/interfe ron-inducible multigene family) | 44 | 36/42 (86%) | 37/42, (88%) | 6e-13 |
| Q95274 | sus scrofa (pig). thymosin beta-4 (fragment) | 46 | 35/43 (81%) | 36/43, (84%) | 2e-12 |
| TYB4_HUMAN | homo sapiens (human), bos taurus (bovine), rattus norvegicus (rat), and equus caballus (horse). thymosin beta-4 (fx) [contains: hematopoietic system regulatory peptide] | 43 | 34/41 (83%) | 35/41, (85%) | 1e-11 |
| TYB4_RABIT | oryctolagus cuniculus (rabbit). thymosin beta-4. | 43 | 33/41 (80%) | 35/41, (85%) | 2e-11 |

The homology of these sequences is shown graphically in the ClustalW analysis shown in Table 7D.

| **Table 7D. Information for the ClustalW proteins** | |
|---|---|
| 1) | NOV7 (SEQ ID NO:35) |
| 2) | TYB4_MOUSE mus musculus (mouse). thymosin beta-4] (SEQ ID NO:63) |
| 3) | Q9NQQ5 homo sapiens (human). dj1071l10.1 (thymosin/interferon-inducible multigene family) (SEQ ID NO:64) |
| 4) | Q95274 sus scrofa (pig). thymosin beta-4 (fragment) (SEQ ID NO:65) |
| 5) | TYB4_HUMAN homo sapiens (human), bos taurus (bovine), rattus norvegicus (rat), and equus caballus (horse). thymosin beta-4 (fx) [contains: hematopoietic system regulatory peptide] (SEQ ID NO:66) |
| 6) | TYB4_RABIT oryctolagus cuniculus (rabbit). thymosin beta-4 (SEQ ID NO:67) |
| 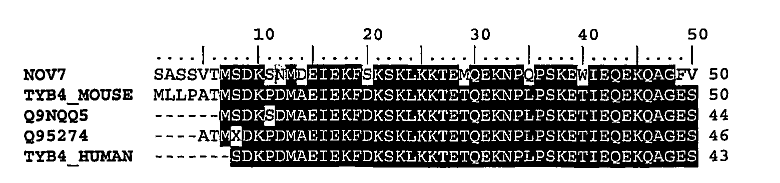 | |
| 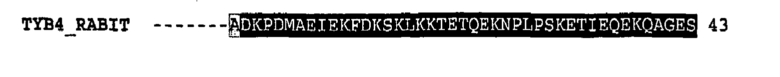 | |

NOV7 also has high homology to members of the patp database shown in Table 7E.

| **Table 7E PATP BLASTX results for NOV7** | | | | | |
|---|---|---|---|---|---|
| Gene Index/ Identifier | Protein/ Organism | Length (aa) | Identity (%) | Positives (%) | Expect |
| patp:AAY76578 | Human ovarian tumor EST fragment encoded protein 74 - Homo sapiens | 86 | 41/57 (71%) | 46/57 (80%) | 6.2e-16 |
| patp:AAY76580 | Human ovarian tumor EST fragment encoded protein 76 - Homo sapiens | 100 | 39/55 (70%) | 40/55 (72%) | 1.0e-13 |
| patp:AAP81169 | Protein produced in myeloma cell differentiation | 68 | 41/57 (71%) | 46/57 (80%) | 6.2e-16 |
| patp:AAY76580 | Human ovarian tumor EST fragment encoded protein 76 Homo sapiens, | 100 | 39/55 (70%) | 40/55 (72%) | 1.0e-13 |
| patp:AAP81169 | Protein produced in myeloma cell differentiation | 68 | 42/60 (70%) | 45/60 (75%) | 1.3e-13 |

Thymosin beta-4 is a small polypeptide whose exact physiological role is not yet known. It was first isolated as a thymic hormone that induces terminal deoxynucleotidyltransferase. It is found in high quantity in thymus and spleen but is widely distributed in many tissues. It has also been shown to bind to actin monomers and thus to inhibit actin polymerization .It has inhibitory activity on the proliferation of hematopoeitic pluripotent stem cells, and is localized in the cytosol of cells. It is induced by alpha interferon, nerve and fibroblast growth factors. It belongs to the thymosin beta family.

Atkinson MJ et al. reported that thymosin beta 4 is expressed in ROS 17/2.8 osteosarcoma cells in a regulated manner. The differential expression of mRNAs between the closely related rat osteosarcoma cell lines ROS 17/2.8 and ROS 25/1 was used to identify genes whose expression is associated with the osteoblast phenotype. Thymosin beta 4 cDNA was cloned from an ROS 17/2.8 complimentary DAN library on the basis of its differential hybridization with radiolabeled cDNA prepared from ROS 17/2.8 and ROS 25/1 cells. Northern blot analysis confirmed that thymosin beta 4, hitherto a putative immunodulatory hormone, was indeed differentially expressed. Steady state mRNA levels were severalfold higher in ROS 17/2.8 cells exhibiting an osteoblast-like phenotype, compared with the less osteoblast-like ROS 25/1. Thymosin beta 4 transcripts were also detected in rat UMR 106 osteosarcoma cells and in intact neonatal and fetal rat calvaria. Sequence analysis of the cDNA indicated that thymosin beta 4 transcripts may arise by processing at a more distal polyadenylation signal. Treatment of ROS 17/2.8 cells with dexamethasone increased, while addition of 1,25-dihydroxyvitamin D3 decreased thymosin beta 4 mRNA. The phenotype-dependent expression in the ROS cells and the response to steroid hormone suggest that thymosin beta 4 expression contributes to the osteoblast phenotype.

Thymosin-beta(4) (Tbeta(4)) binds actin monomers stoichiometrically and maintains the bulk of the actin monomer pool in metazoan cells. Tbeta(4) binding quenches the fluorescence of N-iodoacetyl-N'-(5-sulfo-1-naphthyl)ethylenediamine (AEDANS) conjugated to Cys(374) of actin monomers. The K(d) of the actin-Tbeta(4) complex depends on the cation and nucleotide bound to actin but is not affected by the AEDANS probe. The different stabilities are determined primarily by the rates of dissociation. At 25 degrees C, the free energy of Tbeta(4) binding MgATP-actin is primarily enthalpic in origin but entropic for CaATP-actin. Binding is coupled to the dissociation of bound water molecules, which is greater for CaATP-actin than MgATP-actin monomers. Proteolysis of MgATP-actin, but not CaATP-actin, at Gly(46) on subdomain 2 is >12 times faster when Tbeta(4) is bound. The C terminus of Tbeta(4) contacts actin near this cleavage site, at His(40). By tritium exchange, Tbeta(4) slows the exchange rate of approximately eight rapidly exchanging amide protons on actin. We conclude that Tbeta(4) changes the conformation and structural dynamics ("breathing") of actin monomers. The conformational change may reflect the unique ability of Tbeta(4) to sequester actin monomers and inhibit nucleotide exchange.

The fact that glucocorticoids upregulate the expression of anti-inflammatory mediators is an exciting prospect for therapy in inflammatory diseases, because these molecules could give the therapeutic benefits of steroids without toxic side effects. Supernatants from monocytes and macrophages cultured in the presence of glucocorticoids increase the dispersion ofneutrophils from a cell pellet in the capillary tube migration assay. This supernatant factor, unlike other neutrophil agonists, promotes dispersive locomotion of neutrophils at uniform concentration, lowers their adhesion to endothelial cells, inhibits their chemotactic response to fMLP and induces distinctive morphological changes. Here we show that thymosin beta4 sulfoxide is generated by monocytes in the presence of glucocorticoids and acts as a signal to inhibit an inflammatory response. In vitro, thymosin beta4 sulfoxide inhibited neutrophil chemotaxis, and in vivo, the oxidized peptide, but not the native form, was a potent inhibitor of carrageenin-induced edema in the mouse paw. Thymosin beta4 is unique, because oxidation attenuates its intracellular G-actin sequestering activity, but greatly enhances its extracellular signaling properties. This description of methionine oxidation conferring extracellular function on a cytosolic protein may have far-reaching implications for future strategies of anti-inflammatory therapy.

Angiogenesis is an essential step in the repair process that occurs after injury. In this study, we investigated whether the angiogenic thymic peptide thymosin beta4 (Tbeta4) enhanced wound healing in a rat full thickness wound model. Addition of Tbeta4 topically or intraperitoneally increased reepithelialization by 42% over saline controls at 4 d and by as much as 61 % at 7 d post-wounding. Treated wounds also contracted at least 11% more than controls by day 7. Increased collagen deposition and angiogenesis were observed in the treated wounds. We also found that Tbeta4 stimulated keratinocyte migration in the Boyden chamber assay. After 4-5 h, migration was stimulated 2-3-fold over migration with medium alone when as little as 10 pg of Tbeta4 was added to the assay. These results suggest that Tbeta4 is a potent wound healing factor with multiple activities.

The protein similarity information, expression pattern, and map location for the thymosin beta-4-like protein and nucleic acid (NOV7) disclosed herein suggest that NOV7 may have important structural and/or physiological functions characteristic of the thymosin beta-4-like family. Therefore, the NOV7 nucleic acids and proteins of the invention are useful in potential diagnostic and therapeutic applications. These include serving as a specific or selective nucleic acid or protein diagnostic and/or prognostic marker, wherein the presence or amount of the nucleic acid or the protein are to be assessed, as well as potential therapeutic applications such as the following: (i) a protein therapeutic, (ii) a small molecule drug target, (iii) an antibody target (therapeutic, diagnostic, drug targeting/cytotoxic antibody), (iv) a nucleic acid useful in gene therapy (gene delivery/gene ablation), and (v) a composition promoting tissue regeneration in vitro and in vivo.

The NOV7 nucleic acids and proteins of the invention are useful in potential diagnostic and therapeutic applications implicated in various diseases and disorders described below and/or other pathologies. For example, the compositions of the present invention will have efficacy for treatment of patients suffering from prostate cancer, immunological and autoimmune disorders (ie hyperthyroidism), angiogenesis and wound healing, modulation of apoptosis, neurodegenerative and neuropsychiatric disorders, age-related disorders, pathological disorders involving spleen, thymus, lung, and peritoneal macrophages and/or other pathologies and disorders. The NOV7 nucleic acid, or fragments thereof, may further be useful in diagnostic applications, wherein the presence or amount of the nucleic acid or the protein are to be assessed.

NOV7 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV7 protein has multiple hydrophilic regions, each of which can be used as an immunogen. This novel protein also has value in development of powerful assay system for functional analysis of various human disorders, which will help in understanding of pathology of the disease and development of new drug targets for various disorders.

### NOV8

A disclosed NOV8 nucleic acid of 1348 nucleotides (also referred to as GMba550p23_A) encoding a novel Protein-tyrosine-phosphatase -like protein is shown in Table 8A. An open reading frame was identified beginning with a ATG initiation codon at nucleotides 57-59 and ending with a TAA codon at nucleotides 1302-1304. A putative untranslated region upstream from the initiation codon and downstream from the termination codon is underlined in Table 8A, and the start and stop codons are in bold letters.

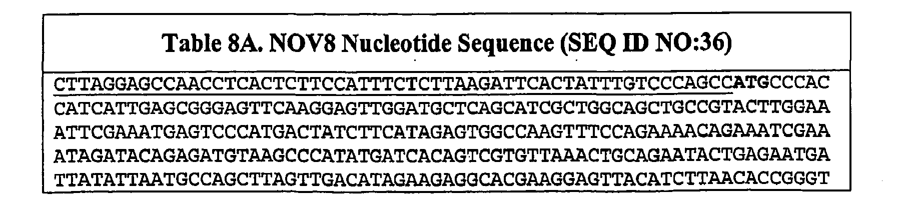

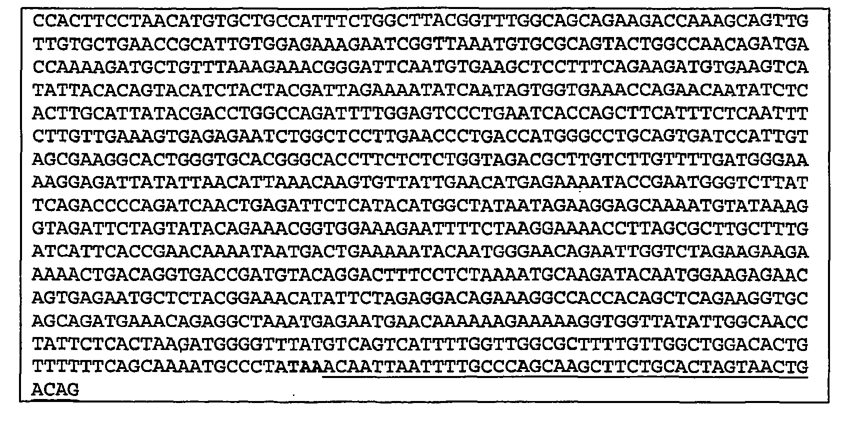

The disclosed NOV 8 nucleic acid sequence maps to chromosome 13 and has 1237 of 1299 bases (95%) identical to *a Homo sapiens* Protein-tyrosine-phosphatase mRNA (GENBANK-ID: HUMPTPASE) (E = 6.8e⁻²⁶²).

A disclosed NOV8 protein (SEQ ID NO:37) encoded by SEQ ID NO:36 has 415 amino acid residues, and is presented using the one-letter code in Table 8B. Signal P, Psort and/or Hydropathy results predict that NOV8 is likely to be localized to the endoplasmic reticulum (membrane) with a certainty of 0.8500.

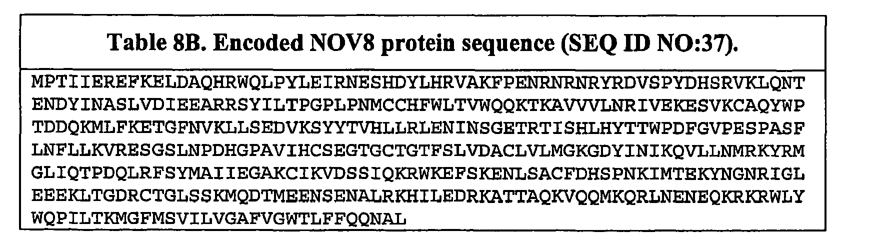

The disclosed NOV8 amino acid has 381 of 415 amino acid residues (91 %) identical to, and 391 of 415 residues (94 %) positive with, the 415 amino acid residue protein-tyrosine-phosphatase, nonreceptor type 2 protein from *Homo sapiens* (ptnr:SPTREMBL-ACC: A33899) (E= 4.9e⁻²⁰⁷).

NOV8 also has homology to the amino acid sequences shown in the BLASTP data listed in Table 8C.

| **Table 8C. BLAST results for NOV8** | | | | | |
|---|---|---|---|---|---|
| Gene Index/ Identifier | Protein/ Organism | Length (aa) | Identity (%) | Positives (%) | Expect |
| PTN2_HUMAN | homo sapiens (human). t-cell protein-tyrosine phosphatase (ec 3.1.3.48) (tcptp). | 415 | 380/415 (92%) | 390/415, (94%) | 0.0 |
| PTN2_MOUSE | mus musculus (mouse). protein- tyrosine phosphatase ptp-2 (ec 3.1.3.48) (mptp). | 382 | 314/381 (82%) | 339/381, (89%) | 0.0 |
| PTN2_RAT | rattus norvegicus (rat). protein- tyrosine phosphatase ptp-s (ec 3.1.3.48). | 363 | 301/381 (79%) | 326/381, (86%) | 1e-172 |
| PTN1_CHICK | gallus gallus (chicken). protein-tyrosine phosphatase 1b (ec 3.1.3.48) (ptp-1b) (cptp1). 12/1998 and (Pa | 434 | 212/435 (49%) | 286/435, (66%) | le-116 |
| PTN1_MOUSE | mus musculus (mouse). protein- tyrosine phosphatase 1b (ec 3.1.3.48) (ptp-1b) (pr otein- protein-tyrosine phosphatase ha2) (ptp-ha2) | 432 | 211/429 (49%) | 285/429, (66%) | 1e-115 |

The homology of these sequences is shown graphically in the ClustalW analysis shown in Table 8D.

| **Table 8D. Information for the ClustalW proteins** |
|---|
| 1) NOV8 (SEQ ID NO:37) |
| 2) PTN2_HUMAN homo sapiens (human). t-cell protein-tyrosine phosphatase (ec 3.1.3.48) (tcptp) (SEQ ID NO:68) |
| 3) PTN2_MOUSE mus musculus (mouse). protein-tyrosine phosphatase ptp-2 (ec 3.1.3.48) (mptp) (SEQ ID NO:69) |
| 4) PTN2_RAT rattus norvegicus (rat). protein-tyrosine phosphatase ptp-s (ec 3.1.3.48) (SEQ ID NO:70) |
| 5) PTN1_CHICK gallus gallus (chicken). protein-tyrosine phosphatase 1b (ec 3.1.3.48) (ptp-1b) (cptp1). (Pa (SEQ ID NO:71) |
| 6) PTN1_MOUSE mus musculus (mouse). protein-tyrosine phosphatase 1b (ec 3.1.3.48) (ptp-1b) (protein- protein-tyrosine phosphatase ha2) (ptp-ha2) (SEQ ID NO:72) |
| 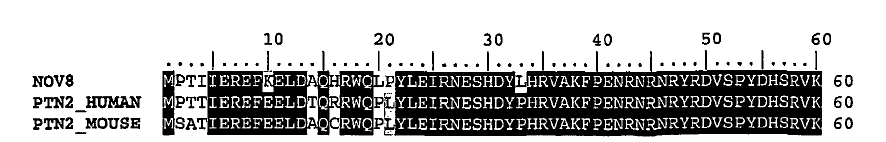 |
| 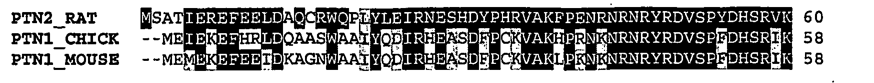 |
| 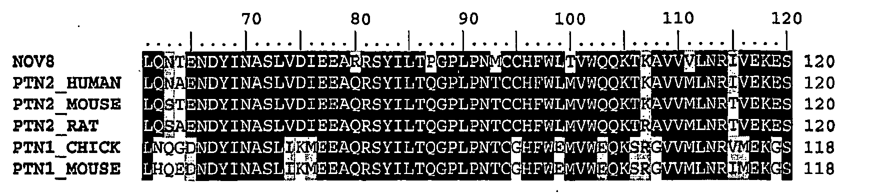 |
| 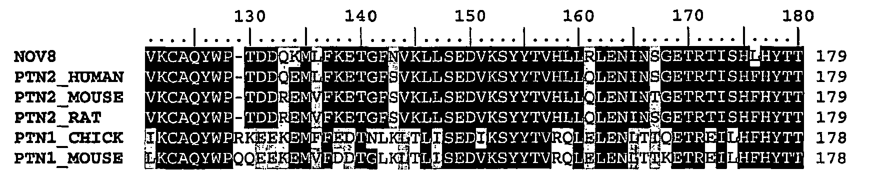 |
| 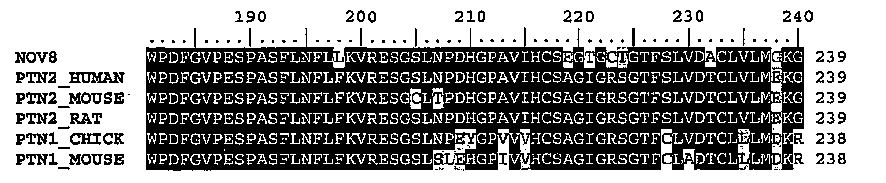 |
| 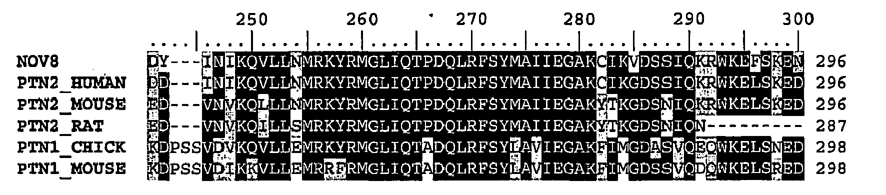 |
| 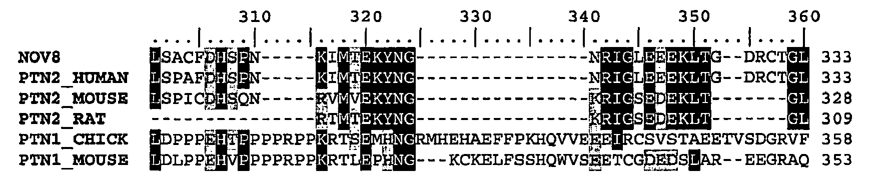 |
| 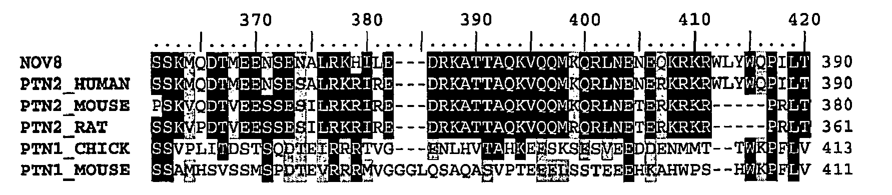 |
| 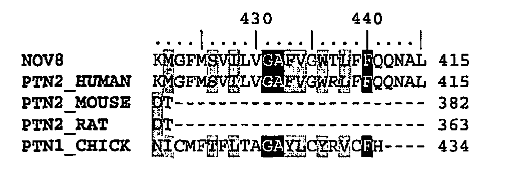 |
| 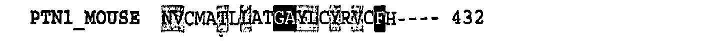 |

Tables 8E-8G list the domain description from DOMAIN analysis results against NOV8. This indicates that the NOV8 sequence has properties similar to those of other proteins known to contain this domain.

| **Table 8E. Domain Analysis of NOV8** |
|---|
| gnl\|Smart\|smart00194, PTPc, Protein tyrosine phosphatase, catalytic domain (SEQ ID NO:90) |
| CD-Length = 264 residues, 98.5% aligned |
| Score = 263 bits (672), Expect = le-71 |

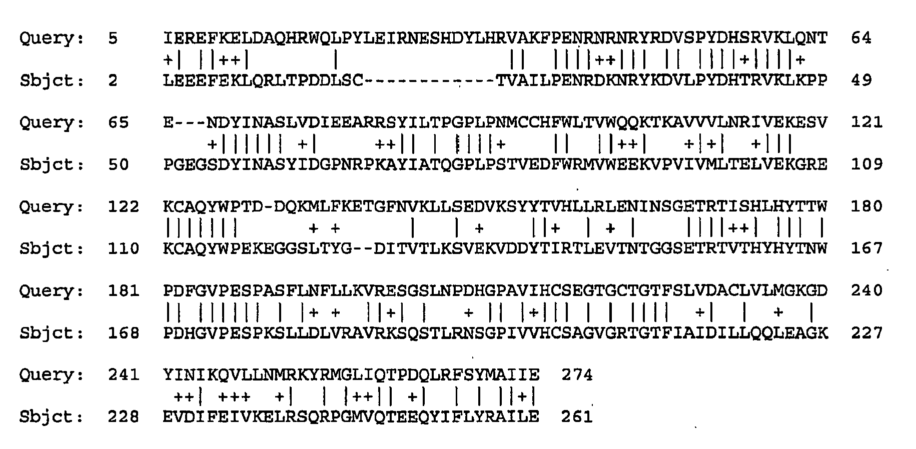

| **Table 8F. Domain Analysis of NOV8** |
|---|
| gnl\|Pfam\|pfam00102, Y_phosphatase, Protein-tyrosine phosphatase. (SEQ ID NO:91) |
| CD-Length = 235 residues, 100.0% aligned |
| Score = 235 bits (599), Expect = 4e-63 |

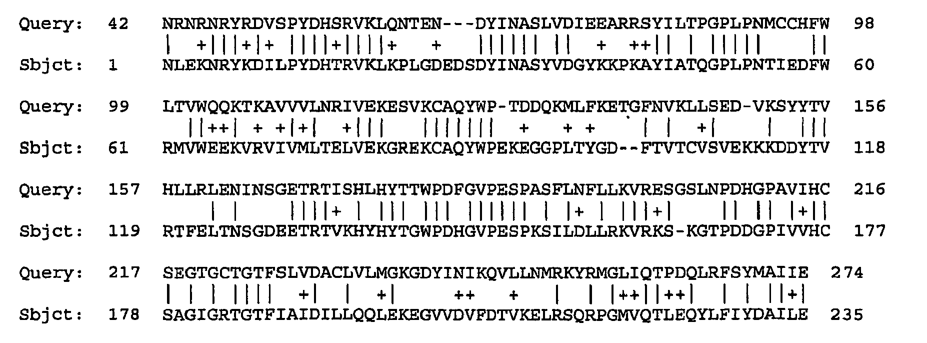

| **Table 8G. Domain Analysis of NOV8** |
|---|
| gn1\|Smart\|smart00404, PTPc_motif, Protein tyrosine phosphatase, catalytic domain motif SEQ ID NO:92) |
| CD-Length = 105 residues, 100.0% aligned |
| Score = 108 bits (269), Expect = 8e-25 |

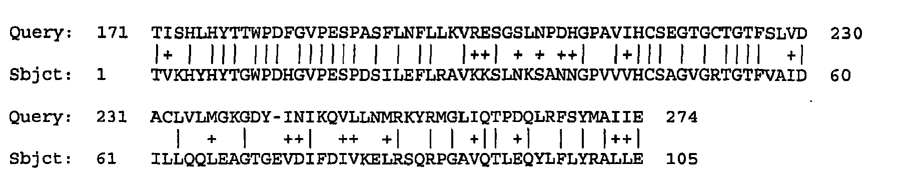

NOV8 also has high homology to members of the patp database shown in Table 8H.

| **Table 8H PATP BLASTX results for NOV8** | | | | | |
|---|---|---|---|---|---|
| Gene Index/ Identifier | Protein/ Organism | Length (aa) | Identity (%) | Positives (%) | Expect |
| patp:AAR14114 | Non-receptor linked protein tyrosine phosphatase - Homo sapiens | 415 | 381/415 (91%) | 391/415 (94%) | 4.2e-207 |
| patp:AAB59365 | Human protein tyrosine phosphatase #2 - Homo sapiens | 251 | 231/254 (90%) | 237/254 (93%) | 1.4e-121 |

Many transmembrane receptors for polypeptide growth factors and oncogenes catalyze the phosphorylation of proteins on tyrosine residues, suggesting a regulatory role in cell growth and possibly differentiation. Whereas many tyrosine kinases had been characterized, relatively little was known about enzymes that remove phosphate specifically from tyrosine residues. Both kinases and phosphatases are involved in the phosphorylation of proteins on tyrosine. The importance of tyrosine dephosphorylation in regulating enzymatic activity is demonstrated, for example, for the SRC oncogene (190090), where dephosphorylation at tyrosine 527 in the carboxy terminus is associated with increased activity. The protein-tyrosine phosphatases for which genes have been cloned and mapped include placental PTPase 1B (PTP1B; 176885), CD45 (151460), and LAR (179590). T-cell PTPase was cloned by Cool et al. (1989) who showed that it shares 72% amino acid sequence identity with PTP1B in a 236-amino acid core region present in all PTPases. Sakaguchi et al. (1991, 1992) used a human PTPT cDNA to assign the gene to human chromosome 18 and mouse chromosome 18 by Southern analysis of human/mouse and mouse/Chinese hamster somatic cell hybrids, respectively. Johnson et al. (1993) mapped the PTPN2 gene to 18p11.3-p11.2 by fluorescence in situ hybridization. They identified pseudogenes on 1q22-q24 and 13q12-q13. Direct comparison of the specificity of genomic and cDNA probes demonstrated that under identical conditions the genomic probes (containing both exon and intron sequences) readily identified a single specific site of hybridization, whereas the cDNA identified sites of both the gene and its pseudogenes. They suggested that this is a strategy for addressing the recurrent problem in gene mapping studies where highly related sequences exist within the genome.

Protein tyrosine phosphatases (PTPs) are a family of enzymes that modulate the cellular level of tyrosine phosphorylation. Based on cellular location, they are classified as receptor like or intracellular PTPs. Structure and function studies have led to the understanding of the enzymatic mechanism of this class of enzymes. Proper targeting of PTPs is essential for many cellular signalling events including antigen induced proliferative responses of B and T cells. The physiological significance of PTPs is further unveiled through mice gene knockout studies and human genome sequencing and mapping projects. Several PTPs are shown to be critical in the pathogenesis of human diseases. PMID: 10723800, UI: 20188875

During neuronal development, cells respond to a variety of environmental cues through cell surface receptors that are coupled to a signaling transduction machinery based on protein tyrosine phosphorylation and dephosphorylation. Receptor and non-receptor tyrosine kinases have received a great deal of attention; however, in the last few years, receptor (plasma membrane associated) and non-receptor protein-tyrosine phosphatases (PTPs) have also been shown to play important roles in development of the nervous system. In many cases PTPs have provocative distribution patterns or have been shown to be associated with specific cell adhesion and growth factor receptors. Additionally, altering PTP expression levels or activity impairs neuronal behavior. In this review we outline what is currently known about the role of PTPs in development, differentiation and neuronal physiology.

The protein similarity information, expression pattern, and map location for the protein-tyrosine-phosphatase-like protein and nucleic acid (NOV8) disclosed herein suggest that NOV8 protein may have important structural and/or physiological functions characteristic of the protein-tyrosine-phosphatase F family. Therefore, the NOV8 nucleic acids and proteins of the invention are useful in potential diagnostic and therapeutic applications. These include serving as a specific or selective nucleic acid or protein diagnostic and/or prognostic marker, wherein the presence or amount of the nucleic acid or the protein are to be assessed, as well as potential therapeutic applications such as the following: (i) a protein therapeutic, (ii) a small molecule drug target, (iii) an antibody target (therapeutic, diagnostic, drug targeting/cytotoxic antibody), (iv) a nucleic acid useful in gene therapy (gene delivery/gene ablation), and (v) a composition promoting tissue regeneration in vitro and in vivo.

The NOV8 nucleic acids and proteins of the invention are useful in potential diagnostic and therapeutic applications implicated in various diseases and disorders described below and/or other pathologies. For example, the compositions of the present invention will have efficacy for treatment of patients suffering from hematopoiesic disorders; leukemogenesis; polycystic kidney disease; Oncogenesis such as lung, liver and ovarian cancers; apoptosis; type 2 diabetes and obesity; cell growth and possibly differentiation; non-Hodgkin lymphomas; musculoskeletal disorders; and CNS development disorders. NOV8 nucleic acid, or fragments thereof, may further be useful in diagnostic applications, wherein the presence or amount of the nucleic acid or the protein are to be assessed.

NOV8 nucleic acids and polypeptides are further useful in the generation of antibodies that bind immunospecifically to the novel substances of the invention for use in therapeutic or diagnostic methods. These antibodies may be generated according to methods known in the art, using prediction from hydrophobicity charts, as described in the "Anti-NOVX Antibodies" section below. For example the disclosed NOV8 protein has multiple hydrophilic regions, each of which can be used as an immunogen. This novel protein also has value in development of powerful assay system for functional analysis of various human disorders, which will help in understanding of pathology of the disease and development of new drug targets for various disorders.

### NOVX Nucleic Acids and Polypeptides

One aspect of the invention pertains to isolated nucleic acid molecules that encode NOVX polypeptides or biologically active portions thereof. Also included in the invention are nucleic acid fragments sufficient for use as hybridization probes to identify NOVX-encoding nucleic acids (*e.g.*, NOVX mRNAs) and fragments for use as PCR primers for the amplification and/or mutation of NOVX nucleic acid molecules. As used herein, the term "nucleic acid molecule" is intended to include DNA molecules (*e.g.*, cDNA or genomic DNA), RNA molecules (*e.g.*, mRNA), analogs of the DNA or RNA generated using nucleotide analogs, and derivatives, fragments and homologs thereof. The nucleic acid molecule may be single-stranded or double-stranded, but preferably is comprised double-stranded DNA.

An NOVX nucleic acid can encode a mature NOVX polypeptide. As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full-length gene product, encoded by the corresponding gene. Alternatively, it may be defined as the polypeptide, precursor or proprotein encoded by an ORF described herein. The product "mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an ORF, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

The term "probes", as utilized herein, refers to nucleic acid sequences of variable length, preferably between at least about 10 nucleotides (nt), 100 nt, or as many as approximately, *e.g.*, 6,000 nt, depending upon the specific use. Probes are used in the detection of identical, similar, or complementary nucleic acid sequences. Longer length probes are generally obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies.

The term "isolated" nucleic acid molecule, as utilized herein, is one, which is separated from other nucleic acid molecules which are present in the natural source of the nucleic acid. Preferably, an "isolated" nucleic acid is free of sequences which naturally flank the nucleic acid (*i.e.,* sequences located at the 5'- and 3'-termini of the nucleic acid) in the genomic DNA of the organism from which the nucleic acid is derived. For example, in various embodiments, the isolated NOVX nucleic acid molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e.g.*, brain, heart, liver, spleen, etc.). Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the invention; *e.g.,* a nucleic acid molecule having the nucleotide sequence SEQ ID NOS:1, 3, 5, 7, 9, 12, 14, 16, 18, 20, 22, 24, 26, 28, 30, 32, 34, and 36, or a complement of this aforementioned nucleotide sequence, can be isolated using standard molecular biology techniques and the sequence information provided herein. Using all or a portion of the nucleic acid sequence of SEQ ID NOS:1, 3, 5, 7, 9, 12, 14, 16, 18, 20, 22, 24, 26, 28, 30, 32, 34, and 36 as a hybridization probe, NOVX molecules can be isolated using standard hybridization and cloning techniques (*e.g.,* as described in Sambrook, et al., (eds.), MOLECULAR CLONING: A LABORATORY MANUAL 2nd Ed., Cold Spring Harbor Laboratory Press, Cold Spring Harbor, NY, 1989; and Ausubel, et al., (eds.), CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, *e.g.,* using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides SEQ ID NOS:1, 3, 5, 7, 9, 12, 14, 16, 18, 20, 22, 24, 26, 28, 30, 32, 34, and 36, or a complement thereof. Oligonucleotides may be chemically synthesized and may also be used as probes.

In another embodiment, an isolated nucleic acid molecule of the invention comprises a nucleic acid molecule that is a complement of the nucleotide sequence shown in SEQ ID NOS:1, 3, 5, 7, 9, 12, 14, 16, 18, 20, 22, 24, 26, 28, 30, 32, 34, and 36, or a portion of this nucleotide sequence (*e.g.*, a fragment that can be used as a probe or primer or a fragment encoding a biologically-active portion of an NOVX polypeptide). A nucleic acid molecule that is complementary to the nucleotide sequence shown SEQ ID NOS:1, 3, 5, 7, 9, 11, 13, 15, 17 or 19 is one that is sufficiently complementary to the nucleotide sequence shown SEQ ID NOS:1, 3, 5, 7, 9, 11, 13, 15, 17 or 19 that it can hydrogen bond with little or no mismatches to the nucleotide sequence shown SEQ ID NOS:1, 3, 5, 7, 9, 12, 14, 16, 18, 20, 22, 24, 26, 28, 30, 32, 34, and 36, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. *See e.g.* Ausubel, et al., CURRENT PROTOCOLS IN MOLECULAR BIOLOGY, John Wiley & Sons, New York, NY, 1993, and below.

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of NOVX polypeptides. Isoforms can be expressed in different tissues of the same organism as a result of, for example, alternative splicing of RNA. Alternatively, isoforms can be encoded by different genes. In the invention, homologous nucleotide sequences include nucleotide sequences encoding for an NOVX polypeptide of species other than humans, including, but not limited to: vertebrates, and thus can include, *e.g.,* frog, mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human NOVX protein. Homologous nucleic acid sequences include those nucleic acid sequences that encode conservative amino acid substitutions (see below) in SEQ ID NOS:1, 3, 5, 7, 9, 12, 14, 16, 18, 20, 22, 24, 26, 28, 30, 32, 34, and 36, as well as a polypeptide possessing NOVX biological activity. Various biological activities of the NOVX proteins are described below.

An NOVX polypeptide is encoded by the open reading frame ("ORF") of an NOVX nucleic acid. An ORF corresponds to a nucleotide sequence that could potentially be translated into a polypeptide. A stretch of nucleic acids comprising an ORF is uninterrupted by a stop codon. An ORF that represents the coding sequence for a full protein begins with an ATG "start" codon and terminates with one of the three "stop" codons, namely, TAA, TAG, or TGA. For the purposes of this invention, an ORF may be any part of a coding sequence, with or without a start codon, a stop codon, or both. For an ORF to be considered as a good candidate for coding for a *bona fide* cellular protein, a minimum size requirement is often set, *e*.*g*., a stretch of DNA that would encode a protein of 50 amino acids or more.

The nucleotide sequences determined from the cloning of the human NOVX genes allows for the generation of probes and primers designed for use in identifying and/or cloning NOVX homologues in other cell types, *e.g.* from other tissues, as well as NOVX homologues from other vertebrates. The probe/primer typically comprises substantially purified oligonucleotide. The oligonucleotide typically comprises a region of nucleotide sequence that hybridizes under stringent conditions to at least about 12, 25, 50, 100, 150, 200, 250, 300, 350 or 400 consecutive sense strand nucleotide sequence SEQ ID NOS:1, 3, 5, 7, 9, 11, 13, 15, 17 or 19; or an anti-sense strand nucleotide sequence of SEQ ID NOS:1, 3, 5, 7, 9, 11, 13, 15, 17 or 19; or of a naturally occurring mutant of SEQ ID NOS:1, 3, 5, 7, 9, 12, 14, 16, 18, 20, 22, 24, 26, 28, 30, 32, 34, and 36.

Probes based on the human NOVX nucleotide sequences can be used to detect transcripts or genomic sequences encoding the same or homologous proteins. In various embodiments, the probe further comprises a label group attached thereto, *e.g.* the label group can be a radioisotope, a fluorescent compound, an enzyme, or an enzyme co-factor. Such probes can be used as a part of a diagnostic test kit for identifying cells or tissues which mis-express an NOVX protein, such as by measuring a level of an NOVX-encoding nucleic acid in a sample of cells from a subject *e.g.,* detecting NOVX mRNA levels or determining whether a genomic NOVX gene has been mutated or deleted.

"A polypeptide having a biologically-active portion of an NOVX polypeptide" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of NOVX" can be prepared by isolating a portion SEQ ID NOS:1, 3, 5, 7, 9, 11, 13, 15, 17 or 19, that encodes a polypeptide having an NOVX biological activity (the biological activities of the NOVX proteins are described below), expressing the encoded portion of NOVX protein (*e.g.,* by recombinant expression *in vitro*) and assessing the activity of the encoded portion of NOVX.

### NOVX Nucleic Acid and Polypeptide Variants

The invention further encompasses nucleic acid molecules that differ from the nucleotide sequences shown in SEQ ID NOS:1, 3, 5, 7, 9, 12, 14, 16, 18, 20, 22, 24, 26, 28, 30, 32, 34, and 36 due to degeneracy of the genetic code and thus encode the same NOVX proteins as that encoded by the nucleotide sequences shown in SEQ ID NOS:1, 3, 5, 7, 9, 12, 14, 16, 18, 20, 22, 24, 26, 28, 30, 32, 34, and 36. In another embodiment, an isolated nucleic acid molecule of the invention has a nucleotide sequence encoding a protein having an amino acid sequence shown in SEQ ID NOS:2, 4, 6, 8, 11, 13, 15, 17, 19, 21, 23, 25, 27, 29, 31, 33, 35, or 37.

In addition to the human NOVX nucleotide sequences shown in SEQ ID NOS:1, 3, 5, 7, 9, 12, 14, 16, 18, 20, 22, 24, 26, 28, 30, 32, 34, and 36, it will be appreciated by those skilled in the art that DNA sequence polymorphisms that lead to changes in the amino acid sequences of the NOVX polypeptides may exist within a population (*e.g.*, the human population). Such genetic polymorphism in the NOVX genes may exist among individuals within a population due to natural allelic variation. As used herein, the terms "gene" and "recombinant gene" refer to nucleic acid molecules comprising an open reading frame (ORF) encoding an NOVX protein, preferably a vertebrate NOVX protein. Such natural allelic variations can typically result in 1-5% variance in the nucleotide sequence of the NOVX genes. Any and all such nucleotide variations and resulting amino acid polymorphisms in the NOVX polypeptides, which are the result of natural allelic variation and that do not alter the functional activity of the NOVX polypeptides, are intended to be within the scope of the invention.

Moreover, nucleic acid molecules encoding NOVX proteins from other species, and thus that have a nucleotide sequence that differs from the human SEQ ID NOS:1,3, 5,7, 9, 12, 14, 16, 18, 20, 22, 24, 26, 28, 30, 32, 34, and 36 are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions.

Accordingly, in another embodiment, an isolated nucleic acid molecule of the invention is at least 6 nucleotides in length and hybridizes under stringent conditions to the nucleic acid molecule comprising the nucleotide sequence of SEQ ID NOS:1, 3, 5, 7, 9, 12, 14, 16, 18, 20, 22, 24, 26, 28, 30, 32, 34, and 36. In another embodiment, the nucleic acid is at least 10, 25, 50, 100, 250, 500, 750, 1000, 1500, or 2000 or more nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (*i.e.,* nucleic acids encoding NOVX proteins derived from species other than human) or other related sequences (*e.g.*, paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5 °C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e.g.*, 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in Ausubel, et al., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C. An isolated nucleic acid molecule of the invention that hybridizes under stringent conditions to the sequences SEQ ID NOS:1, 3, 5, 7, 9, 12, 14, 16, 18, 20, 22, 24, 26, 28, 30, 32, 34, and 36, corresponds to a naturally-occurring nucleic acid molecule. As used herein, a "naturally-occurring" nucleic acid molecule refers to an RNA or DNA molecule having a nucleotide sequence that occurs in nature (*e.g*., encodes a natural protein).

In a second embodiment, a nucleic acid sequence that is hybridizable to the nucleic acid molecule comprising the nucleotide sequence of SEQ ID NOS:1, 3, 5, 7, 9, 12, 14, 16, 18, 20, 22, 24, 26, 28, 30, 32, 34, and 36, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well-known within the art. *See, e.g.,* Ausubel, et al. (eds.), 1993, CURRENT PROTOCOLS IN MOLECULAR BIOLOGY, John Wiley & Sons, NY, and Kriegler, 1990; GENE TRANSFER AND EXPRESSION, A LABORATORY MANUAL, Stockton Press, NY.

In a third embodiment, a nucleic acid that is hybridizable to the nucleic acid molecule comprising the nucleotide sequences SEQ ID NOS:1, 3, 5, 7, 9, 12, 14, 16, 18, 20, 22, 24, 26, 28, 30, 32, 34, and 36, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g*., as employed for cross-species hybridizations). *See, e.g.,* Ausubel, et al. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. Proc Natl Acad Sci USA 78: 6789-6792.

### Conservative Mutations

In addition to naturally-occurring allelic variants of NOVX sequences that may exist in the population, the skilled artisan will further appreciate that changes can be introduced by mutation into the nucleotide sequences SEQ ID NOS: 1, 3, 5, 7, 9, 12, 14, 16, 18, 20, 22, 24, 26, 28, 30, 32, 34, and 36, thereby leading to changes in the amino acid sequences of the encoded NOVX proteins, without altering the functional ability of said NOVX proteins. For example, nucleotide substitutions leading to amino acid substitutions at "non-essential" amino acid residues can be made in the sequence SEQ ID NOS:2, 4, 6, 8, 11, 13, 15, 17, 19, 21, 23, 25, 27, 29, 31, 33, 35, or 37. A "non-essential" amino acid residue is a residue that can be altered from the wild-type sequences of the NOVX proteins without altering their biological activity, whereas an "essential" amino acid residue is required for such biological activity. For example, amino acid residues that are conserved among the NOVX proteins of the invention are predicted to be particularly non-amenable to alteration. Amino acids for which conservative substitutions can be made are well-known within the art.

Another aspect of the invention pertains to nucleic acid molecules encoding NOVX proteins that contain changes in amino acid residues that are not essential for activity. Such NOVX proteins differ in amino acid sequence from SEQ ID NOS:1, 3, 5, 7, 9, 12, 14, 16, 18, 20, 22, 24, 26, 28, 30, 32, 34, and 36 yet retain biological activity. In one embodiment, the isolated nucleic acid molecule comprises a nucleotide sequence encoding a protein, wherein the protein comprises an amino acid sequence at least about 45% homologous to the amino acid sequences SEQ ID NOS:2, 4, 6, 8, 11, 13, 15, 17, 19, 21, 23, 25, 27, 29, 31, 33, 35, and 37. Preferably, the protein encoded by the nucleic acid molecule is at least about 60% homologous to SEQ ID NOS:2, 4, 6, 8, 11, 13, 15, 17, 19, 21, 23, 25, 27, 29, 31, 33, 35, and 37; more preferably at least about 70% homologous SEQ ID NOS:2, 4, 6, 8, 11, 13, 15, 17, 19, 21, 23, 25, 27, 29, 31, 33, 35, or 37; still more preferably at least about 80% homologous to SEQ ID NOS:2, 4, 6, 8, 11, 13, 15, 17, 19, 21, 23, 25, 27, 29, 31, 33, 35, or 37; even more preferably at least about 90% homologous to SEQ ID NOS:2, 4, 6, 8, 11, 13, 15, 17, 19, 21, 23, 25, 27, 29, 31, 33, 35, or 37; and most preferably at least about 95% homologous to SEQ ID NOS:2, 4, 6, 8, 11, 13, 15, 17, 19, 21, 23, 25, 27, 29, 31, 33, 35, or 37.

An isolated nucleic acid molecule encoding an NOVX protein homologous to the protein of SEQ ID NOS:2, 4, 6, 8,11, 13,15, 17, 19, 21, 23, 25, 27, 29, 31, 33, 35, or 37 can be created by introducing one or more nucleotide substitutions, additions or deletions into the nucleotide sequence of SEQ ID NOS:1, 3, 5, 7, 9, 12, 14, 16, 18, 20, 22, 24, 26, 28, 30, 32, 34, and 36, such that one or more amino acid substitutions, additions or deletions are introduced into the encoded protein.

Mutations can be introduced into SEQ ID NOS:1, 3, 5, 7, 9, 12, 14, 16, 18, 20, 22, 24, 26, 28, 30, 32, 34, and 36 by standard techniques, such as site-directed mutagenesis and PCR-mediated mutagenesis. Preferably, conservative amino acid substitutions are made at one or more predicted, non-essential amino acid residues. A "conservative amino acid substitution" is one in which the amino acid residue is replaced with an amino acid residue having a similar side chain. Families of amino acid residues having similar side chains have been defined within the art. These families include amino acids with basic side chains (*e.g.*, lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.,* threonine, valine, isoleucine) and aromatic side chains (*e.g.*, tyrosine, phenylalanine, tryptophan, histidine). Thus, a predicted non-essential amino acid residue in the NOVX protein is replaced with another amino acid residue from the same side chain family. Alternatively, in another embodiment, mutations can be introduced randomly along all or part of an NOVX coding sequence, such as by saturation mutagenesis, and the resultant mutants can be screened for NOVX biological activity to identify mutants that retain activity. Following mutagenesis SEQ ID NOS:1, 3, 5, 7, 9, 12, 14, 16, 18, 20, 22, 24, 26, 28, 30, 32, 34, and 36, the encoded protein can be expressed by any recombinant technology known in the art and the activity of the protein can be determined.

The relatedness of amino acid families may also be determined based on side chain interactions. Substituted amino acids may be fully conserved "strong" residues or fully conserved "weak" residues. The "strong" group of conserved amino acid residues may be any one of the following groups: STA, NEQK, NHQK, NDEQ, QHRK, MILV, MILF, HY, FYW, wherein the single letter amino acid codes are grouped by those amino acids that may be substituted for each other. Likewise, the "weak" group of conserved residues may be any one of the following: CSA, ATV, SAG, STNK, STPA, SGND, SNDEQK, NDEQHK, NEQHRK, VLIM, HFY, wherein the letters within each group represent the single letter amino acid code.

In one embodiment, a mutant NOVX protein can be assayed for (*i*) the ability to form protein:protein interactions with other NOVX proteins, other cell-surface proteins, or biologically-active portions thereof, (*ii*) complex formation between a mutant NOVX protein and an NOVX ligand; or (*ii*i) the ability of a mutant NOVX protein to bind to an intracellular target protein or biologically-active portion thereof; (*e.g.* avidin proteins).

In yet another embodiment, a mutant NOVX protein can be assayed for the ability to regulate a specific biological function (*e.g.,* regulation of insulin release).

### Antisense Nucleic Acids

Another aspect of the invention pertains to isolated antisense nucleic acid molecules that are hybridizable to or complementary to the nucleic acid molecule comprising the nucleotide sequence of SEQ ID NOS:1, 3, 5, 7, 9, 12, 14, 16, 18, 20, 22, 24, 26, 28, 30, 32, 34, and 36, or fragments, analogs or derivatives thereof. An "antisense" nucleic acid comprises a nucleotide sequence that is complementary to a "sense" nucleic acid encoding a protein (*e.g.*, complementary to the coding strand of a double-stranded cDNA molecule or complementary to an mRNA sequence). In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of an NOVX protein of SEQ ID NOS:2, 4, 6, 8, 11, 13, 15, 17, 19, 21, 23, 25, 27, 29, 31, 33, 35, or 37, or antisense nucleic acids complementary to an NOVX nucleic acid sequence of SEQ ID NOS:1, 3, 5, 7, 9, 12, 14, 16, 18, 20, 22, 24, 26, 28, 30, 32, 34, and 36, are additionally provided.

In one embodiment, an antisense nucleic acid molecule is antisense to a "coding region" of the coding strand of a nucleotide sequence encoding an NOVX protein. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding the NOVX protein. The term "noncoding region" refers to 5' and 3' sequences which flank the coding region that are not translated into amino acids (*i.e.,* also referred to as 5' and 3' untranslated regions).

Given the coding strand sequences encoding the NOVX protein disclosed herein, antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site of NOVX mRNA. An antisense oligonucleotide can be, for example, about 5,10,15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e.g.,* an antisense oligonucleotide) can be chemically synthesized using naturally-occurring nucleotides or variously modified nucleotides designed to increase the biological stability of the molecules or to increase the physical stability of the duplex formed between the antisense and sense nucleic acids (*e.g.*, phosphorothioate derivatives and acridine substituted nucleotides can be used).

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding an NOVX protein to thereby inhibit expression of the protein (*e*.*g*., by inhibiting transcription and/or translation). The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (*e.g.*, by linking the antisense nucleic acid molecules to peptides or antibodies that bind to cell surface receptors or antigens). The antisense nucleic acid molecules can also be delivered to cells using the vectors described herein. To achieve sufficient nucleic acid molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other. *See, e.g.,* Gaultier, et al., 1987. Nucl. Acids Res. 15: 6625-6641. The antisense nucleic acid molecule can also comprise a 2'-o-methylribonucleotide (*See, e.g.,* Inoue, et al. 1987. Nucl. Acids Res. 15: 6131-6148) or a chimeric RNA-DNA analogue (*See, e.g.,* Inoue, et al., 1987. FEBS Lett. 215: 327-330.

### Ribozymes and PNA Moieties

Nucleic acid modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In one embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (e.g., hammerhead ribozymes as described in Haselhoff and Gerlach 1988. Nature 334: 585-591) can be used to catalytically cleave NOVX mRNA transcripts to thereby inhibit translation of NOVX mRNA. A ribozyme having specificity for an NOVX-encoding nucleic acid can be designed based upon the nucleotide sequence of an NOVX cDNA disclosed herein (*i.e.,* SEQ ID NOS:1, 3, 5, 7, 9, 12, 14, 16, 18, 20, 22, 24, 26, 28, 30, 32, 34, and 36). For example, a derivative of a *Tetrahymena* L-19 IVS RNA can be constructed in which the nucleotide sequence of the active site is complementary to the nucleotide sequence to be cleaved in an NOVX-encoding mRNA. *See, e.g.,* U.S. Patent 4,987,071 to Cech, et al. and U.S. Patent 5,116,742 to Cech, et al. NOVX mRNA can also be used to select a catalytic RNA having a specific ribonuclease activity from a pool of RNA molecules. *See, e.g.,* Bartel et al., (1993) Science 261:1411-1418*.*

Alternatively, NOVX gene expression can be inhibited by targeting nucleotide sequences complementary to the regulatory region of the NOVX nucleic acid (e.g., the NOVX promoter and/or enhancers) to form triple helical structures that prevent transcription of the NOVX gene in target cells. *See, e.g.,* Helene, 1991. Anticancer Drug Des. 6: 569-84; Helene, et al. 1992. Ann. N. Y. Acad. Sci. 660: 27-36; Maher, 1992. Bioassays 14: 807-15.

In various embodiments, the NOVX nucleic acids can be modified at the base moiety, sugar moiety or phosphate backbone to improve, *e.g*., the stability, hybridization, or solubility of the molecule. For example, the deoxyribose phosphate backbone of the nucleic acids can be modified to generate peptide nucleic acids. *See, e.g.,* Hyrup, et al., 1996. Bioorg Med Chem 4: 5-23. As used herein, the terms "peptide nucleic acids" or "PNAs" refer to nucleic acid mimics (e.g., DNA mimics) in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup, *et al.,* 1996. *supra;* Perry-O'Keefe, et al., 1996. Proc. Natl. Acad. Sci. USA 93: 14670-14675.

PNAs of NOVX can be used in therapeutic and diagnostic applications. For example, PNAs can be used as antisense or antigene agents for sequence-specific modulation of gene expression by, *e.g*., inducing transcription or translation arrest or inhibiting replication. PNAs of NOVX can also be used, for example, in the analysis of single base pair mutations in a gene (*e.g*., PNA directed PCR clamping; as artificial restriction enzymes when used in combination with other enzymes, *e.g.,* S₁ nucleases (*See,* Hyrup, *et al.,* 1996*.supra);* or as probes or primers for DNA sequence and hybridization (*See,* Hyrup, *et al.,* 1996, *supra;* Perry-O'Keefe, *et al.,* 1996*. supra*).

In another embodiment, PNAs of NOVX can be modified, *e.g.,* to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes (e.g., RNase H and DNA polymerases) to interact with the DNA portion while the PNA portion would provide high binding affinity and specificity. PNA-DNA chimeras can be linked using linkers of appropriate lengths selected in terms of base stacking, number of bonds between the nucleobases, and orientation *(see,* Hyrup, et al., 1996. *supra).* The synthesis of PNA-DNA chimeras can be performed as described in Hyrup, *et al.,* 1996. *supra* and Finn, et al., 1996. Nucl Acids Res 24: 3357-3363. For example, a DNA chain can be synthesized on a solid support using standard phosphoramidite coupling chemistry, and modified nucleoside analogs, *e*.*g*., 5'-(4-methoxytrityl)amino-5'-deoxy-thymidine phosphoramidite, can be used between the PNA and the 5' end of DNA. *See, e.g.,* Mag, et al., 1989. Nucl Acid Res 17: 5973-5988. PNA monomers are then coupled in a stepwise manner to produce a chimeric molecule with a 5' PNA segment and a 3' DNA segment. *See, e.g.,* Finn, *et al.,* 1996. *supra.* Alternatively, chimeric molecules can be synthesized with a 5' DNA segment and a 3' PNA segment. *See, e.g.,* Petersen, et al., 1975. Bioorg. Med. Chem. Lett. 5: 1119-11124.

In other embodiments, the oligonucleotide may include other appended groups such as peptides (*e.g.*, for targeting host cell receptors *in vivo*), or agents facilitating transport across the cell membrane *(see, e.g.,* Letsinger, et al., 1989. Proc. Natl. Acad. Sci. U.S.A. 86: 6553-6556; Lemaitre, et al., 1987. Proc. Natl. Acad. Sci. 84: 648-652; PCT Publication No. WO88/09810) or the blood-brain barrier *(see, e.g.,* PCT Publication No. WO 89/10134). In addition, oligonucleotides can be modified with hybridization triggered cleavage agents (*see, e.g.,* Krol, et al., 1988. BioTechniques 6:958-976) or intercalating agents (*see, e.g.,* Zon, 1988. Pharm. Res. 5: 539-549). To this end, the oligonucleotide may be conjugated to another molecule, *e.g*., a peptide, a hybridization triggered cross-linking agent, a transport agent, a hybridization-triggered cleavage agent, and the like.

### NOVX Polypeptides

A polypeptide according to the invention includes a polypeptide including the amino acid sequence of NOVX polypeptides whose sequences are provided in SEQ ID NOS:2, 4, 6, 8, 11, 13, 15, 17, 19, 21, 23, 25, 27, 29, 31, 33, 35, or 37. The invention also includes a mutant or variant protein any of whose residues may be changed from the corresponding residues shown in SEQ ID NOS:2, 4, 6, 8, 11, 13, 15, 17, 19, 21, 23, 25, 27, 29, 31, 33, 35, or 37 while still encoding a protein that maintains its NOVX activities and physiological functions, or a functional fragment thereof.

In general, an NOVX variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated NOVX proteins, and biologically-active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, an NOVX protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX proteins in which the protein is separated from cellular components of the cells from which it is isolated or recombinantly-produced. In one embodiment, the language "substantially free of cellular material" includes preparations of NOVX proteins having less than about 30% (by dry weight) of non-NOVX proteins (also referred to herein as a "contaminating protein"), more preferably less than about 20% of non-NOVX proteins, still more preferably less than about 10% of non-NOVX proteins, and most preferably less than about 5% of non-NOVX proteins. When the NOVX protein or biologically-active portion thereof is recombinantly-produced, it is also preferably substantially free of culture medium, *i*.*e*., culture medium represents less than about 20%, more preferably less than about 10%, and most preferably less than about 5% of the volume of the NOVX protein preparation.

The language "substantially free of chemical precursors or other chemicals" includes preparations of NOVX proteins in which the protein is separated from chemical precursors or other chemicals that are involved in the synthesis of the protein. In one embodiment, the language "substantially free of chemical precursors or other chemicals" includes preparations of NOVX proteins having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically-active portions of NOVX proteins include peptides comprising amino acid sequences sufficiently homologous to or derived from the amino acid sequences of the NOVX proteins (*e.g.*, the amino acid sequence shown in SEQ ID NOS:2, 4, 6, 8, 11, 13, 15, 17, 19, 21, 23, 25, 27, 29, 31, 33, 35, or 37) that include fewer amino acids than the full-length NOVX proteins, and exhibit at least one activity of an NOVX protein. Typically, biologically-active portions comprise a domain or motif with at least one activity of the NOVX protein. A biologically-active portion of an NOVX protein can be a polypeptide which is, for example, 10, 25, 50, 100 or more amino acid residues in length.

Moreover, other biologically-active portions, in which other regions of the protein are deleted, can be prepared by recombinant techniques and evaluated for one or more of the functional activities of a native NOVX protein.

In an embodiment, the NOVX protein has an amino acid sequence shown SEQ ID NOS:2, 4, 6, 8, 11, 13, 15, 17, 19, 21, 23, 25, 27, 29, 31, 33, 35, or 37. In other embodiments, the NOVX protein is substantially homologous to SEQ ID NOS:2,4, 6, 8,11,13,15,17,19, 21, 23, 25, 27, 29, 31, 33, 35, or 37, and retains the functional activity of the protein of SEQ ID NOS:2, 4, 6, 8, 11, 13, 15, 17, 19, 21, 23, 25, 27, 29, 31, 33, 35, or 37, yet differs in amino acid sequence due to natural allelic variation or mutagenesis, as described in detail, below. Accordingly, in another embodiment, the NOVX protein is a protein that comprises an amino acid sequence at least about 45% homologous to the amino acid sequence SEQ ID NOS:2, 4, 6, 8,11,13,15, 17, 19, 21, 23, 25, 27, 29, 31, 33, 35, or 37, and retains the functional activity of the NOVX proteins of SEQ ID NOS:2, 4, 6, 8, 11, 13, 15, 17, 19, 21, 23, 25, 27, 29, 31, 33, 35, or 37.

### Determining Homology Between Two or More Sequences

To determine the percent homology of two amino acid sequences or of two nucleic acids, the sequences are aligned for optimal comparison purposes (*e.g.*, gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e.,* as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See,* Needleman and Wunsch, 1970. J Mol Biol 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence shown in SEQ ID NOS:1, 3, 5, 7, 9, 12, 14, 16, 18, 20, 22, 24, 26, 28, 30, 32, 34, and 36.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g.,* A, T, C, G, U, or I, in the case of nucleic acids) occurs in both sequences to yield the number of matched positions, dividing the number of matched positions by the total number of positions in the region of comparison (*i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides NOVX chimeric or fusion proteins. As used herein, an NOVX "chimeric protein" or "fusion protein" comprises an NOVX polypeptide operatively-linked to a non-NOVX polypeptide. An "NOVX polypeptide" refers to a polypeptide having an amino acid sequence corresponding to an NOVX protein SEQ ID NOS:2, 4, 6, 8, 11, 13, 15, 17, 19, 21, 23, 25, 27, 29, 31, 33, 35, or 37), whereas a "non-NOVX polypeptide" refers to a polypeptide having an amino acid sequence corresponding to a protein that is not substantially homologous to the NOVX protein, *e.g*., a protein that is different from the NOVX protein and that is derived from the same or a different organism. Within an NOVX fusion protein the NOVX polypeptide can correspond to all or a portion of an NOVX protein. In one embodiment, an NOVX fusion protein comprises at least one biologically-active portion of an NOVX protein. In another embodiment, an NOVX fusion protein comprises at least two biologically-active portions of an NOVX protein. In yet another embodiment, an NOVX fusion protein comprises at least three biologically-active portions of an NOVX protein. Within the fusion protein, the term "operatively-linked" is intended to indicate that the NOVX polypeptide and the non-NOVX polypeptide are fused in-frame with one another. The non-NOVX polypeptide can be fused to the N-terminus or C-terminus of the NOVX polypeptide.

In one embodiment, the fusion protein is a GST-NOVX fusion protein in which the NOVX sequences are fused to the C-terminus of the GST (glutathione S-transferase) sequences. Such fusion proteins can facilitate the purification of recombinant NOVX polypeptides.

In another embodiment, the fusion protein is an NOVX protein containing a heterologous signal sequence at its N-terminus. In certain host cells (*e.g.,* mammalian host cells), expression and/or secretion of NOVX can be increased through use of a heterologous signal sequence.

In yet another embodiment, the fusion protein is an NOVX-immunoglobulin fusion protein in which the NOVX sequences are fused to sequences derived from a member of the immunoglobulin protein family. The NOVX-immunoglobulin fusion proteins of the invention can be incorporated into pharmaceutical compositions and administered to a subject to inhibit an interaction between an NOVX ligand and an NOVX protein on the surface of a cell, to thereby suppress NOVX-mediated signal transduction *in vivo.* The NOVX-immunoglobulin fusion proteins can be used to affect the bioavailability of an NOVX cognate ligand.
Inhibition of the NOVX ligand/NOVX interaction may be useful therapeutically for both the treatment of proliferative and differentiative disorders, as well as modulating (*e.g.* promoting or inhibiting) cell survival. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with an NOVX ligand.

An NOVX chimeric or fusion protein of the invention can be produced by standard recombinant DNA techniques. For example, DNA fragments coding for the different polypeptide sequences are ligated together in-frame in accordance with conventional techniques, *e.g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence *(see, e.g.,* Ausubel, et al. (eds.) CURRENT PROTOCOLS IN MOLECULAR BIOLOGY, John Wiley & Sons, 1992). Moreover, many expression vectors are commercially available that already encode a fusion moiety (*e.g.*, a GST polypeptide). An NOVX-encoding nucleic acid can be cloned into such an expression vector such that the fusion moiety is linked in-frame to the NOVX protein.

### NOVX Agonists and Antagonists

The invention also pertains to variants of the NOVX proteins that function as either NOVX agonists (*i.e.,* mimetics) or as NOVX antagonists. Variants of the NOVX protein can be generated by mutagenesis (*e*.*g*., discrete point mutation or truncation of the NOVX protein). An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX proteins that function as either NOVX agonists *(i.e.,* mimetics) or as NOVX antagonists can be identified by screening combinatorial libraries of mutants (*e.g*., truncation mutants) of the NOVX proteins for NOVX protein agonist or antagonist activity. In one embodiment, a variegated library of NOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (*e.g.*, for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are well-known within the art*. See, e.g.,* Narang, 1983. Tetrahedron 39: 3; Itakura, et al., 1984. Annu. Rev. Biochem. 53: 323; Itakura, et al., 1984. Science 198: 1056; Ike, et al., 1983. Nucl. Acids Res. 11: 477.

### Polypeptide Libraries

In addition, libraries of fragments of the NOVX protein coding sequences can be used to generate a variegated population of NOVX fragments for screening and subsequent selection of variants of an NOVX protein. In one embodiment, a library of coding sequence fragments can be generated by treating a double stranded PCR fragment of an NOVX coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with S₁ nuclease, and ligating the resulting fragment library into an expression vector. By this method, expression libraries can be derived which encodes N-terminal and internal fragments of various sizes of the NOVX proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify NOVX variants. *See, e.g.,* Arkin and Yourvan, 1992. Proc. Natl. Acad. Sci. USA 89: 7811-7815; Delgrave, et al., 1993. Protein Engineering 6:327-331.

### Anti-NOVX Antibodies

Also included in the invention are antibodies to NOVX proteins, or fragments of NOVX proteins. The term "antibody" as used herein refers to immunoglobulin molecules and immunologically active portions of immunoglobulin (Ig) molecules, i.e., molecules that contain an antigen binding site that specifically binds (immunoreacts with) an antigen. Such antibodies include, but are not limited to, polyclonal, monoclonal, chimeric, single chain, F_{ab}, F_{ab'} and F_{(ab')2} fragments, and an F_{ab} expression library. In general, an antibody molecule obtained from humans relates to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain. Reference herein to antibodies includes a reference to all such classes, subclasses and types of human antibody species.

An isolated NOVX-related protein of the invention may be intended to serve as an antigen, or a portion or fragment thereof, and additionally can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of NOVX-related protein that is located on the surface of the protein, *e.g.,* a hydrophilic region. A hydrophobicity analysis of the human NOVX-related protein sequence will indicate which regions of a NOVX-related protein are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e.g.,* Hopp and Woods, 1981, Proc. Nat. Acad. Sci. USA 78: 3824-3828; Kyte and Doolittle 1982, J. Mol. Biol. 157: 105-142, each of which is incorporated herein by reference in its entirety. Antibodies that are specific for one or more domains within an antigenic protein, or derivatives, fragments, analogs or homologs thereof, are also provided herein.

A protein of the invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow and Lane, 1988, Cold Spring Harbor Laboratory Press, Cold Spring Harbor, NY, incorporated herein by reference). Some of these antibodies are discussed below.

### Polyclonal Antibodies

For the production of polyclonal antibodies, various suitable host animals (*e.g.*, rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant. Various adjuvants used to increase the immunological response include, but are not limited to, Freund's (complete and incomplete), mineral gels (e.g., aluminum hydroxide), surface active substances (e.g., lysolecithin, pluronic polyols, polyanions, peptides, oil emulsions, dinitrophenol, etc.), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (e.g., from the blood) and further purified by well known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### Monoclonal Antibodies

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro.

The immunizing agent will typically include the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, MONOCLONAL ANTIBODIES: PRINCIPLES AND PRACTICE, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol., 133:3001 (1984); Brodeur et al., MONOCLONAL ANTIBODY PRODUCTION TECHNIQUES AND APPLICATIONS, Marcel Dekker, Inc., New York, (1987) pp. 51-63).

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). Preferably, antibodies having a high degree of specificity and a high binding affinity for the target antigen are isolated.

After the desired hybridoma cells are identified, the clones can be subcloned by limiting dilution procedures and grown by standard methods. Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Patent No. 4,816,567; Morrison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### Humanized Antibodies

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, Curr. Op. Struct. Biol., 2:593-596 (1992)).

### Human Antibodies

Fully human antibodies relate to antibody molecules in which essentially the entire sequences of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R. Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R. Liss, Inc., pp. 77-96).

In addition, human antibodies can also be produced using additional techniques, including phage display libraries (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. (Bio/Technology 10, 779-783 (1992)); Lonberg et al. (Nature 368 856-859 (1994)); Morrison ( Nature 368, 812-13 (1994)); Fishwild et al,( Nature Biotechnology 14, 845-51 (1996)); Neuberger (Nature Biotechnology 14, 826 (1996)); and Lonberg and Huszar (Intern. Rev. Immunol. 13 65-93 (1995)).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse^{™} as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### F_{ab} Fragments and Single Chain Antibodies

According to the invention, techniques can be adapted for the production of single-chain antibodies specific to an antigenic protein of the invention (see e.g., U.S. Patent No. 4,946,778). In addition, methods can be adapted for the construction of F_{ab} expression libraries (see e.g., Huse, et al., 1989 Science 246: 1275-1281) to allow rapid and effective identification of monoclonal F_{ab} fragments with the desired specificity for a protein or derivatives, fragments, analogs or homologs thereof. Antibody fragments that contain the idiotypes to a protein antigen may be produced by techniques known in the art including, but not limited to: (i) an F_{(ab')2} fragment produced by pepsin digestion of an antibody molecule; (ii) an F_{ab} fragment generated by reducing the disulfide bridges of an F_{(ab')2} fragment; (iii) an F_{ab} fragment generated by the treatment of the antibody molecule with papain and a reducing agent and (iv) Fᵥ fragments.

### Bispecific Antibodies

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., 1991 EMBO J., 10:3655-3659.

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Additionally, Fab' fragments can be directly recovered from E. coli and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

### Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### Effector Function Engineering

It can be desirable to modify the antibody of the invention with respect to effector function, so as to enhance, e.g., the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

### Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g., avidin) that is in turn conjugated to a cytotoxic agent.

In one embodiment, methods for the screening of antibodies that possess the desired specificity include, but are not limited to, enzyme-linked immunosorbent assay (ELISA) and other immunologically-mediated techniques known within the art. In a specific embodiment, selection of antibodies that are specific to a particular domain of an NOVX protein is facilitated by generation of hybridomas that bind to the fragment of an NOVX protein possessing such a domain. Thus, antibodies that are specific for a desired domain within an NOVX protein, or derivatives, fragments, analogs or homologs thereof, are also provided herein.

Anti-NOVX antibodies may be used in methods known within the art relating to the localization and/or quantitation of an NOVX protein (*e.g*., for use in measuring levels of the NOVX protein within appropriate physiological samples, for use in diagnostic methods, for use in imaging the protein, and the like). In a given embodiment, antibodies for NOVX proteins, or derivatives, fragments, analogs or homologs thereof, that contain the antibody derived binding domain, are utilized as pharmacologically-active compounds (hereinafter "Therapeutics").

An anti-NOVX antibody (*e.g.*, monoclonal antibody) can be used to isolate an NOVX polypeptide by standard techniques, such as affinity chromatography or immunoprecipitation. An anti-NOVX antibody can facilitate the purification of natural NOVX polypeptide from cells and of recombinantly-produced NOVX polypeptide expressed in host cells. Moreover, an anti-NOVX antibody can be used to detect NOVX protein (*e.g.*, in a cellular lysate or cell supernatant) in order to evaluate the abundance and pattern of expression of the NOVX protein. Anti-NOVX antibodies can be used diagnostically to monitor protein levels in tissue as part of a clinical testing procedure, *e.g*., to, for example, determine the efficacy of a given treatment regimen. Detection can be facilitated by coupling (*i.e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### NOVX Recombinant Expression Vectors and Host Cells

Another aspect of the invention pertains to vectors, preferably expression vectors, containing a nucleic acid encoding an NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g.*, replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g*., in an *in vitro* transcription/translation system or in a host cell when the vector is introduced into the host cell).

The term "regulatory sequence" is intended to includes promoters, enhancers and other expression control elements (*e.g.,* polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g.*, tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e.g.*, NOVX proteins, mutant forms of NOVX proteins, fusion proteins, etc.).

The recombinant expression vectors of the invention can be designed for expression of NOVX proteins in prokaryotic or eukaryotic cells. For example, NOVX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann et al., (1988) Gene 69:301-315) and pET 11d (Studier et al., GENE EXPRESSION. TECHNOLOGY: METHODS IN ENZYMOLOGY 185, Academic Press, San Diego, Calif. (1990) 60-89).

One strategy to maximize recombinant protein expression in *E. coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli* (*see, e.g.,* Wada, et al., 1992. Nucl. Acids Res. 20: 2111-2118). Such alteration of nucleic acid sequences of the invention can be carried out by standard DNA synthesis techniques.

In another embodiment, the NOVX expression vector is a yeast expression vector. Examples of vectors for expression in yeast *Saccharomyces cerivisae* include pYepSec1 (Baldari, et al., 1987. EMBO J. 6: 229-234), pMFa (Kurjan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

Alternatively, NOVX can be expressed in insect cells using baculovirus expression vectors. Baculovirus vectors available for expression of proteins in cultured insect cells (*e.g.,* SF9 cells) include the pAc series (Smith, et al., 1983. Mol. Cell. Biol. 3: 2156-2165) and the pVL series (Lucklow and Summers, 1989. Virology 170: 31-39).

In yet another embodiment, a nucleic acid of the invention is expressed in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e.g.,* Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (*e.g.*, tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Banerji, et al., 1983. Cell 33: 729-740; Queen and Baltimore, 1983. Cell 33: 741-748), neuron-specific promoters *(e.g.,* the neurofilament promoter; Byme and Ruddle, 1989. Proc. Natl. Acad. Sci. USA 86: 5473-5477), pancreas-specific promoters (Edlund, et al., 1985. Science 230: 912-916), and mammary gland-specific promoters (*e.g*., milk whey promoter; U.S. Pat. No. 4,873,316 and European Application Publication No. 264,166). Developmentally-regulated promoters are also encompassed, *e.g*., the murine hox promoters (Kessel and Gruss, 1990. Science 249: 374-379) and the α-fetoprotein promoter (Campes and Tilghman, 1989. Genes Dev. 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, et al., "Antisense RNA as a molecular tool for genetic analysis," Reviews-Trends in Genetics, Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g.*, resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (*e.g.*, cells that have incorporated the selectable marker gene will survive, while the other cells die).

A host cell of the invention, such as a prokaryotic or eukaryotic host cell in culture, can be used to produce (*i.e.,* express) NOVX protein. Accordingly, the invention further provides methods for producing NOVX protein using the host cells of the invention. In one embodiment, the method comprises culturing the host cell of invention (into which a recombinant expression vector encoding NOVX protein has been introduced) in a suitable medium such that NOVX protein is produced. In another embodiment, the method further comprises isolating NOVX protein from the medium or the host cell.

### Transgenic NOVX Animals

The host cells of the invention can also be used to produce non-human transgenic animals. For example, in one embodiment, a host cell of the invention is a fertilized oocyte or an embryonic stem cell into which NOVX protein-coding sequences have been introduced. Such host cells can then be used to create non-human transgenic animals in which exogenous NOVX sequences have been introduced into their genome or homologous recombinant animals in which endogenous NOVX sequences have been altered. Such animals are useful for studying the function and/or activity of NOVX protein and for identifying and/or evaluating modulators of NOVX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g*., an embryonic cell of the animal, prior to development of the animal.

A transgenic animal of the invention can be created by introducing NOVX-encoding nucleic acid into the male pronuclei of a fertilized oocyte (*e.g.*, by microinjection, retroviral infection) and allowing the oocyte to develop in a pseudopregnant female foster animal. The human NOVX cDNA sequences SEQ ID NOS:1, 3, 5, 7, 9, 12, 14, 16, 18, 20, 22, 24, 26, 28, 30, 32, 34, and 36 can be introduced as a transgene into the genome of a non-human animal. Alternatively, a non-human homologue of the human NOVX gene, such as a mouse NOVX gene, can be isolated based on hybridization to the human NOVX cDNA (described further *supra)* and used as a transgene. Intronic sequences and polyadenylation signals can also be included in the transgene to increase the efficiency of expression of the transgene. A tissue-specific regulatory sequence(s) can be operably-linked to the NOVX transgene to direct expression of NOVX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX transgene in its genome and/or expression of NOVX mRNA in tissues or cells of the animals. A transgenic founder animal can then be used to breed additional animals carrying the transgene. Moreover, transgenic animals carrying a transgene-encoding NOVX protein can further be bred to other transgenic animals carrying other transgenes.

To create a homologous recombinant animal, a vector is prepared which contains at least a portion of an NOVX gene into which a deletion, addition or substitution has been introduced to thereby alter, *e*.*g*., functionally disrupt, the NOVX gene. The NOVX gene can be a human gene (*e*.*g*., the cDNA of SEQ ID NOS:1,3, 5, 7, 9, 12, 14, 16, 18, 20, 22, 24, 26, 28, 30, 32, 34, and 36), but more preferably, is a non-human homologue of a human NOVX gene. For example, a mouse homologue of human NOVX gene of SEQ ID NOS:1, 3, 5, 7, 9, 12, 14, 16, 18, 20, 22, 24, 26, 28, 30, 32, 34, and 36 can be used to construct a homologous recombination vector suitable for altering an endogenous NOVX gene in the mouse genome. In one embodiment, the vector is designed such that, upon homologous recombination, the endogenous NOVX gene is functionally disrupted (*i.e.,* no longer encodes a functional protein; also referred to as a "knock out" vector).

Alternatively, the vector can be designed such that, upon homologous recombination, the endogenous NOVX gene is mutated or otherwise altered but still encodes functional protein (*e.g.*, the upstream regulatory region can be altered to thereby alter the expression of the endogenous NOVX protein). In the homologous recombination vector, the altered portion of the NOVX gene is flanked at its 5'- and 3'-termini by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5'- and 3'-termini) are included in the vector. *See, e.g.,* Thomas, et al., 1987. Cell 51: 503 for a description of homologous recombination vectors. The vector is ten introduced into an embryonic stem cell line (*e.g.,* by electroporation) and cells in which the introduced NOVX gene has homologously-recombined with the endogenous NOVX gene are selected. *See, e.g.,* Li, et al., 1992. Cell 69: 915.

The selected cells are then injected into a blastocyst of an animal (*e.g.*, a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. Curr. Opin. Biotechnol. 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e.g.,* Lakso, et al., 1992. Proc. Natl. Acad. Sci. USA 89: 6232-6236. Another example of a recombinase system is the FLP recombinase system of *Saccharomyces cerevisiae. See,* O'Gorman, et al., 1991. Science 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g*., by mating two transgenic animals, one containing a transgene encoding a selected protein and the other containing a transgene encoding a recombinase.

Clones of the non-human transgenic animals described herein can also be produced according to the methods described in Wilmut, et al., 1997. Nature 385: 810-813. In brief, a cell (*e.g.*, a somatic cell) from the transgenic animal can be isolated and induced to exit the growth cycle and enter G₀ phase. The quiescent cell can then be fused, *e.g*., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell (*e.g*., the somatic cell) is isolated.

### Pharmaceutical Compositions

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g*., intravenous, intradermal, subcutaneous, oral (*e.g*., inhalation), transdermal (*i.e*., topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e*.*g*., an NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.*, with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration *(see, e.g.,* U.S. Patent No. 5,328,470) or by stereotactic injection (*see, e.g.,* Chen, et al., 1994. Proc. Natl. Acad. Sci. USA 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g*., retroviral vectors, the pharmaceutical preparation can include one or more cells that produce the gene delivery system.

The pharmaceutical compositions can be included in a container, pack, or dispenser together with instructions for administration.

### Screening and Detection Methods

The isolated nucleic acid molecules of the invention can be used to express NOVX protein (*e.g.*, via a recombinant expression vector in a host cell in gene therapy applications), to detect NOVX mRNA (*e*.*g*., in a biological sample) or a genetic lesion in an NOVX gene, and to modulate NOVX activity, as described further, below. In addition, the NOVX proteins can be used to screen drugs or compounds that modulate the NOVX protein activity or expression as well as to treat disorders characterized by insufficient or excessive production of NOVX protein or production of NOVX protein forms that have decreased or aberrant activity compared to NOVX wild-type protein (*e.g*.; diabetes (regulates insulin release); obesity (binds and transport lipids); metabolic disturbances associated with obesity, the metabolic syndrome X as well as anorexia and wasting disorders associated with chronic diseases and various cancers, and infectious disease(possesses anti-microbial activity) and the various dyslipidemias. In addition, the anti-NOVX antibodies of the invention can be used to detect and isolate NOVX proteins and modulate NOVX activity. In yet a further aspect, the invention can be used in methods to influence appetite, absorption of nutrients and the disposition of metabolic substrates in both a positive and negative fashion.

The invention further pertains to novel agents identified by the screening assays described herein and uses thereof for treatments as described, *supra.*

### Screening Assays

The invention provides a method (also referred to herein as a "screening assay") for identifying modulators, *i.e.,* candidate or test compounds or agents (*e.g.,* peptides, peptidomimetics, small molecules or other drugs) that bind to NOVX proteins or have a stimulatory or inhibitory effect on, *e.g.,* NOVX protein expression or NOVX protein activity. The invention also includes compounds identified in the screening assays described herein.

In one embodiment, the invention provides assays for screening candidate or test compounds which bind to or modulate the activity of the membrane-bound form of an NOVX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. Anticancer Drug Design 12: 145.

A "small molecule" as used herein, is meant to refer to a composition that has a molecular weight of less than about 5 kD and most preferably less than about 4 kD. Small molecules can be, *e.g*., nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, et al., 1993. Proc. Natl. Acad. Sci. U.S.A. 90: 6909; Erb, et al., 1994. Proc. Natl. Acad. Sci. U.S.A. 91: 11422; Zuckermann, et al., 1994. J. Med. Chem. 37: 2678; Cho, et al., 1993. Science 261: 1303; Carrell, et al., 1994. Angew. Chem. Int. Ed. Engl. 33: 2059; Carell, et al., 1994. Angew. Chem. Int. Ed. Engl. 33: 2061; and Gallop, et al., 1994. J. Med. Chem. 37: 1233.

Libraries of compounds may be presented in solution (*e.g*., Houghten, 1992. Biotechniques 13: 412-421), or on beads (Lam, 1991. Nature 354: 82-84), on chips (Fodor, 1993. Nature 364: 555-556), bacteria (Ladner, U.S. Patent No. 5,223,409), spores (Ladner, U.S. Patent 5,233,409), plasmids (Cull, et al., 1992. Proc. Natl. Acad. Sci. USA 89: 1865-1869) or on phage (Scott and Smith, 1990. Science 249: 386-390; Devlin, 1990. Science 249: 404-406; Cwirla, et al., 1990. Proc. Natl. Acad. Sci. U.S.A. 87: 6378-6382; Felici, 1991. J. Mol. Biol. 222: 301-310; Ladner, U.S. Patent No. 5,233,409.).

In one embodiment, an assay is a cell-based assay in which a cell which expresses a membrane-bound form of NOVX protein, or a biologically-active portion thereof, on the cell surface is contacted with a test compound and the ability of the test compound to bind to an NOVX protein determined. The cell, for example, can of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of NOVX protein, or a biologically-active portion thereof, on the cell surface with a known compound which binds NOVX to form an assay mixture, contacting the assay mixture with a test compound, and determining the ability of the test compound to interact with an NOVX protein, wherein determining the ability of the test compound to interact with an NOVX protein comprises determining the ability of the test compound to preferentially bind to NOVX protein or a biologically-active portion thereof as compared to the known compound.

In another embodiment, an assay is a cell-based assay comprising contacting a cell expressing a membrane-bound form of NOVX protein, or a biologically-active portion thereof, on the cell surface with a test compound and determining the ability of the test compound to modulate (*e.g.,* stimulate or inhibit) the activity of the NOVX protein or biologically-active portion thereof. Determining the ability of the test compound to modulate the activity of NOVX or a biologically-active portion thereof can be accomplished, for example, by determining the ability of the NOVX protein to bind to or interact with an NOVX target molecule. As used herein, a "target molecule" is a molecule with which an NOVX protein binds or interacts in nature, for example, a molecule on the surface of a cell which expresses an NOVX interacting protein, a molecule on the surface of a second cell, a molecule in the extracellular milieu, a molecule associated with the internal surface of a cell membrane or a cytoplasmic molecule. An NOVX target molecule can be a non-NOVX molecule or an NOVX protein or polypeptide of the invention. In one embodiment, an NOVX target molecule is a component of a signal transduction pathway that facilitates transduction of an extracellular signal (*e.g.* a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with an NOVX target molecule can be accomplished by one of the methods described above for determining direct binding. In one embodiment, determining the ability of the NOVX protein to bind to or interact with an NOVX target molecule can be accomplished by determining the activity of the target molecule. For example, the activity of the target molecule can be determined by detecting induction of a cellular second messenger of the target (*i.e.* intracellular Ca²⁺, diacylglycerol, IP₃, etc.), detecting catalytic/enzymatic activity of the target an appropriate substrate, detecting the induction of a reporter gene (comprising an NOVX-responsive regulatory element operatively linked to a nucleic acid encoding a detectable marker, *e.g.,* luciferase), or detecting a cellular response, for example, cell survival, cellular differentiation, or cell proliferation.

In yet another embodiment, an assay of the invention is a cell-free assay comprising contacting an NOVX protein or biologically-active portion thereof with a test compound and determining the ability of the test compound to bind to the NOVX protein or biologically-active portion thereof. Binding of the test compound to the NOVX protein can be determined either directly or indirectly as described above. In one such embodiment, the assay comprises contacting the NOVX protein or biologically-active portion thereof with a known compound which binds NOVX to form an assay mixture, contacting the assay mixture with a test compound, and determining the ability of the test compound to interact with an NOVX protein, wherein determining the ability of the test compound to interact with an NOVX protein comprises determining the ability of the test compound to preferentially bind to NOVX or biologically-active portion thereof as compared to the known compound.

In still another embodiment, an assay is a cell-free assay comprising contacting NOVX protein or biologically-active portion thereof with a test compound and determining the ability of the test compound to modulate (*e.g.* stimulate or inhibit) the activity of the NOVX protein or biologically-active portion thereof. Determining the ability of the test compound to modulate the activity of NOVX can be accomplished, for example, by determining the ability of the NOVX protein to bind to an NOVX target molecule by one of the methods described above for determining direct binding. In an alternative embodiment, determining the ability of the test compound to modulate the activity of NOVX protein can be accomplished by determining the ability of the NOVX protein further modulate an NOVX target molecule. For example, the catalytic/enzymatic activity of the target molecule on an appropriate substrate can be determined as described, *supra.*

In yet another embodiment, the cell-free assay comprises contacting the NOVX protein or biologically-active portion thereof with a known compound which binds NOVX protein to form an assay mixture, contacting the assay mixture with a test compound, and determining the ability of the test compound to interact with an NOVX protein, wherein determining the ability of the test compound to interact with an NOVX protein comprises determining the ability of the NOVX protein to preferentially bind to or modulate the activity of an NOVX target molecule.

The cell-free assays of the invention are amenable to use of both the soluble form or the membrane-bound form of NOVX protein. In the case of cell-free assays comprising the membrane-bound form of NOVX protein, it may be desirable to utilize a solubilizing agent such that the membrane-bound form of NOVX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, N-dodecyl--N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either NOVX protein or its target molecule to facilitate separation of complexed from uncomplexed forms of one or both of the proteins, as well as to accommodate automation of the assay. Binding of a test compound to NOVX protein, or interaction of NOVX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (*e.g.,* at physiological conditions for salt and pH). Following incubation, the beads or microtiter plate wells are washed to remove any unbound components, the matrix immobilized in the case of beads, complex determined either directly or indirectly, for example, as described, *supra.* Alternatively, the complexes can be dissociated from the matrix, and the level of NOVX protein binding or activity determined using standard techniques.

Other techniques for immobilizing proteins on matrices can also be used in the screening assays of the invention. For example, either the NOVX protein or its target molecule can be immobilized utilizing conjugation of biotin and streptavidin. Biotinylated NOVX protein or target molecules can be prepared from biotin-NHS (N-hydroxy-succinimide) using techniques well-known within the art (*e.g.*, biotinylation kit, Pierce Chemicals, Rockford, Ill.), and immobilized in the wells of streptavidin-coated 96 well plates (Pierce Chemical). Alternatively, antibodies reactive with NOVX protein or target molecules, but which do not interfere with binding of the NOVX protein to its target molecule, can be derivatized to the wells of the plate, and unbound target or NOVX protein trapped in the wells by antibody conjugation. Methods for detecting such complexes, in addition to those described above for the GST-immobilized complexes, include immunodetection of complexes using antibodies reactive with the NOVX protein or target molecule, as well as enzyme-linked assays that rely on detecting an enzymatic activity associated with the NOVX protein or target molecule.

In another embodiment, modulators of NOVX protein expression are identified in a method wherein a cell is contacted with a candidate compound and the expression of NOVX mRNA or protein in the cell is determined. The level of expression of NOVX mRNA or protein in the presence of the candidate compound is compared to the level of expression of NOVX mRNA or protein in the absence of the candidate compound. The candidate compound can then be identified as a modulator of NOVX mRNA or protein expression based upon this comparison. For example, when expression of NOVX mRNA or protein is greater (*i.e.,* statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay *(see, e.g.,* U.S. Patent No. 5,283,317; Zervos, et al., 1993. Cell 72: 223-232; Madura, et al., 1993. J. Biol. Chem. 268: 12046-12054; Bartel, et al., 1993. Biotechniques 14: 920-924; Iwabuchi, et al., 1993. Oncogene 8: 1693-1696; and Brent WO 94/10300), to identify other proteins that bind to or interact with NOVX ("NOVX-binding proteins" or "NOVX-bp") and modulate NOVX activity. Such NOVX-binding proteins are also likely to be involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (*e.g.*, GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming an NOVX-dependent complex, the DNA-binding and activation domains of the transcription factor are brought into close proximity. This proximity allows transcription of a reporter gene (*e*.*g*., LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with NOVX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) map their respective genes on a chromosome; and, thus, locate gene regions associated with genetic disease; (*ii*) identify an individual from a minute biological sample (tissue typing); and (*iii*) aid in forensic identification of a biological sample. Some of these applications are described in the subsections, below.

### Chromosome Mapping

Once the sequence (or a portion of the sequence) of a gene has been isolated, this sequence can be used to map the location of the gene on a chromosome. This process is called chromosome mapping. Accordingly, portions or fragments of the NOVX sequences, SEQ ID NOS:1, 3, 5, 7, 9, 12, 14, 16, 18, 20, 22, 24, 26, 28, 30, 32, 34, and 36, or fragments or derivatives thereof, can be used to map the location of the NOVX genes, respectively, on a chromosome. The mapping of the NOVX sequences to chromosomes is an important first step in correlating these sequences with genes associated with disease.

Briefly, NOVX genes can be mapped to chromosomes by preparing PCR primers (preferably 15-25 bp in length) from the NOVX sequences. Computer analysis of the NOVX, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the NOVX sequences will yield an amplified fragment.

Somatic cell hybrids are prepared by fusing somatic cells from different mammals (*e.g.*, human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. *See, e.g.,* D'Eustachio, et al., 1983. Science 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the NOVX sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases.
However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, *see,* Verma, et al., HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, *e.g.,* in McKusick, MENDELIAN INHERITANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e*.*g*., Egeland, et al., 1987. Nature, 325: 783-787.

Moreover, differences in the DNA sequences between individuals affected and unaffected with a disease associated with the NOVX gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

The NOVX sequences of the invention can also be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the NOVX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If predicted coding sequences, such as those in SEQ ID NOS:1, 3, 5, 7, 9, 12, 14, 16, 18, 20, 22, 24, 26, 28, 30, 32, 34, and 36 are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining NOVX protein and/or nucleic acid expression as well as NOVX activity, in the context of a biological sample (*e.g.*, blood, serum, cells, tissue) to thereby determine whether an individual is afflicted with a disease or disorder, or is at risk of developing a disorder, associated with aberrant NOVX expression or activity. The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers. The invention also provides for prognostic (or predictive) assays for determining whether an individual is at risk of developing a disorder associated with NOVX protein, nucleic acid expression or activity. For example, mutations in an NOVX gene can be assayed in a biological sample. Such assays can be used for prognostic or predictive purpose to thereby prophylactically treat an individual prior to the onset of a disorder characterized by or associated with NOVX protein, nucleic acid expression, or biological activity.

Another aspect of the invention provides methods for determining NOVX protein, nucleic acid expression or activity in an individual to thereby select appropriate therapeutic or prophylactic agents for that individual (referred to herein as "pharmacogenomics"). Pharmacogenomics allows for the selection of agents (*e.g.,* drugs) for therapeutic or prophylactic treatment of an individual based on the genotype of the individual (*e.g.*, the genotype of the individual examined to determine the ability of the individual to respond to a particular agent.)

Yet another aspect of the invention pertains to monitoring the influence of agents (*e.g.*, drugs, compounds) on the expression or activity of NOVX in clinical trials.

These and other agents are described in further detail in the following sections.

### Diagnostic Assays

An exemplary method for detecting the presence or absence of NOVX in a biological sample involves obtaining a biological sample from a test subject and contacting the biological sample with a compound or an agent capable of detecting NOVX protein or nucleic acid (*e.g.,* mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NOS:1, 3, 5, 7, 9, 12, 14, 16, 18, 20, 22, 24, 26, 28, 30, 32, 34, and 36, or a portion thereof, such as an oligonucleotide of at least 15, 30, 50, 100, 250 or 500 nucleotides in length and sufficient to specifically hybridize under stringent conditions to NOVX mRNA or genomic DNA. Other suitable probes for use in the diagnostic assays of the invention are described herein.

An agent for detecting NOVX protein is an antibody capable of binding to NOVX protein, preferably an antibody with a detectable label. Antibodies can be polyclonal, or more preferably, monoclonal. An intact antibody, or a fragment thereof (*e.g.,* Fab or F(ab')₂) can be used. The term "labeled", with regard to the probe or antibody, is intended to encompass direct labeling of the probe or antibody by coupling (*i.e.,* physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo.* For example, *in vitro* techniques for detection of NOVX mRNA include Northern hybridizations and *in situ* hybridizations. *In vitro* techniques for detection of NOVX protein include enzyme linked immunosorbent assays (ELISAs), Western blots, immunoprecipitations, and immunofluorescence. *In vitro* techniques for detection of NOVX genomic DNA include Southern hybridizations. Furthermore, *in vivo* techniques for detection of NOVX protein include introducing into a subject a labeled anti-NOVX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence of NOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

### Prognostic Assays

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity. Alternatively, the prognostic assays can be utilized to identify a subject having or at risk for developing a disease or disorder. Thus, the invention provides a method for identifying a disease or disorder associated with aberrant NOVX expression or activity in which a test sample is obtained from a subject and NOVX protein or nucleic acid (*e.g.*, mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (*e.g.*, serum), cell sample, or tissue.

Furthermore, the prognostic assays described herein can be used to determine whether a subject can be administered an agent (*e.g.*, an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder. Thus, the invention provides methods for determining whether a subject can be effectively treated with an agent for a disorder associated with aberrant NOVX expression or activity in which a test sample is obtained and NOVX protein or nucleic acid is detected (*e.g.*, wherein the presence of NOVX protein or nucleic acid is diagnostic for a subject that can be administered the agent to treat a disorder associated with aberrant NOVX expression or activity).

The methods of the invention can also be used to detect genetic lesions in an NOVX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding an NOVX-protein, or the misexpression of the NOVX gene. For example, such genetic lesions can be detected by ascertaining the existence of at least one of: (*i*) a deletion of one or more nucleotides from an NOVX gene; (*ii*) an addition of one or more nucleotides to an NOVX gene; (*iii*) a substitution of one or more nucleotides of an NOVX gene, (*iv*) a chromosomal rearrangement of an NOVX gene; (*v*) an alteration in the level of a messenger RNA transcript of an NOVX gene, (*vi*) aberrant modification of an NOVX gene, such as of the methylation pattern of the genomic DNA, (*vii*) the presence of a non-wild-type splicing pattern of a messenger RNA transcript of an NOVX gene, *(viii)* a non-wild-type level of an NOVX protein, (*ix*) allelic loss of an NOVX gene, and (*x*) inappropriate post-translational modification of an NOVX protein. As described herein, there are a large number of assay techniques known in the art which can be used for detecting lesions in an NOVX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) *(see, e.g.,* U.S. Patent Nos. 4,683,195 and 4,683,202), such as anchor PCR or RACE PCR, or, alternatively, in a ligation chain reaction (LCR) *(see, e.g.,* Landegran, et al., 1988. Science 241: 1077-1080; and Nakazawa, et al., 1994. Proc. Natl. Acad. Sci. USA 91: 360-364), the latter of which can be particularly useful for detecting point mutations in the NOVX-gene *(see,* Abravaya, et al., 1995. Nucl. Acids Res. 23: 675-682). This method can include the steps of collecting a sample of cells from a patient, isolating nucleic acid (*e.g.,* genomic, mRNA or both) from the cells of the sample, contacting the nucleic acid sample with one or more primers that specifically hybridize to an NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication *(see,* Guatelli, et al., 1990. Proc. Natl. Acad. Sci. USA 87: 1874-1878), transcriptional amplification system *(see,* Kwoh, et al., 1989. Proc. Natl. Acad. Sci. USA 86: 1173-1177); Qβ Replicase *(see,* Lizardi, et al, 1988. BioTechnology 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in an NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes *(see, e.g.,* U.S. Patent No. 5,493,531) can be used to score for the presence of specific mutations by development or loss of a ribozyme cleavage site.

In other embodiments, genetic mutations in NOVX can be identified by hybridizing a sample and control nucleic acids, *e.g.,* DNA or RNA, to high-density arrays containing hundreds or thousands of oligonucleotides probes. *See, e.g.,* Cronin, et al., 1996. Human Mutation 7: 244-255; Kozal, et al., 1996. Nat. Med. 2: 753-759. For example, genetic mutations in NOVX can be identified in two dimensional arrays containing light-generated DNA probes as described in Cronin, *et al., supra.* Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert, 1977. Proc. Natl. Acad. Sci. USA 74: 560 or Sanger, 1977. Proc. Natl. Acad. Sci. USA 74: 5463. It is also contemplated that any of a variety of automated sequencing procedures can be utilized when performing the diagnostic assays *(see, e.g.,* Naeve, et al., 1995. Biotechniques 19: 448), including sequencing by mass spectrometry (see, *e.g.,* PCT International Publication No. WO 94/16101; Cohen, et al., 1996. Adv. Chromatography 36: 127-162; and Griffin, et al., 1993. Appl. Biochem. Biotechnol. 38: 147-159).

Other methods for detecting mutations in the NOVX gene include methods in which protection from cleavage agents is used to detect mismatched bases in RNA/RNA or RNA/DNA heteroduplexes. *See, e.g.,* Myers, et al., 1985. Science 230: 1242. In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g.,* Cotton, et al., 1988. Proc. Natl. Acad. Sci. USA 85: 4397; Saleeba, et al., 1992. Methods Enzymol. 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E*. *coli* cleaves A at G/A mismatches and the thymidine DNA glycosylase from HeLa cells cleaves T at G/T mismatches. *See, e.g.,* Hsu, et al., 1994. Carcinogenesis 15: 1657-1662. According to an exemplary embodiment, a probe based on an NOVX sequence, *e.g*., a wild-type NOVX sequence, is hybridized to a cDNA or other DNA product from a test cell(s). The duplex is treated with a DNA mismatch repair enzyme, and the cleavage products, if any, can be detected from electrophoresis protocols or the like*. See, e.g.,* U.S. Patent No. 5,459,039.

In other embodiments, alterations in electrophoretic mobility will be used to identify mutations in NOVX genes. For example, single strand conformation polymorphism (SSCP) may be used to detect differences in electrophoretic mobility between mutant and wild type nucleic acids. *See, e.g.,* Orita, et al., 1989. Proc. Natl. Acad. Sci. USA: 86: 2766; Cotton, 1993. Mutat. Res. 285: 125-144; Hayashi, 1992. Genet. Anal. Tech. Appl. 9: 73-79. Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e.g.,* Keen, et al., 1991. Trends Genet. 7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, et al., 1985. Nature 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA*. See, e.g.,* Rosenbaum and Reissner, 1987. Biophys. Chem. 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found*. See, e.g.,* Saiki, et al., 1986. Nature 324: 163; Saiki, et al., 1989. Proc. Natl. Acad. Sci. USA 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e.g.,* Gibbs, et al., 1989. Nucl. Acids Res. 17: 2437-2448) or at the extreme 3'-terminus of one primer where, under appropriate conditions, mismatch can prevent, or reduce polymerase extension *(see, e.g.,* Prossner, 1993. Tibtech. 11: 238). In addition it may be desirable to introduce a novel restriction site in the region of the mutation to create cleavage-based detection. *See, e.g.,* Gasparini, et al., 1992. Mol. Cell Probes 6: 1. It is anticipated that in certain embodiments amplification may also be performed using *Taq* ligase for amplification. *See, e.g.,* Barany, 1991. Proc. Natl. Acad. Sci. USA 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e.g.,* in clinical settings to diagnose patients exhibiting symptoms or family history of a disease or illness involving an NOVX gene.

Furthermore, any cell type or tissue, preferably peripheral blood leukocytes, in which NOVX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on NOVX activity (*e.g.*, NOVX gene expression), as identified by a screening assay described herein can be administered to individuals to treat (prophylactically or therapeutically) disorders (The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers.) In conjunction with such treatment, the pharmacogenomics (*i.e*., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e.g.*, drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content of NOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g*., Eichelbaum, 1996. Clin. Exp. Pharmacol. Physiol., 23: 983-985; Linder, 1997. Clin. Chem., 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g.,* N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with an NOVX modulator, such as a modulator identified by one of the exemplary screening assays described herein.

### Monitoring of Effects During Clinical Trials

Monitoring the influence of agents (*e.g.*, drugs, compounds) on the expression or activity of NOVX (*e.g.*, the ability to modulate aberrant cell proliferation and/or differentiation) can be applied not only in basic drug screening, but also in clinical trials. For example, the effectiveness of an agent determined by a screening assay as described herein to increase NOVX gene expression, protein levels, or upregulate NOVX activity, can be monitored in clinical trails of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trails of subjects exhibiting increased NOVX gene expression, protein levels, or upregulated NOVX activity. In such clinical trials, the expression or activity of NOVX and, preferably, other genes that have been implicated in, for example, a cellular proliferation or immune disorder can be used as a "read out" or markers of the immune responsiveness of a particular cell.

By way of example, and not of limitation, genes, including NOVX, that are modulated in cells by treatment with an agent (*e.g.,* compound, drug or small molecule) that modulates NOVX activity (*e.g.,* identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression (*i.e.,* a gene expression pattern) can be quantified by Northern blot analysis or RT-PCR, as described herein, or alternatively by measuring the amount of protein produced, by one of the methods as described herein, or by measuring the levels of activity of NOVX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e.g.*, an agonist, antagonist, protein, peptide, peptidomimetic, nucleic acid, small molecule, or other drug candidate identified by the screening assays described herein) comprising the steps of (i) obtaining a pre-administration sample from a subject prior to administration of the agent; (*ii*) detecting the level of expression of an NOVX protein, mRNA, or genomic DNA in the preadministration sample; (*iii*) obtaining one or more post-administration samples from the subject; (*iv*) detecting the level of expression or activity of the NOVX protein, mRNA, or genomic DNA in the post-administration samples; (*v*) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity of NOVX to higher levels than detected, i.e., to increase the effectiveness of the agent. Alternatively, decreased administration of the agent may be desirable to decrease expression or activity of NOVX to lower levels than detected, i.e., to decrease the effectiveness of the agent.

### Methods of Treatment

The invention provides for both prophylactic and therapeutic methods of treating a subject at risk of (or susceptible to) a disorder or having a disorder associated with aberrant NOVX expression or activity. The disorders include cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, adrenoleukodystrophy, congenital adrenal hyperplasia, prostate cancer, neoplasm; adenocarcinoma, lymphoma, uterus cancer, fertility, hemophilia, hypercoagulation, idiopathic thrombocytopenic purpura, immunodeficiencies, graft versus host disease, AIDS, bronchial asthma, Crohn's disease; multiple sclerosis, treatment of Albright Hereditary Ostoeodystrophy, and other diseases, disorders and conditions of the like.

These methods of treatment will be discussed more fully, below.

### Disease and Disorders

Diseases and disorders that are characterized by increased (relative to a subject not suffering from the disease or disorder) levels or biological activity may be treated with Therapeutics that antagonize *(i.e.,* reduce or inhibit) activity. Therapeutics that antagonize activity may be administered in a therapeutic or prophylactic manner. Therapeutics that may be utilized include, but are not limited to: (i) an aforementioned peptide, or analogs, derivatives, fragments or homologs thereof; (*ii*) antibodies to an aforementioned peptide; (*iii*) nucleic acids encoding an aforementioned peptide; (*iv*) administration of antisense nucleic acid and nucleic acids that are "dysfunctional" (*i.e.,* due to a heterologous insertion within the coding sequences of coding sequences to an aforementioned peptide) that are utilized to "knockout" endogenous function of an aforementioned peptide by homologous recombination *(see, e.g.,* Capecchi, 1989. Science 244: 1288-1292); or (v) modulators (*i.e*., inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase (*i.e*., are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e.g.*, from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e.g*., by Western blot analysis, immunoprecipitation followed by sodium dodecyl sulfate (SDS) polyacrylamide gel electrophoresis, immunocytochemistry, etc.) and/or hybridization assays to detect expression of mRNAs (*e.g*., Northern assays, dot blots, *in situ* hybridization, and the like).

### Prophylactic Methods

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending upon the type of NOVX aberrancy, for example, an NOVX agonist or NOVX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of an NOVX protein, a peptide, an NOVX peptidomimetic, or other small molecule. In one embodiment, the agent stimulates one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and anti-NOVX antibodies. These modulatory methods can be performed *in vitro* (*e.g.,* by culturing the cell with the agent) or, alternatively, *in vivo (e.g.,* by administering the agent to a subject). As such, the invention provides methods of treating an individual afflicted with a disease or disorder characterized by aberrant expression or activity of an NOVX protein or nucleic acid molecule. In one embodiment, the method involves administering an agent (*e.g.*, an agent identified by a screening assay described herein), or combination of agents that modulates (*e.g*., up-regulates or down-regulates) NOVX expression or activity. In another embodiment, the method involves administering an NOVX protein or nucleic acid molecule as therapy to compensate for reduced or aberrant NOVX expression or activity.

Stimulation of NOVX activity is desirable *in* situations in which NOVX is abnormally downregulated and/or in which increased NOVX activity is likely to have a beneficial effect. One example of such a situation is where a subject has a disorder characterized by aberrant cell proliferation and/or differentiation (*e.g.*, cancer or immune associated disorders). Another example of such a situation is where the subject has a gestational disease (*e.g.*, preclainpsia).

### Determination of the Biological Effect of the Therapeutic

In various embodiments of the invention, suitable *in vitro* or *in vivo* assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Prophylactic and Therapeutic Uses of the Compositions of the Invention

The NOVX nucleic acids and proteins of the invention are useful in potential prophylactic and therapeutic applications implicated in a variety of disorders including, but not limited to: metabolic disorders, diabetes, obesity, infectious disease, anorexia, cancer-associated cancer, neurodegenerative disorders, Alzheimer's Disease, Parkinson's Disorder, immune disorders, hematopoietic disorders, and the various dyslipidemias, metabolic disturbances associated with obesity, the metabolic syndrome X and wasting disorders associated with chronic diseases and various cancers.

As an example, a cDNA encoding the NOVX protein of the invention may be useful in gene therapy, and the protein may be useful when administered to a subject in need thereof. By way of non-limiting example, the compositions of the invention will have efficacy for treatment of patients suffering from: metabolic disorders, diabetes, obesity, infectious disease, anorexia, cancer-associated cachexia, cancer, neurodegenerative disorders, Alzheimer's Disease, Parkinson's Disorder, immune disorders, hematopoietic disorders, and the various dyslipidemias.

Both the novel nucleic acid encoding the NOVX protein, and the NOVX protein of the invention, or fragments thereof, may also be useful in diagnostic applications, wherein the presence or amount of the nucleic acid or the protein are to be assessed. A further use could be as an anti-bacterial molecule (*i.e*., some peptides have been found to possess anti-bacterial properties). These materials are further useful in the generation of antibodies, which immunospecifically-bind to the novel substances of the invention for use in therapeutic or diagnostic methods.

The invention will be further described in the following examples, which do not limit the scope of the invention described in the claims.

### Examples

### Example 1. Identification of NOVX clones

The novel NOVX target sequences identified in the present invention were subjected to the exon linking process to confirm the sequence. PCR primers were designed by starting at the most upstream sequence available, for the forward primer, and at the most downstream sequence available for the reverse primer. Table 17A shows the sequences of the PCR primers used for obtaining different clones. In each case, the sequence was examined, walking inward from the respective termini toward the coding sequence, until a suitable sequence that is either unique or highly selective was encountered, or, in the case of the reverse primer, until the stop codon was reached. Such primers were designed based on in silico predictions for the full length cDNA, part (one or more exons) of the DNA or protein sequence of the target sequence, or by translated homology of the predicted exons to closely related human sequences from other species. These primers were then employed in PCR amplification based on the following pool of human cDNAs: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. Usually the resulting amplicons were gel purified, cloned and sequenced to high redundancy. The PCR product derived from exon linking was cloned into the pCR2.1 vector from Invitrogen. The resulting bacterial clone has an insert covering the entire open reading frame cloned into the pCR2.1 vector. Table 17B shows a list of these bacterial clones. The resulting sequences from all clones were assembled with themselves, with other fragments in CuraGen Corporation's database and with public ESTs. Fragments and ESTs were included as components for an assembly when the extent of their identity with another component of the assembly was at least 95% over 50 bp. In addition, sequence traces were evaluated manually and edited for corrections if appropriate. These procedures provide the sequence reported herein.

**Table 10A. PCR Primers for Exon Linking**

| **NOVX Clone** | **Primer 1 (5' - 3')** | **SEQ ID NO** | **Primer 2 (5' - 3')** | **SEQ ID NO** |
|---|---|---|---|---|
| NOV1 | TGTAAAACAAGACCAGGCACAAGAAGG | 103 | CTCACAGAGAAAGAGACAGGAAAGTAAATGC | 104 |
| NOV3b | GGATCCCGGCTGCCCTTCCAGTGCTCTGCCA GCTACCTGG | 137 | CTCGAGGCTCAGCTCCATGAGCACG GCCACATTGCC | 138 |
| NOV5a | AGTTCCAGTGTGGGGATGGGACAT | 105 | AATGTCATGTGGGTTGTTGCAGGTTCT | 106 |
| NOV5b | AGTTCCAGTGTGGGGATGGGACAT | 107 | AATGTCATGTGGGTTGTTGCAGGTTCT | 108 |
| NOV5c | AGTTCCAGTGTGGGGATGGGACAT | 109 | AATGTCATGTGGGTTGTTGCAGGTTCT | 110 |
| NOV5d | AGTTCCAGTGTGGGGATGGGACAT | 111 | ACTAACTGAGCTTGGGACTGCAGCTGT | 112 |
| NOV5e | GCGGCTGATCCGCTGCTC | 113 | GGTGATCCCATCCTCAGGGTAGTC | 114 |
| NOV5f | GCGGCTGATCCGCTGCTC | 115 | GGTGATCCCATCCTCAGGGTAGTC | 116 |
| NOV5g | AGTTCCAGTGTGGGGATGGGACAT | 117 | ACTAACTGAGCTTGGGACTGCAGCTGT | 118 |
| NOV5h | AGTTCCAGTGTGGGGATGGGACAT | 119 | ACTAACTGAGCTTGGGACTGCAGCTGT | 120 |
| NOV5i | GGTCCCCGCAGATTTCAGTGTAAGAGT | 121 | ACTAACTGAGCTTGGGACTGCAGCTGT | 122 |

Physical clone: Exons were predicted by homology and the intron/exon boundaries were determined using standard genetic rules. Exons were further selected and refined by means of similarity determination using multiple BLAST (for example, tBlastN, BlastX, and BlastN) searches, and, in some instances, GeneScan and Grail. Expressed sequences from both public and proprietary databases were also added when available to further define and complete the gene sequence. The DNA sequence was then manually corrected for apparent inconsistencies thereby obtaining the sequences encoding the full-length protein.

**Table 10B. Physical Clones for PCR products**

| **NOVX Clone** | **Bacterial Clone** |
|---|---|
| NOV1 | Physical clone: hRPK.474 N 24, 211,945 bp |
| NOV2a | Physical clone: RP11-60L3, 172,244 bp |
| NOV3 | Physical clone: AP000501 |
| NOV4 | Physical clone: ba56p10.698056.K10 FLC EL |
| NOV5a-c | Physical clone: RP4-784A16, 139,557 bp |
| NOV5d-i | Physical clone: sggc draft dj784a16 20000723 da3 |
| NOV6 | Physical clone: AL031711.25 |
| NOV7 | Genomic file: 10d7 |
| NOV8 | Genomic file: ba550p23 |

### Example 2. Quantitative expression analysis of clones in various cells and tissues

The quantitative expression of various clones was assessed using microtiter plates containing RNA samples from a variety of normal and pathology-derived cells, cell lines and tissues using real time quantitative PCR (RTQ PCR). RTQ PCR was performed on a Perkin-Elmer Biosystems ABI PRISM® 7700 Sequence Detection System. Various collections of samples are assembled on the plates, and referred to as Panel 1 (containing cells and cell lines from normal and cancer sources), Panel 2 (containing samples derived from tissues, in particular from surgical samples, from normal and cancer sources), Panel 3 (containing samples derived from a wide variety of cancer sources), Panel 4 (containing cells and cell lines from normal cells and cells related to inflammatory conditions) and Panel CNSD.01 (containing samples from normal and diseased brains).

First, the RNA samples were normalized to reference nucleic acids such as constitutively expressed genes (for example, β-actin and GAPDH). Normalized RNA (5 ul) was converted to cDNA and analyzed by RTQ-PCR using One Step RT-PCR Master Mix Reagents (PE Biosystems; Catalog No. 4309169) and gene-specific primers according to the manufacturer's instructions. Probes and primers were designed for each assay according to Perkin Elmer Biosystem's *Primer Express* Software package (version I for Apple Computer's Macintosh Power PC) or a similar algorithm using the target sequence as input. Default settings were used for reaction conditions and the following parameters were set before selecting primers: primer concentration = 250 nM, primer melting temperature (Tₘ) range = 58°-60° C, primer optimal Tm = 59° C, maximum primer difference = 2° C, probe does not have 5' G, probe Tₘ must be 10° C greater than primer Tₘ, amplicon size 75 bp to 100 bp. The probes and primers selected (see below) were synthesized by Synthegen (Houston, TX, USA). Probes were double purified by HPLC to remove uncoupled dye and evaluated by mass spectroscopy to verify coupling of reporter and quencher dyes to the 5' and 3' ends of the probe, respectively. Their final concentrations were: forward and reverse primers, 900 nM each, and probe, 200nM.

PCR conditions: Normalized RNA from each tissue and each cell line was spotted in each well of a 96 well PCR plate (Perkin Elmer Biosystems). PCR cocktails including two probes (a probe specific for the target clone and another gene-specific probe multiplexed with the target probe) were set up using 1X TaqMan™ PCR Master Mix for the PE Biosystems 7700, with 5 mM MgCl2, dNTPs (dA, G, C, U at 1:1:1:2 ratios), 0.25 U/ml AmpliTaq Gold^{™} (PE Biosystems), and 0.4 U/µl RNase inhibitor, and 0.25 U/µl reverse transcriptase. Reverse transcription was performed at 48° C for 30 minutes followed by amplification/PCR cycles as follows: 95° C 10 min, then 40 cycles of 95° C for 15 seconds, 60° C for 1 minute. Results were recorded as CT values (cycle at which a given sample crosses a threshold level of fluorescence) using a log scale, with the difference in RNA concentration between a given sample and the sample with the lowest CT value being represented as 2 to the power of delta CT. The percent relative expression is then obtained by taking the reciprocal of this RNA difference and multiplying by 100.

In the results for Panel 1, the following abbreviations are used:
ca. = carcinoma,
* = established from metastasis,
met = metastasis,
s cell var = small cell variant,
non-s = non-sm = non-small,
squam = squamous,
pl. eff = pl effusion = pleural effusion,
glio = glioma,
astro = astrocytoma, and
neuro = neuroblastoma.

### Panel 2

The plates for Panel 2 generally include 2 control wells and 94 test samples composed of RNA or cDNA isolated from human tissue procured by surgeons working in close cooperation with the National Cancer Institute's Cooperative Human Tissue Network (CHTN) or the National Disease Research Initiative (NDRI). The tissues are derived from human malignancies and in cases where indicated many malignant tissues have "matched margins" obtained from noncancerous tissue just adjacent to the tumor. These are termed normal adjacent tissues and are denoted "NAT" in the results below. The tumor tissue and the "matched margins" are evaluated by two independent pathologists (the surgical pathologists and again by a pathologists at NDRI or CHTN). This analysis provides a gross histopathological assessment of tumor differentiation grade. Moreover, most samples include the original surgical pathology report that provides information regarding the clinical stage of the patient. These matched margins are taken from the tissue surrounding (i.e. immediately proximal) to the zone of surgery (designated "NAT", for normal adjacent tissue, in Table RR). In addition, RNA and cDNA samples were obtained from various human tissues derived from autopsies performed on elderly people or sudden death victims (accidents, etc.). These tissues were ascertained to be free of disease and were purchased from various commercial sources such as Clontech (Palo Alto, CA), Research Genetics, and Invitrogen.

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

### Panel 3D

The plates of Panel 3D are comprised of 94 cDNA samples and two control samples. Specifically, 92 of these samples are derived from cultured human cancer cell lines, 2 samples of human primary cerebellar tissue and 2 controls. The human cell lines are generally obtained from ATCC (American Type Culture Collection), NCI or the German tumor cell bank and fall into the following tissue groups: Squamous cell carcinoma of the tongue, breast cancer, prostate cancer, melanoma, epidermoid carcinoma, sarcomas, bladder carcinomas, pancreatic cancers, kidney cancers, leukemias/lymphomas, ovarian/uterine/cervical, gastric, colon, lung and CNS cancer cell lines. In addition, there are two independent samples of cerebellum. These cells are all cultured under standard recommended conditions and RNA extracted using the standard procedures. The cell lines in panel 3D and 1.3D are of the most common cell lines used in the scientific literature.

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

### Panel 4

Panel 4 includes samples on a 96 well plate (2 control wells, 94 test samples) composed of RNA (Panel 4r) or cDNA (Panel 4d) isolated from various human cell lines or tissues related to inflammatory conditions. Total RNA from control normal tissues such as colon and lung (Stratagene ,La Jolla, CA) and thymus and kidney (Clontech) were employed. Total RNA from liver tissue from cirrhosis patients and kidney from lupus patients was obtained from BioChain (Biochain Institute, Inc., Hayward, CA). Intestinal tissue for RNA preparation from patients diagnosed as having Crohn's disease and ulcerative colitis was obtained from the National Disease Research Interchange (NDRI) (Philadelphia, PA).

Astrocytes, lung fibroblasts, dermal fibroblasts, coronary artery smooth muscle cells, small airway epithelium, bronchial epithelium, microvascular dermal endothelial cells, microvascular lung endothelial cells, human pulmonary aortic endothelial cells, human umbilical vein endothelial cells were all purchased from Clonetics (Walkersville, MD) and grown in the media supplied for these cell types by Clonetics. These primary cell types were activated with various cytokines or combinations of cytokines for 6 and/or 12-14 hours, as indicated. The following cytokines were used; IL-1 beta at approximately 1-5 ng/ml, TNF alpha at approximately 5-10 ng/ml, IFN gamma at approximately 20-50 ng/ml, IL-4 at approximately 5-10 ng/ml, IL-9 at approximately 5-10 ng/ml, IL-13 at approximately 5-10 ng/ml. Endothelial cells were sometimes starved for various times by culture in the basal media from Clonetics with 0.1 % serum.

Mononuclear cells were prepared from blood of employees at CuraGen Corporation, using Ficoll. LAK cells were prepared from these cells by culture in DMEM 5% FCS (Hyclone), 100 µM non essential amino acids (Gibco/Life Technologies, Rockville, MD), 1 mM sodium pyruvate (Gibco), mercaptoethanol 5.5 x 10⁻⁵ M (Gibco), and 10 mM Hepes (Gibco) and Interleukin 2 for 4-6 days. Cells were then either activated with 10-20 ng/ml PMA and 1-2 µg/ml ionomycin, IL-12 at 5-10 ng/ml, IFN gamma at 20-50 ng/ml and IL-18 at 5-10 ng/ml for 6 hours. In some cases, mononuclear cells were cultured for 4-5 days in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco) with PHA (phytohemagglutinin) or PWM (pokeweed mitogen) at approximately 5 µg/ml. Samples were taken at 24, 48 and 72 hours for RNA preparation. MLR (mixed lymphocyte reaction) samples were obtained by taking blood from two donors, isolating the mononuclear cells using Ficoll and mixing the isolated mononuclear cells 1:1 at a final concentration of approximately 2x10⁶ cells/ml in DMEM 5% FCS (Hyclone), 100 µM non essential amino acids (Gibco), 1 mM sodium pyruvate (Gibco), mercaptoethanol (5.5 x 10⁻⁵ M) (Gibco), and 10 mM Hepes (Gibco). The MLR was cultured and samples taken at various time points ranging from 1- 7 days for RNA preparation.

Monocytes were isolated from mononuclear cells using CD14 Miltenyi Beads, +ve VS selection columns and a Vario Magnet according to the manufacturer's instructions. Monocytes were differentiated into dendritic cells by culture in DMEM 5% fetal calf serum (FCS) (Hyclone, Logan, UT), 100 µM non essential amino acids (Gibco), 1 mM sodium pyruvate (Gibco), mercaptoethanol 5.5 x 10⁻⁵ M (Gibco), and 10 mM Hepes (Gibco), 50 ng/ml GMCSF and 5 ng/ml IL-4 for 5-7 days. Macrophages were prepared by culture of monocytes for 5-7 days in DMEM 5% FCS (Hyclone), 100 µM non essential amino acids (Gibco), 1 mM sodium pyruvate (Gibco), mercaptoethanol 5.5 x 10⁻⁵ M (Gibco), 10 mM Hepes (Gibco) and 10% AB Human Serum or MCSF at approximately 50 ng/ml. Monocytes, macrophages and dendritic cells were stimulated for 6 and 12-14 hours with lipopolysaccharide (LPS) at 100 ng/ml. Dendritic cells were also stimulated with anti-CD40 monoclonal antibody (Pharmingen) at 10 µg/ml for 6 and 12-14 hours.

CD4 lymphocytes, CD8 lymphocytes and NK cells were also isolated from mononuclear cells using CD4, CD8 and CD56 Miltenyi beads, positive VS selection columns and a Vario Magnet according to the manufacturer's instructions. CD45RA and CD45RO CD4 lymphocytes were isolated by depleting mononuclear cells of CD8, CD56, CD14 and CD19 cells using CD8, CD56, CD 14 and CD 19 Miltenyi beads and positive selection. Then CD45RO beads were used to isolate the CD45RO CD4 lymphocytes with the remaining cells being CD45RA CD4 lymphocytes. CD45RA CD4, CD45RO CD4 and CD8 lymphocytes were placed in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco) and plated at 10⁶ cells/ml onto Falcon 6 well tissue culture plates that had been coated overnight with 0.5 µg/ml anti-CD28 (Pharmingen) and 3 ug/ml anti-CD3 (OKT3, ATCC) in PBS. After 6 and 24 hours, the cells were harvested for RNA preparation. To prepare chronically activated CD8 lymphocytes, we activated the isolated CD8 lymphocytes for 4 days on anti-CD28 and anti-CD3 coated plates and then harvested the cells and expanded them in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco) and IL-2. The expanded CD8 cells were then activated again with plate bound anti-CD3 and anti-CD28 for 4 days and expanded as before. RNA was isolated 6 and 24 hours after the second activation and after 4 days of the second expansion culture. The isolated NK cells were cultured in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco) and IL-2 for 4-6 days before RNA was prepared.

To obtain B cells, tonsils were procured from NDRI. The tonsil was cut up with sterile dissecting scissors and then passed through a sieve. Tonsil cells were then spun down and resupended at 10⁶ cells/ml in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco). To activate the cells, we used PWM at 5 µg/ml or anti-CD40 (Pharmingen) at approximately 10 µg/ml and IL-4 at 5-10 ng/ml. Cells were harvested for RNA preparation at 24,48 and 72 hours.

To prepare the primary and secondary Th1/Th2 and Tr1 cells, six-well Falcon plates were coated overnight with 10 µg/ml anti-CD28 (Pharmingen) and 2 µg/ml OKT3 (ATCC), and then washed twice with PBS. Umbilical cord blood CD4 lymphocytes (Poietic Systems, German Town, MD) were cultured at 10⁵ -10⁶ cells/ml in DMEM 5% FCS (Hyclone), 100 µM non essential amino acids (Gibco), 1 mM sodium pyruvate (Gibco), mercaptoethanol 5.5 x 10⁻⁵M (Gibco), 10 mM Hepes (Gibco) and IL-2 (4 ng/ml). IL-12 (5 ng/ml) and anti-IL4 (1 □g/ml) were used to direct to Th1, while IL-4 (5 ng/ml) and anti-IFN gamma (1 □g/ml) were used to direct to Th2 and IL-10 at 5 ng/ml was used to direct to Tr1. After 4-5 days, the activated Th1, Th2 and Tr1 lymphocytes were washed once in DMEM and expanded for 4-7 days in DMEM 5% FCS (Hyclone), 100 µM non essential amino acids (Gibco), 1 mM sodium pyruvate (Gibco), mercaptoethanol 5.5 x 10⁻⁵ M (Gibco), 10 mM Hepes (Gibco) and IL-2 (1 ng/ml). Following this, the activated Th1, Th2 and Tr1 lymphocytes were re-stimulated for 5 days with anti-CD28/OKT3 and cytokines as described above, but with the addition of anti-CD95L (1 □g/ml) to prevent apoptosis. After 4-5 days, the Th1, Th2 and Tr1 lymphocytes were washed and then expanded again with IL-2 for 4-7 days. Activated Th1 and Th2 lymphocytes were maintained in this way for a maximum of three cycles. RNA was prepared from primary and secondary Th1, Th2 and Tr1 after 6 and 24 hours following the second and third activations with plate bound anti-CD3 and anti-CD28 mAbs and 4 days into the second and third expansion cultures in Interleukin 2.

The following leukocyte cells lines were obtained from the ATCC: Ramos, EOL-1, KU-812. EOL cells were further differentiated by culture in 0.1 mM dbcAMP at 5 x10⁵ cells/ml for 8 days, changing the media every 3 days and adjusting the cell concentration to 5 x10⁵ cells/ml. For the culture of these cells, we used DMEM or RPMI (as recommended by the ATCC), with the addition of 5% FCS (Hyclone), 100 µM non essential amino acids (Gibco), 1 mM sodium pyruvate (Gibco), mercaptoethanol 5.5 x 10⁻⁵ M (Gibco), 10 mM Hepes (Gibco). RNA was either prepared from resting cells or cells activated with PMA at 10 ng/ml and ionomycin at 1 µg/ml for 6 and 14 hours. Keratinocyte line CCD106 and an airway epithelial tumor line NCI-H292 were also obtained from the ATCC. Both were cultured in DMEM 5% FCS (Hyclone), 100 µM non essential amino acids (Gibco), 1 mM sodium pyruvate (Gibco), mercaptoethanol 5.5 x 10⁻⁵ M (Gibco), and 10 mM Hepes (Gibco). CCD1106 cells were activated for 6 and 14 hours with approximately 5 ng/ml TNF alpha and 1 ng/ml IL-1 beta, while NCI-H292 cells were activated for 6 and 14 hours with the following cytokines: 5 ng/ml IL-4, 5 ng/ml IL-9,5 ng/ml IL-13 and 25 ng/ml IFN gamma.

For these cell lines and blood cells, RNA was prepared by lysing approximately 10⁷ cells/ml using Trizol (Gibco BRL). Briefly, 1/10 volume of bromochloropropane (Molecular Research Corporation) was added to the RNA sample, vortexed and after 10 minutes at room temperature, the tubes were spun at 14,000 rpm in a Sorvall SS34 rotor. The aqueous phase was removed and placed in a 15 ml Falcon Tube. An equal volume of isopropanol was added and left at -20 degrees C overnight. The precipitated RNA was spun down at 9,000 rpm for 15 min in a Sorvall SS34 rotdr and washed in 70% ethanol. The pellet was redissolved in 300 µl of RNAse-free water and 35 µl buffer (Promega) 5 µl DTT, 7 µl RNAsin and 8 µl DNAse were added. The tube was incubated at 37 degrees C for 30 minutes to remove contaminating genomic DNA, extracted once with phenol chloroform and re-precipitated with 1/10 volume of 3 M sodium acetate and 2 volumes of 100% ethanol. The RNA was spun down and placed in RNAse free water. RNA was stored at -80 degrees C.

### Panel CNSD.01

The plates for Panel CNSD.01 include two control wells and 94 test samples comprised of cDNA isolated from postmortem human brain tissue obtained from the Harvard Brain Tissue Resource Center. Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains two brains from each of the following diagnoses: Alzheimer's disease, Parkinson's disease, Huntington's disease, Progressive Supernuclear Palsy, Depression, and "Normal controls". Within each of these brains, the following regions are represented: cingulate gyrus, temporal pole, globus palladus, substantia nigra, Brodman Area 4 (primary motor strip), Brodman Area 7 (parietal cortex), Brodman Area 9 (prefrontal cortex), and Brodman area 17 (occipital cortex). Not all brain regions are represented in all cases; e.g., Huntington's disease is characterized in part by neurodegeneration in the globus palladus, thus this region is impossible to obtain from confirmed Huntington's cases. Likewise Parkinson's disease is characterized by degeneration of the substantia nigra making this region more difficult to obtain. Normal control brains were examined for neuropathology and found to be free of any pathology consistent with neurodegeneration.

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

In the labels employed to identify tissues in the CNS panel, the following abbreviations are used:
**PSP = Progressive supranuclear palsy**
Sub Nigra = Substantia nigra
Glob Palladus= Globus palladus
Temp Pole = Temporal pole
Cing Gyr = Cingulate gyrus
BA 4 = Brodman Area 4

The AC068339_A gene encodes a G protein-coupled receptor (GPCR), a type of cell surface receptor involved in signal transduction. The AC068339_A gene product is most similar to members of the odorant receptor subfamily of GPCRs. Based on analogy to other odorant receptor genes, we predict that expression of the AC068339_A gene may be highest in nasal epithelium, a sample not represented on these panels.

### NOV1

Expression of gene AC013554_da1 was assessed using the primer-probe set Ag2025, described in Table 11. Results from RTQ-PCR runs are shown in Table 12.

**Table 11. Probe Name Ag2025**

| Primers | Sequences | TM | Length | Start Position | SEQ ID NO: |
|---|---|---|---|---|---|
| Forward | 5'-GTGAGGAGGTGGTGAGAGAGT-3' | 58.3 | 21 | 487 | 123 |
| Probe | FAM-5'- CCATCATGTGTGTCCCAACCTTTCAG- 3'-TAMRA | 69.5 | 26 | 511 | 124 |
| Reverse | 5'-GCAAAGTTCATCCACCTTTTCT- 3' | 59.6 | 22 | 551 | 125 |

**Table 12. Panel 1.3D**

| **Tissue Name** | **Relative Expression(%)** | **Tissue Name** | **Relative Expression(%)** |
|---|---|---|---|
| | **1.3dtm3204f_ ag2025** | | **1.3dtm3204f_ ag2025** |
| Liver adenocarcinoma | 1.1 | Kidney (fetal) | 0.0 |
| Pancreas | 0.0 | Renal ca. 786-0 | 0.0 |
| Pancreatic ca. CAPAN 2 | 0.0 | Renal ca. A498 | 0.5 |
| Adrenal gland | 0.0 | Renal ca. RXF 393 | 0.0 |
| Thyroid | 0.0 | Renal ca. ACHN | 0.0 |
| Salivary gland | 0.0 | Renal ca. UO-31 | 0.0 |
| Pituitary gland | 0.0 | Renal ca. TK-10 | 0.0 |
| Brain (fetal) | 0.2 | Liver | 0.0 |
| Brain (whole) | 0.0 | Liver (fetal) | 0.0 |
| Brain (amygdala) | 0.3 | Liver ca. (hepatoblast) HepG2 | 0.0 |
| Brain (cerebellum) | 0.0 | Lung | 0.8 |
| Brain (hippocampus) | 0.0 | Lung (fetal) | 0.0 |
| Brain (substantia nigra) | 0.0 | Lung ca. (small cell) LX-1 | 0.0 |
| Brain (thalamus) | 0.0 | Lung ca. (small cell) NCI-H69 | 4.2 |
| Cerebral Cortex | 0.0 | Lung ca. (s.cell var.) SHP-77 | 0.0 |
| Spinal cord | 0.5 | Lung ca. (large cell)NCI-H460 | 0.0 |
| CNS ca. (glio/astro) U87-MG | 0.5 | Lung ca. (non-sm. cell) A549 | 0.0 |
| CNS ca. (glio/astro) U-118-MG | 0.3 | Lung ca. (non-s.cell) NCI-H23 | 0.3 |
| CNS ca. (astro) SW1783 | 0.3 | Lung ca (non-s.cell) HOP-62 | 0.0 |
| CNS ca.* (neuro; met) SK-N-AS | 0.0 | Lung ca. (non-s.cl) NCI-H522 | 0.0 |
| CNS ca. (astro) SF-539 | 0.6 | Lung ca. (squam.) SW 900 | 0.0 |
| CNS ca. (astro) SNB-75 | 0.2 | Lung ca. (squam.) NCI-H596 | 0.6 |
| CNS ca. (glio) SNB-19 | 0.0 | Mammary gland | 0.0 |
| CNS ca. (glio) U251 | 0.2 | Breast ca.* (pl. effusion) MCF-7 | 0.0 |
| CNS ca. (glio) SF-295 | 0.0 | Breast ca.* (pl.ef) MDA-MB-231 | 1.0 |
| Heart (fetal) | 0.0 | Breast ca.* (pl. effusion) T47D | 0.3 |
| Heart | 0.4 | Breast ca. BT-549 | 0.0 |
| Fetal Skeletal | 0.0 | Breast ca. MDA-N | 0.0 |
| Skeletal muscle | 0.0 | Ovary | 0.0 |
| Bone marrow | 0.0 | Ovarian ca. OVCAR-3 | 0.0 |
| Thymus | 0.0 | Ovarian ca. OVCAR-4 | 0.0 |
| Spleen | 0.2 | Ovarian ca. OVCAR-5 | 0.0 |
| Lymph node | 0.0 | Ovarian ca. OVCAR-8 | 0.5 |
| Colorectal | 0.0 | Ovarian ca. IGROV-1 | 0.0 |
| Stomach | 0.3 | Ovarian ca.* (ascites) SK-OV-3 | 0.4 |
| Small intestine | 0.3 | Uterus | 0.3 |
| Colon ca. SW480 | 0.0 | Placenta | 0.0 |
| Colon ca.* (SW480 met)SW620 | 0.0 | Prostate | 0.0 |
| Colon ca. HT29 | 0.0 | Prostate ca.* (bone met)PC-3 | 0.0 |
| Colon ca. HCT-116 | 0.0 | Testis | 100.0 |
| Colon ca. CaCo-2 | 0.3 | Melanoma Hs688(A).T | 0.0 |
| 83219 CC Well to Mod Diff (OD03866) | 0.0 | Melanoma* (met) Hs688(B).T | 0.0 |
| Colon ca. HCC-2998 | 0.0 | Melanoma UACC-62 | 0.0 |
| Gastric ca.* (liver met) NCI-N87 | 0.0 | Melanoma M14 | 0.0 |
| Bladder | 0.0 | Melanoma LOX IMVI | 0.4 |
| Trachea | 0.0 | Melanoma* (met) SK-MEL-5 | 0.0 |
| Kidney | 0.0 | Adipose | 0.0 |

**Panel 1.3D Summary:** Ag2025 Expression of the NOV1 gene is detected exclusively in testis among the samples on this panel and the transcript is present at moderate levels (CT = 29.6). Therefore, this gene could be used to distinguish testis from the other tissues on this panel. The NOV1 gene encodes a protein with homology to thioredoxins, small ubiquitous proteins that participate in different cellular processes via redox-mediated reactions. An example of a thioredoxin that is expressed exclusively in spermatozoa has recently been reported (ref. 1). Therefore, the NOV1 gene product may also play a role in spermiogenesis and/or fertility.

**Panel 2.2 Summary:** Ag2025 Expression of the NOV1 gene is low/undetectable (CT values > 35) across the samples on this panel (data not shown).

**Panel 4D Summary**: Ag2025 Expression of the NOV1 gene is low/undetectable (CT values > 35) across the samples on this panel (data not shown).

### References:

### 1. Miranda-Vizuete A., Ljung J., Damdimopoulos A.E., Gustafsson J.A., Oko R., Pelto-Huikko M., Spyrou G. (2001) Characterization of Sptrx, a novel member of the thioredoxin family specifically expressed in human spermatozoa. J. Biol. Chem. Jun 8 [epub ahead of print].

Thioredoxins (Trx) are small ubiquitous proteins that participate in different cellular processes via redox-mediated reactions. We report here the identification and characterization of a novel member of the thioredoxin family in human, named Sptrx (sperm-specific trx), the first with a tissue-specific distribution, located exclusively in spermatozoa. Sptrx ORF encodes for a protein of 486 amino acids comprised of two clear domains: an N-terminal domain consisting of 23 highly conserved repetitions of a 15-residue motif and a C-terminal domain typical of thioredoxins. Northern analysis and in situ hybridization shows that Sptrx mRNA is only expressed in human testis, specifically in round and elongating spermatids. Immunostaining of human testis sections identified Sptrx protein in spermatids while immunofluorescence and immunogold electron microscopy analysis demonstrated Sptrx localization in the cytoplasmic droplet of ejaculated sperm. Sptrx appears to have a multimeric structure in native conditions and is able to reduce insulin disulfide bonds in the presence of NADPH and thioredoxin reductase. During mammalian spermiogenesis in testis seminiferous tubules and later maturation in epididymis, extensive reorganization of disulfide bonds is required to stabilize cytoskeletal sperm structures. However, the molecular mechanisms that control these processes are not known. The identification of Sptrx with an expression pattern restricted to the post meiotic phase of spermatogenesis, when sperm tail is organized, suggests that Sptrx might be an important factor in regulating critical steps of human spermiogenesis.

### NOV2a

Expression of the NOV2a gene was assessed using the primer-probe set Ag850, described in Table 13. Results from RTQ-PCR runs are shown in Tables 14 and 15.

**Table 13. Probe Name Ag850 (please note that there is single base mismatch within the forward primer that is not expected to disrupt primer binding)**

| Primers | Sequences | TM | Length | Start Position | SEQ ID NO: |
|---|---|---|---|---|---|
| Forward | 5'-CCTTTCTTCTCTTCCTCCTCAA-3' | 59.1 | 22 | 25 | 126 |
| Probe | FAM-5'- CACCTGGCGAGTGCTCCTCTCTG-3'- TAMRA | 70 | 23 | 71 | 127 |
| Reverse | 5'-GGTGGATGGCGTTGTAGAG-3' | 59.1 | 19 | 96 | 128 |

**Table 14. Panel 2.2**

| **Tissue Name** | **Relative Expression(%)** | **Tissue Name** | **Relative Expression(%)** |
|---|---|---|---|
| | **2.2x4tm6324f_ag850_a1** | | **2.2x4tm6324f_ag850_a1** |
| Normal Colon GENPAK 061003 | 0.8 | 83793 Kidney NAT (OD04348) | 19.5 |
| 97759 Colon cancer (OD06064) | 3.0 | 98938 Kidney malignant cancer (OD06204B) | 3.0 |
| 97760 Colon cancer NAT (OD06064) | 0.0 | 98939 Kidney normal adjacent tissue (OD06204E) | 12.8 |
| 97778 Colon cancer (OD06159) | 2.1 | 85973 Kidney Cancer (OD04450-01) | 0.0 |
| 97779 Colon cancer NAT (OD06159) | 0.5 | 85974 Kidney NAT (OD04450-03) | 7.8 |
| 98861 Colon cancer (OD06297- 04) | 0.2 | Kidney Cancer Clontech 8120613 | 0.4 |
| 98862 Colon cancer NAT (OD06297-015) | 0.3 | Kidney NAT Clontech 8120614 | **100.0** |
| 83237 CC Gr.2 ascend colon (ODO3921) | 0.4 | Kidney Cancer Clontech 9010320 | 1.2 |
| 83238 CC NAT (ODO3921) | 0.3 | Kidney NAT Clontech 9010321 | 11.9 |
| 97766 Colon cancer metastasis (OD06104) | 3.3 | Kidney Cancer Clontech 8120607 | 0.5 |
| 97767 Lung NAT (OD06104) | 0.0 | Kidney NAT Clontech 8120608 | 28.9 |
| 87472 Colon mets to lung (OD04451-01) | 0.0 | Normal Uterus GENPAK 061018 | 2.8 |
| 87473 Lung NAT (OD04451- 02) | 0.0 | Uterus Cancer GENPAK 064011 | 6.2 |
| Normal Prostate Clontech A+ 6546-1 (8090438) | 7.7 | Normal Thyroid Clontech A+ 6570-1 (7080817) | 0.0 |
| 84140 Prostate Cancer (OD04410) | 1.3 | Thyroid Cancer GENPAK 064010 | 5.1 |
| 84141 Prostate NAT (OD04410) | 6.6 | Thyroid Cancer INVITROGEN A302152 | 25.0 |
| Normal Ovary Res. Gen. | 2.4 | Thyroid NAT INVITROGEN A302153 | 0.8 |
| 98863 Ovarian cancer (OD06283-03) | 4.7 | Normal Breast GENPAK 061019 | 7.1 |
| 98865 Ovarian cancer NAT/fallopian tube (OD06283-07) | 0.5 | 84877 Breast Cancer (OD04566) | 1.4 |
| Ovarian Cancer GENPAK 064008 | 5.2 | Breast Cancer Res. Gen. 1024 | 6.2 |
| 97773 Ovarian cancer (OD06145) | 3.1 | 85975 Breast Cancer (OD04590-01) | 12.9 |
| 97775 Ovarian cancer NAT (OD06145) | 4.0 | 85976 Breast Cancer Mets (OD04590-03) | 9.8 |
| 98853 Ovarian cancer (OD06455-03) | 0.2 | 87070 Breast Cancer Metastasis (OD04655-05) | 2.0 |
| 98854 Ovarian NAT (OD06455-07) Fallopian tube | 0.5 | GENPAK Breast Cancer 064006 | 4.0 |
| Normal Lung GENPAK 061010 | 0.3 | Breast Cancer Clontech 9100266 | 3.2 |
| 92337 Invasive poor diff. lung adeno (OD04945-01 | 28.1 | Breast NAT Clontech 9100265 | 8.0 |
| 92338 Lung NAT (ODO4945-03) | 0.3 | Breast Cancer INVITROGEN A209073 | 1.3 |
| 84136 Lung Malignant Cancer (OD03126) | 6.5 | Breast NAT INVITROGEN A2090734 | 13.1 |
| 84137 Lung NAT (OD03126) | 0.0 | 97763 Breast cancer (OD06083) | 20.6 |
| 90372 Lung Cancer (OD05014A) | 5.6 | 97764 Breast cancer node metastasis (OD06083) | 27.6 |
| 90373 Lung NAT (OD05014B) | 0.4 | Normal Liver GENPAK 061009 | 0.0 |
| 97761 Lung cancer (OD06081) | 0.5 | Liver Cancer Research Genetics RNA 1026 | 1.3 |
| 97762 Lung cancer NAT (OD06081) | 0.0 | Liver Cancer Research Genetics RNA 1025 | 1.8 |
| 85950 Lung Cancer (OD04237-01) | 0.9 | Paired Liver Cancer Tissue Research Genetics RNA 6004-T | 0.3 |
| 85970 Lung NAT (OD04237-02) | 0.7 | Paired Liver Tissue Research Genetics RNA 6004-N | 3.3 |
| 83255 Ocular Mel Met to Liver (OD04310) | 0.0 | Paired Liver Cancer Tissue Research Genetics RNA 6005-T | 2.1 |
| 83256 Liver NAT (ODO4310) | 0.0 | Paired Liver Tissue Research Genetics RNA 6005-N | 0.0 |
| 84139 Melanoma Mets to Lung (OD04321) | 2.5 | Liver Cancer GENPAK 064003 | 0.0 |
| 84138 Lung NAT (OD04321) | 0.1 | Normal Bladder GENPAK 061001 | 1.0 |
| Normal Kidney GENPAK 061008 | 13.7 | Bladder Cancer Research Genetics RNA 1023 | 5.6 |
| 83786 Kidney Ca, Nuclear grade 2 (OD04338) | 11.6 | Bladder Cancer INVTTROGEN A302173 | 1.1 |
| 83787 Kidney NAT (OD04338) | 9.1 | Normal Stomach GENPAK 061017 | 1.7 |
| 83788 Kidney Ca Nuclear grade 1/2 (OD04339) | 1.0 | Gastric Cancer Clontech 9060397 | 1.8 |
| 83789 Kidney NAT (OD04339) | 57.3 | NAT Stomach Clontech 9060396 | 2.2 |
| 83790 Kidney Ca, Clear cell type (OD04340) | 0.0 | Gastric Cancer Clontech 9060395 | 1.3 |
| 83791 Kidney NAT (OD04340) | 12.3 | NAT Stomach Clontech 9060394 | 0.1 |
| 83792 Kidney Ca, Nuclear grade 3 (OD04348) | 21.1 | Gastric Cancer GENPAK 064005 | 0.2 |

**Table 15. Panel 4.1D**

| **Tissue Name** | **Relative Expression(%)** | **Tissue Name** | **Relative Expression(%)** |
|---|---|---|---|
| | **4.1dx4tm6089f_ag850_b2** | | **4.1dx4tm6089f_ag850_b2** |
| 93768_Secondary Th1_anti-CD28/anti-CD3 | 0.0 | 93100_HUVEC (Endothelial)_IL-lb | 0.0 |
| 93769_Secondary Th2_anti-CD28/anti-CD3 | 0.0 | 93779_HUVEC (Endothelial)_IFN gamma | 0.8 |
| 93770_Secondary Tr1_anti-CD28/anti-CD3 | 0.0 | 93102_HUVEC (Endothelial)_TNF alpha + IFN gamma | 1.0 |
| 93573_Secondary Th1_resting day 4-6 in IL-2 | 0.0 | 93101_HUVEC (Endothelial)_TNF alpha + IL4 | 0.0 |
| 93572_Secondary Th2_resting day 4-6 in IL-2 | 0.0 | 93781_HUVEC (Endothelial)_IL-11 | 0.0 |
| 93571_Secondary Tr1_resting day 4-6 in IL-2 | 0.7 | 93583_Lung Microvascular Endothelial Cells_none | 0.0 |
| 93568_primary Th1_anti-CD28/anti-CD3 | 0.2 | 93584_Lung Microvascular Endothelial Cells_TNFa (4 ng/ml) and ILlb (1 ng/ml) | 0.0 |
| 93569_primary Th2_anti-CD28/anti-CD3 | 0.0 | 92662_Microvascular Dermal endothelium_none | 0.3 |
| 93570_primary Tr1_anti-CD28/anti-CD3 | 0.6 | 92663_Microsvasular Dermal endothelium_TNFa (4 ng/ml) and ILlb (1 ng/ml) | 0.0 |
| 93565_primary Th1_resting dy 4-6 in IL-2 | 0.0 | 93773_Bronchial epithelium_TNFa (4 ng/ml) and ILlb (1 ng/ml) ** | 0.9 |
| 93566_primary Th2_resting dy 4-6 in IL-2 | 0.0 | 93347 _Small Airway Epithelium none | 1.8 |
| 93567_primary Tr1_resting dy 4-6 in IL-2 | 0.0 | 93348_Small Airway Epithelium_TNFa (4 ng/ml) and ILlb (1 ng/ml) | 4.0 |
| 93351_CD45RA CD4 lymphocyte_anti-CD28/anti-CD3 | 7.4 | 92668_Coronery Artery SMC_resting | 1.8 |
| 93352_CD45RO CD4 lymphocyte_anti-CD28/anti-CD3 | 0.2 | 92669_Coronery Artery SMC_TNFa (4 ng/ml) and IL1b (1 ng/ml) | 1.3 |
| 93251_CD8 Lymphocytes_anti-CD28/anti-CD3 | 0.0 | 93107_astrocytes_resting | 31.9 |
| 93353_chronic CD8 Lymphocytes 2ry_resting dy 4-6 in IL-2 | 0.2 | 93108_astrocytes_TNFa (4 ng/ml) and IL1b (1 ng/ml) | 33.4 |
| 93574_chronic CD8 Lymphocytes 2ry_activated CD3/CD28 | 0.3 | 92666_KU-812 (Basophil)_resting | 0.0 |
| 93354_CD4_none | 0.0 | 92667_KU-812 (Basophil)_PMA/ionoycin | 0.0 |
| 93252_Secondary Th1/Th2/Tr1_anti-CD95 CH11 | 0.1 | 93579_CCD1106 (Keratinocytes)_none | 0.0 |
| 93103_LAK cells_resting | 0.0 | 93580_CCD1106 (Keratinocytes)_TNFa and IFNg** | 0.0 |
| 93788_LAK cells_IL-2 | 0.0 | 93791_Liver Cirrhosis | 1.0 |
| 93787 LAK cells_IL-2+IL-12 | 0.0 | 93577_NCI-H292 | 2.2 |
| 93789_LAK cells_IL-2+IFN gamma | 0.0 | 93358_NCI-H292 IL-4 | 1.1 |
| 93790_LAK cells_IL-2+ IL-18 | 0.0 | 93360_NCI-H292_IL-9 | 1.5 |
| 93104_LAK cells_PMA/ionomycin and IL-18 | 0.6 | 93359_NCI-H292 IL-13 | 0.9 |
| 93578_NK Cells IL-2_resting | 0.0 | 93357_NCI-H292_IFN gamma | 0.3 |
| 93109_Mixed Lymphocyte Reaction_Two Way MLR | 0.0 | 93777_HPAEC - | 0.0 |
| 93110_Mixed Lymphocyte Reaction_Two Way MLR | 0.0 | 93778_HPAEC_IL-1 beta/TNA alpha | 0.0 |
| 93111_Mixed Lymphocyte Reaction_Two Way MLR | 0.2 | 93254_Normal Human Lung Fibroblast_none | 0.0 |
| 93112_Mononuclear Cells (PBMCs)_resting | 0.0 | 93253_Normal Human Lung Fibroblast_TNFa (4 ng/ml) and IL-1b (1 ng/ml) | 0.0 |
| 93113_Mononuclear Cells (PBMCs)_PWM | 0.0 | 93257_Normal Human Lung Fibroblast_IL-4 | 0.1 |
| 93114_Mononuclear Cells (PBMCs)_PHA-L | 0.0 | 93256_Normal Human Lung Fibroblast_IL-9 | 0.0 |
| 93249_Ramos (B cell)_none | 0.0 | 93255_Normal Human Lung Fibroblast_IL-13 | 0.1 |
| 93250_Ramos (B cell)_ionomycin | 0.0 | 93258_Normal Human Lung Fibroblast_IFN gamma | 0.8 |
| 93349_B lymphocytes_PWM | 0.0 | 93106_Dermal Fibroblasts CCD1070_resting | 14.5 |
| 93350_B lymphoytes_CD40L and IL-4 | 0.1 | 93361_Dermal Fibroblasts CCD1070_TNF alpha 4 ng/ml | 15.2 |
| 92665_EOL-1 (Eosinophil)_dbcAMP | 0.0 | 93105_Dermal Fibroblasts CCD1070_IL-1 beta 1 ng/ml | 10.1 |
| differentiated | | | |
| 93248_EOL-1 (Eosinophil)_dbcAMP/PMAion omycin | 0.0 | 93772_dermal fibroblast_IFN gamma | 0.0 |
| 93356_Dendritic Cells_none | 0.0 | 93771_dermal fibroblast_IL-4 | 0.0 |
| 93355_Dendritic Cells_LPS 100 ng/ml | 0.3 | 93892_Dermal fibroblasts_none | 0.0 |
| 93775_Dendritic Cells_anti-CD40 | 0.0 | 99202_Neutrophils_TNFa+LPS | 0.0 |
| 93774_Monocytes_resting | 0.0 | 99203_Neutrophils_none | 0.0 |
| 93776_Monocytes_LPS 50 ng/ml | 0.0 | 735010_Colon_normal | 0.6 |
| 93581_Macrophages_resting | 0.0 | 735019_Lung_none | 0.9 |
| 93582_Macrophages_LPS 100 ng/ml | 0.8 | 64028-1_Thymus_none | 6.0 |
| 93098_HUVEC (Endothelial)_none | 0.0 | 64030-1_Kidney_none | 100.0 |
| 93099_HUVEC (Endothelial)_starved | 0.0 | | |

**Panel 2.2 Summary**: Ag850 The NOV2a gene is most highly expressed in a sample derived from normal kidney tissue adjacent to a kidney cancer (CT = 28.4), among the samples on this panel. Furthermore, in 7 out of 9 cases this gene is expressed more highly in normal kidney tissues adjacent to kidney cancers. In addition, there also appears to be over expression of the NOV2a gene in lung cancers when compared to their normal adjacent tissue. Thus, the expression of the NOV2a gene could be used to distinguish normal kidney tissue from kidney cancer or lung cancer tissue from normal lung tissue. In addition, therapeutic modulation of the protein encoded by this gene may be of use in the treatment of kidney cancer or lung cancer.

**Panel 4.1D Summary:** Ag850 The NOV2a gene is expressed in normal kidney, thymus, a dermal fibroblast cell line (CCD1070) and in astrocytes, among the samples on this panel. Expression of this gene is highest in kidney (CT = 28.1), consistent with what is observed in Panel 2.2. The NOV2a gene encodes a protein that has homology to retinoic acid-responsive protein, which is known to be expressed at blood organ barriers and may function in transport (ref. 1). Astrocytes contribute to the blood brain barrier (ref. 2), so based on the homology of this gene to Stra6 and its expression in astrocytes, the NOV2a gene product may be important in maintaining the blood brain barrier and perhaps in the transport of small molecules across this barrier. Therefore, regulation ofNOV2a gene product with small molecule therapeutics could allow the passage of specific therapeutic molecules into the brain usually blocked by tight junctions. Furthermore, modulation of the function of this protein may also be important in the treatment of autism, since it has been recently shown that autism may be linked to pathology associated with infection and this particular gene is found within a chromosomal locus associated with autism (ref. 3).

### References:

1. Bouillet P., Sapin V, Chazaud C., Messaddeq N., Decimo D., Dolle P., Chambon P. (1997) Developmental expression pattern of Stra6, a retinoic acid-responsive gene encoding a new type of membrane protein. Mech. Dev. 63: 173-186.

Retinoic acid plays important roles in development, growth and differentiation by regulating the expression of target genes. A new retinoic acid-inducible gene, Stra6, has been identified in P19 embryonal carcinoma cells using a subtractive hybridization cDNA cloning technique. Stra6 codes for a very hydrophobic membrane protein of a new type, which does not display similarities with previously characterized integral membrane proteins. Stra6, which exhibits a specific pattern of expression during development and in the adult, is strongly expressed at the level of blood-organ barriers. Interestingly, in testis Sertoli cells, Stra6 has a spermatogenic cycle-dependent expression which is lost in testes of RAR alpha null mutants where Stra6 is expressed in all tubules. We suggest that the Stra6 protein may be a component of an as yet unidentified transport machinery.

PMID: 9203140

2. Pardridge W.M. (1999) Blood-brain barrier biology and methodology. J. Neurovirol. 5: 556-69.

The blood-brain barrier (BBB) is formed by epithelial-like high resistance tight junctions within the endothelium of capillaries perfusing the vertebrate brain. Because of the presence of the BBB, circulating molecules gain access to brain cells only via one of two processes: (i) lipid-mediated transport of small molecules through the BBB by free diffusion, or (ii) catalyzed transport. The latter includes carrier-mediated transport processes for low molecular weight nutrients and water soluble vitamins or receptor-mediated transport for circulating peptides (e.g., insulin), plasma proteins (e.g., transferrin), or viruses. While BBB permeability, per se, is controlled by the biochemical properties of the plasma membranes of the capillary endothelial cells, overall brain microvascular biology is a function of the paracrine interactions between the capillary endothelium and the other two major cells comprising the microcirculation of brain, i.e., the capillary pericyte, which shares the basement membrane with the endothelial cell, and the astrocyte foot process, which invests 99% of the abluminal surface of the capillary basement membrane in brain. Microvascular functions frequently ascribed to the capillary endothelium are actually executed by either the capillary pericyte or the capillary astrocyte foot process. With respect to BBB methodology, there are a variety of in vivo methods for studying biological transport across this important membrane. The classical physiologic techniques may now be correlated with modem biochemical and molecular biological approaches using freshly isolated animal or human brain capillaries. Isolated brain capillary endothelial cells can also be grown in tissue culture to form an 'in vitro BBB' model. However, BBB research cannot be performed using only the in vitro BBB model, but rather it is necessary to correlate observations made with the in vitro BBB model with in vivo studies.

PMID: 10602397

3. Hornig M., Weissenbock H., Horscroft N., Lipkin W.I. (1999) An infection-based model ofneurodevelopmental damage. Proc. Natl. Acad. Sci. U S A 96: 12102-12107.

Perinatal exposure to infectious agents and toxins is linked to the pathogenesis of neuropsychiatric disorders, but the mechanisms by which environmental triggers interact with developing immune and neural elements to create neurodevelopmental disturbances are poorly understood. We describe a model for investigating disorders of central nervous system development based on neonatal rat infection with Borna disease virus, a neurotropic noncytolytic RNA virus. Infection results in abnormal righting reflexes, hyperactivity, inhibition of open-field exploration, and stereotypic behaviors. Architecture is markedly disrupted in hippocampus and cerebellum, with reduction in granule and Purkinj e cell numbers. Neurons are lost predominantly by apoptosis, as supported by increased mRNA levels for pro-apoptotic products (Fas, caspase-1), decreased mRNA levels for the anti-apoptotic bcl-x, and in situ labeling of fragmented DNA. Although inflammatory infiltrates are observed transiently in frontal cortex, glial activation (microgliosis > astrocytosis) is prominent throughout the brain and persists for several weeks in concert with increased levels of proinflammatory cytokine mRNAs (interleukins 1alpha, 1beta, and 6 and tumor necrosis factor alpha) and progressive hippocampal and cerebellar damage. The resemblance of these functional and neuropathologic abnormalities to human neurodevelopmental disorders suggests the utility of this model for defining cellular, biochemical, histologic, and functional outcomes of interactions of environmental influences with the developing central nervous system.

PMID: 10518583

### NOV3a

Expression of the NOV3a gene was assessed using the primer-probe set Ag1402, described in Table 16. Results from RTQ-PCR runs are shown in Tables 17, 18, 19, and 20.

**Table 16. Probe Name Ag1402**

| Primers | Sequences | TM | Length | Start Position | SEQ ID NO: |
|---|---|---|---|---|---|
| Forward | 5'-CTTACAACACCACGGGACTAAG-3' | 58.7 | 22 | 4102 | 139 |
| Probe | FAM-5'- AAGAGCACTTCCTTGCCTCCGTAAGG- 3'-TAMRA | 69.4 | 26 | 4125 | 140 |
| Reverse | 5'-AGATCAAATGATTGGGATGGTT-3' | 59.5 | 22 | 4161 | 141 |

**Table 17. Panel 1.2**

| **Tissue Name** | **Relative Expression(%)** | |
|---|---|---|
| | **1.2tm1660f_ ag1402*** | **1.2tm1691f_ag1402*** |
| Endothelial cells | 15.3 | 15.8 |
| Heart (fetal) | 45.4 | 33.9 |
| Pancreas | 1.3 | 0.5 |
| Pancreatic ca. CAPAN 2 | 0.2 | 0.5 |
| Adrenal Gland (new lot*) | 22.2 | 17.7 |
| Thyroid | 1.5 | 0.5 |
| Salavary gland | 12.0 | 10.4 |
| Pituitary gland | 1.7 | 2.5 |
| Brain (fetal) | 0.2 | 0.3 |
| Brain (whole) | 1.3 | 1.6 |
| Brain (amygdala) | 3.0 | 3.0 |
| Brain (cerebellum) | 2.8 | 0.8 |
| Brain (hippocampus) | 9.5 | 9.5 |
| Brain (thalamus) | 5.8 | 5.6 |
| Cerebral Cortex | 15.4 | 12.3 |
| Spinal cord | 1.5 | 1.1 |
| CNS ca. (glio/astro) U87-MG | 1.2 | 1.4 |
| CNS ca. (glio/astro) U-118-MG | 6.7 | 7.7 |
| CNS ca. (astro) SW1783 | 1.8 | 2.3 |
| CNS ca.* (neuro; met) SK-N-AS | 32.1 | 18.9 |
| CNS ca. (astro) SF-539 | 43.5 | 30.4 |
| CNS ca. (astro) SNB-75 | 2.9 | 2.7 |
| CNS ca. (glio) SNB-19 | 1.7 | 1.1 |
| CNS ca. (glio) U251 | 7.0 | 5.8 |
| CNS ca. (glio) SF-295 | 24.8 | 19.5 |
| Heart | **100.0** | **100.0** |
| Skeletal Muscle (new lot*) | 19.6 | 16.4 |
| Bone marrow | 1.7 | 2.2 |
| Thymus | 1.6 | 0.9 |
| Spleen | 10.2 | 7.6 |
| Lymph node | 1.4 | 1.3 |
| Colorectal | 1.6 | 4.2 |
| Stomach | 5.1 | 4.7 |
| Small intestine | 40.1 | 35.6 |
| Colon ca. SW480 | 1.2 | 0.5 |
| Colon ca.* (SW480 met)SW620 | 3.1 | 2.0 |
| Colon ca. HT29 | 0.2 | 0.2 |
| Colon ca. HCT-116 | 5.6 | 3.0 |
| Colon ca. CaCo-2 | 1.7 | 0.9 |
| 83219 CC Well to Mod Diff (ODO3866) | 2.2 | 2.3 |
| Colon ca. HCC-2998 | 8.7 | 4.6 |
| Gastric ca.* (liver met) NCI-N87 | 1.2 | 1.3 |
| Bladder | 20.9 | 18.6 |
| Trachea | 1.4 | 0.8 |
| Kidney | 27.5 | 30.6 |
| Kidney (fetal) | 46.3 | 13.7 |
| Renal ca. 786-0 | 0.5 | 0.4 |
| Renal ca. A498 | 1.6 | 1.4 |
| Renal ca. RXF 393 | 2.5 | 2.3 |
| Renal ca. ACHN | 1.7 | 2.1 |
| Renal ca. UO-31 | 1.6 | 1.1 |
| Renal ca. TK-10 | 3.3 | 3.3 |
| Liver | 13.2 | 12.2 |
| Liver (fetal) | 8.3 | 6.2 |
| Liver ca. (hepatoblast) HepG2 | 2.5 | 0.8 |
| Lung | 1.5 | 1.4 |
| Lung (fetal) | 12.2 | 5.4 |
| Lung ca. (small cell) LX-1 | 3.3 | 1.9 |
| Lung ca. (small cell) NCI-H69 | 3.2 | 2.7 |
| Lung ca. (s.cell var.) SHP-77 | 0.9 | 0.5 |
| Lung ca. (large cell)NCI-H460 | 2.8 | 2.1 |
| Lung ca. (non-sm. cell) A549 | 3.1 | 1.5 |
| Lung ca. (non-s.cell) NCI-H23 | 2.3 | 3.4 |
| Lung ca (non-s.cell) HOP-62 | 5.3 | 11.7 |
| Lung ca. (non-s.cl) NCI-H522 | 11.3 | 9.3 |
| Lung ca. (squam.) SW 900 | 1.2 | 1.1 |
| Lung ca. (squam.) NCI-H596 | 2.0 | 2.0 |
| Mammary gland | 4.2 | 29.5 |
| Breast ca.* (pl. effusion) MCF-7 | 0.6 | 0.7 |
| Breast ca.* (pl.ef) MDA-MB-231 | 0.3 | 0.2 |
| Breast ca.* (pl. effusion) T47D | 3.5 | 2.5 |
| Breast ca. BT-549 | 0.4 | 0.4 |
| Breast ca. MDA-N | 12.2 | 14.2 |
| Ovary | 79.6 | 54.3 |
| Ovarian ca. OVCAR-3 | 1.0 | 0.8 |
| Ovarian ca. OVCAR-4 | 1.8 | 0.0 |
| Ovarian ca. OVCAR-5 | 7.6 | 4.4 |
| Ovarian ca. OVCAR-8 | 20.4 | 12.2 |
| Ovarian ca. IGROV-1 | 1.4 | 1.1 |
| Ovarian ca.* (ascites) SK-OV-3 | 14.4 | 11.2 |
| Uterus | 34.4 | 19.6 |
| Placenta | 6.7 | 4.8 |
| Prostate | 70.2 | 74.2 |
| Prostate ca.* (bone met)PC-3 | 7.9 | 8.5 |
| Testis | 1.2 | 0.6 |
| Melanoma Hs688(A).T | 15.8 | 15.3 |
| Melanoma* (met) Hs688(B).T | 33.0 | 26.6 |
| Melanoma UACC-62 | 9.9 | 8.8 |
| Melanoma M14 | 3.3 | 2.9 |
| Melanoma LOX IMVI | 0.8 | 1.0 |
| Melanoma* (met) SK-MEL-5 | 5.8 | 6.1 |
| Adipose | 21.8 | 36.6 |

**Table 18. Panel 2.2**

| **Tissue Name** | **Relative Expression(%)** | **Tissue Name** | **Relative Expression(%)** |
|---|---|---|---|
| | **2.2x4tm6342f_ag1402_a2** | | **2.2x4tm6342f_ag1402_a2** |
| Normal Colon GENPAK 061003 | 29.8 | 83793 Kidney NAT (OD04348) | 32.2 |
| 97759 Colon cancer (OD06064) | 41.0 | 98938 Kidney malignant cancer (OD06204B) | 2.2 |
| 97760 Colon cancer NAT (OD06064) | 34.1 | 98939 Kidney normal adjacent tissue (OD06204E) | 9.1 |
| 97778 Colon cancer (OD06159) | 2.5 | 85973 Kidney Cancer (OD04450-01) | 2.9 |
| 97779 Colon cancer NAT (OD06159) | 30.1 | 85974 Kidney NAT (OD04450-03) | 7.8 |
| 98861 Colon cancer (OD06297-04) | 7.0 | Kidney Cancer Clontech 8120613 | 1.2 |
| 98862 Colon cancer NAT (OD06297-015) | 36.5 | Kidney NAT Clontech 8120614 | 9.4 |
| 83237 CC Gr.2 ascend colon (ODO3921) | 7.1 | Kidney Cancer Clontech 9010320 | 5.8 |
| 83238 CC NAT (ODO3921) | 8.6 | Kidney NAT Clontech 9010321 | 3.7 |
| 97766 Colon cancer metastasis (OD06104) | 2.7 | Kidney Cancer Clontech 8120607 | 16.6 |
| 97767 Lung NAT (OD06104) | 14.2 | Kidney NAT Clontech 8120608 | 6.0 |
| 87472 Colon mets to lung (OD04451-01) | 1.7 | Normal Uterus GENPAK 061018 | 88.6 |
| 87473 Lung NAT (OD04451-02) | 17.5 | Uterus Cancer GENPAK 064011 | 14.2 |
| Normal Prostate Clontech A+ 6546-1 (8090438) | 86.0 | Normal Thyroid Clontech A+ 6570-1 (7080817) | 7.5 |
| 84140 Prostate Cancer (OD04410) | 12.4 | Thyroid Cancer GENPAK 064010 | 2.2 |
| 84141 Prostate NAT (OD04410) | 32.3 | Thyroid Cancer INVITROGEN A302152 | 11.5 |
| Normal Ovary Res. Gen. | 74.3 | Thyroid NAT INVITROGEN A302153 | 3.9 |
| 98863 Ovarian cancer (OD06283-03) | 8.5 | Normal Breast GENPAK 061019 | 95.2 |
| 98865 Ovarian cancer NAT/fallopian tube (OD06283-07) | 34.3 | 84877 Breast Cancer (OD04566) | 4.0 |
| Ovarian Cancer GENPAK 064008 | 17.3 | Breast Cancer Res. Gen. 1024 | 58.3 |
| 97773 Ovarian cancer (OD06145) | 17.2 | 85975 Breast Cancer (OD04590-01) | 19.3 |
| 97775 Ovarian cancer NAT (OD06145) | 24.9 | 85976 Breast Cancer Mets (OD04590-03) | 22.6 |
| 98853 Ovarian cancer (OD06455-03) | 6.4 | 87070 Breast Cancer Metastasis (OD04655-05) | 12.0 |
| 98854 Ovarian NAT (OD06455-07) Fallopian tube | **100.0** | GENPAK Breast Cancer 064006 | 16.7 |
| Normal Lung GENPAK 061010 | 18.9 | Breast Cancer Clontech 9100266 | 13.8 |
| 92337 Invasive poor diff. lung adeno (ODO4945-01 | 7.7 | Breast NAT Clontech 9100265 | 51.2 |
| 92338 Lung NAT (ODO4945-03) | 20.2 | Breast Cancer INVITROGEN A209073 | 13.7 |
| 84136 Lung Malignant Cancer (OD03126) | 8.8 | Breast NAT INVITROGEN A2090734 | 41.9 |
| 84137 Lung NAT (OD03126) | 7.7 | 97763 Breast cancer (OD06083) | 26.5 |
| 90372 Lung Cancer (OD05014A) | 8.8 | 97764 Breast cancer node metastasis (OD06083) | 39.3 |
| 90373 Lung NAT (OD05014B) | 29.0 | Normal Liver GENPAK 061009 | 16.4 |
| 97761 Lung cancer (OD06081) | 11.1 | Liver Cancer Research Genetics RNA 1026 | 11.5 |
| 97762 Lung cancer NAT (OD06081) | 6.5 | Liver Cancer Research Genetics RNA 1025 | 13.3 |
| 85950 Lung Cancer (OD04237-01) | 8.7 | Paired Liver Cancer Tissue Research Genetics RNA 6004-T | 11.3 |
| 85970 Lung NAT (OD04237-02) | 27.6 | Paired Liver Tissue Research Genetics RNA 6004-N | 11.8 |
| 83255 Ocular Mel Met to Liver (ODO4310) | 3.1 | Paired Liver Cancer Tissue Research Genetics RNA 6005-T | 28.4 |
| 83256 Liver NAT (ODO4310) | 9.2 | Paired Liver Tissue Research Genetics RNA 6005-N | 23.1 |
| 84139 Melanoma Mets to Lung (OD04321) | 10.2 | Liver Cancer GENPAK 064003 | 4.3 |
| 84138 Lung NAT (OD04321) | 18.2 | Normal Bladder GENPAK 061001 | 12.2 |
| Normal Kidney GENPAK 061008 | 10.6 | Bladder Cancer Research Genetics RNA 1023 | 16.4 |
| 83786 Kidney Ca, Nuclear | 24.4 | Bladder Cancer INVITROGEN | 8.9 |
| grade 2 (OD04338) | | A302173 | |
| 83787 Kidney NAT (OD04338) | 4.9 | Normal Stomach GENPAK 061017 | 46.7 |
| 83788 Kidney Ca Nuclear grade 1/2 (OD04339) | 5.2 | Gastric Cancer Clontech 9060397 | 5.8 |
| 83789 Kidney NAT (OD04339) | 10.7 | NAT Stomach Clontech 9060396 | 17.5 |
| 83790 Kidney Ca, Clear cell type (OD04340) | 12.8 | Gastric Cancer Clontech 9060395 | 19.0 |
| 83791 Kidney NAT (OD04340) | 10.0 | NAT Stomach Clontech 9060394 | 49.8 |
| 83792 Kidney Ca, Nuclear grade 3 (OD04348) | 14.5 | Gastric Cancer GENPAK 064005 | 8.3 |

**Table 19. Panel 3D**

| **Tissue Name** | **Relative Expression(%)** | **Tissue Name** | **Relative Expression(%)** |
|---|---|---|---|
| | **3dx4tm6576f_ ag1402_a1** | | **3dx4tm6576f_ag1402_a1** |
| 94905_Daoy_Medulloblastoma/ Cerebellum_sscDNA | 1.9 | 94954_Ca Ski_Cervical epidermoid carcinoma (metastasis)_sscDNA | 1.9 |
| 94906_TE671_Medulloblastom /Cerebellum_sscDNA | 8.0 | 94955_ES-2_Ovarian clear cell carcinoma_sscDNA | 3.2 |
| 94907_D283 Med_Medulloblastoma/Cerebell um_sscDNA | 13.0 | 94957_Ramos/6h stim_ Stimulated with PMA/ionomycin 6h_sscDNA | 0.8 |
| 94908_PFSK-1_Primitive Neuroectodermal/Cerebellum_s scDNA | 60.9 | 94958_Ramos/14h stim_ Stimulated with PMA/ionomycin 14h_sscDNA | 0.6 |
| 94909_XF-498_CNS_sscDNA | 16.0 | 94962_MEG-01_Chronic myelogenous leukemia (megokaryoblast)_sscDNA | 1.0 |
| 94910_SNB-78_CNS/glioma_sscDNA | 81.8 | 94963_Raji_Burkitt's lymphoma_sscDNA | 0.1 |
| 94911_SF-268_CNS/glioblastoma_sscDN A | 4.5 | 94964_Daudi_Burkitt's lymphoma_sscDNA | 1.0 |
| 94912_T98G_Glioblastoma_ssc DNA | 16.2 | 94965_U266_B-cell plasmacytoma/myeloma_sscDN A | 1.0 |
| 96776_SK-N-SH_Neuroblastoma (metastasis)_sscDNA | 39.1 | 94968_CA46_Burkitt's lymphoma_sscDNA | 2.0 |
| 94913_SF-295_CNS/glioblastoma_sscDN A | 5.2 | 94970_RL_non-Hodgkin's B-cell lymphoma_sscDNA | 0.2 |
| 94914_Cerebellum_sscDNA | 7.7 | 94972_JM1_pre-B-cell lymphoma/leukemia_sscDNA | 2.6 |
| 96777_Cerebellum_sscDNA | 13.4 | 94973_Jurkat_T cell leukemia_sscDNA | 5.1 |
| 94916_NCI-H292_Mucoepidermoid lung carcinoma_sscDNA | 2.1 | 94974_TF-1 Erythroleukemia_sscDNA | 1.0 |
| 94917_DMS-114_Small cell lung cancer_sscDNA | 31.2 | 94975_HUT 78_T-cell lymphoma_sscDNA | 3.6 |
| 94918_DMS-79_Small cell lung cancer/neuroendocrine_sscDNA | 31.8 | 94977_U937_Histiocytic lymphoma_sscDNA | **100.0** |
| 94919_NCI-H146_Small cell lung cancer/neuroendocrine_sscDNA | 3.1 | 94980_KU-812_Myelogenous leukemia_sscDNA | 1.5 |
| 94920_NCI-H526_Small cell lung cancer/neuroendocrine_sscDNA | 9.4 | 94981_769-P_Clear cell renal carcinoma_sscDNA | 0.7 |
| 94921_NCI-N417_Small cell lung cancer/neuroendocrine_sscDNA | 0.2 | 94983_Caki-2_Clear cell renal carcinoma_sscDNA | 0.9 |
| 94923_NCI-H82_Small cell lung cancer/neuroendocrine_sscDNA | 3.7 | 94984_SW 839_Clear cell renal carcinoma_sscDNA | 0.5 |
| 94924_NCI-H157_Squamous cell lung cancer (metastasis)_sscDNA | 1.4 | 94986_G401_Wilms' tumor_sscDNA | 34.9 |
| 94925_NCI-H1155_Large cell lung cancer/neuroendocrine_sscDNA | 9.2 | 94987_Hs766T_Pancreatic carcinoma (LN metastasis)_sscDNA | 0.9 |
| 94926_NCI-H1299_Large cell lung cancer/neuroendocrine_sscDNA | 1.5 | 94988_CAPAN-1_Pancreatic adenocarcinoma (liver metastasis)_sscDNA | 5.5 |
| 94927_NCI-H727_Lung carcinoid_sscDNA | 1.5 | 94989_SU86.86_Pancreatic carcinoma (liver metastasis)_sscDNA | 1.7 |
| 94928_NCI-LTMC-11_Lung carcinoid_sscDNA | 2.0 | 94990_BxPC-3_Pancreatic adenocarcinoma_sscDNA | 0.8 |
| 94929_LX-1_Small cell lung cancer_sscDNA | 1.5 | 94991_HPAC_Pancreatic adenocarcinoma_sscDNA | 0.2 |
| 94930_Colo-205_Colon cancer_sscDNA | 0.9 | 94992_MIA PaCa-2_Pancreatic carcinoma_sscDNA | 0.0 |
| 94931_KM12_Colon cancer_sscDNA | 0.9 | 94993_CFPAC-1_Pancreatic ductal adenocarcinoma sscDNA | 2.1 |
| 94932_KM20L2_Colon cancer_sscDNA | 0.4 | 94994_PANC-1_Pancreatic epithelioid ductal carcinoma_sscDNA | 0.7 |
| 94933_NCI-H716_Colon cancer_sscDNA | 5.6 | 94996_T24_Bladder carcinma (transitional cell)_sscDNA | 1.1 |
| 94935_SW-48_Colon adenocarcinoma_sscDNA | 0.3 | 94997_5637_Bladder carcinoma_sscDNA | 0.9 |
| 94936_SW1116_Colon adenocarcinoma_sscDNA | 5.3 | 94998_HT-1197_Bladder carcinoma_sscDNA | 0.6 |
| 94937_LS 174T_Colon adenocarcinoma_sscDNA | 1.1 | 94999_UM-UC-3_Bladder carcinma (transitional cell)_sscDNA | 0.6 |
| 94938_SW-948_Colon adenocarcinoma_sscDNA | 0.0 | 95000_A204_Rhabdomyosarco ma_sscDNA | 12.3 |
| 94939_SW-480_Colon adenocarcinoma_sscDNA | 0.4 | 95001_HT-1080_Fibrosarcoma_sscDNA | 7.3 |
| 94940_NCI-SNU-5_Gastric carcinoma_sscDNA | 0.8 | 95002_MG-63_Osteosarcoma (bone)_sscDNA | 11.9 |
| 94941_KATO III_Gastric carcinoma_sscDNA | 2.4 | 95003_SK-LMS-1_Leiomyosarcoma (vulva)_sscDNA | 5.0 |
| 94943_NCI-SNU-16_Gastric carcinoma_sscDNA | 0.2 | 95004_SJRH30_Rhabdomyosar coma (met to bone marrow)_sscDNA | 2.3 |
| 94944_NCI-SNU-1_Gastric carcinoma_sscDNA | 1.6 | 95005_A431_Epidermoid carcinoma_sscDNA | 1.3 |
| 94946_RF-1_Gastric adenocarcinoma_sscDNA | 0.5 | 95007_WM266-4_Melanoma_sscDNA | 8.7 |
| 94947_RF-48_Gastric adenocarcinoma_sscDNA | 0.9 | 95010_DU 145_Prostate carcinoma (brain metastasis)_sscDNA | 0.0 |
| 96778_MKN-45_Gastric carcinoma_sscDNA | 0.7 | 95012_MDA-MB-468_Breast adenocarcinoma_sscDNA | 1.8 |
| 94949_NCI-N87_Gastric carcinoma_sscDNA | 0.9 | 95013_SCC-4_Squamous cell carcinoma of tongue sscDNA | 0.0 |
| 94951_OVCAR-5_Ovarian carcinoma_sscDNA | 1.7 | 95014_SCC-9_Squamous cell carcinoma of tongue sscDNA | 0.3 |
| 94952_RL95-2_Uterine carcinoma_sscDNA | 6.7 | 95015_SCC-15_Squamous cell carcinoma of tongue sscDNA | 0.0 |
| 94953_HelaS3_Cervical adenocarcinoma_sscDNA | 3.3 | 95017_CAL 27_Squamous cell carcinoma of tongue_sscDNA | 0.7 |

**Table 20. Panel 4D**

| **Tissue Name** | **Relative Expression(%)** | |
|---|---|---|
| | **4Dtm1934f_ ag1402** | **4Dtm2430f_ag1402** |
| 93768_Secondary Th1_anti-CD28/anti-CD3 | 0.5 | 1.0 |
| 93769_Secondary Th2_anti-CD28/anti-CD3 | 0.2 | 0.4 |
| 93770_Secondary Tr1_anti-CD28/anti-CD3 | 0.9 | 0.6 |
| 93573_Secondary Th1_resting day 4-6 in IL-2 | 0.3 | 0.1 |
| 93572_Secondary Th2_resting day 4-6 in IL-2 | 0.4 | 0.2 |
| 93571_Secondary Tr1_resting day 4-6 in IL-2 | 0.3 | 0.2 |
| 93568_primary Th1_anti-CD28/anti-CD3 | 1.1 | 0.8 |
| 93569_primary Th2_anti-CD28/anti-CD3 | 0.9 | 0.8 |
| 93570_primary Tr1_anti-CD28/anti-CD3 | 1.9 | 1.4 |
| 93565_primary Th1_resting dy 4-6 in IL-2 | 1.6 | 1.1 |
| 93566_primary Th2_resting dy 4-6 in IL-2 | 0.7 | 0.5 |
| 93567_primary Tr1_resting dy 4-6 in IL-2 | 1.6 | 1.2 |
| 93351_CD45RA CD4 lymphocyte_anti-CD28/anti-CD3 | 16.7 | 13.4 |
| 93352_CD45RO CD4 lymphocyte_anti-CD28/anti-CD3 | 0.5 | 0.3 |
| 93251_CD8 Lymphocytes_anti-CD28/anti-CD3 | 0.4 | 0.3 |
| 93353_chronic CD8 Lymphocytes 2ry_resting dy 4-6 in IL-2 | 0.5 | 0.4 |
| 93574_chronic CD8 Lymphocytes 2ry_activated CD3/CD28 | 0.6 | 0.3 |
| 93354_CD4_none | 0.5 | 0.4 |
| 93252_Secondary Th1/Th2/Tr1_anti-CD95 CH11 | 0.6 | 0.3 |
| 93103_LAK cells_resting | 0.4 | 0.4 |
| 93788_LAK cells_IL-2 | 0.5 | 0.4 |
| 93787_LAK cells_1L-2+1L-12 | 0.7 | 0.5 |
| 93789_LAK cells_IL-2+IFN gamma | 0.9 | 0.5 |
| 93790_LAK cells_IL-2+IL-18 | 0.5 | 0.3 |
| 93104_LAK cells_PMA/ionomycin and IL-18 | 0.0 | 0.0 |
| 93578_NK Cells IL-2_resting | 0.6 | 0.5 |
| 93109_Mixed Lymphocyte Reaction_Two Way MLR | 0.5 | 0.3 |
| 93110_Mixed Lymphocyte Reaction_Two Way MLR | 0.4 | 0.2 |
| 93111_Mixed Lymphocyte Reaction_Two Way MLR | 0.3 | 0.3 |
| 93112_Mononuclear Cells (PBMCs)_resting | 0.9 | 0.6 |
| 93113_Mononuclear Cells (PBMCs)_PWM | 1.5 | 1.2 |
| 93114_Mononuclear Cells (PBMCs)_PHA-L | 2.1 | 1.5 |
| 93249_Ramos (B cell)_none | 0.6 | 1.0 |
| 93250_Ramos (B cell)_ionomycin | 1.1 | 0.9 |
| 93349_B lymphocytes_PWM | 1.0 | 0.7 |
| 93350_B lymphoytes_CD40L and IL-4 | 0.5 | 0.3 |
| 92665_EOL-1 (Eosinophil)_dbcAMP differentiated | 7.0 | 6.6 |
| 93248_EOL-1 (Eosinophil)_dbcAMP/PMAionomycin | 1.0 | 0.7 |
| 93356_Dendritic Cells_none | 1.0 | 0.5 |
| 93355_Dendritic Cells_LPS 100 ng/ml | 0.1 | 0.1 |
| 93775_Dendritic Cells_anti-CD40 | 0.5 | 0.9 |
| 93774_Monocytes_resting | 3.7 | 2.5 |
| 93776_Monocytes_LPS 50 ng/ml | 4.4 | 3.7 |
| 93581_Macrophages_resting | 0.2 | 0.3 |
| 93582_Macrophages_LPS 100 ng/ml | 0.3 | 0.2 |
| 93098_HUVEC (Endothelial)_none | 7.6 | 5.5 |
| 93099_HUVEC (Endothelial)_starved | 13.4 | 12.4 |
| 93100_HUVEC (Endothelial)_IL-1b | 2.6 | 2.2 |
| 93779_HUVEC (Endothelial)_IFN gamma | 20.3 | 18.2 |
| 93102_HUVEC (Endothelial)_TNF alpha + IFN gamma | 1.5 | 1.1 |
| 93101_HUVEC (Endothelial)_TNF alpha + IL4 | 2.4 | 2.4 |
| 93781_HUVEC (Endothelial)_IL-11 | 14.0 | 12.5 |
| 93583_Lung Microvascular Endothelial Cells none | 14.0 | 12.0 |
| 93584_Lung Microvascular Endothelial Cells_TNFa (4 ng/ml) and ILlb (1 ng/ml) | 7.0 | 7.2 |
| 92662_Microvascular Dermal endothelium_none | 39.2 | 40.1 |
| 92663_Microsvasular Dermal endothelium_TNFa (4 ng/ml) and IL1b (1 ng/ml) | 14.4 | 12.4 |
| 93773_Bronchial epithelium_TNFa (4 ng/ml) and IL 1b (1 ng/ml)** | 0.5 | 0.5 |
| 93347_Small Airway Epithelium_none | 2.1 | 1.5 |
| 93348_Small Airway Epithelium_TNFa (4 ng/ml) and IL 1b (1 ng/ml) | 0.2 | 0.3 |
| 92668_Coronery Artery SMC_resting | 18.7 | 17.9 |
| 92669_Coronery Artery SMC_TNFa (4 ng/ml) and IL 1b (1 ng/ml) | 17.4 | 13.6 |
| 93107_astrocytes_resting | 0.5 | 0.3 |
| 93108_astrocytes_TNFa (4 ng/ml) and IL1b (1 ng/ml) | 0.6 | 0.6 |
| 92666_KU-812 (Basophil)_resting | 0.6 | 0.4 |
| 92667_KU-812 (Basophil)_PMA/ionoycin | 1.0 | 0.6 |
| 93579_CCD1106 (Keratinocytes)_none | 0.0 | 0.2 |
| 93580_CCD1106 (Keratinocytes)_TNFa and IFNg** | 0.6 | 0.2 |
| 93791_Liver Cirrhosis | 4.9 | 5.7 |
| 93792_Lupus Kidney | 5.3 | 4.9 |
| 93577_NCI-H292 | 0.6 | 0.7 |
| 93358_NCI-H292_IL-4 | 0.4 | 0.7 |
| 93360_NCI-H292_IL-9 | 1.1 | 0.7 |
| 93359_NCI-H292_IL-13 | 0.6 | 0.5 |
| 93357_NCI-H292_IFN gamma | 0.7 | 0.5 |
| 93777_HPAEC_- | 12.0 | 11.7 |
| 93778_HPAEC_IL-1 beta/TNA alpha | 6.2 | 5.1 |
| 93254_Normal Human Lung Fibroblast_none | 64.6 | 66.0 |
| 93253_Normal Human Lung Fibroblast_TNFa (4 ng/ml) and IL-1b (1 ng/ml) | 26.6 | 24.7 |
| 93257_Normal Human Lung Fibroblast_IL-4 | 64.6 | 66.9 |
| 93256_Normal Human Lung Fibroblast_IL-9 | 51.4 | 42.9 |
| 93255_Normal Human Lung Fibroblast_IL-13 | 100.0 | 100.0 |
| 93258_Normal Human Lung Fibroblast_IFN gamma | 84.1 | 82.4 |
| 93106_Dermal Fibroblasts CCD1070_resting | 71.2 | 60.7 |
| 93361_Dermal Fibroblasts CCD1070_TNF alpha 4 ng/ml | 41.8 | 39.0 |
| 93105_Dermal Fibroblasts CCD1070_IL-1 beta 1 ng/ml | 45.7 | 43.8 |
| 93772_dermal fibroblast_IFN gamma | 25.3 | 19.5 |
| 93771_dermal fibroblast_IL-4 | 45.7 | 42.0 |
| 93259_IBD Colitis 1** | 3.4 | 1.8 |
| 93260_IBD Colitis 2 | 1.2 | 1.1 |
| 93261_IBD Crohns | 2.0 | 1.6 |
| 735010_Colon_normal | 8.4 | 8.2 |
| 735019_Lung_none | 29.9 | 23.7 |
| 64028-1_Thymus_none | 11.3 | 10.8 |
| 64030-1_Kidney_none | 7.1 | 5.2 |

**Panel 1.2 Summary:** Ag1402 Results from two experiments using the same probe/primer are in good agreement. The NOV3a gene is expressed at highest levels in heart (CT = 22-23), prostate, and ovarian tissue and is expressed to a lesser degree across a number of samples in Panel 1.2. Of note is the observation that this gene is expressed largely in normal tissues when compared to cultured cell lines. Thus, expression of the NOV3a gene could be used to distinguish heart, prostate and ovarian tissue from other tissues.

Among other metabolically relevant tissues, the NOV3a gene is moderately expressed in thyroid, pituitary gland, and pancreas, and is highly expressed in adrenal gland, skeletal muscle and fetal/adult liver. High expression of this gene in insulin-responsive tissues such as skeletal muscle and liver suggests that the NOV3a gene product might be an effective drug target for the treatment of Type 2 diabetes. High expression in adrenal gland and heart also suggests that this gene may be an antibody target for the treatment of diseases involving these two tissues.

The NOV3a gene encodes a putative leucine-rich-repeat (LRR), GPCR-like protein. In Drosophilia, the LRR region of axon guidance proteins has been shown to be critical for function, especially in axon repulsion (ref. 1). The leucine-rich-repeat protein encoded by the NOV3a gene shows high expression across all brain regions, with expression detected in amygdala, cerebellum, hippocampus, thalamus, cerebral cortex and spinal cord, making it an excellent candidate neuronal guidance protein for axons, dendrites and/or growth cones in general. Therapeutic modulation of the levels of this protein, or possible signaling via this protein, may be of utility in enhancing/directing compensatory synaptogenesis and fiber growth in the CNS in response to neuronal death (stroke, head trauma), axon lesion (spinal cord injury), or neurodegeneration (Alzheimer's, Parkinson's, Huntington's, vascular dementia or any neurodegenerative disease). In addition, the NOV3 gene shows highest expression in the substantia nigra, where loss of dopaminergic neurons is a hallmark of Parkinson's disease and progressive supranuclear palsy; thus, therapies based upon this gene may be particularly useful in the treatment of these diseases. This gene also shows overexpression in cell lines such as those derived from brain tumors (Cura 438).

The NOV3 a protein also contains homology to the GPCR family of receptors. Several neurotransmitter receptors are GPCRs, including the dopamine receptor family, the serotonin receptor family, the GABAB receptor, muscarinic acetylcholine receptors, and others; thus this GPCR may represent a novel neurotransmitter receptor. Targeting various neurotransmitter receptors (dopamine, serotonin) has proven to be an effective therapy in psychiatric illnesses such as schizophrenia, bipolar disorder and depression. Furthermore the cerebral cortex and hippocampus are regions of the brain that are known to play critical roles in Alzheimer's disease, seizure disorders, and in the normal process of memory formation. Therapeutic modulation of this gene or its protein product may be beneficial in one or more of these diseases, as may stimulation and/or blockade of the receptor coded for by the gene. Levels of this gene are high, however, in areas outside of the central nervous system (such as the heart, ovaries, and prostate), suggesting the possibility of a wider role in intercellular signaling.

**Panel 2.2 Summary:** Ag1402 Among the samples on this panel, expression of the NOV3a gene is highest in a sample of normal ovarian tissue adjacent to an ovarian cancer (CT = 28.5). There is also a predominance of this expression pattern for a number of other matched normal tissue samples including lung, colon and kidney. Furthermore, unmatched normal tissue samples, such as breast, uterus, ovary and prostate also show appreciable expression of the NOV3a gene. This gene also shows expression by certain breast, liver and gastric tumors (Cura 438). Thus, in general, this gene might be used to distinguish adjacent normal tissue from corresponding malignant tissue in the above listed tissue types. In addition, the therapeutic modulation of the protein encoded by the NOV3 gene might be of use in the treatment of the above listed malignancies.

**Panel 3D Summary**: Ag1402 Expression of the NOV3a gene is highest in a sample derived from a histiocytic lymphoma cell line (CT = 28.2). In addition, this gene is also highly expressed in several CNS cancer cell lines and cell lines derived from a variety of sarcomas. Thus, the expression of the NOV3a gene could be used to distinguish between cell lines derived from sarcomas and CNS cancers vs. other cell lines. In addition, this expression pattern indicates that the therapeutic modulation of this gene product might be of therapeutic benefit in the treatment of sarcomas or CNS cancers.

**Panel 4D Summary:** Ag1402 Results from two experiments using the same probe/primer set are in excellent agreement. The NOV3a gene is highly expressed in endothelium and fibroblasts and is selectively down regulated in these tissues by treatment with IL-1beta and TNFalpha. Thus, the NOV3a gene encodes a putative LRR-containing GPCR that may be down regulated in response to the proinflammatory cytokines IL-1beta and TNFalpha in endothelium and fibroblasts. One possible hypothesis is that the natural ligand for this GPCR may be induced by treatment with TNF and IL-1 and that subsequent ligand binding to this putative GPCR could result in feedback inhibition. Alternatively, other signaling pathways induced by TNF and IL-1 could reduce the level of transcription of the NOV3a gene. Antibodies, small molecule or protein therapeutics which prevent the signalling through this putative GPCR could therefore be important in the treatment of diseases such as asthma, emphysema, psoriasis, and arthritis.

### References:

1. Battye R., Stevens A., Perry R.L., Jacobs J.R. (2001) Repellent signaling by Slit requires the trypsin inhibitors. J. Neurosci. 21: 4290-4298.

Slit is a repellent axon guidance cue produced by the midline glia in Drosophila that is required to regulate the formation of contralateral projections and the lateral position of longitudinal tracts. Four sequence motifs comprise the structure of Slit: a trypsin inhibitor (LRR), epidermal growth factor-like (EGF) repeats, a laminin-like globular (G)-domain, and a cysteine domain. Here we demonstrate that the LRR is required for repellent signaling and in vitro binding to Robo. Repellent signaling by slit is reduced by point mutations that encode single amino acid changes in the LRR domain. By contrast to the EGF or G-domains, the LRR domain is required in transgenes to affect axon guidance. Finally, we show that the midline repellent receptor, Robo, binds Slit proteins with internal deletions that also retain repellent activity. However, Robo does not bind Slit protein missing the LRR. Taken together, our data demonstrate that Robo binding and repellent signaling by Slit require the LRR region.

### NOV7

Expression of gene NOV7 was assessed using the primer-probe sets Ag1631 and Ag2448, described in Tables 21 and 22. Results from RTQ-PCR runs are shown in Tables 23 and 24.

**Table 21. Probe Name Ag1631**

| Primers | Sequences | TM | Length | Start Position | SEQ ID NO: |
|---|---|---|---|---|---|
| Forward | 5'-AAGCAGACCAGATTCCACTTG-3' | 59.3 | 21 | 5 | 129 |
| Probe | FAM-5'-CTAGTGGGCATTGCTCAGCTTCCTCT-3'-TAMRA | 68.8 | 26 | 26 | 130 |
| Reverse | 5'-CTGAATTTCTCGATCTCATCCA-3' | 59.3 | 22 | 77 | 131 |

**Table 22. Probe Name Ag2448**

| Primers | Sequences | TM | Length | Start Position | SEQ ID NO: |
|---|---|---|---|---|---|
| Forward | 5'-ACTTGCTAGTGGGCATTGCT-3' | 59.9 | 20 | 21 | 132 |
| Probe | FAM-5'-TCCTCTGTGACTATGTCTGACAAGTCC A-3'-TAMRA | 66.4 | 28 | 46 | 133 |
| Reverse | 5'-TGAATTTCTCGATCTCATCCA-3' | 58.2 | 21 | 77 | 134 |

**Table 23. Panel 2D**

| **Tissue Name** | **Relative Expression(%)** | **Tissue Name** | **Relative Expression(%)** |
|---|---|---|---|
| | **2dtm4325f_ ag2448** | | **2dtm4325f_ ag2448** |
| Normal Colon GENPAK 061003 | 16.2 | Kidney NAT Clontech 8120608 | 0.0 |
| 83219 CC Well to Mod Diff (OD03866) | 0.0 | Kidney Cancer Clontech 8120613 | 0.0 |
| 83220 CC NAT (OD03866) | 11.9 | Kidney NAT Clontech 8120614 | 0.0 |
| 83221 CC Gr.2 rectosigmoid (OD03868) | 0.0 | Kidney Cancer Clontech 9010320 | 6.2 |
| 83222 CC NAT (ODO3868) | 0.0 | Kidney NAT Clontech 9010321 | 0.0 |
| 83235 CC Mod Diff (OD03920) | 0.0 | Normal Uterus GENPAK 061018 | 5.6 |
| 83236 CC NAT (ODO3920) | 0.0 | Uterus Cancer GENPAK 064011 | 29.3 |
| 83237 CC Gr.2 ascend colon (ODO3921) | 4.0 | Normal Thyroid Clontech A+ 6570-1 | 5.7 |
| 83238 CC NAT (ODO3921) | 6.7 | Thyroid Cancer GENPAK 064010 | 0.0 |
| 83241 CC from Partial Hepatectomy (ODO4309) | 0.0 | Thyroid Cancer INVITROGEN A302152 | 0.0 |
| 83242 Liver NAT (ODO4309) | 13.3 | Thyroid NAT INVITROGEN A302153 | 43.5 |
| 87472 Colon mets to lung (OD04451-01) | 0.0 | Normal Breast GENPAK 061019 | 7.6 |
| 87473 Lung NAT (OD04451-02) | 7.9 | 84877 Breast Cancer (OD04566) | 0.0 |
| Normal Prostate Clontech A+ 6546-1 | 0.0 | 85975 Breast Cancer (OD04590-01) | 0.0 |
| 84140 Prostate Cancer (OD04410) | 0.0 | 85976 Breast Cancer Mets (OD04590-03) | 4.9 |
| 84141 Prostate NAT (OD04410) | 0.0 | 87070 Breast Cancer Metastasis (OD04655-05) | 11.2 |
| 87073 Prostate Cancer (OD04720-01) | 0.0 | GENPAK Breast Cancer 064006 | 0.0 |
| 87074 Prostate NAT (OD04720-02) | 0.0 | Breast Cancer Res. Gen. 1024 | 5.6 |
| Normal Lung GENPAK 061010 | 12.2 | Breast Cancer Clontech 9100266 | 0.0 |
| 83239 Lung Met to Muscle (ODO4286) | 2.3 | Breast NAT Clontech 9100265 | 0.0 |
| 83240 Muscle NAT (ODO4286) | 3.6 | Breast Cancer INVITROGEN A209073 | 4.0 |
| 84136 Lung Malignant Cancer (OD03126) | 0.0 | Breast NAT INVITROGEN A2090734 | 9.3 |
| 84137 Lung NAT (OD03126) | 93.3 | Normal Liver GENPAK 061009 | 0.0 |
| 84871 Lung Cancer (OD04404) | 18.6 | Liver Cancer GENPAK 064003 | 0.0 |
| 84872 Lung NAT (OD04404) | 0.0 | Liver Cancer Research Genetics RNA 1025 | 0.0 |
| 84875 Lung Cancer (OD04565) | 0.0 | Liver Cancer Research Genetics RNA 1026 | 9.7 |
| 84876 Lung NAT (OD04565) | 4.2 | Paired Liver Cancer Tissue Research Genetics RNA 6004-T | 9.5 |
| 85950 Lung Cancer (OD04237-01) | 12.3 | Paired Liver Tissue Research Genetics RNA 6004-N | 0.0 |
| 85970 Lung NAT (OD04237-02) | 0.0 | Paired Liver Cancer Tissue Research Genetics RNA 6005-T | 6.2 |
| 83255 Ocular Mel Met to Liver (ODO4310) | 100.0 | Paired Liver Tissue Research Genetics RNA 6005-N | 0.0 |
| 83256 Liver NAT (ODO4310) | 0.0 | Normal Bladder GENPAK 061001 | 0.0 |
| 84139 Melanoma Mets to Lung (OD04321) | 3.8 | Bladder Cancer Research Genetics RNA 1023 | 0.0 |
| 84138 Lung NAT (OD04321) | 0.0 | Bladder Cancer INVITROGEN A302173 | 16.3 |
| Normal Kidney GENPAK 061008 | 12.2 | 87071 Bladder Cancer (OD04718-O1) | 0.0 |
| 83786 Kidney Ca, Nuclear grade 2 (OD04338) | 2.5 | 87072 Bladder Normal Adjacent (OD04718-03) | 0.0 |
| 83787 Kidney NAT (OD04338) | 4.6 | Normal Ovary Res. Gen. | 0.0 |
| 83788 Kidney Ca Nuclear grade 1/2 (OD04339) | 3.6 | Ovarian Cancer GENPAK 064008 | 3.8 |
| 83789 Kidney NAT (OD04339) | 0.0 | 87492 Ovary Cancer (OD04768-07) | 0.0 |
| 83790 Kidney Ca, Clear cell type (OD04340) | 4.1 | 87493 Ovary NAT (OD04768-08) | 0.0 |
| 83791 Kidney NAT (OD04340) | 6.4 | Normal Stomach GENPAK 061017 | 0.0 |
| 83792 Kidney Ca, Nuclear grade 3 (OD04348) | 0.0 | Gastric Cancer Clontech 9060358 | 0.0 |
| 83793 Kidney NAT (OD04348) | 2.6 | NAT Stomach Clontech 9060359 | 0.0 |
| 87474 Kidney Cancer (OD04622-01) | 0.0 | Gastric Cancer Clontech 9060395 | 13.2 |
| 87475 Kidney NAT (OD04622-03) | 0.0 | NAT Stomach Clontech 9060394 | 6.3 |
| 85973 Kidney Cancer (OD04450-01) | 0.0 | Gastric Cancer Clontech 9060397 | 0.0 |
| 85974 Kidney NAT (OD04450-03) | 0.0 | NAT Stomach Clontech 9060396 | 0.0 |
| Kidney Cancer Clontech 8120607 | 0.0 | Gastric Cancer GENPAK 064005 | 0.0 |

**Table 24. Panel 4D**

| **Tissue Name** | **Relative Expression(%)** | **Tissue Name** | **Relative Expression(%)** |
|---|---|---|---|
| | **4dx4tm5099f_ ag1631_a1** | | **4dx4tm5099f_ ag1631_a1** |
| 93768_Secondary Th1_anti-CD28/anti-CD3 | 0.0 | 93100_HUVEC (Endothelial)_IL-1b | 0.0 |
| 93769_Secondary Th2_anti-CD28/anti-CD3 | 53.2 | 93779_HUVEC (Endothelial)_IFN gamma | 0.0 |
| 93770_Secondary Tr1_anti-CD28/anti-CD3 | 38.0 | 93102_HUVEC (Endothelial)_TNF alpha + IFN gamma | 0.0 |
| 93573_Secondary Th1_resting day 4-6 in IL-2 | 25.4 | 93101_HUVEC (Endothelial)_TNF alpha + IL4 | 0.0 |
| 93572_Secondary Th2_resting day 4-6 in IL-2 | 0.0 | 93781_HUVEC (Endothelial)_IL-11 | 0.0 |
| 93571_Secondary Tr1_resting day 4-6 in IL-2 | 15.7 | 93583_Lung Microvascular Endothelial Cells_none | 0.0 |
| 93568_primary Th1_anti-CD28/anti-CD3 | 0.0 | 93584_Lung Microvascular Endothelial Cells_TNFa (4 ng/ml) and ILlb (1 ng/ml) | 0.0 |
| 93569_primary Th2_anti-CD28/anti-CD3 | 0.0 | 92662_Microvascular Dermal endothelium_none | 0.0 |
| 93570_primary Tr1_anti-CD28/anti-CD3 | 17.9 | 92663_Microsvasular Dermal endothelium_TNFa (4 ng/ml) and ILlb (1 ng/ml) | 0.0 |
| 93565_primary Th1_resting dy 4-6 in IL-2 | 43.4 | 93773_Bronchial epithelium_TNFa (4 ng/ml) and IL1b (1 ng/ml)** | 0.0 |
| 93566_primary Th2_resting dy 4-6 in IL-2 | 37.8 | 93347_Small Airway Epithelium none | 0.0 |
| 93567_primary Tr1_resting dy 4-6 in IL-2 | 0.0 | 93348_Small Airway Epithelium_TNFa (4 ng/ml) and ILlb (1 ng/ml) | 0.0 |
| 93351_CD45RA CD4 lymphocyte_anti-CD28/anti-CD3 | 0.0 | 92668_Coronery Artery SMC_resting | 0.0 |
| 93352_CD45RO CD4 lymphocyte_anti-CD28/anti-CD3 | 0.0 | 92669_Coronery Artery SMC_TNFa (4 ng/ml) and ILlb (1 ng/ml) | 0.0 |
| 93251_CD8 Lymphocytes_anti-CD28/anti-CD3 | 0.0 | 93107_astrocytes_resting | 40.8 |
| 93353_chronic CD8 Lymphocytes 2ry_resting dy 4-6 in IL-2 | 0.0 | 93108_astrocytes_TNFa (4 ng/ml) and ILlb (1 ng/ml) | 9.9 |
| 93574_chronic CD8 Lymphocytes 2ry_activated CD3/CD28 | 17.9 | 92666_KU-812 (Basophil)_resting | 1.8 |
| 93354_CD4_none | 25.7 | 92667_KU-812 (Basophil)_PMA/ionoycin | 0.0 |
| 93252_Secondary Th1/Th2/Tr1_anti-CD95 CH11 | 42.8 | 93579_CCD1106 (Keratinocytes)_none | 0.0 |
| 93103_LAK cells_resting | 0.0 | 93580_CCD1106 (Keratinocytes)_TNFa and IFNg** | 0.0 |
| 93788_LAK cells_IL-2 | 0.0 | 93791_Liver Cirrhosis | 54.5 |
| 93787_LAK cells_IL-2+IL-12 | 19.5 | 93792_Lupus Kidney | 0.0 |
| 93789_LAK cells_IL-2+IFN gamma | 18.6 | 93577_NCI-H292 | 15.9 |
| 93790_LAK cells_IL-2+IL-18 | 50.8 | 93358_NCI-H292_IL-4 | 0.0 |
| 93104_LAK cells_PMA/ionomycin and IL- 18 | 23.4 | 93360_NCI-H292_IL-9 | 0.0 |
| 93578_NK Cells IL-2_resting | 25.1 | 93359_NCI-H292_IL-13 | 0.0 |
| 93109_Mixed Lymphocyte Reaction_Two Way MLR | 18.9 | 93357_NCI-H292_IFN gamma | 0.0 |
| 93110_Mixed Lymphocyte Reaction_Two Way MLR | 16.7 | 93777_HPAEC - | 14.2 |
| 93111_Mixed Lymphocyte Reaction_Two Way MLR | 0.0 | 93778_HPAEC_IL-1 beta/TNA alpha | 0.0 |
| 93112_Mononuclear Cells (PBMCs)_resting | 14.5 | 93254_Normal Human Lung Fibroblast_none | 0.0 |
| 93113_Mononuclear Cells (PBMCs)_PWM | 0.0 | 93253_Normal Human Lung Fibroblast_TNFa (4 ng/ml) and IL-1b (1 ng/ml) | 0.0 |
| 93114_Mononuclear Cells (PBMCs)_PHA-L | 0.0 | 93257_Normal Human Lung Fibroblast_IL-4 | 0.0 |
| 93249_Ramos (B cell)_none | 0.0 | 93256_Normal Human Lung Fibroblast_IL-9 | 0.0 |
| 93250_Ramos (B cell)_ionomycin | 19.4 | 93255_Normal Human Lung Fibroblast_IL-13 | 0.0 |
| 93349_B lymphocytes_PWM | 100.0 | 93258_Normal Human Lung Fibroblast_IFN gamma | 0.0 |
| 93350_B lymphoytes_CD40L and IL-4 | 90.7 | 93106_Dermal Fibroblasts CCD1070_resting | 0.0 |
| 92665_EOL-1 (Eosinophil)_dbcAMP differentiated | 31.1 | 93361_Dermal Fibroblasts CCD1070_TNF alpha 4 ng/ml | 0.0 |
| 93248_EOL-1 (Eosinophil)_dbcANT/PMAion omycin | 0.0 | 93105_Dermal Fibroblasts CCD1070_IL-1 beta 1 ng/ml | 0.0 |
| 93356_Dendritic Cells_none | 0.0 | 93772_dermal fibroblast_IFN gamma | 0.0 |
| 93355_Dendritic Cells_LPS 100 ng/ml | 0.0 | 93771_dermal fibroblast IL-4 | 0.0 |
| 93775_Dendritic Cells_anti-CD40 | 0.0 | 93259_IBD Colitis 1** | 0.0 |
| 93774_Monocytes_resting | 0.0 | 93260_IBD Colitis 2 | 19.6 |
| 93776_Monocytes_LPS 50 ng/ml | 0.0 | 93261_IBD Crohns | 0.0 |
| 93581_Macrophages_resting | 0.0 | 735010_Colon_normal | 0.0 |
| 93582_Macrophages_LPS 100 ng/ml | 0.0 | 735019_Lung_none | 0.0 |
| 93098_HUVEC (Endothelial)_none | 0.0 | 64028-1_Thymus_none | 0.0 |
| 93099_HUVEC (Endothelial)_starved | 0.0 | 64030-1_Kidney_none | 38.4 |

**Panel 1.3D Summary:** Ag1631/Ag2448 Expression of the NOV7 gene is low/undetectable (CT values > 35) across the samples on this panel (data not shown).

**Panel 2D Summary:** Ag2448 The expression of the NOV7 gene is highest in a sample derived from an ocular melanoma metastasis (CT = 34.3). Other observed expression of this gene on this panel is below the level of reliable detection. Ocular melanoma is a unique form of melanoma, and thus, the expression of this gene could be used to distinguish ocular melanoma metastases from other melanomas and also other tissues in general. In addition, therapeutic modulation of the NOV7 gene product, through the use of antibodies or small molecule drugs might be of use in the treatment of ocular melanoma.

**Panel 2.2 Summary**: Ag1631 Expression of the NOV7 gene is low/undetectable (CT values > 35) across the samples on this panel (data not shown).

**Panel 4D Summary:** Ag 1631 The NOV7 gene is expressed in B cells treated with poke weed mitogen or with CD40L and IL-4 (CT = 34). The protein encoded for by this gene has homology to thymosin-beta 4, which is also upregulated during B cell activation (ref. 1). Therefore, the NOV7 protein may have functions similar to those of other thymosin-beta 4 homologs, including induction of metallo-proteinases, chemotaxis, angiogenesis and inhibition of inflammation as well as the inhibition of bone marrow stem cell proliferation (ref. 2). Thus, protein therapeutics designed with the protein encoded for by the NOV7 gene could be useful for treating inflammatory diseases such as arthritis, asthma, diabetes, lupus, psorisis, and allergy. Ag2448 Expression of the NOV7 gene is low/undetectable (CT values > 35) across the samples on this panel (data not shown).

### References:

1. Gondo H., Kudo J., White J.W., Barr C., Selvanayagam P., Saunders G.F. (1987) Differential expression of the human thymosin-beta 4 gene in lymphocytes, macrophages, and granulocytes. J. Immunol. 139: 3840-3848.

A cDNA clone encoding human thymosin-beta 4 was isolated from a cDNA library prepared from peripheral blood leukocytes of a patient with acute lymphocytic leukemia. This clone contained the entire coding sequence of 43 amino acid residues of thymosin-beta 4 and had an initiation codon and two termination codons. The amino acid and nucleotide sequences in the coding region were well conserved between rat and human. Nine of 132 nucleotides were different in the coding sequences (93% homology), but the deduced amino acid sequences were identical. No signal peptide was found in the deduced protein sequence. Human thymosin-beta 4 mRNA, approximately 830 nucleotides in length, was about 30 nucleotides larger than rat thymosin-beta 4 mRNA. Expression of the human thymosin-beta 4 gene in various primary myeloid and lymphoid malignant cells and in a few human hemopoietic cell lines was studied. Northern blot analyses of different neoplastic B lymphocytes revealed that steady state levels of thymosin-beta 4 mRNA varied as a function of differentiation stage. Thymosin-beta 4 mRNA levels were decreased in myeloma cells as are class II human leukocyte antigen, Fc receptor, and complement receptor, suggesting a relationship between thymosin-beta 4 and the immune response. Thymosin-beta 4 mRNA was more highly expressed in mature granulocytes than in immature blastic cells. Treatment of THP-1 cells, a human monocytic cell line, with recombinant human interferon-lambda reduced the levels of thymosin-beta 4 mRNA. Its level decreased after differentiation of THP-1 cells into Ia+ macrophages, but increased after differentiation of HL-60 cells into Ia- macrophages. The pattern of thymosin-beta 4 gene expression suggests that it may play a fundamental role in the host defense mechanism.

PMID: 3500230

2. Huff T., Muller C.S., Otto A.M., Netzker R, Hannappel E. (2001) beta-Thymosins, small acidic peptides with multiple functions. Int. J. Biochem. Cell. Biol. 33: 205-220.

The beta-thymosins are a family of highly conserved polar 5 kDa peptides originally thought to be thymic hormones. About 10 years ago, thymosin beta(4) as well as other members of this ubiquitous peptide family were identified as the main intracellular G-actin sequestering peptides, being present in high concentrations in almost every cell. beta-Thymosins bind monomeric actin in a 1:1 complex and act as actin buffers, preventing polymerization into actin filaments but supplying a pool of actin monomers when the cell needs filaments. Changes in the expression of beta-thymosins appear to be related to the differentiation of cells. Increased expression of beta-thymosins or even the synthesis of a beta-thymosin normally not expressed might promote metastasis possibly by increasing mobility of the cells. Thymosin beta(4) is detected outside of cells in blood plasma or in wound fluid. Several biological effects are attributed to thymosin beta(4), oxidized thymosin beta(4), or to the fragment, acSDKP, possibly generated from thymosin beta(4). Among the effects are induction of metallo-proteinases, chemotaxis, angiogenesis and inhibition of inflammation as well as the inhibition of bone marrow stem cell proliferation. However, nothing is known about the molecular mechanisms mediating the effects attributed to extracellular beta-thymosins.

PMID: 11311852

### NOV8

Expression of geneNOV8 was assessed using the primer-probe set Ag2447, described in Table 25.

**Table 25. Probe Name Ag2447**

| Primers | Sequences | TM | Length | Start Position | SEQ ID NO: |
|---|---|---|---|---|---|
| Forward | 5'-AAAACCTTAGCGCTTGCTTT-3' | 58.3 | 20 | 944 | 135 |
| Probe | TET-5'-TCATTCACCGAACAAAATAATGACTG A-3'-TAMRA | 64.7 | 27 | 966 | 136 |
| Reverse | 5'-AGACCAATTCTGTTCCCATTG-3' | 58.9 | 21 | 999 | 137 |

Expression of this gene is low/undetectable (CT values > 35) across the samples on Panels 1.3D, 2D, and 4D (data not shown).

### Example 3. SNP analysis of NOVX clones

**SeqCallingTM Technology:** cDNA was derived from various human samples representing multiple tissue types, normal and diseased states, physiological states, and developmental states from different donors. Samples were obtained as whole tissue, cell lines, primary cells or tissue cultured primary cells and cell lines. Cells and cell lines may have been treated with biological or chemical agents that regulate gene expression for example, growth factors, chemokines, steroids. The cDNA thus derived was then sequenced using CuraGen's proprietary SeqCalling technology. Sequence traces were evaluated manually and edited for corrections if appropriate. cDNA sequences from all samples were assembled with themselves and with public ESTs using bioinformatics programs to generate CuraGen's human SeqCalling database of SeqCalling assemblies. Each assembly contains one or more overlapping cDNA sequences derived from one or more human samples. Fragments and ESTs were included as components for an assembly when the extent of identity with another component of the assembly was at least 95% over 50 bp. Each assembly can represent a gene and/or its variants such as splice forms and/or single nucleotide polymorphisms (SNPs) and their combinations.

Variant sequences are included in this application. A variant sequence can include a single nucleotide polymorphism (SNP). A SNP can, in some instances, be referred to as a "cSNP" to denote that the nucleotide sequence containing the SNP originates as a cDNA. A SNP can arise in several ways. For example, a SNP may be due to a substitution of one nucleotide for another at the polymorphic site. Such a substitution can be either a transition or a transversion. A SNP can also arise from a deletion of a nucleotide or an insertion of a nucleotide, relative to a reference allele. In this case, the polymorphic site is a site at which one allele bears a gap with respect to a particular nucleotide in another allele. SNPs occurring within genes may result in an alteration of the amino acid encoded by the gene at the position of the SNP. Intragenic SNPs may also be silent, however, in the case that a codon including a SNP encodes the same amino acid as a result of the redundancy of the genetic code. SNPs occurring outside the region of a gene, or in an intron within a gene, do not result in changes in any amino acid sequence of a protein but may result in altered regulation of the expression pattern for example, alteration in temporal expression, physiological response regulation, cell type expression regulation, intensity of expression, stability of transcribed message.

**Method of novel SNP Identification:** SNPs are identified by analyzing sequence assemblies using CuraGen's proprietary SNPTool algorithm. SNPToo1 identifies variation in assemblies with the following criteria: SNPs are not analyzed within 10 base pairs on both ends of an alignment; Window size (number of bases in a view) is 10; The allowed number of mismatches in a window is 2; Minimum SNP base quality (PHRED score) is 23; Minimum number of changes to score an SNP is 2/assembly position. SNPTool analyzes the assembly and displays SNP positions, associated individual variant sequences in the assembly, the depth of the assembly at that given position, the putative assembly allele frequency, and the SNP sequence variation. Sequence traces are then selected and brought into view for manual validation. The consensus assembly sequence is imported into CuraTools along with variant sequence changes to identify potential amino acid changes resulting from the SNP sequence variation. Comprehensive SNP data analysis is then exported into the SNPCalling database.

**Method of novel SNP Confirmation:** SNPs are confirmed employing a validated method know as Pyrosequencing (Pyrosequencing, Westborough, MA). Detailed protocols for Pyrosequencing can be found in: Alderborn et al. Determination of Single Nucleotide Polymorphisms by Real-time Pyrophosphate DNA Sequencing. (2000). Genome Research. 10, Issue 8, August. 1249-1265. In brief, Pyrosequencing is a real time primer extension process of genotyping. This protocol takes double-stranded, biotinylated PCR products from genomic DNA samples and binds them to streptavidin beads. These beads are then denatured producing single stranded bound DNA. SNPs are characterized utilizing a technique based on an indirect bioluminometric assay of pyrophosphate (PPi) that is released from each dNTP upon DNA chain elongation. Following Klenow polymerase-mediated base incorporation, PPi is released and used as a substrate, together with adenosine 5'-phosphosulfate (APS), for ATP sulfurylase, which results in the formation of ATP. Subsequently, the ATP accomplishes the conversion of luciferin to its oxi-derivative by the action of luciferase. The ensuing light output becomes proportional to the number of added bases, up to about four bases. To allow processivity of the method dNTP excess is degraded by apyrase, which is also present in the starting reaction mixture, so that only dNTPs are added to the template during the sequencing. The process has been fully automated and adapted to a 96-well format, which allows rapid screening of large SNP panels. The DNA and protein sequences for the novel single nucleotide polymorphic variants are reported. Variants are reported individually but any combination of all or a select subset of variants are also included. In addition, the positions of the variant bases and the variant amino acid residues are underlined.

### Results

Variants are reported individually but any combination of all or a select subset of variants are also included as contemplated NOVX embodiments of the invention.

### NOV1 SNP data:

NOV1 has seven SNP variants, whose variant positions for its nucleotide and amino acid sequences is numbered according to SEQ ID NOs:1 and 2, respectively. The nucleotide sequence of the NOV1 variant differs as shown in Table 26.

| **Table 26. cSNP and Coding Variants for NOV1** | | | | |
|---|---|---|---|---|
| **NT Position of cSNP** | **Wild Type NT** | **Variant NT** | **Amino Acid position** | **Amino Acid Change** |
| 311 | A | G | 10 | No change |
| 339 | A | G | 20 | T->A |
| 426 | T | C | 49 | F->L |
| 479 | T | C | 66 | No change |
| 480 | G | A | 67 | D->N |
| 490 | A | G | 70 | E->G |
| 505 | A | G | 75 | E->G |

### NOV4 SNP data:

In the following positions, one or more consensus positions (Cons. Pos.) of the nucleotide sequence have been identified as SNPs. "Depth" rerepresents the number of clones covering the region of the SNP. The Putative Allele Frequency (Putative Allele Freq.) is the fraction of all the clones containing the SNP. A dash ("-"), when shown, means that a base is not present. The sign ">" means "is changed to".
Cons.Pos.: 97 Depth: 118 Change: C > A Putative Allele Freq.: 0.034 -> 133000338(+,i) unrev. Fpos: 81
   -> 133000555(+,i) unrev. Fpos: 69
   -> 133001366(+,i) unrev. Fpos: 68
   -> 133001516(+,i) unrev. Fpos: 61
Cons.Pos.: 97 Depth: 118 Change: C > G Putative Allele Freq.: 0.017 -> 133741176(+,i) unrev. Fpos: 107
   -> 133742288(-,i) unrev. Fpos: 379
Cons.Pos.: 116 Depth: 220 Change: C > - Putative Allele Freq.: 0.009 -> 132999287(+,i) unrev. Fpos: 67
   -> 133012157(-,i) unrev. Fpos: 387
Cons.Pos.: 122 Depth: 220 Change: T > C Putative Allele Freq.: 0.009 -> 133001338(+,i) unrev. Fpos: 88
   -> 133018706(-,i) unrev. Fpos: 360
Cons.Pos.: 130 Depth: 220 Change: C > T Putative Allele Freq.: 0.009 -> 133001002(-,i) unrev. Fpos: 334
   -> 133018604(+,i) unrev. Fpos: 117
Cons.Pos.: 203 Depth: 219 Change: G > A Putative Allele Freq.: 0.009 -> 133001421(+,i) unrev. Fpos: 151
   -> 133018747(-,i) unrev. Fpos: 289
Cons.Pos.: 204 Depth: 219 Change: C > T Putative Allele Freq.: 0.009 -> 132999373(+,i) unrev. Fpos: 150
   -> 133012199(-,i) unrev. Fpos: 304
Cons.Pos.: 221 Depth: 219 Change: A > G Putative Allele Freq.: 0.009 -> 133001494(-,i) unrev. Fpos: 278
   -> 133018759(+,i) unrev. Fpos: 200
Cons.Pos.: 235 Depth: 219 Change: A > G Putative Allele Freq.: 0.009 -> 133001613(+,i) unrev. Fpos: 182
   -> 133018773(-,i) unrev. Fpos: 278
Cons.Pos.: 243 Depth: 219 Change: A > G Putative Allele Freq.: 0.009 -> 133001409(+,i) unrev. Fpos: 180
   -> 133018744(-,i) unrev. Fpos: 253
Cons.Pos.: 249 Depth: 219 Change: G > A Putative Allele Freq.: 0.009 -> 133001292(+,i) unrev. Fpos: 195
   -> 133018686(-,i) unrev. Fpos: 246
Cons.Pos.: 255 Depth: 219 Change: C > T Putative Allele Freq.: 0.009 -> 133006504(-,i) unrev. Fpos: 230
   -> 133026853(+,i) unrev. Fpos: 250
Cons.Pos.: 300 Depth: 273 Change: G > A Putative Allele Freq.: 0.007 -> 133555265(-,i) unrev. Fpos: 208
   -> 133634522(+,i) unrev. Fpos: 84
Cons.Pos.: 308 Depth: 273 Change: G > A Putative Allele Freq.: 0.007 -> 133001516(+,i). unrev. Fpos: 254
   -> 133018761(-,i) unrev. Fpos: 190
Cons.Pos.: 330 Depth: 273 Change: C > T Putative Allele Freq.: 0.007 -> 133001366(+,i) unrev. Fpos: 282
   -> 133018735(-,i) unrev. Fpos: 171
Cons.Pos.: 339 Depth: 273 Change: T > C Putative Allele Freq.: 0.007 -> 133605071(-,i) unrev. Fpos: 167
   -> 133635377(+,i) unrev. Fpos: 122
Cons.Pos.: 354 Depth: 273 Change: A > T Putative Allele Freq.: 0.007 -> 132999419(-,i) unrev. Fpos: 132
   -> 133012245(+,i) unrev. Fpos: 325
Cons.Pos.: 358 Depth: 273 Change: G > A Putative Allele Freq.: 0.007 -> 133001351(+,i) unrev. Fpos: 301
   -> 133018729(-,i) unrev. Fpos: 151.

### NOV5a SNP data:

NOV5a has four SNP variants, whose variant positions for its nucleotide and amino acid sequences is numbered according to SEQ ID NOs:X and Y, respectively. The nucleotide sequence of the NOV5a variant differs as shown in Table 27.

| **Table 27. cSNP and Coding Variants for NOV5a** | | | | |
|---|---|---|---|---|
| **NT Position of cSNP** | **Wild Type NT** | **Variant NT** | **Amino Acid position** | **Amino Acid Change** |
| 986 | C | A | 319 | No change |
| 1276 | C | T | 416 | S->L |
| 1612 | T | C | 528 | L->P |
| 1632 | G | A | 535 | A->T |

### NOVSb SNP data:

NOV5b has three SNP variants, whose variant positions for its nucleotide and amino acid sequences is numbered according to SEQ ID NOs:X and Y, respectively. The nucleotide sequence of the NOV5b variant differs as shown in Table 28.

| **Table 28. cSNP and Coding Variants for NOV5b** | | | | |
|---|---|---|---|---|
| **NT Position of cSNP** | **Wild Type NT** | **Variant NT** | **Amino Acid position** | **Amino Acid Change** |
| 1375 | C | T | 449 | S->L |
| 1711 | T | C | 561 | L->P |
| 1731 | G | A | 568 | A->T |

### NOV5g SNP data:

In the following positions, one or more consensus positions (Cons. Pos.) of the nucleotide sequence have been identified as SNPs. "Depth" rerepresents the number of clones covering the region of the SNP. The Putative Allele Frequency (Putative Allele Freq.) is the fraction of all the clones containing the SNP. A dash ("-"), when shown, means that a base is not present. The sign ">" means "is changed to".

Cons.Pos.: 814 Depth: 13 Change: C > A Putative Allele Freq.: 0.231

### NOV5h SNP data:

In the following positions, one or more consensus positions (Cons. Pos.) of the nucleotide sequence have been identified as SNPs. "Depth" rerepresents the number of clones covering the region of the SNP. The Putative Allele Frequency (Putative Allele Freq.) is the fraction of all the clones containing the SNP. A dash ("-"), when shown, means that a base is not present. The sign ">" means "is changed to".

Cons.Pos.: 820 Depth: 8 Change: G > - Putative Allele Freq.: 0.250

### NOV8 SNP data:

NOV8 has one SNP variant, whose variant position for its nucleotide and amino acid sequences is numbered according to SEQ ID NOs:X and Y, respectively. The nucleotide sequence of the NOV8 variant differs as shown in Table 29.

| **Table 29. cSNP and Coding Variants for NOV8** | | | | |
|---|---|---|---|---|
| **NT Position of cSNP** | **Wild Type NT** | **Variant NT** | **Amino Acid position** | **Amino Acid Change** |
| 1276 | C | G | 407 | T->R |

### Example 4: Sage analysis for NOV3

### 6A: Unigene Cluster Hs. 17270

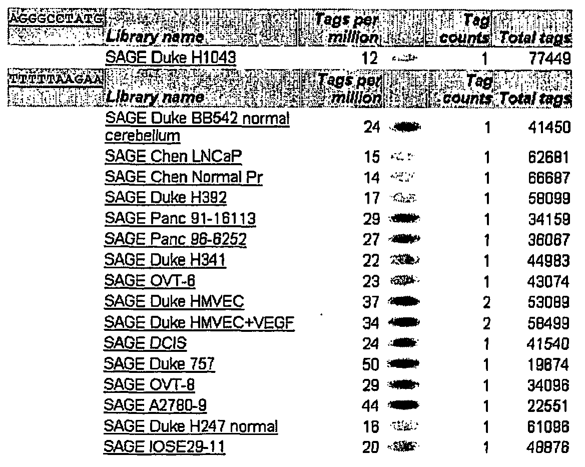

### 6B. Unigene cluster 274136

UniGene cluster: Hs. 274136 Submit
Hs.274136 : **hypothetical protein FLJ11052**
SAGE library data and reliable tag summary:
Reliable tags found in SAGE libraries:

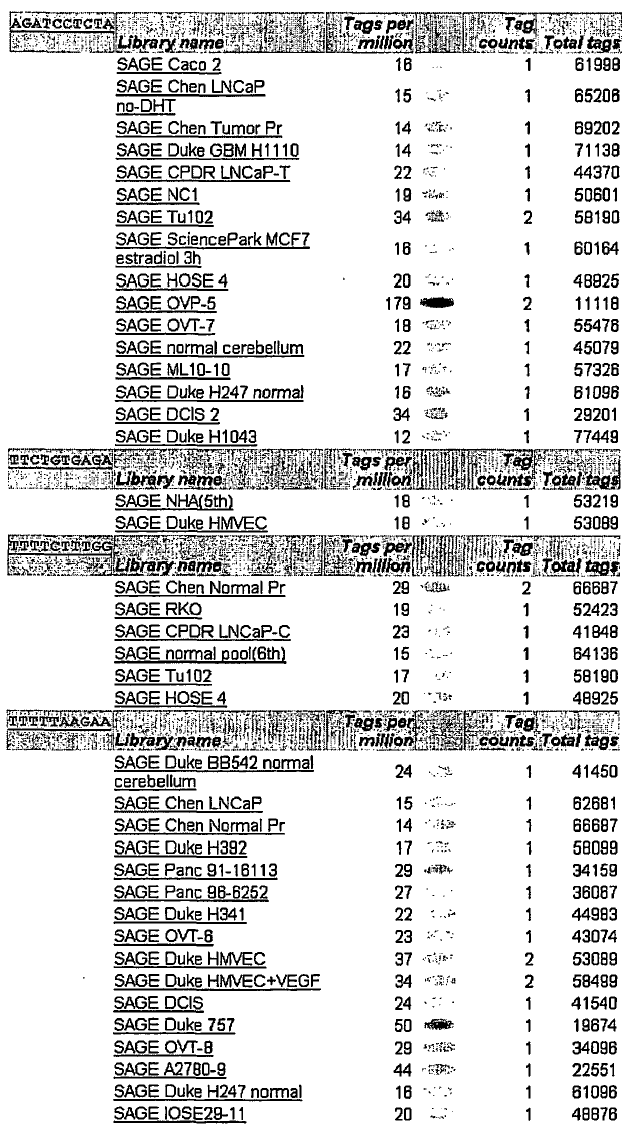

### OTHER EMBODIMENTS

Although particular embodiments have been disclosed herein in detail, this has been done by way of example for purposes of illustration only, and is not intended to be limiting with respect to the scope of the appended claims, which follow. In particular, it is contemplated by the inventors that various substitutions, alterations, and modifications may be made to the invention without departing from the spirit and scope of the invention as defined by the claims. The choice of nucleic acid starting material, clone of interest, or library type is believed to be a matter of routine for a person of ordinary skill in the art with knowledge of the embodiments described herein. Other aspects, advantages, and modifications considered to be within the scope of the following claims.

## Claims

1. An isolated polypeptide comprising an amino acid sequence selected from the group consisting of:
(a) a mature form of an amino acid sequence selected from the group consisting of SEQ ID NOS: 31, 2, 4, 6, 8, 11, 13, 15, 17, 19, 21, 23, 25, 27, 29, 33, 35, and 37;
(b) a variant of a mature form of an amino acid sequence selected from the group consisting of SEQ ID NOS: 31, 2, 4, 6, 8, 11, 13, 15, 17, 19, 21, 23, 25, 27, 29, 33, 35, and 37, wherein one or more amino acid residues in said variant differs from the amino acid sequence of said mature form, provided that said variant differs in no more than 15% of the amino acid residues from the amino acid sequence of said mature form;
(c) an amino acid sequence selected from the group consisting of SEQ ID NOS: 31, 2, 4, 6, 8, 11, 13, 15, 17, 19, 21, 23, 25, 27, 29, 33, 35, and 37; and
(d) a variant of an amino acid sequence selected from the group consisting of SEQ ID NOS: 31, 2, 4, 6, 8, 11, 13, 15, 17, 19, 21, 23, 25, 27, 29, 33, 35, and 37, wherein one or more amino acid residues in said variant differs from the amino acid sequence of said mature form, provided that said variant differs in no more than 15% of amino acid residues from said amino acid sequence.

2. The polypeptide of claim 1, wherein said polypeptide comprises the amino acid sequence of a naturally-occurring allelic variant of an amino acid sequence selected from the group consisting SEQ ID NOS: 31, 2, 4, 6, 8, 11, 13, 15, 17, 19, 21, 23, 25, 27, 29, 33, 35, and 37.

3. The polypeptide of claim 2, wherein said allelic variant comprises an amino acid sequence that is the translation of a nucleic acid sequence differing by a single nucleotide from a nucleic acid selected from the group consisting of SEQ ID NOS: 30, 1, 3, 5, 7, 9, 12, 14, 16, 18, 20, 22, 24, 26, 28, 32, 34, and 36.

4. The polypeptide of claim 1, wherein the amino acid sequence of said variant comprises a conservative amino acid substitution.

5. An isolated nucleic acid molecule comprising a nucleic acid sequence encoding a polypeptide comprising an amino acid sequence selected from the group consisting of:
(a) a mature form of an amino acid sequence selected from the group consisting of SEQ ID NOS: 31, 2, 4, 6, 8, 11, 13, 15, 17, 19, 21, 23, 25, 27, 29, 33, 35, and 37;
(b) a variant of a mature form of an amino acid sequence selected from the group consisting of SEQ ID NOS: 31, 2, 4, 6, 8, 11, 13, 15, 17, 19, 21, 23, 25, 27, 29, 33, 35, and 37, wherein one or more amino acid residues in said variant differs from the amino acid sequence of said mature form, provided that said variant differs in no more than 15% of the amino acid residues from the amino acid sequence of said mature form;
(c) an amino acid sequence selected from the group consisting of SEQ ID NOS: 31, 2, 4, 6, 8, 11, 13, 15, 17, 19, 21, 23, 25, 27, 29, 33, 35, and 37;
(d) a variant of an amino acid sequence selected from the group consisting SEQ ID NOS: 31, 2, 4, 6, 8, 11, 13, 15, 17, 19, 21, 23, 25, 27, 29, 33, 35, and 37, wherein one or more amino acid residues in said variant differs from the amino acid sequence of said mature form, provided that said variant differs in no more than 15% of amino acid residues from said amino acid sequence;
(e) a nucleic acid fragment encoding at least a portion of a polypeptide comprising an amino acid sequence chosen from the group consisting of SEQ ID NOS: 31, 2, 4, 6, 8, 11, 13, 15, 17, 19, 21, 23, 25, 27, 29, 33, 35, and 37, or a variant of said polypeptide, wherein one or more amino acid residues in said variant differs from the amino acid sequence of said mature form, provided that said variant differs in no more than 15% of amino acid residues from said amino acid sequence; and
(f) a nucleic acid molecule comprising the complement of (a), (b), (c), (d) or (e).

6. The nucleic acid molecule of claim 5, wherein the nucleic acid molecule comprises the nucleotide sequence of a naturally-occurring allelic nucleic acid variant.

7. The nucleic acid molecule of claim 5, wherein the nucleic acid molecule encodes a polypeptide comprising the amino acid sequence of a naturally-occurring polypeptide variant.

8. The nucleic acid molecule of claim 5, wherein the nucleic acid molecule differs by a single nucleotide from a nucleic acid sequence selected from the group consisting of SEQ ID NOS: 30, 1, 3, 5, 7, 9, 12, 14, 16, 18, 20, 22, 24, 26, 28, 32, 34, and 36.

9. The nucleic acid molecule of claim 5, wherein said nucleic acid molecule comprises a nucleotide sequence selected from the group consisting of:
(a) a nucleotide sequence selected from the group consisting of SEQ ID NOS: 30, 1, 3, 5, 7, 9, 12, 14, 16, 18, 20, 22, 24, 26, 28, 32, 34, and 36;
(b) a nucleotide sequence differing by one or more nucleotides from a nucleotide sequence selected from the group consisting of SEQ ID NOS: 30, 1, 3, 5, 7, 9, 12, 14, 16, 18, 20, 22, 24, 26, 28, 32, 34, and 36, provided that no more that 20% of the nucleotides differ from said nucleotide sequence;
(c) a nucleic acid fragment of (a); and
(d) a nucleic acid fragment of (b).

10. The nucleic acid molecule of claim 5, wherein said nucleic acid molecule hybridizes under stringent conditions to a nucleotide sequence chosen from the group consisting SEQ ID NOS: 30, 1, 3, 5, 7, 9, 12, 14, 16, 18, 20, 22, 24, 26, 28, 32, 34, and 36, or a complement of said nucleotide sequence.

11. The nucleic acid molecule of claim 5, wherein the nucleic acid molecule comprises a nucleotide sequence selected from the group consisting of:
(a) a first nucleotide sequence comprising a coding sequence differing by one or more nucleotide sequences from a coding sequence encoding said amino acid sequence, provided that no more than 20% of the nucleotides in the coding sequence in said first nucleotide sequence differ from said coding sequence;
(b) an isolated second polynucleotide that is a complement of the first polynucleotide; and
(c) a nucleic acid fragment of (a) or (b).

12. A vector comprising the nucleic acid molecule of claim 11.

13. The vector of claim 12, further comprising a promoter operably-linked to said nucleic acid molecule.

14. A cell comprising the vector of claim 12.

15. An antibody that binds immunospecifically to the polypeptide of claim 1.

16. The antibody of claim 15, wherein said antibody is a monoclonal antibody.

17. The antibody of claim 15, wherein the antibody is a humanized antibody.

18. A method for determining the presence or amount of the polypeptide of claim 1 in a sample, the method comprising:
(a) providing the sample;
(b) contacting the sample with an antibody that binds immunospecifically to the polypeptide; and
(c) determining the presence or amount of antibody bound to said polypeptide, thereby determining the presence or amount of polypeptide in said sample.

19. A method for determining the presence or amount of the nucleic acid molecule of claim 5 in a sample, the method comprising:
(a) providing the sample;
(b) contacting the sample with a probe that binds to said nucleic acid molecule; and
(c) determining the presence or amount of the probe bound to said nucleic acid molecule,
thereby determining the presence or amount of the nucleic acid molecule in said sample.

20. The method of claim 19 wherein presence or amount of the nucleic acid molecule is used as a marker for cell or tissue type.

21. The method of claim 20 wherein the cell or tissue type is cancerous.

22. A method of identifying an agent that binds to a polypeptide of claim 1, the method comprising:
(a) contacting said polypeptide with said agent; and
(b) determining whether said agent binds to said polypeptide.

23. The method of claim 22 wherein the agent is a cellular receptor or a downstream effector.

24. A method for identifying an agent that modulates the expression or activity of the polypeptide of claim 1, the method comprising:
(a) providing a cell expressing said polypeptide;
(b) contacting the cell with said agent, and
(c) determining whether the agent modulates expression or activity of said polypeptide,
whereby an alteration in expression or activity of said peptide indicates said agent modulates expression or activity of said polypeptide.

25. A method for modulating the activity of the polypeptide of claim 1, the method comprising contacting a cell sample expressing the polypeptide of said claim with a compound that binds to said polypeptide in an amount sufficient to modulate the activity of the polypeptide.

26. A method of treating or preventing a NOVX-associated disorder, said method comprising administering to a subject in which such treatment or prevention is desired the polypeptide of claim 1 in an amount sufficient to treat or prevent said NOVX-associated disorder in said subject.

27. The method of claim 26 wherein the disorder is selected from the group consisting of cardiomyopathy and atherosclerosis.

28. The method of claim 26 wherein the disorder is related to cell signal processing and metabolic pathway modulation.

29. The method of claim 26, wherein said subject is a human.

30. A method of treating or preventing a NOVX-associated disorder, said method comprising administering to a subject in which such treatment or prevention is desired the nucleic acid of claim 5 in an amount sufficient to treat or prevent said NOVX-associated disorder in said subject.

31. The method of claim 30 wherein the disorder is selected from the group consisting of cardiomyopathy and atherosclerosis.

32. The method of claim 30 wherein the disorder is related to cell signal processing and metabolic pathway modulation.

33. The method of claim 30, wherein said subject is a human.

34. A method of treating or preventing a NOVX-associated disorder, said method comprising administering to a subject in which such treatment or prevention is desired the antibody of claim 15 in an amount sufficient to treat or prevent said NOVX-associated disorder in said subject.

35. The method of claim 34 wherein the disorder is diabetes.

36. The method of claim 34 wherein the disorder is related to cell signal processing and metabolic pathway modulation.

37. The method of claim 34, wherein the subject is a human.

38. A pharmaceutical composition comprising the polypeptide of claim 1 and a pharmaceutically-acceptable carrier.

39. A pharmaceutical composition comprising the nucleic acid molecule of claim 5 and a pharmaceutically-acceptable carrier.

40. A pharmaceutical composition comprising the antibody of claim 15 and a pharmaceutically-acceptable carrier.

41. A kit comprising in one or more containers, the pharmaceutical composition of claim 38.

42. A kit comprising in one or more containers, the pharmaceutical composition of claim 39.

43. A kit comprising in one or more containers, the pharmaceutical composition of claim 40.

44. A method for determining the presence of or predisposition to a disease associated with altered levels of the polypeptide of claim 1 in a first mammalian subject, the method comprising:
(a) measuring the level of expression of the polypeptide in a sample from the first mammalian subject; and
(b) comparing the amount of said polypeptide in the sample of step (a) to the amount of the polypeptide present in a control sample from a second mammalian subject known not to have, or not to be predisposed to, said disease;
wherein an alteration in the expression level of the polypeptide in the first subject as compared to the control sample indicates the presence of or predisposition to said disease.

45. The method of claim 44 wherein the predisposition is to cancers.

46. A method for determining the presence of or predisposition to a disease associated with altered levels of the nucleic acid molecule of claim 5 in a first mammalian subject, the method comprising:
(a) measuring the amount of the nucleic acid in a sample from the first mammalian subject; and
(b) comparing the amount of said nucleic acid in the sample of step (a) to the amount of the nucleic acid present in a control sample from a second mammalian subject known not to have or not be predisposed to, the disease;
wherein an alteration in the level of the nucleic acid in the first subject as compared to the control sample indicates the presence of or predisposition to the disease.

47. The method of claim 46 wherein the predisposition is to a cancer.

48. A method of treating a pathological state in a mammal, the method comprising administering to the mammal a polypeptide in an amount that is sufficient to alleviate the pathological state, wherein the polypeptide is a polypeptide having an amino acid sequence at least 95% identical to a polypeptide comprising an amino acid sequence of at least one of SEQ ID NOS: 31, 2, 4, 6, 8, 11, 13, 15, 17, 19, 21, 23, 25, 27, 29, 33, 35, and 37, or a biologically active fragment thereof.

49. A method of treating a pathological state in a mammal, the method comprising administering to the mammal the antibody of claim 15 in an amount sufficient to alleviate the pathological state.
